# EUROPEAN PATENT SPECIFICATION

(11) **EP 1 763 350 B1**
(45) Date of publication and mention of the grant of the patent: **21.12.2011**
(21) Application number: 04756752.4
(22) Date of filing: 06.07.2004
(51) Int. Cl.: A61K 31/496, C07D 401/00, A61P 43/00, A61P 3/04, A61P 3/06, A61P 3/10

(54) **NICOTINAMIDE DERIVATIVES AND THEIR USE AS THERAPEUTIC AGENTS**
NICOTINAMID DERIVATE UND IHRE VERWENDUNG ALS THERAPEUTIKA
DÉRIVÉS DE NICOTINAMIDE ET UTILISATION DE CEUX-CI COMME AGENTS THÉRAPEUTIQUES

(43) Date of publication of application: 21.03.2007
(60) Divisional application: 10011647.4
(73) Proprietor: Xenon Pharmaceuticals Inc., Burnaby, BC V5G 4W8 (CA)
(72) Inventor: FU, Jian-Min, Coquitlam, BC V3E 3B6 (CA); KODUMURU, Vishnumurthy, Burnaby, British Columbia V5H 3P3 (CA); SUN, Shaoyi, Coquitlam, BC V3K 1G3 (CA); WINTHER, Michael, D., Vancouver, British Columbia V6J 2K1 (CA); FINE, Richard, M., Ridgewood, NJ 07450 (US); HARVEY, Daniel, F., San Diego, CA 92122 (US); KLEBANSKY, Boris, Demarest, NJ 07627 (US); GRAY-KELLER, Mark, Madison, WI 53706 (US); GSCHWEND, Heinz, W., Santa Rosa, CA 95403 (US); LI, Wenbao, San Diego, California 92129 (US)
(74) Representative: Grünecker, Kinkeldey, Stockmair & Schwanhäusser
(86) International application number: PCT/US2004/021792
(87) International publication number: WO 2006/014168

(56) References cited:
- EP-A- 1 035 115
- EP-A- 1 156 045
- EP-A- 1 396 487
- EP-A- 1 452 525
- WO-A-01/25203
- WO-A-01/62954
- WO-A-02/10154
- WO-A-03/043636
- WO-A-03/050088
- WO-A-2004/010927
- DATABASE REGISTRY STN; Chemical Library 24 April 2003 (2003-04-24), XP002317630
- DATABASE REGISTRY STN; Chemical Library 14 October 2001 (2001-10-14), XP002317631

## Description

### FIELD OF THE INVENTION

The present invention relates generally to the field of inhibitors of stearoyl-CoA desaturase, such as nicotinamide derivatives, and uses for such compounds in treating and/or preventing various human diseases, including those mediated by stearoyl-CoA desaturase (SCD) enzymes, preferably SCD1, especially diseases related to elevated lipid levels, cardiovascular disease, diabetes, obesity, metabolic syndrome.

### BACKGROUND OF THE INVENTION

Acyl desaturase enzymes catalyze the formation of double bonds in fatty acids derived from either dietary sources or *de novo* synthesis in the liver. Mammals synthesize at least three fatty acid desaturases of differing chain length specificity that catalyze the addition of double bonds at the delta-9, delta-6, and delta-5 positions. Stearoyl-CoA desaturases (SCDs) introduce a double bond in the C9-C10 position of saturated fatty acids. The preferred substrates are palmitoyl-CoA (16:0) and stearoyl-CoA (18:0), which are converted to palmitoleoyl-CoA (16:1) and oleoyl-CoA (18:1), respectively. The resulting mono-unsaturated fatty acids are substrates for incorporation into phospholipids, triglycerides, and cholesteryl esters.

A number of mammalian SCD genes have been cloned. For example, two genes have been cloned from rat (SCD1, SCD2) and four SCD genes have been isolated from mouse (SCD1, 2, 3, and 4). While the basic biochemical role of SCD has been known in rats and mice since the 1970's (Jeffcoat, R. et al., Elsevier Science (1984), Vol. 4, pp. 85-112; de Antueno, RJ, Lipids (1993), Vol. 28, No. 4, pp. 285-290), it has only recently been directly implicated in human disease processes.

A single SCD gene, SCD1, has been characterized in humans. SCD1 is described in Brownlie et al, PCT published patent application, WO 01/62954, the disclosure of which is hereby incorporated by reference in its entirety. A second human SCD isoform has recently been identified, and because it bears little sequence homology to alternate mouse or rat isoforms it has been named human SCD5 or hSCD5 (PCT published patent application, WO 02/26944, incorporated herein by reference in its entirety).

To date, no small-molecule, drug-like compounds are known that specifically inhibit or modulate SCD activity. Certain long-chain hydrocarbons have been used historically to study SCD activity. Known examples include thia-fatty acids, cyclopropenoid fatty acids, and certain conjugated linoleic acid isomers. Specifically, *cis*-12, *trans*-10 conjugated linoleic acid is believed to inhibit SCD enzyme activity and reduce the abundance of SCD1 mRNA while *cis*-9, *trans*-11 conjugated linoleic acid does not. Cyclopropenoid fatty acids, such as those found in stercula and cotton seeds, are also known to inhibit SCD activity. For example, sterculic acid (8-(2-octylcyclopropenyl)octanoic acid) and malvalic acid (7-(2-octylcyclopropenyl)heptanoic acid) are C18 and C16 derivatives of sterculoyl- and malvaloyl fatty acids, respectively, having cyclopropene rings at their C9-C10 position. These agents are believed to inhibit SCD enzymatic activity by direct interaction with the enzyme, thus inhibiting delta-9 desaturation. Other agents that may inhibit SCD activity include thia-fatty acids, such as 9-thiastearic acid (also called 8-nonylthiooctanoic acid) and other fatty acids with a sulfoxy moiety.

These known modulators of delta-9 desaturase activity are not useful for treating the diseases and disorders linked to SCD1 biological activity. None of the known SCD inhibitor compounds are selective for SCD or delta-9 desaturases, as they also inhibit other desaturases and enzymes. The thia-fatty acids, conjugated linoleic acids and cyclopropene fatty acids (malvalic acid and sterculic acid) are neither useful at reasonable physiological doses, nor are they specific inhibitors of SCD1 biological activity, rather they demonstrate cross inhibition of other desaturases, in particular the delta-5 and delta-6 desaturases by the cyclopropene fatty acids.

The absence of small molecule inhibitors of SCD enzyme activity is a major scientific and medical disappointment because evidence is now compelling that SCD activity is directly implicated in common human disease processes: See *e.g.*, Attie, A.D. et al., "Relationship between stearoyl-CoA desaturase activity and plasma triglycerides in human and mouse hypertriglyceridemia", J. Lipid Res. (2002) Vol. 43, No. 11, pp. 1899-907; Cohen, P. et al., "Role for stearoyl-CoA desaturase-1 in leptin-mediated weight loss", Science (2002), Vol. 297, No. 5579, pp. 240-3, Ntambi, J. M. et al., "Loss of stearoyl-CoA desaturase-1 function protects mice against adiposity", Proc. Natl. Acad. Sci. U S A. (2002), Vol. 99, No. 7, pp. 11482-6.

WO-A-01/25203 describes pyridine carboxamide or sulfonamide derivatives.

EP-A-1 452 525 relates to amide derivatives acting as TGF-β inhibitor.

WO-A-02/10154 relates to substituted heterocyclic amides and their use as an inhibitor of factor Xa.

WO-A-03/043636 discloses nicotine or isonicotine benzothiazol derivatives.

EP-A-1 396 487 relates to a phenylpyridine carbonyl piperazine derivative useful as a type 4 phosphodiesterase inhibitor.

WO-A-03/050088 relates to substituted heterocyclic carboxamides with antithrombotic activity.

EP-A-1 156 045 describes a composition comprising an amide derivative for inhibiting TGF-β.

E-A-1 035 115 relates to phenylpyridine derivatives and their use as NK-1 receptor antagonist.

WO-A-2004/010927 describes a method for increasing insulin sensitivity by reducing the amount of SCD1 protein by inhibiting the SCD1 enzymatic activity.

WO-A-01/62954 relates to the identification of diseases and disorders linked to SCD1 biological activity.

The present invention solves this problem by presenting new classes of compounds that are useful in modulating SCD activity and regulating lipid levels, especially plasma lipid levels, and which are useful in the treatment of SCD-mediated diseases such as diseases related to dyslipidemia and disorders of lipid metabolism, especially diseases related to elevated lipid levels, cardiovascular disease, diabetes, obesity, metabolic syndrome.

### Related Literature

U.S. Patent No. 6,677,452 discloses novel pyridine carboxamide or sulfonamide derivative compounds.

### SUMMARY OF THE INVENTION

The present invention provides nicotinamide derivatives to modulate the activity of stearoyl-CoA desaturase. Pharmaceutical compositions comprising such derivatives are also encompassed. The present invention is solved on the basis of claims 1 to 101.

One object of the present invention is the use of a compound of formula (I): 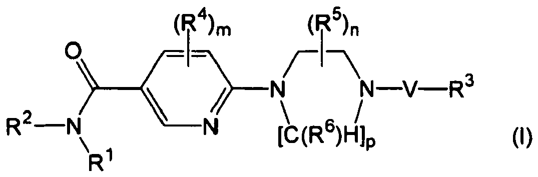 wherein:
m is 1, 2 or 3;
n is 1, 2, 3 or 4;
p is 2, 3 or 4;
V is -C(O)-, -S(O)- or -S(O)₂-;
R¹ is hydrogen, alkyl, alkenyl, aryl, aralkyl, aralkenyl or cycloalkyl;
R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, aralkyl, where the aryl radical may be optionally substituted, aralkenyl, where the aryl radical may be optionally substituted, optionally substituted cycloalkyl, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
R³ is selected from the group consisting of hydrogen, -R⁹-OR⁸, -R⁹-N(R⁸)₂, alkyl, alkenyl, optionally substituted aryl, aralkyl, where the aryl radical may be optionally substituted, aralkenyl, where the aryl radical may be optionally substituted, optionally substituted cycloalkyl, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
each R⁴ is independently hydrogen, alkyl, alkenyl, halo, haloalkyl, aryl, cyano, nitro, -R⁹-OR⁸, -R⁹-N(R⁸)₂ or -S(O)ₜR¹⁰ (where t is 0, 1 or 2);
each R⁵ and R⁶ is independently hydrogen, oxo, alkyl, alkenyl, halo, haloalkyl or aryl;
or one R⁵ and one R⁶ may together form an straight or branched alkylene bridge;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl;
each R⁹ is independently a direct bond or a straight or branched alkylene or alkenylene chain; and
R¹⁰ is alkyl, alkenyl, haloalkyl, cycloalkyl, cycloalkylalkyl, aryl, aralkyl, heterocyclyl, heterocylylalkyl, heteroaryl or heteroarylalkyl;
as a single stereoisomer, a mixture of stereoisomers, a racemic mixture thereof of stereoisomers, or as a tautomer;
or as a pharmaceutically acceptable salt, solvate, polymorph or prodrug, selected from among ester and amide derivatives of hydroxyl, carboxy, mecapto or amino functional groups in the compounds of formula (I), thereof for the manufacture of a medicament for treating an SCD mediated disease or condition in a mammal, wherein the disease or condition is selected from the group consisting of Type II diabetes, impaired glucose tolerance, insulin resistance, hypertension, obesity, hypertriglyceridemia, low HDL, lipidemia, dyslipidemia, microalbuminemia, hyperuricaemia, hyperleptinaemia, metabolic syndrome, and any combination of these; and
wherein the optional substituent is one or more substituents independently selected from the group consisting of alkyl, alkenyl, halo, haloalkyl, haloalkenyl, cyano, nitro, aryl, aralkyl, cycloalkyl, cycloalkylalkyl, heterocylyl, heterocyclylalkyl, heteroaryl, heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-S(O)ₜOR¹⁰ (where t is 1 or 2), -R⁹-S(O)ₜR¹⁰ (where t is 0, 1 or 2), and -R⁹-S(O)ₜN(R⁸)₂ (where t is 1 or 2).

Another object of the present invention is a compound of formula (I): 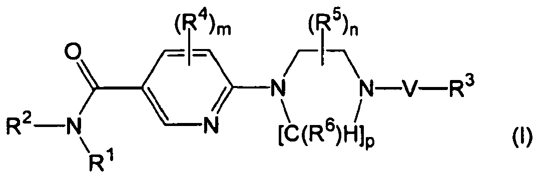 wherein:
m is 1, 2 or 3;
n is 1, 2, 3 or 4;
p is 2, 3 or 4;
V is -C(O)-, -S(O)- or -S(O)₂-;
R' is hydrogen, alkyl, alkenyl, aryl, aralkyl, aralkenyl or cycloalkyl;
R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, aralkyl, where the aryl radical may be optionally substituted, aralkenyl, where the aryl radical may be optionally substituted, optionally substituted cycloalkyl, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
R³ is selected from the group consisting of hydrogen, -R⁹-OR⁸, -R⁹-N(R⁸)₂, alkyl, alkenyl, optionally substituted aryl, aralkyl, where the aryl radical may be optionally substituted, aralkenyl, where the aryl radical may be optionally substituted, optionally substituted cycloalkyl, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
each R⁴ is independently hydrogen, alkyl, alkenyl, halo, haloalkyl, aryl, cyano, nitro, -R⁹-OR⁸, -R⁹-N(R⁸)₂ or -S(O)ₜR¹⁰ (where t is 0, 1 or 2);
each R⁵ and R⁶ is independently hydrogen, oxo, alkyl, alkenyl, halo, haloalkyl or aryl;
or one R⁵ and one R⁶ may together form an straight or branched alkylene bridge;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl;
each R⁹ is independently a direct bond or a straight or branched alkylene or alkenylene chain; and
R¹⁰ is alkyl, alkenyl, haloalkyl, cycloalkyl, cycloalkylalkyl, aryl, aralkyl, heterocyclyl, heterocylylalkyl, heteroaryl or heteroarylalkyl;
as a single stereoisomer, a mixture of stereoisomers, a racemic mixture thereof of stereoisomers, or as a tautomer;
or as a pharmaceutically acceptable salt, solvate, polymorph or prodrug, selected from among ester and amide derivatives of hydroxyl, carboxy, mecapto or amino functional groups in the compounds of formula (I), thereof for use in the treatment of SCD mediated disease or condition in a mammal, wherein the disease or condition is selected from the group consisting of Type II diabetes, impaired glucose tolerance, insulin resistance, hypertension, obesity, hypertriglyceridemia, low HDL, lipidemia, dyslipidemia, microalbuminemia, hyperuricaemia, hyperleptinaemia, metabolic syndrome, and any combination of these; and
wherein the optional substituent is one or more substituents independently selected from the group consisting of alkyl, alkenyl, halo, haloalkyl, haloalkenyl, cyano, nitro, aryl, aralkyl, cycloalkyl, cycloalkylalkyl, heterocylyl, heterocyclylalkyl, heteroaryl, heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-S(O)ₜOR¹⁰ (where t is 1 or 2), -R⁹-S(O)ₜR¹⁰ (where t is 0, 1 or 2), and-R⁹-S(O)ₜN(R⁸)₂ (where t is 1 or 2).

The present invention further relates to a compound for treating a patient for, or protecting a patient from developing, a disease or condition mediated by stearoyl-CoA desaturase (SCD), which comprises administering to a patient afflicted with such disease or condition, or at risk of developing such disease or condition, a therapeutically effective amount of a compound that inhibits activity of SCD in a patient when administered thereto.

The present invention further relates to a compound for treating a range of diseases involving lipid metabolism utilizing compounds identified by the methods disclosed herein. In accordance therewith, there is disclosed herein a range of compounds having said activity, based on a screening assay for identifying, from a library of test compounds, a therapeutic agent which modulates the biological activity of said SCD and is useful in treating a human disorder or condition relating to serum levels of lipids, such as triglycerides, VLDL, HDL, LDL, and/or total cholesterol.

It is a still further object of the present invention to provide compounds or pharmaceutical compositions useful in treating, preventing and/or diagnosing a disease or condition relating to SCD biological activity such as the diseases encompassed by cardiovascular disorders and/or metabolic syndrome (including dyslipidemia, insulin resistance and obesity).

It is yet a further object of the present invention to provide a compound for preventing or treating a disease or condition related to elevated lipid levels, such as plasma lipid levels, especially elevated triglyceride or cholesterol levels, in a patient afflicted with such elevated levels, comprising administering to said patient a therapeutically or prophylactically effective amount of a composition as disclosed herein. The present invention also relates to novel compounds having therapeutic ability to reduce lipid levels in an animal, especially triglyceride and cholesterol levels.

The present invention also relates to pharmaceutical compositions comprising the compounds of formula (I) as defined in claim 89 and pharmaceutically acceptable excipients. In one embodiment, the present invention relates to a pharmaceutical composition comprising a compound of the invention in a pharmaceutically acceptable carrier and in an amount effective to modulate triglyceride level, or to treat diseases related to dyslipidemia and disorders of lipid metabolism, when administered to an animal, preferably a mammal, most preferably a human patient. In an embodiment of such composition, the patient has an elevated lipid level, such as elevated plasma triglycerides or cholesterol, before administration of said compound and said compound is present in an amount effective to reduce said lipid level.

The present invention also relates to compounds of formula (I): 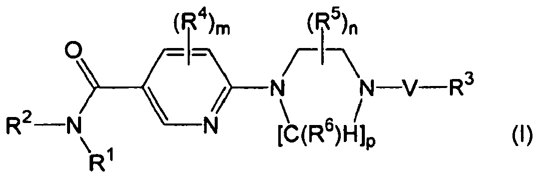 wherein:
m is 1, 2 or 3;
n is 1, 2, 3 or 4;
p is 2, 3 or 4;
V is -C(O)-, -S(O)- or -S(O)₂-;
R¹ is hydrogen, alkyl, alkenyl, aryl, aralkyl or aralkenyl;
R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, aralkyl, where the aryl radical may be optionally substituted, aralkenyl, where the aryl radical may be optionally substituted, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
R³ is cyclopropyl substituted by optionally substituted aryl or optionally substituted heteroaryl;
each R⁴ is independently hydrogen, alkyl, alkenyl, halo, haloalkyl, aryl, cyano, nitro, -R⁹-OR⁸, -R⁹-N(R⁸)₂ or -S(O)ₜR¹⁰ (where t is 0, 1 or 2);
each R⁵ and R⁶ is independently hydrogen, oxo, alkyl, alkenyl, halo, haloalkyl or aryl; or one R⁵ and one R⁶ may together form an straight or branched alkylene bridge;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl;
each R⁹ is independently a direct bond or a straight or branched alkylene or alkenylene chain; and R¹⁰ is alkyl, alkenyl, haloalkyl, cycloalkyl, cycloalkylalkyl, aryl, aralkyl, heterocyclyl, heterocylylalkyl, heteroaryl or heteroarylalkyl;
as a single stereoisomer, a mixture of stereoisomers, a racemic mixture thereof of stereoisomers, or as a tautomer; or as a pharmaceutically acceptable salt, solvate polymorph or prodrug, selected from among ester and amide derivatives of hydroxyl, carboxy, mercapto or amino functional groups in the compounds of formula (I) thereof; and wherein the optional substituent is one or more substituents independently selected from the group consisting of alkyl, alkenyl, halo, haloalkyl, haloalkenyl, cyano, nitro, aryl, aralkyl, cycloalkyl, cycloalkylalkyl, heterocylyl, heterocyclylalkyl, heteroaryl, heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O),R¹⁰) (where t is 1 or 2), -R⁹-S(O)ₜOR¹⁰ (where t is 1 or 2), -R⁹-S(O)ₜR¹⁰ (where t is 0, 1 or 2), and -R⁹-S(O)ₜN(R⁸)₂ (where t is 1 or 2).

### BRIEF DESCRIPTION OF THE DRAWING

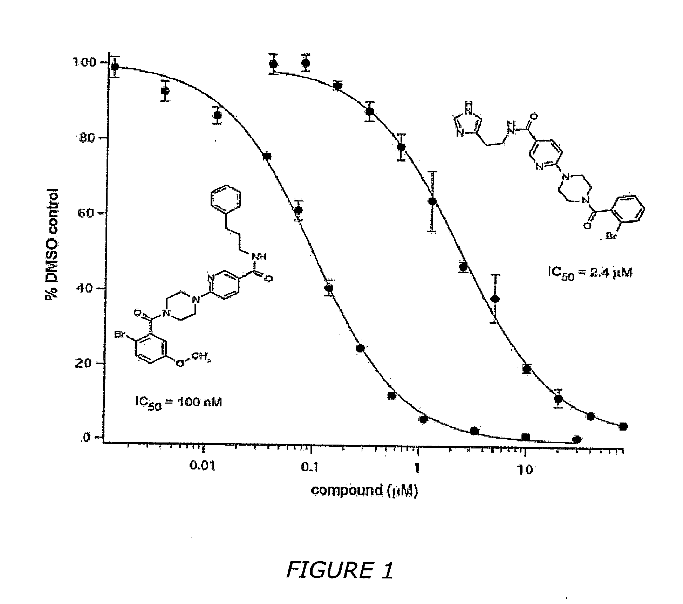

Figure 1 shows dose response curves for two of the compounds of the invention. The compound at the left of the chart shows much greater inhibitory ability (with IC₅₀ of 100 nM) than the compound at the right (with IC₅₀ of 2.4 µM).

### DETAILED DESCRIPTION OF THE INVENTION

### Definitions

As used in the specification and appended claims, unless specified to the contrary, the following terms have the meaning indicated:
"Alkyl" refers to a straight or branched hydrocarbon chain radical consisting solely of carbon and hydrogen atoms, containing no unsaturation, having from one to twelve carbon atoms, preferably one to eight carbon atoms, and which is attached to the rest of the molecule by a single bond, *e.g.*, methyl, ethyl, *n*-propyl, 1-methylethyl (*iso*-propyl), *n*-butyl, *n*-pentyl, 1,1-dimethylethyl (*t*-buty).
"Alkenyl" refers to a straight or branched hydrocarbon chain radical group consisting solely of carbon and hydrogen atoms, containing at least one double bond, having from two to twelve carbon atoms, preferably one to eight carbon atoms and which is attached to the rest of the molecule by a single bond, *e.g.*, ethenyl, prop-1-enyl, but-1-enyl, pent-1-enyl, penta-1,4-dienyl.
"Aryl" refers to aromatic monocyclic or multicyclic hydrocarbon ring system consisting only of hydrogen and carbon and containing from 6 to 19 carbon atoms, where the ring system may be partially or fully saturated. Aryl groups include groups such as fluorenyl, phenyl and naphthyl. Unless stated otherwise specifically in the specification, the term "aryl" or the prefix "ar-" (such as in "aralkyl") is meant to include aryl radicals optionally substituted by one or more substituents independently selected from the group consisting of alkyl, alkenyl, halo, haloalkyl, haloalkenyl, cyano, nitro, aryl, aralkyl, cycloalkyl, cycloalkylalkyl, heterocyclyl, heterocyclylalkyl, heteroaryl, heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-S(O)ₜOR¹⁰ (where t is 1 or 2), -R⁹-S(O)ₜR¹⁰ (where t is 0, 1 or 2), and -R⁹-S(O),N(R⁸)₂ (where t is 1 or 2) where each R⁸, R⁹ and R¹⁰ are as defined above in the Summary of the Invention.
"Aralkyl" refers to a radical of the formula -RₐR_{b} where Rₐ is an alkyl radical as defined above and R_{b} is one or more aryl radicals as defined above, e.g., benzyl, diphenylmethyl. The aryl radical(s) may be optionally substituted as described above.
"Aralkenyl" refers to a radical of the formula -R_{c}R_{b} where R_{c} is an alkenyl radical as defined above and R_{b} is one or more aryl radicals as defined above, which may be optionally substituted as described above.
"Alkylene" and "alkylene chain" refer to a straight or branched divalent hydrocarbon chain, linking the rest of the molecule to a radical group, consisting solely of carbon and hydrogen, containing no unsaturation and having from one to twelve carbon atoms, preferably having from one to eight carbons, *e.g.*, methylene, ethylene, propylene, *n*-butylene. The alkylene chain may be attached to the rest of the molecule and to the radical group can be through any two carbons within the chain.
"Alkylene" and "alkylene bridge" refer to a straight or branched divalent hydrocarbon bridge, linking two different carbons of the same ring structure, consisting solely of carbon and hydrogen, containing no unsaturation and having from one to twelve carbon atoms, preferably having from one to eight carbons, *e.g.*, methylene, ethylene, propylene, *n*-butylene. The alkylene bridge may link any two carbons within the ring structure.
"Alkenylene" and "alkenylene chain" refer to a straight or branched divalent hydrocarbon chain linking the rest of the molecule to a radical group, consisting solely of carbon and hydrogen, containing at least one double bond and having from two to seven carbon atoms, *e.g.*, ethenylene, propenylene, *n*-butenylene. The alkenylene chain is attached to the rest of the molecule through a single bond and to the radical group through a double bond or a single bond. The points of attachment of the alkenylene chain to the rest of the molecule and to the radical group can be through any two carbons within the chain.
"Cycloalkyl" refers to a stable non-aromatic monocyclic or bicyclic hydrocarbon radical consisting solely of carbon and hydrogen atoms, having from three to fifteen carbon atoms, preferably having from three to ten carbon atoms, and which is saturated or unsaturated and attached to the rest of the molecule by a single bond, *e.g.*, cyclopropyl, cyclobutyl, cyclopentyl, cyclohexyl, decalinyl. Unless otherwise stated specifically in the specification, the term "cycloalkyl" is meant to include cycloalkyl radicals which are optionally substituted by one or more substituents independently selected from the group consisting of alkyl, alkenyl, halo, haloalkyl, haloalkenyl, cyano, nitro, aryl, aralkyl, cycloalkyl, cycloalkylalkyl, heterocyclyl, heterocyclylalkyl, heteroaryl, heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-S(O)ₜOR¹⁰ (where t is 1 or 2), -R⁹-S(O)ₜR¹⁰ (where t is 0, 1 or 2), and -R⁹-S(O)ₜN(R⁸)₂ (where t is 1 or 2) where each R⁸, R⁹ and R¹⁰ are as defined above in the Summary of the Invention.
"Cycloalkylalkyl" refers to a radical of the formula -RₐR_{d} where Rₐ is an alkyl radical as defined above and R_{d} is a cycloalkyl radical as defined above. The alkyl radical and the cycloalkyl radical may be optionally substituted as defined above.
"Halo" refers to bromo, chloro, fluoro or iodo.
"Haloalkyl" refers to an alkyl radical, as defined above, that is substituted by one or more halo radicals, as defined above, *e.g.*, trifluoromethyl, difluoromethyl, trichloromethyl, 2,2,2-trifluoroethyl, 1-fluoromethyl-2-fluoroethyl, 3-bromo-2-fluoropropyl, 1-bromomethyl-2-bromoethyl.
"Haloalkenyl" refers to an alkenyl radical, as defined above, that is substituted by one or more halo radicals, as defined above, *e.g.*, 2-bromoethenyl, 3-bromoprop-1-enyl.
"Heterocyclyl" refers to a stable 3- to 18-membered non-aromatic ring radical which consists of carbon atoms and from one to five heteroatoms selected from the group consisting of nitrogen, oxygen and sulfur. For purposes of this invention, the heterocyclyl radical may be a monocyclic, bicyclic, tricyclic or tetracyclic ring system, which may include fused or bridged ring systems; and the nitrogen, carbon or sulfur atoms in the heterocyclyl radical may be optionally oxidized; the nitrogen atom may be optionally quaternized; and the heterocyclyl radical may be partially or fully saturated. Examples of such heterocyclyl radicals include dioxolanyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Unless stated otherwise specifically in the specification, the term "heterocyclyl" is meant to include heterocyclyl radicals as defined above which are optionally substituted by one or more substituents independently selected from the group consisting of alkyl, alkenyl, halo, haloalkyl, haloalkenyl, cyano, nitro, aryl, aralkyl, cycloalkyl, cycloalkylalkyl, heterocyclyl, heterocyclylalkyl, heteroaryl, heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-S(O),OR¹⁰ (where t is 1 or 2), -R⁹-S(O)ₜR¹⁰ (where t is 0, 1 or 2), and -R⁹-S(O)ₜN(R⁸)₂ (where t is 1 or 2) where each R⁸, R⁹ and R¹⁰ are as defined above in the Summary of the Invention.
"Heterocyclylalkyl" refers to a radical of the formula -RₐRₑ where Rₐ is an alkyl radical as defined above and Rₑ is a heterocyclyl radical as defined above, and if the heterocyclyl is a nitrogen-containing heterocyclyl, the heterocyclyl may be attached to the alkyl radical at the nitrogen atom. The heterocyclyl radical may be optionally substituted as defined above.
"Heteroaryl" refers to a 3- to 18-membered aromatic ring radical which consists of carbon atoms and from one to five heteroatoms selected from the group consisting of nitrogen, oxygen and sulfur. For purposes of this invention, the heteroaryl radical may be a monocyclic, bicyclic, tricyclic or tetracyclic ring system, which may include fused or bridged ring systems; and the nitrogen, carbon or sulfur atoms in the heteroaryl radical may be optionally oxidized; the nitrogen atom may be optionally quaternized. Examples include azepinyl, acridinyl, benzimidazolyl, benzthiazolyl, benzindolyl, benzothiadiazolyl, benzonaphthofuranyl, benzoxazolyl, benzodioxolyl, benzodioxinyl, benzopyranyl, benzopyranonyl, benzofuranyl, benzofuranonyl, benzothienyl (benzothiophenyl), benzotriazolyl, benzo[4,6]imidazo[1,2-a]pyridinyl, carbazolyl, cinnolinyl, dibenzofuranyl, furanyl, furanonyl, isothiazolyl, imidazolyl, indolyl, indazolyl, isoindolyl, indolinyl, isoindolinyl, indolizinyl, isoxazolyl, naphthyridinyl, oxadiazolyl, 2-oxoazepinyl, oxazolyl, oxiranyl, phenazinyl, phenothiazinyl, phenoxazinyl, phthalazinyl, pteridinyl, purinyl, pyrrolyl, pyrazolyl, pyridinyl, pyrazinyl, pyrimidinyl, pyridazinyl, quinazolinyl, quinoxalinyl, quinolinyl, quinuclidinyl, isoquinolinyl, thiazolyl, thiadiazolyl, triazolyl, tetrazolyl, triazinyl, and thiophenyl. Unless stated otherwise specifically in the specification, the term "heteroaryl" is meant to include heteroaryl radicals as defined above which are optionally substituted by one or more substituents selected from the group consisting of alkyl, alkenyl, halo, haloalkyl, haloalkenyl, cyano, nitro, aryl, aralkyl, cycloalkyl, cycloalkylalkyl, heterocyclyl, heterocyclylalkyl, heteroaryl, heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-S(O)ₜOR¹⁰ (where t is 1 or 2), -R⁹-S(O)ₜR¹⁰ (where t is 0, 1 or 2), and -R⁹-S(O)ₜN(R⁸)₂ (where t is 1 or 2) where each R⁸, R⁹ and R¹⁰ are as defined above in the Summary of the Invention.
"Heteroarylalkyl" refers to a radical of the formula -RₐR_{f} where Rₐ is an alkyl radical as defined above and R_{f} is a heteroaryl radical as defined above. The heteroaryl radical may be optionally substituted as defined above.
"Heteroarylalkenyl" refers to a radical of the formula -R_{b}R_{f} where R_{b} is an alkenyl radical as defined above and R_{f} is a heteroaryl radical as defined above. The heteroaryl radical may be optionally substituted as defined above.
"Prodrugs" is meant to indicate a compound that may be converted under physiological conditions or by solvolysis to a biologically active compound of the invention. Thus, the term "prodrug" refers to a metabolic precursor of a compound of the invention that is pharmaceutically acceptable. A prodrug may be inactive when administered to a subject in need thereof, but is converted *in vivo* to an active compound of the invention. Prodrugs are typically rapidly transformed *in vivo* to yield the parent compound of the invention, for example, by hydrolysis in blood. The prodrug compound often offers advantages of solubility, tissue compatibility or delayed release in a mammalian organism (see, Bundgard, H., Design of Prodrugs (1985), pp. 7-9, 21-24 (Elsevier, Amsterdam).

A discussion of prodrugs is provided in Higuchi, T., et al., "Pro-drugs as Novel Delivery Systems," A.C.S. Symposium Series, Vol. 14, and in Bioreversible Carriers in Drug Design, ed. Edward B. Roche, American Pharmaceutical Association and Pergamon Press, 1987.

The term "prodrug" is also meant to include any covalently bonded carriers which release the active compound of the invention *in vivo* when such prodrug is administered to a mammalian subject. Prodrugs of a compound of the invention may be prepared by modifying functional groups present in the compound of the invention in such a way that the modifications are cleaved, either in routine manipulation or *in vivo,* to the parent compound of the invention. Prodrugs include compounds of the invention wherein a hydroxy, amino or mercapto group is bonded to any group that, when the prodrug of the compound of the invention is administered to a mammalian subject, cleaves to form a free hydroxy, free amino or free mercapto group, respectively. Examples of prodrugs include, ester and amide derivatives of hydroxy, carboxy, mercapto or amino functional groups in the compounds of the invention.

"Stable compound" and "stable structure" are meant to indicate a compound that is sufficiently robust to survive isolation to a useful degree of purity from a reaction mixture, and formulation into an efficacious therapeutic agent.

"Mammal" includes humans and domestic animals, such as cats, dogs, swine, cattle, sheep, goats, horses, rabbits.

"Optional" or "optionally" means that the subsequently described event of circumstances may or may not occur, and that the description includes instances where said event or circumstance occurs and instances in which it does not. For example, "optionally substituted aryl" means that the aryl radical may or may not be substituted and that the description includes both substituted aryl radicals and aryl radicals having no substitution.

"Pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsfier which has been approved by the United States Food and Drug Administration as being acceptable for use in humans or domestic animals.

"Pharmaceutically acceptable salt" includes both acid and base addition salts.

"Pharmaceutically acceptable acid addition salt" refers to those salts which retain the biological effectiveness and properties of the free bases, which are not biologically or otherwise undesirable, and which are formed with inorganic acids such at hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsutfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, *p*-toluenesulfonic acid, trifluoroacetic acid, undecylenic acid.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include the sodium, potassium, lithium, ammonium, calcium, magnesium, iron, zinc, copper, manganese, aluminum salts. Preferred inorganic salts are the ammonium, sodium, potassium, calcium, and magnesium salts. Salts derived from organic bases include salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, *N*-ethylpiperidine, polyamine resins. Particularly preferred organic bases are isopropylamine, diethylamine, ethanolamine, trimethylamine, dicyclohexylamine, choline and caffeine.

The compounds of the invention may, and typically do, exist as solids, including crystalline solids which can be crystallized from common solvents such as ethanol, *N,N*-dimethylformamide, water. The crystallization process may, depending on the crystallization conditions, provide various polymorphic structures. Typically, a more thermodynamically stable polymorph is advantageous to the commercial scale manufacture of a compound of the invention, and is a preferred form of the compound. Such polymorphs are considered to be within the scope of the invention.

Often crystallizations produce a solvate of the compound of the invention. As used herein, the term "solvate" refers to an aggregate that comprises one or more molecules of a compound of the invention with one or more molecules of solvent. The solvent may be water, in which case the solvate may be a hydrate. Alternatively, the solvent may be an organic solvent. Thus, the compounds of the present invention may exist as a hydrate, including a monohydrate, dihydrate, hemihydrate, sesquihydrate, trihydrate, tetrahydrate, as well as the corresponding solvated forms. The compound of the invention may be true solvates, white in other cases, the compound of the invention may merely retain adventitious water or be a mixture of water plus some adventitious solvent.

A "pharmaceutical composition" refers to a formulation of a compound of the invention and a medium generally accepted in the art for the delivery of the biologically active compound to mammals, e.g., humans. Such a medium includes all pharmaceutically acceptable carriers, diluents or excipients therefor.

"Therapeutically effective amount" refers to that amount of a compound of the invention which, when administered to a mammal, preferably a human, is sufficient to effect treatment, as defined below, of an SCD-mediated disease or condition in the mammal, preferably a human. The amount of a compound of the invention which constitutes a "therapeutically effective amount" will vary depending on the compound, the condition and its severity, and the age of the mammal to be treated, but can be determined routinely by one of ordinary skill in the art having regard to his own knowledge and to this disclosure.

"Treating" or "treatment" as used herein covers the treatment of the disease or condition of interest in a mammal, preferably a human, having the disease or disorder of interest, and includes:
(i) preventing the disease or condition from occurring in a mammal, in particular, when such mammal is predisposed to the condition but has not yet been diagnosed as having it;
   (ii) inhibiting the disease or condition, *i.e*., arresting its development; or
   (iii) relieving the disease or condition, *i.e*., causing regression of the disease or condition.

As used herein, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms have been identified by clinicians.

The compounds of the invention, or their pharmaceutically acceptable salts may contain one or more asymmetric centers and may thus give rise to enantiomers, diastereomers, and other stereoisomeric forms that may be defined, in terms of absolute stereochemistry, as (*R*)- or (*S*)- or, as (D)- or (L)- for amino acids. The present invention is meant to include all such possible isomers, as well as their racemic and optically pure forms. Optically active (+) and (-), (*R*)- and (*S*)-, or (D)- and (L)- isomer may be prepared using chiral synthons or chiral reagents, or resolved using conventional techniques, such as HPLC using a chiral column. When the compounds described herein contain olefinic double bonds or other centers of geometric asymmetry, and unless specified otherwise, it is intended that the compounds include both E and Z geometric isomers. Likewise, all tautomeric forms are also intended to be included.

A "stereoisomer" refers to a compound made up of the same atoms bonded by the same bonds but having different three-dimensional structures, which are not interchangeable. The present invention contemplates various stereoisomers and mixtures thereof and includes "enantiomers", which refers to two stereoisomers whose molecules are nonsuperimposeable mirror images of one another.

A "tautomer" refers to a proton shift from one atom of a molecule to another atom of the same molecule. The present invention includes tautomers of any said compounds.

The chemical naming protocol and structure diagrams used herein employ and rely the chemical naming features as utilized by Chemdraw version 7.0.1. (available from Cambridgesoft Corp., Cambridge, MA). For complex chemical names employed herein, a substituent group is named before the group to which it attaches. In chemical structure diagrams, all bonds are identified, except for some carbon atoms which are assumed to be bonded to sufficient hydrogen atoms to complete the valency. For examples, a compound of the following formula: 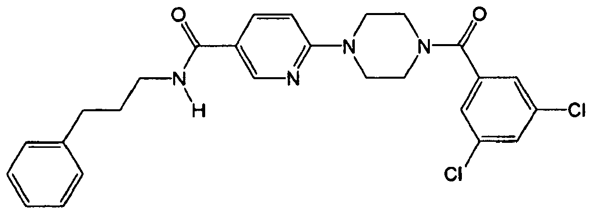 is named herein as 6-[4-(3,5-dichloro-benzoyl)-piperazin-1-yl]-*N*-(3-phenyl-propyl)-nicotinamide.

### Embodiments of the Invention

Of the various embodiments of the invention as set forth above in the Summary of the Invention, one group of embodiments is directed to a compound for the treatment of an SCD-mediated disease or condition in a mammal are methods wherein the compound of formula (I) is a compound of formula (I) wherein m is 1 or 2; n is 1 or 2; p is 2 or 3; V is -C(O)- or -S(O)₂-; R' is hydrogen or alkyl; R² is selected from the group consisting of hydrogen, -R⁷-OR⁸, -R⁷-N(R⁸)₂, -R⁷-S(O)ₜR¹⁰(where t is 0, 1or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is alkyl, alkenyl or -R⁹-N(R⁸)₂; each R⁴ is independently hydrogen, alkyl, alkenyl, halo, haloalkyl, aryl or -R⁹-OR⁸; each R⁵ and R⁶ is independently hydrogen, oxo, alkyl, alkenyl, halo, haloalkyl or aryl; or one R⁵ and one R⁶ may together form an straight or branched alkylene bridge; each R⁷ is independently a straight or branched alkylene or alkenylene chain; each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl; each R⁹ is independently a direct bond or a straight or branched alkylene chain; and R¹⁰ is alkyl, aryl or aralkyl.

Of this group of embodiments, one subgroup of embodiments is directed to the compound wherein the compound of formula (I) is a compound of formula (I) wherein m is 1 or 2; n is 1 or 2; p is 2; V is -C(O)-; R¹ is hydrogen or alkyl; R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl; R³ is alkyl; each R⁴ is independently hydrogen, alkyl, halo, or haloalkyl; each R⁵ and R⁸ is independently hydrogen, oxo, alkyl, halo or haloalkyl; each R⁷ is a straight or branched alkylene chain; each R⁸ is independently hydrogen, alkyl, haloalkyl, cycloalkyl, cycloalkylalkyl, aryl and aralkyl; and R¹⁰ is alkyl, aryl or aralkyl.

Of this subgroup of embodiments, one class of embodiments is directed to the compound wherein the compound of formula (I) is a compound of formula (I) wherein m is 1; n is 1; p is 2; V is -C(O)-; R¹ is hydrogen or alkyl; R² is selected from the group consisting of -R⁷-OR⁸, -R⁷-N(R⁸)₂, -R⁷-S(O)ₜR¹⁰ (where t is 0, 1 or 2) or alkyl; R³ is alkyl; R⁴ is hydrogen; R⁵ is hydrogen; each R⁶ is hydrogen; R⁷ is a straight or branched alkylene chain; R³ is hydrogen or alkyl; and R¹⁰ is alkyl, aryl or aralkyl.

Of the group of embodiments set forth above, another subgroup of embodiments is directed to the compound wherein the compound of formula (I) is a compound of formula (I) wherein m is 1 or 2; n is 1 or 2; p is 2 or 3; V is -C(O)- or-S(O)₂-; R¹ is hydrogen or alkyl; R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is alkyl or -R⁷-N(R⁸)₂; each R⁴ is independently hydrogen, alkyl, halo, or haloalkyl; and each R⁵ and R⁶ is independently hydrogen, oxo, alkyl, alkenyl, halo, haloalkyl or aryl; or one R⁵ and one R⁶ may together form an straight or branched alkylene bridge; R⁷ is a direct bond; and each R³ is independently hydrogen or alkyl.

Of this subgroup of embodiments, one class of embodiments is directed to the compound wherein the compound of formula (I) is a compound of formula (I) wherein m is 1; n is 1; p is 2 or 3; V is -C(O)- or -S(O)₂-; R¹ is hydrogen or alkyl; R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl; R³ is alkyl or -R⁷-N(R⁸)₂; R⁴ is hydrogen, alkyl, halo or haloalkyl; and each R⁵ and R⁶ is independently hydrogen, oxo, alkyl, halo or haloalkyl; or one R⁵ and one R⁶ may together form a methylene bridge; R⁷ is a direct bond; and each R⁸ is independently hydrogen or alkyl.

Of the various embodiments of the invention as set forth above in the Summary of the Invention, another group of embodiments is directed to a compound for the treatment of an SCD-mediated disease or condition in a mammal wherein the compound of formula (I) is a compound of formula (I) wherein m is 1 or 2; n is 1 or 2; p is 2; V is -C(O)-; R¹ is hydrogen or alkyl; R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is optionally substituted cycloalkyl, optionally substituted cycloalkylalkyl, or optionally substituted cycloalkylalkenyl; each R⁴ is independently hydrogen, alkyl, alkenyl, halo, haloalkyl, aryl or -R⁹-OR⁸; each R⁵ and R⁶ is independently hydrogen, oxo, alkyl, alkenyl, halo, haloalkyl or aryl; each R⁷ is independently a straight or branched alkylene or alkenylene chain; each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl; R⁹ is a direct bond or a straight or branched alkylene chain; and R¹⁰ is alkyl, aryl or aralkyl.

Of this group of embodiments, a subgroup of embodiments is directed to the compound wherein the compound of formula (I) is a compound of formula (I) wherein m is 1 or 2; n is 1 or 2; p is 2; V is -C(O)-; R¹ is hydrogen or alkyl; R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl; R³ is optionally substituted cycloalkyl or optionally substituted cycloalkylalkyl; each R⁴ is independently hydrogen, alkyl, halo, or haloalkyl; each R⁵ and R⁶ is independently hydrogen, oxo, alkyl, halo or haloalkyl; each R⁷ is a straight or branched alkylene chain; each R⁸ is independently hydrogen, alkyl, haloalkyl, cycloalkyl, cycloalkylalkyl, aryl and aralkyl; and R¹⁰ is alkyl, aryl or aralkyl.

Of this subgroup of embodiments, a class of embodiments is directed to the methods wherein the compound of formula (I) is a compound of formula (I) wherein m is 1; n is 1; p is 2; V is -C(O)-; R¹ is hydrogen or alkyl; R² is selected from the group consisting of -R⁷-OR⁸, -R⁷-N(R⁸)₂, -R⁷-S(O)ₜR¹⁰ (where t is 0 to 2) or alkyl; R³ is optionally substituted cycloalkyl or optionally substituted cycloalkylalkyl; R⁴ is hydrogen; R⁵ is hydrogen; each R⁶ is hydrogen; R⁷ is a straight or branched alkylene chain; R⁸ is hydrogen or alkyl; and R¹⁰ is alkyl, aryl or aralkyl.

Of the group of embodiments set forth above, another subgroup of embodiments is directed to the compound wherein the compound of formula (I) is a compound of formula (I) wherein m is 1 or 2; n is 1 or 2; p is 2; V is -C(O)-; R¹ is hydrogen or alkyl; R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is optionally substituted cycloalkyl or optionally substituted cycloalkylalkyl; each R⁴ is independently hydrogen, alkyl, halo, or haloalkyl; and each R⁵ and R⁶ is independently hydrogen, oxo, alkyl, halo or haloalkyl.

Of this subgroup of embodiments, a class of embodiments is directed to the compound wherein the compound of formula (I) is a compound of formula (I) wherein m is 1; n is 1; p is 2; V is -C(O)-; R¹ is hydrogen or alkyl; R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl; R³ is optionally substituted cycloalkyl or optionally substituted cycloalkylalkyl; R⁴ is hydrogen, alkyl, halo or haloalkyl; R⁵ is independently hydrogen, oxo, alkyl, halo or haloalkyl; and each R⁶ is independently hydrogen, oxo, alkyl, halo or haloalkyl.

Of the various embodiments of the invention as set of the Invention, another group of embodiments is directed to a compound for the treatment of an SCD-mediated disease or condition in a mammal wherein the compound of formula (I) is a compound of formula (I) wherein m is 1 or 2; n is 1 or 2; p is 2; V is -C(O)-; R¹ is hydrogen or alkyl; R² is selected from the group consisting of hydrogen, -R⁷-OR⁸, -R⁷-N(R⁸)₂, -R⁷-S(O)ₜR¹⁰ (where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is optionally substituted aryl; each R⁴ is independently hydrogen, alkyl, alkenyl, halo, haloalkyl, aryl or -R⁹-OR⁸; each R⁵ and R⁶ is independently hydrogen, oxo, alkyl, alkenyl, halo, haloalkyl or aryl; each R⁷ is independently a straight or branched alkylene or alkenylene chain; each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl; R⁹ is a direct bond or a straight or branched alkylene chain; and R¹⁰ is alkyl, aryl or aralkyl.

Of this group of embodiments, a subgroup of embodiments is directed to the compound wherein the compound of formula (I) is a compound of formula (I) wherein m is 1 or 2; n is 1 or 2; p is 2; V is -C(O)-; R¹ is hydrogen or alkyl; R² is selected from the group consisting of -R⁷-OR⁸, -R⁷-N(R⁸)₂, -R⁷-S(O)ₜR¹⁰ (where t is 0 to 2), alkyl, alkenyl, optionally substituted cycloalkyl, optionally substituted cycloalkylalkyl or cycloalkylalkenyl; R³ is optionally substituted aryl; each R⁴ is independently hydrogen, alkyl, halo, haloalkyl or -R⁹-OR⁸; each R⁵ and R⁶ is independently hydrogen, oxo, alkyl, halo or haloalkyl; each R⁷ is a straight or branched alkylene chain; each R⁸ is independently hydrogen, alkyl, haloalkyl, cycloalkyl, cycloalkylalkyl, aryl and aralkyl; R⁹ is a direct bond or a straight or branched alkylene chain; and R¹⁰ is alkyl, aryl or aralkyl.

Of this subgroup of embodiments, a class of embodiments is directed to the compound wherein the compound of formula (I) is a compound of formula (I) wherein m is 1 or 2; n is 1; p is 2; V is -C(O)-; R¹ is hydrogen or alkyl; R² is selected from the group consisting of -R⁷-OR⁸, -R⁷-N(R⁸)₂, -R⁷-S(O)ₜR¹⁰ (where t is 0, 1 or 2), alkyl, optionally substituted cycloalkyl or optionally substituted cycloalkylalkyl; R³ is optionally substituted aryl; each R⁴ is independently hydrogen, halo, haloalkyl or -R⁹-OR⁸; R⁵ is hydrogen; each R⁶ is hydrogen; R⁷ is a straight or branched alkylene chain; R⁸ is hydrogen or alkyl; R⁹ is a direct bond or a straight or branched alkylene chain; and R¹⁰ is alkyl, aryl or aralkyl.

Of the group of embodiments set forth above, another subgroup of embodiments is directed to the compound wherein the compound of formula (I) is a compound of formula (I) wherein m is 1 or 2; n is 1 or 2; p is 2; V is -C(O)-; R¹ is hydrogen or alkyl; R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is optionally substituted aryl; each R⁴ is independently hydrogen, alkyl, halo, or haloalkyl; and each R⁵ and R⁶ is independently hydrogen, oxo, alkyl, halo or haloalkyl.

Of his subgroup of embodiments, a class of embodiments is directed to the compound wherein the compound of formula (I) is a compound of formula (I) wherein m is 1 or 2; n is 1; p is 2; V is -C(O)-; R¹ is hydrogen or alkyl; R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl; R³ is optionally substituted aryl; each R⁴ is independently hydrogen, alkyl, halo or haloalkyl; R⁵ is hydrogen, oxo, alkyl, halo or haloalkyl; and each R⁶ is independently hydrogen, alkyl, halo or haloalkyl.

Of the various embodiments of the invention as set forth above in the Summary of the invention, another group of embodiments is directed to the compound of treating an SCD-mediated disease or condition in a mammal wherein the compound of formula (I) is a compound of formula (I) wherein m is 1 or 2; n is 1 or 2; p is 2; V is -C(O)-; R¹ is hydrogen or alkyl; R² is selected from the group consisting of hydrogen, -R⁷-OR⁸, -R⁷-N(R⁸)₂, -R⁷-S(O)ₜR¹⁰ (where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is optionally substituted heteroaryl, optionally substituted heteroarylalkyl or optionally substituted heteroarylalkenyl; each R⁴ is independently hydrogen, alkyl, alkenyl, halo, haloalkyl or aryl; each R⁵ and R⁸ is independently hydrogen, oxo, alkyl, alkenyl, halo, haloalkyl or aryl; each R⁷ is independently a straight or branched alkylene or alkenylene chain; each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl; and R¹⁰ is alkyl, aryl or aralkyl.

Of this group of embodiments, a subgroup of embodiments is directed to the compound wherein the compound of formula (I) is a compound wherein m is 1 or 2; n is 1 or 2; p is 2; V is -C(O)-; R¹ is hydrogen or alkyl; R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl; R³ is optionally substituted heteroaryl, optionally substituted heteroarylalkyl or optionally substituted heteroarylalkenyl; each R⁴ is independently hydrogen, alkyl, halo, or haloalkyl; each R⁶ and R⁶ is independently hydrogen, oxo, alkyl, halo or haloalkyl; each R⁷ is a straight or branched alkylene chain; each R⁸ is independently hydrogen, alkyl, haloalkyl, cycloalkyl, cycloalkylalkyl, aryl and aralkyl; and R¹⁰ is alkyl, aryl or aralkyl.

Of this subgroup of embodiments, a class of embodiments is directed to the compound wherein the compound of formula (I) is a compound wherein m is 1 or 2; n is 1; p is 2; V is -C(O)-; R¹ is hydrogen or alkyl; R² is selected from the group consisting of -R⁷-OR⁶, -R⁷-N(R⁸)₂, -R⁷-S(O)ₜR¹⁰ (where t is 0, 1 or 2) or alkyl; R³ is optionally substituted heteroaryl, optionally substituted heteroarylalkyl or optionally substituted heteroarylalkenyl; each R⁴ is independently hydrogen, halo or haloalkyl; R⁵ is hydrogen; each R⁶ is hydrogen; R⁷ is a straight or branched alkylene chain; R⁶ is hydrogen or alkyl; and R¹⁰ is alkyl, aryl or aralkyl.

Of the group of embodiments set forth above, another subgroup of embodiments is directed to the compound wherein the compound of formula (I) is a compound of formula (I) wherein m is 1 or 2; n is 1 or 2; p is 2; V is -C(O)-; R¹ is hydrogen or alkyl; R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is optionally substituted heteroaryl, optionally substituted heteroarylalkyl or optionally substituted heteroarylalkenyl; each R⁴ is independently hydrogen, alkyl, halo, or haloalkyl; and each R⁵ and R⁶ is independently hydrogen, oxo, alkyl, halo or haloalkyl.

Of this subgroup of embodiments, a class of embodiments is directed to the compound wherein the compound of formula (I) is a compound of formula (I) wherein m is 1 or 2; n is 1; p is 2; V is -C(O)-; R¹ is hydrogen or alkyl; R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl; R³ is optionally substituted heteroaryl, optionally substituted heteroarylalkyl or optionally substituted heteroarylalkenyl; each R⁴ is independently hydrogen, alkyl, halo, or haloalkyl; R⁵ is hydrogen, oxo, alkyl, halo or haloalkyl; and each R⁶ independently hydrogen, oxo, alkyl, halo or haloalkyl.

Of the various embodiments of the invention as set forth above in the Summary of the Invention, another group of embodiments is directed to the compound of treating an SCD-mediated disease or condition in a mammal wherein the compound of formula (I) is a compound of formula (I) wherein m is 1 or 2; n is 1 or 2; p is 2; V is -C(O)-; R¹ is hydrogen or alkyl; R² is selected from the group consisting of hydrogen, -R⁷-OR⁸, -R⁷-N(R⁸)₂, -R⁷-S(O)ₜR¹⁰ (where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is optionally substituted aralkyl or optionally substituted aralkenyl; each R⁴ is independently hydrogen, alkyl, alkenyl, halo, haloalkyl, aryl or -R⁹-OR⁸; each R⁵ and R⁸ is independently hydrogen, oxo, alkyl, alkenyl, halo, haloalkyl or aryl; each R⁷ is independently a straight or branched alkylene or alkenylene chain; each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl; R⁹ is a direct bond or a straight or branched alkylene chain; and R¹⁰ is alkyl, aryl or aralkyl.

Of the group of embodiments set forth above, a subgroup of embodiments is directed to the compound wherein the compound of formula (I) is a compound of formula (I) wherein m is 1 or 2; n is 1 or 2; p is 2; V is -C(O)-; R¹ is hydrogen or alkyl; R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl; R³ is optionally substituted aralkyl or optionally substituted aralkenyl; each R⁴ is independently hydrogen, alkyl, halo, or haloalkyl; each R⁵ and R⁶ is independently hydrogen, oxo, alkyl, halo or haloalkyl; each R⁷ is a straight or branched alkylene chain; each R⁸ is independently hydrogen, alkyl, haloalkyl, cycloalkyl, cycloalkylalkyl, aryl and aralkyl; and R¹⁰ is alkyl, aryl or aralkyl.

Of this subgroup of embodiments, a class of embodiments is directed to the compound wherein the compound of formula (I) is a compound of formula (I) wherein m is 1 or 2; n is 1; p is 2; V is -C(O)-; R¹ is hydrogen or alkyl; R² is selected from the group consisting of -R⁷-OR⁸, -R⁷-N(R⁸)₂, -R⁷-S(O)ₜR¹⁰ (where t is 0, 1 or 2) or alkyl; R³ is optionally substituted aralkyl or optionally substituted aralkenyl; each R⁴ is independently hydrogen, halo or haloalkyl; R⁵ is hydrogen; each R⁸ is hydrogen; R⁷ is a straight or branched alkylene chain; R⁸ is hydrogen or alkyl; and R¹⁰ is alkyl, aryl or aralkyl.

Of the group of embodiments set forth above, another subgroup of embodiments is directed to the compound wherein the compound of formula (I) is a compound wherein m is 1 or 2; n is 1 or 2; p is 2; V is -C(O)-; R¹ is hydrogen or alkyl; R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is optionally substituted aralkyl or optionally substituted aralkenyl; each R⁴ is independently hydrogen, alkyl, halo, or haloalkyl; and each R⁵ and R⁶ is independently hydrogen, oxo, alkyl, halo or haloalkyl.

This subgroup of embodiments, a class of embodiments is directed to the compound wherein the compound of formula (I) is a compound of formula (1) wherein m is 1 or 2; n is 1; p is 2; V is -C(O)-; R¹ is hydrogen or alkyl; R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl; R³ is optionally substituted aralkyl or optionally substituted aralkenyl; each R⁴ is independently hydrogen, alkyl, halo or haloalkyl; and R⁵ is hydrogen, oxo, alkyl, halo or haloalkyl; and each R⁶ is independently hydrogen, oxo, alkyl, halo or haloalkyl.

Of the various embodiments of the invention as set forth above in the Summary of the Invention, another group of embodiments is directed to the compound for treating an SCD-mediated disease or condition in a mammal wherein the mammal is a human. Of this group of embodiments, a subgroup of embodiments is directed to the compound for use in the treatment of a disease or condition related to serum levels of triglyceride, VLDL, HDL, LDL, total cholesterol or the process of reverse cholesterol transport. Another subgroup of embodiments is directed to the compound method wherein the disease or condition is a disease or condition related to serum triglyceride levels. Another subgroup of embodiments is directed to the compound wherein the disease or condition is a disease or condition releated to serum cholesterol levels. Another subgroup of embodiments is directed to the compound wherein the disease or condition is selected from the group consisting of Type II diabetes, impaired glucose tolerance, insulin resistance, hypertension, obesity, hypertriglyceridemia, low HDL, lipidemia, dyslipidemia, microalbuminemia, hyperuricaemia hyperleptinaemia, metabolic syndrome and any combination of these. Of these subgroups of embodiments, classes of embodiments are directed to the compound wherein the disease or condition is Type II diabetes, obesity, dyslipidemia and/or metabolic syndrome.

Of the pharmaceutical compositions of the invention set forth above in the Summary of the Invention, one group of embodiments is directed to the pharmaceutical compositions wherein the therapeutically effective amound of the compound of formula (I) is an amount effective to modulate a lipid level in a mamal when administered to the mammal. Of this group of embodiments, a subgroup of embodiments is wherein the lipid is triglyceride. Another subgroup of embodiments is wherein the lipid is cholesterol. Another group of embodiments is directed to pharmaceutical compositions wherein the therapeutically effective amound of the compound of formula (I) is an amount effective to modulate HDL-cholesterol levels when administered to a mammal.

Of the compounds of formula (I) set forth above in the Summary of the Invention, one group of embodiments is directed to the compounds of formula (I) wherein m is 1, 2 or 3; n is 1, 2, 3 or 4; p is 2, 3 or 4; V is -C(O)-, -S(O)- or -S(O)₂-; R¹ is hydrogen, alkyl, alkenyl, aryl, aralkyl, or aralkenyl ; R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; R³ is selected from the group consisting of cycloalkyl substituted by one or more substituents independently selected from the group consisting of alkyl, alkenyl, halo, haloalkyl, haloalkenyl, cyano, nitro, aryl, aralkyl, cycloalkyl, cycloalkylalkyl, heterocyclyl, heterocyclylalkyl, heteroaryl, heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-S(O)ₜOR¹⁰ (where t is 1 or 2), -R⁹-S(O)ₜR¹⁰ (where t is 0, 1 or 2), and -R⁹-S(O)ₜN(R⁸)₂ (where t is 1 or 2); each R⁴ is independently hydrogen, alkyl, alkenyl, halo, haloalkyl, aryl, cyano, nitro, -R⁹-OR⁸, -R⁹-N(R⁸)₂ or -S(O)ₜR¹⁰ (where t is 0, 1 or 2); each R⁵ and R⁶ is independently hydrogen, oxo, alkyl, alkenyl, halo, haloalkyl or aryl; or one R⁵ and one R⁶ may together form an straight or branched alkylene bridge; each R⁷ is independently a straight or branched alkylene or alkenylene chain; each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl; each R⁹ is independently a direct bond or a straight or branched alkylene or alkenylene chain; and R¹⁰ is alkyl, alkenyl, haloalkyl, cycloalkyl, cycloalkylalkyl, aryl, aralkyl, heterocyclyl, heterocylylalkyl, heteroaryl or heteroarylalkyl.

Of this group of embodiments, a subgroup of embodiments is directed the compounds wherein R³ is cyclopropyl substituted by optionally substituted aryl or optionally substituted heteroaryl.

Of this subgroup of embodiments, a class of embodiments is directed to the compounds wherein m is 1 or 2; n is 1 or 2; p is 2; V is -C(O)-; R¹ is hydrogen or alkyl; R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; each R⁴ is independently hydrogen, alkyl, alkenyl, halo, haloalkyl, aryl or -R⁹-OR⁸; each R⁵ and R⁶ is independently hydrogen, oxo, alkyl, alkenyl, halo, haloalkyl or aryl; each R⁷ is independently a straight or branched alkylene or alkenylene chain; each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl; R⁹ is a direct bond or a straight or branched alkylene chain; and R¹⁰ is alkyl, aryl or aralkyl.

Of this class of embodiments, a subclass of embodiments is directed to the compounds wherein m is 1 or 2; n is 1 or 2; p is 2; V is -C(O)-; R¹ is hydrogen or alkyl; R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl; each R⁴ is independently hydrogen, alkyl, halo, or haloalkyl; and each R⁵ and R⁶ is independently hydrogen, oxo, alkyl, halo or haloalkyl.

Of this subclass of embodiments, a set of embodiments is directed to the compounds wherein m is 1; n is 1; p is 2; V is -C(O)-; R¹ is hydrogen or alkyl; R² is optionally substituted aralkyl, optionally substituted heteroarylalkyl, or optionally substituted heterocyclylalkyl; R³ is cyclopropyl substituted by phenyl; R⁴ is hydrogen, alkyl, halo or haloalkyl; R⁵ is hydrogen, oxo, alkyl, halo or haloalkyl; and each R⁶ is hydrogen.

Of the class of embodiments set forth above, another subclass of embodiments is directed to the compounds wherein m is 1 or 2; n is 1 or 2; p is 2; V is -C(O)-; R¹ is hydrogen or alkyl; R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkylalkyl or cycloalkylalkenyl; each R⁴ is independently hydrogen, alkyl, halo, or haloalkyl; each R⁵ and R⁶ is independently hydrogen, oxo, alkyl, halo or haloalkyl; each R⁷ is a straight or branched alkylene chain; each R⁸ is independently hydrogen, alkyl, haloalkyl, cycloalkyl, cycloalkylalkyl, aryl and aralkyl; and R¹⁰ is alkyl, aryl or aralkyl.

Of this subclass of embodiments, a set of embodiments is directed to the compounds wherein m is 1; n is 1; p is 2; V is -C(O)-; R¹ is hydrogen or alkyl; R² is selected from the group consisting of alkyl, -R⁷-OR⁸, -R⁷-N(R⁸)₂, or -R⁷-S(O)ₜR¹⁰ (where t is 0); R³ is cyclopropyl substituted by phenyl; R⁴ is hydrogen; R⁵ is hydrogen; each R⁶ is hydrogen; R⁷ is a straight or branched alkylene chain; R⁸ is hydrogen or alkyl; and R¹⁰ is alkyl, aryl or aralkyl.

Of the compounds described above, the most preferred are selected from the group consisting of the following:
6-[4-(2-Phenyl-cyclopropanecarbonyl)-piperazin-1-yl]-*N*-(3-phenyl-propyl)-nicotinamide;
*N*-(4-Phenyl-butyl)-6-[4-(2-phenyl-cyclopropanecarbonyl)-piperazin-1-yl]-nicotinamide;
*N*-(1-Methyl-3-phenyl-propyl)-6-[4-(2-phenyl-cyclopropanecarbonyl)-piperazin-1-yl]-nicotinamide;
*N*-Phenethyl-6-[4-(2-phenyl-cyclopropanecarbonyl)-piperazin-1-yl]-nicotinamide;
6-[4-(2-Phenyl-cyclopropanecarbonyl)-piperazin-1-yl]-*N*-(tetrahydro-furan-2-ylmethyl)-nicotinamide;
*N*-[2-(3H-1midazol-4-yl)-ethyl]-6-[4-(2-phenyl-cyclopropanecarbonyl)-piperazin-1-yl]-nicotinamide;
*N*-(3-Imidazol-1-yl-propyl)-6-[4-(2-phenyl-cyclopropanecarbonyl)-piperazin-1-yl]-nicotinamide;
*N*-(3-Ethoxy-propyl)-6-[4-(2-phenyl-cyclopropanecarbonyl)-piperazin-1-yl]-nicotinamide;
*N*-(2-Ethylsulfanyl-ethyl)-6-[4-(2-phenyl-cyclopropanecarbonyl)-piperazin-1-yl]-nicotinamide;
*N*-(1,3-Dimethylbutyl)-6-[4-(2-phenyl-cyclopropanecarbonyl)-piperazin-1-yl]-nicotinamide;
*N*-(3-Methyl-butyl)-6-[4-(2-phenyl-cyclopropanecarbonyl)-piperazin-1-yl]-nicotinamide;
*N*-(3-Methoxy-propyl)-6-[4-(2-phenyl-cyclopropanecarbonyl)-piperazin-1-yl]-nicotinamide;
*N*-(3-Butoxy-propyl)-6-[4-(2-phenyl-cyclopropanecarbonyl)-piperazin-1-yl]-nicotinamide;
*N*-Pentyl-6-[4-(2-phenyl-cyclopropanecarbonyl)-piperazin-1-yl]-nicotinamide;
*N*-(3-Dimethylamino-propyl)-6-[4-(2-phenyl-cyclopropanecarbonyl)-piperazin-1-yl]-nicotinamide;
*N*-(3-lsopropoxy-propyl)-6-[4-(2 -phenyl-cyclopropanecarbonyl)-piperazin-1-yl]-nicotinamide
*N*-(1-Methyl-butyl)-6-[4-(2-phenyl-cyclopropanecarbonyl)-piperazin-1-yl]-nicotinamide;
*N*-Butyl-6-[4-(2-phenyl-cyclopropanecarbonyl)-piperazin-1-yl]-nicotinamide;
*N*-Hexyl-6-[4-(2-phenyl-cyclopropanecarbonyl)-piperazin-1-yl]-nicotinamide; and
*N*-(2-Methyl-butyl)-6-[4-(2-phenyl-cyclopropanecarbonyl)-piperazin-1-yl]-nicotinamide. In addition to the foregoing compounds, other specific embodiments of the invention are set below in Example 10.

In another embodiment, the present invention is directed towards the treatment and/or prevention of diseases mediated by stearoyl-CoA desaturase (SCD), especially human SCD (hSCD), preferably diseases related to dyslipidemia and disorders of lipid metabolism, and especially a disease related to elevated plasma lipid levels, cardiovascular disease, diabetes, obesity, metabolic syndrome by administering an effective amount of an agent of the invention.

### Utility and Testing of the Compounds of the Invention

The present invention relates to compounds, pharmaceutical compositions and the compounds and pharmaceutical compositions for the treatment and/or prevention of diseases mediated by stearoyl-CoA desaturase (SCD), especially human SCD (hSCD), preferably diseases related to dyslipidemia and disorders of lipid metabolism, and especially a disease related to elevated plasma lipid levels, especially cardiovascular disease, diabetes, obesity, metabolic syndrome, by administering to a patient in need of such treatment an effective amount of an SCD-modulating, especially inhibiting, agent.

In general, the present invention provides a compound or treating a patient for, or protecting a patient from developing, a disease related to dyslipidemia and/or a disorder of lipid metabolism, wherein lipid levels in an animal, especially a human being, are outside the normal range (*i.e*., abnormal lipid level, such as elevated plasma lipid levels), especially levels higher than normal, preferably where said lipid is a fatty acid, such as a free or complexed fatty acid, triglycerides, phospholipids, or cholesterol, such as where LDL-cholesterol levels are elevated or HDL-cholesterol levels are reduced, or any combination of these, where said lipid-related condition or disease is an SCD-mediated disease or condition, comprising administering to an animal, such as a mammal, especially a human patient, a therapeutically effective amount of a compound of formula (I) or a pharmaceutical composition comprising a compound of formula (I) wherein the compound modulates the activity of SCD, preferably human SCD1.

All of these compounds modulate, preferably inhibit, the activity of human SCD enzymes, especially human SCD1.

The general value of the compounds of the invention in modulating, especially inhibiting, the activity of SCD is demonstrated by the data of Table 3 in Example 10 below, wherein the ability of a sample of such compounds to inhibit SCD biological activity is disclosed. Alternatively, the general value of the compounds in treating disorders and diseases may be established in industry standard animal models for demonstrating the efficacy of compounds in treating obesity, diabetes or elevated triglyceride or cholesterol levels or for improving glucose tolerance. Such models include Zucker obese *fa*/*fa* rats (available from Harlan Sprague Dawley, Inc. (Indianapolis, Indiana)), or the Zucker diabetic fatty rat (ZDF/GmiCrl-*fa*/*fa*) (available from Charles River Laboratories (Montréal, Quebec)).

The present invention also relates to pharmaceutical composition as defined in claim 89 containing the novel compounds disclosed herein. In one embodiment, the present invention relates to a composition comprising compounds of the invention in a pharmaceutically acceptable carrier and in an amount effective to modulate triglyceride level or to treat diseases related to dyslipidemia and disorders of lipid metabolism, when administered to an animal, preferably a mammal, most preferably a human patient. In an embodiment of such composition, the patient has an elevated lipid level, such as elevated triglycerides or cholesterol, before administration of said compound of the invention and the compound of the invention is present in an amount effective to reduce said lipid level.

The compounds of the instant invention are inhibitors of delta-9 desaturases and are useful for treating diseases and disorders in humans and other organisms, including all those human diseases and disorders which are the result of aberrant delta-9 desaturase biological activity or which may be ameliorated by modulation of delta-9 desaturase biological activity.

As defined herein, an SCD-mediated disease or condition includes a disease or condition which is, or is related to, cardiovascular disease, dyslipidemias (including but not limited to disorders of serum levels of triglycerides, hypertriglyceridemia, VLDL, HDL, LDL, fatty acid Desaturation Index (e.g. the ratio of 18:1/18:0 fatty acids, or other fatty acids, as defined elsewhere herein), cholesterol, and total cholesterol, hypercholesterolemia, as well as cholesterol disorders (including disorders characterized by defective reverse cholesterol transport), familial combined hyperlipidemia, coronary artery disease, atherosclerosis, heart disease, cerebrovascular disease (including stroke, ischemic stroke and transient ischemic attack (TIA)), peripheral vascular disease, and ischemic retinopathy. In a preferred embodiment, compounds of the invention will, in a patient, increase HDL levels and/or decrease triglyceride levels and/or decrease LDL or non-HDL-cholesterol levels.

An SCD-mediated disease or condition also includes metabolic syndrome (including dyslipidemia, obesity and insulin resistance, hypertension, microalbuminemia, hyperuricaemia, and hypercoagulability), Syndrome X, diabetes, insulin resistance, decreased glucose tolerance, non-insulin-dependent diabetes mellitus, Type II diabetes, Type I diabetes, diabetic complications, body weight disorders (including but not limited to obesity, overweight, cachexia and anorexia), weight loss, body mass index and leptin related diseases. In a preferred embodiment, compounds of the invention will be used to treat diabetes mellitus and obesity.

As used herein, the term "metabolic syndrome" is a recognized clinical term used to describe a condition comprising combinations of Type II diabetes, impaired glucose tolerance, insulin resistance, hypertension, obesity, increased abdominal girth, hypertriglyceridemia, low HDL, hyperuricaemia, hypercoagulability and/or microalbuminemia.

An SCD-mediated disease or condition also includes fatty liver, hepatic steatosis, hepatitis, non-alcoholic hepatitis, non-alcoholic steatohepatitis (NASH), alcoholic hepatitis, acute fatty liver, fatty liver of pregnancy, drug-induced hepatitis, erythrohepatic protoporphyria, iron overload disorders, hereditary hemochromatosis, hepatic fibrosis, hepatic cirrhosis, hepatoma and conditions related thereto.

An SCD-mediated disease or condition also includes a disease or condition which is, or is related to primary hypertriglyceridemia, or hypertriglyceridemia secondary to another disorder or disease, such as hyperlipoproteinemias, familial histiocytic reticulosis, lipoprotein lipase deficiency, apolipoprotein deficiency (such as ApoCII deficiency or ApoE deficiency), and the like, or hypertriglyceridemia of unknown or unspecified etiology.

An SCD-mediated disease or condition also includes a disorder of polyunsaturated fatty acid (PUFA) disorder, or a skin disorder, including eczema, acne, psoriasis, keloid scar formation or prevention, diseases related to production or secretions from mucous membranes, such as monounsaturated fatty acids, wax esters.

An SCD-mediated disease or condition also includes inflammation, sinusitis, asthma, pancreatitis, osteoarthritis, rheumatoid arthritis, cystic fibrosis, and premenstrual syndrome.

An SCD-mediated disease or condition also includes a disease or condition which is, or is related to cancer, neoplasia, malignancy, metastases, tumours (benign or malignant), carcinogenesis, hepatomas.

An SCD-mediated disease or condition also includes a condition where increasing lean body mass or lean muscle mass is desired, such as is desirable in enhancing performance through muscle building. Myopathies and lipid myopathies such as carnitine palmitoyltransferase deficiency (CPT I or CPT II) are also included herein. Such treatments are useful in humans and in animal husbandry, including for administration to bovine, porcine or avian domestic animals or any other animal to reduce triglyceride production and/or provide leaner meant products and/or healthier animals.

An SCD-mediated disease or condition also includes a disease or condition which is, or is related to, neurological diseases, psychiatric disorders, multiple sclerosis, eye diseases, and immune disorders.

An SCD-mediated disease or condition also includes a disease or condition which is, or is related to, viral diseases or infections including all positive strand RNA viruses, coronaviruses, SARS virus, SARS-associated coronavirus, Togaviruses, Picornaviruses, Coxsackievirus, Yellow Fever virus, Flaviviridae, ALPHAVIRUS (TOGAVIRIDAE) including Rubella virus, Eastern equine encephalitis virus, Western equine encephalitis virus, Venezuelan equine encephalitis virus, Sindbis virus, Semliki forest virus, Chikungunya virus, O'nyong'nyong virus, Ross river virus, Mayaro virus, Alphaviruses; ASTROVIRIDAE including Astrovirus, Human Astroviruses; CALICIVIRIDAE including Vesicular exanthema of swine virus, Norwalk virus, Calicivirus, Bovine calicivirus, Pig calcivirus, Hepatitis E; CORONAVIRIDAE including Coronavirus, SARS virus, Avian infectious bronchitis virus, Bovine coronavirus, Canine coronavirus, Feline infectious peritonitis virus, Human coronavirus 299E, Human coronavirus OC43, Murine hepatitis virus, Porcine epidemic diarrhea virus, Porcine hemagglutinating encephalomyelitis virus, Porcine transmissible gastroenteritis virus, Rat coronavirus, Turkey coronavirus, Rabbit coronavirus, Berne virus, Breda virus; FLAVIVIRIDAE including Hepatitis C virus, West Nile virus, Yellow Fever virus, St. Louis encephalitis virus, Dengue Group, Hepatitis G virus, Japanese B encephalitis virus, Murray Valley encephalitis virus, Central European tick-borne encephalitis virus, Far Eastern tick-borne encephalitis virus, Kyasanur forest virus, Louping ill virus, Powassan virus, Omsk hemorrhagic fever virus, Kumilinge virus, Absetarov anzalova hypr virus, Ilheus virus, Rocio encephalitis virus, Langat virus, Pestivirus , Bovine viral diarrhea, Hog cholera virus, Rio Bravo Group, Tyuleniy Group, Ntaya Group, Uganda S Group, Modoc Group; PICORNAVIRIDAE including Coxsackie A virus, Rhinovirus, Hepatitis A virus, Encephalomyocarditis virus, Mengovirus, ME virus, Human poliovirus 1, Coxsackie B; POTYVIRIDAE including Potyvirus, Rymovirus, Bymovirus. Additionally it can be a disease or infection caused by or linked to Hepatitis viruses, Hepatitis B virus, Hepatitis C virus, human immunodeficiency virus (HIV). Treatable viral infections include those where the virus employs an RNA intermediate as part of the replicative cycle (hepatitis or HIV); additionally it can be a disease or infection caused by or linked to RNA negative strand viruses such as influenza and parainfluenza viruses.

The compounds identified in the instant specification inhibit the desaturation of various fatty acids (such as the C9-C10 desaturation of stearoyl-CoA) which is accomplished by delta-9 desaturases, such as stearoyl-CoA desaturase 1 (SCD1). As such these compounds inhibit the formation of various fatty acids and downstream metabolites thereof. This may lead to an accumulation of stearoyl-CoA or palmitoyl-CoA and other upstream precursors of various fatty acids; which may possibly result in a negative feedback loop causing an overall change in fatty acid metabolism. Any of these consequences may ultimately be responsible for the overall therapeutic benefit provided by these compounds.

Typically, a successful SCD inhibitory therapeutic agent will meet some or all of the following criteria. Oral availability should be at or above 20%. Animal model efficacy is less than about 2 mg/Kg, 1 mg/Kg, or 0.5 mg/Kg and the target human dose is between 50 and 250 mg/70 Kg, although doses outside of this range may be acceptable.("mg/Kg" means milligrams of compound per kilogram of body mass of the subject to whom it is being administered). The therapeutic index (or ratio of toxic dose to therapeutic dose) should be greater than 100. The potency (as expressed by IC₅₀ value) should be less than 10 µM, preferably below 1 µM and most preferably below 50 nM. The IC₅₀ ("Inhibitory Concentration - 50%") is a measure of the amount of compound required to achieve 50% inhibition of SCD activity, over a specific time period, in an SCD biological activity assay. Any process for measuring the activity of SCD enzymes, preferably mouse or human SCD enzymes, may be utilized to assay the activity of the compounds useful in the methods of the invention in inhibiting said SCD activity. Compounds of the invention demonstrate an IC₅₀ in a 15 minute microsomal assay of preferably less than 10 µM, less than 5 µM, less than 2.5 µM, less than 1 µM, less than 750 nM, less than 500 nM, less than 250 nM, less than 100 nM, less than 50 nM, and most preferably less than 20 nM. The compound of the invention may show reversible inhibition (i.e., competitive inhibition) and preferably does not inhibit other iron binding proteins. The required dosage should preferably be no more than about once or twice a day or at meal times.

The identification of compounds of the invention as SCD inhibitors was readily accomplished using the SCD enzyme and microsomal assay procedure described in Brownlie et al, *supra.*

Data showing inhibition of SCD by compounds of the invention as tested in this assay are presented in Table 3 in Example 10 below. In particular, Table 3 sets forth the % remaining SCD activity at 10 µM of of a compound of the invention in the indicated assay.

These results provide the basis for analysis of the structure-activity relationship (SAR) between test compounds and SCD. Certain R groups tend to provide more potent inhibitory compounds. SAR analysis is one of the tools those skilled in the art may now employ to identify preferred embodiments or the compounds of the invention for use as therapeutic agents.

Other methods of testing the compounds disclosed herein are also readily available to those skilled in the art. Thus, in addition, said contacting may be accomplished *in vivo.* In one such embodiment, said contacting in step (a) is accomplished by administering said chemical agent to an animal afflicted with a triglyceride (TG)- or very low density lipoprotein (VLDL)-related disorder and subsequently detecting a change in plasma triglyceride level in said animal thereby identifying a therapeutic agent useful in treating a triglyceride (TG)- or very low density lipoprotein (VLDL)-related disorder. In such embodiment, the animal may be a human, such as a human patient afflicted with such a disorder and in need of treatment of said disorder.

In specific embodiments of such *in vivo* processes, said change in SCD1 activity in said animal is a decrease in activity, preferably wherein said SCD1 modulating agent does not substantially inhibit the biological activity of a delta-5 desaturase, delta-6 desaturase or fatty acid synthetase.

The model systems useful for compound evaluation may include, but are not limited to, the use of liver microsomes, such as from mice that have been maintained on a high carbohydrate diet, or from human donors, including persons suffering from obesity. Immortalized cell lines, such as HepG2 (from human liver), MCF-7 (from human breast cancer) and 3T3-L1 (from mouse adipocytes) may also be used. Primary cell lines, such as mouse primary hepatocytes, are also useful in testing the compounds of the invention. Where whole animals are used, mice used as a source of primary hepatocyte cells may also be used wherein the mice have been maintained on a high carbohydrate diet to increase SCD activity in mirocrosomes and/or to elevate plasma triglyceride levels (i.e., the 18:1/18:0 ratio); alternatively mice on a normal diet or mice with normal triglyceride levels may be used. Mouse models employing transgenic mice designed for hypertriglyceridemia are also available as is the mouse phenome database. Rabbits and hamsters are also useful as animal models, especially those expressing CETP (cholesteryl ester transfer protein).

Another suitable method for determining the *in vivo* efficacy of the compounds of the invention is to indirectly measure their impact on inhibition of SCD enzyme by measuring a subject's Desaturation Index after administration of the compound. "Desaturation Index" as employed in this specification means the ratio of the product over the substrate for the SCD enzyme as measured from a given tissue sample. This may be calculated using three different equations 18:1n-9/18:0 (oleic acid over stearic acid); 16:1n-7/16:0 (palmitoleic acid over palmitic acid); and/or 16:1n-7 + 18:1n-7/16:0 (measuring all reaction products of 16:0 desaturation over 16:0 substrate). Desaturation Index is primarily measured in liver or plasma triglycerides, but may also be measured in other selected lipid fractions from a variety of tissues. Desaturation Index, generally speaking, is a tool for plasma lipid profiling.

A number of human diseases and disorders are the result of aberrant SCD1 biological activity and may be ameliorated by modulation of SCD1 biological activity using the therapeutic agents of the invention.

Inhibition of SCD expression may also affect the fatty acid composition of membrane phospholipids, as well as production or levels of triglycerides and cholesterol esters. The fatty acid composition of phospholipids ultimately determines membrane fluidity, white the effects on the composition of triglycerides and cholesterol esters can affect lipoprotein metabolism and adiposity.

In carrying out the procedures of the present invention it is of course to be understood that reference to particular buffers, media, reagents, cells, culture conditions are not intended to be limiting, but are to be read so as to include all related materials that one of ordinary skill in the art would recognize as being of interest or value in the particular context in which that discussion is presented. For example, it is often possible to substitute one buffer system or culture medium for another and still achieve similar, if not identical, results. Those of skill in the art will have sufficient knowledge of such systems and methodologies so as to be able, without undue experimentation, to make such substitutions as will optimally serve their purposes in using the methods and procedures disclosed herein.

### Pharmaceutical Composition of the Invention and Administration

The present invention also relates to pharmaceutical composition as defined in claim 89 containing the compounds of the invention disclosed herein. In one embodiment, the present invention relates to a composition comprising compounds of the invention in a pharmaceutically acceptable carrier and in an amount effective to modulate triglyceride level or to treat diseases related to dyslipidemia and disorders of lipid metabolism, when administered to an animal, preferably a mammal, most preferably a human patient. In an embodiment of such composition, the patient has an elevated lipid level, such as elevated triglycerides or cholesterol, before administration of said compound of the invention and the compound of the invention is present in an amount effective to reduce said lipid level.

The pharmaceutical compositions useful herein also contain a pharmaceutically acceptable carrier, including any suitable diluent or excipient, which includes any pharmaceutical agent that does not itself induce the production of antibodies harmful to the individual receiving the composition, and which may be administered without undue toxicity. Pharmaceutically acceptable carriers include liquids, such as water, saline, glycerol and ethanol, and the like. A thorough discussion of pharmaceutically acceptable carriers, diluents, and other excipients is presented in REMINGTON'S PHARMACEUTICAL SCIENCES (Mack Pub. Co., N.J. current edition).

Those skilled in the art know how to determine suitable doses of the compounds for use in treating the diseases and disorders contemplated herein. Therapeutic doses are generally identified through a dose ranging study in humans based on preliminary evidence derived from animal studies. Doses must be sufficient to result in a desired therapeutic benefit without causing unwanted side-effects for the patient. The preferred dosage range for an animal is 0.001 mg/Kg to 10,000 mg/Kg, including 0.5 mg/Kg, 1.0 mg/Kg and 2.0 mg/Kg, though doses outside this range may be acceptable. The dosing schedule may be once or twice per day, although more often or less often may be satisfactory.

Those skilled in the art are also familiar with determining administration methods (oral, intravenous, inhalation, sub-cutaneous, etc.), dosage forms, suitable pharmaceutical excipients and other matters relevant to the delivery of the compounds to a subject in need thereof.

In an alternative use of the invention, the compounds of the invention can be used in *in vitro* or *in vivo* studies as exemplary agents for comparative purposes to find other compounds also useful in treatment of, or protection from, the various diseases disclosed herein.

### Preparation of the Compounds of Formula (I)

It is understood that in the following description, combinations of substituents and/or variables of the depicted formulae are permissible only if such contributions result in stable compounds.

It will also be appreciated by those skilled in the art that in the process described below the functional groups of intermediate compounds may need to be protected by suitable protecting groups. Such functional groups include hydroxy, amino, mercapto and carboxylic acid. Suitable protecting groups for hydroxy include trialkylsilyl or diarylalkylsilyl (*e.g., t*-butyldimethylsilyl, *t*-butyldiphenylsilyl or trimethylsilyl), tetrahydropyranyl, benzyl. Suitable protecting groups for amino, amidino and guanidino include *t*-butoxycarbonyl, benzyloxycarbonyl.

Suitable protecting groups for mercapto include -C(O)-R" (where R" is alkyl, aryl or arylalkyl), *p*-methoxybenzyl, trityl. Suitable protecting groups for carboxylic acid include alkyl, aryl or arylalkyl esters.

Protecting groups may be added or removed in accordance with standard techniques, which are well-known to those skilled in the art and as described herein.

The use of protecting groups is described in detail in Green, T.W. and P.G.M. Wutz, Protective Groups in Organic Synthesis (1999), 3rd Ed., Wiley. The protecting group may also be a polymer resin such as a Wang resin or a 2-chlorotrityl-chloride resin.

It will also be appreciated by those skilled in the art, although such protected derivatives of compounds of this invention may not possess pharmacological activity as such, they may be administered to a mammal and thereafter metabolized in the body to form compounds of the invention which are pharmacologically active. Such derivatives may therefore be described as "prodrugs".

The following Reaction Schemes illustrate methods to make compounds of this invention. The various R groups in the Reaction Schemes have the same meaning as described above in the Summary of the Invention unless otherwised noted. It is understood that one of those skilled in the art would be able to make these compounds by similar methods or by methods known to one skilled in the art. In general, starting components may be obtained from sources such as Sigma Aldrich, Lancaster Synthesis, Inc., Maybridge, Matrix Scientific, TCI, and Fluorochem USA, etc. or synthesized according to sources known to those skilled in the art (see, e.g., Advanced Organic Chemistry: Reactions, Mechanisms, and Structure, 5th edition (Wiley, December 2000)) or prepared as described in this invention.

The following Reaction Scheme 1 illustrates a method of preparing the compounds of the invention by solid phase technology.

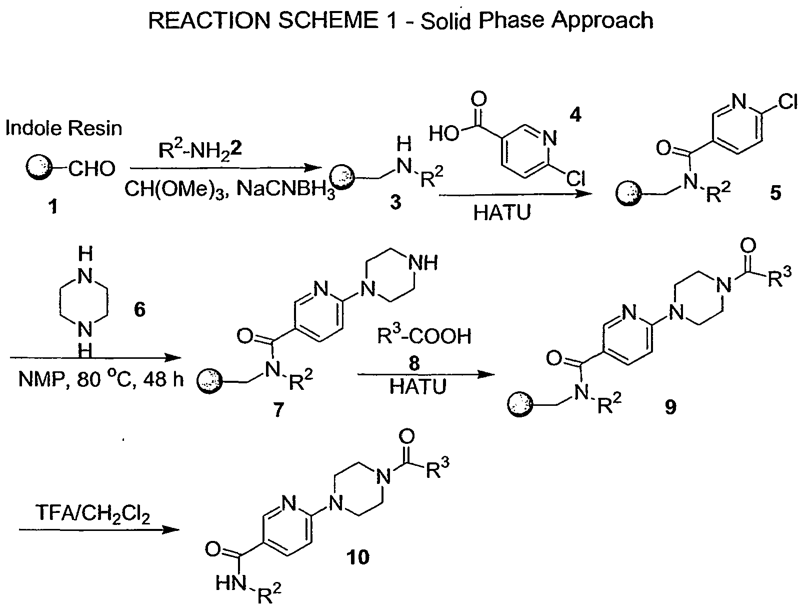

### Experimental Procedures - Solid Phase Approach

A preferred synthetic scheme employs solid phase synthesis using Kan™ reactors. Kans^{™} reactors are rigid containers with mesh side walls. A single compound is synthesized in each one, and each one contains a unique, miniature radiofrequency label along with the actual solid phase resin.

Kan reactors are designed to be loaded with solid phase resin beads and an Rf tag. The synthesis takes place by allowing reagents to flow through the outer mesh walls of the Kan. Syntheses are performed using normal laboratory glassware and apparatus for heating, cooling, mixing, etc.

There are 3 sizes of Kan reactors available which may be used interchangeably with the AccuTag system - MicroKans, MiniKans, and MacroKans. Typically about 30-300 mg of most commercial resins are loaded into a Kan leaving enough space available for the resin to swell and still remain loose within the Kan.

Synthesis takes place by the flow of reagents through the mesh walls of the Kan. The Kan reactors permit virtually any synthetic chemistry which can be performed using loose solid phase resin and conventional laboratory glassware to be done using the AccuTag system.

The Kan is made of high-grade polypropylene with a polypropylene mesh side wall. The Kan is filled with the solid phase resin and the radiofrequency tag before being used in the synthesis. The appropriate Kan size can be selected by one skilled in the art familiar with the Kan technology.

Further information on the lrori Kan technology can be obtained from Discovery Partners, Inc., at www.discoverypartners.com.

All of the reagents, amines and carboxylic acids necessary to carry out the syntheses described below are commercially available from widely available sources.

Kans **1** (12 Kans) containing indole resin (90 mg per Kan, 0.9 mmol/g) were suspended in anhydrous trimethylorthoformate (40 mL). The amine **2** (10 mmol, 10 eq) was added, and the reaction was shaken at ambient temperature for 16 hours. Sodium cyanoborohydride (1.3 g, 20 eq) was added and the reaction was shaken at ambient temperature for 1 hour. Aqueous acetic acid (3.2 mL, 8% v/v) was slowly added and the reaction was shaken at ambient temperature for 3 h. The MiniKans **3** were washed alternately with MeOH and DCM for four cycles and dried under vacuum.

Kans containing **3** from seven reactions (total 84 MiniKans) were suspended in anhydrous DMF (250 mL). 6-Chloronicotinic acid (11.2 g, 10 eq), diisopropylethylamine (25 mL, 20 eq) and HATU (26.2 g, 10 eq) were added and the reaction was shaken at ambient temperature for 24 hours. The Kans 5 were washed alternately with MeOH and DCM for four cycles and dried under vacuum.

The Kans containing **5** were suspended in anhydrous *N*-methylpyrrolidinone (250 mL). Piperazine **6** (12.0 g, 20 eq) and diisopropylethylamine (49 mL, 40 eq) were added and the reaction mixture was heated at 80°C for 48 h. The MiniKans containing 7 were washed alternately with MeOH and DCM for 4 cycles and dried under vacuum.

After sorting, Kans containing **7** (12 Kans) were suspended in anhydrous DMF (40 mL). The carboxylic acid **8** (10 mmol, 10 eq), diisopropylethylamine (3.5 mL, 20 eq) and HATU (3.8 g, 10 eq) were added and the reaction was shaken at ambient temperature for 24 hours. The Kans containing **9** were then washed alternately with MeOH and DCM for five cycles and dried under vacuum.

Kans containing **9** (3 Kans) were treated with 20 % TFA/DCM (9 mL) at ambient temperature for 2 hour. Pyridylpiperazine **10** was obtained and purified by HPLC.

This solid phase approach is used to prepare compounds of this invention. Examples of amine and carboxylic acids that can be used in this solid phase approach to produce compounds of the invention are set forth below in Tables 1 and 2.

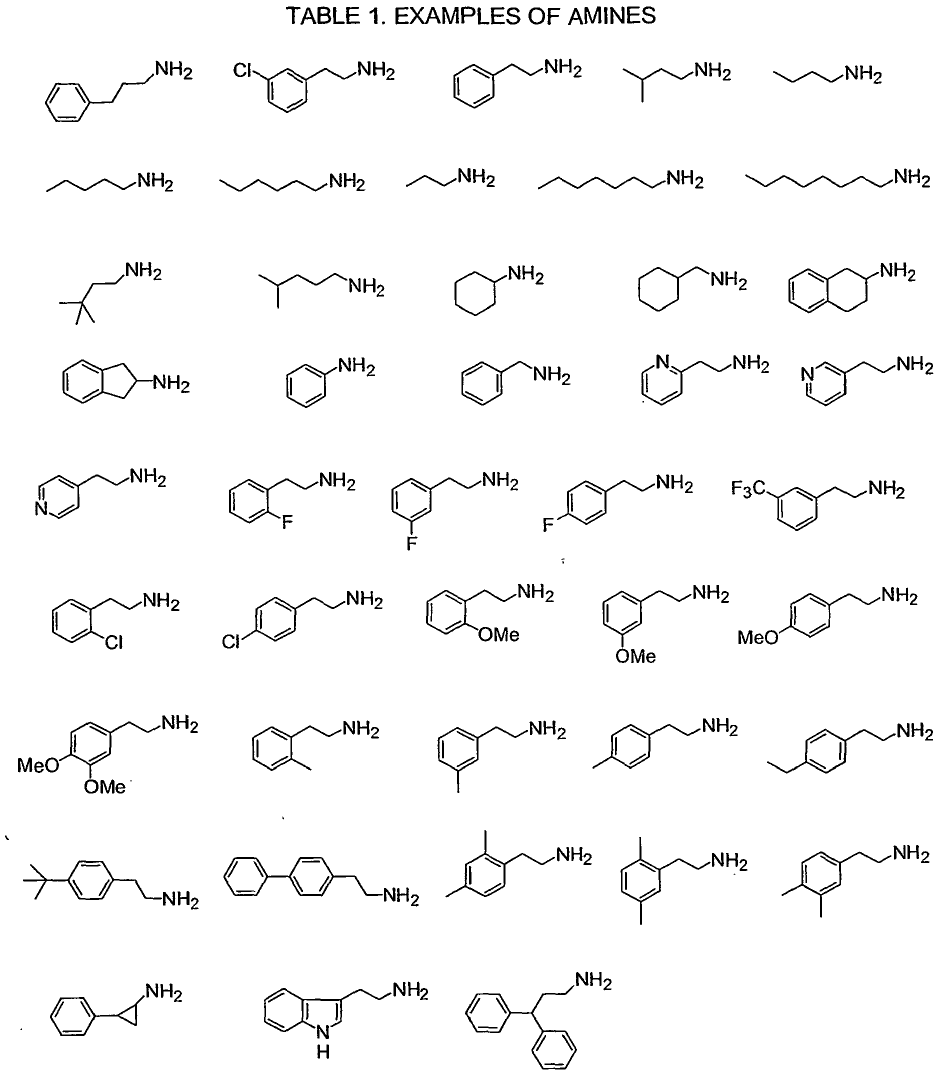

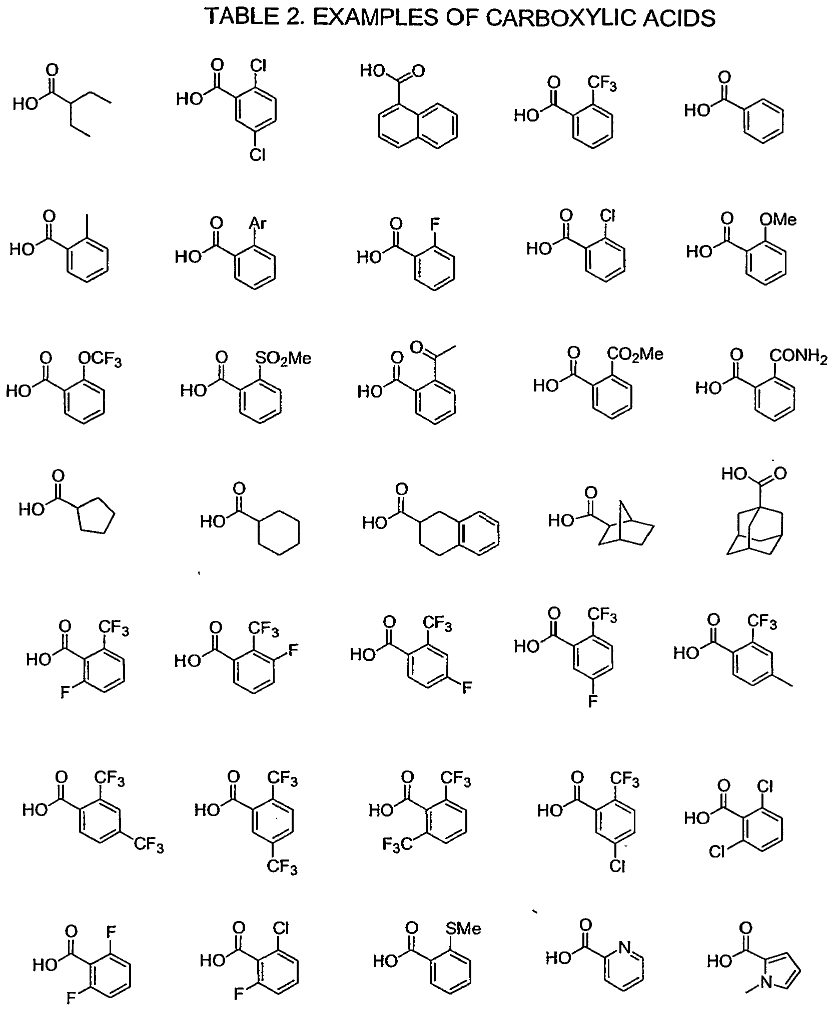

An alternative synthetic scheme for the preparation of the compounds of the invention, as set forth below in Reaction Scheme 2, includes a convergent solution-phase approach. For example, the acyl chloride **12** can be prepared from the corresponding acid **11** by reacting with thionyl chloride under conditions known to one skilled in the art or can be purchased from a commercial source, such as Aldrich Chemical Co. Reaction of **12** with piperazine **13** under conditions known to one skilled in the art generates the amide bond of **14,** in which the protecting group can be removed in an acidic media under conditions known to one skilled in the art, such as trifluoroacetic acid in dichloromethane, to give compound **15.** On the other hand, 6-chloronicotinic acid (**16**) is converted to its chloride **17** under conditions known to one skilled in the art, which is then coupled with amine **18** to afford nicotinamide **19.** Coupling of **15** with **19** under conditions known to one skilled in the art gives the final product **20.**

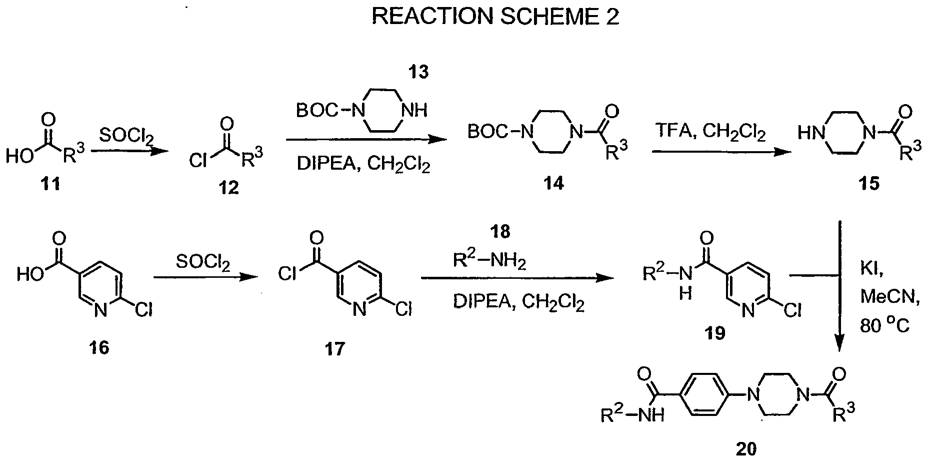

Alternatively, the compounds of this invention can be prepared according to the synthetic approach set forth in Reaction Scheme 3 below. 6-Chloronicotinic methyl ester (**21**) couples with piperazine **22** under conditions known to one skilled in the art to givecompound **23** followed by reaction with acyl chloride **12** under conditions known to one skilled in the art to generate compound **24.** Hydrolysis of methyl ester by using a base under conditions known to one skilled in the art, such as lithium hydroxide, gives acid **25,** which can react with amine **18** under conditions known to one skilled in the art to afford the final product **26.**

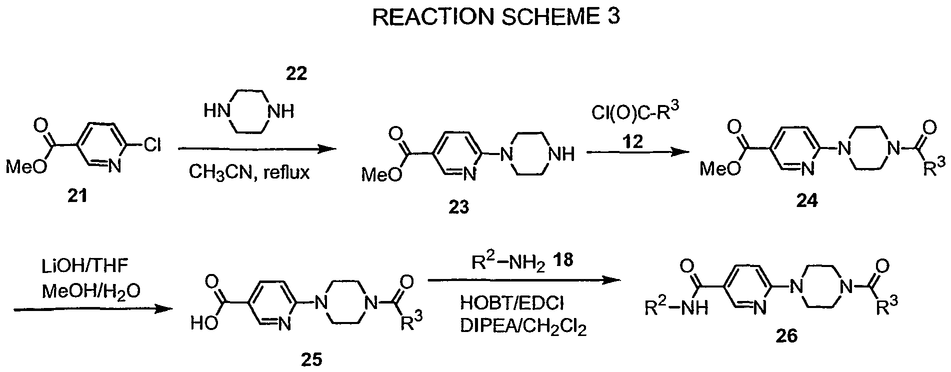

Alternatively, compounds of the invention where R⁴ is -OCH₃ or -OH can be prepared according to the synthetic approach set forth in Reaction Scheme 4 below. 2,6-Dichloronicotinic acid **27** is converted to its ester **28** under conditions known to one skilled in the art, in which the 2-position chloro group can then be substituted with a methoxy group under conditions known to one skilled in the art to generate compound **29.** Coupling of **29** with piperazine under conditions known to one skilled in the art gives compound **30,** which can then react with acyl chloride **12** under conditions known to one skilled in the art to generate compound **31.** After hydrolysis, acid **32** reacts with amine **18** under conditions known to one skilled in the art to produce nicotinamide **33.** The methyl group in compound **33** can be removed by using a Lewis acid under conditions known to one skilled in the art, such as *in situ* generated trimethylsilyl iodide, to afford compound **34.**

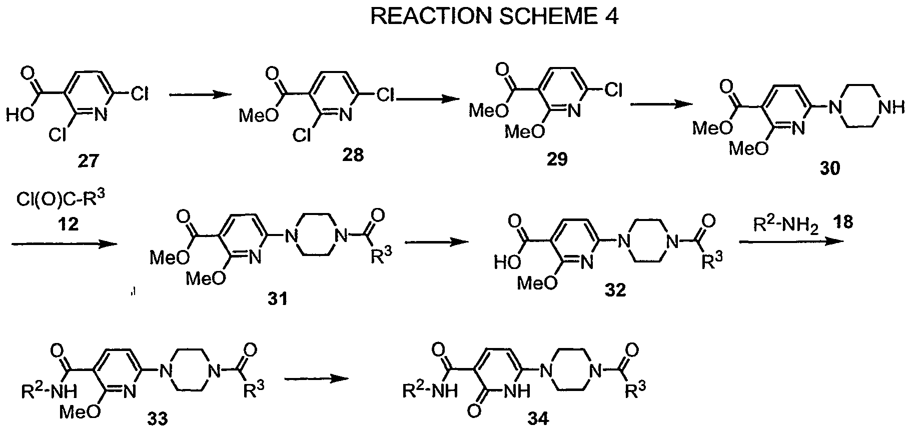

All reagents and reaction conditions employed in these syntheses are known to those skilled in the art and are available from ordinary commercial sources. Other compounds of the invention can be prepared according to the methods described above by one skilled in the art utilizing known starting materials and experimental parameters, or by the methods disclosed in the following experimentals. In addition, one skilled in the art can prepare the compounds of the invention by the methods disclosed in U.S. Patent No. 6,677,452.

All compounds of the invention as prepared above and below which exist in free base or acid form may be converted to their pharmaceutically acceptable salt by treatment with the appropriate inorganic or organic base or acid. Salts of the compounds prepared herein may be converted to their free base or acid by standard techniques.

The following specific examples are provided as a guide to assist in the practice of the invention.

### EXAMPLE 1

### N-(2-Cyclopropylethyl)-6-[4-(5-fluoro-2-trifluoromethylbenzoyl)piperazin-1-yl]nicotinamide

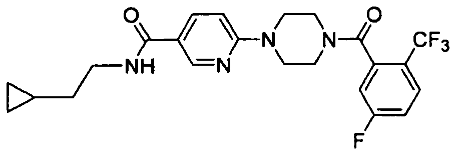

To a solution of 6-[4-(5-fluoro-2-trifluoromethylbenzoyl)piperazin-1-yl]nicotinic acid (0.100 g, 0.25 mmol), 1-hydroxy benzotriazole hydrate (0.041 g, 0.30 mmol) in dichloromethane (20 mL) were added 1-(3-dimethylaminopropyl)-3-ethyl carbodiimide (0.047 g, 0.30 mmol) and ethyldiisobutylamine (0.065 g, 0.50 mmol). The resulted mixture was stirred for 15 minutes at ambient temperature, followed by the addition of a solution of 2-cyclopropylethyl amine (0.021 g, 0.25 mmol) in 5 mL of dichloromethane. The reaction mixture was stirred at ambient temperature for 24 hours, and then diluted with dichloromethane (50 mL). The organic phase was washed with water, followed by brine solution, dried over anhydrous Na₂SO₄ and concentrated *in vacuo* to generate a white solid. This was purified by column chromatography (2:1, ethyl acetate: hexane) to obtain title compound as a white solid (0.087 g, 75% yield); ¹H NMR (CDCl₃, 300 MHz) δ 8.52 (d, J = 2.4 Hz, 1H), 7.92 (dd, J = 2.4, 6.6 Hz, 1H), 7.72 (d, J = 7.8 Hz, 1H), 7.61-7.5 (m, 2H), 7.34 (d, J = 7.5 Hz, 1H), 6.62 (d, J = 9 Hz, 1H), 6.14 (t, J = 5.1 Hz, 1H), 3.98-3.92 (m, 1H), 3.87-3.78 (m, 1H), 3.75-3.66 (m, 2H), 3.6-3.47 (m, 4H), 3.29-3.25 (m, 2H), 1.52-1.45 (m, 2H), 0.72-0.65 (m, 1H), 0.49-0.43 (m, 2H), 0.1-0.04 (m, 2H). ¹³C NMR (CDCl₃, 75 MHz) δ 167.5, 165.7, 159.7, 147.1, 136.9, 134.4, 134.3, 132.3, 129.3, 127.4, 126.74, 126.68, 126.62, 120.0, 105.8, 46.5, 44.4, 41.3, 40.1, 34.3, 8.6, 4.1 (2 peaks). MS (ES+) *m*/*z* 447 (M+1).

### EXAMPLE 2

### N-(2-Cyclobutylethyl)-6-[4-(5-fluoro-2-trifluoromethylbenzoyl)piperazin-1-yl]nicotinamide

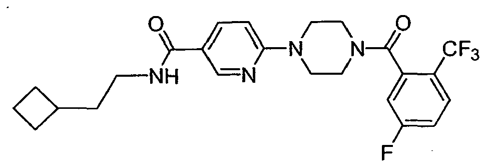

Following the procedure set forth above in Example 1, the title compound was obtained as a white solid (0.053g, 44% yield); ¹H NMR (CDCl₃, 300 MHz): δ 8.51-8.50 (m, 1H), 7.94-7.90 (m, 1H), 7.75-7.7 (m, 1H), 7.21-7.19 (m, 1H), 7.07-7.04 (m, 1H), 6.63 (d, *J* = 9 Hz, 1H), 5.94 (t, *J* = 5.5 Hz, 1H), 3.96-3.89 (m, 1H), 3.87-3.77 (m, 1H), 3.75-3.68 (m, 2H), 3.65-3.58 (m, 2H), 3.37-3.27 (m, 4H), 2.38-2.28 (m, 1H), 2.12-1.98 (m, 2H), 1.94-1.73 (m, 3H), 1.7-1.58 (m, 3H). ¹³C NMR (CDCl₃, 75 MHz): δ 165.9, 165.6, 162.5, 159.5, 146.9, 137.1, 136.95, 129.6, 129.5, 129.48, 129.42, 125.0, 121.4, 120.1, 116.7, 116.4, 114.9, 114.6, 105.99, 46.5, 44.5, 41.4, 38.2, 36.5, 33.85, 28.3, 18.7. MS (ES+): *m*/*z* 479.1 (M+1)

### EXAMPLE 3

### N-(3-Cyclopropyl propyl)-6-[4-(5-fluoro-2-trifluoromethyl benzoyl)-piperazin-1-yl] nicotinamide

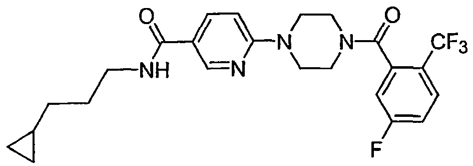

Following the procedure set forth above in Example 1, the title compound was obtained as a white solid (0.086 g, 77% yield); ¹H NMR (CDCl₃, 300 MHz) δ 8.53 (s, 1H), 7.94-7.9 (m, 1H), 7.74-7.7 (m, 1H), 7.21-7.18 (m, 1H), 7.07-7.03 (m, 1H), 6.61 (d, J = 8.7 Hz, 1H), 6.18 (t, *J* = 5.4 Hz, 1H), 3.97-3.58 (m, 6H), 3.46-3.39 (m, 2H), 3.29-3.26 (m, 2H), 1.72-1.63 (m, 2H), 1.28-1.21 (m, 2H), 0.70-0.61 (m, 1H), 0.43-0.37 (m, 2H), 0.01-0.04 (m, 2H). ¹³C NMR (CDCl₃, 75 MHz) δ 165.9, 165.7, 162.4, 159.5, 147.2, 136.9, 129.5, 129.5, 129.4, 129.35, 128.6, 124.95, 123.1, 122.7, 121.3, 120.1, 116.6, 116.3, 114.8, 114.5, 105.8, 46.4, 44.3, 41.3, 39.6, 31.9, 29.5, 10.4, 4.4. MS (ES+) m/z 479.0 (M+1).

### EXAMPLE 4

### 6-[4-(5-Fluoro-2-trifluoromethylbenzoyl)piperazin-1-yl]-N-(4-methylpentyl)-nicotinamide

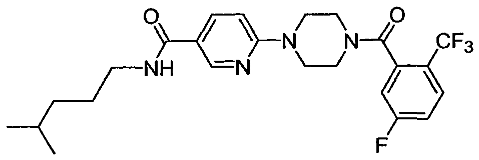

Following the procedure set forth above in Example 1, the title compound was obtained as a white solid (0.075g, 66% yield); ¹H NMR (CDCl₃, 300 MHz) δ 8.52 (s, 1H), 7.94-7.91 (m, 1H), 7.72-7.7 (m, 1H), 7.21-7.18 (m, 1H), 7.06-7.04 (m, 1H), 6.62 (d, *J* = 9 Hz, 1H), 6.02 (br. t, 1H), 3.98-3.55 (m, 6H), 3.43-3.34 (m, 2H), 3.3-3.26 (m, 2H), 1.62-1.48 (m, 3H), 1.25-1.18 (m, 2H), 0.86 (d, *J* = 6.6 Hz, 6H). ¹³C NMR (CDCl₃, 75 MHz) δ 165.9, 165.7, 162.5, 159.5, 147.1, 137.02, 129.6, 129.5, 129.4, 129.38, 128.6, 127.6, 124.5, 122.7, 121.4, 120.1, 117.7, 116.6, 116.4, 114.8, 114.5, 105.8, 46.4, 44.4, 41.3, 40.1, 36.0, 27.7, 27.5, 22.4. MS (ES+): *m*/*z* 481.1 (M+1).

### EXAMPLE 5

### N-(3,3-Dimethylbutyl)-6-[4-(5-fluoro-2-trifluoromethylbenzoyl)piperazin-1-yl]-nicotinamide

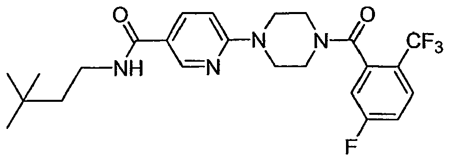

Following the procedure set forth above in Example 1, the title compound was obtained as a white solid (0.085 g, 76% yield); ¹H NMR (CDCl₃, 300 MHz) δ 8.52 (s, 1H), 7.93-7.89 (m, 1H), 7.75-7.7 (m, 1H), 7.24-7.18 (m, 1H), 7.06-7.03 (m, 1H), 6.61 (d, *J* = 9 Hz, 1H), 5.94 (br, 1H), 3.98-3.75 (m, 2H), 3.75-3.66 (m, 2H), 3.63-3.56 (m, 2H), 3.48-3.35 (m, 2H), 3.32-3.23 (m, 2H), 1.51-1.46 (m, 2H), 0.93 (s, 9H). ¹³C NMR (CDCl₃, 75 MHz) δ 165.9, 165.6, 162.5, 159.5, 147.2, 136.9, 129.6, 129.5, 129.4, 129.37, 128.6, 124.96, 123.2, 122.3, 120.1, 116.6, 116.3, 114.8, 114.5, 105.8, 46.4, 44.3, 43.1, 41.3, 36.5, 29.8, 29.3. MS (ES+) *m*/*z* 481.0 (M+1),

### EXAMPLE 6

### N-(2-Cyclopropylethyl)-6-[4-(2-trifluoromethyl-benzoyl)-piperazin-1-yl]-nicotinamide

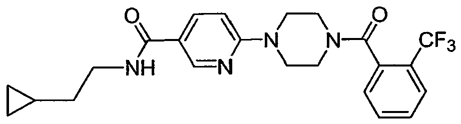

A mixture of 6-[4-(2-trifluoromethyl-benzoyl)-piperazin-1-yl]-nicotinic acid (280 mg, 0.738 mmol), diisopropylethylamine (0.19 mL, 1.1mmol), 1-hydroxybenzotriazole hydrate (130 mg, 0.96 mmol) and EDCI (0.19 mL, 1.1mmol) in dichloromethane (5 mL) was stirred for 15 min, 2-cyclopropylethylamine (70 mg, 0.82 mmol) was added. After stirring for 10 h at ambient temperature, the reaction mixture was diluted with dichloromethane (50 mL), washed with water, saturated NaHCO₃ and brine, dried (anhydrous Na₂SO₄) and concentrated. Purification via flash chromatography over silica gel(ethyl acetate) afforded *N*-(2-cyclopropyl-ethyl)-6-[4-(2-trifluoromethylbenzoyl)-piperazin-1-yl]-nicotinamide (264 mg, 80%). ¹HNMR:(400 MHz, CDCl₃) δ: 8.52 (d, *J* = 1.9, 1H), 7.94 (dd, *J* = 1.9, 8.7 Hz, 1H), 7.73 (d, *J* = 9.2Hz, 1H), 7.63(t, *J* = 8.5 Hz, 1H), 7.56 (t, *J* = 8.5 Hz, 1H), 7.37 (d, J= 8.7 Hz, 1H), 6.62 (d, *J*= 9.2 Hz, 1H), 6.14 (br, 1H), 4.02-3.27 (m, 10H), 1.52(q, *J* = 6.7 Hz, 2H), 0.78-0.70 (m, 1H), 0.53-0.48 (m, 2H), 0.13-0.08 (m, 2H). MS (ES+) m/z 447.2 (M+1).

### EXAMPLE 7

### N-(2-cyclopropylethyl)-2-methoxy-6-(4-(2-trifluoromethylbenzoyl)piperazin-1-yl]nicotinamide

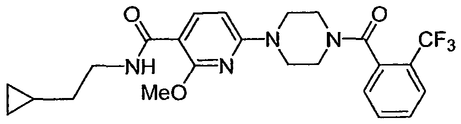

A. To a solution of 2,6-dichloronicotinic acid (5.0 g, 26 mmol) in MeOH (100 mL) was added SOCl₂ (0.1 mL). The reaction mixture was heated to reflux overnight. The solvent was removed by evaporation. The residue was dissolved in ethyl acetate and washed with saturated NaHCO₃ and brine; dried over anhydrous Na₂SO₄ and concentrated to afford 2,6-dichloronicotinic acid methyl ester (5.37 g, 100%); ¹HNMR:(300 MHz, CDCl₃) δ 8.13 (d, *J* = 8.1 Hz, 1H), 7.33 (d. *J* = 8.1 Hz, 1H), 3.94 (s, 3H).
B. A solution of 2,6-dichloronicotinic acid methyl ester (2.39 g, 11.6 mmol) and NaOMe (800 mg, 14.06 mmol) in THF (15 mL) was stirred at ambient temperature for 3 days. The reaction mixture was quenched by adding 10 mL of 10% NH₄Cl solution and extracted with ether. The combined organic layers was dried over anhydrous Na₂SO₄ and concentrated to afford an unseparable mixture (1.7 g), containing 6-chloro-2-methoxynicotinic acid methyl ester.
C. The above crude material was dissolved in CH₃CN (30 ml) followed by the addition of piperazine (3.0 g, 34.8 mmol). The reaction mixture was heated at reflux overnight. Acetonitrile was removed by evaporation to afford crude 2-methoxy-6-piperazin-1-yl-nicotinic acid methyl ester (1. 82 g). To the solution of the residue in dichloromethane (20 mL) was added Et₃N (1 mL, 7.17 mmol) and cooled to 0°C. 2-Trifluoromethylbenzoic chloride (0.6 mL, 4.05 mmol) was added. The reaction mixture was stirred at ambient temperature for 2 hours, diluted with ethyl acetate and washed , with water; dried over anhydrous Na₂SO₄, and concentrated. The residue was purified over silica gel chromatography to give 2-methoxy-6-(4-(2-trifluoromethylbenzoyl)piperazin-1-yl]nicotinic acid methyl ester (1.46 g, 30% over 3 steps); ¹HNMR (300 MHz, CDCl₃) δ 8.04 (d, *J* = 8.7 Hz, 1H), 7.72 (d, *J* = 7.8 Hz, 1H), 7.61 (dd, *J* = 7.5, 7.8 Hz, 1H), 7.53 (dd, *J* = 7.5, 7.8 Hz, 1H), 7.34 (d, *J* = 7.8 Hz, 1H), 6.14 (d, *J* = 8.7 HZ, 1H), 4.00-3.59 (m, 12H), 3.29-3.26 (m, 2H). MS (ES⁺) m/z 446.0 (M+Na).
D. Lithium hydroxide hydrate (476 mg, 11.34 mmol) was added to a solution of 2-methoxy-6- (4-(2-trifluoromethylbenzoyl) piperazin-1-yl] nicotinic acid methyl ester (1.2 g, 2.83 mmol) in THF (30 mL) and water (10 mL). The reaction mixture was heated to reflux for 8 hours. THF was removed by evaporation. The residue was neutralized with 5% HCl solution and extracted with ethyl acetate. The combined organic phase was washed with water and brine; dried over anhydrous Na₂SO₄ and concentrated to give 2-methoxy-6-(4-(2-trifluoromethylbenzoyl)piperazin-1-yl]nicotinic acid (1.08 g, 96%); ¹HNMR (300 MHz, CDCl₃) δ 8.19 (d, *J* = 8.7 Hz, 1H), 7.73 (d, *J* = 7.8 Hz, 1H), 7.62 (dd, *J* = 7.5, 7.8 Hz, 1H), 7.53 (dd, *J* = 7.5, 7.8 Hz, 1H), 7.34 (d, *J* = 7.8 Hz, 1H), 6.29 (d, *J* = 8.7 Hz, 1H), 4.13-3.57 (m, 9H), 3.36-3.28 (m, 2H); MS (ES⁺) m/z 432.1 (M+1).
E. To a solution of 2-methoxy-6-(4-(2-trifluoromethylbenzoyl)piperazin-1-yl]nicotinic acid (440 mg, 1.07 mmol) in dichloromethane (20 mL) was added diisopropylethylamine (0.5 mL, 2.8 mmol), 1-hydroxybenzotriazole hydrate (215 mg, 1.5 mmol) and EDCI (0.28 mL, 1.5 mmol). The resulting mixture was stirred for 15 min; and then cyclopropylethylamine (110 mg, 1.2 mmol) was added. After stirring for 20 hours, the reaction mixture was diluted with dichloromethane (100 mL), washed with water, brine, dried over anhydrous Na₂SO₄ and concentrated. Purification via flash chromatography over silica gel (ethyl acetate) and recrystallization from ethyl acetate and hexanes gave *N*-(2-cyclopropylethyl)-2-methoxy-6-(4-(2-trifluoromethylbenzoyl)piperazin-1-yl]nicotinamide (340 mg, 67%); m.p. 49-51°C. ¹H NMR(300 MHz, CDCl₃) δ 8.32 (d, *J* = 8.7 Hz, 1H), 7.80 (t, *J* = 4.8 Hz, 1H), 7.72(d, *J* = 7.8 Hz, 1H), 7.63-7.50(m, 2H), 7.34(d, *J* = 7.5 Hz, 1H), 6.25 (d, *J* = 8.7 Hz, 1H), 3.96-3.80(m, 4H), 3.74-3.66(m, 2H), 3.56-3.47(m, 4H), 3.29-3.26(m, 2H), 1.49 (q, *J* = 6.0 Hz, 2H), 0.76-0.66(m, 1H), 0.49-0.43(m, 2H), 0.12-0.07(m, 2H); ¹³C NMR (CDCl₃, 75 MHz) δ 167.5, 164.3, 160.0, 158.1, 143.5, 134.5, 132.3, 129.3, 127.2, 126.8, 104.9, 99.1, 53.4, 46.5, 44.5, 44.4, 41.3, 39.8, 34.4, 8.9, 4.1. MS (ES⁺) m/z 477.4 (M+1).

### EXAMPLE 8

### 2-Oxo-6-[4-(2-trifluoromethyl-benzoyl)-piperazin-1-yl]-1,2-dihydro-pyridine-3-carboxylic acid (2-cyclopropylethyl)amide

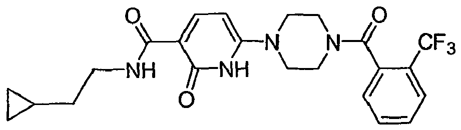

Chlorotrimethylsilane (0.28 mL, 2.2 mmol) was added to a mixture of *N*-(2-cyclopropylethyl)-2-methoxy-6-(4-(2-trifluoromethylbenzoyl)piperazin-1-yl]nicotinamide (270 mg, 0.56 mmol) and sodium iodide (332 mg, 2.2 mmol) in CH₃CN (5 mL) and 1 drop water at 0°C. The reaction mixture was stirred at ambient temperature overnight. The reaction was quenched by addition of 5 mL MeOH at 0°C and concentrated. The residue was purified over silica gel chromatography (ethyl acetate) and recrystalized from ethyl acetate and hexanes to give *N*-(2-cyclopropylethyl)-2-hydroxy-6-(4-(2-trifluoromethylbenzoyl)piperazin-1-yl]nicotinamide (79 mg, 30%); ¹H NMR (300 MHz, CDCl₃) δ 8.02(d, *J* = 8.7 Hz, 1H), 7.80(t, *J* = 7.5 Hz, 1H), 7.64(t, *J* = 7.5 Hz, 1H), 7.50(d, *J* = 7.5 Hz, 1H), 5.92(br, 1H), 3.75-3.05(m, 10H), 1.34(q, *J* = 1.34 Hz, 2H), 0.71-0.57(m, 1H), 0.44-0.28(m, 2H), 0.08-0.03 (m, 2H); MS (ES⁺) m/z 463.1 (M+1).

### EXAMPLE 9

The following representative compounds of the invention can be prepared according to the synthetic approaches set forth above in Reaction Schemes 1 and 2, or in a similar manner to the methods disclosed herein by utilizing the appropriately substituted starting materials, or by methods known to one skiled in the art:
6-[4-(2-Ethylbutyryl)piperazin-1-yl]-n-(3-phenylpropyl)nicotinamide; 423.3 (M+1);
6-[4-(4-Methyl-hexanoyl)piperazin-1-yl]-*n*-(3-phenylpropyl)nicotinamide; 423.2 (M+1);
6-[4-(2-Ethylbutyryl)-3-methylpiperazin-1-yl]-*n*-(3-phenylpropyl)nicotinamide; 437.3 (M+1);
6-[4-(2-Phenylcyclopropanecarbonyl)piperazin-1-yl]-*n*-(3-phenylpropyl) nicotinamide; 469.1 (M+1);
6-[4-(3-Cyclohexylpropionyl)piperazin-1-yl]-*n*-(3-methylbutyl)nicotinamide; 415.3 (M+1);
6-[4-(2-Cyclohexylacetyl)piperazin-1-yl]-*n*-hexyl-nicotinamide; 415.2 (M+1);
*N*-Butyl-6-[4-(3-cyclohexylpropionyl)piperazin-1-yl]nicotinamide; 401.2 (M+1);
6-[4-(2-Cyclohexylacetyl)piperazin-1-yl]-*n*-pentyl-nicotinamide; 401.2 (M+1);
6-[4-(3-Cyclohexylpropionyl)piperazin-1-yl]-*n*-pentyl-nicotinamide; 415.2 (M+1);
*N*-[2-(3-Chlorophenyl)ethyl]-6-[4-(3-cyclohexylpropionyl)piperazin-1-yl]nicotinamide;483.1 (M+1);
*N*-[2-(3-Chlorophenyl)ethyl]-6-[4-(2-cyclohexylacetyl)piperazin-1-yl]nicotinamide469.1 (M+1);
*N*-Butyl-6-[4-(2-mercapto-benzoyl)piperazin-1-yl]nicotinamide; 447.2 (M+1);
*N*-(3-Methylbutyl)-6-[4-(2-*o*-tolylacetyl)piperazin-1-yl]nicotinamide; 409.2 (M+1);
6-{4-[2-(2,4-Dimethylphenyl)-acetyl]-piperazin-1-yl}-*N*-(3-methylbutyl)nicotinamide; 409.2 (M+1);
6-[4-(2-Bromo-benzoyl)piperazin-1-yl]-*n*-(3-ethoxy-propyl)nicotinamide; 475.1 (M+1);
6-{4-[2-(2-Chloro-6-fluorophenyl)-acetyl]-piperazin-1-yl}-*N*-(3-methylbutyl)nicotinamide; 447.1 (M+1);
*N*-(3-Methylbutyl)-6-[4-(2-trifluoromethylbenzoyl)piperazin-1-yl]nicotinamide; 449.0 (M+1);
*N*-(3-Methylbutyl)-6-[4-(2-trifluoromethylbenzoyl)piperazin-1-yl]nicotinamide hydrochloride; 448.7 (M-HCl);
*N*-(3-Methylbutyl)-4-trifluoromethyl-6-[4-(2-trifluoromethylbenzoyl)piperazin-1-yl]nicotinamide; 517.3 (M+1);
2-Chloro-5-fluoro-*N*-(3-methylbutyl)-6-[4-(2-trifluoromethylbenzoyl)piperazin-1-yl]nicotinamide; 501.1 (M+1);
2-Chloro-*N*-(3-methylbutyl)-6-[4-(2-trifluoromethylbenzoyl)piperazin-1-yl]nicotinamide; 483.1 (M+1);
*N*-(3-Methylbutyl)-6-[3-oxo-4-(2-trifluoromethylbenzyl)piperazin-1-yl]nicotinamide; 449.2 (M+1);
6-[4-(2,5-Dichloro-benzoyl)piperazin-1-yl]-*n*-(3-imidazol-1-ylpropyl)nicotinamide;487.0 (M+1);
6-[4-(2,4-Dichloro-benzoyl)piperazin-1-yl]-*n*-(3-imidazol-1 - ylpropyl)nicotinamide;487.1 (M+1);
6-[4-(2-Bromo-5-methoxy-benzoyl)piperazin-1-yl]-*n*-(3-phenylpropyl)nicotinamide; 539.0 (M+1);
6-[4-(2-Bromo-benzoyl)piperazin-1-yl]-*n*-[2-(3H-imidazol-4-yl)ethyl]nicotinamide;483.0 (M+1);
*N*-[2-(3-Chlorophenyl)ethyl]-6-[4-(2,4-dichloro-benzoyl)piperazin-1-yl]nicotinamide; 519.1 (M+3);
6-[2-Oxo-4-(2-trifluoromethylbenzoyl)piperazin-1-yl]-*n*-(3-phenylpropyl)nicotinamide; 511.2 (M+1);
*N*-(3-Methylbutyl)-6-[2-oxo-4-(2-trifluoromethylbenzoyl)piperazin-1-yl]nicotinamide; 463.1 (M+1);
6-[4-(3-Methyl-3H-114-thiophene-2-carbonyl)piperazin-1-yl]-*n*-pentyl-nicotinamide; 401.1 (M+1);
*N*-[2-(3-Chlorophenyl)ethyl]-6-[4-(3-methyl-thiophene-2-carbonyl)piperazin-1-yl]nicotinamide; 469.1 (M+1);
6-{4-[2-(4-Chlorophenyl)propionyl]-piperazin-1-yl}-*N*-(3-methylbutyl)nicotinamide; 443.0 (M+1);
6-[4-(2-Phenylbutyryl)piperazin-1-yl]-*n*-(3-phenylpropyl)nicotinamide;471.3 (M+1);
*N*-Pentyl-6-[4-(2-phenylcyclopropanecarbonyl)piperazin-1-yl]nicotinamide; 421.2 (M+1);
*N*-(3-Methylbutyl)-6-[4-(naphthalene-1-carbonyl)piperazin-1-yl]nicotinamide; 431.5 (M+1);
*N*-(3-Methylbutyl)-6-[4-(naphthalene-1-carbonyl)piperazin-1-yl]nicotinamide; 431.2 (M+1);
*N*-[2-(3-Chlorophenyl)ethyl]-6-[4-(2-phenylcyclopropanecarbonyl)piperazin-1-yl]nicotinamide; 489.2 (M+1);
6-[5-(2-Ethylbutyryl)-2,5-diaza-bicyclo[2.2.1]hept-2-yl]-*n*-(3-phenylpropyl)nicotinamide; 435.3 (M+1);
6-[4-(2-Ethylbutyl)piperazin-1-yl]-*n*-(3-phenylpropyl)nicotinamide; 409.3 (M+1);
6-[4-(Butane-1-sulfonyl)piperazin-1-yl]-*n*-(3-phenylpropyl)nicotinamide; 444.9 (M+1);
4-[5-(3-Phenylpropylcarbamoyl)pyridin-2-yl]-piperazine-1-carboxylic acid butylamide; 424.3 (M+1);
*N*-(3-Ethoxy-propyl)-6-[4-(2-ethylbutyryl)-piperazin-1-yl]-nicotinamide;
*N*-(3-Ethoxy-propyl)-6-(4-pentanoyl-piperazin-1-yl)-nicotinamide;
*N*-(3-Ethoxy-propyl)-6-[4-(3-methyl-pentanoyl)-piperazin-1-yl]nicotinamide
*N*-(3-Butoxy-propyl)-6-(4-pentanoyl-piperazin-1-yl)-nicotinamide
*N*-(3-Methyl-butyl)-6-[4-(3-methyl-pentanoyl)-piperazin-1-yl]-nicotinamide
6-[4-(3-Methyl-pentanoyl)-piperazin-1-yl]-*N*-pentyl-nicotinamide;
*N*-Butyl-6-[4-(3-methyl-pentanoyl)-piperazin-1-yl]-nicotinamide
*N*-Butyl-6-[4-(2-ethylbutyryl)-piperazin-1-yl]-nicotinamide
*N*-(3-Methyl-butyl)-6-(4-pentanoyl-piperazin-1-yl)-nicotinamide;
*N*-(2-Ethylsulfanyl-ethyl)-6-(4-pentanoyl-piperazin-1-yl)-nicotinamide;
*N*-(3-Methoxy-propyl)-6-[4-(3-methyl-pentanoyl)-piperazin-1-yl]-nicotinamide;
6-(4-Pentanoyl-piperazin-1-yl)-*N*-pentyl-nicotinamide;
*N*-Hexy1-6-[4-(3-methyl-pentanoyl)-piperazin-1-yl]-nicotinamide;
*N*-(1,3-Dimethylbutyl)-6-[4-(3-methyl-pentanoyl)-piperazin-1-yl]-nicotinamide;
*N*-(1-Methyl-butyl)-6-(4-pentanoyl-piperazin-1-yl)-nicotinamide;
*N*-(3-Dimethylamino-propyl)-6-[4-(2-ethylbutyryl)-piperazin-1-yl]-nicotinamide;
*N*-(3-Methoxy-propyl)-6-(4-pentanoyl-piperazin-1-yl)-nicotinamide;
*N*-(1,3-Dimethylbutyl)-6-(4-pentanoyl-piperazin-1-yl)-nicotinamide;
*N*-(2-Methyl-butyl)-6-(4-pentanoyl-piperazin-1-yl)-nicotinamide;
*N*-(3-Butoxy-propyl)-6-[4-(2-ethylbutyryl)-piperazin-1-yl]-nicotinamide;
6-[4-(2-Ethylbutyryl)-piperazin-1-yl]-*N*-(3-methylbutyl)-nicotinamide;
*N*-Butyl-6-(4-pentanoyl-piperazin-1-yl)-nicotinamide;
6-[4-(2-Ethylbutyryl)-piperazin-1-yl]-*N*-(2-methylbutyl)-nicotinamide;
*N*-(3-Isopropoxy-propyl)-6-(4-pentanoyl-piperazin-1-yl)-nicotinamide;
*N*-(2-Ethylsulfanyl-ethyl)-6-[4-(3-methyl-pentanoyl)-piperazin-1-yl]-nicotinamide;
6-[4-(2-Ethylbutyryl)-piperazin-1-yl]-*N*-(2-ethylsulfanyl-ethyl)-nicotinamide;
6-[4-(2-Ethylbutyryl)-piperazin-1-yl]-*N*-hexyl-nicotinamide;
6-[4-(2-Ethylbutyryl)-piperazin-1-yl]-*N*-(1-methylbutyl)-nicotinamide;
*N*-(2-Methyl-butyl)-6-[4-(3-methyl-pentanoylrpiperazin-1-yl]-nicotiriamide;
*N*-(1,3-Dimethylbutyl)-6-[4-(2-ethylbutyryl)-piperazin-1-yl]-nicotinamide;
6-[4-(2-Ethylbutyryl)-piperazin-1-yl]-*N*-pentyl-nicotinamide;
*N*-(3-Isopropoxy-propyl)-6-[4-(3-methyl-pentanoyl)-piperazin-1-yl]-nicotinamide;
*N*-(3-Dimethylamino-propyl)-6-[4-(3-methyl-pentanoyl)-piperazin-1-yl]-nicotinamide;
6-[4-(2-Ethylbutyryl)-piperazin-1-yl]-*N*-(3-methoxy-propyl)-nicotinamide;
6-[4-(2-Ethylbutyryl)-piperazin-1-yl]-*N*-(3-isopropoxy-propylynicotinamide;
*N*-(3-Butoxy-propyl)-6-[4-(3-methyl-pentanoyl)-piperazin-1-yl]-nicotinamide;
*N*-(1-Methyl-butyl)-6-[4-(3-methyl-pentanoyl)-piperazin-1-yl]-nicotinamide;
*N*-Hexyl-6-(4-pentanoyl-piperazin-1-yl)-nicotinamide;

*N*-(3-Dimethylamino-propyl)-6-(4-pentanoyl-piperazin-1-yl)-nicotinamide;
*N*-[2-(3-Chlorophenyl)-1-methylethyl]-6-[4-(2-ethylbutyryl)-piperazin-1-yl]-nicotinamide
*N*-[2-(3-Chlorophenyl)-ethyl]-6-[4-(3-methyl-pentanoyl)-piperazin-1-yl]-nicotinamide;
*N*-[2-(3-Chlorophenyl)-ethyl]-6-[4-(2-ethylbutyryl)-piperazin-1-yl]-nicotinamide;
6-(4-Pentanoyl-piperazin-1-yl)-*N*-(4-phenyl-butyl)-nicotinamide;
*N*-[2-(3-Chlorophenyl)-ethyl]-6-(4-pentanoyl-piperazin-1-yl)-nicotinamide;
6-[4-(2-Ethylbutyryl)-piperazin-1-yl]-*N*-phenethyl-nicotinamide;
6-[4-(3-Methyl-pentanoyl)-piperazin-1-yl]-*N*-phenethyl-nicotinamide;
6-(4-Pentanoyl-piperazin-1-yl)-*N*-(3-phenyl-propyl)nicotinamide;
*N*-(1-Methyl-3-phenyl-propyl)-6-(4-pentanoyl-piperazin-1-yl)-nicotinamide;
6-[4-(3-Methyl-pentanoyl)-piperazin-1-yl]-*N*-(tetrahydro-furan-2-ylmethyl)-nicotinamide;
6-[4-(2-Ethylbutyryl)-piperazin-1-yl]-*N*-(tetrahydro-furan-2-ylmethyl)-nicotinamide;
6-(4-Pentanoyl-piperazin-1-yl)-*N*-(tetrahydro-furan-2-ylmethyl)-nicotinamide;
6-[4-(2-Ethylbutyryl)-piperazin-1-yl]-*N*-(4-phenyl-butyl)-nicotinamide;
*N*-(3-Imidazol-1-yl-propyl)-6-(4-pentanoyl-piperazin-1-yl)-nicotinamide;
6-[4-(2-Ethylbutyryl)-piperazin-1-yl]-*N*-(3-imidazol-1-yl-propyl)-nicotinamide;
*N*-[2-(3H-lmidazol-4-yl)-ethyl]-6-(4-pentanoyl-piperazin-1-yl)-nicotinamide;
*N*-[2-(3H-Imidazol-4-yl)-ethyl]-6-[4-(3-methyl-pentanoyl)-piperazin-1-yl]-nicotinamide;
6-(4-Pentanoyl-piperazin-1-yl)-*N*-phenethyl-nicotinamide;
6-[4-(2-Ethylbutyryl)-piperazin-1-yl]-*N*-[2-(3H-imidazol-4-yl)-ethyl]-nicotinamide;
*N*-(3-imidazol-1-yl-propyl)-6-[4-(3-methyl-pentanoylypiperazin-1-yl]-nicotinamide;
6-[4-(2-Cyclohexyl-acetyl)-piperazin-1-yl]-*N*-(3-ethoxy-propyl)-nicotinamide;
*N*-Butyl-6-[4-(2-cyclohexyl-acetyl)-piperazin-1-yl]-nicotinamide;
6-[4-(3-Cyclohexyl-propionyl)-piperazin-1-yl]-*N*-(3-methoxy-propyl)-nicotinamide;
*N*-Pentyl-6-[4-(3-trifluoromethyl-benzoyl)-piperazin-1-yl]-nicotinamide;
6-[4-(2-Cyclohexyl-acetyl)-piperazin-1-yl]-*N*-(2-methylbutyl)-nicotinamide;
6-[4-(3-Cyclohexyl-propionyl)-piperazin-1-yl]-*N*-(3-isopropoxy-propyl)-nicotinamide;
6-[4-(2-Cyclohexyl-acetyl)-piperazin-1-yl]-*N*-(1,3-dimethylbutyl)-nicotinamide;
6-[4-(2-Cyclohexyl-acetyl)-piperazin-1-yl]-*N*-(1-methylbutyl)-nicotinamide;
6-[4-(2-Cyclohexyl-acetyl)-piperazin-1-yl]-*N*-(3-methylbutyl)-nicotinamide;
6-[4-(2-Cyclohexyl-acetyl)-piperazin-1-yl]-*N*-(2-ethylsulfanyl-ethyl)-nicotinamide;
*N*-(3-Butoxy-propyl)-6-[4-(2-cyclohexyl-acetyl)-piperazin-1-yl]-nicotinamide;
6-[4-(3-Cyclohexyl-propionyl)-piperazin-1-yl]-*N*-(2-ethylsulfanyl-ethyl)-nicotinamide;
6-[4-(2-Cyclohexyl-acetyl)-piperazin-1-yl]-*N*-(3-methoxy-propyl)-nicotinamide;
6-[4-(3-Cyclohexyl-propionyl)-piperazin-1-yl]-*N*-(1-methylbutyl)-nicotinamide;
6-[4-(2-Cyclohexyl-acetyl)-piperazin-1-yl]-*N*-(3-isopropoxy-propyl)-nicotinamide;
6-[4-(3-Cyclohexyl-propionyl)-piperazin-1-yl]-*N*-(3-ethoxy-propyl)-nicotinamide;
6-[4-(3-Cyclohexyl-propionyl)-piperazin-1-yl]-*N*-(1,3-dimethylbutyly nicotinamide;
6-[4-(3-Cyclohexyl-propionyl)-piperazin-1-yl]-*N*-(2-methylbutyl)-nicotinamide;
6-[4-(3-Cyclohexyl-propionyl)-piperazin-1-yl]-*N*-hexyl-nicotinamide;
6-[4-(3-Cyclohexyf-propionyl)-piperazin-1-yl]-*N*-(3-dimethylamino-propyl)-nicotinamide;
*N*-(3-Ethoxy-propyl)-6-[4-(2-phenyl-cyclopropanecarbonyl)-piperazin-1-yl]-nicotinamide
*N*-(2-Ethylsulfanyl-ethyl)-6-[4-(2-phenyl-cyclopropanecarbonyl)-piperazin-1-yl]-nicotinamide;
*N*-(1,3-Dimethylbutyl)-6-[4-(2-phenyl-cyclopropanecarbonyl)-piperazin-1-yl]-nicotinamide;
*N*-(3-Methyl-butyl)-6-[4-(2-phenyl-cyclopropanecarbonyl)-piperazin-1-yl]-nicotinamide;
*N*-(3-Methoxy-propyl)-6-[4-(2-phenyl-cyclopropanecarbonyl)-piperazin-1-yl]-nicotinamide;
*N*-(3-Butoxy-propyl)-6-[4-(2-phenyl-cyclopropanecarbonyl)-piperazin-1-yl]-nicotinamide;
*N*-(3-Dimethylamino-propyl)-6-[4-(2-phenyl-cyclopropanecarbonyl)-piperazin-1-yl]-nicotinamide;
*N*-(3-Isopropoxy-propyl)-6-[4-(2-phenyl-cyclopropanecarbonyl)-piperazin-1-yl]-nicotinamide;
*N*-(1-Methyl-butyl)-6-[4-(2-phenyl-cyclopropanecarbonyl)-piperazin-1-yl]-nicotinamide;
*N*-Butyl-6-[4-(2-phenyl-cyclopropanecarbonyl)-piperazin-1-yl]-nicotinamide;
*N*-Hexyl-6-[4-(2-phenyl-cyclopropanecarbonyl)-piperazin-1-yl]-nicotinamide;
*N*-(2-Methyl-butyl)-6-[4-(2-phenyl-cyclopropanecarbonyl)-piperazin-1-yl]-nicotinamide;
6-[4-(2-Cyclohexyl-acetyl)-piperazin-1-yl]-*N*-(3-dimethylamino-propyl)-nicotinamide;
6-[4-(2-Phenyl-cyclopropanecarbonyl)-piperazin-1-yl]-*N*-(3-phenyl-propyl)-nicotinamide;
*N*-(4-Phenyl-butyl)-6-[4-(2-phenyl-cyclopropanecarbonyl)-piperazin-1-yl]-nicotinamide;
6-[4-(2-Cyclohexyl-acetyl)-piperazin-1-yl]-*N*-phenethyl-nicotinamide;
*N*-[2-(3-Chlorophenyl)-ethyl]-6-[4-(2-phenyl-cyclopropanecarbonyl)-piperazin-1-yl]-nicotinamide
*N*-(1-Methyl-3-phenyl-propyl)-6-[4-(2-phenyl-cyclopropanecarbonyl)-piperazin-1-yl]-nicotinamide
*N*-Phenethyl-6-[4-(2-phenyl-cyclopropanecarbonyl)-piperazin-1-yl]-nicotinamide;
6-[4-(2-Phenyl-cyclopropanecarbonyl)-piperazin-1-yl]-*N*-(tetrahydro-furan-2-ylmethyl)-nicotinamide;
*N*-[2-(3H-Imidazol-4-yl)-ethyl]-6-[4-(2-phenyl-cyclopropanecarbonyl)-piperazin-1-yl]-nicotinamide;
*N*-(3-Imidazol-1-yl-propyl)-6-[4-(2-phenyl-cyclopropanecarbonyl)-piperazin-1-yl]-nicotinamide;
6-[4-(2-Cyclohexyl-acetyl)-piperazin-1-yl]-*N*-[2-(3H-imidazol-4-yl)-ethyl]-nicotinamide;
6-[4-(2-Cyclohexyl-acetyl)-piperazin-1-yl]-*N*-(3-phenyl-propyl)-nicotinamide;
6-[4-(3-Cyclohexyl-propionyl)-piperazin-1-yl]-*N*-phenethyl-nicotinamide;
6-[4-(3-Cyclohexyl-propionyl)-piperazin-1-yl]-*N*-(3-phenyl-propyl)-nicotinamide;
6-[4-(3-Methyl-pentanoyl)-piperazin-1-yl]-*N*-(4-phenyl-butyl)-nicotinamide;
6-[4-(2-Cyclohexyl-acetyl)-piperazin-1-yl]-*N*-(4-phenyl-butyl)-nicotinamide;
6-[4-(2-Cyclohexyl-acetyl)-piperazin-1-yl]-*N*-(1-methyl-3-phenyl-propyl)-nicotinamide;
6-[4-(3-Cyclohexyl-propionyl)-piperazin-1-yl]-*N*-[2-(3H-imidazol-4-yl)-ethyl]-nicotinamide;
6-[4-(3-Cyclohexyl-propionyl)-piperazin-1-yl]-*N*-(tetrahydro-furan-2-ylmethyl)-nicotinamide;
6-[4-(2-Cyclohexyl-acetyl)-piperazin-1-yl]-*N*-(3-imidazol-1-yl-propyl)-nicotinamide;
6-[4-(3-Cyclohexyl-propionyl)-piperazin-1-yl]-*N*-(3-imidazol-1-yl-propyl)-nicotinamide;
6-[4-(2-Cyclohexyl-acetyl)-piperazin-1-yl]-*N*-(tetrahydro-furan-2-ylmethyl)-nicotinamide;
6-[4-(3,5-Dichloro-benzoyl)-piperazin-1-yl]-*N*-(3-methoxy-propyl)-nicotinamide;
6-[4-(2-Bromo-5-methoxy-benzoyl)-piperazin-1-yl]-*N*-(3-dimethylamino-propyl)-nicotinamide
6-[4-(2,5-Dichloro-benzoyl)-piperazin-1-yl]-*N*-(3-methylbutyl)-nicotinamide
6-[4-(2,5-Dichloro-benzoyl)-piperazin-1-yl]-*N*-(3-ethoxy-propyl)-nicotinamide
*N*-Butyl-6-[4-(3,5-dichloro-benzoyl)-piperazin-1-yl]-nicotinamide
6-[4-(2,4-Dichloro-benzoyl)-piperazin-1-yl]-*N*-(3-ethoxy-propyl)-nicotinamide
6-[4-(2,4-Dichloro-benzoyl)-piperazin-1-yl]-*N*-(2-ethylsulfanyl-ethyl)-nicotinamide
*N*-(3-Ethoxy-propyl)-6-[4-(4-trifluoromethyl-benzoyl)-piperazin-1-yl]-nicotinamide
*N*-(3-Dimethylamino-propyl)-6-[4-(2,3,4,5-tetrafluoro-benzoyl)-piperazin-1-yl]-nicotinamide
6-[4-(2-Bromo-5-methoxy-benzoyl)-piperazin-1-yl]-*N*-(2-ethylsulfanyl-ethyl)-nicotinamide;
*N*-(2-Ethylsulfanyl-ethyl)-6-[4-(3-trifluoromethyl-benzoyl)-piperazin-1-yl]-nicotinamide
6-[4-(2-Bromo-benzoyl)-piperazin-1-yl]-*N*-(3-methylbutyl)-nicotinamide
*N*-Butyl-6-[4-(2,4-dimethyl-benzoyl)-piperazin-1-yl]-nicotinamide
6-[4-(3,5-Dichloro-benzoyl)-piperazin-1-yl]-*N*-(3-isopropoxy-propyl)-nicotinamide;
6-[4-(2-Bromo-5-methoxy-benzoyl)-piperazin-1-yl]-*N*-(3-methylbutyl)-nicotinamide
*N*-(3-Butoxy-propyl)-6-[4-(2,5-dichloro-benzoyl)-piperazin-1-yl]-nicotinamide;
*N*-(3-Methyl-butyl)-6-[4-(3-trifluoromethyl-benzoyl)-piperazin-1-yl]-nicotinamide;
6-[4-(2-Bromo-benzoyl)-piperazin-1-yl]-*N*-(3-butoxy-propyl)-nicotinamide;
6-[4-(2,5-Dichloro-benzoyl)-piperazin-1-yl]-*N*-pentyl-nicotinamide;
*N*-(3-Butoxy-propyl)-6-[4-(2,4-dimethyl-benzoyl)-piperazin-1-yl]-nicotinamide;
6-[4-(2-Bromo-5-methoxy-benzoyl)-piperazin-1-yl]-*N*-(3-ethoxy-propyl)-nicotinamide;
6-[4-(2,4-Dichloro-benzoyl)-piperazin-1-yl]-*N*-(3-isopropoxy-propyl)-nicotinamide;
*N*-(3-Ethoxy-propyl)-6-[4-(2-trifluoromethyl-benzoyl)-piperazin-1-yl]-nicotinamide;
*N*-Pentyl-6-[4-(3-trifluoromethyl-benzoyl)-piperazin-1-yl]-nicotinamide;
*N*-Butyl-6-[4-(2-trifluoromethyl-benzoyl)-piperazin-1-yl]-nicotinamide;
*N*-Butyl-6-[4-(2,5-dichloro-benzoyl)-piperazin-1-yl]-nicotinamide;
6-[4-(2-Bromo-benzoyl)-piperazin-1-yl]-*N*-(3-isopropoxy-propyl)-nicotinamide;
6-[4-(2,5-Dichloro-benzoyl)-piperazin-1-yl]-*N*-(3-isopropoxy-propyl)-nicotinamide;
*N*-(3-Butoxy-propyl)-6-[4-(4-trifluoromethyl-benzoyl)-piperazin-1-yl]-nicotinamide;
6-[4-(2,5-Dichloro-benzoyl)-piperazin-1-yl]-*N*-(2-ethylsulfanyl-ethyl)-nicotinamide;
*N*-(3-Butoxy-propyl)-6-[4-(2-trifluoromethyl-benzoyl)-piperazin-1-yl]-nicotinamide;
6-[4-(2,5-Dichloro-benzoyl)-piperazin-1-yl]-*N*-(3-methoxy-propyl)-nicotinamide;
6-[4-(2,4-Dimethyl-benzoyl)-piperazin-1-yl]-*N*-(1,3-dimethylbutyl)-nicotinamide;
6-[4-(2,4-Dichloro-benzoyl)-piperazin-1-yl]-*N*-(2-methylbutyl)-nicotinamide;
6-[4-(3,5-Dichloro-benzoyl)-piperazin-1-yl]-*N*-(2-methylbutyl)-nicotinamide;
6-[4-(2,4-Dimethyl-benzoylypiperazin-1-yl]-*N*-(2-ethylsulfanyl-ethyl)-nicotinamide;
*N*-(1,3-Dimethylbutyl)-6-[4-(3-trifluoromethyl-benzoyl)-piperazin-1-yl]-nicotinamide;
6-[4-(2-Bromo-5-methoxy-benzoyl)-piperazin-1-yl]-*N*-(2-methylbutyl)-nicotinamide;
*N*-(3-Dimethylamino-propyl)-6-[4-(2-trifluoromethyl-benzoyl)-piperazin-1-yl]-nicotinamide;
*N*-Hexyl-6-[4-(3-trifluoromethyl-benzoyl)-piperazin-1-yl]-nicotinamide;
6-[4-(2,4-Dichloro-benzoyl)-piperazin-1-yl]-*N*-(1,3-dimethylbutyl)-nicotinamide;
*N*-(3-Isopropoxy-propyl)-6-[4-(3-trifluoromethyl-benzoyl)-piperazin-1-yl]-nicotinamide;
6-[4-(2-Bromo-5-methoxy-benzoyl)-piperazin-1-yl]-*N*-hexyl-nicotinamide;

6-[4-(2-Bromo-benzoyl)-piperazin-1-yl]-*N*-hexyl-nicotinamide;
*N*-(3-Isopropoxy-propyl)-6-[4-(2-trifluoromethyl-benzoyl)-piperazin-1-yl]-nicotinamide;
6-[4-(2-Bromo-5-methoxy-benzoyl)-piperazin-1-yl]-*N*-(3-methoxy-propyl)-nicotinamide;
6-[4-(2-Bromo-benzoyl)-piperazin-1-yl]-*N*-(1-methylbutyl)-nicotinamide;
6-[4-(3,5-Dichloro-benzoyl)-piperazin-1-yl]-*N*-hexyl-nicotinamide;
*N*-(3-Methoxy-propyl)-6-[4-(2-trifluoromethyl-benzoyl)-piperazin-1-yl]-nicotinamide;
6-[4-(2,4-Dichloro-benzoyl)-piperazin-1-yl]-*N*-hexyl-nicotinamide;
*N*-(2-Ethylsulfanyl-ethyl)-6-[4-(2-trifluoromethyl-benzoyl)-piperazin-1-yl]-nicotinamide;
*N*-Pentyl-6-[4-(2-trifluoromethyl-benzoyl)-piperazin-1-yl]-nicotinamide;
*N*-(2-Methyl-butyl)-6-[4-(3-trifluoromethyl-benzoyl)-piperazin-1-yl]-nicotinamide;
*N*-(2-Ethylsulfanyl-ethyl)-6-[4-(4-trifluoromethyl-benzoyl)-piperazin-1-yl]-nicotinamide;
6-[4-(2,4-Dichloro-benzoyl)-piperazin-1-yl]-*N*-pentyl-nicotinamide;
6-[4-(2-Bromo-benzoyl)-piperazin-1-yl]-*N*-(2-methylbutyl)-nicotinamide;
6-[4-(2-Bromo-5-methoxy-benzoyl)-piperazin-1-yl]-*N*-(3-butoxy-propyl)-nicotinamide;
*N*-(1,3-Dimethylbutyl)-6-[4-(4-trifluoromethyl-benzoyl)-piperazin-1-yl]-nicotinamide;
6-[4-(3,5-Dichloro-benzoyl)-piperazin-1-yl]-*N*-pentyl-nicotiriamide;
*N*-(3-Butoxy-propyl)-6-[4-(3,5-dichloro-benzoyl)-piperazin-1-yl]-nicotinamide;
6-[4-(2-Bromo-5-methoxy-benzoyl)-piperazin-1-yl]-*N*-butyl-nicotinamide;
6-[4-(2-Bromo-benzoyl)-piperazin-1-yl]-*N*-pentyl-nicotinamide;
*N*-(1,3-Dimethylbutyl)-6-[4-(2-trifluoromethyl-benzoyl)-piperazin-1-yl]-nicotinamide;
*N*-(3-Methoxy-propyl)-6-[4-(4-trifluoromethyl-benzoyl)-piperazin-1-yl]-nicotinamide;
6-[4-(2,4-Dimethyl-benzoyl)-piperazin-1-yl]-*N*-(1-methylbutyl)-nicotinamide;
6-[4-(2,4-Dichloro-benzoyl)-piperazin-1-yl]-*N*-(3-methoxy-propyl)-nicotinamide;
*N*-(1-Methyl-butyl)-6-[4-(2-trifluoromethyl-benzoyl)-piperazin-1-yl]-nicotinamide;
*N*-(3-Dimethylamino-propyl)-6-[4-(3-trifluoromethyl-benzoyl)-piperazin-1-yl]-nicotinamide;
6-[4-(2,4-Dichloro-benzoyl)-piperazin-1-yl]-*N*-(1-methylbutyl)-nicotinamide;
6-[4-(2,5-Dichloro-benzoyl)-piperazin-1-yl]-*N*-(1-methylbutyl)-nicotinamide;
*N*-(1-Methyl-butyl)-6-[4-(3-trifluoromethyl-benzoyl)-piperazin-1-yl]-nicotinamide;
6-[4-(2,5-Dichloro-benzoyl)-piperazin-1-yl]-*N*-hexyl-nicotinamide;
6-[4-(2,4-Dimethyl-benzoyl)-piperazin-1-yl]-*N*-(3-methoxy-propyl)-nicotinamide;
6-[4-(3,5-Dichloro-benzoyl)-piperazin-1-yl]-*N*-(1-methylbutyl)-nicotinamide;
6-[4-(2,4-Dichloro-benzoyl)-piperazin-1-yl]-*N*-(3-methylbutyl)-nicotinamide;
*N*-(3-Butoxy-propyl)-6-[4-(3-trifluoromethyl-benzoyl)-piperazin-1-yl]-nicotinamide;
6-[4-(2,4-Dimethyl-benzoyl)-piperazin-1-yl]-*N*-(3-isopropoxy-propyl)-nicotinamide;
*N*-(1-Methyl-butyl)-6-[4-(4-trifluoromethyl-benzoyl)-piperazin-1-yl]-nicotinamide;
*N*-Butyl-6-[4-(2,4-dichloro-benzoyl)-piperazin-1-yl]-nicotinamide;
6-[4-(3,5-Dichloro-benzoyl)-piperazin-1-yl]-*N*-(1,3-dimethylbutyl)-nicotinamide;
*N*-Butyl-6-[4-(3-trifluoromethyl-benzoyl)-piperazin-1-yl]-nicotinamide;
6-[4-(2-Bromo-5-methoxy-benzoyl)-piperazin-1-yl]-*N*-(1,3-dimethylbutyl)-nicotinamide;
6-[4-(2,4-Dimethyl-benzoyl)-piperazin-1-yl]-*N*-hexyl-nicotinamide;
6-[4-(2,5-Dichloro-benzoyl)-piperazin-1-yl]-*N*-(1,3-dimethylbuty)-nicotinamide;
*N*-(3-Butoxy-propyl)-6-[4-(2,4-dichloro-benzoyl)-piperazin-1-yl]-nicotinamide;
*N*-(3-Methoxy-propyl)-6-[4-(3-trifluoromethyl-benzoyl)-piperazin-1-yl]-nicotinamide;
6-[4-(2,4-Dimethyl-benzoyl)-piperazin-1-yl]-*N*-(3-ethoxy-propyl)-nicotinamide;
6-[4-(2,5-Dichloro-benzoyl)-piperazin-1-yl]-*N*-(2-methylbutyl)-nicotinamide;
*N*-Hexyl-6-[4-(4-trifluoromethyl-benzoyl)-piperazin-1-yl]-nicotinamide;
6-[4-(2,4-Dimethyl-benzoyl)-piperazin-1-yl]-*N*-pentyl-nicotinamide;
6-[4-(2-Bromo-benzoyl)-piperazin-1-yl]-*N*-(1,3-dimethylbutyl)-nicotinamide;
*N*-Hexyl-6-[4-(2-trifluoromethyl-benzoyl)-piperazin-1-yl]-nicotinamide;
6-[4-(2-Bromo-benzoyl)-piperazin-1-yl]-*N*-(3-methoxy-propyl)-nicotinamide;
*N*-(2-Methyl-butyl)-6-[4-(4-trifluoromethyl-benzoyl)-piperazin-1-yl]-nicotinamide;
6-[4-(2-Bromo-5-methoxy-benzoyl)-piperazin-1-yl]-*N*-(1-methylbutyl)-nicotinamide;
6-[4-(2,4-Dimethyl-benzoyl)-piperazin-1-yl]-*N*-(2-methylbutyl)-nicotinamide;
6-[4-(2-Bromo-5-methoxy-benzoyl)-piperazin-1-yl]-*N*-pentyl-nicotinamide;
*N*-(2-Methyl-butyl)-6-[4-(2-trifluoromethyl-benzoyl)-piperazin-1-yl]-nicotinamide; 6-[4-(3,5-Dichloro-benzoyl)-piperazin-1-yl]-*N*-(3-methylbutyl)-nicotinamide;
6-[4-(2-Bromo-benzoyl)-piperazin-1-yl]-*N*-(2-ethylsulfanyl-ethyl)-nicotinamide;
6-[4-(3,5-Dichloro-benzoyl)-piperazin-1-yl]-*N*-(3-methoxy-propyl)-nicotinamide;
6-[4-(2-Bromo-5-methoxy-benzoyl)-piperazin-1-yl]-*N*-(3-isopropoxy-propyl)-nicotinamide;
*N*-(3-Butoxy-propyl)-6-[4-(2,3,4,5-tetrafluoro-benzoyl)-piperazin-1-yl]-nicotinamide;
*N*-(1,3-Dimethylbutyl)-6-[4-(2,3,4,5-tetrafluoro-benzoyl)-piperazin-1-yl]-nicotinamide;
*N*-Butyl-6-[4-(2,3,4,5-tetrafluoro-benzoyl)-piperazin-1-yl]-nicotinamide;
*N*-(1-Methyl-butyl)-6-[4-(2,3,4,5-tetrafluoro-benzoyl)-piperazin-1-yl]-nicotinamide;
*N*-(3-Methoxy-propyl)-6-[4-(2,3,4,5-tetrafluoro-benzoyl)-piperazin-1-yl]-nicotinamide;
*N*-(3-Isopropoxy-propyl)-6-[4-(2,3,4,5-tetrafluoro-benzoyl)-piperazin-1-yl]-nicotinamide;
*N*-Pentyl-6-[4-(2,3,4,5-tetrafluoro-benzoyl)-piperazin-1-yl]-nicotinamide;
*N*-(2-Methyl-butyl)-6-[4-(2,3,4,5-tetrafluoro-benzoyl)-piperazin-1-yl]-nicotinamide;
*N*-Hexyl-6-[4-(2,3,4,5-tetrafluoro-benzoyl)-piperazin-1-yl]-nicotinamide;
*N*-(3-Methyl-butyl)-6-[4-(2,3,4,5-tetrafluoro-benzoyl)-piperazin-1-yl]-nicotinamide;
*N*-(2-Ethylsulfanyl-ethyl)-6-[4-(2,3,4,5-tetrafluoro-benzoyl)-piperazin-1-yl]-nicotinamide;
*N*-(3-Ethoxy-propyl)-6-[4-(2,3,4,5-tetrafluoro-benzoyl)-piperazin-1-yl]-nicotinamide;
*N*-(3-Dimethylamino-propyl)-6-[4-(4-trifluoromethyl-benzoyl)-piperazin-1-yl]-nicotinamide;
*N*-(3-Ethoxy-propyl)-6-[4-(3-trifluoromethyl-benzoyl)-piperazin-1-yl]-nicotinamide;
6-[4-(3,5-Dichloro-benzoyl)-piperazin-1-yl]-*N*-(2-ethylsulfanyl-ethyl)-nicotinamide;
*N*-(3-Methyl-butyl)-6-[4-(4-trifluoromethyl-benzoyl)-piperazin-1-yl]-nicotinamide;
*N*-Butyl-6-[4-(4-trifluoromethyl-benzoyl)-piperazin-1-yl]-nicotinamide;
*N*-(3-Isopropoxy-propyl)-6-[4-(4-trifluoromethyl-benzoyl)-piperazin-1-yl]-nicotinamide;
*N*-Pentyl-6-[4-(4-trifluoromethyl-benzoyl)-piperazin-1-yl]-nicotinamide;
6-[4-(3,5-Dichloro-benzoyl)-piperazin-1-yl]-*N*-(3-dimethylamino-propyl)-nicotinamide;
6-[4-(2-Bromo-benzoyl)-piperazin-1-yl]-*N*-(3-dimethylamino-propyl)-nicotinamide;
6-[4-(2,4-Dichloro-benzoyl)-piperazin-1-yl]-*N*-(3-dimethylamino-propyl)-nicotinamide;
6-[4-(2,5-Dichloro-benzoyl)-piperazin-1-yl]-*N*-(3-dimethylamino-propyl)-nicotinamide;
*N*-(3-Dimethylamino-propyl)-6-[4-(2,4-dimethyl-benzoyl)-piperazin-1-yl]-nicotinamide;
*N*-(3-Ethoxy-propyl)-6-[4-(2-naphthalen-2-yl-acetyl)-piperazin-1-yl]-nicotinamide;
6-[4-(2-Naphthalen-2-yl-acetyl)-piperazin-1-yl]-*N*-pentyl-nicotinamide
*N*-Butyl-6-[4-(naphthalene-1-carbonyl)-piperazin-1-yl]-nicotinamide
*N*-(3-Butoxy-propyl)-6-[4-(2-naphthalen-2-yl-acetyl)-piperazin-1-yl]-nicotinamide
*N*-Butyl-6-[4-(2-naphthalen-2-yl-acetyl)-piperazin-1-yl]-nicotinamide;
*N*-(3-Butoxy-propyl)-6-[4-(naphthalene-1-carbonyl)-piperazin-1-yl]-nicotinamide;
*N*-(3-Methoxy-propyl)-6-[4-(naphthalene-1-carbonyl)-piperazin-1-yl]-nicotinamide;
*N*-(1-Methyl-butyl)-6-[4-(naphthalene-1-carbonyl)-piperazin-1-yl]-nicotinamide;
6-[4-(Naphthalene-1-carbonyl)-piperazin-1-yl]-*N*-pentyl-nicotinamide;
*N*-(3-Isopropoxy-propyl)-6-[4-(2-naphthalen-2-yl-acetyl)-piperazin-1-yl]-nicotinamide;
*N*-(1-Methyl-butyl)-6-[4-(2-naphthalen-2-yl-acetyl)-piperazin-1-yl]-nicotinamide;
*N*-(3-Methyl-butyl)-6-[4-(2-naphthalen-2-yl-acetyl)-piperazin-1-yl]-nicotinamide;
*N*-(3-Dimethylamino-propyl)-6-[4-(2-naphthalen-2-yl-acetyl)-piperazin-1-yl]-nicotinamide;
*N*-(3-Ethoxy-propyl)-6-[4-(naphthalene-1-carbonyl)-piperazin-1-yl]-nicotinamide;
*N*-Hexyl-6-[4-(naphthalene-1-carbonyl)-piperazin-1-yl]-nicotinamide;
*N*-(2-Ethylsulfanyl-ethyl)-6-[4-(2-naphthalen-2-yl-acetyl)-piperazin-1-yl]-nicotinamide;
*N*-Hexyl-6-[4-(2-naphthalen-2-yl-acetyl)-piperazin-1-yl]-nicotinamide;
*N*-(1,3-Dimethylbutyl)-6-[4-(naphthalene-1-carbonyl)-piperazin-1-yl]-nicotinamide;
*N*-(3-Isopropoxy-propyl)-6-[4-(naphthalene-1-carbonyl)-piperazin-1-yl]-nicotinamide;
*N*-(1,3-Dimethylbutyl)-6-[4-(naphthalene-2-carbonyl)-piperazin-1-yl]-nicotinamide;
*N*-(1,3-Dimethylbutyl)-6-[4-(2-naphthalen-2-yl-acetyl)-piperazin-1-yl]-nicotinamide;
*N*-(2-Methyl-butyl)-6-[4-(naphthalene-1-carbonyl)-piperazin-1-yl]-nicotinamide;
*N*-(2-Ethylsulfanyl-ethyl)-6-[4-(naphthalene-1-carbonyl)-piperazin-1-yl]-nicotinamide;
*N*-(2-Methyl-butyl)-6-[4-(2-naphthalen-2-yl-acetyl)-piperazin-1-yl]-nicotinamide;
*N*-(3-Ethoxy-propyl)-6-[4-(naphthalene-2-carbonyl)-piperazin-1-yl]-nicotinamide;
*N*-(2-Methyl-butyl)-6-[4-(naphthalene-2-carbonyl)-piperazin-1-yl]-nicotinamide;
*N*-(3-Methyl-butyl)-6-[4-(naphthalene-2-carbonyl)-piperazin-1-yl]-nicotinamide;
*N*-(3-Methoxy-propyl)-6-[4-(naphthalene-2-carbonyl)-piperazin-1-yl]-nicotinamide;
*N*-Butyl-6-[4-(naphthalene-2-carbonyl)-piperazin-1-yl]-nicotinamide;
*N*-(3-Butoxy-propyl)-6-[4-(naphthalene-2-carbonyl)-piperazin-1-yl]-nicotinamide;
*N*-(1-Methyl-butyl)-6-[4-(naphthalene-2-carbonyl)-piperazin-1-yl]-nicotinamide;
*N*-(3-Isopropoxy-propyl)-6-[4-(naphthalene-2-carbonyl)-piperazin-1-yl]-nicotinamide;
*N*-Hexyl-6-[4-(naphthalene-2-carbonyl)-piperazin-1-yl]-nicotinamide;
*N*-(3-Dimethylamino-propyl)-6-[4-(naphthalene-1-carbonyl)-piperazin-1-yl]-nicotinamide;
*N*-(2-Ethylsulfanyl-ethyl)-6-[4-(naphthalene-2-carbonyl)-piperazin-1-yl]-nicotinamide;
*N*-(3-Dimethylamino-propyl)-6-[4-(naphthalene-2-carbonyl)-piperazin-1-yl]-nicotinamide;
*N*-(3-Methoxy-propyl)-6-[4-(2-naphthalen-2-yl-acetyl)-piperazin-1-yl]-nicotinamide;
*N*-(2-Cyclopropyl-ethyl)-6-[4-(3-trifluoromethyl-benzoyl)-piperazin-1-yl]-nicotinamide ;
*N*-(2-Cyclopropyl-ethyl)-6-[4-(5-fluoro-2-trifluoromethyl-benzoyl)-piperazin-1-yl]-nicotinamide ;
*N*-(2-Cyclopropyl-ethyl)-2-hydroxy-6-[4-(2-trifluoromethyl-benzoyl)-piperazin-1-yl]-nicotinamide ;

*N*-(2-Cyclobutyl-ethyl)-6-[4-(5-fluoro-2-trifluoromethyl-benzoyl)-piperazin-1-yl]-nicotinamide ;
*N*-(3-Cyclopropyl-propyl)-6-[4-(5-fluoro-2-trifluoromethyl-benzoyl)-piperazin-1-yl]-nicotinamide ;
6-[4-(5-Fluoro-2-trifluoromethyl-benzoyl)-piperazin-1-yl]-*N*-(4-methyl-pentyl)-nicotinamide;
*N*-(3,3-Dimethylbutyl)-6-[4-(5-fluoro-2-trifluoromethyl-benzoyl)-piperazin-1-yl]-nicotinamide ;
*N*-(1-Methyl-2-phenyl-ethyl)-6-[4-(2-trifluoromethyl-benzoyl)-piperazin-1-yl]-nicotinamide;
*N*-(1-Methyl-2-phenyl-ethyl)-6-[4-(4-trifluoromethyl-benzoyl)-piperazin-1-yl]-nicotinamide;
6-[4-(Naphthalene-1-carbonyl)-piperazin-1-yl]-*N*-(4-phenyl-butyl)-nicotinamide*N*-[2-(3-Chlorophenyl)-ethyl]-6-[4-(2-naphthalen-2-yl-acetyl)-piperazin-1-yl]-nicotinamide;
6-[4-(Naphthalene-2-carbonyl)-piperazin-1-yl]-*N*-(3-phenyl-propyl)-nicotinamide;
*N*-(3-Imidazol-1-yl-propyl)-6-[4-(2-naphthalen-2-yl-acetyl)-piperazin-1-yl]-nicotinamide;
6-[4-(Naphthalene-1-carbonyl)-piperazin-1-yl]-*N*-(3-phenyl-propyl)-nicotinamide;
*N*-(1-Methyl-3-phenyl-propyl)-6-[4-(2-naphthalen-2-yl-acetyl)-piperazin-1-yl]-nicotinamide;
6-[4-(2-Naphthalen-2-yl-acetyl)-piperazin-1-yl]-*N*-phenethyl-nicotinamide;
*N*-[2-(3-Chlorophenyl)-ethyl]-6-[4-(naphthalene-1-carbonyl)-piperazin-1-yl]-nicotinamide;
*N*-(1-Methyl-3-phenyl-propyl)-6-[4-(naphthalene-1-carbonyl)-piperazin-1-yl]-nicotinamide;
6-[4-(Naphthalene-1-carbonyl)-piperazin-1-yl]-*N*-phenethyl-nicotinamide;
*N*-[2-(3H-Imidazol-4-yl)-ethyl]-6-[4-(2-naphthalen-2-yl-acetyl)-piperazin-1-yl]-nicotinamide;
6-[4-(2-Naphthalen-2-yl-acetyl)-piperazin-1-yl]-*N*-(4-phenyl-butyl)-nicotinamide;
6-[4-(Naphthalene-2-carbonyl)-piperazin-1-yl]-*N*-(3-phenyl-propyl)-nicotinamide;
*N*-(1-Methyl-3-phenyl-propyl)-6-[4-(naphthalene-2-carbonyl)-piperazin-1-yl]-nicotinamide;
*N*-[2-(3H-Imidazol-4-yl)-ethyl]-6-[4-(naphthalene-1-carbonyl)-piperazin-1-yl]-nicotinamide;
6-[4-(Naphthalene-2-carbonyl)-piperazin-1-yl]-*N*-(tetrahydro-furan-2-ylmethyl)-nicotinamide;
6-[4-(Naphthalene-1-carbonyl)-piperazin-1-yl]-*N*-(tetrahydro-furan-2-ylmethyl)-nicotinamide;
*N*-(3-Imidazol-1-yl-propyl)-6-[4-(naphthalene-2-carbonyl)-piperazin-1-yl]-nicotinamide;
*N*-[2-(3H-Imidazol-4-yl)-ethyl]-6-[4-(naphthalene-2-carbonyl)-piperazin-1-yl]-nicotinamide;
6-[4-(Naphthalene-2-carbonyl)-piperazin-1-yl]-*N*-phenethyl-nicotinamide;
6-[4-(Naphthalene-2-carbonyl)-piperazin-1-yl]-*N*-(4-phenyl-butyl)-nicotinamide;
*N*-[2-(3-Chlorophenyl)-ethyl]-6-[4-(naphthalene-2-carbonyl)-piperazin-1-yl]-nicotinamide;
*N*-(3-Imidazol-1-yl-propyl)-6-[4-(naphthalene-1-carbonyl)-piperazin-1-yl]-nicotinamide;
6-[4-(2-Naphthalen-2-yl-acetyl)-piperazin-1-yl]-*N*-(tetrahydro-furan-2-ylmethyl)-nicotinamide;
6-[4-(2-Bromo-benzoyl)-piperazin-1-yl]-*N*-(3-phenyl-propyl)-nicotinamide;
6-[4-(2,4-Dimethyl-benzoyl)-piperazin-1-yl]-*N*-(4-phenyl-butyl)-nicotinamide;
*N*-Phenethyl-6-[4-(2-p-tolyl-acetyl)-piperazin-1-yl]-nicotinamide;
6-{4-[2-(2,5-Dichloro-phenyl)-acetyl]-piperazin-1-yl}-*N*-(2-methyl-3-phenylpropyl)-nicotinamide;
6-[4-(2,4-Dimethyl-benzoyl)-piperazin-1-yl]-*N*-(3-phenyl-propyl)-nicotinamide;
*N*-[2-(3-Chlorophenyl)-ethyl]-6-[4-(2-fluoro-4-methyl-benzoyl)-piperazin-1-yl]-nicotinamide;
*N*-[2-(3-Chlorophenyl)-ethyl]-6-[4-(2,5-dichloro-benzoyl)-piperazin-1-yl]-nicotinamide;
*N*-(3-Phenyl-propyl)-6-[4-(4-trifluoromethyl-benzoyl)-piperazin-1-yl]-nicotinamide;
6-[4-(2-Bromo-benzoyl)-piperazin-1-yl]-*N*-(3-imidazol-1-yl-propyl)-nicotinamide;
*N*-(4-Phenyl-butyl)-6-[4-(2-trifluoromethyl-benzoyl)-piperazin-1-yl]-nicotinamide;
*N*-(3-Phenyl-propyl)-6-[4-(3-trifluoromethyl-benzoyl)-piperazin-1-yl]-nicotinamide;
6-[4-(2-Bromo-benzoyl)-piperazin-1-yl]-*N*-[2-(3-chloro-phenyl)-ethyl]-nicotinamide;
6-[4-(2-Bromo-benzoyl)-piperazin-1-yl]-*N*-(2-methyl-3-phenyl-propyl)-nicotinamide;
6-[4-(2-Bromo-5-methoxy-benzoyl)-piperazin-1-yl]-*N*-(5-phenyl-pentyl)-nicotinamide;
6-[4-(2-Bromo-5-methoxy-benzoyl)-piperazin-1-yl]-*N*-[2-(3-chloro-phenyl)-ethyl]-nicotinamide;
*N*-[2-(3-Chlorophenyl)-ethyl]-6-[4-(2-trifluoromethyl-benzoylypiperazin-1-yl]-nicotinamide;
*N*-(3-Phenyl-propyl)-6-[4-(2-trifluoromethyl-benzoyl)-piperazin-1-yl]-nicotinamide;
*N*-(4-Phenyl-butyl)-6-[4-(3-trifluoromethyl-benzoyl)-piperazin-1-yl]-nicotinamide;
6-[4-(2,4-Dichloro-benzoyl)-piperazin-1-yl]-*N*-phenethyl-nicotinamide;
6-[4-(2-Bromo-benzoyl)-piperazin-1-yl]-*N*-(4-phenyl-butyl)-nicotinamide;
6-[4-(2-Bromo-benzoyl)-piperazin-1-yl]-*N*-phenethyl-nicotinamide;
6-[4-(2-Bromo-5-methoxy-benzoyl)-piperazin-1-yl]-*N*-(1-methyl-3-phenylpropyl)-nicotinamide;
6-[4-(3,5-Dichloro-benzoyl)-piperazin-1-yl]-*N*-(3-phenyl-propyl)-nicotinamide;
6-[4-(3,5-Dichloro-benzoyl)-piperazin-1-yl]-*N*-(1-methyl-3-phenyl-propyl)-nicotinamide;
*N*-Phenethyl-6-[4-(4-trifluoromethyl-benzoyl)-piperazin-1-yl]-nicotinamide;
6-[4-(2,4-Dimethyl-benzoyl)-piperazin-1-yl]-*N*-phenethyl-nicotinamide;
*N*-Phenethyl-6-[4-(2-trifluoromethyl-benzoyl)-piperazin-1-yl]-nicotinamide;
6-[4-(2,4-Dichloro-benzoyl)-piperazin-1-yl]-*N*-(1-methyl-3-phenyl-propyl)-nicotinamide;
6-[4-(2,5-Dichloro-benzoyl)-piperazin-1-yl]-*N*-(3-phenyl-propyl)-nicotinamide;
6-[4-(2-Bromo-5-methoxy-benzoyl)-piperazin-1-yl]-*N*-phenethyl-nicotinamide;
6-[4-(2-Bromo-5-methoxy-benzoyl)-piperazin-1-yl]-*N*-(3-imidazol-1-yl-propyl)-nicotinamide;
6-[4-(3,5-Dichloro-benzoyl)-piperazin-1-yl]-*N*-(3-imidazol-1-yl-propyl)-nicotinamide;
*N*-[2-(3H-Imidazol-4-yl)-ethyl]-6-[4-(2,3,4,5-tetrafluoro-benzoyl)-piperazin-1-yl]-nicotinamide;
*N*-Phenethyl-6-[4-(3-trifluoromethyl-benzoyl)-piperazin-1-yl]-nicotinamide;
6-[4-(2,5-Dichloro-benzoyl)-piperazin-1-yl]-*N*-phenethyl-nicotinamide;
6-[4-(3-Methyl-pentanoyl)-piperazin-1-yl]-*N*-(1-methyl-3-phenyl-propyl)-nicotinamide;
6-[4-(2,3,4,5-Tetrafluoro-benzoyl)-piperazin-1-yl]-*N*-(tetrahydro-furan-2-ylmethyl)-nicotinamide;
6-[4-(2-Bromo-5-methoxy-benzoyl)-piperazin-1-yl]-*N*-(tetrahydro-furan-2-ylmethyl)-nicotinamide;
*N*-(Tetrahydro-furan-2-ylmethyl)-6-[4-(4-trifluoromethyl-benzoyl)-piperazin-1-yl]-nicotinamide;
6-[4-(3,5-Dichloro-benzoyl)-piperazin-1-yl]-*N*-(4-phenyl-butyl)-nicotinamide;
6-[4-(2,4-Dimethyl-benzoyl)-piperazin-1-yl]-*N*-(1-methyl-3-phenyl-propyl)-nicotinamide;
6-[4-(2,4-Dimethyl-benzoyl)-piperazin-1-yl]-*N*-(tetrahydro-furan-2-ylmethyl)-nicotinamide;
6-[4-(2-Bromo-benzoyl)-piperazin-1-yl]-*N*-(tetrahydro-furan-2-ylmethyl)-nicotinamide;
*N*-(1-Methyl-3-phenyl-propyl)-6-[4-(3-trifluoromethyl-benzoyl)-piperazin-1-yl]-nicotinamide;
*N*-Phenethyl-6-[4-(2,3,4,5-tetrafluoro-benzoyl)-piperazin-1-yl]-nicotinamide;
*N*-(4-Phenyl-butyl)-6-[4-(2,3,4,5-tetrafluoro-benzoyl)-piperazin-1-yl]-nicotinamide;
*N*-(1-Methyl-3-phenyl-propyl)-6-[4-(2,3,4,5-tetrafluoro-benzoyl)-piperazin-1-yl]-nicotinamide;
*N*-[2-(3-Chlorophenyl)-ethyl]-6-[4-(2,3,4,5-tetrafluoro-benzoyl)-piperazin-1-yl]-nicotinamide;
*N*-(3-Phenyl-propyl)-6-[4-(2,3,4,5-tetrafluoro-benzoyl)-piperazin-1-yl]-nicotinamide;
6-[4-(2,5-Dichloro-benzoyl)-piperazin-1-yl]-*N*-(tetrahydro-furan-2-ylmethyl)-nicotinamide;
6-[4-(2,4-Dimethyl-benzoyl)-piperazin-1-yl]-*N*-[2-(3H-imidazol-4-yl)-ethyl]-nicotinamide;
*N*-[2-(3H-Imidazol-4-yl)-ethyl]-6-[4-(3-trifluoromethyl-benzoyl)-piperazin-1-yl]-nicotinamide;
*N*-(3-lmidazol-1-yl-propyl)-6-[4-(3-trifluoromethyl-benzoyl)-piperazin-1-yl]-nicotinamide;
6-[4-(2,4-Dimethyl-benzoyl)-piperazin-1-yl]-*N*-(3-imidazol-1-yl-propyl)-nicotinamide;
6-[4-(2,5-Dichloro-benzoyl)-piperazin-1-yl]-*N*-[2-(3H-imidazol-4-yl)-ethyl]-nicotinamide;
*N*-[2-(3H-Imidazol-4-yl)-ethyl]-6-[4-(4-trifluoromethyl-benzoyl)-piperazin-1-yl]-nicotinamide;
6-[4-(2,4-Dichloro-benzoyl)-piperazin-1-yl]-*N*-[2-(3H-imidazol-4-yl)-ethyl]-nicotinamide;
6-[4-(3,5-Dichloro-benzoyl)-piperazin-1-yl]-*N*-(tetrahydro-furan-2-ylmethyl)-nicotinamide;
*N*-(3-Imidazol-1-yl-propyl)-6-[4-(2-trifluoromethyl-benzoyl)-piperazin-1-yl]-nicotinamide;
*N*-(Tetrahydro-furan-2-ylmethyl)-6-[4-(3-trifluoromethyl-benzoyl)-piperazin-1-yl]-nicotinamide;
*N*-(Tetrahydro-furan-2-ylmethyl)-6-[4-(2-trifluoromethyl-benzoyl)-piperazin-1-yl]-nicotinamide;
6-[4-(2,4-Dichloro-benzoyl)-piperazin-1-yl]-*N*-(tetrahydro-furan-2-ylmethyl)-nicotinamide;
*N*-[2-(3H-Imidazol-4-yl)-ethyl]-6-[4-(2-trifluoromethyl-benzoyl)-piperazin-1-yl]-nicotinamide;
6-[4-(2-Bromo-5-methoxy-benzoyl)-piperazin-1-yl]-*N*-[2-(3H-imidazol-4-yl)-ethyl]-nicotinamide;
*N*-[2-(3-Chlorophenyl)-ethyl]-6-[4-(3-trifluoromethyl-benzoyl)-piperazin-1-yl]-nicotinamide;
*N*-[2-(3-Chlorophenyl)-ethyl]-6-[4-(4-trifluoromethyl-benzoyl)-piperazin-1-yl]-nicotinamide;
*N*-[2-(3-Chlorophenyl)-ethyl]-6-[4-(3,5-dichloro-benzoyl)-piperazin-1-yl]-nicotinamide;
*N*-(4-Phenyl-butyl)-6-[4-(4-trifluoromethyl-benzoyl)-piperazin-1-yl]-nicotinamide;
6-[4-(2,4-Dichloro-benzoyl)-piperazin-1-yl]-*N*-(4-phenyl-butyl)-nicotinamide;
6-[4-(2,4-Dichloro-benzoyl)-piperazin-1-yl]-*N*-(3-phenyl-propyl)-nicotinamide;
6-[4-(2,5-Dichloro-benzoyl)-piperazin-1-yl]-*N*-(4-phenyl-butyl)-nicotinamide;
6-[4-(3,5-Dichloro-benzoyl)-piperazin-1-yl]-*N*-phenethyl-nicotinamide;
*N*-(3-Imidazol-1-yl-propyl)-6-[4-(4-trifluoromethyl-benzoyl)-piperazin-1-yl]-nicotinamide;
6-[4-(3,5-Dichloro-benzoyl)-piperazin-1-yl]-*N*-[2-(3H-imidazol-4-yl)-ethyl]-nicotinamide;

*N*-(3-Imidazol-1-yl-propyl)-6-[4-(2,3,4,5-tetrafluoro-benzoyl)-piperazin-1-yl]-nicotinamide;
*N*-(3-Dimethylamino-propyl)-6-[4-(3-methyl-thiophene-2-carbonyl)-piperazin-1-yl]-nicotinamide;
*N*-(3-Dimethylamino-propyl)-6-[4-(thiophene-2-carbonyl)-piperazin-1-yl]-nicotinamide;
6-[4-(2-Chloro-pyridine-3-carbonyl)-piperazin-1-yl]-*N*-(3-ethoxy-propyl)-nicotinamide;
6-[4-(2-Chloro-pyridine-3-carbonyl)-piperazin-1-yl]-*N*-(2-ethylsulfanyl-ethyl)-nicotinamide;
6-[4-(2-Chloro-pyridine-3-carbonyl)-piperazin-1-yl]-*N*-(3-methylbutyl)-nicotinamide;
6-[4-(2-Chloro-pyridine-3-carbonyl)-piperazin-1-yl]-*N-*(3-isopropoxy-propyl)-nicotinamide;
*N*-(2-Ethylsulfanyl-ethyl)-6-[4-(thiophene-2-carbonyl)-piperazin-1-yl]-nicotinamide;
*N*-(3-Ethoxy-propyl)-6-[4-(thiophene-2-carbonyl)-piperazin-1-yl]-nicotinamide;
*N*-(3-Methyl-butyl)-6-[4-(thiophene-2-carbonyl)-piperazin-1-yl]-nicotinamide;
6-[4-(2-Chloro-pyridine-3-carbonyl)-piperazin-1-yl]-*N*-(3-methoxy-propyl)-nicotinamide;
*N*-Pentyl-6-[4-(thiophene-2-carbonyl)-piperazin-1-yl]-nicotinamide;
6-[4-(2-Chloro-pyridine-3-carbonyl)-piperazin-1-yl]-*N*-hexyl-nicotinamide;
*N*-(3-Isopropoxy-propyl)-6-[4-(thiophene-2-carbonyl)-piperazin-1-yl]-nicotinamide;
*N*-(1-Methyl-butyl)-6-[4-(thiophene-2-carbonyl)-piperazin-1-yl]-nicotinamide;
*N*-Hexyl-6-[4-(thiophene-2-carbonyl)-piperazin-1-yl]-nicotinamide;
*N*-(1,3-Dimethylbutyl)-6-[4-(thiophene-2-carbonyl)-piperazin-1-yl]-nicotinamide;
*N*-Butyl-6-[4-(thiophene-2-carbonyl)-piperazin-1-yl]-nicotinamide;
*N*-(3-Methoxy-propyl)-6-[4-(thiophene-2-carbonyl)-piperazin-1-yl]-nicotinamide;
6-[4-(2-Chloro-pyridine-3-carbonyl)-piperazin-1-yl]-*N*-(1,3-dimethylbutyl)-nicotinamide;
*N*-Butyl-6-[4-(2-chloro-pyridine-3-carbonyl)-piperazin-1-yl]-nicotinamide;
6-[4-(2-Chloro-pyridine-3-carbonyl)-piperazin-1-yl]-*N*-(2-methylbutyl)-nicotinamide;
*N*-(2-Methyl-butyl)-6-[4-(thiophene-2-carbonyl)-piperazin-1-yl]-nicotinamide;
6-[4-(2-Chloro-pyridine-3-carbonyl)-piperazin-1-yl]-*N*-(1-methylbutyl)-nicotinamide;
*N*-(1,3-Dimethylbutyl)-6-[4-(3-methyl-thiophene-2-carbonyl)-piperazin-1-yl]-nicotinamide;
*N*-Butyl-6-[4-(3-methyl-thiophene-2-carbonyl)-piperazin-1-yl]-nicotinamide;
*N*-(3-Butoxy-propyl)-6-[4-(3-methyl-thiophene-2-carbonyl)-piperazin-1-yl]-nicotinamide;
*N*-(3-Isopropoxy-propyl)-6-[4-(3-methyl-thiophene-2-carbonyl)-piperazin-1-yl]-nicotinamide;
*N*-(3-Ethoxy-propyl)-6-[4-(3-methyl-thiophene-2-carbonyl)-piperazin-1-yl]-nicotinamide;
*N*-(2-Methyl-butyl)-6-[4-(3-methyl-thiophene-2-carbonyl)-piperazin-1-yl]-nicotinamide;
*N*-(2-Ethylsulfanyl-ethyl)-6-[4-(3-methyl-thiophene-2-carbonyl)-piperazin-1-yl]-nicotinamide;
*N*-(3-Methoxy-propyl)-6-[4-(3-methyl-thiophene-2-carbonyl)-piperazin-1-yl]-nicotinamide;
*N*-(1-Methyl-butyl)-6-[4-(3-methyl-thiophene-2-carbonyl)-piperazin-1-yl]-nicotinamide;
*N*-(3-Methyl-butyl)-6-[4-(3-methyl-thiophene-2-carbonyl)-piperazin-1-yl]-nicotinamide;
*N*-(3-Butoxy-propyl)-6-[4-(2-chloro-pyridine-3-carbonyl)-piperazin-1-yl]-nicotinamide;
*N*-(3-Butoxy-propyl)-6-[4-(thiophene-2-carbonyl)-piperazin-1-yl]-nicotinamide;
6-[4-(2-Chloro-pyridine-3-carbonyl)-piperazin-1-yl]-*N*-pentyl-nicotinamide;
*N*-Hexyl-6-[4-(3-methyl-thiophene-2-carbonyl)-piperazin-1-yl]-nicotinamide;
6-[4-(2-Chloro-pyridine-3-carbonyl)-piperazin-1-yl]-*N*-(3-dimethylamino-propyl)-nicotinamide;
*N*-[2-(3-Chlorophenyl)-ethyl]-6-{4-[2-(2-chloro-pyridin-3-yl)-acetyl]-piperazin-1-yl}-nicotinamide;
6-[4-(2-Chloro-pyridine-3-carbonyl)-piperazin-1-yl]-*N*-(4-phenyl-butyl)-nicotinamide;
*N*-[2-(3-Chlorophenyl)-ethyl]-6-[4-(thiophene-2-carbonyl)-piperazin-1-yl]-nicotinamide;
6-[4-(2-Chloro-pyridine-3-carbonyl)-piperazin-1-yl]-*N*-(3-phenyl-propyl)-nicotinamide;
*N*-(3-Phenyl-propyl)-6-[4-(thiophene-2-carbonyl)-piperazin-1-yl]-nicotinamide;
*N*-Phenethyl-6-[4-(thiophene-2-carbonyl)-piperazin-1-yl]-nicotinamide;
*N*-(1-Methyl-3-phenyl-propyl)-6-[4-(thiophene-2-carbonyl)-piperazin-1-yl]-nicotinamide;
*N*-(4-Phenyl-butyl)-6-[4-(thiophene-2-carbonyl)-piperazin-1-yl]-nicotinamide;
6-[4-(2-Chloro-pyridine-3-carbonyl)-piperazin-1-yl]-*N*-(1-methyl-3-phenylpropyl)-nicotinamide;
6-[4-(2-Chloro-pyridine-3-carbonyl)-piperazin-1-yl]-*N*-phenethyl-nicotinamide;
6-[4-(2-Chloro-pyridine-3-carbonyl)-piperazin-1-yl]-*N*-(tetrahydro-furan-2-ylmethyl)-nicotinamide;
6-[4-(3-Methyl-thiophene-2-carbonyl)-piperazin-1-yl]-*N*-(3-phenyl-propyl)-nicotinamide;
*N*-(1-Methyl-3-phenyl-propyl)-6-[4-(3-methyl-thiophene-2-carbonyl)-piperazin-1-yl]-nicotinamide;
6-[4-(3-Methyl-thiophene-2-carbonyl)-piperazin-1-yl]-*N*-(4-phenyl-butyl)-nicotinamide;
6-[4-(3-Methyl-thiophene-2-carbonyl)-piperazin-1-yl]-*N*-phenethyl-nicotinamide;
6-[4-(3-Methyl-thiophene-2-carbonyl)-piperazin-1-yl]-*N*-(tetrahydro-furan-2-ylmethyl)-nicotinamide;
*N*-(3-Imidazol-1-yl-propyl)-6-[4-(thiophene-2-carbonyl)-piperazin-1-yl]-nicotinamide;
*N*-(Tetrahydro-furan-2-ylmethyl)-6-[4-(thiophene-2-carbonyl)-piperazin-1-yl]-nicotinamide;
*N*-[2-(3H-Imidazol-4-yl)-ethyl]-6-[4-(thiophene-2-carbonyl)-piperazin-1-yl]-nicotinamide;
*N*-(3-Imidazol-1-yl-propyl)-6-[4-(3-methyl-thiophene-2-carbonyl)-piperazin-1-yl]-nicotinamide;
*N*-[2-(3H-Imidazol-4-yl)-ethyl]-6-[4-(3-methyl-thiophene-2-carbonyl)-piperazin-1-yl]-nicotinamide;
6-[4-(2-Chloro-pyridine-3-carbonyl)-piperazin-1-yl]-*N*-(3-imidazol-1-yl-propyl)-nicotinamide;
6-[4-(2-Chloro-pyridine-3-carbonyl)-piperazin-1-yl]-*N*-[2-(3H-imidazol-4-yl)-ethyl]-nicotinamide;
6-{4-[2-(2-Chloro-6-fluoro-phenyl)-acetyl]-piperazin-1-yl)-*N*-(3-ethoxy-propyl)-nicotinamide;
6-{4-[3-(3,4-Difluoro-phenyl)-propionyl]-piperazin-1-yl}-*N*-(3-dimethylaminopropyl)-nicotinamide;
6-{4-[3-(3,4-Difluoro-phenyl)-propionyl]-piperazin-1-yl}-*N*-(3-ethoxy-propyl)-nicotinamide;
6-{4-[2-(4-Chlorophenyl)-propionyl]-piperazin-1-yl}-*N*-(3-ethoxy-propyl)-nicotinamide;
*N*-(3-Ethoxy-propyl)-6-[4-(2-phenyl-butyryl)-piperazin-1-yl]-nicotinamide;
*N*-(2-Ethylsulfanyl-ethyl)-6-[4-(2-o-tolyl-acetyl)-piperazin-1-yl]-nicotinamide;
6-[4-(3-Methyl-pentanoyl)-piperazin-1-yl]-*N*-(4-phenyl-butyl)-nicotinamide;
*N*-Butyl-6-{4-[3-(3,4-difluoro-phenyl)-propionyl]-piperazin-1-yl}-nicotinamide;
6-{4-[2-(2-Chloro-6-fluoro-phenyl)-acetyl]-piperazin-1-yl}-*N*-(3-dimethylaminopropyl)-nicotinamide;
6-{4-[3-(3,4-Difluoro-phenyl)-propionyl]-piperazin-1-yl}-*N*-(2-ethylsulfanyl-ethyl)-nicotinamide;
*N*-(3-Methoxy-propyl)-6-[4-(2-phenyl-butyryl)-piperazin-1-yl]-nicotinamide;
*N*-Butyl-6-[4-(2-phenyl-butyryl)-piperazin-1-yl]-nicotinamide;
*N*-(3-Butoxy-propyl)-6-{4-[2-(4-chloro-phenyl)-propionyl]-piperazin-1-yl}-nicotinamide;
*N*-(3-Methoxy-propyl)-6-[4-(2-o-tolyl-acetyl)-piperazin-1-yl]-nicotinamide;
*N*-(3-Methyl-butyl)-6-[4-(2-phenyl-butyryl)-piperazin-1-yl]-nicotinamide;
*N*-(1-Methyl-butyl)-6-[4-(2-p-tolyl-acetyl)-piperazin-1-yl]-nicotinamide;
*N*-Butyl-6-{4-[2-(2-chloro-6-fluoro-phenyl)-acetyl]-piperazin-1-yl}-nicotinamide;
*N*-(2-Methyl-butyl)-6-[4-(2-o-tolyl-acetyl)-piperazin-1-yl]-nicotinamide;
6-{4-[3-(3,4-Difluoro-phenyl)-propionyl]-piperazin-1-yl}-*N*-(2-methylbutyl)-nicotinamide;
*N*-(3-Isopropoxy-propyl)-6-[4-(2-o-tolyl-acetyl)-piperazin-1-yl]-nicotinamide;
6-{4-[3-(3,4-Difluoro-phenyl)-propionyl]-piperazin-1-yl}-*N*-(3-methylbutyl)-nicotinamide;
6-{4-[3-(3,4-Difluoro-phenyl)-propionyl]-piperazin-1-yl}-*N*-(3-methoxy-propyl)-nicotinamide;
*N*-(1-Methyl-butyl)-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-(2-methylbutyl)-nicotinamide;
6-{4-[2-(2-Chloro-6-fluoro-phenyl)-acetyl]-piperazin-1-yl}-*N*-(1-methylbutyl)-nicotinamide;
*N*-(2-Methyl-butyl)-6-[4-(2-phenyl-butyryl)-piperazin-1-yl]-nicotinamide;
*N*-(1,3-Dimethylbutyl)-6-[4-(2-phenyl-butyryl)-piperazin-1-yl]-nicotinamide;
6-{4-[2-(4-Chlorophenyl)-propionyl]-piperazin-1-yl}-*N*-(2-methylbutyl)-nicotinamide;
6-{4-[2-(2-Chloro-6-fluoro-phenyl)-acetyl]-piperazin-1-yl}-*N*-(3-isopropoxypropyl)-nicotinamide;
*N*-(1,3-Dimethylbutyl)-6-[4-(2-p-tolyl-acetyl)-piperazin-1-yl]-nicotinamide;
6-{4-[3-(3,4-Difluoro-phenyl)-propionyl]-piperazin-1-yl}-*N*-hexyl-nicotinamide;
*N*-(1-Methyl-butyl)-6-[4-(2-phenyl-butyryl)-piperazin-1-yl]-nicotinamide;
6-{4-[2-(4-Chlorophenyl)-propionyl]-piperazin-1-yl}-*N*-(1-methylbutyl)-nicotinamide;
6-{4-[2-(4-Chlorophenyl)-propionyl]-piperazin-1-yl}-*N*-(1,3-dimethylbutyl)-nicotinamide;
*N*-Butyl-6-[4-(2-p-tolyl-acetyl)-piperazin-1-yl]-nicotinamide;
*N*-(1,3-Dimethylbutyl)-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-(1,3-dimethylbutyl)-nicotinamide;
*N*-(3-Methyl-butyl)-6-[4-(2-p-tolyl-acetyl)-piperazin-1-yl]-nicotinamide;
*N*-Butyl-6-[4-(2-o-tolyl-acetyl)-piperazin-1-yl]-nicotinamide;
*N*-(3-Butoxy-propyl)-6-[4-(2-p-tolyl-acetyl)-piperazin-1-yl]-nicotinamide;
*N*-Pentyl-6-[4-(2-p-tolyl-acetyl)-piperazin-1-yl]-nicotinamide;
*N*-(3-Butoxy-propyl)-6-[4-(2-phenyl-butyryl)-piperazin-1-yl]-nicotinamide;
*N*-Pentyl-6-[4-(2-phenyl-butyryl)-piperazin-1-yl-nicotinamide;
*N*-(2-Ethylsulfanyl-ethyl)-6-[4-(2-p-tolyl-acetyl)-piperazin-1-yl]-nicotinamide;
6-{4-[2-(4-Chlorophenyl)-propionyl]-piperazin-1-yl}-*N*-(2-ethylsulfanyl-ethyl)-nicotinamide;
*N*-(3-Ethoxy-propyl)-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-(3-methoxy-propyl)-nicotinamide;
6-{4-[3-(3,4-Difluoro-phenyl)-propionyl]-piperazin-1-yl}-*N*-(3-isopropoxy-propyl)-nicotinamide;
6-{4-[2-(4-Chlorophenyl)-propionyl]-piperazin-1-yl}-*N*-pentyl-nicotinamide;
*N*-(3-Butoxy-propyl)-6-{4-[2-(2-chloro-6-fluoro-phenyl)-acetyl]-piperazin-1-yl}-nicotinamide;

6-{4-[2-(2-Chloro-6-fluoro-phenyl)-acetyl]-piperazin-1-yl}-*N*-hexyl-nicotinamide;
6-{4-[2-(4-Chlorophenyl)-propionyl]-piperazin-1-yl}-*N*-(3-methoxy-propyl)-nicotinamide;
*N*-Hexyl-6-[4-(2-o-tolyl-acetyl)-piperazin-1-yl]-nicotinamide;
6-{4-[3-(3,4-Difluoro-phenyl)-propionyl]-piperazin-1-yl}-*N*-(4-phenyl-butyl)-nicotinamide;
*N*-(3-Isopropoxy-propyl)-6-[4-(2-p-tolyl-acetyl)-piperazin-1-yl]-nicotinamide;
6-{4-[3-(3,4-Difluoro-phenyl)-propionyl]-piperazin-1-yl}-*N*-(1-methylbutyl)-nicotinamide;
6-{4-[3-(3,4-Difluoro-phenyl)-propionyl]-piperazin-1-yl}-*N*-pentyl-nicotinamide;
6-{4-[2-(4-Chlorophenyl)-propionyl]-piperazin-1-yl}-*N*-hexyl-nicotinamide;
*N*-Butyl-6-{4-[2-(4-chloro-phenyl)-propionyl]-piperazin-1-yl}-nicotinamide;
6-{4-[2-(2-Chloro-6-fluoro-phenyl)-acetyl]-piperazin-1-yl}-*N*-(2-ethylsulfanylethyl)-nicotinamide;
6-{4-[2-(2-Chloro-6-fluoro-phenyl)-acetyl]-piperazin-1-yl}-*N*-pentyl-nicotinamide;
*N*-Hexyl-6-[4-(2-phenyl-butyryl)-piperazin-1-yl]-nicotinamide;
*N*-(3-Isopropoxy-propyl)-6-[4-(2-phenyl-butyryl)-piperazin-1-yl]-nicotinamide;
*N*-Pentyl-6-[4-(2-o-tolyl-acetyl)-piperazin-1-yl]-nicotinamide;
*N*-Hexyl-6-[4-(2-p-tolyl-acetyl)-piperazin-1-yl]-nicotinamide;
6-{4-[3-(3,4-Difluoro-phenyl)-propionyl]-piperazin-1-yl}-*N*-(1,3-dimethylbutyl)-nicotinamide;
6-[4-(Naphthalene-2-carbonyl)-piperazin-1-yl]-*N*-pentyl-nicotinamide;
6-{4-[2-(4-Chlorophenyl)-propionyl]-piperazin-1-yl}-*N*-(3-dimethylamino-propyl)-nicotinamide;
*N*-(3-Dimethylamino-propyl)-6-[4-(2-phenyl-butyryl)-piperazin-1-yl]-nicotinamide;
*N*-(3-Dimethylamino-propyl)-6-[4-(2-p-tolyl-acetyl)-piperazin-1-yl]-nicotinamide;
*N*-(3-Dimethylamino-propyl)-6-[4-(2-o-tolyl-acetyl)-piperazin-1-yl]-nicotinamide;
*N*-(3-Butoxy-propyl)-6-{4-[3-(3,4-difluoro-phenyl)-propionyl]-piperazin-1-yl}-nicotinamide;
*N*-(3-Methoxy-propyl)-6-[4-(2-p-tolyl-acetyl)-piperazin-1-yl]-nicotinamide;
*N*-(2-Methyl-butyl)-6-[4-(2-p-tolyl-acetyl)-piperazin-1-yl]-nicotinamide;
*N*-(3-Butoxy-propyl)-6-[4-(2-o-tolyl-acetyl)-piperazin-1-yl]-nicotinamide;
6-{4-[2-(4-Chlorophenyl)-propionyl]-piperazin-1-yl}-*N*-(3-isopropoxy-propyl)-nicotinamide;
6-{4-[3-(3,4-Difluoro-phenyl)-propionyl]-piperazin-1-yl}-*N*-phenethylnicotinamide;
*N*-[2-(3-Chlorophenyl)-ethyl]-6-[4-(2-o-tolyl-acetyl)-piperazin-1-yl]-nicotinamide
6-{4-[2-(2-Chloro-6-fluoro-phenyl)-acetyl]-piperazin-1-yl}-*N*-(3-phenyl-propyl)-nicotinamide
*N*-(1-Methyl-3-phenyl-propyl)-6-[4-(2-p-tolyl-acetyl)-piperazin-1-yl]-nicotinamide
*N*-[2-(3-Chlorophenyl)-ethyl]-6-[4-(2-phenyl-butyryl)-piperazin-1-yl]-nicotinamide
6-{4-[2-(2-Chloro-6-fluoro-phenyl)-acetyl]-piperazin-1-yl}-*N*-phenethylnicotinamide
*N*-(3-Phenyl-propyl)-6-[4-(2-o-tolyl-acetyl)-piperazin-1-yl]-nicotinamide
6-{4-[2-(2-Chloro-6-fluoro-phenyl)-acetyl]-piperazin-1-yl}-*N*-[2-(3-chloro-phenyl)-ethyl]-nicotinamide
6-{4-[2-(4-Chlorophenyl)-propionyl]-piperazin-1-yl}-*N*-(3-phenyl-propyl)-nicotinamide
6-{4-[2-(4-Chlorophenyl)-propionyl]-piperazin-1-yl}-*N*-(1-methyl-3-phenylpropyl)-nicotinamide
*N*-[2-(3-Chlorophenyl)-ethyl]-6-{4-[2-(4-chloro-phenyl)-propionyl]-piperazin-1-yl}-nicotinamide
*N*-[2-(3-Chlorophenyl)-ethyl]-6-[4-(2-p-tolyl-acetyl)-piperazin-1-yl]-nicotinamide
*N*-Phenethyl-6-[4-(2-p-tolyl-acetyl)-piperazin-1-yl]-nicotinamide
*N*-(3-Phenyl-propyl)-6-[4-(2-p-tolyl-acetyl)-piperazin-1-yl]-nicotinamide
*N*-(1-Methyl-3-phenyl-propyl)-6-[4-(2-phenyl-butyryl)-piperazin-1-yl]-nicotinamide
6-{4-[2-(4-Chlorophenyl)-propionyl]-piperazin-1-yl}-*N*-(4-phenyl-butyl)-nicotinamide
*N*-(4-Phenyl-butyl)-6-[4-(2-phenyl-butyryl)-piperazin-1-yl]-nicotinamide
6-{4-[2-(2-Chloro-6-fluoro-phenyl)-acetyl]-piperazin-1-yl}-*N*-(1-methyl-3-phenylpropyl)-nicotinamide
6-{4-[3-(3,4-Difluoro-phenyl)-propionyl]-piperazin-1-yl}-*N*-(3-phenyl-propyl)-nicotinamide
*N*-Phenethyl-6-[4-(2-o-tolyl-acetyl)-piperazin-1-yl]-nicotinamide
6-{4-[3-(3,4-Difluoro-phenyl)-propionyl]-piperazin-1-yl}-*N*-(1-methyl-3-phenylpropyl)-nicotinamide;
*N*-(4-Phenyl-butyl)-6-[4-(2-o-tolyl-acetyl)-piperazin-1-yl]-nicotinamide;
*N*-(3-Imidazol-1-yl-propyl)-6-[4-(2-phenyl-butyryl)-piperazin-1-yl]-nicotinamide;
6-{4-[2-(2-Chloro-6-fluoro-phenyl)-acetyl]-piperazin-1-yl}-*N*-(4-phenyl-butyl)-nicotinamide;
6-{4-[2-(4-Chlorophenyl)-propionyl]-piperazin-1-yl}-*N*-[2-(3H-imidazol-4-yl)-ethyl]-nicotinamide;
6-{4-[2-(2-Chloro-6-fluoro-phenyl)-acetyl]-piperazin-1-yl}-*N*-(3-imidazol-1-yl-propyl)-nicotinamide;
*N*-[2-(3H-Imidazol-4-yl)-ethyl]-6-[4-(2-p-tolyl-acetyl)-piperazin-1-yl]-nicotinamide;
6-{4-[2-(4-Chlorophenyl)-propionyl]-piperazin-1-yl}-*N*-(3-imidazol-1-yl-propyl)-nicotinamide;
*N*-(2-Ethylsulfanyl-ethyl)-6-[4-(2-phenyl-butyryl)-piperazin-1-yl]-nicotinamide;
*N*-(1-Methyl-3-phenyl-propyl)-6-[4-(2-o-tolyl-acetyl)-piperazin-1-yl]-nicotinamide;
6-{4-[3-(3,4-Difluoro-phenyl)-propionyl]-piperazin-1-yl}-*N*-phenethyl-nicotinamide;
*N*-Phenethyl-6-[4-(2-phenyl-butyryl)-piperazin-1-yl]-nicotinamide;
*N*-(Tetrahydro-furan-2-ylmethyl)-6-[4-(2-p-tolyl-acetyl)-piperazin-1-yl]-nicotinamide;
6-{4-[2-(4-Chlorophenyl)-propionyl]-piperazin-1-yl}-*N*-(tetrahydro-furan-2-ylmethyl)-nicotinamide;
*N*-(4-Phenyl-butyl)-6-[4-(2-p-tolyl-acetyl)-piperazin-1-yl]-nicotinamide;
6-{4-[3-(3,4-Difluoro-phenyl)-propionyl]-piperazin-1-yl}-*N*-(tetrahydro-furan-2-ylmethyl)-nicotinamide;
*N*-(Tetrahydro-furan-2-ylmethyl)-6-[4-(2-o-tolyl-acetyl)-piperazin-1-yl]-nicotinamide;
6-[4-(2-Phenyl-butyryl)-piperazin-1-yl]-*N*-(tetrahydro-furan-2-ylmethyl)-nicotinamide;
6-{4-[3-(3,4-Difluoro-phenyl)-propionyl]-piperazin-1-yl}-*N*-[2-(3H-imidazol-4-yl)-ethyl]-nicotinamide;
*N*-[2-(3H-Imidazol-4-yl)-ethyl]-6-[4-(2-o-tolyl-acetyl)-piperazin-1-yl]-nicotinamide;
*N*-(3-Imidazol-1-yl-propyl)-6-[4-(2-o-tolyl-acetyl)-piperazin-1-yl]-nicotinamide;
6-{4-[3-(3,4-Difluoro-phenyl)-propionyl]-piperazin-1-yl}-*N*-(3-imidazol-1-yl-propyl)-nicotinamide;
*N*-[2-(3H-imidazol-4-yl)-ethyl]-6-[4-(2-phenyl-butyryl)-piperazin-1-yl]-nicotinamide;
*N*-(3-Imidazol-1-yl-propyl)-6-[4-(2-p-tolyl-acetyl)-piperazin-1-yl]-nicotinamide;
6-{4-[2-(2-Chloro-6-fluoro-phenyl)-acetyl]-piperazin-1-yl}-*N*-(tetrahydro-furan-2-ylmethyl)-nicotinamide;
6-{4-[2-(2-Chloro-6-fluoro-phenyl)-acetyl]-piperazin-1-yl}-*N*-[2-(3H-imidazol-4-yl)-ethyl]-nicotinamide;
*N*-(4-Phenyl-butyl)-6-[4-(2-phenyl-cyclopropanecarbonyl)-piperazin-1-yl]-nicotinamide;
*N*-(1-Methyl-3-phenyl-propyl)-6-[4-(2-phenyl-cyclopropanecarbonyl)-piperazin-1-yl]-nicotinamide
*N*-Phenethyl-6-[4-(2-phenyl-cyclopropanecarbonyl)-piperazin-1-yl]-nicotinamide;
6-[4-(2-Phenyl-cyclopropanecarbonyl)-piperazin-1-yl]-*N*-(tetrahydro-furan-2-ylmethyl)-nicotinamide;
*N*-[2-(3H-Imidazol-4-yl)-ethyl]-6-[4-(2-phenyl-cyclopropanecarbonyl)-piperazin-1-yl]-nicotinamide;
*N*-(3-imidazol-1-yl-propyl)-6-[4-(2-phenyl-cyclopropanecarbonyl)-piperazin-1-yl]-nicotinamide;
*N*-(3-Ethoxy-propyl)-6-[4-(2-phenyl-cyclopropanecarbonyl)-piperazin-1-yl]-nicotinamide
*N*-(2-Ethylsulfanyl-ethyl)-6-[4-(2-phenyl-cyclopropanecarbonyl)-piperazin-1-yl]-nicotinamide;
*N*-(1,3-Dimethylbutyl)-6-[4-(2-phenyl-cyclopropanecarbonyl)-piperazin-1-yl]-nicotinamide;
*N*-(3-Methyl-butyl)-6-[4-(2-phenyl-cyclopropanecarbonyl)-piperazin-1-yl]-nicotinamide;
*N*-(3-Methoxy-propyl)-6-[4-(2-phenyl-cyclopropanecarbonyl)-piperazin-1-yl]-nicotinamide;
*N*-(3-Butoxy-propyl)-6-[4-(2-phenyl-cyclopropanecarbonyl)-piperazin-1-yl]-nicotinamide;
*N*-Pentyl-6-[4-(2-phenyl-cyclopropanecarbonyl)-piperazin-1-yl]-nicotinamide;
*N*-(3-Dimethylamino-propyl)-6-[4-(2-phenyl-cyclopropanecarbonyl)-piperazin-1-yl]-nicotinamide;
*N*-(3-isopropoxy-propyl)-6-[4-(2-phenyl-cyclopropanecarbonyl)-piperazin-1-yl]-nicotinamide;
*N*-(1-Methyl-butyl)-6-[4-(2-phenyl-cyclopropanecarbonyl)-piperazin-1-yl]-nicotinamide;
*N*-Butyl-6-[4-(2-phenyl-cyclopropanecarbonyl)-piperazin-1-yl]-nicotinamide;
*N*-Hexyl-6-[4-(2-phenyl-cyclopropanecarbonyl)-piperazin-1-yl]-nicotinamide; and
*N*-(2-Methyl-butyl)-6-[4-(2-phenyl-cyclopropanecarbonyl)-piperazin-1-yl]-nicotinamide..

### EXAMPLE 10

The identification of compounds of the invention as SCD inhibitors was readily accomplished using the SCD enzymes and microsomal assay procedure described in Brownlie *et al,* PCT published patent application, WO 01/62954. Briefly, mouse or human liver microsomes (induced for SCD1 expression and containing cytochrome b₅ and cytochrome b₅ reductase) and NADH are suspended in buffer with a compound of the invention, and reaction is initiated by addition of 0.025 mM tritiated stearoyl-CoA. This ligand is tritiated at the C9 and C10 position only. The reaction is allowed to proceed for between 5 and 20 minutes at ambient temperature, whereupon it is halted by addition of acid. Activated charcoal is then added, mixed, and centrifuged to separate labeled substrate from labeled water. An aliquot of supernatant is than tested for radioactivity using liquid scintillation counting. This is taken as a measure of delta-9 desaturase activity.

Data showing inhibition of SCD by compounds of the invention when tested by this assay are presented below in Table 3, which sets forth the % remaining SCD activity at 10 µM of test compound in the indicated assay. Compounds were tested in mouse liver microsomes, 100 µg, preincubation with the test compound for 15 minutes at ambient temperature, a desaturation period for 15 minutes at ambient temperature, utilizing the large volume bench-top tube method.

**TABLE 3. SCD INHIBITORY ACTIVITY FOR SELECTED COMPOUNDS.**

| Compound | % Activity 10 µM | Mol. weight |
|---|---|---|
| 6-[4-(2-Bromo-5-methoxy-benzoyl)-piperazin-1-yl]-*N*-(2-ethylsulfanylethyl)-nicotinamide | 1.4 | 516.15 |
| *N*-(3-Phenyl-propyl)-6-[4-(2-trifluoromethyl-benzoyl)-piperazin-1-yl]-nicotinamide | 1.3 | 458.13 |
| 6-[4-(2-Naphthalen-2-yl-acetyl)-piperazin-1-yl]-*N*-pentylnicotinamide | 0.7 | 496.21 |
| 6-[4-(3-Cyclohexyl-propionyl)-piperazin-1-yl]-*N*-(3-methylbutyl)-nicotinamide | 1.3 | 492.12 |
| 6-[4-(2-Bromo-benzoyl)-piperazin-1-yl]-*N*-(3-butoxy-propyl)-nicotinamide | 0.8 | 448.21 |
| *N*-Butyl-6-[4-(2-ethylbutyryl)-piperazin-1-yl]-nicotinamide | 2.0 | 448.14 |
| *N*-(3-Butoxy-propyl)-6-[4-(2,4-dimethylbenzoyl)-piperazin-1-yl]-nicotinamide | 0.7 | 482.19 |
| *N*-(3-Methyl-butyl)-6-[4-(2-o-tolyl-acetyl)-piperazin-1-yl]-nicotinamide | 3.2 | 442.27 |
| 6-[4-(2,4-Dichloro-benzoyl)-piperazin-1-yl]-*N*-(1-methyl-3-phenyl-propyl)-nicotinamide | 2.7 | 434.19 |
| 6-[4-(2-Bromo-5-methoxy-benzoyl)-piperazin-1-yl]-*N*-phenethylnicotinamide | 1.1 | 496.14 |
| 6-[4-(2-Ethylbutyryl)-piperazin-1-yl]-*N*-(3-methylbutyl)-nicotinamide | 1.7 | 448.21 |
| 6-[4-(2,4-Dichloro-benzoyl)-piperazin-1-yl]-*N*-pentyl-nicotinamide | 1.2 | 482.13 |
| *N*-(1,3-Dimethylbutyl)-6-[4-(2-trifluoromethyl-benzoyl)-piperazin-1-yl]-nicotinamide | 1.8 | 458.13 |
| 6-[4-(3-Cyclohexyl-propionyl)-piperazin-1-yl]-*N*-(2-ethylsulfanyl-ethyl)-nicotinamide | 1.4 | 448.14 |
| 6-[4-(2-Bromo-benzoyl)-piperazin-1-yl]-*N*-(3-methoxy-propyl)-nicotinamide | 1.2 | 462.22 |
| 6-[4-(3-Cyclohexyl-propionyl)-piperazin-1-yl]-*N*-(1-methylbutyl)-nicotinamide | 2.1 | 430.24 |
| 6-[4-(2-Bromo-benzoyl)-piperazin-1-yl]-*N*-(3-ethoxy-propyl)-nicotinamide | 1.0 | 537.5 |
| *N*-(1-Methyl-3-phenyl-propyl)-6-[4-(3-methyl-thiophene-2-carbonyl)-piperazin-1-yl]-nicotinamide | 3.4 | 469 |
| 6-[4-(2-Phenyl-butyryl)-piperazin-1-yl]-*N*-(3-phenyl-propyl)-nicotinamide | 3.6 | 445.4 |
| *N*-(2-Ethylsulfanyl-ethyl)-6-[4-(2-p-tolylacetyl)-piperazin-1-yl]-nicotinamide | 1.8 | 470.6 |
| *N*-(3-Phenyl-propyl)-6-[4-(2-trifluoromethyl-benzoyl)-piperazin-1-yl]-nicotinamide | 3.9 | 408.5 |

## Claims

1. Use of a compound of formula (I): 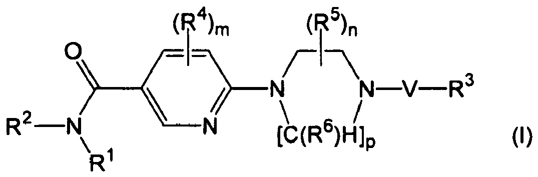 wherein:
m is 1, 2 or 3;
n is 1, 2, 3 or 4;
p is 2, 3 or 4;
V is -C(O)-, -S(O)- or -S(O)₂-;
R¹ is hydrogen, alkyl, alkenyl, aryl, aralkyl, aralkenyl or cycloalkyl;
R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, aralkyl, where the aryl radical may be optionally substituted, aralkenyl, where the aryl radical may be optionally substituted, optionally substituted cycloalkyl, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
R³ is selected from the group consisting of hydrogen, -R⁹-OR⁸, -R⁹-N(R⁸)₂, alkyl, alkenyl, optionally substituted aryl, aralkyl, where the aryl radical may be optionally substituted, aralkenyl, where the aryl radical may be optionally substituted, optionally substituted cycloalkyl, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
each R⁴ is independently hydrogen, alkyl, alkenyl, halo, haloalkyl, aryl, cyano, nitro, -R⁹-OR⁸, -R⁹-N(R⁸)₂ or -S(O)ₜR¹⁰ (where t is 0, 1 or 2);
each R⁵ and R⁶ is independently hydrogen, oxo, alkyl, alkenyl, halo, haloalkyl or aryl;
or one R⁵ and one R⁶ may together form an straight or branched alkylene bridge;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl;
each R⁹ is independently a direct bond or a straight or branched alkylene or alkenylene chain; and
R¹⁰ is alkyl, alkenyl, haloalkyl, cycloalkyl, cycloalkylalkyl, aryl, aralkyl, heterocyclyl, heterocyclylalkyl, heteroaryl or heteroarylalkyl;
as a single stereoisomer, a mixture of stereoisomers, a racemic mixture thereof of stereoisomers, or as a tautomer;
or as a pharmaceutically acceptable salt, solvate, polymorph or prodrug, selected from among ester and amide derivatives of hydroxyl, carboxy, mecapto or amino functional groups in the compounds of formula (I), thereof for the manufacture of a medicament for treating an stearoyl- CoA desaturase (SCD) mediated disease or condition in a mammal, wherein the disease or condition is selected from the group consisting of Type II diabetes, impaired glucose tolerance, insulin resistance, hypertension, obesity, hypertriglyceridemia, low HDL, lipidemia, dyslipidemia, microalbuminemia, hyperuricaemia, hyperleptinaemia, metabolic syndrome, and any combination of these; and
wherein the optional substituent is one or more substituents independently selected from the group consisting of alkyl, alkenyl, halo, haloalkyl, haloalkenyl, cyano, nitro, aryl, aralkyl, cycloalkyl, cycloalkylalkyl, heterocylyl, heterocyclylalkyl, heteroaryl, heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-S(O)ₜOR¹⁰ (where t is 1 or 2), -R⁹-S(O)ₜR¹⁰ (where t is 0, 1 or 2), and -R⁹-S(O)ₜN(R⁸)₂ (where t is 1 or 2).

2. The use of the compound of Claim 1 wherein the compound of formula (I) is a compound wherein:
m is 1 or 2;
n is 1 or 2;
p is 2 or 3;
V is -C(O)- or -S(O)₂-;
R¹ is hydrogen or alkyl;
R² is selected from the group consisting of hydrogen, -R⁷-OR⁸, -R⁷-N(R⁸)₂, -A⁷-S(O)ₜA¹⁰(where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl, wherein the optionally substituted groups are as defined in Claim 1;
R³ is alkyl, alkenyl or -R⁹-N(R⁸)₂;
each R⁴ is independently hydrogen, alkyl, alkenyl, halo, haloalkyl, aryl or -R⁹-OR⁸;
each R⁵ and R⁶ is independently hydrogen, oxo, alkyl, alkenyl, halo, haloalkyl or aryl;
or one R⁵ and one R⁶ may together form an straight or branched alkylene bridge;
each A⁷ is independently a straight or branched alkylene or alkenylene chain;
each R³ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl;
each R⁹ is independently a direct bond or a straight or branched alkylene chain; and
R¹⁰ is alkyl, aryl or aralkyl.

3. The use of the compound of Claim 2 wherein the compound of formula (I) is a compound wherein:
m is 1 or 2;
n is 1 or 2;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl, wherein the optionally substituted groups are as defined in Claim 1;
R³ is alkyl;
each R⁴ is independently hydrogen, alkyl, halo, or haloalkyl;
each R⁵ and R⁶ is independently hydrogen, oxo, alkyl, halo or haloalkyl;
each R⁷ is a straight or branched alkylene chain;
each R⁸ is independently hydrogen, alkyl, haloalkyl, cycloalkyl, cycloalkylalkyl, aryl and aralkyl; and
R¹⁰ is alkyl, aryl or aralkyl.

4. The use of the compound of Claim 3 wherein the compound of formula (I) is a compound wherein:
m is 1;
n is 1;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2) or alkyl;
R³ is alkyl;
R⁴ is hydrogen;
R⁵ is hydrogen;
each R⁶ is hydrogen;
R⁷ is a straight or branched alkylene chain;
R⁸ is hydrogen or alkyl; and
R¹⁰ is alkyl, aryl or aralkyl.

5. The use of the compound of Claim 4 wherein the compound of formula (I) is selected from the group consisting of the following:
*N*-(3-Ethoxy-propyl)-6-[4-(2-ethylbutyryl)-piperazin-1-yl]-nicotinamide;
*N*-(3-Ethoxy-propyl)-6-(4-pentanoyl-piperazin-1-yl)-nicotinamide;
*N*-(3-Ethoxy-propyl)-6-[4-(3-methyl-pentanoyl)-piperazin-1-yl]nicotinamide;
*N*-(3-Butoxy-propyl)-6-(4-pentanoyl-piperazin-1-yl)-nicotinamide;
*N*-(3-Methyl-butyl)-6-[4-(3-methyl-pentanoyl)-piperazin-1-yl]-nicotinamide;
6-[4-(3-Methyl-pentanoyl)-piperazin-1-yl]-*N*-pentyl-nicotinamide;
*N*-Butyl-6-[4-(3-methyl-pentanoyl)-piperazin-1-yl]-nicotinamide;
*N*-Butyl-6-[4-(2-ethylbutyryl)-piperazin-1-yl]-nicotinamide;
*N*-(3-Methyl-butyl)-6-(4-pentanoyl-piperazin-1-yl)-nicotinamide;
*N*-(2-Ethylsulfanyl-ethyl)-6-(4-pentanoyl-piperazin-1-yl)-nicotinamide;
*N*-(3-Methoxy-propyl)-6-[4-(3-methyl-pentanoyl)-piperazin-1-yl]-nicotinamide;
6-(4-Pentanoyl-piperazin-1-yl)-*N*-pentyl-nicotinamide;
*N*-Hexyl-6-[4-(3-methyl-pentanoyl)-piperazin-1-yl]-nicotinamide;
*N*-(1,3-Dimethylbutyl)-6-[4-(3-methyl-pentanoyl)-piperazin-1-yl]-nicotinamide;
*N*-(1-Methyl-butyl)-6-(4-pentanoyl-piperazin-1-yl)-nicotinamide;
*N*-(3-Dimethylamino-propyl)-6-[4-(2-ethylbutyryl)-piperazin-1-yl]-nicotinamide;
*N*-(3-Methoxy-propyl)-6-(4-pentanoyl-piperazin-1-yl)-nicotinamide;
*N*-(1,3-Dimethylbutyl)-6-(4-pentanoyl-piperazin-1-yl)-nicotinamide;
*N*-(2-Methyl-butyl)-6-(4-pentanoyl-piperazin-1-yl)-nicotinamide;
*N*-(3-Butoxy-propyl)-6-[4-(2-ethylbutyryl)-piperazin-1-yl]-nicotinamide;
6-[4-(2-Ethylbutyryl)-piperazin-1-yl]-*N*-(3-methylbutyl)-nicotinamide;
*N*-Butyl-6-(4-pentanoyl-piperazin-1-yl)-nicotinamide;
6-[4-(2-Ethylbutyryl)-piperazin-1-yl]-*N*-(2-methylbutyl)-nicotinamide;
*N*-(3-Isopropoxy-propyl)-6-(4-pentanoyl-piperazin-1-yl)-nicotinamide;
*N*-(2-Ethylsulfanyl-ethyl)-6-[4-(3-methyl-pentanoyl)-piperazin-1-yl]-nicotinamide;
6-[4-(2-Ethylbutyryl)-piperazin-1-yl]-*N*-(2-ethylsulfanyl-ethyl)-nicotinamide;
6-[4-(2-Ethylbutyryl)-piperazin-1-yl]-*N*-hexyl-nicotinamide;
6-[4-(2-Ethylbutyryl)-piperazin-1-yl]-*N*-(1-methylbutyl)-nicotinamide;
*N*-(2-Methyl-butyl)-6-[4-(3-methyl-pentanoyl)-piperazin-1-yl]-nicotinamide;
*N*-(1,3-Dimethylbutyl)-6-[4-(2-ethylbutyryl)-piperazin-1-yl]-nicotinamide;
6-[4-(2-Ethylbutyryl)-piperazin-1-yl]-*N*-pentyl-nicotinamide;
*N*-(3-Isopropoxy-propyl)-6-[4-(3-methyl-pentanoyl)-piperazin-1-yl]-nicotinamide;
*N*-(3-Dimethylamino-propyl)-6-[4-(3-methyl-pentanoyl)-piperazin-1-yl]-nicotinamide;
6-[4-(2-Ethylbutyryl)-piperazin-1-yl]-*N*-(3-methoxy-propyl)-nicotinamide;
6-[4-(2-Ethylbutyryl)-piperazin-1-yl]-*N*-(3-isopropoxy-propyl)-nicotinamide;
*N*-(3-Butoxy-propyl)-6-[4-(3-methyl-pentanoyl)-piperazin-1-yl]-nicotinamide;
*N*-(1-Methyl-butyl)-6-[4-(3-methyl-pentanoyl)-piperazin-1-yl]-nicotinamide;
*N*-Hexyl-6-(4-pentanoyl-piperazin-1-yl)-nicotinamide; and
*N*-(3-Dimethylamino-propyl)-6-(4-pentanoyl-piperazin-1-yl)-nicotinamide.

6. The use of the compound of Claim 2 wherein the compound of formula (I) is a compound wherein:
m is 1 or 2;
n is 1 or 2;
p is 2 or 3;
V is -C(O)- or -S(O)₂-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl, wherein the optionally substituted groups are as defined in Claim 1;
R³ is alkyl or -R⁷-N(R⁸)₂;
each R⁴ is independently hydrogen, alkyl, halo, or haloalkyl; and
each R⁵ and R⁶ is independently hydrogen, oxo, alkyl, alkenyl, halo, haloalkyl or aryl;
or one R⁵ and one R⁶ may together form an straight or branched alkylene bridge;
A⁷ is a direct bond; and
each R⁸ is independently hydrogen or alkyl.

7. The use of the compound of Claim 6 wherein the compound of formula (I) is a compound wherein:
m is 1;
n is 1;
p is 2 or 3;
V is -C(O)- or -S(O)₂-;
R¹ is hydrogen or alkyl;
R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl, wherein the optionally substituted groups are as defined in Claim 1;
R³ is alkyl or -R⁷-N(R⁸)₂;
R⁴ is hydrogen, alkyl, halo or haloalkyl; and
each R⁵ and R⁶ is independently hydrogen, oxo, alkyl, halo or haloalkyl;
or one R⁵ and one R⁶ may together form a methylene bridge;
R⁷ is a direct bond; and
each R⁸ is independently hydrogen or alkyl.

8. The use of the compound of Claim 7 wherein the compound of formula (I) is selected from the group consisting of the following:
6-[4-(2-Ethylbutyryl)-piperazin-1-yl]-*N*-(3-phenyl-propyl)-nicotinamide;
6-[4-(4-Methyl-hexanoyl)-piperazin-1-yl]-*N*-(3-phenyl-propyl)-nicotinamide;
*N*-[2-(3-Chlorophenyl)-1-methylethyl]-6-[4-(2-ethylbutyryl)-piperazin-1-yl]-nicotinamide;
*N*-[2-(3-Chlorophenyl)-ethyl]-6-[4-(3-methyl-pentanoyl)-piperazin-1-yl]-nicotinamide;
*N*-[2-(3-Chlorophenyl)-ethyl]-6-[4-(2-ethylbutyryl)-piperazin-1-yl]-nicotinamide;
6-(4-Pentanoyl-piperazin-1-yl)-*N*-(4-phenyl-butyl)-nicotinamide;
*N*-[2-(3-Chlorophenyl)-ethyl]-6-(4-pentanoyl-piperazin-1-yl)-nicotinamide;
6-[4-(2-Ethylbutyryl)-piperazin-1-yl]-*N*-phenethyl-nicotinamide;
6-[4-(3-Methyl-pentanoyl)-piperazin-1-yl]-*N*-phenethyl-nicotinamide;
6-(4-Pentanoyl-piperazin-1-yl)-*N*-(3-phenyl-propyl)nicotinamide;
*N*-(1-Methyl-3-phenyl-propyl)-6-(4-pentanoyl-piperazin-1-yl)-nicotinamide;
6-[4-(3-Methyl-pentanoyl)-piperazin-1-yl]-*N*-(tetrahydro-furan-2-ylmethyl)-nicotinamide;
6-[4-(2-Ethylbutyryl)-piperazin-1-yl]-*N*-(tetrahydro-furan-2-ylmethyl)-nicotinamide;
6-(4-Pentanoyl-piperazin-1-yl)-*N*-(tetrahydro-furan-2-ylmethyl)-nicotinamide;
6-[4-(2-Ethylbutyryl)-piperazin-1-yl]-*N*-(4-phenyl-butyl)-nicotinamide;
*N*-(3-Imidazol-1-yl-propyl)-6-(4-pentanoyl-piperazin-1-yl)-nicotinamide;
6-[4-(2-Ethylbutyryl)-piperazin-1-yl]-*N*-(3-imidazol-1-yl-propyl)-nicotinamide
*N*-[2-(3H-Imidazol-4-yl)-ethyl]-6-(4-pentanoyl-piperazin-1-yl)-nicotinamide;
*N*-[2-(3H-imidazol-4-yl)-ethyl]-6-[4-(3-methyl-pentanoyl)-piperazin-1-yl]-nicotinamide;
6-(4-Pentanoyl-piperazin-1-yl)-*N*-phenethyl-nicotinamide;
6-[4-(2-Ethylbutyryl)-piperazin-1-yl]-*N*-[2-(3H-imidazol-4-yl)-ethyl]-nicotinamide;
*N*-(3-Imidazol-1-yl-propyl)-6-[4-(3-methyl-pentanoyl)-piperazin-1-yl]-nicotinamide;
6-[4-(2-Ethylbutyryl)-[1,4]diazepan-1-yl]-*N*-(3-phenyl-propyl)-nicotinamide;
6-[4-(2-Ethylbutyryl)-3-methyl-piperazin-1-yl]-*N*-(3-phenyl-propyl)-nicotinamide;
6-[5-(2-Ethylbutyryl)-2,5-diaza-bicyclo[2.2.1]hept-2-yl]-*N*-(3-phenyl-proyl)-nicotinamide;
6-[4-(Butane-1-sulfonyl)-piperazine-1-yl]-*N*-(3-phenyl-propyl)-nicotinamide;
4-[5-(3-Phenyl-propylcarbamoyl)-pyridin-2-yl]-piperazine-1-carboxylic acid butylamide.

9. The use of the compound of Claim 1 wherein the compound of formula (I) is a compound wherein:
m is 1 or 2;
n is 1 or 2;
p is 2;
V is -C(O)-;
R¹ is hydrogen or alkyl;
R² is selected from the group consisting of hydrogen, -R⁷-OR⁸, -R⁷-N(R⁸)₂, R⁷-S(O)ₜR¹⁰(where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl, wherein the optionally substituted groups are as defined in Claim 1;
R³ is optionally substituted cycloalkyl, optionally substituted cycloalkylalkyl, or cycloalkylalkenyl, wherein the optionally substituted groups are as defined in Claim 1;
each R⁴ is independently hydrogen, alkyl, alkenyl, halo, haloalkyl, aryl or -R⁹-OR⁸;
each R⁵ and R⁶ is independently hydrogen, oxo, alkyl, alkenyl, halo, haloalkyl or aryl;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl;
R⁹ is a direct bond or a straight or branched alkylene chain; and
R¹⁰ is alkyl, aryl or aralkyl.

10. The use of the compound of Claim 9 wherein the compound of formula (I) is a compound wherein:
m is 1 or 2;
n is 1 or 2;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl, wherein the optionally substituted groups are as defined in Claim 1;
R³ is optionally substituted cycloalkyl or optionally substituted cycloalkylalkyl, wherein the optionally substituted groups are as defined in Claim 1;
each R⁴ is independently hydrogen, alkyl, halo, or haloalkyl;
each R⁵ and R⁶ is independently hydrogen, oxo, alkyl, halo or haloalkyl;
each R⁷ is a straight or branched alkylene chain;
each R⁸ is independently hydrogen, alkyl, haloalkyl, cycloalkyl, cycloalkylalkyl, aryl and aralkyl; and
A¹⁰ is alkyl, aryl or aralkyl.

11. The use of the compound of Claim 10 wherein the compound of formula (I) is a compound wherein:
m is 1;
n is 1;
p is 2;
V is -C(O)-;
R¹ is hydrogen or alkyl;
R² is selected from the group consisting of -R⁷-R⁸, -R⁷-N(R⁸)₂, -R⁷-S(O)ₜR¹⁰ (where t is 0 to 2) or alkyl;
R³ is optionally substituted cycloalkyl or optionally substituted cycloalkylalkyl;
R⁴ is hydrogen;
R⁵ is hydrogen;
each R⁶ is hydrogen;
R⁷ is a straight or branched alkylene chain;
R⁸ is hydrogen or alkyl; and
A¹⁰ is alkyl, aryl or aralkyl.

12. The use of the compound of Claim 11 wherein the compound of formula (I) is selected from the group consisting of the following:
6-[4-(2-Cyclohexyl-acetyl)-piperazin-1-yl]-*N*-(3-ethoxy-propyl)-nicotinamide;
*N*-Butyl-6-[4-(2-cyclohexyl-acetyl)-piperazin-1-yl]-nicotinamide;
6-[4-(3-Cyclohexyl-propionyl)-piperazin-1-yl]-*N*-(3-methylbutyl)-nicotinamide;
6-[4-(3-Cyclohexyl-propionyl)-piperazin-1-yl]-*N*-(3-methoxy-propyl)-nicotinamide;
*N*-Pentyl-6-[4-(3-trifluoromethyl-benzoyl)-piperazin-1-yl]-nicotinamide;
6-[4-(2-Cyclohexyl-acetyl)-piperazin-1-yl]-*N*-(2-methylbutyl)-nicotinamide;
6-[4-(3-Cyclohexyl-propionyl)-piperazin-1-yl]-*N*-(3-isopropoxy-propyl)-nicotinamide;
6-[4-(2-Cyclohexyl-acetyl)-piperazin-1-yl]-*N*-(1,3-dimethylbutyl)-nicotinamide;
6-[4-(2-Cyclohexyl-acetyl)-piperazin-1-yl]-*N*-hexyl-nicotinamide;
6-[4-(2-Cyclohexyl-acetyl)-piperazin-1-yl]-*N*-(1-methylbutyl)-nicotinamide;
6-[4-(2-Cyclohexyl-acetyl)-piperazin-1-yl]-*N*-(3-methylbutyl)-nicotinamide;
6-[4-(2-Cyclohexyl-acetyl)-piperazin-1-yl]-*N*-(2-ethylsulfanyl-ethyl)-nicotinamide;
*N*-(3-Butoxy-propyl)-6-[4-(2-cyclohexyl-acetyl)-piperazin-1-yl]-nicotinamide;
*N*-Butyl-6-[4-(3-cyclohexyl-propionyl)-piperazin-1-yl]-nicotinamide;
6-[4-(2-Cyclohexyl-acetyl)-piperazin-1-yl]-*N-*pentyl-nicotinamide;
6-[4-(3-Cyclohexyl-propionyl)-piperazin-1-yl]-*N*-(2-ethylsulfanyl-ethyl)-nicotinamide;
6-[4-(2-Cyclohexyl-acetyl)-piperazin-1-yl]-*N*-(3-methoxy-propyl)-nicotinamide;
6-[4-(3-Cyclohexyl-propionyl)-piperazin-1-yl]-*N*-(1-methylbutyl)-nicotinamide;
6-[4-(2-Cyclohexyl-acetyl)-piperazin-1-yl]-*N*-(3-isopropoxy-propyl)-nicotinamide;
6-[4-(3-Cyclohexyl-propionyl)-piperazin-1-yl]-*N*-(3-ethoxy-propyl)-nicotinamide;
6-[4-(3-Cyclohexyl-propionyl)-piperazin-1-yl]-*N*-(1,3-dimethylbutyl)-nicotinamide;
6-[4-(3-Cyclohexyl-propionyl)-piperazin-1-yl]-*N*-(2-methylbutyl)-nicotinamide;
6-[4-(3-Cyclohexyl-propionyl)-piperazin-1-yl]-*N*-hexyl-nicotinamide;
6-[4-(3-Cyclohexyl-propionyl)-piperazin-1-yl]-*N*-pentyl-nicotinamide;
6-[4-(3-Cyclohexyl-propionyl)-piperazin-1-yl]-*N*-(3-dimethylamino-propyl)-nicotinamide;
*N*-(3-Ethoxy-propyl)-6-[4-(2-phenyl-cyclopropanecarbonyl)-piperazin-1-yl]-nicotinamide;
*N*-(2-Ethylsulfanyl-ethyl)-6-[4-(2-phenyl-cyclopropanecarbonyl)-piperazin-1-yl]-nicotinamide;
*N*-(1,3-Dimethylbutyl)-6-[4-(2-phenyl-cyclopropanecarbonyl)-piperazin-1-yl]-nicotinamide;
*N*-(3-Methyl-butyl)-6-[4-(2-phenyl-cyclopropanecarbonyl)-piperazin-1-yl]-nicotinamide;
*N*-(3-Methoxy-propyl)-6-[4-(2-phenyl-cyclopropanecarbonyl)-piperazin-1-yl]-nicotinamide;
*N*-(3-Butoxy-propyl)-6-[4-(2-phenyl-cyclopropanecarbonyl)-piperazin-1-yl]-nicotinamide;
*N*-Pentyl-6-[4-(2-phenyl-cyclopropanecarbonyl)-piperazin-1-yl]-nicotinamide;
*N*-(3-Dimethylamino-propyl)-6-[4-(2-phenyl-cyclopropanecarbonyl)-piperazin-1-yl]-nicotinamide;
*N*-(3-Isopropoxy-propyl)-6-[4-(2-phenyl-cyclopropanecarbonyl)-piperazin-1-yl]-nicotinamide;
*N*-(1-Methyl-butyl)-6-[4-(2-phenyl-cyclopropanecarbonyl)-piperazin-1-yl]-nicotinamide;
*N*-Butyl-6-[4-(2-phenyl-cyclopropanecarbonyl)-piperazin-1-yl]-nicotinamide;
*N*-Hexyl-6-[4-(2-phenyl-cyclopropanecarbonyl)-piperazin-1-yl]-nicotinamide;
*N*-(2-Methyl-butyl)-6-[4-(2-phenyl-cyclopropanecarbonyl)-piperazin-1-yl]-nicotinamide; and
6-[4-(2-Cyclohexyl-acetyl)-piperazin-1-yl]-*N*-(3-dimethylamino-propyl)-nicotinamide.

13. The use of the compound of Claim 9 wherein the compound of formula (I) is a compound wherein:
m is 1 or 2;
n is 1 or 2;
p is 2;
V is -C(O)-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl, wherein the optionally substituted groups are as defined in Claim 1;
R³ is optionally substituted cycloalkyl or optionally substituted cycloalkylalkyl, wherein the optionally substituted groups are as defined in Claim 1;
each R⁴ is independently hydrogen, alkyl, halo, or haloalkyl; and
each R⁵ and R⁶ is independently hydrogen, oxo, alkyl, halo or haloalkyl.

14. The use of the compound of Claim 13 wherein the compound of formula (I) is a compound wherein:
m is 1;
n is 1;
p is 2;
V is -C(O)-;
R¹ is hydrogen or alkyl;
R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl, wherein the optionally substituted groups are as defined in Claim 1;
R³ is optionally substituted cycloalkyl or optionally substituted cycloalkylalkyl, wherein the optionally substituted groups are as defined in Claim 1;
R⁴ is hydrogen, alkyl, halo or haloalkyl;
R⁵ is independently hydrogen, oxo, alkyl, halo or haloalkyl; and
each R⁶ is independently hydrogen, oxo, alkyl, halo or haloalkyl.

15. The use of the compound of Claim 14 wherein the compound of formula (I) is selected from the group consisting of the following:
6-[4-(2-Phenyl-cyclopropanecarbonyl)-piperazin-1-yl]-*N*-(3-phenyl-propyl)-nicotinamide;
*N*-(4-Phenyl-butyl)-6-[4-(2-phenyl-cyclopropanecarbonyl)-piperazin-1-yl]-nicotinamide;
6-[4-(2-Cyclohexyl-acetyl)-piperazin-1-yl]-*N*-phenethyl-nicotinamide;
*N*-[2-(3-Chlorophenyl)-ethyl]-6-[4-(2-phenyl-cyclopropanecarbonyl)-piperazin-1-yl]-nicotinamide;
*N*-(1-Methyl-3-phenyl-propyl)-6-[4-(2-phenyl-cyclopropanecarbonyl)-piperazin-1-yl]-nicotinamide;
*N*-Phenethyl-6-[4-(2-phenyl-cyclopropanecarbonyl)-piperazin-1-yl]-nicotinamide;
6-[4-(2-Phenyl-cyclopropanecarbonyl)-piperazin-1-yl]-*N*-(tetrahydro-furan-2-ylmethyl)-nicotinamide;
*N*-[2-(3H-Imidazol-4-yl)-ethyl]-6-[4-(2-phenyl-cyclopropanecarbonyl)-piperazin-1-yl]-nicotinamide;
*N*-(3-Imidazol-1-yl-propyl)-6-[4-(2-phenyl-cyclopropanecarbonyl)-piperazin-1-yl]-nicotinamide;
*N*-[2-(3-Chlorophenyl)-ethyl]-6-[4-(3-cyclohexyl-propionyl)-piperazin-1-yl]-nicotinamide;
6-[4-(2-Cyclohexyl-acetyl)-piperazin-1-yl]-*N*-[2-(3H-imidazol-4-yl)-ethyl]-nicotinamide;
6-[4-(2-Cyclohexyl-acetyl)-piperazin-1-yl]-*N*-(3-phenyl-propyl)-nicotinamide;
*N*-[2-(3-Chlorophenyl)-ethyl]-6-[4-(2-cyclohexyl-acetyl)-piperazin-1-yl]-nicotinamide;
6-[4-(3-Cyclohexyl-propionyl)-piperazin-1-yl]-*N*-phenethyl-nicotinamide;
6-[4-(3-Cyclohexyl-propionyl)-piperazin-1-yl]-*N*-(3-phenyl-propyl)-nicotinamide;
6-[4-(3-Methyl-pentanoyl)-piperazin-1-yl]-*N*-(4-phenyl-butyl)-nicotinamide;
6-[4-(2-Cyclohexyl-acetyl)-piperazin-1-yl]-*N*-(4-phenyl-butyl)-nicotinamide;
6-[4-(2-Cyclohexyl-acetyl)-piperazin-1-yl]-*N*-(1-methyl-3-phenyl-propyl)-nicotinamide;
6-[4-(3-Cyclohexyl-propionyl)-piperazin-1-yl]-*N*-[2-(3H-imidazol-4-yl)-ethyl]-nicotinamide;
6-[4-(3-Cyclohexyl-propionyl)-piperazin-1-yl]-*N*-(tetrahydro-furan-2-ylmethyl)-nicotinamide;
6-[4-(2-Cyclohexyl-acetyl)-piperazin-1-yl]-*N*-(3-imidazol-1-yl-propyl)-nicotinamide;
6-[4-(3-Cyclohexyl-propionyl)-piperazin-1-yl]-*N*-(3-imidazol-1-yl-propyl)-nicotinamide; and
6-[4-(2-Cyclohexyl-acetyl)-piperazin-1-yl]-*N*-(tetrahydro-furan-2-ylmethyl)-nicotinamide.

16. The use of the compound of Claim 1 wherein the compound of formula (I) is a compound wherein:
m is 1 or 2;
n is 1 or 2;
p is 2;
V is -C(O)-;
R¹ is hydrogen or alkyl;
R² is selected from the group consisting of hydrogen, -R⁷-OR⁸, -R⁷-N(R⁸)₂, -R⁷-S(O)ₜR¹⁰ (where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl, wherein the optionally substituted groups are as defined in Claim 1;
R³ is optionally substituted aryl, wherein the optionally substituted group is as defined in Claim 1;
each R⁴ is independently hydrogen, alkyl, alkenyl, halo, haloalkyl, aryl or -R⁹-OR⁸;
each R⁵ and R⁶ is independently hydrogen, oxo, alkyl, alkenyl, halo, haloalkyl or aryl;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl;
R⁹ is a direct bond or a straight or branched alkylene chain; and
R¹⁰ is alkyl, aryl or aralkyl.

17. The use of the compound of Claim 16 wherein the compound of formula (I) is a compound wherein:
m is 1 or 2;
n is 1 or 2;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0 to 2), alkyl, alkenyl, optionally substituted cycloalkyl, optionally substituted cycloalkylalkyl or cycloalkylalkenyl, wherein the optionally substituted groups are as defined in Claim 1;
R³ is optionally substituted aryl, wherein the optionally substituted group is as defined in Claim 1;
each R⁴ is independently hydrogen, alkyl, halo, haloalkyl or -R⁹-OR⁸;
each R⁵ and R⁶ is independently hydrogen, oxo, alkyl, halo or haloalkyl;
each R⁷ is a straight or branched alkylene chain;
each R⁸ is independently hydrogen, alkyl, haloalkyl, cycloalkyl, cycloalkylalkyl, aryl and aralkyl;
R⁹ is a direct bond or a straight or branched alkylene chain; and
R¹⁰ is alkyl, aryl or aralkyl.

18. The use of the compound of Claim 17 wherein the compound of formula (I) is a compound wherein:
m is 1 or 2;
n is 1;
pis2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2), alkyl, optionally substituted cycloalkyl or optionally substituted cycloalkylalkyl, wherein the optionally substituted groups are as defined in Claim 1;
R³ is optionally substituted aryl, wherein the optionally substituted group is as defined in Claim 1;
each R⁴ is independently hydrogen, halo, haloalkyl or -R⁹-OR⁸;
R⁵ is hydrogen;
each R⁶ is hydrogen;
R⁷ is a straight or branched alkylene chain;
R⁸ is hydrogen or alkyl;
R⁹ is a direct bond or a straight or branched alkylene chain; and
R¹⁰ is alkyl, aryl or aralkyl.

19. The use of the compound of Claim 18 wherein the compound of formula (I) is selected from the group consisting of the following:
*N*-Butyl-6-[4-(2-mercapto-benzoyl)-piperazin-1-yl]-nicotinamide;
*N*-(3-Methyl-butyl)-6-[4-(2-o-tolyl-acetyl)-piperazin-1-yl]-nicotinamide;
6-{4-[2-(2,4-Dimethyl-phenyl)-acetyl]-piperazin-1-yl}-*N*-(3-methylbutyl)-nicotinamide;
6-[4-(2-Bromo-benzoyl)-piperazin-1-yl]-*N*-(3-ethoxypropyl)nicotinamide;
6-{4-[2-(2-Chloro-6-fluoro-phenyl)-acetyl]-piperazin-1-yl}-*N*-(3-methylbutyl)-nicotinamide;
6-[4-(3,5-Dichloro-benzoyl)-piperazin-1-yl]-*N*-(3-methoxy-propyl)-nicotinamide;
6-[4-(2-Bromo-5-methoxy-benzoyl)-piperazin-1-yl]-*N*-(3-dimethylamino-propyl)-nicotinamide;
6-[4-(2,5-Dichloro-benzoyl)-piperazin-1-yl]-*N*-(3-methylbutyl)-nicotinamide;
6-[4-(2,5-Dichloro-benzoyl)-piperazin-1-yl]-*N*-(3-ethoxy-propyl)-nicotinamide;
*N*-Butyl-6-[4-(3,5-dichloro-benzoyl)-piperazin-1-yl]-nicotinamide;
6-[4-(2,4-Dichloro-benzoyl)-piperazin-1-yl]-*N*-(3-ethoxy-propyl)-nicotinamide;
6-[4-(2,4-Dichloro-benzoyl)-piperazin-1-yl]-*N*-(2-ethylsulfanyl-ethyl)-nicotinamide;
*N*-(3-Ethoxy-propyl)-6-[4-(4-trifluoromethyl-benzoyl)-piperazin-1-yl]-nicotinamide;
*N*-(3-Dimethylamino-propyl)-6-[4-(2,3,4,5-tetrafluoro-benzoyl)-piperazin-1-yl]-nicotinamide;
6-[4-(2-Bromo-5-methoxy-benzoyl)-piperazin-1-yl]-*N*-(2-ethylsulfanyl-ethyl)-nicotinamide;
*N*-(2-Ethylsulfanyl-ethyl)-6-[4-(3-trifluoromethyl-benzoyl)-piperazin-1-yl]-nicotinamide;
6-[4-(2-Bromo-benzoyl)-piperazin-1-yl]-*N*-(3-methylbutyl)-nicotinamide;
*N*-Butyl-6-[4-(2,4-dimethyl-benzoyl)-piperazin-1-yl]-nicotinamide;
6-[4-(3,5-Dichloro-benzoyl)-piperazin-1-yl]-*N*-(3-isopropoxy-propyl)-nicotinamide;
6-[4-(2-Bromo-5-methoxy-benzoyl)-piperazin-1-yl]-*N*-(3-methylbutyl)-nicotinamide;
*N*-(3-Butoxy-propyl)-6-[4-(2,5-dichloro-benzoyl)-piperazin-1-yl]-nicotinamide;
*N*-(3-Methyl-butyl)-6-[4-(3-trifluoromethyl-benzoyl)-piperazin-1-yl]-nicotinamide;
*N*-(3-Methyl-butyl)-6-[4-(2-trifluoromethyl-benzoyl)-piperazin-1-yl]-nicotinamide;
6-[4-(2-Bromo-benzoyl)-piperazin-1-yl]-*N*-(3-butoxy-propyl)-nicotinamide;
6-[4-(2,5-Dichloro-benzoyl)-piperazin-1-yl]-*N*-pentyl-nicotinamide;
*N*-(3*-*Butoxy-propyl)-6-[4-(2,4-dimethyl-benzoyl)-piperazin-1-yl]-nicotinamide;
6-[4-(2-Bromo-5-methoxy-benzoyl)-piperazin-1-yl]-*N*-(3-ethoxy-propyl)-nicotinamide;
6-[4-(2,4-Dichloro-benzoyl)-piperazin-1-yl]-*N*-(3-isopropoxy-propyl)-nicotinamide;
*N*-(3-Ethoxy-propyl)-6-[4-(2-trifluoromethyl-benzoyl)-piperazin-1-yl]-nicotinamide;
*N-*Pentyl-6-[4-(3-trifluoromethyl-benzoyl)-piperazin-1-yl]-nicotinamide;
*N*-Butyl-6-[4-(2-trifluoromethyl-benzoyl)-piperazin-1-yl]-nicotinamide;
*N*-Butyl-6-[4-(2,5-dichloro-benzoyl)-piperazin-1-yl]-nicotinamide;
6-[4-(2-Bromo-benzoyl)-piperazin-1-yl]-*N*-(3-isopropoxy-propyl)-nicotinamide;
6-[4-(2,5-Dichloro-benzoyl)-piperazin-1-yl]-*N*-(3-isopropoxy-propyl)-nicotinamide;
*N*-(3-Butoxy-propyl)-6-[4-(4-trifluoromethyl-benzoyl)-piperazin-1-yl]-nicotinamide;
6-[4-(2,5-Dichloro-benzoyl)-piperazin-1-yl]-*N*-(2-ethylsulfanyl-ethyl)-nicotinamide;
*N*-(3-Butoxy-propyl)-6-[4-(2-trifluoromethyl-banzoyl)-piperazin-1-yl]-nicotinamide;
6-[4-(2,5-Dichloro-benzoyl)-piperazin-1-yl]-*N*-(3-methoxy-propyl)-nicotinamide;
6-[4-(2,4-Dimethyl-benzoyl)-piperazin-1-yl]-*N*-(1,3-dimethylbutyl)-nicotinamide;
6-[4-(2,4-Dichloro-benzoyl)-piperazin-1-yl]-*N-*(2-methylbutyl)-nicotinamide;
6-[4-(3,5-Dichloro-benzoyl)-piperazin-1-yl]-*N*-(2-methylbutyl)-nicotinamide;
6-[4-(2,4-Dimethyl-benzoyl)-piperazin-1-yl]-*N*-(2-ethylsulfanyl-ethyl)-nicotinamide;
*N*-(1,3-Dimethylbutyl)-6-[4-(3-trifluoromethyl-benzoyl)-piperazin-1-yl]-nicotinamide;
6-[4-(2-Bromo-5-methoxy-benzoyl)-piperazin-1-yl]-*N*-(2-methylbutyl)-nicotinamide;
*N*-(3-Dimethylamino-propyl)-6-[4-(2-trifluoromethyl-benzoyl)-piperazin-1-yl]-nicotinamide;
*N*-Hexyl-6-[4-(3-trifluoromethyl-benzoyl)-piperazin-1-yl]-nicotinamide;
6-[4-(2,4-Dichloro-benzoyl)-piperazin-1-yl]-*N*-(1,3-dimethylbutyl)-nicotinamide;
*N*-(3-Isopropoxy-propyl)-6-[4-(3-trifluoromethyl-benzoyl)-piperazin-1-yl]-nicotinamide;
6-[4-(2-Bromo-5-methoxy-benzoyl)-piperazin-1-yl]-*N*-phexyl-nicotinamide;
6-[4-(2-Bromo-benzoyl)-piperazin-1-yl]-*N*-hexyl-nicotinamide;
*N*-(3-Isopropoxy-propyl)-6-[4-(2-trifluoromethyl-benzoyl)-piperazin-1-yl]-nicotinamide;
6-[4-(2-Bromo-5-methoxy-benzoyl)-piperazin-1-yl]-*N*-(3-methoxy-propyl)-nicotinamide;
6-[4-(2-Bromo-benzoyl)-piperazin-1-yl]-*N*-(1-methylbutyl)-nicotinamide;
6-[4-(3,5-Dichloro-benzoyl)-piperazin-1-yl]-*N*-hexyl-nicotinamide;
*N*-(3-Methoxy-propyl)-6-[4-(2-trifluoromethyl-benzoyl)-piperazin-1-yl]-nicotinamide;
6-[4-(2,4-Dichloro-benzoyl)-piperazin-1-yl]-*N*-hexyl-nicotinamide;
*N*-(2-Ethylsulfanyl-ethyl)-6-[4-(2-trifluoromethyl-benzoyl)-piperazin-1-yl]-nicotinamide;
*N*-Pentyl-6-[4-(2-trifluoromethyl-benzoyl)-piperazin-1-yl]-nicotinamide;
*N*-(2-Methyl-butyl)-6-[4-(3-trifluoromethyl-benzoy)-piperazin-1-yl]-nicotinamide;
*N*-(2-Ethylsulfanyl-ethyl)-6-[4-(4-trifluoromethyl-benzoyl)-piperazin-1-yl]-nicotinamide;
6-[4-(2,4-Dichloro-benzoyl)-piperazin-1-yl]-*N*-pentyl-nicotinamide;
6-[4-(2-Bromo-benzoyl)-piperazin-1-yl]-*N*-(2-methylbutyl)-nicotinamide;
6-[4-(2-Bromo-5-methoxy-benzoyl)-piperazin-1-yl]-*N*-(3-butoxy-propyl)-nicotinamide;
*N*-(1,3-Dimethylbutyl)-6-[4-(4-trifluoromethyl-benzoyl)-piperazin-1-yl]-nicotinamide;
6-[4-(3,5-Dichloro-benzoyl)-piperazin-1-yl]-*N*-pentyl-nicotinamide;
*N*-(3-Butoxy-propyl)-6-[4-(3,5-dichloro-benzoyl)-piperazin-1-yl]-nicotinamide;
6-[4-(2-Bromo-5-methoxy-benzoyl)-piperazin-1-yl]-*N*-butyl-nicotinamide;
6-[4-(2-Bromo-benzoyl)-piperazin-1-yl]-*N*-pentyl-nicotinamide;
*N*-(1,3-Dimethylbutyl)-6-[4-(2-trifluoromethyl-benzoyl)-piperazin-1-yl]-nicotinamide;
*N*-(3-Methoxy-propyl)-6-[4-(4-trifluoromethyl-benzoyl)-piperazin-1-yl]-nicotinamide;
6-[4-(2,4-Dimethyl-benzoyl)-piperazin-1-yl]-*N*-(1-methylbutyl)-nicotinamide;
6-[4-(2,4-Dichloro-benzoyl)-piperazin-1-yl]-*N*-(3-methoxy-propyl)-nicotinamide;
*N*-(1-Methyl-butyl)-6-[4-(2-trifluoromethyl-benzoyl)-piperazin-1-yl]-nicotinamide;
*N*-(3-Dimethylamino-propyl)-6-[4-(3-trifluoromethyl-benzoyl)-piperazin-1-yl]-nicotinamide;
6-[4-(2,4-Dichloro-benzoyl)-piperazin-1-yl]-*N*-(1-methylbutyl)-nicotinamide;
6-[4-(2,5-Dichloro-benzoyl)-piperazin-1-yl]-*N*-(1-methylbutyl)-nicotinamide;
*N*-(1-Methyl-butyl)-6-[4-(3-trifluoromethyl-benzoyl)-piperazin-1-yl]-nicotinamide;
6-[4-(2,5-Dichloro-benzoyl)-piperazin-1-yl]-*N*-hexyl-nicotinamide;
6-[4-(2,4-Dimethyl-benzoyl)-piperazin-1-yl]-*N*-(3-methoxy-propyl)-nicotinamide;
6-[4-(3,5-Dichloro-benzoyl)-piperazin-1-yl]-*N*-(1-methylbutyl)-nicotinamide;
6-[4-(2,4-Dichloro-benzoyl)-piperazin-1-yl]-*N*-(3-methylbutyl)-N-nicotinamide;
*N*-(3-Butoxy-propyl)-6-[4-(3-trifluoromethyl-benzoyl)-piperazin-1-yl]-nicotinamide;
6-[4-(2,4-Dimethyl-benzoyl)-piperazin-1-yl]-*N*-(3-isopropoxy-propyl)-nicotinamide;
*N*-(1-Methyl-butyl)-6-[4-(4-trifluoromethyl-benzoyl)-piperazin-1-yl]-nicotinamide;
*N*-Butyl-6-[4-(2,4-dichloro-benzoyl)-piperazin-1-yl]-nicotinamide;
6-[4-(3,5-Dichloro-benzoyl)-piperazin-1-yl]-*N*-(1,3-dimethylbutyl)-nicotinamide;
*N*-Butyl-6-[4-(3-trifluoromethyl-benzoyl)-piperazin-1-yl]-nicotinamide;
6-[4-(2-Bromo-5-methoxy-benzoyl)-piperazin-1-yl]-*N*-(1,3-dimethylbutyl)-nicotinamide;
6-[4-(2,4-Dimethyl-benzoyl)-piperazin-1-yl]-*N*-hexyl-nicotinamide;
6-[4-(2,5-Dichloro-benzoyl)-piperazin-1-yl]-*N*-(1,3-dimethylbutyl)-nicotinamide;
*N*-(3-Butoxy-propyl)-6-[4-(2,4-dichloro-benzoyl)-piperazin-1-yl]-nicotinamide;
*N*-(3-Methoxy-propyl)-6-[4-(3-trifluoromethyl-benzoyl)-piperazin-1-yl]-nicotinamide;
6-[4-(2,4-Dimethyl-benzoyl)-piperazin-1-yl]-*N*-(3-ethoxy-propyl)-nicotinamide;
6-[4-(2,5-Dichloro-benzoyl)-piperazin-1-yl]-*N*-(2-methylbutyl)-nicotinamide;
*N*-Hexyl-6-[4-(4-trifluoromethyl-benzoyl)-piperazin-1-yl]-nicotinamide;
6-[4-(2,4-Dimethyl-benzoyl)-piperazin-1-yl]-*N*-pentyl-nicotinamide;
6-[4-(2-Bromo-benzoyl)-piperazin-1-yl]-*N*-(1,3-dimethylbutyl)-nicotinamide;
*N*-Hexyl-6-[4-(2-trifluoromethyl-benzoyl)-piperazin-1-yl]-nicotinamide;
6-[4-(2-Bromo-benzoyl)-piperazin-1-yl]-*N*-(3-methoxy-propyl)-nicotinamide;
*N*-(2-Methyl-butyl)-6-[4-(4-trifluoromethyl-benzoyl)-piperazin-1-yl]-nicotinamide;
6-[4-(2-Bromo-5-methoxy-benzoyl)-piperazin-1-yl]-*N*-(1-methylbutyl)-nicotinamide;
6-[4-(2,4-Dimethyl-benzoyl)-piperazin-1-yl]-*N*-(2-methylbutyl)-nicotinamide;
6-[4-(2-Bromo-5-methoxy-benzoyl)-piperazin-1-yl]-*N*-pentyl-nicotinamide;
*N*-(2-Methyl-butyl)-6-[4-(2-trifluoromethyl-benzoyl)-piperazin-1-yl]-nicotinamide;
6-[4-(3,5-Dichloro-benzoyl)-piperazin-1-yl]-*N*-(3-methylbutyl)-nicotinamide;
6-[4-(2-Bromo-benzoyl)-piperazin-1-yl]-*N*-(2-ethylsulfanyl-ethyl)-nicotinamide;
6-[4-(3,5-Dichloro-benzoyl)-piperazin-1-yl]-*N*-(3-methoxy-propyl)-nicotinamide;
6-[4-(2-Bromo-5-methoxy-benzoyl)-piperazin-1-yl]-*N*-(3-isopropoxy-propyl)-nicotinamide;
*N*-(3-Butoxy-propyl)-6-[4-(2,3,4,5-tetrafluoro-benzoyl)-piperazin-1-yl]-nicotinamide;
*N*-(1,3-Dimethylbutyl)-6-[4-(2,3,4,5-tetrafluoro-benzoyl)-piperazin-1-yl]-nicotinamide;
*N*-Butyl-6-[4-(2,3,4,5-tetrafluoro-benzoyl)-piperazin-1-yl]-nicotinamide;
*N*-(1-Methyl-butyl)-6-[4-(2,3,4,5-tetrafluoro-benzoyl)-piperazin-1-yl]-nicotinamide;
*N-*(3-Methoxy-propyl)-6-[4-(2,3,4,5-tetrafluoro-benzoyl)-piperazin-1-yl]-nicotinamide;
*N*-(3-Isopropoxy-propyl)-6-[4-(2,3,4,5-tetrafluoro-benzoyl)-piperazin-1-yl]-nicotinamide;
*N*-Pentyl-6-[4-(2,3,4,5-tetrafluoro-benzoyl)-piperazin-1-yl]-nicotinamide;
*N*-(2-Methyl-butyl)-6-[4-(2,3,4,5-tetrafluoro-benzoyl)-piperazin-1-yl]-nicotinamide;
*N*-Hexyl-6-[4-(2,3,4,5-tetrafluoro-benzoyl)-piperazin-1-yl]-nicotinamide;
*N*-(3-Methyl-butyl)-6-[4-(2,3,4,5-tetrafluoro-benzoyl)-piperazin-1-yl]-nicotinamide;
*N*-(2-Ethylsulfanyl-ethyl)-6-[4-(2,3,4,5-tetrafluoro-benzoyl)-piperazin-1-yl]-nicotinamide;
*N*-(3-Ethoxy-propyl)-6-[4-(2,3,4,5-tetrafluoro-benzoyl)-piperazin-1-yl]-nicotinamide;
*N*-(3-Dimethylamino-propyl)-6-[4-(4-trifluoromethyl-benzoyl)-piperazin-1-yl]-nicotinamide;
*N*-(3-Ethoxy-propyl)-6-[4-(3-trifluoromethyl-benzoyl)-piperazin-1-yl]-nicotinamide;
6-[4-(3,5-Dichloro-benzoyl)-piperazin-1-yl]-*N*-(2-ethylsulfanyl-ethyl)-nicotinamide;
*N*-(3-Methyl-butyl)-6-[4-(4-trifluoromethyl-benzoyl)-piperazin-1-yl]-nicotinamide;
*N*-Butyl-6-[4-(4-trifluoromethyl-benzoyl)-piperazin-1-yl]-nicotinamide;
*N*-(3-Isopropoxy-propyl)-6-[4-(4-trifluoromethyl-benzoyl)-piperazin-1-yl]-nicotinamide;
*N*-Pentyl-6-[4-(4-trifluoromethyl-benzoyl)-piperazin-1-yl]-nicotinamide;
6-[4-(3,5-Dichloro-benzoyl)-piperazin-1-yl]-*N*-(3-dimethylamino-propyl)-nicotinamide;
6-[4-(2-Bromo-benzoyl)-piperazin-1-yl]-*N*-(3-dimethylamino-propyl)-nicotinamide;
6-[4-(2,4-Dichloro-benzoyl)-piperazin-1-yl]-*N*-(3-dimethylamino-propyl)-nicotinamide;
6-[4-(2,5-Dichloro-benzoyl)-piperazin-1-yl]-*N*-(3-dimethylamino-propyl)-nicotinamide;
*N*-(3-Dimethylamino-propyl)-6-[4-(2,4-dimethyl-benzoyl)-piperazin-1-yl]-nicotinamide;
*N*-(3-Methyl-butyl)-6-[4-(2-trifluoromethyl-benzoyl)-piperazin-1-yl]-nicotinamide;
*N*-(3-Methyl-butyl)-4-trifluoromethyl-6-[4-(2-trifluoromethyl-benzoyl)-piperazin-1-yl]-nicotinamide;
2-Chloro-5-fluoro-*N*-(3-methylbutyl)-6-[4-(2-trifluoromethyl-benzoyl)-piperazin-1-yl]-nicotinamide;
*N*-(3-Ethoxy-propyl)-6-[4-(2-naphthalen-2-yl-acetyl)-piperazin-1-yl]-nicotinamide;
6-[4-(2-Naphthalen-2-yl-acetyl)-piperazin-1-yl]-*N*-pentyl-nicotinamide;
*N*-Butyl-6-[4-(naphthalene-1-carbonyl)-piperazin-1-yl]-nicotinamide;
*N*-(3-Butoxy-propyl)-6-[4-(2-naphthalen-2-yl-acetyl)-piperazin-1-yl]-nicotinamide;
*N*-Butyl-6-[4-(2-naphthalen-2-yl-acetyl)-piperazin-1-yl]-nicotinamide;
*N*-(3-Butoxy-propyl)-6-[4-(naphthalene-1-carbonyl)-piperazin-1-yl]-nicotinamide;
*N*-(3-Methoxy-propyl)-6-[4-(naphthalene-1-carbonyl)-piperazin-1-yl]-nicotinamide;
*N*-(1-Methyl-butyl)-6-[4-(naphthalene-1-carbonyl)-piperazin-1-yl]-nicotinamide;
6-[4-(Naphthalene-1-carbonyl)-piperazin-1-yl]-*N*-pentyl-nicotinamide;
*N*-(3-Isopropoxy-propyl)-6-[4-(2-naphthalen-2-yl-acetyl)-piperazin-1-yl]-nicotinamide;
*N*-(1-Methyl-butyl)-6-[4-(2-naphthalen-2-yl-acetyl)-piperazin-1-yl]-nicotinamide;
*N*-(3-Methyl-butyl)-6-[4-(2-naphthalen-2-yl-acetyl)-piperazin-1-yl]-nicotinamide;
*N*-(3-Dimethylamino-propyl)-6-[4-(2-naphthalen-2-yl-acetyl)-piperazin-1-yl]-nicotinamide;
*N*-(3-Ethoxy-propyl)-6-[4-(naphthalene-1-carbonyl)-piperazin-1-yl]-nicotinamide;
*N*-Hexyl-6-[4-(naphthalene-1-carbonyl)-piperazin-1-yl]-nicotinamide;
*N*-(2-Ethylsulfanyl-ethyl)-6-[4-(2-naphthalen-2-yl-acetyl)-piperazin-1-yl]-nicotinamide;
*N*-Hexyl-6-[4-(2-naphthalen-2-yl-acetyl)-piperazin-1-yl]-nicotinamide;
*N*-(1,3-Dimethylbutyl)-6-[4-(naphthalene-1-carbonyl)-piperazin-1-yl]-nicotinamide;
*N*-(3-Isopropoxy-propyl)-6-[4-(naphthalene-1-carbonyl)-piperazin-1-yl]-nicotinamide;
*N*-(1,3-Dimethylbutyl)-6-[4-(naphthalene-2-carbonyl)-piperazin-1-yl]-nicotinamide;
*N*-(1,3-Dimethylbutyl)-6-[4-(2-naphthaten-2-yl-acetyl)-piperazin-1-yl]-nicotinamide;
*N*-(2-Methyl-butyl)-6-[4-(naphthalene-1-carbonyl)-piperazin-1-yl]-nicotinamide;
*N*-(2-Ethylsulfanyl-ethyl)-6-[4-(naphthalene-1-carbonyl)-piperazin-1-yl]-nicotinamide;
*N*-(2-Methyl-butyl)-6-[4-(2-naphthalen-2-yl-acetyl)-piperazin-1-yl]-nicotinamide;
*N*-(3-Methyl-butyl)-6-[4-(naphthalene-1-carbonyl)-piperazin-1-yl]-nicotinamide;
*N*-(3-Ethoxy-propyl)-6-[4-(naphthalene-2-carbonyl)-piperazin-1-yl]-nicotinamide;
*N*-(2-Methyl-butyl)-6-[4-(naphthalene-2-carbonyl)-piperazin-1-yl]-nicotinamide;
*N*-(3-Methyl-butyl)-6-[4-(naphthalene-2-carbonyl)-piperazin-1-yl]-nicotinamide;
*N*-(3-Methoxy-propyl)-6-[4-(naphthalene-2-carbonyl)-piperazin-1-yl]-nicotinamide;
*N*-Butyl-6-[4-(naphthalene-2-carbonyl)-piperazin-1-yl]-nicotinamide;
*N*-(3-Butoxy-propyl)-6-[4-(naphthalene-2-carbonyl)-piperazin-1-yl]-nicotinamide;
*N*-(1-Methyl-butyl)-6-[4-(naphthalene-2-carbonyl)-piperazin-1-yl]-nicotinamide;
*N*-(3-Isopropoxy-propyl)-6-[4-(naphthalene-2-carbonyl)-piperazin-1-yl]-nicotinamide;
*N*-Hexyl-6-[4-(naphthalene-2-carbonyl)-piperazin-1-yl]-nicotinamide;
*N*-(3-Dimethylamino-propyl)-6-[4-(naphthalene-1-carbonyl)-piperazin-1-yl]-nicotinamide;
*N*-(2-Ethylsulfanyl-ethyl)-6-[4-(naphthalene-2-carbonyl)-piperazin-1-yl]-nicotinamide;
*N*-(3-Dimethylamino-propyl)-6-[4-(naphthalene-2-carbonyl)-piperazin-1-yl]-nicotinamide;
*N*-(3-Methyl-butyl)-6-[4-(naphthalene-1-carbonyl)-piperazin-1-yl]-nicotinamide;
*N*-(3-Methoxy-propyl)-6-[4-(2-naphthalen-2-yl-acetyl)-piperain-1-yl]-nicotinamide;
2-Chloro-*N*-(3-methylbutyl)-6-[4-(2-trifluoromethyl-benzoyl)-piperazin-1-yl]-nicotinamide;
*N*-(2-Cyclopropyl-ethyl)-6-[4-(3-trifluoromethyl-benzoyl)-piperazin-1-yl]-nicotinamide;
*N*-(2-Cyclopropyl-ethyl)-2-methoxy-6-[4-(2-trifluoromethyl-benzoyl)-piperazin-1-yl]-nicotinamide;
*N*-(2-Cyclopropyl-ethyl)-6-[4-(5-fluoro-2-trifluoromethyl-benzoyl)-piperazin-1-yl]-nicotinamide;
2-Oxo-6-[4-(2-trifluoromethyl-benzoyl)-piperazin-1-yl]-1,2-dihydro-pyridine-3-carboxylic acid (2-cyclopropylethyl)amide;
*N*-(2-Cyclopropyl-ethyl)-2-hydroxy-6-[4-(2-trifluoromethyl-benzoyl)-piperazin-1-yl]-nicotinamide;
*N*-(2-Cyclobutyl-ethyl)-6-[4-(5-fluoro-2-trifluoromethyl-benzoyl)-piperazin-1-yl]-nicotinamide;
*N*-(3-Cyclopropyl-propyl)-6-[4-(5-fluoro-2-trifluoromethyl-benzoyl)-piperazin-1-yl]-nicotinamide;
6-[4-(5-Fluoro-2-trifluoromethyl-benzoyl)-piperazin-1-yl]-*N*-(4-methyl-pentyl)-nicotinamide; and
*N*-(3,3-Dimethylbutyl)-6-[4-(5-fluoro-2-trifluoromethyl-benzoyl)-piperazin-1-yl]-nicotinamide.

20. The use of the compound of Claim 16 wherein the compound of formula (I) is a compound wherein:
m is 1 or 2;
n is 1 or 2;
p is 2;
V is -C(O)-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl, wherein the optionally substituted groups are as defined in Claim 1;
R³ is optionally substituted aryl, wherein the optionally substituted group is as defined in Claim 1;
each R⁴ is independently hydrogen, alkyl, halo, or haloalkyl; and
each R⁵ and R⁶ is independently hydrogen, oxo, alkyl, halo or haloalkyl.

21. The use of the compound of Claim 20 wherein the compound of formula (I) is a compound wherein:
m is 1 or 2;
n is 1;
p is 2;
V is -C(O)-;
R¹ is hydrogen or alkyl;
R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl, wherein the optionally substituted groups are as defined in Claim 1;
R³ is optionally substituted aryl, wherein the optionally substituted group is as defined in Claim 1;
each R⁴ is independently hydrogen, alkyl, halo or haloalkyl;
R⁵ is hydrogen, oxo, alkyl, halo or haloalkyl; and
each R⁶ is independently hydrogen, alkyl, halo or haloalkyl.

22. The use of the compound of Claim 21 wherein the compound of formula (I) is selected from the group consisting of the following:
6-[4-(2,5-Dichloro-benzoyl)-piperazin-1-yl]-*N*-(3-imidazol-1-yl-propyl)-nicotinamide;
6-[4-(2,4-Dichloro-benzoyl)-piperazin-1-yl]-*N*-(3-imidazol-1-yl-propyl)-nicotinamide;
6-[4-(2-Bromo-5-methoxy-benzoyl)-piperazin-1-yl]-*N*-(3-phenyl-propyl)-nicotinamide;
6-[4-(2-Bromo-benzoyl)-piperazin-1-yl]-*N*-[2-(3H-imidazol-4-yl)-ethyl]-nicotinamide;
*N*-(1-Methyl-2-phenyl-ethyl)-6-[4-(2-trifluoromethyl-benzoyl)-piperazin-1-yl]-nicotinamide;
*N*-(1-Methyl-2-phenyl-ethyl)-6-[4-(4-trifluoromethyl-benzoyl)-piperazin-1-yl]-nicotinamide;
6-[4-(Naphthalene-1-carbonyl)-piperazin-1-yl]-*N*-(4-phenyl-butyl)-nicotinamide;
*N*-[2-(3-Chlorophenyl)-ethyl]-6-[4-(2-naphthalen-2-yl-acetyl)-piperazin-1-yl]-nicotinamide;
6-[4-(Naphthalene-2-carbonyl)-piperain-1-yl]-*N*-(3-phenyl-propyl)-nicotinamide;
*N*-(3-Imidazol-1-yl-propyl)-6-[4-(2-naphthalen-2-yl-acetyl)-piperazin-1-yl]-nicotinamide;
6-[4-(Naphthalene-1-carbonyl)-piperazin-1-yl]-*N*-(3-phenyl-propyl)-nicotinamide;
*N*-(1-Methyl-3-phenyl-propyl)-6-[4-(2-naphthalen-2-yl-acetyl)-piperazin-1-yl]-nicotinamide;
6-[4-(2-Naphthalen-2-yl-acetyl)-piperazin-1-yl]-*N*-phenethyl-nicotinamide;
*N*-[2-(3-Chlorophenyl)-ethyl]-6-[4-(naphthalene-1-carbonyl)-piperazin-1-yl]-nicotinamide;
*N*-(1-Methyl-3-phenyl-propyl)-6-[4-(naphthalene-1-carbonyl)-piperazin-1-yl]-nicotinamide;
6-[4-(Naphthalene-1-carbonyl)-piperazin-1-yl]-*N*-phenethyl-nicotinamide;
*N*-[2-(3H-Imidazol-4-yl)-ethyl]-6-[4-(2-naphthalen-2-yl-acetyl)-piperazin-1-yl]-nicotinamide;
6-[4-(2-Naphthalen-2-yl-acetyl)-piperazin-1-yl]-*N*-(4-phenyl-butyl)-nicotinamide;
6-[4-(Naphthalene-2-carbonyl)-piperazin-1-yl]-*N*-(3-phenyl-propyl)-nicotinamide;
*N*-(1-Methyl-3-phenyl-propyl)-6-[4-(naphthalene-2-carbonyl)-piperazin-1-yl]-nicotinamide;
*N*-[2-(3H-Imidazol-4-yl)-ethyl]-6-[4-(naphthalene-1-carbonyl)-piperazin-1-yl]-nicotinamide;
6-[4-(Naphthalene-2-carbonyl)-piperazin-1-yl]-*N*-(tetrahydro-furan-2-ylmethyl)-nicotinamide;
6-[4-(Naphthalene-1-carbonyl)-piperazin-1-yl]-*N*-(tetrahydro-furan-2-ylmethyl)-nicotinamide;
*N*-(3-Imidazol-1-yl-propyl)-6-[4-(naphthalene-2-carbonyl)-piperazin-1-yl]-nicotinamide;
*N*-[2-(3H-Imidazol-4-yl)-ethyl]-6-[4-(naphthalene-2-carbonyl)-piperazin-1-yl]-nicotinamide;
6-[4-(Naphthalene-2-carbonyl)-piperazin-1-yl]-*N*-phenethyl-nicotinamide;
6-[4-(Naphthalene-2-carbonyl)-piperazin-1-yl]-*N*-(4-phenyl-butyl)-nicotinamide;
*N*-[2-(3-Chlorophenyl)-ethyl]-6-[4-(naphthalene-2-carbonyl)-piperazin-1-yl]-nicotinamide;
*N*-(3-Imidazol-1-yl-propyl)-6-[4(naphthalene-1-carbonyl)-piperazin-1-yl]-nicotinamide;
6-[4-(2-Naphthalen-2-yl-acetyl)-piperazin-1-yl]-*N*-(tetrahydro-furan-2-ylmethyl)-nicotinamide;
6-[4-(2-Bromo-benzoyl)-piperazin-1-yl]-*N*-(3-phenyl-propyl)-nicotinamide;
*N*-[2-(3-Chlorophenyl)-ethyl]-6-[4-(2,4-dichloro-benzoyl)-piperazin-1-yl]-nicotinamide;
6-[4-(2,4-Dimethyl-benzoyl)-piperazin-1-yl]-*N*-(4-phenyl-butyl)-nicotiamide;
*N*-Phenethyl-6-[4-(2-p-tolyl-acetyl)-piperazin-1-yl]-nicotinamide;
6-{4-[2-(2,5-Dichloro-phenyl)-acetyl]-piperazin-1-yl}-*N*-(2-methyl-3-phenyl-propyl)-nicotinamide;
6-[4-(2,4-Dimethyl-benzoyl)-piperazin-1-yl]-*N*-(3-phenyl-propyl)-nicotinamide;
*N*-[2-(3-Chlorophenyl)-ethyl]-6-[4-(2-fluoro-4-methyl-benzoyl)-piperazin-1-yl]-nicotinamide;
*N*-[2-(3-Chlorophenyl)-ethyl]-6-[4-(2,5-dichloro-benzoyl)-piperazin-1-yl]-nicotinamide;
*N*-(3-Phenyl-propyl)-6-[4-(4-trifluoromethyl-benzoyl)-piperazin-1-yl]-nicotinamide;
6-[4-(2-Bromo-benzoyl)-piperazin-1-yl]-*N*-(3-imidazol-1-yl-propyl)-nicotinamide;
*N*-(4-Phenyl-butyl)-6-[4-(2-trifluoromethyl-benzoyl)-piperazin-1-yl]-nicotinamide;
*N*-(3-Phenyl-propyl)-6-[4-(3-trifluoromethyl-benzoyl)-piperazin-1-yl]-nicotinamide;
6-[4-(2-Bromo-benzoyl)-piperazin-1-yl]-*N*-[2-(3-chloro-phenyl)-ethyl]-nicotinamide;
6-[4-(2-Bromo-benzoyl)-piperazin-1-yl]-*N*-(2-methyl-3-phenyl-propyl)-nicotinamide;
6-[4-(2-Bromo-5-methoxy-benzoyl)-piperazin-1-yl]-*N*-(5-phenyl-pentyl)-nicotinamide;
6-[4-(2-Bromo-5-methoxy-benzoyl)-piperazin-1-yl]-*N*-[2-(3-chloro-phenyl)-ethyl]-nicotinamide;
*N*-[2-(3-Chlorophenyl)-ethyl]-6-[4-(2-trifluoromethyl-benzoyl)-piperazin-1-yl]-nicotinamide;
*N*-(3-Phenyl-propyl)-6-[4-(2-trifluoromethyl-benzoyl)-piperazin-1-yl]-nicotinamide;
*N*-(4-Phenyl-butyl)-6-[4-(3-trifluoromethyl-benzoyl)-piperazin-1-yl]-nicotinamide;
6-[4-(2,4-Dichloro-benzoyl)-piperazin-1-yl]-*N*-phenethyl-nicotinamide;
6-[4-(2-Bromo-benzoyl)-piperazin-1-yl]-*N*-(4-phenyl-butyl)-nicotinamide;
6-[4-(2-Bromo-benzoyl)-piperazin-1-y1]-*N*-phenethyl-nicotinamide;
6-[4-(2-Bromo-5-methoxy-benzoyl)-piperazin-1-yl]-*N*-(1-methyl-3-phenyl-propyl)-nicotinamide;
6-[4-(3,5-Dichloro-benzoyl)-piperazin-1-yl]-*N*-(3-phenyl-propyl)-nicotinamide;
6-[4-(3,5-Dichloro-benzoyl)-piperazin-1-yl]-*N*-(1-methyl-3-phenyl-propyl)-nicotinamide;
*N*-Phenethyl-6-[4-(4-trifluoromethyl-benzoyl)-piperazin-1-yl]-nicotinamide;
6-[4-(2,4-Dimethyl-benzoyl)-piperazin-1-yl]-*N*-phenethyl-nicotinamide;
*N*-Phenethyl-6-[4-(2-triffuoromethyl-benzoyl)-piperazin-1-yl]-nicotinamide;
6-[4-(2,4-Dichloro-benzoyl)-piperazin-1-yl]-*N*-(1-methyl-3-phenyl-propyl)-nicotinamide;
6-[4-(2,5-Dichloro-benzoyl)-piperazin-1-yl]-*N*-(3-phenyl-propyl)-nicotinamide;
6-[4-(2-Bromo-5-methoxy-benzoyl)-piperazin-1-yl]-*N*-phenethyl-nicotinamide;
6-[4-(2-Bromo-5-methoxy-benzoyl)-piperazin-1-yl]-*N*-(3-imidazol-1-yl-propyl)-nicotinamide;
6-[4-(3,5-Dichloro-benzoyl)-piperazin-1-yl]-*N*-(3-imidazol-1-yl-propyl)-nicotinamide;
*N*-[2-(3H-Imidazol-4-yl)-ethyl]-6-[4-(2,3,4,5-tetrafluoro-benzoyl)-piperazin-1-yl]-nicotinamide;
*N*-Phenethyl-6-[4-(3-trifluoromethyl-benzoyl)-piperazin-1-yl]-nicotinamide;
6-[4-(2,5-Dichloro-benzoyl)-piperazin-1-yl]-*N*-phenethyl-nicotinamide;
6-[4-(3-Methyl-pentanoyl)-piperazin-1-yl]-*N*-(1-methyl-3-phenyl-propyl)-nicotinamide;
6-[4-(2,3,4,5-Tetrafluoro-benzoyl)-piperazin-1-yl]-*N*-(tetrahydro-furan-2-ylmethyl)-nicotinamide;
6-[4-(2-Bromo-5-methoxy-benzoyl)-piperazin-1-yl]-*N*-(tetrahydro-furan-2-ylmethyl)-nicotinamide;
*N*-(Tetrahydro-furan-2-ylmethyl)-6-[4-(4-trifluoromethyl-benzoyl)-piperazin-1-yl]-nicotinamide;
6-[4-(3,5-Dichloro-benzoyl)-piperazin-1-yl]-*N*-(4-phenyl-butyl)-nicotinamide;
6-[4-(2,4-Dimethyl-benzoyl)-piperazin-1-yl]-*N*-(1-methyl-3-phenyl-propyl)-nicotinamide;
6-[4-(2,4-Dimethyl-benzoyl)-piperazin-1-yl]-*N*-(tetrahydro-furan-2-ylmethyl)-nicotinamide;
6-[4-(2-Bromo-benzoyl)-piperazin-1-yl]-*N*-(tetrahydro-furan-2-ylmethyl)-nicotinamide;
*N*-(1-Methyl-3-phenyl-propyl)-6-[4-(3-trifluoromethyl-benzoyl)-piperazin-1-yl]-nicotinamide;
*N*-Phenethyl-6-[4-(2,3,4,5-tetrafluoro-benzoyl)-piperazin-1-yl]-nicotinamide;
*N*-(4-Phenyl-butyl)-6-[4-(2,3,4,5-tetrafluoro-benzoyl)-piperazin-1-yl]-nicotinamide;
*N*-(1-Methyl-3-phenyl-propyl)-6-[4-(2,3,4,5-tetrafluoro-benzoyl)-piperazin-1-yl]-nicotinamide;
*N*-[2-(3-Chlorophenyl)-ethyl]-6-[4-(2,3,4,5-tetrafluoro-benzoyl)-piperazin-1-yl]-nicotinamide;
*N*-(3-Phenyl-propyl)-6-[4-(2,3,4,5-tetrafluoro-benzoyl)-piperazin-1-yl]-nicotinamide;
6-[4-(2,5-Dichloro-benzoyl)-piperazin-1-yl]-*N*-(tetrahydro-furan-2-ylmethyl)-nicotinamide;
6-[4-(2,4-Dimethyl-benzoyl)-piperazin-1-yl]-*N*-[2-(3H-imidazol-4-yl)-ethyl]-nicotinamide;
*N*-[2-(3H-Imidazol-4-yl)-ethyl]-6-[4-(3-trifluoromethyl-benzoyl)-piperazin-1-yl]-nicotinamide;
*N*-(3-Imidazol-1-yl-propyl)-6-[4-(3-trifluoromethyl-benzoyl)-piperazin-1-yl]-nicotinamide;
6-[4-(2,4-Dimethyl-benzoyl)-piperazin-1-yl]-*N*-(3-imidazol-1-yl-propyl)-nicotinamide;
6-[4-(2,5-Dichloro-benzoyl)-piperazin-1-yl]-*N*-[2-(3H-imidazol-4-yl)-ethyl]-nicotinamide;
*N*-[2-(3H-Imidazol-4-yl)-ethyl]-6-[4-(4-trifluoromethyl-benzoyl)-piperazin-1-yl]-nicotinamide;
6-[4-(2,4-Dichloro-benzoyl)-piperazin-1-yl]-*N*-[2-(3H-imidazol-4-yl)-ethyl]-nicotinamide;
6-[4-(3,5-Dichloro-benzoyl)-piperazin-1-yl]-*N*-(tetrahydro-furan-2-ylmethyl)-nicotinamide;
*N*-(3-Imidazol-1-yl-propyl)-6-[4-(2-trifluoromethyl-benzoyl)-piperazin-1-yl]-nicotinamide;
*N*-(Tetrahydro-furan-2-ylmethyl)-6-[4-(3-trifluoromethyl-benzoyl)-piperazin-1-yl]-nicotinamide;
*N*-(Tetrahydro-furan-2-ylmethyl)-6-[4-(2-trifluoromethyl-benzoyl)-piperazin-1-yl]-nicotinamide;
6-[4-(2,4-Dichloro-benzoyl)-piperazin-1-yl]-*N*-(tetrahydro-furan-2-ylmethyl)-nicotinamide;
*N*-[2-(3H-Imidazol-4-yl)-ethyl]-6-[4-(2-trifluoromethyl-benzoyl)-piperazin-1-yl]-nicotinamide;
6-[4-(2-Bromo-5-methoxy-benzoyl)-piperazin-1-yl]-*N*-[2-(3H-imidazol-4-yl)-ethyl]-nicotinamide;
*N*-[2-(3-Chlorophenyl)-ethyl]-6-[4-(3-trifluoromethyl-benzoyl)-piperazin-1-yl]-nicotinamide;
*N*-[2-(3-Chlorophenyl)-ethyl]-6-[4-(4-trifluoromethyl-benzoyl)-piperazin-1-yl]-nicotinamide;
*N*-[2-(3-Chlorophenyl)-ethyl]-6-[4-(3,5-dichloro-benzoyl)-piperazin-1-yl]-nicotinamide;
*N*-(4-Phenyl-butyl)-6-[4-(4-trifluoromethyl-benzoyl)-piperazin-1-yl]-nicotinamide;
6-[4-(2,4-Dichloro-benzoyl)-piperazin-1-yl]-*N*-(4-phenyl-butyl)-nicotinamide;
6-[4-(2,4-Dichloro-benzoyl)-piperazin-1-yl]-*N*-(3-phenyl-propyl)-nicotinamide;
6-[4-(2,5-Dichloro-benzoyl)-piperazin-1-yl]-*N*-(4-phenyl-butyl)-nicotinamide;
6-[4-(3,5-Dichloro-benzoyl)-piperazin-1-yl]-*N*-phenethyl-nicotinamide;
*N*-(3-Imidazol-1-yl-propyl)-6-[4-(4-trifluoromethyl-benzoyl)-piperazin-1-yl]-nicotinamide;
6-[4-(3,5-Dichloro-benzoyl)-piperazin-1-yl]-*N*-[2-(3H-imidazol-4-yl)-ethyl]-nicotinamide;
*N*-(3-Imidazol-1-yl-propyl)-6-[4-(2,3,4,5-tetrafluoro-benzoyl)-piperazin-1-yl]-nicotinamide;
6-[2-Oxo-4-(2-trifluoromethyl-benzoyl)-piperazin-1-yl]-*N*-(3-phenyl-propyl)-nicotinamide;
*N*-(3-Methyl-butyl)-6-[2-oxo-4-(2-trifluoromethyl-benzoyl)-piperazin-1-yl]-nicotinamide.

23. The use of the compound of Claim 1 wherein the compound of formula (I) is a compound wherein:
m is 1 or 2;
n is 1 or 2;
p is 2;
V is -C(O)-;
R¹ is hydrogen or alkyl;
R² is selected from the group consisting of hydrogen, -R⁷-OR⁸, -R⁷-N(R⁸)₂, -R⁷-S(O)ₜR¹⁰ (where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted heteroaryl, optionally substituted heteroarylalkyl or optionally substituted heteroarylalkenyl;
each R⁴ is independently hydrogen, alkyl, alkenyl, halo, haloalkyl or aryl;
each R⁵ and R⁶ is independently hydrogen, oxo, alkyl, alkenyl, halo, haloalkyl or aryl;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl; and
R¹⁰ is alkyl, aryl or aralkyl.

24. The use of the compound of Claim 23 wherein the compound of formula (I) is a compound wherein:
m is 1 or 2;
n is 1 or 2;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl;
R³ is optionally substituted heteroaryl, optionally substituted heteroarylalkyl or optionally substituted heteroarylalkenyl;
each R⁴ is independently hydrogen, alkyl, halo, or haloalkyl;
each R⁵ and R⁶ is independently hydrogen, oxo, alkyl, halo or haloalkyl;
each R⁷ is a straight or branched alkylene chain;
each R⁸ is independently hydrogen, alkyl, haloalkyl, cycloalkyl, cycloalkylalkyl, aryl and aralkyl; and
R¹⁰ is alkyl, aryl or aralkyl.

25. The use of the compound of Claim 24 wherein the compound of formula (I) is a compound wherein:
m is 1 or 2;
n is 1;
pis2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2) or alkyl;
R³ is optionally substituted heteroaryl, optionally substituted heteroarylalkyl or optionally substituted heteroarylalkenyl;
each R⁴ is independently hydrogen, halo or haloalkyl;
R⁵ is hydrogen;
each R⁶ is hydrogen;
R⁷ is a straight or branched alkylene chain;
R⁸ is hydrogen or alkyl; and
R¹⁰ is alkyl, aryl or aralkyl.

26. The use of the compound of Claim 25 wherein the compound of formula (I) is selected from the group consisting of the following:
6-[4-(3-Methyl-3H-1]4-thiophene-2-carbonyl)-piperazin-1-yl]-*N*-pentylnicotinamide;
*N*-(3-Dimethylamino-propyl)-6-[4-(3-methyl-thiophene-2-carbonyl)-piperazin-1-yl]-nicotinamide;
*N*-(3-Dimethylamino-propyl)-6-[4-(thiophene-2-carbonyl)-piperazin-1-yl]-nicotinamide;
6-[4-(2-Chloro-pyridine-3-carbonyl)-piperazin-1-yl]-*N*-(3-ethoxy-propyl)-nicotinamide;
6-[4-(2-Chloro-pyridine-3-carbonyl)-piperazin-1-yl]-*N*-(2-ethylsulfanyl-ethyl)-nicotinamide;
6-[4-(2-Chloro-pyridine-3-carbonyl)-piperazin-1-yl]-*N*-(3-methylbutyl)-nicotinamide;
6-[4-(2-Chloro-pyridine-3-carbonyl)-piperazin-1-yl]-*N*-(3-isopropoxy-propyl)-nicotinamide;
*N*-(2-Ethylsulfanyl-ethyl)-6-[4-(thiophene-2-carbonyl)-piperazin-1-yl]-nicotinamide;
*N*-(3-Ethoxy-propyl)-6-[4-(thiophene-2-carbonyl)-piperazin-1-yl]-nicotinamide;
*N*-(3-Methyl-butyl)-6-[4-(thiophene-2-carbonyl)-piperazin-1-yl]-nicotinamide;
6-[4-(2-Chloro-pyridine-3-carbonyl)-piperazin-1-yl]-*N*-(3-methoxy-propyl)-nicotinamide;
*N*-Pentyl-6-[4-(thiophene-2-carbonyl)-piperazin-1-yl]-nicotinamide;
6-[4-(2-Chloro-pyridine-3-carbonyl)-piperazin-1-yl]-*N*-hexyl-nicotinamide;
*N*-(3-Isopropoxy-propyl)-6-[4-(thiophene-2-carbonyl)-piperazin-1-yl]-nicotinamide;
*N*-(1-Methyl-butyl)-6-[4-(thiophene-2-carbonyl)-piperazin-1-yl]-nicotinamide;
*N*-Hexyl-6-[4-(thiophene-2-carbonyl)-piperazin-1-yl]-nicotinamide;
*N*-(1,3-Dimethylbutyl)-6-[4-(thiophene-2-carbonyl)-piperazin-1-yl]-nicotinamide;
*N*-Butyl-6-[4-(thiophene-2-carbonyl)-piperazin-1-yl]-nicotinamide;
*N*-(3-Methoxy-propyl)-6-[4-(thiophene-2-carbonyl)-piperazin-1-yl]-nicotinamide;
6-[4-(2-Chloro-pyridine-3-carbonyl)-piperazin-1-yl]-*N*-(1,3-dimethylbutyl)-nicotinamide;
*N*-Butyl-6-[4-(2-chloro-pyridine-3-carbonyl)-piperazin-1-yl]-nicotinamide;
6-[4-(2-Chloro-pyridine-3-carbonyl)-piperazin-1-yl]-*N*-(2-methylbutyl)-nicotinamide;
*N*-(2-Methyl-butyl)-6-[4-(thiophene-2-carbonyl)-piperazin-1-yl]-nicotinamide;
6-[4-(2-Chloro-pyridine-3-carbonyl)-piperazin-1-yl]-*N*-(1-methylbutyl)-nicotinamide;
*N*-(1,3-Dimethylbutyl)-6-[4-(3-methyl-thiophene-2-carbonyl)-piperazin-1-yl]-nicotinamide;
*N*-Butyl-6-[4-(3-methyl-thiophene-2-carbonyl)-piperazin-1-yl]-nicotinamide;
*N*-(3-Butoxy-propyl)-6-[4-(3-methyl-thiophene-2-carbonyl)-piperazin-1-yl]-nicotinamide;
*N*-(3-Isopropoxy-propyl)-6-[4-(3-methyl-thiophene-2-carbonyl)-piperazin-1-yl]-nicotinamide;
*N*-(3-Ethoxy-propyl)-6-[4-(3-methyl-thiophene-2-carbonyl)-piperazin-1-yl]-nicotinamide;
*N*-(2-Methyl-butyl)-6-[4-(3-methyl-thiophene-2-carbonyl)-piperazin-1-yl]-nicotinamide;
*N*-(2-Ethylsulfanyl-ethyl)-6-[4-(3-methyl-thiophene-2-carbonyl)-piperazin-1-yl]-nicotinamide;
*N*-(3-Methoxy-propyl)-6-[4-(3-methyl-thiophene-2-carbonyl)-piperazin-1-yl]-nicotinamide;
*N*-(1-Methyl-butyl)-6-[4-(3-methyl-thiophene-2-carbonyl)-piperazin-1-yl]-nicotinamide;
*N*-(3-Methyl-butyl)-6-[4-(3-methyl-thiophene-2-carbonyl)-piperazin-1-yl]-nicotinamide;
*N*-(3-Butoxy-propyl)-6-[4-(2-chloro-pyridine-3-carbonyl)-piperazin-1-yl]-nicotinamide;
*N*-(3-Butoxy-propyl)-6-[4-(thiophene-2-carbonyl)-piperazin-1-yl]-nicotinamide; 6-[4-(2-Chloro-pyridine-3-carbonyl)-piperazin-1-yl]-*N*-pentyl-nicotinamide; *N*-Hexyl-6-[4-(3-methyl-thiophene-2-carbonyl)-piperazin-1-yl]-nicotinamide; and 6-[4-(2-Chloro-pyridine-3-carbonyl)-piperazin-1-yl]-*N*-(3-dimethylamino-propyl)-nicotinamide.

27. The use of the compound of Claim 23 wherein the compound of formula (I) is a compound wherein:
m is 1 or 2;
n is 1 or 2;
p is 2;
V is -C(O)-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted heteroaryl, optionally substituted heteroarylalkyl or optionally substituted heteroarylalkenyl;
each R⁴ is independently hydrogen, alkyl, halo, or haloalkyl; and
each R⁵ and R⁶ is independently hydrogen, oxo, alkyl, halo or haloalkyl.

28. The use of the compound of Claim 27 wherein the compound of formula (I) is a compound wherein:
m is 1 or 2;
n is 1;
p is 2;
V is -C(O)-;
R¹ is hydrogen or alkyl;
R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl;
R³ is optionally substituted heteroaryl, optionally substituted heteroarylalkyl or optionally substituted heteroarylalkenyl;
each R⁴ is independently hydrogen, alkyl, halo, or haloalkyl;
R⁵ is hydrogen, oxo, alkyl, halo or haloalkyl; and
each R⁶ independently hydrogen, oxo, alkyl, halo or haloalkyl.

29. The use of the compound of Claim 21 wherein the compound of formula (I) is selected from the group consisting of the following:
*N*-[2-(3-Chlorophenyl)-ethyl]-6-[4-(3-methyl-thiophene-2-carbonyl)-piperazin-1-yl]-nicotinamide;
*N*-[2-(3-Chlorophenyl)-ethyl]-6-{4-[2-(2-chloro-pyridin-3-yl)-acetyl]-piperazin-1-yl}-nicotinamide;
6-[4-(2-Chloro-pyridine-3-carbonyl)-piperazin-1-yl]-*N*-(4-phenyl-butyl)-nicotinamide;
*N*-[2-(3-Chlorophenyl)-ethyl]-6-[4-(thiophene-2-carbonyl)-piperazin-1-yl]-nicotinamide;
6-[4-(2-Chloro-pyridine-3-carbonyl)-piperazin-1-yl]-*N*-(3-phenyl-propyl)-nicotinamide;
*N*-(3-Phenyl-propyl)-6-[4-(thiophene-2-carbonyl)-piperazin-1-yl]-nicotinamide;
*N*-Phenethyl-6-[4-(thiophene-2-carbonyl)-piperazin-1-yl]-nicotinamide;
*N*-(1-Methyl-3-phenyl-propyl)-6-[4-(thiophene-2-carbonyl)-piperazin-1-yl]-nicotinamide;
*N*-(4-Phenyl-butyl)-6-[4-(thiophene-2-carbonyl)-piperazin-1-yl]-nicotinamide;
6-[4-(2-Chloro-pyridine-3-carbonyl)-piperazin-1-yl]-*N*-(1-methyl-3-phenyl-propyl)-nicotinamide;
6-[4-(2-Chloro-pyridine-3-carbonyl)-piperazin-1-yl]-*N*-phenethyl-nicotinamide;
6-[4-(2-Chloro-pyridine-3-carbonyl)-piperazin-1-yl]-*N*-(tetrahydro-furan-2-ylmethyl)-nicotinamide;
6-[4-(3-Methyl-thiophene-2-carbonyl)-piperazin-1-yl]-*N*-(3-phenyl-propyl)-nicotinamide;
*N*-(1-Methyl-3-phenyl-propyl)-6-[4-(3-methyl-thiophene-2-carbonyl)-piperazin-1-yl]-nicotinamide;
6-[4-(3-Methyl-thiophene-2-carbonyl)-piperazin-1-yl]-*N*-(4-phenyl-butyl)-nicotinamide;
6-[4-(3-Methyl-thiophene-2-carbonyl)-piperazin-1-yl]-*N*-phenethyl-nicotinamide;
6-[4-(3-Methyl-thiophene-2-carbonyl)-piperazin-1-yl]-*N*-(tetrahydro-furan-2-ylmethyl)-nicotinamide;
*N*-(3-Imidazol-1-yl-propyl)-6-[4-(thiophene-2-carbonyl)-piperazin-1-yl]-nicotinamide;
*N*-(Tetrahydro-furan-2-ylmethyl)-6-[4-(thiophene-2-carbonyl)-piperazin-1-yl]-nicotinamide;
*N*-[2-(3H-Imidazol-4-yl)-ethyl]-6-[4-(thiophene-2-carbonyl)-piperazin-1-yl]-nicotinamide;
*N*-(3-imidazol-1-yl-propyl)-6-[4-(3-methyl-thiophene-2-carbonyl)-piperazin-1-yl]-nicotinamide;
*N*-[2-(3H-Imidazol-4-yl)-ethyl]-6-[4-(3-methyl-thiophene-2-carbonyl)-piperazin-1-yl]-nicotinamide;
6-[4-(2-Chloro-pyridine-3-carbonyl)-piperazin-1-yl]-*N*-(3-imidazol-1-yl-propyl)-nicotinamide; and
6-[4-(2-Chloro-pyridine-3-carbonyl)-piperazin-1-yl]-*N*-[2-(3H-imidazol-4-yl)-ethyl]-nicotinamide.

30. The use of the compound of Claim 1 wherein the compound of formula (I) is a compound wherein:
m is 1 or 2;
n is 1 or 2;
p is 2;
V is -C(O)-;
R¹ is, hydrogen or alkyl;
R² is selected from the group consisting of hydrogen, -R⁷-OR⁸, -R⁷-N(R⁸)₂, -R⁷-S(O)ₜR¹⁰ (where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted aralkyl or optionally substituted aralkenyl;
each R⁴ is independently hydrogen, alkyl, alkenyl, halo, haloalkyl, aryl or -R⁹-OR⁸;
each R⁵ and R⁶ is independently hydrogen, oxo, alkyl, alkenyl, halo, haloalkyl or aryl;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl;
R⁹ is a direct bond or a straight or branched alkylene chain; and
R¹⁰ is alkyl, aryl or aralkyl.

31. The use of the compound of Claim 30 wherein the compound of formula (I) is a compound wherein:
m is 1 or 2;
n is 1 or 2;
pis2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl;
R³ is optionally substituted aralkyl or optionally substituted aralkenyl;
each R⁴ is independently hydrogen, alkyl, halo, or haloalkyl;
each R⁵ and R⁶ is independently hydrogen, oxo, alkyl, halo or haloalkyl;
each R⁷ is a straight or branched alkylene chain;
each R⁸ is independently hydrogen, alkyl, haloalkyl, cycloalkyl, cycloalkylalkyl, aryl and aralkyl; and
R¹⁰ is alkyl, aryl or aralkyl.

32. The use of the compound of Claim 31 wherein the compound of formula (I) is a compound wherein:
m is 1 or 2;
n is 1;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2) or alkyl;
R³ is optionally substituted aralkyl or optionally substituted aralkenyl;
each R⁴ is independently hydrogen, halo or haloalkyl;
R⁵ is hydrogen;
each R⁶ is hydrogen;
R⁷ is a straight or branched alkylene chain;
R⁸ is hydrogen or alkyl; and
R¹⁰ is alkyl, aryl or aralkyl.

33. The use of the compound of Claim 32 wherein the compound of formula (I) is selected from the group consisting of the following:
6-{4-[2-(4-Chlorophenyl)-propionyl]-piperazin-1-yl}-*N*-(3-methylbutyl)-nicotinamide;
6-{4-[2-(2-Chloro-6-fluoro-phenyl)-acetyl]-piperazin-1-yl}-*N*-(3-ethoxy-propyl)-nicotinamide;
6-{4-[3-(3,4-Difluoro-phenyl)-propionyl]-piperazin-1-yl}-*N*-(3-dimethylaminopropyl)-nicotinamide;
6-{4-[3-(3,4-Difluoro-phenyl)-propionyl]-piperazin-1-yl}-*N*-(3-ethoxy-propyl)-nicotinamide;
6-{4-[2-(4-Chlorophenyl)-propionyl]-piperazin-1-yl}-*N*-(3-ethoxy-propyl)-nicotinamide;
*N*-(3-Ethoxy-propyl)-6-[4-(2-phenyl-butyryl)-piperazin-1-yl]-nicotinamide;
*N*-(2-Ethylsulfanyl-ethyl)-6-[4-(2-o-tolyl-acetyl)-piperazin-1-yl]-nicotinamide;
6-[4-(3-Methyl-pentanoyl)-piperazin-1-yl]-*N*-(4-phenyl-butyl)-nicotinamide;
*N*-Butyl-6-{4-[3-(3,4-difluoro-phenyl)-propionyl]-piperazin-1-yl}-nicotinamide;
6-{4-[2-(2-Chloro-6-fluoro-phenyl)-acetyl]-piperazin-1-yl}-*N*-(3-dimethylaminopropyl)-nicotinamide;
6-{4-[3-(3,4-Difluoro-phenyl)-propionyl]-piperazin-1-yl}-*N*-(2-ethylsulfanyl-ethyl)-nicotinamide;
*N*-(3-Methoxy-propyl)-6-[4-(2-phenyl-butyryl)-piperazin-1-yl]-nicotinamide;
*N*-Butyl-6-[4-(2-phenyl-butyryl)-piperazin-1-yl]-nicotinamide;
*N*-(3-Butoxy-propyl)-6-{4-[2-(4-chloro-phenyl)-propionyl]-piperazin-1-yl}-nicotinamide;
*N*-(3-Methoxy-propyl)-6-[4-(2-o-tolyl-acetyl)-piperazin-1-yl]-nicotinamide;
*N*-(3-Methyl-butyl)-6-[4-(2-phenyl-butyryl)-piperazin-1-yl]-nicotinamide;
*N*-(1-Methyl-butyl)-6-[4-(2-p-tolyl-acetyl)-piperazin-1-yl]-nicotinamide;
*N*-Butyl-6-{4-[2-(2-chloro-6-fluoro-phenyl)-acetyl]-piperazin-1-yl}-nicotinamide;
*N*-(2-Methyl-butyl)-6-[4-(2-o-tolyl-acetyl)-piperazin-1-yl]-nicotinamide;
6-{4-[3-(3,4-Difluoro-phenyl)-propionyl]-piperazin-1-yl}-*N*-(2-methylbutyl)-nicotinamide;
*N*-(3-Isopropoxy-propyl)-6-[4-(2-o-tolyl-acetyl)-piperazin-1-yl]-nicotinamide;
6-(4-[3-(3,4-Difluoro-phenyl)-propionyl]-piperazin-1-yl)-*N*-(3-methylbutyl)-nicotinamide;
6-{4-[3-(3,4-Difluoro-phenyl)-propionyl]-piperazin-1-yl}-*N*-(3-methoxy-propyl)-nicotinamide;
*N*-(1-Methyl-butyl)-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-(2-methylbutyl)-nicotinamide;
6-{4-[2-(2-Chloro-6-fluoro-phenyl)-acetyl]-piperazin-1-yl}-*N*-(1-methylbutyl)-nicotinamide;
*N*-(2-Methyl-butyl)-6-[4-(2-phenyl-butyryl)-piperazin-1-yl]-nicotinamide;
*N*-(1,3-Dimethylbutyl)-6-[4-(2-phenyl-butyryl)-piperazin-1-yl]-nicotinamide;
6-{4-[2-(4-Chlorophenyl)-propionyl]-piperazin-1-yl}-*N*-(2-methylbutyl)-nicotinamide;
6-{4-[2-(2-Chloro-6-fluoro-phenyl)-acetyl]-piperazin-1-yl}-*N*-(3-isopropoxy-propyl)-nicotinamide;
*N*-(1,3-Dimethylbutyl)-6-[4-(2-p-tolyl-acetyl)-piperazin-1-yl]-nicotinamide;
6-{4-[3-(3,4-Difluoro-phenyl)-propionyl]-piperazin-1-yl}-*N*-hexyl-nicotinamide;
*N*-(1-Methyl-butyl)-6-[4-(2-phenyl-butyryl)-piperazin-1-yl]-nicotinamide;
6-{4-[2-(4-Chlorophenyl)-propionyl]-piperazin-1-yl}-*N*-(1-methylbutyl)-nicotinamide;
6-{4-[2-(4-Chlorophenyl)-propionyl]-piperazin-1-yl}-*N*-(1,3-dimethylbutyl)-nicotinamide;
*N*-Butyl-6-[4-(2-p-tolyl-acetyl)-piperazin-1-yl]-nicotinamide;
*N*-(1,3-Dimethylbutyl)-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-(1,3-dimethylbutyl)-nicotinamide;
*N*-(3-Methyl-butyl)-6-[4-(2-p-tolyl-acetyl)-piperazin-1-yl]-nicotinamide;
*N*-Butyl-6-[4-(2-o-tolyl-acetyl)-piperazin-1-yl]-nicotinamide;
*N*-(3-Butoxy-propyl)-6-[4-(2-p-tolyl-acetyl)-piperazin-1-yl]-nicotinamide;
*N*-Pentyl-6-[4-(2-p-tolyl-acetyl)-piperazin-1-yl]-nicotinamide;
*N*-(3-Butoxy-propyl)-6-[4-(2-phenyl-butyryl)-piperazin-1-yl]-nicotinamide;
*N*-Pentyl-6-[4-(2-phenyl-butyryl)-piperazin-1-yl]-nicotinamide;
*N*-(2-Ethylsulfanyl-ethyl)-6-[4-(2-p-tolyl-acetyl)-piperazin-1-yl]-nicotinamide;
6-{4-[2-(4-Chlorophenyl)-propionyl]-piperazin-1-yl}-*N*-(2-ethylsulfanyl-ethyl)-nicotinamide;
*N*-(3-Ethoxy-propyl)-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-(3-methoxy-propyl)-nicotinamide;
6-{4-[3-(3,4-Difluoro-phenyl)-propionyl]-piperazin-1-yl}-*N*-(3-isopropoxy-propyl)-nicotinamide;
6-{4-[2-(4-Chlorophenyl)-propionyl]-piperazin-1-yl}-*N*-pentyl-nicotinamide;
*N*-(3-Butoxy-propyl)-6-{4-[2-(2-chloro-6-fluoro-phenyl)-acetyl]-piperazin-1-yl}-nicotinamide;
6-{4-[2-(2-Chloro-6-fluoro-phenyl)-acetyl]-piperazin-1-yl}-*N*-hexyl-nicotinamide;
6-{4-[2-(4-Chlorophenyl)-propionyl]-piperazin-1-yl}-*N*-(3-methoxy-propyl)-nicotinamide;
*N*-Hexyl-6-[4-(2-o-tolyl-acetyl)-piperazin-1-yl]-nicotinamide;
6-{4-[3-(3,4-Difluoro-phenyl)-propionyl]-piperazin-1-yl}-*N*-(4-phenyl-butyl)-nicotinamide;
*N*-(3-isopropoxy-propyl)-6-[4-(2-p-tolyl-acetyl)-piperazin-1-yl]-nicotinamide;
6-{4-[3-(3,4-Difluoro-phenyl)-propionyl]-piperazin-1-yl}-*N*-(1-methylbutyl)-nicotinamide;
6-{4-[3-(3,4-Difluoro-phenyl)-propionyl]-piperazin-1-yl}-*N*-pentyl-nicotinamide;
6-{4-[2-(4-Chlorophenyl)-propionyl]-piperazin-1-yl}-*N*-hexyl-nicotinamide;
*N*-Butyl-6-{4-[2-(4-chloro-phenyl)-propionyl]-piperazin-1-yl}-nicotinamide;
6-{4-[2-(2-Chloro-6-fluoro-phenyl)-acetyl]-piperazin-1-yl}-*N*-(2-ethylsulfanyl-ethyl)-nicotinamide;
6-{4-[2-(2-Chloro-6-fluoro-phenyl)-acetyl]-piperazin-1-yl}-*N*-pentyl-nicotinamide;
*N*-Hexyl-6-[4-(2-phenyl-butyryl)-piperazin-1-yl]-nicotinamide;
*N*-(3-Isopropoxy-propyl)-6-[4-(2-phenyl-butyryl)-piperazin-1-yl]-nicotinamide;
*N*-Penty)-6-[4-(2-o-tolyl-acetyl)-piperazin-1-yl]-nicotinamide;
*N*-Hexyl-6-[4-(2-p-tolyl-acetyl)-piperazin-1-yl]-nicotinamide;
6-{4-[3-(3,4-Difluoro-phenyl)-propionyl]-piperazin-1-yl}-*N*-(1,3-dimethylbutyl)-nicotinamide;
6-[4-(Naphthalene-2-carbonyl)-piperazin-1-yl]-*N*-pentyl-nicotinamide;
6-{4-[2-(4-Chlorophenyl)-propionyl]-piperazin-1-yl}-*N*-(3-dimethylamino-propyl)-nicotinamide;
*N*-(3-Dimethylamino-propyl)-6-[4-(2-phenyl-butyryl)-piperazin-1-yl]-nicotinamide;
*N*-(3-Dimethylamino-propyl)-6-[4-(2-p-tolyl-acetyl)-piperazin-1-yl]-nicotinamide;
*N*-(3-Dimethylamino-propyl)-6-[4-(2-o-tolyl-acetyl)-piperazin-1-yl]-nicotinamide;
*N*-(3-Butoxy-propyl)-6-{4-[3-(3,4-difluoro-phenyl)-propionyl]-piperazin-1-yl}-nicotinamide;
*N*-(3-Methoxy-propyl)-6-[4-(2-p-tolyl-acetyl)-piperain-1-yl]-nicotinamide;
*N*-(2-Methyl-butyl)-6-[4-(2-p-tolyl-acetyl)-piperazin-1-yl]-nicotinamide;
*N*-(3-Butoxy-propyl)-6-[4-(2-o-tolyl-acetyl)-piperazin-1-yl]-nicotinamide; and
6-{4-[2-(4-Chlorophenyl)-propionyl]-piperazin-1-yl}-*N*-(3-isopropoxy-propyl)-nicotinamide.

34. The use of the compound of Claim 30 wherein the compound of formula (I) is a compound wherein:
m is 1 or 2;
n is 1 or 2;
p is 2;
V is -C(O)-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted aralkyl or optionally substituted aralkenyl;
each R⁴ is independently hydrogen, alkyl, halo, or haloalkyl; and
each R⁵ and R⁶ is independently hydrogen, oxo, alkyl, halo or haloalkyl.

35. The use of the compound of Claim 34 wherein the compound of formula (I) is a compound wherein:
m is 1 or 2;
n is 1;
p is 2;
V is -C(O)-;
R¹ is hydrogen or alkyl;
R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl;
R³ is optionally substituted aralkyl or optionally substituted aralkenyl;
each R⁴ is independently hydrogen, alkyl, halo or haloalkyl; and
R⁵ is hydrogen, oxo, alkyl, halo or haloalkyl; and
each R⁶ is independently hydrogen, oxo, alkyl, halo or haloalkyl.

36. The use of the compound of Claim 35 wherein the compound of formula (I) is selected from the group consisting of the following:
6-[4-(2-Phenyl-butyryl)-piperazin-1-yl]-*N*-(3-phenyl-propyl)-nicotinamide;
6-{4-[3-(3,4-Difluoro-phenyl)-propionyl]-piperazin-1-yl}-*N*-phenethyl-nicotinamide;
*N*-[2-(3-Chlorophenyl)-ethyl]-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-(3-phenyl-propyl)-nicotinamide;
*N*-(1-Methyl-3-phenyl-propyl)-6-[4-(2-p-tolyl-acetyl)-piperazin-1-yl]-nicotinamide;
*N*-[2-(3-Chlorophenyl)-ethyl]-6-[4-(2-phenyl-butyryl)-piperazin-1-yl]-nicotinamide;
6-{4-[2-(2-Chloro-6-fluoro-phenyl)-acetyl]-piperazin-1-yl}-*N*-phenethylnicotinamide;
*N*-(3-Phenyl-propyl)-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-[2-(3-chloro-phenyl)-ethyl]-nicotinamide;
6-{4-[2-(4-Chlorophenyl)-propionyl]-piperazin-1-yl}-*N*-(3-phenyl-propyl)-nicotinamide;
6-{4-[2-(4-Chlorophenyl)-propionyl]-piperazin-1-yl}-*N*-(1-methyl-3-phenyl-propyl)-nicotinamide;
*N*-[2-(3-Chlorophenyl)-ethyl]-6-{4-[2-(4-chloro-phenyl)-propionyl]-piperazin-1-yl}-nicotinamide;
*N*-[2-(3-Chlorophenyl)-ethyl]-6-[4-(2-p-tolyl-acetyl)-piperazin-1-yl]-nicotinamide;
*N*-Phenethyl-6-[4-(2-p-tolyl-acetyl)-piperazin-1-yl]-nicotinamide;
*N*-(3-Phenyl-propyl)-6-[4-(2-p-tolyl-acetyl)-piperazin-1-yl]-nicotinamide;
*N*-(1-Methyl-3-phenyl-propyl)-6-[4-(2-phenyl-butyryl)-piperazin-1-yl]-nicotinamide;
6-{4-[2-(4-Chlorophenyl)-propionyl]-piperazin-1-yl}-*N*-(4-phenyl-butyl)-nicotinamide;
*N*-(4-Phenyl-butyl)-6-[4-(2-phenyl-butyryl)-piperazin-1-yl]-nicotinamide;
6-{4-[2-(2-Chloro-6-fluoro-phenyl)-acetyl]-piperazin-1-yl}-*N*-(1-methyl-3-phenylpropyl)-nicotinamide;
6-{4-[3-(3,4-Difluoro-phenyl)-propionyl]-piperazin-1-yl}-*N*-(3-phenyl-propyl)-nicotinamide;
*N*-Phenethyl-6-[4-(2-o-tolyl-acetyl)-piperazin-1-yl]-nicotinamide;
6-{4-[3-(3,4-Difluoro-phenyl)-propionyl]-piperazin-1-yl}-*N*-(1-methyl-3-phenylpropyl)-nicotinamide;
*N*-(4-Phenyl-butyl)-6-[4-(2-o-tolyl-acetyl)-piperazin-1-yl]-nicotinamide;
*N*-(3-Imidazol-1-yl-propyl)-6-[4-(2-phenyl-butyryl)-piperazin-1-yl]-nicotinamide;
6-{4-[2-(2-Chloro-6-fluoro-phenyl)-acetyl]-piperazin-1-yl}-*N*-(4-phenyl-butyl)-nicotinamide;
6-{4-[2-(4-Chlorophenyl)-propionyl]-piperazin-1-yl}-*N*-[2-(3H-imidazol-4-yl)-ethyl]-nicotinamide;
6-{4-[2-(2-Chloro-6-fluoro-phenyl)-acetyl]-piperazin-1-yl}-*N*-(3-imidazol-1-yl-propyl)-nicotinamide;
*N*-[2-(3H-Imidazol-4-yl)-ethyl]-6-[4-(2-p-tolyl-acetyl)-piperazin-1-yl]-nicotinamide;
6-{4-[2-(4-Chlorophenyl)-propionyl]-piperazin-1-yl}-*N*-(3-imidazol-1-yl-propyl)-nicotinamide;
*N*-(2-Ethylsulfanyl-ethyl)-6-[4-(2-phenyl-butyryl)-piperazin-1-yl]-nicotinamide;
*N*-(1-Methyl-3-phenyl-propyl)-6-[4-(2-o-tolyl-acetyl)-piperazin-1-yl]-nicotinamide;
6-(4-[3-(3,4-Difluoro-phenyl)-propionyl]-piperazin-1-yl)-*N*-phenethyl-nicotinamide;
*N*-Phenethyl-6-[4-(2-phenyl-butyryl)-piperazin-1-yl]-nicotinamide;
*N*-(Tetrahydro-furan-2-ylmethyl)-6-[4-(2-p-tolyl-acetyl)-piperazin-1-yl]-nicotinamide;
6-{4-[2-(4-Chlorophenyl)-propionyl]-piperazin-1-yl}-*N*-(tetrahydro-furan-2-ylmethyl)-nicotinamide;
*N*-(4-Phenyl-butyl)-6-[4-(2-p-tolyl-acetyl)-piperazin-1-yl]-nicotinamide;
6-{4-[3-(3,4-Difluoro-phenyl)-propionyl]-piperazin-1-yl}-*N*-(tetrahydro-furan-2-ylmethyl)-nicotinamide;
*N*-(Tetrahydro-furan-2-ylmethyl)-6-[4-(2-o-tolyl-acetyl)-piperazin-1-yl]-nicotinamide;
6-[4-(2-Phenyl-butyryl)-piperazin-1-yl]-*N*-(tetrahydro-furan-2-ylmethyl)-nicotinamide;
6-{4-[3-(3,4-Difluoro-phenyl)-propionyl]-piperazin-1-yl}-*N*-[2-(3H-imidazol-4-yl)-ethyl]-nicotinamide;
*N*-[2-(3H-Imidazol-4-yl)-ethyl]-6-[4-(2-o-tolyl-acetyl)-piperazin-1-yl]-nicotinamide;
*N*-(3-Imidazol-1-yl-propyl)-6-[4-(2-o-tolyl-acetyl)-piperazin-1-yl]-nicotinamide;
6-{4-[3-(3,4-Difluoro-phenyl)-propionyl]-piperazin-1-yl}-*N*-(3-imidazol-1-yl-propyl)-nicotinamide;
*N*-[2-(3H-Imidazol-4-yl)-ethyl]-6-[4-(2-phenyl-butyryl)-piperazin-1-yl]-nicotinamide;
*N*-(3-Imidazol-1-yl-propyl)-6-[4-(2-p-tolyl-acetyl)-piperazin-1-yl]-nicotinamide;
6-{4-[2-(2-Chloro-6-fluoro-phenyl)-acetyl]-piperazin-1-yl}-*N*-(tetrahydro-furan-2-ylmethyl)-nicotinamide; and
6-{4-[2-(2-Chloro-6-fluoro-phenyl)-acetyl]-piperazin-1-yl}-*N*-[2-(3H-imidazol-4-yl)-ethyl]-nicotinamide.

37. The use of the compound of Claim 1 wherein the mammal is a human.

38. The use of the compound of Claim 37 wherein the disease or condition is a disease or condition related to serum levels of triglyceride, VLDL, HDL, LDL, total cholesterol or the process of reverse cholesterol transport.

39. The use of the compound of Claim 37 wherein the disease or condition is a disease or condition related to serum triglyceride levels.

40. The use of the compound of Claim 37 wherein the disease or condition is a disease or condition releated to serum cholesterol levels.

41. The use of the compound of Claim 37, wherein the disease or condition is Type II diabetes.

42. The use of the compound of Claim 37, wherein the disease or condition is obesity.

43. The use of the compound of Claim 37, wherein the disease or condition is dyslipidemia.

44. The use of the compound of Claim 37, wherein the disease or condition is metabolic syndrome.

45. A compound of formula (I): 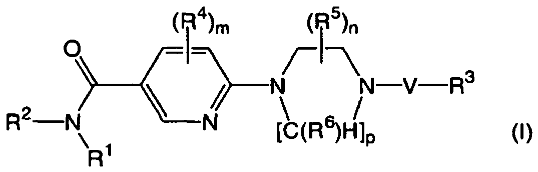 wherein:
m is 1, 2 or 3;
n is 1, 2, 3 or 4;
p is 2, 3 or 4;
V is -C(O)-, -S(O)- or -S(O)₂-;
R¹ is hydrogen, alkyl, alkenyl, aryl, aralkyl, aralkenyl or cycloalkyl;
R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, aralkyl, where the aryl radical may be optionally substituted, aralkenyl, where the aryl radical may be optionally substituted, optionally substituted cycloalkyl, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
R³ is selected from the group consisting of hydrogen, -R⁹-OR⁸, -R⁹-N(R⁸)₂, alkyl,
alkenyl, optionally substituted aryl, aralkyl, where the aryl radical may be optionally substituted, aralkenyl, where the aryl radical may be optionally substituted, optionally substituted cycloalkyl, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
each R⁴ is independently hydrogen, alkyl, alkenyl, halo, haloalkyl, aryl, cyano, nitro, -R⁹-OR⁸, -R⁹-N(R⁸)₂ or -S(O)ₜR¹⁰ (where t is 0, 1 or 2);
each R⁵ and R⁶ is independently hydrogen, oxo, alkyl, alkenyl, halo, haloalkyl or aryl;
or one R⁵ and one R⁶ may together form an straight or branched alkylene bridge;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl;
each R⁹ is independently a direct bond or a straight or branched alkylene or alkenylene chain; and
R¹⁰ is alkyl, alkenyl, haloalkyl, cycloalkyl, cycloalkylalkyl, aryl, aralkyl, heterocyclyl, heterocyclylalkyl, heteroaryl or heteroarylalkyl;
as a single stereoisomer, a mixture of stereoisomers, a racemic mixture thereof of stereoisomers, or as a tautomer;
or as a pharmaceutically acceptable salt, solvate, polymorph or prodrug, selected from among ester and amide derivatives of hydroxyl, carboxy, mecapto or amino functional groups in the compounds of formula (I), thereof for use in the treatment of SCD mediated disease or condition in a mammal, wherein the disease or condition is selected from the group consisting of Type II diabetes, impaired glucose tolerance, insulin resistance, hypertension, obesity, hypertriglyceridemia, low HDL, lipidemia, dyslipidemia, microalbuminemia, hyperuricaemia, hyperleptinaemia, metabolic syndrome, and any combination of these; and
wherein the optional substituent is one or more substituents independently selected from the group consisting of alkyl, alkenyl, halo, haloalkyl, haloalkenyl, cyano, nitro, aryl, aralkyl, cycloalkyl, cycloalkylalkyl, heterocylyl, heterocyclylalkyl, heteroaryl, heteroarylalkyl, -R⁹-OR⁸, -R⁹-N(R⁸)₂, -R⁹-C(O)R⁸,
-R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-(S(O)ₜR¹⁰) (where t is 1 or 2), -R⁹-(S(O)ₜR¹⁰) (where t is 0, 1 or 2), and -R⁹-S(O)ₜN(R⁸)₂ (where t is 1 or 2).

46. The compound for use as in Claim 45 wherein the compound of formula (I) is a compound wherein:
m is 1 or 2;
n is 1 or 2;
p is 2 or 3;
V is -C(O)- or -S(O)₂-;
R¹ is hydrogen or alkyl;
R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is alkyl, alkenyl or -R⁹-N(R⁸)₂;
each R⁴ is independently hydrogen, alkyl, alkenyl, halo, haloalkyl, aryl or -R⁹-OR⁸;
each R⁵ and R⁶ is independently hydrogen, oxo, alkyl, alkenyl, halo, haloalkyl or aryl;
or one R⁵ and one R⁶ may together form an straight or branched alkylene bridge;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl;
each R⁹ is independently a direct bond or a straight or branched alkylene chain; and
R¹⁰ is alkyl, aryl or aralkyl.

47. The compound for use as in Claim 46 wherein the compound of formula (I) is a compound wherein:
m is 1 or 2;
n is 1 or 2;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl;
R³ is alkyl;
each R⁴ is independently hydrogen, alkyl, halo, or haloalkyl;
each R⁵ and R⁶ is independently hydrogen, oxo, alkyl, halo or haloalkyl;
each R⁷ is a straight or branched alkylene chain;
each R⁸ is independently hydrogen, alkyl, haloalkyl, cycloalkyl, cycloalkylalkyl, aryl and aralkyl; and
R¹⁰ is alkyl, aryl or aralkyl.

48. The compound for use as in Claim 47 wherein the compound of formula (I) is a compound wherein:
mist;
n is 1;
pis2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2) or alkyl;
R³ is alkyl;
R⁴ is hydrogen;
R⁵ is hydrogen;
each R⁶ is hydrogen;
R⁷ is a straight or branched alkylene chain;
R⁸ is hydrogen or alkyl; and
R¹⁰ is alkyl, aryl or aralkyl.

49. The compound for use as in Claim 48 wherein the compound of formula (I) is selected from the group consisting of the following:
*N*-(3-Ethoxy-propyl)-6-[4-(2-ethylbutyryl)-piperazin-1-yl]-nicotinamide;
*N*-(3-Ethoxy-propyl)-6-(4-pentanoyl-piperazin-1-yl)-nicotinamide;
*N*-(3-Ethoxy-propyl)-6-[4-(3-methyl-pentanoyl)-piperazin-1-yl]nicotinamide;
*N*-(3-Butoxy-propyl)-6-(4-pentanoyl-piperazin-1-yl)-nicotinamide;
*N*-(3-Methyl-butyl)-6-[4-(3-methyl-pentanoyl)-piperazin-1-yl]-nicotinamide;
6-[4-(3-Methyl-pentanoyl)-piperazin-1-yl]-*N*-pentyl-nicotinamide;
*N*-Butyl-6-[4-(3-methyl-pentanoyl)-piperazin-1-yl]-nicotinamide;
*N*-Butyl-6-[4-(2-ethylbutyryl)-piperazin-1-yl]-nicotinamide;
*N*-(3-Methyl-butyl)-6-(4-pentanoyl-piperazin-1-yl)-nicotinamide;
*N*-(2-Ethylsulfanyl-ethyl)-6-(4-pentanoyl-piperazin-1-yl)-nicotinamide;
*N*-(3-Methoxy-propyl)-6-[4-(3-methyl-pentanoyl)-piperazin-1-yl]-nicotinamide;
6-(4-Pentanoyl-piperazin-1-yl)-*N*-pentyl-nicotinamide;
*N*-Hexyl-6-[4-(3-methyl-pentanoyl)-piperazin-1-yl]-nicotinamide;
*N*-(1,3-Dimethylbutyl)-6-[4-(3-methyl-pentanoyl)-piperazin-1-yl]-nicotinamide;
*N*-(1-Methyl-butyl)-6-(4-pentanoyl-piperazin-1-yl)-nicotinamide;
*N*-(3-Dimethylamino-propyl)-6-[4-(2-ethylbutyryl)-piperazin-1-yl]-nicotinamide;
*N*-(3-Methoxy-propyl)-6-(4-pentanoyl-piperazin-1-yl)-nicotinamide;
*N*-(1,3-Dimethylbutyl)-6-(4-pentanoyl-piperazin-1-yl)-nicotinamide;
*N*-(2-Methyl-butyl)-6-(4-pentanoyl-piperazin-1-yl)-nicotinamide;
*N*-(3-Butoxy-propyl)-6-[4-(2-ethylbutyryl)-piperazin-1-yl]-nicotinamide;
6-[4-(2-Ethylbutyryl)-piperazin-1-yl]-*N*-(3-methylbutyl)-nicotinamide;
*N*-Butyl-6-(4-pentanoyl-piperazin-1-yl)-nicotinamide;
6-[4-(2-Ethylbutyryl)-piperazin-1-yl]-*N*-(2-methylbutyl)-nicotinamide;
*N*-(3-Isopropoxy-propyl)-6-(4-pentanoyl-piperazin-1-yl)-nicotinamide;
*N*-(2-Ethylsulfanyl-ethyl)-6-[4-(3-methyl-pentanoyl)-piperazin-1-yl]-nicotinamide;
6-[4-(2-Ethylbutyryl)-piperazin-1-yl]-*N*-(2-ethylsulfanyl-ethyl)-nicotinamide;
6-[4-(2-Ethylbutyryl)-piperazin-1-yl]-*N*-hexyl-nicotinamide;
6-[4-(2-Ethylbutyryl)-piperazin-1-yl]-*N*-(1-methylbutyl)-nicotinamide;
*N*-(2-Methyl-butyl)-6-[4-(3-methyl-pentanoyl)-piperazin-1-yl]-nicotinamide;
*N*-(1,3-Dimethylbutyl)-6-[4-(2-ethylbutyryl)-piperazin-1-yl]-nicotinamide;
6-[4-(2-Ethylbutyryl)-piperazin-1-yl]-*N*-pentyl-nicotinamide;
*N*-(3-Isopropoxy-propyl)-6-[4-(3-methyl-pentanoyl)-piperazin-1-yl]-nicotinamide;
*N*-(3-Dimethylamino-propyl)-6-[4-(3-methyl-pentanoyl)-piperazin-1-yl]-nicotinamide;
6-[4-(2-Ethylbutyryl)-piperazin-1-yl]-*N*-(3-methoxy-propyl)-nicotinamide;
6-[4-(2-Ethylbutyryl)-piperazin-1-yl]-*N*-(3-isopropoxy-propyl)-nicotinamide;
*N*-(3-Butoxy-propyl)-6-[4-(3-methyl-pentanoyl)-piperazin-1-yl]-nicotinamide;
*N*-(1-Methyl-butyl)-6-[4-(3-methyl-pentanoyl)-piperazin-1-yl]-nicotinamide;
*N*-Hexyl-6-(4-pentanoyl-piperazin-1-yl)-nicotinamide; and
*N*-(3-Dimethylamino-propyl)-6-(4-pentanoyl-piperazin-1-yl)-nicotinamide.

50. The compound for use as in Claim 46 wherein the compound of formula (I) is a compound wherein:
m is 1 or 2;
n is 1 or 2;
p is 2 or 3;
V is -C(O)- or -S(O)₂-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is alkyl or -R⁷-N(R⁸)₂;
each R⁴ is independently hydrogen, alkyl, halo, or haloalkyl; and
each R⁵ and R⁶ is independently hydrogen, oxo, alkyl, alkenyl, halo, haloalkyl or aryl;
or one R⁵ and one R⁶ may together form an straight or branched alkylene bridge;
R⁷ is a direct bond; and
each R⁸ is independently hydrogen or alkyl.

51. The compound for use as in Claim 50 wherein the compound of formula (I) is a compound wherein:
m is 1;
n is 1;
p is 2 or 3;
V is -C(O)- or -S(O)₂-;
R¹ is hydrogen or alkyl;
R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl;
R³ is alkyl or -R⁷-N(R⁸)₂;
R⁴ is hydrogen, alkyl, halo or haloalkyl; and
each R⁵ and R⁶ is independently hydrogen, oxo, alkyl, halo or haloalkyl;
or one R⁵ and one R⁸ may together form a methylene bridge;
R⁷ is a direct bond; and
each R⁸ is independently hydrogen or alkyl.

52. The compound for use as in Claim 51 wherein the compound of formula (I) is selected from the group consisting of the following:
6-[4-(2-Ethylbutyryl)-piperazin-1-yl]-*N*-(3-phenyl-propyl)-nicotinamide;
6-[4-(4-Methyl-hexanoyl)-piperazin-1-yl]-*N*-(3-phenyl-propyl)-nicotinamide;
*N*-[2-(3-Chlorophenyl)-1-methylethyl]-6-[4-(2-ethylbutyryl)-piperazin-1-yl]-nicotinamide;
*N*-[2-(3-Chlorophenyl)-ethyl]-6-[4-(3-methyl-pentanoyl)-piperazin-1-yl]-nicotinamide;
*N*-[2-(3-Chlorophenyl)-ethyl]-6-[4-(2-ethylbutyryl)-piperazin-1-yl]-nicotinamide;
6-(4-Pentanoyl-piperazin-1-yl)-*N*-(4-phenyl-butyl)-nicotinamide;
*N*-[2-(3-Chlorophenyl)-ethyl]-6-(4-pentanoyl-piperazin-1-yl)-nicotinamide;
6-[4-(2-Ethylbutyryl)-piperazin-1-yl]-*N*-phenethyl-nicotinamide;
6-[4-(3-Methyl-pentanoyl)-piperazin-1-yl]-*N*-phenethyl-nicotinamide;
6-(4-Pentanoyl-piperazin-1-yl)-*N*-(3-phenyl-propyl)nicotinamide;
*N*-(1-Methyl-3-phenyl-propyl)-6-(4-pentanoyl-piperazin-1-yl)-nicotinamide;
6-[4-(3-Methyl-pentanoyl)-piperazin-1-yl]-*N*-(tetrahydro-furan-2-ylmethyl)-nicotinamide;
6-[4-(2-Ethylbutyryl)-piperazin-1-yl]-*N*-(tetrahydro-furan-2-ylmethyl)-nicotinamide;
6-(4-Pentanoyl-piperazin-1-yl)-*N*-(tetrahydro-furan-2-ylmethyl)-nicotinamide;
6-[4-(2-Ethylbutyryl)-piperazin-1-yl]-*N*-(4-phenyl-butyl)-nicotinamide;
*N*-(3-Imidazol-1-yl-propyl)-6-(4-pentanoyl-piperazin-1-yl)-nicotinamide;
6-[4-(2-Ethylbutyryl)-piperazin-1-yl]-*N*-(3-imidazol-1-yl-propyl)-nicotinamide;
*N*-[2-(3H-Imidazol-4-yl)-ethyl]-6-(4-pentanoyl-piperazin-1-yl)-nicotinamide;
*N*-[2-(3H-Imidazol-4-yl)-ethyl]-6-[4-(3-methyl-pentanoyl)-piperazin-1-yl]-nicotinamide;
6-(4-Pentanoyl-piperazin-1-yl)-*N*-phenethyl-nicotinamide;
6-[4-(2-Ethylbutyryl)-piperazin-1-yl]-*N*-[2-(3H-imidazol-4-yl)-ethyl]-nicotinamide;
*N*-(3-Imidazol-1-yl-propyl)-6-[4-(3-methyl-pentanoyl)-piperazin-1-yl]-nicotinamide;
6-[4-(2-Ethylbutyryl)-[1,4]diazepan-1-yl]-*N*-(3-phenyl-propyl)-nicotinamide;
6-[4-(2-Ethylbutyryl)-3-methyl-piperazin-1-yl]-*N*-(3-phenyl-propyl)-nicotinamide;
6-[5-(2-Ethylbutyryl)-2,5-diaza-bicyclo[2.2.1]hept-2-yl]-*N*-(3-phenyl-proyl)-nicotinamide;
6-[4-(Butane-1-sulfonyl)-piperazine-1-yl]-*N*-(3-phenyl-propyl)-nicotinamide;
4-[5-(3-Phenyl-propylcarbamoyl)-pyridin-2-yl]-piperazine-1-carboxylic acid butylamide.

53. The compound for use as in Claim 45 wherein the compound of formula (I) is a compound wherein:
m is 1 or 2;
n is 1 or 2;
p is 2;
V is -C(O)-;
R¹ is hydrogen or alkyl;
R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted cycloalkyl, optionally substituted cycloalkylalkyl, or cycloalkylalkenyl;
each R⁴ is independently hydrogen, alkyl, alkenyl, halo, haloalkyl, aryl or -R⁹-OR⁸;
each R⁵ and R⁶ is independently hydrogen, oxo, alkyl, alkenyl, halo, haloalkyl or aryl;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl;
R⁹ is a direct bond or a straight or branched alkylene chain; and
R¹⁰ is alkyl, aryl or aralkyl.

54. The compound for use as in Claim 53 wherein the compound of formula (I) is a compound wherein:
m is 1 or 2;
n is 1 or 2;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl;
R³ is optionally substituted cycloalkyl or optionally substituted cycloalkylalkyl;
each R⁴ is independently hydrogen, alkyl, halo, or haloalkyl;
each R⁵ and R³ is independently hydrogen, oxo, alkyl, halo or haloalkyl;
each R⁷ is a straight or branched alkylene chain;
each R³ is independently hydrogen, alkyl, haloalkyl, cycloalkyl, cycloalkylalkyl, aryl and aralkyl; and
R¹⁰ is alkyl, aryl or aralkyl.

55. The compound for use as in Claim 54 wherein the compound of formula (I) is a compound wherein:
m is 1;
n is 1;
p is 2;
V is -C(O)-;
R¹ is hydrogen or alkyl;
R² is selected from the group consisting of -R⁷-OR⁶, -R⁷-N(R⁸)₂, -R⁷-S(O)ₜR¹⁰ (where t is 0 to 2) or alkyl;
R³ is optionally substituted cycloalkyl or optionally substituted cycloalkylalkyl;
R⁴ is hydrogen;
R⁵ is hydrogen;
each R⁶ is hydrogen;
R⁷ is a straight or branched alkylene chain;
R⁸ is hydrogen or alkyl; and
R¹⁰ is alkyl, aryl or aralkyl.

56. The compound for use as in Claim 55 wherein the compound of formula (I) is selected from the group consisting of the following:
6-[4-(2-Cyclohexyl-acetyl)-piperazin-1-yl]-*N*-(3-ethoxy-propyl)-nicotinamide;
*N*-Butyl-6-[4-(2-cyclohexyl-acetyl)-piperazin-1-yl]-nicotinamide;
6-[4-(3-Cyclohexyl-propionyl)-piperazin-1-yl]-*N*-(3-methylbutyl)-nicotinamide;
6-[4-(3-Cyclohexyl-propionyl)-piperazin-1-yl]-*N*-(3-methoxy-propyl)-nicotinamide;
*N*-Pentyl-6-[4-(3-trifluoromethyl-benzoyl)-piperazin-1-yl]-nicotinamide;
6-[4-(2-Cyclohexyl-acetyl)-piperazin-1-yl]-*N*-(2-methylbutyl)-nicotinamide;
6-[4-(3-Cyclohexyl-propionyl)-piperazin-1-yl]-*N*-(3-isopropoxy-propyl)-nicotinamide;
6-[4-(2-Cyclohexyl-acetyl)-piperazin-1-yl]-*N*-(1,3-dimethylbutyl)-nicotinamide;
6-[4-(2-Cyclohexyl-acetyl)-piperazin-1-yl]-*N*-hexyl-nicotinamide;
6-[4-(2-Cyclohexyl-acetyl)-piperazin-1-yl]-*N*-(1-methylbutyl)-nicotinamide;
6-[4-(2-Cyclohexyl-acetyl)-piperazin-1-yl]-*N*-(3-methylbutyl)-nicotinamide;
6-[4-(2-Cyclohexyl-acetyl)-piperazin-1-yl]-*N*-(2-ethylsulfanyl-ethyl)-nicotinamide;
*N*-(3-Butoxy-propyl)-6-[4-(2-cyclohexyl-acetyl)-piperazin-1-yl]-nicotinamide;
*N*-Butyl-6-[4-(3-cyclohexyl-propionyl)-piperazin-1-yl]-nicotinamide;
6-[4-(2-Cyclohexyl-acetyl)-piperazin-1-yl]-*N*-pentyl-nicotinamide;
6-[4-(3-Cyclohexyl-propionyl)-piperazin-1-yl]-*N*-(2-ethylsulfanyl-ethyl)-nicotinamide;
6-[4-(2-Cyclohexyl-acetyl)-piperazin-1-yl]-*N*-(3-methoxy-propyl)-nicotinamide;
6-[4-(3-Cyclohexyl-propionyl)-piperazin-1-yl]-*N*-(1-methylbutyl)-nicotinamide;
6-[4-(2-Cyclohexyl-acetyl)-piperazin-1-yl]-*N*-(3-isopropoxy-propyl)-nicotinamide;
6-[4-(3-Cyclohexyl-propionyl)-piperazin-1-yl]-*N*-(3-ethoxy-propyl)-nicotinamide;
6-[4-(3-Cyclohexyl-propionyl)-piperazin-1-yl]-*N*-(1,3-dimethylbutyl)-nicotinamide;
6-[4-(3-Cyclohexyl-propionyl)-piperazin-1-yl]-*N*-(2-methylbutyl)-nicotinamide;
6-[4-(3-Cyclohexyl-propionyl)-piperazin-1-yl]-*N*-hexyl-nicotinamide;
6-[4-(3-Cyclohexyl-propionyl)-piperazin-1-yl]-*N*-pentyl-nicotinamide;
6-[4-(3-Cyclohexyl-propionyl)-piperazin-1-yl]-*N*-(3-dimethylamino-propyl)-nicotinamide;
*N*-(3-Ethoxy-propyl)-6-[4-(2-phenyl-cyclopropanecarbonyl)-piperazin-1-yl]-nicotinamide;
*N*-(2-Ethylsulfanyl-ethyl)-6-[4-(2-phenyl-cyclopropanecarbonyl)-piperazin-1-yl]-nicotinamide;
*N*-(1,3-Dimethylbutyl)-6-[4-(2-phenyl-cyclopropanecarbonyl)-piperazin-1-yl]-nicotinamide;
*N*-(3-Methyl-butyl)-6-[4-(2-phenyl-cyclopropanecarbonyl)-piperazin-1-yl]-nicotinamide;
*N*-(3-Methoxy-propyl)-6-[4-(2-phenyl-cyclopropanecarbonyl)-piperazin-1-yl]-nicotinamide;
*N*-(3-Butoxy-propyl)-6-[4-(2-phenyl-cyclopropanecarbonyl)-piperazin-1-yl]-nicotinamide;
*N*-Pentyl-6-[4-(2-phenyl-cyclopropanecarbonyl)-piperazin-1-yl]-nicotinamide;
*N*-(3-Dimethylamino-propyl)-6-[4-(2-phenyl-cyclopropanecarbonyl)-piperazin-1-yl]-nicotinamide;
*N*-(3-Isopropoxy-propyl)-6-[4-(2-phenyl-cyclopropanecarbonyl)-piperazin-1-yl]-nicotinamide;
*N*-(1-Methyl-butyl)-6-[4-(2-phenyl-cyclopropanecarbonyl)-piperazin-1-yl]-nicotinamide;
*N*-Butyl-6-[4-(2-phenyl-cyclopropanecarbonyl)-piperazin-1-yl]-nicotinamide;
*N*-Hexyl-6-[4-(2-phenyl-cyclopropanecarbonyl)-piperazin-1-yl]-nicotinamide;
*N*-(2-Methyl-butyl)-6-[4-(2-phenyl-cyclopropanecarbonyl)-piperazin-1-yl]-nicotinamide; and
6-[4-(2-Cyclohexyl-acetyl)-piperazin-1-yl]-*N*-(3-dimethylamino-propyl)-nicotinamide.

57. The compound for use as in Claim 53 wherein the compound of formula (I) is a compound wherein:
m is 1 or 2;
n is 1 or 2;
p is 2;
V is -C(O)-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted cycloalkyl or optionally substituted cycloalkylalkyl;
each R⁴ is independently hydrogen, alkyl, halo, or haloalkyl; and
each R⁵ and R⁸ is independently hydrogen, oxo, alkyl, halo or haloalkyl.

58. The compound for use as in Claim 57 wherein the compound of formula (I) is a compound wherein:
m is 1;
n is 1;
p is 2;
V is -C(O)-;
R¹ is hydrogen or alkyl;
R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl;
R³ is optionally substituted cycloalkyl or optionally substituted cycloalkylalkyl;
R⁴ is hydrogen, alkyl, halo or haloalkyl;
R⁵ is independently hydrogen, oxo, alkyl, halo or haloalkyl; and
each R⁶ is independently hydrogen, oxo, alkyl, halo or haloalkyl.

59. The compound for use as in Claim 58 wherein the compound of formula (I) is selected from the group consisting of the following:
6-[4-(2-Phenyl-cyclopropanecarbonyl)-piperazin-1-yl]-*N*-(3-phenyl-propyl)-nicotinamide;
*N*-(4-Phenyl-butyl)-6-[4-(2-phenyl-cyclopropanecarbonyl)-piperazin-1-yl]-nicotinamide;
6-[4-(2-Cyclohexyl-acetyl)-piperazin-1-yl]-*N*-phenethyl-nicotinamide;
*N*-[2-(3-Chlorophenyl)-ethyl]-6-[4-(2-phenyl-cyclopropanecarbonyl)-piperazin-1-yl]-nicotinamide;
*N*-(1-Methyl-3-phenyl-propyl)-6-[4-(2-phenyl-cyclopropanecarbonyl)-piperazin-1-yl]-nicotinamide;
*N*-Phenethyl-6-[4-(2-phenyl-cyclopropanecarbonyl)-piperazin-1-yl]-nicotinamide;
6-[4-(2-Phenyl-cyclopropanecarbonyl)-piperazin-1-yl]-*N*-(tetrahydro-furan-2-ylmethyl)-nicotinamide;
*N*-[2-(3H-Imidazol-4-yl)-ethyl]-6-[4-(2-phenyl-cyclopropanecarbonyl)-piperazin-1-yl]-nicotinamide;
*N*-(3-Imidazol-1-yl-propyl)-6-[4-(2-phenyl-cyclopropanecarbonyl)-piperazin-1-yl]-nicotinamide;
*N*-[2-(3-Chlorophenyl)-ethyl]-6-[4-(3-cyclohexyl-propionyl)-piperazin-1-yl]-nicotinamide;
6-[4-(2-Cyclohexyl-acetyl)-piperazin-1-yl]-*N*-[2-(3H-imidazol-4-yl)-ethyl]-nicotinamide;
6-[4-(2-Cyclohexyl-acetyl)-piperazin-1-yl]-*N*-(3-phenyl-propyl)-nicotinamide;
*N*-[2-(3-Chlorophenyl)-ethyl]-6-[4-(2-cyclohexyl-acetyl)-piperazin-1-yl]-nicotinamide;
6-[4-(3-Cyclohexyl-propionyl)-piperazin-1-yl]-*N*-phenethyl-nicotinamide;
6-[4-(3-Cyclohexyl-propionyl)-piperazin-1-yl]-N-(3-phenyl-propyl)-nicotinamide;
6-[4-(3-Methyl-pentanoyl)-piperazin-1-yl]-*N*-(4-phenyl-butyl)-nicotinamide;
6-[4-(2-Cyclohexyl-acetyl)-piperazin-1-yl]-N-(4-phenyl-butyl)-nicotinamide;
6-[4-(2-Cyclohexyl-acetyl)-piperazin-1-yl]-*N*-(1-methyl-3-phenyl-propyl)-nicotinamide;
6-[4-(3-Cyclohexyl-propionyl)-piperazin-1-yl]-*N*-[2-(3H-imidazol-4-yl)-ethyl]-nicotinamide;
6-[4-(3-Cyclohexyl-propionyl)-piperazin-1-yl]-*N*-(tetrahydro-furan-2-ylmethyl)-nicotinamide;
6-[4-(2-Cyclohexyl-acetyl)-piperazin-1-yl]-*N*-(3-imidazol-1-yl-propyl)-nicotinamide;
6-[4-(3-Cyclohexyl-propionyl)-piperazin-1-yl]-*N*-(3-imidazol-1-yl-propyl)-nicotinamide; and
6-[4-(2-Cyclohexyl-acetyl)-piperazin-1-yl]-*N*-(tetrahydro-furan-2-ylmethyl)-nicotinamide.

60. The compound for use as in Claim 45 wherein the compound of formula (I) is a compound wherein:
m is 1 or 2;
n is 1 or 2;
p is 2;
V is -C(O)-;
R¹ is hydrogen or alkyl;
R² is selected from the group consisting of hydrogen, -A⁷-OR⁸, -R⁷-N(R⁸)₂, -A⁷-S(O)ₜR¹⁰ (where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted aryl;
each R⁴ is independently hydrogen, alkyl, alkenyl, halo, haloalkyl, aryl or -R⁹-OR⁸;
each R⁵ and R⁵ is independently hydrogen, oxo, alkyl, alkenyl, halo, haloalkyl or aryl;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl;
R⁹ is a direct bond or a straight or branched alkylene chain; and
R¹⁰ is alkyl, aryl or aralkyl.

61. The compound for use as in Claim 60, wherein the compound of formula (I) is a compound wherein:
m is 1 or 2;
n is 1 or 2;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0 to 2), alkyl, alkenyl, optionally substituted cycloalkyl, optionally substituted cycloalkylalkyl or cycloalkylalkenyl;.
R³ is optionally substituted aryl;
each R⁴ is independently hydrogen, alkyl, halo, haloalkyl or -R⁹-OR⁸;
each R⁵ and R⁶ is independently hydrogen, oxo, alkyl, halo or haloalkyl;
each R⁷ is a straight or branched alkylene chain;
each R⁸ is independently hydrogen, alkyl, haloalkyl, cycloalkyl, cycloalkylalkyl, aryl and aralkyl;
R⁹ is a direct bond or a straight or branched alkylene chain; and
R¹⁰ is alkyl, aryl or aralkyl.

62. The compound for use as in Claim 61 wherein the compound of formula (I) is a compound wherein:
m is 1 or 2;
n is 1;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2), alkyl, optionally substituted cycloalkyl or optionally substituted cycloalkylalkyl;
R³ is optionally substituted aryl;
each R⁴ is independently hydrogen, halo, haloalkyl or -R⁹-OR⁸;
R⁵ is hydrogen;
each R⁶ is hydrogen;
R⁷ is a straight or branched alkylene chain;
R⁸ is hydrogen or alkyl;
R⁹ is a direct bond or a straight or branched alkylene chain; and
R¹⁰ is alkyl, aryl or aralkyl.

63. The compound for use as in Claim 62, wherein the compound of formula (I) is selected from the group consisting of the following:
*N*-Butyl-6-[4-(2-mercapto-benzoyl)-piperazin-1-yl]-nicotinamide;
*N*-(3-Methyl-butyl)-6-[4-(2-o-tolyl-acetyl)-piperazin-1-yl]-nicotinamide;
6-{4-[2-(2,4-Dimethyl-phenyl)-acetyl]-piperazin-1-yl}-*N*-(3-methylbutyl)-nicotinamide;
6-[4-(2-Bromo-benzoyl)-piperazin-1-yl]-*N*-(3-ethoxypropyl)nicotinamide;
6-{4-[2-(2-Chloro-6-fluoro-phenyl)-acetyl]-piperazin-1-yl}-*N*-(3-methylbutyl)-nicotinamide;
6-[4-(3,5-Dichloro-benzoyl)-piperazin-1-yl]-*N*-(3-methoxy-propyl)-nicotinamide;
6-[4-(2-Bromo-5-methoxy-benzoyl)-piperazin-1-yl]-*N*-(3-dimethylamino-propyl)-nicotinamide;
6-[4-(2,5-Dichloro-benzoyl)-piperazin-1-yl]-*N*-(3-methylbutyl)-nicotinamide;
6-[4-(2,5-Dichloro-benzoyl)-piperazin-1-yl]-*N*-(3-ethoxy-propyl)-nicotinamide;
*N*-Butyl-6-[4-(3,5-dichloro-benzoyl)-piperazin-1-yl]-nicotinamide;
6-[4-(2,4-Dichloro-benzoyl)-piperazin-1-yl]-*N*-(3-ethoxy-propyl)-nicotinamide;
6-[4-(2,4-Dichloro-benzoyl)-piperazin-1-yl]-*N*-(2-ethylsulfanyl-ethyl)-nicotinamide;
*N*-(3-Ethoxy-propyl)-6-[4-(4-trifluoromethyl-benzoyl)-piperazin-1-yl]-nicotinamide;
*N*-(3-Dimethylamino-propyl)-6-[4-(2,3,4,5-tetrafluoro-benzoyl)-piperazin-1-yl]-nicotinamide;
6-[4-(2-Bromo-5-methoxy-benzoyl)-piperazin-1-yl]-*N*-(2-ethylsulfanyl-ethyl)-nicotinamide;
*N*-(2-Ethylsulfanyl-ethyl)-6-[4-(3-trifluoromethyl-benzoyl)-piperazin-1-yl]-nicotinamide;
6-[4-(2-Bromo-benzoyl)-piperazin-1-yl]-*N*-(3-methylbutyl)-nicotinamide;
*N*-Butyl-6-[4-(2,4-dimethyl-benzoyl)-piperazin-1-yl]-nicotinamide;
6-[4-(3,5-Dichloro-benzoyl)-piperazin-1-yl]-*N*-(3-isopropoxy-propyl)-nicotinamide;
6-[4-(2-Bromo-5-methoxy-benzoyl)-piperazin-1-yl]-*N*-(3-methylbutyl)-nicotinamide;
*N*-(3-Butoxy-propyl)-6-[4-(2,5-dichloro-benzoyl)-piperazin-1-yl]-nicotinamide;
*N*-(3-Methyl-butyl)-6-[4-(3-trifluoromethyl-benzoyl)-piperazin-1-yl]-nicotinamide;
*N*-(3-Methyl-butyl)-6-[4-(2-trifluoromethyl-benzoyl)-piperazin-1-yl]-nicotinamide;
6-[4-(2-Bromo-benzoyl)-piperazin-1-yl]-*N*-(3-butoxy-propyl)-nicotinamide;
6-[4-(2,5-Dichloro-benzoyl)-piperazin-1-yl]-*N*-pentyl-nicotinamide;
*N*-(3-Butoxy-propyl)-6-[4-(2,4-dimethyl-benzoyl)-piperazin-1-yl]-nicotinamide;
6-[4-(2-Bromo-5-methoxy-benzoyl)-piperazin-1-yl]-*N*-(3-ethoxy-propyl)-nicotinamide;
6-[4-(2,4-Dichloro-benzoyl)-piperazin-1-yl]-*N*-(3-isopropoxy-propyl)-nicotinamide;
*N*-(3-Ethoxy-propyl)-6-[4-(2-trifluoromethyl-benzoyl)-piperazin-1-yl]-nicotinamide;
*N*-Pentyl-6-[4-(3-trifluoromethyl-benzoyl)-piperazin-1-yl]-nicotinamide;
*N*-Butyl-6-[4-(2-trifluoromethyl-benzoyl)-piperazin-1-yl]-nicotinamide;
*N*-Butyl-6-[4-(2,5-dichloro-benzoyl)-piperazin-1-yl]-nicotinamide;
6-[4-(2-Bromo-benzoyl)-piperazin-1-yl]-*N*-(3-isopropoxy-propyl)-nicotinamide;
6-[4-(2,5-Dichloro-benzoyl)-piperazin-1-yl]-*N*-(3-isopropoxy-propyl)-nicotinamide;
*N*-(3-Butoxy-propyl)-6-[4-(4-trifluoromethyl-benzoyl)-piperazin-1-yl]-nicotinamide;
6-[4-(2,5-Dichloro-benzoyl)-piperazin-1-yl]-*N*-(2-ethylsulfanyl-ethyl)-nicotinamide;
*N*-(3-Butoxy-propyl)-6-[4-(2-trifluoromethyl-benzoyl)-piperazin-1-yl]-nicotinamide;
6-[4-(2,5-Dichloro-benzoyl)-piperazin-1-yl]-*N*-(3-methoxy-propyl)-nicotinamide;
6-[4-(2,4-Dimethyl-benzoyl)-piperazin-1-yl]-*N*-(1,3-dimethylbutyl)-nicotinamide;
6-[4-(2,4-Dichloro-benzoyl)-piperazin-1-yl]-*N*-(2-methylbutyl)-nicotinamide;
6-[4-(3,5-Dichloro-benzoyl)-piperazin-1-yl]-*N*-(2-methylbutyl)-nicotinamide;
6-[4-(2,4-Dimethyl-benzoyl)-piperazin-1-yl]-*N*-(2-ethylsulfanyl-ethyl)-nicotinamide;
*N*-(1,3-Dimethylbutyl)-6-[4-(3-trifluoromethyl-benzoyl)-piperazin-1-yl]-nicotinamide;
6-[4-(2-Bromo-5-methoxy-benzoyl)-piperazin-1-yl]-*N*-(2-methylbutyl)-nicotinamide;
*N*-(3-Dimethylamino-propyl)-6-[4-(2-trifluoromethyl-benzoyl)-piperazin-1-yl]-nicotinamide;
*N*-Hexyl-6-[4-(3-trifluoromethyl-benzoyl)-piperazin-1-yl]-nicotinam ide;
6-[4-(2,4-Dichloro-benzoyl)-piperazin-1-yl]-*N*-(1,3-dimethylbutyl)-nicotinamide;
*N*-(3-Isopropoxy-propyl)-6-[4-(3-trifluoromethyl-benzoyl)-piperazin-1-yl]-nicotinamide;
6-[4-(2-Bromo-5-methoxy-benzoyl)-piperazin-1-yl]-*N*-hexyl-nicotinamide;
6-[4-(2-Bromo-benzoyl)-piperazin-1-yl]-*N*-hexyl-nicotinamide;
*N*-(3-Isopropoxy-propyl)-6-[4-(2-trifluoromethyl-benzoyl)-piperazin-1-yl]-nicotinamide;
6-[4-(2-Bromo-5-methoxy-benzoyl)-piperazin-1-yl]-*N*-(3-methoxy-propyl)-nicotinamide;
6-[4-(2-Bromo-benzoyl)-piperazin-1-yl]-*N*-(1-methylbutyl)-nicotinamide;
6-[4-(3,5-Dichloro-benzoyl)-piperazin-1-yl]-*N*-hexyl-nicotinamide;
*N*-(3-Methoxy-propyl)-6-[4-(2-trifluoromethyl-benzoyl)-piperazin-1-yl]-nicotinamide;
6-[4-(2,4-Dichloro-benzoyl)-piperazin-1-yl]-*N*-hexyl-nicotinamide;
*N*-(2-Ethylsulfanyl-ethyl)-6-[4-(2-trifluoromethyl-benzoyl)-piperazin-1-yl]-nicotinamide;
*N*-Pentyl-6-[4-(2-trifluoromethyl-benzoyl)-piperazin-1-yl]-nicotinamide;
*N*-(2-Methyl-butyl)-6-[4-(3-trifluoromethyl-benzoyl)-piperazin-1-yl]-nicotinamide;
*N*-(2-Ethylsulfanyl-ethyl)-6-[4-(4-trifluoromethyl-benzoyl)-piperazin-1-yl]-nicotinamide;
6-[4-(2,4-Dichloro-benzoyl)-piperazin-1-yl]-*N*-pentyl-nicotinamide;
6-[4-(2-Bromo-benzoyl)-piperazin-1-yl]-*N*-(2-methylbutyl)-nicotinamide;
6-[4-(2-Bromo-5-methoxy-benzoyl)-piperazin-1-yl]-*N*-(3-butoxy-propyl)-nicotinamide;
*N*-(1,3-Dimethylbutyl)-6-[4-(4-trifluoromethyl-benzoyl)-piperazin-1-yl]-nicotinamide;
6-[4-(3,5-Dichloro-benzoyl)-piperazin-1-yl]-*N*-pentyl-nicotinamide;
*N*-(3-Butoxy-propyl)-6-[4-(3,5-dichloro-benzoyl)-piperazin-1-yl]-nicotinamide;
6-[4-(2-Bromo-5-methoxy-benzoyl)-piperazin-1-yl]-*N*-butyl-nicotinamide;
6-[4-(2-Bromo-benzoyl)-piperazin-1-yl]-*N*-pentyl-nicotinamide;
*N*-(1,3-Dimethylbutyl)-6-[4-(2-trifluoromethyl-benzoyl)-piperazin-1-yl]-nicotinamide;
*N*-(3-Methoxy-propyl)-6-[4-(4-trifluoromethyl-benzoyl)-piperazin-1-yl]-nicotinamide;
6-[4-(2,4-Dimethyl-benzoyl)-piperazin-1-yl]-*N*-(1-methylbutyl)-nicotinamide;
6-[4-(2,4-Dichloro-benzoyl)-piperazin-1-yl]-*N*-(3-methoxy-propyl)-nicotinamide;
*N*-(1-Methyl-butyl)-6-[4-(2-trifluoromethyl-benzoyl)-piperazin-1-yl]-nicotinamide;
*N*-(3-Dimethylamino-propyl)-6-[4-(3-trifluoromethyl-benzoyl)-piperazin-1-yl]-nicotinamide;
6-[4-(2,4-Dichloro-benzoyl)-piperazin-1-yl]-*N*-(1-methylbutyl)-nicotinamide;
6-[4-(2,5-Dichloro-benzoyl)-piperazin-1-yl]-*N*-(1-methylbutyl)-nicotinamide;
*N*-(1-Methyl-butyl)-6-[4-(3-trifluoromethyl-benzoyl)-piperazin-1-yl]-nicotinamide;
6-[4-(2,5-Dichloro-benzoyl)-piperazin-1-yl]-*N*-hexyl-nicotinamide;
6-[4-(2,4-Dimethyl-benzoyl)-piperazin-1-yl]-*N*-(3-methoxy-propyl)-nicotinamide;
6-[4-(3,5-Dichloro-benzoyl)-piperazin-1-yl]-*N*-(1-methylbutyl)-nicotinamide;
6-[4-(2,4-Dichloro-benzoyl)-piperazin-1-yl]-*N*-(3-methylbutyl)-nicotinamide;
*N*-(3-Butoxy-propyl)-6-[4-(3-trifluoromethyl-benzoyl)-piperazin-1-yl]-nicotinamide;
6-[4-(2,4-Dimethyl-benzoyl)-piperazin-1-yl]-*N*-(3-isopropoxy-propyl)-nicotinamide;
*N*-(1-Methyl-butyl)-6-[4-(4-trifluoromethyl-benzoyl)-piperazin-1-yl]-nicotinamide;
*N*-Butyl-6-[4-(2,4-dichloro-benzoyl)-piperazin-1-yl]-nicotinamide;
6-[4-(3,5-Dichloro-benzoyl)-piperazin-1-yl]-*N*-(1,3-dimethylbutyl)-nicotinamide;
*N*-Butyl-6-[4-(3-trifluoromethyl-benzoyl)-piperazin-1-yl]-nicotinamide;
6-[4-(2-Bromo-5-methoxy-benzoyl)-piperazin-1-yl]-*N*-(1,3-dimethylbutyl)-nicotinamide;
6-[4-(2,4-Dimethyl-benzoyl)-piperazin-1-yl]-*N*-hexyl-nicotinamide;
6-[4-(2,5-Dichloro-benzoyl)-piperazin-1-yl]-*N*-(1,3-dimethylbutyl)-nicotinamide;
*N*-(3-Butoxy-propyl)-6-[4-(2,4-dichloro-benzoyl)-piperazin-1-yl]-nicotinamide;
*N*-(3-Methoxy-propyl)-6-[4-(3-trifluoromethyl-benzoyl)-piperazin-1-yl]-nicotinamide;
6-[4-(2,4-Dimethyl-benzoyl)-piperazin-1-yl]-*N*-(3-ethoxy-propyl)-nicotinamide;
6-[4-(2,5-Dichloro-benzoyl)-piperazin-1-yl]-*N*-(2-methylbutyl)-nicotinamide;
*N*-Hexyl-6-[4-(4-trifluoromethyl-benzoyl)-piperazin-1-yl]-nicotinamide;
6-[4-(2,4-Dimethyl-benzoyl)-piperazin-1-yl]-*N*-pentyl-nicotinamide;
6-[4-(2-Bromo-benzoyl)-piperazin-1-yl]-*N*-(1,3-dimethylbutyl)-nicotinamide;
*N*-Hexyl-6-[4-(2-trifluoromethyl-benzoyl)-piperazin-1-yl]-nicotinamide;
6-[4-(2-Bromo-benzoyl)-piperazin-1-yl]-*N*-(3-methoxy-propyl)-nicotinamide;
*N*-(2-Methyl-butyl)-6-[4-(4-trifluoromethyl-benzoyl)-piperazin-1-yl]-nicotinamide;
6-[4-(2-Bromo-5-methoxy-benzoyl)-piperazin-1-yl]-*N*-(1-methylbutyl)-nicotinamide;
6-[4-(2,4-Dimethyl-benzoyl)-piperazin-1-yl]-*N*-(2-methylbutyl)-nicotinamide;
6-[4-(2-Bromo-5-methoxy-benzoyl)-piperazin-1-yl]-*N*-pentyl-nicotinamide;
*N*-(2-Methyl-butyl)-6-[4-(2-trifluoromethyl-benzoyl)-piperazin-1-yl]-nicotinamide;
6-[4-(3,5-Dichloro-benzoyl)-piperazin-1-yl]-*N*-(3-methylbutyl)-nicotinamide;
6-[4-(2-Bromo-benzoyl)-piperazin-1-yl]-*N*-(2-ethylsulfanyl-ethyl)-nicotinamide;
6-[4-(3,5-Dichloro-benzoyl)-piperazin-1-yl]-*N*-(3-methoxy-propyl)-nicotinamide;
6-[4-(2-Bromo-5-methoxy-benzoyl)-piperazin-1-yl]-*N*-(3-isopropoxy-propyl)-nicotinamide;
*N*-(3-Butoxy-propyl)-6-[4-(2,3,4,5-tetrafluoro-benzoyl)-piperazin-1-yl]-nicotinamide;
*N*-(1,3-Dimethylbutyl)-6-[4-(2,3,4,5-tetrafluoro-benzoyl)-piperazin-1-yl]-nicotinamide;
*N*-Butyl-6-[4-(2,3,4,5-tetrafluoro-benzoyl)-piperazin-1-yl]-nicotinamide;
*N*-(1-Methyl-butyl)-6-[4-(2,3,4,5-tetrafluoro-benzoyl)-piperazin-1-yl]-nicotinamide;
*N*-(3-Methoxy-propyl)-6-[4-(2,3,4,5-tetrafluoro-benzoyl)-piperazin-1-yl]-nicotinamide;
*N*-(3-Isopropoxy-propyl)-6-[4-(2,3,4,5-tetrafluoro-benzoyl)-piperazin-1-yl]-nicotinamide;
*N*-Pentyl-6-[4-(2,3,4,5-tetrafluoro-benzoyl)-piperazin-1-yl]-nicotinamide;
*N*-(2-Methyl-butyl)-6-[4-(2,3,4,5-tetrafluoro-benzoyl)-piperazin-1-yl]-nicotinamide;
*N*-Hexyl-6-[4-(2,3,4,5-tetrafluoro-benzoyl)-piperazin-1-yl]-nicotinamide;
*N*-(3-Methyl-butyl)-6-[4-(2,3,4,5-tetrafluoro-benzoyl)-piperazin-1-yl]-nicotinamide;
*N*-(2-Ethylsulfanyl-ethyl)-6-[4-(2,3,4,5-tetrafluoro-benzoyl)-piperazin-1-yl]-nicotinamide;
*N*-(3-Ethoxy-propyl)-6-[4-(2,3,4,5-tetrafluoro-benzoyl)-piperazin-1-yl]-nicotinamide;
*N*-(3-Dimethylamino-propyl)-6-[4-(4-trifluoromethyl-benzoyl)-piperazin-1-yl]-nicotinamide;
*N*-(3-Ethoxy-propyl)-6-[4-(3-trifluoromethyl-benzoyl)-piperazin-1-yl]-nicotinamide;
6-[4-(3,5-Dichloro-benzoyl)-piperazin-1-yl]-*N*-(2-ethylsulfanyl-ethyl)-nicotinamide;
*N*-(3-Methyl-butyl)-6-[4-(4-trifluoromethyl-benzoyl)-piperazin-1-yl]-nicotinamide;
*N*-Butyl-6-[4-(4-trifluoromethyl-benzoyl)-piperazin-1-yl]nicotinamide;
*N*-(3-Isopropoxy-propyl)-6-[4-(4-trifluoromethyl-benzoyl)-piperazin-1-yl]-nicotinamide;
*N*-Pentyl-6-[4-(4-trifluoromethyl-benzoyl)-piperazin-1-yl]-nicotinamide;
6-[4-(3,5-Dichloro-benzoyl)-piperazin-1-yl]-*N*-(3-dimethylamino-propyl)-nicotinamide;
6-[4-(2-Bromo-benzoyl)-piperazin-1-yl]-*N*-(3-dimethylamino-propyl)-nicotinamide;
6-[4-(2,4-Dichloro-benzoyl)-piperazin-1-yl]-*N*-(3-dimethylamino-propyl)-nicotinamide;
6-[4-(2,5-Dichloro-benzoyl)-piperazin-1-yl]-*N*-(3-dimethylamino-propyl)-nicotinamide;
*N*-(3-Dimethylamino-propyl)-6-[4-(2,4-dimethyl-benzoyl)-piperazin-1-yl]-nicotinamide;
*N*-(3-Methyl-butyl)-6-[4-(2-trifluoromethyl-benzoyl)-piperazin-1-yl]-nicotinamide;
*N*-(3-Methyl-butyl)-4-trifluoromethyl-6-[4-(2-trifluoromethyl-benzoyl)-piperazin-1-yl]-nicotinamide;
2-Chloro-5-fluoro-*N*-(3-methylbutyl)-6-[4-(2-trifluoromethyl-benzoyl)-piperazin-1-yl]-nicotinamide;
*N*-(3-Ethoxy-propyl)-6-[4-(2-naphthalen-2-yl-acetyl)-piperazin-1-yl]-nicotinamide;
6-[4-(2-Naphthalen-2-yl-acetyl)-piperazin-1-yl]-*N*-pentyl-nicotinamide;
*N*-Butyl-6-[4-(naphthalene-1-carbonyl)-piperazin-1-yl]-nicotinamide;
*N*-(3-Butoxy-propyl)-6-[4-(2-naphthalen-2-yl-acetyl)-piperazin-1-yl]-nicotinamide;
*N*-Butyl-6-[4-(2-naphthalen-2-yl-acetyl)-piperazin-1-yl]-nicotinamide;
*N*-(3-Butoxy-propyl)-6-[4-(naphthalene-1-carbonyl)-piperazin-1-yl]-nicotinamide;
*N*-(3-Methoxy-propyl)-6-[4-(naphthalene-1-carbonyl)-piperazin-1-yl]-nicotinamide;
*N*-(1-Methyl-butyl)-6-[4-(naphthalene-1-carbonyl)-piperazin-1-yl]-nicotinamide;
6-[4-(Naphthalene-1-carbonyl)-piperazin-1-yl]-*N*-pentyl-nicotinamide;
*N*-(3-isopropoxy-propyl)-6-[4-(2-naphthalen-2-yl-acetyl)-piperazin-1-yl]-nicotinamide;
*N*-(1-Methyl-butyl)-6-[4-(2-naphthalen-2-yl-acetyl)-piperazin-1-yl]-nicotinamide;
*N*-(3-Methyl-butyl)-6-[4-(2-naphthalen-2-yl-acetyl)-piperazin-1-yl]-nicotinamide;
*N*-(3-Dimethylamino-propyl)-6-[4-(2-naphthalen-2-yl-acetyl)-piperazin-1-yl]-nicotinamide;
*N*-(3-Ethoxy-propyl)-6-[4-(naphthalene-1-carbonyl)-piperazin-1-yl]-nicotinamide;
*N*-Hexyl-6-[4-(naphthalene-1-carbonyl)-piperazin-1-yl]-nicotinamide;
*N*-(2-Ethylsulfanyl-ethyl)-6-[4-(2-naphthalen-2-yl-acetyl)-piperazin-1-yl]-nicotinamide;
*N*-Hexyl-6-[4-(2-naphthalen-2-yl-acetyl)-piperazin-1-yl]-nicotinamide;
*N*-(1,3-Dimethylbutyl)-6-[4-(naphthalene-1-carbonyl)-piperazin-1-yl]-nicotinam ide;
*N*-(3-isopropoxy-propyl)-6-[4-(naphthalene-1-carbonyl)-piperazin-1-yl]-nicotinamide;
*N*-(1,3-Dimethylbutyl)-6-[4-(naphthalene-2-carbonyl)-piperazin-1-yl]-nicotinamide;
*N*-(1,3-Dimethylbutyl)-6-[4-(2-naphthalen-2-yl-acetyl)-piperazin-1-yl]-nicotinamide;
*N*-(2-Methyl-butyl)-6-[4-(naphthalene-1-carbonyl)-piperazin-1-yl]-nicotinamide;
*N*-(2-Ethylsulfanyl-ethyl)-6-[4-(naphthalene-1-carbonyl)-piperazin-1-yl]-nicotinamide;
*N*-(2-Methyl-butyl)-6-[4-(2-naphthalen-2-yl-acetyl)-piperazin-1-yl]-nicotinamide;
*N*-(3-Methyl-butyl)-6-[4-(naphthalene-1-carbonyl)-piperazin-1-yl]-nicotinamide;
*N*-(3-Ethoxy-propyl)-6-[4-(naphthalene-2-carbonyl)-piperazin-1-yl]-nicotinamide;
*N*-(2-Methyl-butyl)-6-[4-(naphthalene-2-carbonyl)-piperazin-1-yl]-nicotinamide;
*N*-(3-Methyl-butyl)-6-[4-(naphthalene-2-carbonyl)-piperazin-1-yl]-nicotinamide;
*N*-(3-Methoxy-propyl)-6-[4-(naphthalene-2-carbonyl)-piperazin-1-yl]-nicotinamide;
*N*-Butyl-6-[4-(naphthalene-2-carbonyl)-piperazin-1-yl]-nicotinamide;
*N*-(3-Butoxy-propyl)-6-[4-(naphthalene-2-carbonyl)-piperazin-1-yl]-nicotinamide;
*N*-(1-Methyl-butyl)-6-[4-(naphthalene-2-carbonyl)-piperazin-1-yl]-nicotinamide;
*N*-(3-Isopropoxy-propyl)-6-[4-(naphthalene-2-carbonyl)-piperazin-1-yl]-nicotinamide;
*N*-Hexyl-6-[4-(naphthalene-2-carbonyl)-piperazin-1-yl]-nicotinamide;
*N*-(3-Dimethylamino-propyl)-6-[4-(naphthalene-1-carbonyl)-piperazin-1-yl]-nicotinamide;
*N*-(2-Ethylsulfanyl-ethyl)-6-[4-(naphthalene-2-carbonyl)-piperazin-1-yl]-nicotinamide;
*N*-(3-Dimethylamino-propyl)-6-[4-(naphthalene-2-carbonyl)-piperazin-1-yl]-nicotinamide;
*N*-(3-Methyl-butyl)-6-[4-(naphthalene-1-carbonyl)-piperazin-1-yl]-nicotinamide;
*N*-(3-Methoxy-propyl)-6-[4-(2-naphthalen-2-yl-acetyl)-piperazin-1-yl]-nicotinamide;
2-Chloro-*N*-(3-methylbutyl)-6-[4-(2-trifluoromethyl-benzoyl)-piperazin-1-yl]-nicotinamide;
*N*-(2-Cyclopropyl-ethyl)-6-[4(3-trifluoromethyl-benzoyl)-piperazin-1-yl]-nicotinamide;
*N*-(2-Cyclopropyl-ethyl)-2-methoxy-6-[4-(2-trifluoromethyl-benzoyl)-piperazin-1-yl]-nicotinamide;
*N*-(2-Cyclopropyl-ethyl)-6-[4-(5-fluoro-2-trifluoromethyl-benzoyl)-piperazin-1-yl]-nicotinamide;
2-Oxo-6-[4-(2-trifluoromethyl-benzoyl)-piperazin-1-yl]-1,2-dihydro-pyridine-3-carboxylic acid (2-cyclopropylethyl)amide;
*N*-(2-Cyclopropyl-ethyl)-2-hydroxy-6-[4-(2-trifluoromethyl-benzoyl)-piperazin-1-yl]-nicotinamide;
*N*-(2-Cyclobutyl-ethyl)-6-[4-(5-fluoro-2-trifluoromethyl-benzoyl)-piperazin-1-yl]-nicotinamide;
*N*-(3-Cyclopropyl-propyl)-6-[4-(5-fluoro-2-trifluoromethyl-benzoyl)-piperazin-1-yl]-nicotinamide;
6-[4-(5-Fluoro-2-trifluoromethyl-benzoyl)-piperazin-1-yl]-*N*-(4-methyl-pentyl)-nicotinamide; and
*N*-(3,3-Dimethylbutyl)-6-[4-(5-fluoro-2-trifluoromethyl-benzoyl)-piperazin-1-yl]-nicotinamide.

64. The compound for use as in Claim 60, wherein the compound of formula (I) is a compound wherein:
m is 1 or 2;
n is 1 or 2;
p is 2;
V is -C(O)-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted aryl;
each R⁴ is independently hydrogen, alkyl, halo, or haloalkyl; and
each R⁵ and R⁶ is independently hydrogen, oxo, alkyl, halo or haloalkyl.

65. The compound for use as in Claim 64 wherein the compound of formula (I) is a compound wherein:
m is 1 or 2;
n is 1;
p is 2;
V is -C(O)-;
R¹ is hydrogen or alkyl;
R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl;
R³ is optionally substituted aryl;
each R⁴ is independently hydrogen, alkyl, halo or haloalkyl;
R⁵ is hydrogen, oxo, alkyl, halo or haloalkyl; and
each R⁶ is independently hydrogen, alkyl, halo or haloalkyl.

66. The compound for use as in Claim 65 wherein the compound of formula (I) is selected from the group consisting of the following:
6-[4-(2,5-Dichloro-benzoyl)-piperazin-1-yl]-*N*-(3-imidazol-1-yl-propyl)-nicotinamide;
6-[4-(2,4-Dichloro-benzoyl)-piperazin-1-yl]-*N*-(3-imidazol-1-yl-propyl). nicotinamide;
6-[4-(2-Bromo-5-methoxy-benzoyl)-piperazin-1-yl]-*N*-(3-phenyl-propyl)-nicotinamide;
6-[4-(2-Bromo-benzoyl)-piperazin-1-yl]-*N*-[2-(3H-imidazol-4-yl)-ethyl]-nicotinamide;
*N*-(1-Methyl-2-phenyl-ethyl)-6-[4-(2-trifluoromethyl-benzoyl)-piperazin-1-yl]-nicotinamide;
*N*-(1-Methyl-2-phenyl-ethyl)-6-[4-(4-trifluoromethyl-benzoyl)-piperazin-1-yl]-nicotinamide;
6-[4-(Naphthalene-1-carbonyl)-piperazin-1-yl]-*N*-(4-phenyl-butyl)-nicotinamide;
*N*-[2-(3-Chlorophenyl)-ethyl]-6-[4-(2-naphthalen-2-yl-acetyl)-piperazin-1-yl]-nicotinamide;
6-[4-(Naphthalene-2-carbonyl)-piperazin-1-yl]-*N*-(3-phenyl-propyl)-nicotinamide;
*N*-(3-Imidazol-1-yl-propyl)-6-[4-(2-naphthalen-2-yl-acetyl)-piperazin-1-yl]-nicotinamide;
6-[4-(Naphthalene-1-carbonyl)-piperazin-1-yl]-*N*-(3-phenyl-propyl)-nicotinamide;
*N*-(1-Methyl-3-phenyl-propyl)-6-[4-(2-naphthalen-2-yl-acetyl)-piperazin-1-yl]-nicotinamide;
6-[4-(2-Naphthalen-2-yl-acetyl)-piperazin-1-yl]-*N*-phenethyl-nicotinamide;
*N*-[2-(3-Chlorophenyl)-ethyl]-6-[4-(naphthalene-1-carbonyl)-piperazin-1-yl]-nicotinamide;
*N*-(1*-*Methyl-3-phenyl-propyl)-6-[4-(naphthalene-1-carbonyl)-piperazin-1-yl]-nicotinamide;
6-[4-(Naphthalene-1-carbonyl)-piperazin-1-yl]-*N*-phenethyl-nicotinamide;
*N*-[2-(3H-Imidazol-4-yl)-ethyl]-6-[4-(2-naphthalen-2-yl-acetyl)-piperazin-1-yl]-nicotinamide;
6-[4-(2-Naphthalen-2-yl-acetyl)-piperazin-1-yl]-*N*-(4-phenyl-butyl)-nicotinamide;
6-[4-(Naphthalene-2-carbonyl)-piperazin-1-yl]-*N*-(3-phenyl-propyl)-nicotinamide;
*N*-(1-Methyl-3-phenyl-propyl)-6-[4-(naphthalene-2-carbonyl)-piperazin-1-yl]-nicotinamide;
*N*-[2-(3H-Imidazol-4-yl)-ethyl]-6-[4-(naphthalene-1-carbonyl)-piperazin-1-yl]-nicotinamide;
6-[4-(Naphthalene-2-carbonyl)-piperazin-1-yl]-*N*-(tetrahydro-furan-2-ylmethyl)-nicotinamide;
6-[4-(Naphthalene-1-carbonyl)-piperazin-1-yl]-*N*-(tetrahydro-furan-2-ylmethyl)-nicotinamide;
*N*-(3-Imidazol-1-yl-propyl)-6-[4-(naphthalene-2-carbonyl)-piperazin-1-yl]-nicotinamide;
*N*-[2-(3H-Imidazol-4-yl)-ethyl]-6-[4-(naphthalene-2-carbonyl)-piperazin-1-yl]-nicotinamide;
6-[4-(Naphthalene-2-carbonyl)-piperazin-1-yl]-*N*-phenethyl-nicotinamide;
6-[4-(Naphthalene-2-carbonyl)-piperazin-1-yl]-*N*-(4-phenyl-butyl)-nicotinamide;
*N*-[2-(3-Chlorophenyl)-ethyl]-6-[4-(naphthalene-2-carbonyl)-piperazin-1-yl]-nicotinamide;
*N*-(3-Imidazol-1-yl-propyl)-6-[4-(naphthalene-1-carbonyl)-piperazin-1-yl]-nicotinamide;
6-[4-(2-Naphthalen-2-yl-acetyl)-piperazin-1-yl]-*N*(tetrahydro-furan-2-ylmethyl)-nicotinamide;
6-[4-(2-Bromo-benzoyl)-piperazin-1-yl]-*N*-(3-phenyl-propyl)-nicotinamide;
*N*-[2-(3-Chlorophenyl)-ethyl]-6-[4-(2,4-dichloro-benzoyl)-piperazin-1-yl]-nicotinamide;
6-[4-(2,4-Dimethyl-benzoyl)-piperazin-1-yl]-*N*-(4-phenyl-butyl)-nicotinamide;
*N*-Phenethyl-6-[4-(2-p-tolyl-acetyl)-piperazin-1-yl]-nicotinamide;
6-{4-[2-(2,5-Dichloro-phenyl)-acetyl]-piperazin-1-yl}-*N*-(2-methyl-3-phenyl-propyl)-nicotinamide;
6-[4-(2,4-Dimethyl-benzoyl)-piperazin-1-yl]-*N*-(3-phenyl-propyl)-nicotinamide;
*N*-[2-(3-Chlorophenyl)-ethyl]-6-[4-(2-fluoro-4-methyl-benzoyl)-piperazin-1-yl]-nicotinamide;
*N*-[2-(3-Chlorophenyl)-ethyl]-6-[4-(2,5-dichloro-benzoyl)-piperazin-1-yl]-nicotinamide;
*N-(3-*Phenyl-propyl)-6-[4-(4-trifluoromethyl-benzoyl)-piperazin-1-yl]-nicotinamide;
6-[4-(2-Bromo-benzoyl)-piperazin-1-yl]-*N*-(3-imidazol-1-yl-propyl)-nicotinamide;
*N*-(4-Phenyl-butyl)-6-[4-(2-trifluoromethyl-benzoyl)-piperazin-1-yl]-nicotinamide;
*N*-(3-Phenyl-propyl)-6-[4-(3-trifluoromethyl-benzoyl)-piperazin-1-yl]-nicotinamide;
6-[4-(2-Bromo-benzoyl)-piperazin-1-yl]-*N*-[2-(3-chloro-phenyl)-ethyl]-nicotinamide;
6-[4-(2-Bromo-benzoyl)-piperazin-1-yl]-*N*-(2-methyl-3-phenyl-propyl)-nicotinamide;
6-[4-(2-Bromo-5-methoxy-benzoyl)-piperazin-1-yl]-*N*-(5-phenyl-pentyl)-nicotinamide;
6-[4-(2-Bromo-5-methoxy-benzoyl)-piperazin-1-yl]-*N*-[2-(3-chloro-phenyl)-ethyl]-nicotinamide;
*N*-[2-(3-Chlorophenyl)-ethyl]-6-[4-(2-trifluoromethyl-benzoyl)-piperazin-1-yl]-nicotinamide;
*N*-(3-Phenyl-propyl)-6-[4-(2-trifluoromethyl-benzoyl)-piperazin-1-yl]-nicotinamide;
*N*-(4-Phenyl-butyl)-6-[4-(3-trifluoromethyl-benzoyl)-piperazin-1-yl]-nicotinamide;
6-[4-(2,4-Dichloro-benzoyl)-piperazin-1-yl]-*N*-phenethyl-nicotinamide;
6-[4-(2-Bromo-benzoyl)-piperazin-1-yl]-*N-(*4-phenyl-butyl)-nicotinamide;
6-[4-(2-Bromo-benzoyl)-piperazin-1-yl]-*N*-phenethyl-nicotinamide;
6-[4-(2-Bromo-5-methoxy-benzoyl)-piperazin-1-yl]-*N*-(1-methyl-3-phenyl-propyl)-nicotinamide;
6-[4-(3,5-Dichloro-benzoyl)-piperazin-1-yl]-*N*-(3-phenyl-propyl)-nicotinamide;
6-[4-(3,5-Dichloro-benzoyl)-piperazin-1-yl]-*N*-(1-methy)-3-phenyl-propyl)-nicotinamide;
*N*-Phenethyl-6-[4-(4-trifluoromethyl-benzoyl)-piperazin-1-yl]-nicotinamide;
6-[4-(2,4-Dimethyl-benzoyl)-piperazin-1-yl]-*N*-phenethyl-nicotinamide;
*N*-Phenethyl-6-[4-(2-trifluoromethyl-benzoyl)-piperazin-1-yl]-nicotinamide;
6-[4-(2,4-Dichloro-benzoyl)-piperazin-1-yl]-*N*-(1-methyl-3-phenyl-propyl)-nicotinamide;
6-[4-(2,5-Dichloro-benzoyl)-piperazin-1-yl]-*N*-(3-phenyl-propyl)-nicotinamide;
6-[4-(2-Bromo-5-methoxy-benzoyl)-piperazin-1-yl]-*N*-phenethyl-nicotinamide;
6-[4-(2-Bromo-5-methoxy-benzoyl)-piperazin-1-yl]-*N*-(3-imidazol-1-yl-propyl)-nicotinamide;
6-[4-(3,5-Dichloro-benzoyl)-piperazin-1-yl]-*N*-(3-imidazol-1-yl-propyl)-nicotinamide;
*N*-[2-(3H-Imidazol-4-yl)-ethyl]-6-[4-(2,3,4,5-tetrafluoro-benzoyl)-piperazin-1-yl]-nicotinamide;
*N*-Phenethyl-6-[4-(3-trifluoromethyl-benzoyl)-piperazin-1-yl]-nicotinamide;
6-[4-(2,5-Dichloro-benzoyl)-piperazin-1-yl]-*N*-phenethyl-nicotinamide;
6-[4-(3-Methyl-pentanoyl)-piperazin-1-yl]-*N-(*1-methyl-3-phenyl-propyl)-nicotinamide;
6-[4-(2,3,4,5-Tetrafluoro-benzoyl)-piperazin-1-yl]-*N*-(tetrahydro-furan-2-ylmethyl)-nicotinamide;
6-[4-(2-Bromo-5-methoxy-benzoyl)-piperazin-1-yl]-*N*-(tetrahydro-furan-2-ylmethyl)-nicotinamide;
*N*-(Tetrahydro-furan-2-ylmethyl)-6-[4-(4-trifluoromethyl-benzoyl)-piperazin-1-yl]-nicotinamide;
6-[4-(3,5-Dichloro-benzoyl)-piperazin-1-yl]-*N*-(4-phenyl-butyl)-nicotinamide;
6-[4-(2,4-Dimethyl-benzoyl)-piperazin-1-yl]-*N*-(1-methyl-3-phenyl-propyl)-nicotinamide;
6-[4-(2,4-Dimethyl-benzoyl)-piperazin-1-yl]-*N*-(tetrahydro-furan-2-ylmethyl)-nicotinamide;
6-[4-(2-Bromo-benzoyl)-piperazin-1-yl)-*N*-(tetrahydro-furan-2-ylmethyl)-nicotinamide;
*N*-(1-Methyl-3-phenyl-propyl)-6-[4-(3-trifluoromethyl-benzoyl)-piperazin-1-yl]-nicotinamide;
*N*-Phenethyl-6-[4-(2,3,4,5-tetrafluoro-benzoyl)-piperazin-1-yl]-nicotinamide;
*N*-(4-Phenyl-butyl)-6-[4-(2,3,4,5-tetrafluoro-benzoyl)-piperazin-1-yl]-nicotinamide;
*N*-(1-Methyl-3-phenyl-propyl)-6-[4-(2,3,4,5-tetrafluoro-benzoyl)-piperazin-1-yl]-nicotinamide;
*N*-[2-(3-Chlorophenyl)-ethyl]-6-[4-(2,3,4,5-tetrafluoro-benzoyl)-piperazin-1-yl]-nicotinamide;
*N*-(3-Phenyl-propyl)-6-[4-(2,3,4,5-tetrafluoro-benzoyl)-piperazin-1-yl]-nicotinamide;
6-[4-(2,5-Dichloro-benzoyl)-piperazin-1-yl]-*N*-(tetrahydro-furan-2-ylmethyl)-nicotinamide;
6-[4-(2,4-Dimethyl-benzoyl)-piperazin-1-yl]-*N*-[2-(3H-imidazol-4-yl)-ethyl]-nicotinamide;
*N*-[2-(3H-Imidazol-4-yl)-ethyl]-6-[4-(3-trifluoromethyl-benzoyl)-piperazin-1-yl]-nicotinamide;
*N*-(3-Imidazol-1-yl-propyl)-6-[4-(3-trifluoromethyl-benzoyl)-piperazin-1-yl]-nicotinamide;
6-[4-(2,4-Dimethyl-benzoyl)-piperazin-1-yl]-*N*-(3-imidazol-1-yl-propyl)-nicotinamide;
6-[4-(2,5-Dichloro-benzoyl)-piperazin-1-yl]-*N*-[2-(3H-imidazol-4-yl)-ethyl]-nicotinamide;
*N*-[2-(3H-Imidazol-4-yl)-ethyl]-6-[4-(4-trifluoromethyl-benzoyl)-piperazin-1-yl]-nicotinamide;
6-[4-(2,4-Dichloro-benzoyl)-piperazin-1-yl]-*N*-[2-(3H-imidazol-4-yl)-ethyl]-nicotinamide;
6-[4-(3,5-Dichloro-benzoyl)-piperazin-1-yl]-*N*-(tetrahydro-furan-2-ylmethyl)-nicotinamide;
*N*-(3-lmidazol-1-yl-propyl)-6-[4-(2-trifluoromethyl-benzoyl)-piperazin-1-yl]-nicotinamide;
*N*-(Tetrahydro-furan-2-ylmethyl)-6-[4-(3-trifluoromethyl-benzoyl)-piperazin-1-yl]-nicotinamide;
*N*-(Tetrahydro-furan-2-ylmethyl)-6-[4-(2-trifluoromethyl-benzoyl)-piperazin-1-yl]-nicotinamide;
6-[4-(2,4-Dichloro-benzoyl)-piperazin-1-yl]-*N*-(tetrahydro-furan-2-ylmethyl)-nicotinamide;
*N*-[2-(3H-Imidazol-4-yl)-ethyl]-6-[4-(2-trifluoromethyl-benzoyl)-piperazin-1-yl]-nicotinamide;
6-[4-(2-Bromo-5-methoxy-benzoyl)-piperazin-1-yl]-*N*-[2-(3H-imidazol-4-yl)-ethyl]-nicotinamide;
*N*-[2-(3-Chlorophenyl)-ethyl]-6-[4-(3-trifluoromethyl-benzoyl)-piperazin-1-yl]-nicotinamide;
*N*-[2-(3-Chlorophenyl)-ethyl]-6-[4-(4-trifluoromethyl-benzoyl)-piperazin-1-yl]-nicotinamide;
*N*-[2-(3-Chlorophenyl)-ethyl]-6-[4-(3,5-dichloro-benzoyl)-piperazin-1-yl]-nicotinamide;
*N*-(4-Phenyl-butyl)-6-[4-(4-trifluoromethyl-benzoyl)-piperazin-1-yl]-nicotinamide;
6-[4-(2,4-Dichloro-benzoyl)-piperazin-1-yl]-*N*-(4-phenyl-butyl)-nicotinamide;
6-[4-(2,4-Dichloro-benzoyl)-piperazin-1-yl-*N*-(3-phenyl-propyl)-nicotinamide;
6-[4-(2,5-Dichloro-benzoyl)-piperazin-1-yl]-*N*-(4-phenyl-butyl)-nicotinamide;
6-[4-(3,5-Dichloro-benzoyl)-piperazin-1-yl]-*N*-phenethyl-nicotinamide;
*N*-(3-Imidazol-1-yl-propyl)-6-[4-(4-trifluoromethyl-benzoyl)-piperazin-1-yl]-nicotinamide;
6-[4-(3,5-Dichloro-benzoyl)-piperazin-1-yl]-*N*-[2-(3H-imidazol-4-yl)-ethyl]-nicotinamide;
*N*-(3-Imidazol-1-yl-propyl)-6-[4-(2,3,4,5-tetrafluoro-benzoyl)-piperazin-1-yl]-nicotinamide;
6-[2-Oxo-4-(2-trifluoromethyl-benzoyl)-piperazin-1-yl]-*N*-(3-phenyl-propyl)-nicotinamide;
*N*-(3-Methyl-butyl)-6-[2-oxo-4-(2-trifluoromethyl-benzoyl)-piperazin-1-yl]-nicotinamide.

67. The compound for use as in Claim 45 wherein the compound of formula (I) is a compound wherein:
m is 1 or 2;
n is 1 or 2;
pis2;
V is -C(O)-;
R¹ is hydrogen or alkyl;
R² is selected from the group consisting of hydrogen, -R⁷-OR⁸, -R⁷-N(R⁸)₂, -R⁷-S(O)ₜR¹⁰ (where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted heteroaryl, optionally substituted heteroarylalkyl or optionally substituted heteroarylalkenyl;
each R⁴ is independently hydrogen, alkyl, alkenyl, halo, haloalkyl or aryl;
each R⁵ and R⁶ is independently hydrogen, oxo, alkyl, alkenyl, halo, haloalkyl or aryl;
each R⁷ is independently a straight or branched alkylene or alkenylene chain;
each R³ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl; and
R¹⁰ is alkyl, aryl or aralkyl.

68. The compound for use as in Claim 67 wherein the compound of formula (I) is a compound wherein:
m is 1 or 2;
n is 1 or 2;
pis2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkyl, optionally substituted cycloalkylalkyl or cycloalkylalkenyl;
R³ is optionally substituted heteroaryl, optionally substituted heteroarylalkyl or optionally substituted heteroarylalkenyl;
each R⁴ is independently hydrogen, alkyl, halo, or haloalkyl;
each R⁵ and R⁶ is independently hydrogen, oxo, alkyl, halo or haloalkyl;
each R⁷ is a straight or branched alkylene chain;
each R⁸ is independently hydrogen, alkyl, haloalkyl, cycloalkyl, cycloalkylalkyl, aryl and aralkyl; and
R¹⁰ is alkyl, aryl or aralkyl.

69. The compound for use as in Claim 68 wherein the compound of formula (I) is a compound wherein:
m is 1 or 2;
n is 1;
pis2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2) or alkyl;
R³ is optionally substituted heteroaryl, optionally substituted heteroarylalkyl or optionally substituted heteroarylalkenyl;
each R⁴ is independently hydrogen, halo or haloalkyl;
R⁵ is hydrogen;
each R⁶ is hydrogen;
R⁷ is a straight or branched alkylene chain;
R⁸ is hydrogen or alkyl; and
R¹⁰ is alkyl, aryl or aralkyl.

70. The compound for use as in Claim 69 wherein the compound of formula (I) is selected from the group consisting of the following:
6-[4-(3-Methyl-3H-114-thiophene-2-carbonyl)-piperazin-1-yl]-*N*-pentylnicotinamide;
*N*-(3-Dimethylamino-propyl)-6-[4-(3-methyl-thiophene-2-carbonyl)-piperazin-1-yl]-nicotinamide;
*N*-(3-Dimethylamino-propyl)-6-[4-(thiophene-2-carbonyl)-piperazin-1-yl]-nicotinamide;
6-[4-(2-Chloro-pyridine-3-carbonyl)-piperazin-1-yl]-*N*-(3-ethoxy-propyl)-nicotinamide;
6-[4-(2-Chloro-pyridine-3-carbonyl)-piperazin-1-yl]-*N*-(2-ethylsulfanyl-ethyl)-nicotinamide;
6-[4-(2-Chloro-pyridine-3-carbonyl)-piperazin-1-yl]-*N*-(3-methylbutyl)-nicotinamide;
6-[4-(2-Chloro-pyridine-3-carbonyl)-piperazin-1-yl]-*N*-(3-isopropoxy-propyl)-nicotinamide;
*N*-(2*-*Ethylsulfanyl-ethyl)-6-[4-(thiophene-2-carbonyl)-piperazin-1-yl]-nicotinamide;
*N*-(3-Ethoxy-propyl)-6-[4-(thiophene-2-carbonyl)-piperazin-1-yl]-nicotinamide;
*N*-(3-Methyl-butyl)-6-[4-(thiophene-2-carbonyl)-piperazin-1-yl]-nicotinamide;
6-[4-(2-Chloro-pyridine-3-carbonyl)-piperazin-1-yl]-*N*-(3-methoxy-propyl)-nicotinamide;
*N*-Pentyl-6-[4-(thiophene-2-carbonyl)-piperazin-1-yl]-nicotinamide;
6-[4-(2-Chloro-pyridine-3-carbonyl)-piperazin-1-yl]-*N*-hexyl-nicotinamide;
*N*-(3-Isopropoxy-propyl)-6-[4-(thiophene-2-carbonyl)-piperazin-1-yl]-nicotinamide;
*N*-(1-Methyl-butyl)-6-[4-(thiophene-2-carbonyl)-piperazin-1-yl]-nicotinamide;
*N*-Hexyl-6-[4-(thiophene-2-carbonyl)-piperazin-1-yl]-nicotinamide;
*N*-(1,3-Dimethylbutyl)-6-[4-(thiophene-2-carbonyl)-piperazin-1-yl]-nicotinamide;
*N*-Butyl-6-[4-(thiophene-2-carbonyl)-piperazin-1-yl]-nicotinamide;
*N*-(3-Methoxy-propyl)-6-[4-(thiophene-2-carbonyl)-piperazin-1-yl]-nicotinamide;
6-[4-(2-Chloro-pyridine-3-carbonyl)-piperazin-1-yl]-*N*-(1,3-dimethylbutyl)-nicotinamide;
*N*-Butyl-6-[4-(2-chloro-pyridine-3-carbonyl)-piperazin-1-yl]-nicotinamide;
6-[4-(2-Chloro-pyridine-3-carbonyl)-piperazin-1-yl]-*N*-(2-methylbutyl)-nicotinamide;
*N*-(2-Methyl-butyl)-6-[4-(thiophene-2-carbonyl)-piperazin-1-yl]-nicotinamide;
6-[4-(2-Chloro-pyridine-3-carbonyl)-piperazin-1-yl]-*N*-(1-methylbutyl)-nicotinamide;
*N*-(1,3-Dimethylbutyl)-6-[4-(3-methyl-thiophene-2-carbonyl)-piperazin-1-yl]-nicotinamide;
*N*-Butyl-6-[4-(3-methyl-thiophene-2-carbonyl)-piperazin-1-yl]-nicotinamide;
*N*-(3-Butoxy-propyl)-6-[4-(3-methyl-thiophene-2-carbonyl)-piperazin-1-yl]-nicotinamide;
*N*-(3-Isopropoxy-propyl)-6-[4-(3-methyl-thiophene-2-carbonyl)-piperazin-1-yl]-nicotinamide;
*N*-(3-Ethoxy-propyl)-6-[4-(3-methyl-thiophene-2-carbonyl)-piperazin-1-yl]-nicotinamide;
*N*-(2-Methyl-butyl)-6-[4-(3-methyl-thiophene-2-carbonyl)-piperazin-1-yl]-nicotinamide;
*N*-(2*-*Ethylsulfanyl-ethyl)-6-[4-(3-methyl-thiophene-2-carbonyl)-piperazin-1-yl]-nicotinamide;
*N-*(3*-*Methoxy-propyl)-6-[4-(3-methyl-thiophene-2-carbonyl)-piperazin-1-yl]-nicotinamide;
*N*-(1-Methyl-butyl)-6-[4-(3-methyl-thiophene-2-carbonyl)-piperazin-1-yl]-nicotinamide;
*N*-(3-Methyl-butyl)-6-[4-(3-methyl-thiophene-2-carbonyl)-piperazin-1-yl]-nicotinamide;
*N*-(3-Butoxy-propyl)-6-[4-(2-chloro-pyridine-3-carbonyl)-piperazin-1-yl]-nicotinamide;
*N*-(3-Butoxy-propyl)-6-[4-(thiophene-2-carbonyl)-piperazin-1-yl]-nicotinamide;
6-[4-(2-Chloro-pyridine-3-carbonyl)-piperazin-1-yl]-*N*-pentyl-nicotinamide;
*N*-Hexyl-6-[4-(3-methyl-thiophene-2-carbonyl)-piperazin-1-yl]-nicotinamide; and
6-[4-(2-Chloro-pyridine-3-carbonyl)-piperazin-1-yl]-*N*-(3-dimethylamino-propyl)-nicotinamide.

71. The compound for use as in Claim 67 wherein the compound of formula (I) is a compound wherein:
m is 1 or 2;
n is 1 or 2;
p is 2;
V is -C(O)-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted heteroaryl, optionally substituted heteroarylalkyl or optionally substituted heteroarylalkenyl;
each R⁴ is independently hydrogen, alkyl, halo, or haloalkyl; and
each R⁵ and R³ is independently hydrogen, oxo, alkyl, halo or haloalkyl.

72. The compound for use as in Claim 71 wherein the compound of formula (I) is a compound wherein:
m is 1 or 2;
n is 1;
p is 2;
V is -C(O)-;
R¹ is hydrogen or alkyl;
R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl;
R³ is optionally substituted heteroaryl, optionally substituted heteroarylalkyl or optionally substituted heteroarylalkenyl;
each R⁴ is independently hydrogen, alkyl, halo, or haloalkyl;
R⁵ is hydrogen, oxo, alkyl, halo or haloalkyl; and
each R⁶ independently hydrogen, oxo, alkyl, halo or haloalkyl.

73. The compound for use as in Claim 65 wherein the compound of formula (I) is selected from the group consisting of the following:
*N*-[2-(3-Chlorophenyl)-ethyl]-6-[4-(3-methyl-thiophene-2-carbonyl)-piperazin-1-yl]-nicotinamide;
*N*-[2-(3-Chlorophenyl)-ethyl]-6-{4-[2-(2-chloro-pyridin-3-yl)-acetyl]-piperazin-1-yl}-nicotinamide;
6-[4-(2-Chloro-pyridine-3-carbonyl)-piperazin-1-yl]-*N*-(4-phenyl-butyl)-nicotinamide;
*N*-[2-(3-Chlorophenyl)-ethyl]-6-[4-(thiophene-2-carbonyl)-piperazin-1-yl]-nicotinamide;
6-[4-(2-Chloro-pyridine-3-carbonyl)-piperazin-1-yl]-*N*-(3-phenyl-propyl)-nicotinamide;
*N*-(3-Phenyl-propyl)-6-[4-(thiophene-2-carbonyl)-piperazin-1-yl]-nicotinamide;
*N*-Phenethyl-6-[4-(thiophene-2-carbonyl)-piperazin-1-yl]-nicotinamide;
*N*-(1-Methyl-3-phenyl-propyl)-6-[4-(thiophene-2-carbonyl)-piperazin-1-yl]-nicotinamide;
*N*-(4-Phenyl-butyl)-6-[4-(thiophene-2-carbonyl)-piperazin-1-yl]-nicotinamide;
6-[4-(2-Chloro-pyridine-3-carbonyl)-piperazin-1-yl]-*N*-(1-methyl-3-phenyl-propyl)-nicotinamide;
6-[4-(2-Chloro-pyridine-3-carbonyl)-piperazin-1-yl]-*N*-phenethyl-nicotinamide;
6-[4-(2-Chloro-pyridine-3-carbonyl)-piperazin-1-yl]-*N*-(tetrahydro-furan-2-ylmethyl)-nicotinamide;
6-[4-(3-Methyl-thiophene-2-carbonyl)-piperazin-1-yl]-*N*-(3-phenyl-propyl)-nicotinamide;
*N*-(1-Methyl-3-phenyl-propyl)-6-[4-(3-methyl-thiophene-2-carbonyl)-piperazin-1-yl]-nicotinamide;
6-[4-(3-Methyl-thiophene-2-carbonyl)-piperazin-1-yl]-*N*-(4-phenyl-butyl)-nicotinamide;
6-[4-(3-Methyl-thiophene-2-carbonyl)-piperazin-1-yl]-*N*-phenethyl-nicotinamide;
6-[4-(3-Methyl-thiophene-2-carbonyl)-piperazin-1-yl]-*N*-(tetrahydro-furan-2-ylmethyl)-nicotinamide;
*N*-(3-Imidazol-1-yl-propyl)-6-[4-(thiophene-2-carbonyl)-piperazin-1-yl]-nicotinamide;
*N*-(Tetrahydro-furan-2-ylmethyl)-6-[4-(thiophene-2-carbonyl)-piperazin-1-yl]-nicotinamide;
*N*-[2-(3H-Imidazol-4-yl)-ethyl]-6-[4-(thiophene-2-carbonyl)-piperazin-1-yl]-nicotinamide;
*N*-(3-Imidazol-1-yl-propyl)-6-[4-(3-methyl-thiophene-2-carbonyl)-piperazin-1-yl]-nicotinamide;
*N*-[2-(3H-Imidazol-4-yl)-ethyl]-6-[4-(3-methyl-thiophene-2-carbonyl)-piperazin-1-yl]-nicotinamide;
6-[4-(2-Chloro-pyridine-3-carbonyl)-piperazin-1-yl]-*N*-(3-imidazol-1-yl-propyl)-nicotinamide; and
6-[4-(2-Chloro-pyridine-3-carbonyl)-piperazin-1-yl]-*N*-[2-(3H-imidazol-4-yl)-ethyl]-nicotinamide.

74. The compound for use as in Claim 45 wherein the compound of formula (I) is a compound wherein:
m is 1 or 2;
n is 1 or 2;
pis2;
V is -C(O)-;
R¹ is hydrogen or alkyl;
R² is selected from the group consisting of hydrogen, -R⁷-OR⁸, -R⁷-N(R⁸)₂, -R⁷-S(O)ₜR¹⁰ (where t is 0, 1 or 2), alkyl, alkenyl, optionally substituted aryl, optionally substituted aralkyl, optionally substituted aralkenyl, optionally substituted cycloalkyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted aralkyl or optionally substituted aralkenyl;
each R⁴ is independently hydrogen, alkyl, alkenyl, halo, haloalkyl, aryl or -R⁹-OR⁸;
each R⁵ and R⁶ is independently hydrogen, oxo, alkyl, alkenyl, halo, haloalkyl or aryl;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocylalkyl, heteroaryl or heteroarylalkyl;
R⁹ is a direct bond or a straight or branched alkylene chain; and
R¹⁰ is alkyl, aryl or aralkyl.

75. The compound for use as in Claim 74 wherein the compound of formula (I) is a compound wherein:
m is 1 or 2;
n is 1 or 2;
pis2;
V is -C(O)-;
R¹ is hydrogen or alkyl;
R² is selected from the group consisting of -R⁷-OR⁸, -R⁷-N(R⁸)₂, -R⁷S(O)ₜR¹⁰ (where t is 0, 1 or 2), alkyl, alkenyl, optionally substituted cycloalkyl, optionally substituted cycloalkylalkyl or cycloalkylalkenyl;
R³ is optionally substituted aralkyl or optionally substituted aralkenyl;
each R⁴ is independently hydrogen, alkyl, halo, or haloalkyl;
each R⁵ and R⁶ is independently hydrogen, oxo, alkyl, halo or haloalkyl;
each R⁷ is a straight or branched alkylene chain;
each R⁸ is independently hydrogen, alkyl, haloalkyl, cycloalkyl, cycloalkylalkyl, aryl and aralkyl; and
R¹⁰ is alkyl, aryl or aralkyl.

76. The compound for use in Claim 75 wherein the compound of formula (I) is a compound wherein:
m is 1 or 2;
n is 1;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2) or alkyl;
R³ is optionally substituted aralkyl or optionally substituted aralkenyl;
each R⁴ is independently hydrogen, halo or haloalkyl;
R⁵ is hydrogen;
each R⁶ is hydrogen;
R⁷ is a straight or branched alkylene chain;
R⁸ is hydrogen or alkyl; and
R¹⁰ is alkyl, aryl or aralkyl.

77. The compound for use as in Claim 76 wherein the compound of formula (I) is selected from the group consisting of the following:
6-{4-[2-(4-Chlorophenyl)-propionyl]-piperazin-1-yl}-*N*-(3-methylbutyl)-nicotinamide;
6-{4-[2-(2-Chloro-6-fluoro-phenyl)-acetyl]-piperazin-1-yl}-*N*-(3-ethoxy-propyl)-nicotinamide;
6-{4-[3-(3,4-Difluoro-phenyl)-propionyl]-piperazin-1-yl}-*N*-(3-dimethylaminopropyl)-nicotinamide;
6-{4-[3-(3,4-Difluoro-phenyl)-propionyl]-piperazin-1-yl}-*N*-(3-ethoxy-propyl)-nicotinamide;
6-{4-[2-(4-Chlorophenyl)-propionyl]-piperazin-1-yl}-*N*-(3-ethoxy-propyl)-nicotinamide;
*N*-(3-Ethoxy-propyl)-6-[4-(2-phenyl-butyryl)-piperazin-1-yl]-nicotinamide;
*N*-(2-Ethylsulfanyl-ethyl)-6-[4-(2-o-tolyl-acetyl)-piperazin-1-yl]-nicotinamide;
6-[4-(3-Methyl-pentanoyl)-piperazin-1-yl]-*N*-(4-phenyl-butyl)-nicotinamide;
*N*-Butyl-6-{4-[3-(3,4-difluoro-phenyl)-propionyl]-piperazin-1-yl}-nicotinamide;
6-{4-[2-(2-Chloro-6-fluoro-phenyl)-acetyl]-piperazin-1-yl}-*N*-(3-dimethylaminopropyl)-nicotinamide;
6-{4-[3-(3,4-Difluoro-phenyl)-propionyl]-piperazin-1-yl}-*N*-(2-ethylsulfanyl-ethyl)-nicotinamide;
*N*-(3-Methoxy-propyl)-6-[4-(2-phenyl-butyryl)-piperazin-1-yl]-nicotinamide;
*N*-Butyl-6-[4-(2-phenyl-butyryl)-piperazin-1-yl]-nicotinamide;
*N*-(3-Butoxy-propyl)-6-{4-[2-(4-chloro-phenyl)-propionyl]-piperazin-1-yl}-nicotinamide;
*N*-(3-Methoxy-propyl)-6-[4-(2-o-tolyl-acetyl)-piperazin-1-yl]-nicotinamide;
*N*-(3-Methyl-butyl)-6-[4-(2-phenyl-butyryl)-piperazin-1-yl]-nicotinamide;
*N*-(1-Methyl-butyl)-6-[4-(2-p-tolyl-acetyl)-piperazin-1-yl]-nicotinamide;
*N*-Butyl-6-{4-[2-(2-chloro-6-fluoro-phenyl)-acetyl]-piperazin-1-yl}-nicotinamide;
*N*-(2-Methyl-butyl)-6-[4-(2-o-tolyl-acetyl)-piperazin-1-yl]-nicotinamide;
6-{4-[3-(3,4-Difluoro-phenyl)-propionyl]-piperazin-1-yl}-*N*-(2-methylbutyl)-nicotinamide;
*N*-(3-Isopropoxy-propyl)-6-[4-(2-o-tolyl-acetyl)-piperazin-1-yl]-nicotinamide;
6-{4-[3-(3,4-Difluoro-phenyl)-propionyl]-piperazin-1-yl}-*N*-(3-methylbutyl)-nicotinamide;
6-{4-[3-(3,4-Difluoro-phenyl)-propionyl]-piperazin-1-yl}-*N*-(3-methoxy-propyl)-nicotinamide;
*N*-(1-Methyl-butyl)-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-(2-methylbutyl)-nicotinamide;
6-{4-[2-(2-Chloro-6-fluoro-phenyl)-acetyl]-piperazin-1-yl}-*N*-(1-methylbutyl)-nicotinamide;
*N*-(2-Methyl-butyl)-6-[4-(2-phenyl-butyryl)-piperazin-1-yl]-nicotinamide;
*N*-(1,3-Dimethylbutyl)-6-[4-(2-phenyl-butyryl)-piperazin-1-yl]-nicotinamide;
6-{4-[2-(4-Chlorophenyl)-propionyl]-piperazin-1-yl}-*N*-(2-methylbutyl)-nicotinamide;
6-{4-[2-(2-Chloro-6-fluoro-phenyl)-acetyl]-piperazin-1-yl}-*N*-(3-isopropoxy-propyl)-nicotinamide;
*N*-(1,3-Dimethylbutyl)-6-[4-(2-p-tolyl-acetyl)-piperazin-1-yl]-nicotinamide;
6-{4-[3-(3,4-Difluoro-phenyl)-propionyl]-piperazin-1-yl}-*N*-hexyl-nicotinamide;
*N*-(1-Methyl-butyl)-6-[4-(2-phenyl-butyryl)-piperazin-1-yl]-nicotinamide;
6-{4-[2-(4-Chlorophenyl)-propionyl]-piperazin-1-yl}-*N-(*1-methylbutyl)-nicotinamide;
6-{4-[2-(4-Chlorophenyl)-propionyl]-piperazin-1-yl}-*N*-(1,3-dimethylbutyl)-nicotinamide;
*N*-Butyl-6-[4-(2-p-tolyl-acetyl)-piperazin-1-yl]-nicotinamide;
*N*-(1,3-Dimethylbutyl)-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-(1,3-dimethylbutyl)-nicotinamide;
*N-*(3-Methyl-butyl)-6-[4-(2-p-tolyl-acetyl)-piperazin-1-yl]-nicotinamide;
*N*-Butyl-6-[4-(2-o-tolyl-acetyl)-piperazin-1-yl]-nicotinamide;
*N*-(3-Butoxy-propyl)-6-[4-(2-p-tolyl-acetyl)-piperazin-1-yl]-nicotinamide;
*N*-Pentyl-6-[4-(2-p-tolyl-acetyl)-piperazin-1-yl]-nicotinamide;
*N*-(3-Butoxy-propyl)-6-[4-(2-phenyl-butyryl)-piperazin-1-yl]-nicotinamide;
*N*-Pentyl-6-[4-(2-phenyl-butyryl)-piperazin-1-yl]-nicotinamide;
*N*-(2-Ethylsulfanyl-ethyl)-6-[4-(2-p-tolyl-acetyl)-piperazin-1-yl]-nicotinamide;
6-{4-[2-(4-Chlorophenyl)-propionyl]-piperazin-1-yl}-*N*-(2-ethylsulfanyl-ethyl)-nicotinamide;
*N-*(3*-*Ethoxy-propyl)-6-[4-(2-o-tolyl-acetyl)-piperazin-1-yl]-nicotinamide;
6-{4-[2-(2-Chloro-6-fluoro-phenyl)-acetyl]-piperazin-1-yl)-*N*-(3-methoxy-propyl)-nicotinamide;
6-{4-[3-(3,4-Difluoro-phenyl)-propionyl]-piperazin-1-yl}-*N*-(3-isopropoxy-propyl)-nicotinamide;
6-{4-[2-(4-Chlorophenyl)-propionyl]-piperazin-1-yl)-*N*-pentyl-nicotinamide;
*N*-(3-Butoxy-propyl)-6-{4-[2-(2-chloro-6-fluoro-phenyl)-acetyl]-piperazin-1-yl}-nicotinamide;
6-(4-[2-(2-Chloro-6-fluoro-phenyl)-acetyl]-piperazin-1-yl}-*N*-phexyl-nicotinamide;
6-(4-[2-(4-Chlorophenyl)-propionyl]-piperazin-1-yl)-*N*-(3-methoxy-propyl)-nicotinamide;
*N*-Hexyl-6-[4-(2-o-tolyl-acetyl)-piperazin-1-yl]-nicotinamide;
6-{4-[3-(3,4-Difluoro-phenyl)-propionyl]-piperazin-1-yl)-*N*-(4-phenyl-butyl)-nicotinamide;
*N*-(3-Isopropoxy-propyl)-6-[4-(2-p-tolyl-acetyl)-piperazin-1-yl]-nicotinamide;
6-{4-[3-(3,4-Difluoro-phenyl)-propionyl]-piperazin-1-yl}-*N*-(1-methylbutyl)-nicotinamide;
6-{4-[3-(3,4-Difluoro-phenyl)-propionyl]-piperazin-1-yl}-*N*-pentyl-nicotinamide;
6-{4-[2-(4-Chlorophenyl)-propionyl]-piperain-1-yl)-*N*-hexyl-nicotinamide;
*N*-Butyl-6-{4-[2-(4-chloro-phenyl)-propionyl]-piperazin-1-yl}-nicotinamide;
6-{4-[2-(2-Chloro-6-fluoro-phenyl)-acetyl]-piperazin-1-yl}-*N*-(2-ethylsulfanyl-ethyl)-nicotinamide;
6-{4-[2-(2-Chloro-6-fluoro-phenyl)-acetyl]-piperazin-1-yl}-*N*-pentyl-nicotinamide;
*N*-Hexyl-6-[4-(2-phenyl-butyryl)-piperazin-1-yl]-nicotinamide;
*N*-(3-Isopropoxy-propyl)-6-[4-(2-phenyl-butyryl)-piperazin-1-yl]-nicotinamide;
*N*-Pentyl-6-[4-(2-o-tolyl-acetyl)-piperazin-1-yl]-nicotinamide;
*N*-Hexyl-6-[4-(2-p-tolyl-acetyl)-piperazin-1-yl]-nicotinamide;
6-{4-[3-(3,4-Difluoro-phenyl)-propionyl]-piperazin-1-yl}-*N*-(1,3-dimethylbutyl)-nicotinamide;
6-[4-(Naphthalene-2-carbonyl)-piperazin-1-yl]-*N*-pentyl-nicotinamide;
6-{4-[2-(4-Chlorophenyl)-propionyl]-piperazin-1-yl}-*N*-(3-dimethylamino-propyl)-nicotinamide;
*N*-(3-Dimethylamino-propyl)-6-[4-(2-phenyl-butyryl)-piperazin-1-yl]-nicotinamide;
*N*-(3-Dimethylamino-propyl)-6-[4-(2-p-tolyl-acetyl)-piperazin-1-yl]-nicotinamide;
*N*-(3-Dimethylamino-propyl)-6-[4-(2-o-tolyl-acetyl)-piperazin-1-yl]-nicotinamide;
*N*-(3-Butoxy-propyl)-6-{4-[3-(3,4-difluoro-phenyl)-propionyl]-piperazin-1-yl}-nicotinamide;
*N*-(3-Methoxy-propyl)-6-[4-(2-p-tolyl-acetyl)-piperazin-1-yl]-nicotinamide;
*N*-(2-Methyl-butyl)-6-[4-(2-p-tolyl-acetyl)-piperazin-1-yl]-nicotinamide;
*N*-(3-Butoxy-propyl)-6-[4-(2-o-tolyl-acetyl)-piperazin-1-yl]-nicotinamide; and
6-{4-[2-(4-Chlorophenyl)-propionyl]-piperazin-1-yl}-*N*-(3-isopropoxy-propyl)-nicotinamide.

78. The compound for use as in Claim 74 wherein the compound of formula (I) is a compound wherein:
m is 1 or 2;
n is 1 or 2;
p is 2;
V is -C(O)-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
R³ is optionally substituted aralkyl or optionally substituted aralkenyl;
each R⁴ is independently hydrogen, alkyl, halo, or haloalkyl; and
each R⁵ and R⁶ is independently hydrogen, oxo, alkyl, halo or haloalkyl.

79. The compound for use as in Claim 78 wherein the compound of formula (I) is a compound wherein:
m is 1 or 2;
n is 1;
p is 2;
V is -C(O)-;
R¹ is hydrogen or alkyl;
R² is selected from the group consisting of optionally substituted aryl, optionally substituted aralkyl, optionally substituted heterocyclyl, optionally substituted heterocyclylalkyl, optionally substituted heteroaryl and optionally substituted heteroarylalkyl;
R³ is optionally substituted aralkyl or optionally substituted aralkenyl;
each R⁴ is independently hydrogen, alkyl, halo or haloalkyl; and
R⁵ is hydrogen, oxo, alkyl, halo or haloalkyl; and
each R⁶ is independently hydrogen, oxo, alkyl, halo or haloalkyl.

80. The compound for use as in Claim 79 wherein the compound of formula (I) is selected from the group consisting of the following:
6-[4-(2-Phenyl-butyryl)-piperazin-1-yl]-*N*-(3-phenyl-propyl)-nicotinamide;
6-{4-[3-(3,4-Difluoro-phenyl)-propionyl]-piperazin-1-yl}-*N*-phenethyl-nicotinamide;
*N*-[2-(3-Chlorophenyl)-ethyl]-6-[4-(2-o-tolyl-acetyl)-piperazin-1-yl]-nicotinamide;
6-(4-[2-(2-Chloro-6-fluoro-phenyl)-acetyl]-piperazin-1-yl}-*N*-(3-phenyl-propyl)-nicotinamide;
*N*-(1-Methyl-3-phenyl-propyl)-6-[4-(2-p-tolyl-acetyl)-piperazin-1-yl]-nicotinamide;
*N*-[2-(3-Chlorophenyl)-ethyl]-6-[4-(2-phenyl-butyryl)-piperazin-1-yl]-nicotinamide;
6-{4-[2-(2-Chloro-6-fluoro-phenyl)-acetyl]-piperazin-1-yl}-*N*-phenethylnicotinamide;
*N*-(3*-*Phenyl-propyl)-6-[4-(2-o-tolyl-acetyl)-piperazin-1-yl]-nicotinamide;
6-{4-[2-(2-Chloro-6-fluoro-phenyl)-acetyl]-piperazin-1-yl}-*N*-[2-(3-chloro-phenyl)-ethyl]-nicotinamide;
6-{4-[2-(4-Chlorophenyl)-propionyl]-piperazin-1-yl}-*N*-(3-phenyl-propyl)-nicotinamide;
6-{4-[2-(4-Chlorophenyl)-propionyl]-piperazin-1-yl}-*N*-(1-methyl-3-phenyl-propyl)-nicotinamide;
*N*-[2-(3-Chlorophenyl)-ethyl]-6-{4-[2-(4-chloro-phenyl)-propionyl]-piperazin-1-yl}-nicotinamide;
*N*-[2-(3-Chlorophenyl)-ethyl]-6-[4-(2-p-tolyl-acetyl)-piperazin-1-yl]-nicotinamide;
*N*-Phenethyl-6-[4-(2-p-tolyl-acetyl)-piperazin-1-yl]-nicotinamide;
*N*-(3-Phenyl-propyl)-6-[4-(2-p-tolyl-acetyl)-piperazin-1-yl]-nicotinamide;
*N*-(1-Methyl-3-phenyl-propyl)-6-[4-(2-phenyl-butyryl)-piperazin-1-yl]-nicotinamide;
6-{4-[2-(4-Chlorophenyl)-propionyl]-piperazin-1-yl)-*N*-(4-phenyl-butyl)-nicotinamide;
*N*-(4-Phenyl-butyl)-6-[4-(2-phenyl-butyryl)-piperazin-1-yl]-nicotinamide;
6-{4-[2-(2-Chloro-6-fluoro-phenyl)-acetyl]-piperazin-1-yl}-*N*-(1-methyl-3-phenylpropyl)-nicotinamide;
6-[4-[3-(3,4-Difluoro-phenyl)-propionyl]-piperazin-1-yl}-*N*-(3-phenyl-propyl)-nicotinamide;
*N*-phenethyl-6-[4-(2-o-tolyl-acetyl)-piperazin-1-yl]-nicotinamide;
6-{4-[3-(3,4-Difluoro-phenyl)-propionyl]-piperazin-1-yl}-*N*-(1-methyl-3-phenylpropyl)-nicotinamide;
*N*-(4-Phenyl-butyl)-6-[4-(2-0-tolyl-acetyl)-piperazin-1-yl]-nicotinamide;
*N*-(3-Imidazol-1-yl-propyl)-6-[4-(2-phenyl-butyryl)-piperazin-1-yl]-nicotinamide;
6-{4-[2-(2-Chloro-6-fluoro-phenyl)-acetyl]-piperazin-1-yl}-*N*-(4-phenyl-butyl)-nicotinamide;
6-{4-[2-(4-Chlorophenyl)-propionyl]-piperazin-1-yl}-*N*-[2-(3H-imidazol-4-yl)-ethyl]-nicotinamide;
6-{4-[2-(2-Chloro-6-fluoro-phenyl)-acetyl]-piperazin-1-yl}-*N*-(3-imidazol-1-yl-propyl)-nicotinamide;
*N*-[2-(3H-Imidazol-4-yl)-ethyl]-6-[4-(2-p-tolyl-acetyl)-piperazin-1-yl]-nicotinamide;
6-{4-[2-(4-Chlorophenyl)-propionyl]-piperazin-1-yl}-*N*-(3-imidazol-1-yl-propyl)-nicotinamide;
*N*-(2-Ethylsulfanyl-ethyl)-6-[4-(2-phenyl-butyryl)-piperazin-1-yl]-nicotinamide;
*N*-(1-Methyl-3-phenyl-propyl)-6-[4-(2-o-tolyl-acetyl)-piperazin-1-yl]-nicotinamide;
6-{4-[3-(3,4-Difluoro-phenyl)-propionyl]-piperazin-1-yl}-*N*-phenethyl-nicotinamide;
*N*-Phenethyl-6-[4-(2-phenyl-butyryl)-piperazin-1-yl]-nicotinamide;
*N*-(Tetrahydro-furan-2-ylmethyl)-6-[4-(2-p-tolyl-acetyl)-piperazin-1-yl]-nicotinamide;
6-{4-[2-(4-Chlorophenyl)-propionyl]-piperazin-1-yl}-*N*-(tetrahydro-furan-2-ylmethyl)-nicotinamide;
*N*-(4-Phenyl-butyl)-6-[4-(2-p-tolyl-acetyl)-piperazin-1-yl]-nicotinamide;
6-{4-[3-(3,4-Difluoro-phenyl)-propionyl]-piperazin-1-yl}-*N*-(tetrahydro-furan-2-ylmethyl)-nicotinamide;
*N*-(Tetrahydro-furan-2-ylmethyl)-6-[4-(2-o-tolyl-acetyl)-piperazin-1-yl]-nicotinamide;
6-[4-(2-Phenyl-butyryl)-piperazin-1-yl]-*N*-(tetrahydro-furan-2-ylmethyl)-nicotinamide;
6-{4-[3-(3,4-Difluoro-phenyl)-propionyl]-piperazin-1-yl}-*N*-[2-(3H-imidazol-4-yl)-ethyl]-nicotinamide;
*N*-[2-(3H-Imidazol-4-yl)-ethyl]-6-[4-(2-o-tolyl-acetyl)-piperazin-1-yl]-nicotinamide;
*N*-(3-Imidazol-1-yl-propyl)-6-[4-(2-o-tolyl-acetyl)-piperazin-1-yl]-nicotinamide;
6-{4-[3-(3,4-Difluoro-phenyl)-propionyl]-piperazin-1-yl}-*N*-(3-imidazol-1-yl-propyl)-nicotinamide;
*N*-[2-(3H-Imidazol-4-yl)-ethyl]-6-[4-(2-phenyl-butyryl)-piperazin-1-yl]-nicotinamide;
*N*-(3-Imidazol-1-yl-propyl)-6-[4-(2-p-tolyl-acetyl)-piperazin-1-yl]-nicotinamide;
6-(4-[2-(2-Chloro-6-fluoro-phenyl)-acetyl]-piperazin-1-yl)-*N*-(tetrahydro-furan-2-ylmethyl)-nicotinamide; and
6-{4-[2-(2-Chloro-6-fluoro-phenyl)-acetyl]-piperazin-1-yl}-*N*-[2-(3H-imidazol-4-yl)-ethyl]-nicotinamide.

81. The compound for use as in Claim 45 wherein the mammal is a human.

82. The compound for use as in Claim 81 wherein the disease or condition is a disease or condition related to serum levels of triglyceride, VLDL, HDL, LDL, total cholesterol or the process of reverse cholesterol transport.

83. The compound for use as in Claim 81 wherein the disease or condition is a disease or condition related to serum triglyceride levels.

84. The compound for use as in Claim 81 wherein the disease or condition is a disease or condition releated to serum cholesterol levels.

85. The compound for use as in Claim 81 wherein the disease or condition is Type II diabetes.

86. The compound for use as in Claim 81 wherein the disease or condition is obesity.

87. The compound for use as in Claim 81 wherein the disease or condition is dyslipidemia.

88. The compound for use as in Claim 81 wherein the disease or condition is metabolic syndrome.

89. A pharmaceutical composition comprising a pharmaceutically acceptable excipient or carrier and a therapeutically effective amount of a compound of formula (I): 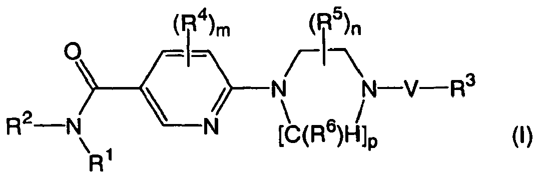 wherein:
m is 1 to 3;
n is 1, 2, 3 or 4;
p is 2 or 3;
V is -C(O)- or -S(O)₂-;
R¹ is hydrogen, alkyl, alkenyl, aryl, aralkyl, or aralkenyl;
R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, aralkyl, where the aryl radical may be optionally substituted, aralkenyl, where the aryl radical may be optionally substituted, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
R³ is selected from the group consisting of -R⁹-OR⁸, -R⁹-N(R⁸)₂, alkenyl, optionally substituted aralkyl, optionally substituted aralkenyl, optionally substituted cycloalkyl, cycloalkylalkyl, where the cycloalkyl radical may be optionally substituted, cycloalkylalkenyl, optionally substituted heterocyclyl, heterocyclylalkyl, where the heterocyclyl radical may be optionally substituted, heterocyclylalkenyl, heteroarylalkyl, where the heteroaryl radical may be optionally substituted and heteroarylalkenyl, where the heteroaryl radical may be optionally substituted;
each R⁴ is independently hydrogen, alkyl, alkenyl, halo, haloalkyl, aryl or -R⁹-OR⁸;
each R⁵ and R⁶ is independently hydrogen, oxo, alkyl, alkenyl, halo, haloalkyl or aryl;
or one R⁵ and one R⁶ may together form an straight or branched alkylene bridge;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl;
each R⁹ is independently a straight or branched alkylene or alkenylene chain; and
R¹⁰ is alkyl, alkenyl, haloalkyl, cycloalkyl, cycloalkylalkyl, aryl, aralkyl, heterocyclyl, heterocyclylalkyl, heteroaryl or heteroarylalkyl;
as a single stereoisomer, a mixture of stereoisomers, or a racemic mixture thereof of stereoisomers;
or as a pharmaceutically acceptable salt, solvate polymorph or prodrug, selected from among ester and amide derivatives of hydroxyl, carboxy, mecapto or amino functional groups in the compounds of formula (I), thereof; and
wherein the optional substituent is one or more substituents independently selected from the group consisting of alkyl, alkenyl, halo, haloalkyl, haloalkenyl, cyano, nitro, aryl, aralkyl, cycloalkyl, cycloalkylalkyl, heterocylyl, heterocyclylalkyl, heteroaryl, heteroarylalkyl, -R⁹-OR⁸, -R⁹-N(R⁸)₂, -R⁹-C(O)R^{a}, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-S(O)ₜOR¹⁰ (where t is 1 or 2), -R⁹-S(O)ₜR¹⁰ (where t is 0, 1 or 2), and -R⁹-S(O)ₜN(R⁸)₂ (where t is 1 or 2).

90. The pharmaceutical composition of Claim 89 wherein the therapeutically effective amound of the compound of formula (I) is an amount effective to modulate a lipid level in a mamal when administered to the mammal.

91. The pharmaceutical composition of Claim 90 wherein the lipid is triglyceride.

92. The pharmaceutical composition of Claim 90 wherein the lipid is cholesterol.

93. The pharmaceutical composition of Claim 89 wherein the therapeutically effective amound of the compound of formula (I) is an amount effective to modulate HDL-cholesterol levels when administered to a mammal.

94. A compound of formula (I): 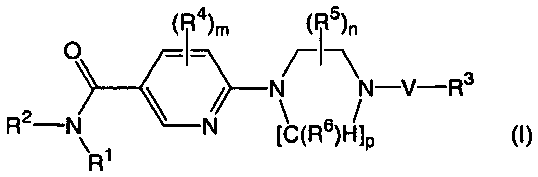 wherein:
m is 1, 2 or 3;
n is 1, 2, 3 or 4;
p is 2, 3 or 4;
V is -C(O)-, -S(O)- or -S(O)₂-;
R¹ is hydrogen, alkyl, alkenyl, aryl, aralkyl or aralkenyl;
R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, aralkyl, where the aryl radical may be optionally substituted, aralkenyl, where the aryl radical may be optionally substituted, optionally substituted cycloalkylalkyl, cycloalkylalkenyl, optionally substituted heterocyclyl, heterocyclylalkyl, where the heterocyclyl radical may be optionally substituted, heterocyclylalkenyl, optionally substituted heteroaryl, heteroarylalkyl, where the heteroaryl radical may be optionally substituted and heteroarylalkenyl, where the heteroaryl radical may be optionally substituted;
R³ is cyclopropyl substituted by optionally substituted aryl or optionally substituted heteroaryl;
each R⁴ is independently hydrogen, alkyl, alkenyl, halo, haloalkyl, aryl, cyano, nitro, -R⁹-OR⁸, -R⁹-N(R⁸)₂ or -S(O)ₜR¹⁰ (where t is 0, 1 or 2);
each R⁵ and R⁶ is independently hydrogen, oxo, alkyl, alkenyl, halo, haloalkyl or aryl;
or one R⁵ and one R⁶ may together form an straight or branched alkylene bridge;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocylylalkyl, heteroaryl or heteroarylalkyl;
each R⁹ is independently a direct bond or a straight or branched alkylene or alkenylene chain; and
R¹⁰ is alkyl, alkenyl, haloalkyl, cycloalkyl, cycloalkylalkyl, aryl, aralkyl, heterocyclyl, heterocyclylalkyl, heteroaryl or heteroarylalkyl;
as a single stereoisomer, a mixture of stereoisomers, a racemic mixture thereof of stereoisomers, or as a tautomer;
or as a pharmaceutically acceptable salt, solvate polymorph or prodrug, selected from among ester and amide derivatives of hydroxyl, carboxy, mercapto or amino functional groups in the compounds of formula (I) thereof; and
wherein the optional substituent is one or more substituents independently selected from the group consisting of alkyl, alkenyl, halo, haloalkyl, haloalkenyl, cyano, nitro, aryl, aralkyl, cycloalkyl, cycloalkylalkyl, heterocylyl, heterocyclylalkyl, heteroaryl, heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (where t is 1 or 2), -R⁹-S(O)ₜOR¹⁰ (where t is 1 or 2), -R⁹-S(O)ₜR¹⁰ (where t is 0, 1 or 2), and -R⁹-S(O),N(R⁸)₂ (where t is 1 or 2).

95. The compound of Claim 94 wherein:
m is 1 or 2;
n is 1 or 2;
p is 2;
V is -C(O)-;
R¹ is hydrogen or alkyl;
R² is selected from the group consisting of hydrogen, -R⁷-OR⁸, -R⁷-N(R⁸)₂, -R⁷-S(O)ₜR¹⁰(where t is 0, 1 or 2), alkyl, alkenyl, optionally substituted aryl, optionally substituted aralkyl, optionally substituted aralkenyl, optionally substituted cycloalkylalkyl, cycloalkylalkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
each R⁴ is independently hydrogen, alkyl, alkenyl, halo, haloalkyl, aryl or -R⁹-OR⁸;
each R⁵ and R⁶ is independently hydrogen, oxo, alkyl, alkenyl, halo, haloalkyl or aryl;
each R⁷ is independently a straight or branched alkylene or alkenylene chain;
each R⁸ is independently hydrogen, alkyl, alkenyl, haloalkyl, cycloalkyl, cycloalkylalkyl, aryl, aralkyl, heterocyclyl, heterocyclylalkyl, heteroaryl or heteroarylalkyl;
R⁹ is a direct bond or a straight or branched alkylene chain; and
R¹⁰ is alkyl, aryl or aralkyl.

96. The compound of Claim 95 wherein:
m is 1 or 2;
n is 1 or 2;
pis 2;
V is -C(O)-;
R¹ is hydrogen or alkyl;
R² is selected from the group consisting of optionally substituted aryl, optionally substituted aralkyl, optionally substituted aralkenyl, optionally substituted heterocyclyl, optionally substituted heterocyclylalkyl, heterocyclylalkenyl, optionally substituted heteroaryl, optionally substituted heteroarylalkyl and optionally substituted heteroarylalkenyl;
each R⁴ is independently hydrogen, alkyl, halo, or haloalkyl; and
each R⁵ and R⁶ is independently hydrogen, oxo, alkyl, halo or haloalkyl.

97. The compound of Claim 96 wherein:
m is 1;
n is 1;
p is 2;
V is -C(O)-;
R¹ is hydrogen or alkyl;
R² is optionally substituted aralkyl, optionally substituted heteroarylalkyl, or optionally substituted heterocyclylalkyl;
R³ is cyclopropyl substituted by phenyl;
R⁴ is hydrogen, alkyl, halo or haloalkyl;
AS is hydrogen, oxo, alkyl, halo or haloalkyl; and
each R⁶ is hydrogen.

98. The compound of Claim 97 selected from the group consisting of the following:
6-[4-(2-Phenyl-cyclopropanecarbonyl)-piperazin-1-yl]-(3-phenyl-propyl)-nicotinamide;
*N*-(4-Phenyl-butyl)-6-[4-(2-phenyl-cyclopropanecarbonyl)-piperazin-1-yl]-nicotinamide;
*N*-(1-Methyl-3-phenyl-propyl)-6-[4-(2-phenyl-cyclopropanecarbonyl)-piperazin-1-yl]-nicotinamide;
*N*-Phenethyl-6-[4-(2-phenyl-cyclopropanecarbonyl)-piperazin-1-yl]-nicotinamide;
6-[4-(2-Phenyl-cyclopropanecarbonyl)-piperazin-1-yl]-*N*-(tetrahydro-furan-2-ylmethyl)-nicotinamide;
*N*-[2-(3H-Imidazol-4-yl)-ethyl]-6-[4-(2-phenyl-cyclopropanecarbonyl)-piperazin-1-yl]-nicotinamide; and
*N*-(3-Imidazol-1-yl-propyl)-6-[4-(2-phenyl-cyclopropanecarbonyl)-piperazin-1-yl]-nicotinamide.

99. The compound of Claim 95 wherein:
m is 1 or 2;
n is 1 or 2;
p is 2;
V is -C(O)-;
R¹ is hydrogen or alkyl;
R² is selected from the group consisting of -R⁷-OR⁸, -R⁷-N(R⁸)₂, -R⁷-S(O)ₜR¹⁰ (where t is 0, 1 or 2), alkyl, alkenyl, optionally substituted cycloalkylalkyl or cycloalkylalkenyl;
each R⁴ is independently hydrogen, alkyl, halo, or haloalkyl;
each R⁵ and R⁶ is independently hydrogen, oxo, alkyl, halo or haloalkyl;
each R⁷ is a straight or branched alkylene chain;
each R⁸ is independently hydrogen, alkyl, haloalkyl, cycloalkyl, cycloalkylalkyl, aryl and aralkyl; and
R¹⁰ is alkyl, aryl or aralkyl.

100. The compound of Claim 99 wherein:
m is 1;
n is 1;
p is 2;
V is -C(O)-;
R¹ is hydrogen or alkyl;
R² is selected from the group consisting of alkyl, -R⁷-OR⁸, -R⁷-N(R⁸)₂, or -R⁷-S(O)ₜR¹⁰ (where t is 0);
R³ is cyclopropyl substituted by phenyl;
R⁴ is hydrogen;
R⁵ is hydrogen;
each R⁶ is hydrogen;
R⁷ is a straight or branched alkylene chain;
R⁸ is hydrogen or alkyl; and
R¹⁰ is alkyl, aryl or aralkyl.

101. The compound of Claim 100 selected from the group consisting of the following:
*N*-(3-Ethoxy-propyl)-6-[4-(2-phenyl-cyclopropanecarbonyl)-piperazin-1-yl]-nicotinamide;
*N*-(2-Ethylsulfanyl-ethyl)-6-[4-(2-phenyl-cyclopropanecarbonyl)-piperazin-1-yl]-nicotinamide;
*N*-(1,3-Dimethylbutyl)-6-[4-(2-phenyl-cyclopropanecarbonyl)-piperazin-1-yl]-nicotinamide;
*N*-(3-Methyl-butyl)-6-[4-(2-phenyl-cyclopropanecarbonyl)-piperazin-1-yl]-nicotinamide;
*N*-(3-Methoxy-propyl)-6-[4-(2-phenyl-cyclopropanecarbonyl)-piperazin-1-yl]-nicotinamide;
*N*-(3-Butoxy-propyl)-6-[4-(2-phenyl-cyclopropanecarbonyl)-piperazin-1-yl]-nicotinamide;
*N*-Pentyl-6-[4-(2-phenyl-cyclopropanecarbonyl)-piperazin-1-yl]-nicotinamide;
*N*-(3-Dimethylamino-propyl)-6-[4-(2-phenyl-cyclopropanecarbonyl)-piperazin-1-yl]-nicotinamide;
*N*-(3-Isopropoxy-propyl)-6-[4-(2-phenyl-cyclopropanecarbonyl)-piperazin-1-yl]-nicotinamide;
*N*-(1-Methyl-butyl)-6-[4-(2-phenyl-cyclopropanecarbonyl)-piperazin-1-yl]-nicotinamide;
*N*-Butyl-6-[4-(2-phenyl-cyclopropanecarbonyl)-piperazin-1-yl]-nicotinamide;
*N*-Hexyl-6-[4-(2-phenyl-cyclopropanecarbonyl)-piperazin-1-yl]-nicotinamide; and
*N*-(2-Methyl-butyl)-6-[4-(2-phenyl-cyclopropanecarbonyl)-piperazin-1-yl]-nicotinamide.

## Patentansprüche

1. Verwendung einer Verbindung der Formel (I): 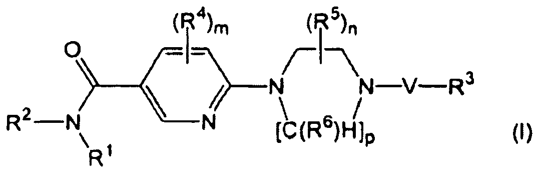 worin:
m ist 1, 2 oder 3;
n ist 1, 2, 3 oder 4;
p ist 2, 3 oder 4;
V ist -C(O)-, -S(O)- oder -S(O)₂-;
R¹ ist Wasserstoff, Alkyl, Alkenyl, Aryl, Aralkyl, Aralkenyl oder Cycloalkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, Aralkyl, wobei der Arylrest gegebenenfalls substituiert sein kann, Aralkenyl, wobei der Arylrest gegebenenfalls substituiert sein kann, gegebenenfalls substituiertem Cycloalkyl, gegebenenfalls substituiertem Cycloalkylalkyl, Cycloalkylalkenyl, gegebenenfalls substituiertem Heterocyclyl, Heterocyclylalkyl, wobei der Heterocyclylrest gegebenenfalls substituiert sein kann, Heterocyclylalkenyl, gegebenenfalls substituiertem Heteroaryl, Heteroarylalkyl, wobei der Heteroarylrest gegebenenfalls substituiert sein kann, und Heteroarylalkenyl, wobei der Heteroarylrest gegebenenfalls substituiert sein kann;
R³ ist ausgewählt aus der Gruppe bestehend aus Wasserstoff, -R⁹-OR⁸, -R⁹-N(R⁸)₂, Alkyl, Alkenyl, gegebenenfalls substituiertem Aryl, Aralkyl, wobei der Arylrest gegebenenfalls substituiert sein kann, Aralkenyl, wobei der Arylrest gegebenenfalls substituiert sein kann, gegebenenfalls substituiertem Cycloalkyl, gegebenenfalls substituiertem Cycloalkylalkyl, Cycloalkylalkenyl, gegebenenfalls substituiertem Heterocyclyl, Heterocyclylalkyl, wobei der Heterocyclylrest gegebenenfalls substituiert sein kann, Heterocyclylalkenyl, gegebenenfalls substituiertem Heteroaryl, Heteroarylalkyl, wobei der Heteroarylrest gegebenenfalls substituiert sein kann, und Heteroarylalkenyl, wobei der Heteroarylrest gegebenenfalls substituiert sein kann;
jedes R⁴ ist unabhängig Wasserstoff, Alkyl, Alkenyl, Halogen, Halogenalkyl, Aryl, Cyano, Nitro, -R⁹-OR⁸, -R⁹-N(R⁸)₂ oder -S(O)ₜR¹⁰ (wobei t 0, 1 oder 2 ist);
jedes R⁵ und R⁶ ist unabhängig Wasserstoff, Oxo, Alkyl, Alkenyl, Halogen, Halogenalkyl oder Aryl;
oder ein R⁵ und ein R⁶ können zusammen eine geradkettige oder verzweigte Alkylenbrücke bilden;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
jedes R⁹ ist unabhängig eine direkte Bindung oder eine gerade oder verzweigte Alkylen- oder Alkenylenkette; und
R¹⁰ ist Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
als ein einzelnes Stereoisomer, eine Mischung von Stereoisomeren, eine racemische Mischung davon von Stereoisomeren, oder als ein Tautomer;
oder als ein pharmazeutisch verträgliches Salz, Solvat, Polymorph oder Prodrug, ausgewählt aus Ester- und Amidderivaten von funktionellen Hydroxyl-, Carboxy-, Mercapto- oder
Amino-Gruppen in den Verbindungen der Formel (I), davon für die Herstellung eines Medikaments zum Behandeln einer bzw. eines durch Stearyl-CoA-Desaturase (SCD) vermittelten Krankheit oder Zustands in einem Säuger, wobei die Krankheit oder der Zustand ausgewählt ist aus der Gruppe bestehend aus Typ II-Diabetes, gestörter Glukosetoleranz, Insulinresistenz, Hypertension, Fettleibigkeit, Hypertriglyceridämie, niedrigem HDL, Lipidämie, Dyslipidämie, Mikroalbuminämie, Hyperurikämie, Hyperleptinämie, dem metabolischen Syndrom und einer beliebigen Kombination von diesen; und
wobei es sich bei dem gegebenenfalls vorhandenen Substituenten um einen oder mehrere Substituenten handelt, die unabhängig ausgewählt sind aus der Gruppe bestehend aus Alkyl, Alkenyl, Halogen, Halogenalkyl, Halogenalkenyl, Cyano, Nitro, Aryl, Aralkyl, Cycloalkyl, Cycloalkylalkyl, Heterocyclyl, Heterocyclylalkyl, Heteroaryl, Heteroarylalkyl, -R⁹-OR⁸, -R⁹-N(R⁸)₂, -R⁹-C(O)R⁸, -R⁹-C(O)OR⁸, -R⁹-C(O)N(R^{e})₂, -R⁹-N(R⁸)C(O)OR¹⁰, -R⁹-N(R⁸)C(O)R¹⁰, -R⁹-N(R⁸)(S(O)ₜR¹⁰) (wobei t 1 oder 2 ist), -R⁹-S(O)ₜOR¹⁰ (wobei t 1 oder 2 ist), -R⁹-S(O)ₜR¹⁰ (wobei t 0, 1 oder 2 ist), und -R⁹-S(O)ₜN(R⁸)₂ (wobei t 1 oder 2 ist).

2. Verwendung der Verbindung nach Anspruch 1, wobei die Verbindung der Formel (I) eine Verbindung ist, worin:
m ist 1 oder 2;
n ist 1 oder 2;
p ist 2 oder 3;
V ist -C(O)- oder -S(O)₂-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl, wobei die gegebenenfalls substituierten Gruppen wie in Anspruch 1 definiert sind;
R³ ist Alkyl, Alkenyl oder -R⁹-N(R⁸)₂;
jedes R⁴ ist unabhängig Wasserstoff, Alkyl, Alkenyl, Halogen, Halogenalkyl, Aryl oder -R⁹-OR⁸;
jedes R⁵ und R⁶ ist unabhängig Wasserstoff, Oxo, Alkyl, Alkenyl, Halogen, Halogenalkyl oder Aryl;
oder ein R⁵ und ein R⁶ können zusammen eine geradkettige oder verzweigte Alkylenbrücke bilden;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
jedes R⁹ ist unabhängig eine direkte Bindung oder eine gerade oder verzweigte Alkylenkette; und
R¹⁰ ist Alkyl, Aryl oder Aralkyl.

3. Verwendung der Verbindung nach Anspruch 2, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Cycloalkyl, gegebenenfalls substituiertem Cycloalkylalkyl oder Cycloalkylalkenyl, wobei die gegebenenfalls substituierten Gruppen wie in Anspruch 1 definiert sind;
R³ ist Alkyl;
jedes R⁴ ist unabhängig Wasserstoff, Alkyl, Halogen oder Halogenalkyl;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl
und Aralkyl; und
R¹⁰ ist Alkyl, Aryl oder Aralkyl.

4. Verwendung der Verbindung nach Anspruch 3, wobei die Verbindung der Formel (I) eine Verbindung ist, worin:
m ist 1;
n ist 1;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist) oder Alkyl;
R³ ist Alkyl;
R⁴ ist Wasserstoff;
R⁵ ist Wasserstoff;
jedes R⁶ ist Wasserstoff;
R⁷ ist eine gerade oder verzweigte Alkylenkette;
R⁸ ist Wasserstoff oder Alkyl; und
R¹⁰ ist Alkyl, Aryl oder Aralkyl.

5. Verwendung der Verbindung nach Anspruch 4, wobei die Verbindung der Formel (I) ausgewählt ist aus der Gruppe bestehend aus den folgenden:
*N*-(3-Ethoxy-propyl)-6-[4-(2-ethylbutyryl)-piperazin-1-yl]-nicotinamid;
*N*-(3-Ethoxy-propyl)-6-(4-pentanoyl-piperazin-1-yl)-nicotinamid;
*N*-(3-Ethoxy-propyl)-6-[4-(3-methyl-pentanoyl)-piperazin-1-yl]-nicotinamid;
*N*-(3-Butoxy-propyl)-6-(4-pentanoyl-piperazin-1-yl)-nicotinamid;
*N*-(3-Methyl-butyl)-6-[4-(3-methyl-pentanoyl)-piperazin-1-yl]-nicotinamid;
6-[4-(3-Methyl-pentanoyl)-piperazin-1-yl]-*N*-pentyl-nicotinamid;
*N*-Butyl-6-[4-(3-methyl-pentanoyl)-piperazin-1-yl]-nicotinamid;
*N*-Butyl-6-[4-(2-ethylbutyryl)-piperazin-1-yl]-nicotinamid;
*N*-(3-Methyl-butyl)-6-(4-pentanoyl-piperazin-1-yl)-nicotinamid;
*N*-(2-Ethylsulfanyl-ethyl)-6-(4-pentanoyl-piperazin-1-yl)-nicotinamid;
*N*-(3-Methoxy-propyl)-6-[4-(3-methyl-pentanoyl)-piperazin-1-yl]-nicotinamid;
6-(4-Pentanoyl-piperazin-1-yl)-*N*-pentyl-nicotinamid;
*N*-Hexyl-6-[4-(3-methyl-pentanoyl)-piperazin-1-yl]-nicotinamid;
*N*-(1,3-Dimethylbutyl)-6-[4-(3-methyl-pentanoyl)-piperazin-1-yl]-nicotinamid;
*N*-(1-Methyl-butyl)-6-(4-pentanoyl-piperazin-1-yl)-nicotinamid;
*N*-(3-Dimethylamino-propyl)-6-[4-(2-ethylbutyryl)-piperazin-1-yl]-nicotinamid;
*N*-(3-Methoxy-propyl)-6-(4-pentanoyl-piperazin-1-yl)-nicotinamid;
*N*-(1,3-Dimethylbutyl)-6-(4-pentanoyl-piperazin-1-yl)-nicotinamid;
*N*-(2-Methyl-butyl)-6-(4-pentanoyl-piperazin-1-yl)-nicotinamid;
*N*-(3-Butoxy-propyl)-6-[4-(2-ethylbutyryl)-piperazin-1-yl]-nicotinamid;
6-[4-(2-Ethylbutyryl)-piperazin-1-yl]-*N*-(3-methylbutyl)-nicotinamid;
*N*-Butyl-6-(4-pentanoyl-piperazin-1-yl)-nicotinamid;
6-[4-(2-Ethylbutyryl)-piperazin-1-yl]-*N*-(2-methylbutyl)-nicotinamid;
*N*-(3-Isopropoxy-propyl)-6-(4-pentanoyl-piperazin-1-yl)-nicotinamid;
*N*-(2-Ethylsulfanyl-ethyl)-6-[4-(3-methyl-pentanoyl)-piperazin-1-yl]-nicotinamid;
6-[4-(2-Ethylbutyryl)-piperazin-1-yl]-*N*-(2-ethylsulfanyl-ethyl)-nicotinamid;
6-[4-(2-Ethylbutyryl)-piperazin-1-yl]-*N*-hexyl-nicotinamid;
6-[4-(2-Ethylbutyryl)-piperazin-1-yl]-*N*-(1-methylbutyl)-nicotinamid;
*N*-(2-Methyl-butyl)-6-[4-(3-methyl-pentanoyl)-piperazin-1-yl]-nicotinamid;
*N*-(1,3-Dimethylbutyl)-6-[4-(2-ethylbutyryl)-piperazin-1-yl]-nicotinamid;
6-[4-(2-Ethylbutyryl)-piperazin-1-yl]-*N*-pentyl-nicotinamid;
*N*-(3-Isopropoxy-propyl)-6-[4-(3-methyl-pentanoyl)-piperazin-1-yl]-nicotinamid;
*N*-(3-Dimethylamino-propyl)-6-[4-(3-methyl-pentanoyl)-piperazin-1-yl]-nicotinamid;
6-[4-(2-Ethylbutyryl)-piperazin-1-yl)-*N*-(3-methoxy-propyl)-nicotinamid;
6-[4-(2-Ethylbutyryl)-piperazin-1-yl]-*N*-(3-isopropoxy-propyl)-nicotinamid;
*N*-(3-Butoxy-propyl)-6-[4-(3-methyl-pentanoyl)-piperazin-1-yl]-nicotinamid;
*N*-(1-Methyl-butyl)-6-[4-(3-methyl-pentanoyl)-piperazin-1-yl]-nicotinamid;
*N*-Hexyl-6-(4-pentanoyl-piperazin-1-yl)-nicotinamid; und
*N*-(3-Dimethylamino-propyl)-6-(4-pentanoyl-piperazin-1-yl)-nicotinamid.

6. Verwendung der Verbindung nach Anspruch 2, wobei die Verbindung der Formel (I) eine Verbindung ist, worin:
m ist 1 oder 2;
n ist 1 oder 2;
p ist 2 oder 3;
V ist -C(O)- oder -S(O)₂-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl, wobei die gegebenenfalls substituierten Gruppen wie in Anspruch 1 definiert sind;
R³ ist Alkyl oder -R⁷-N(R⁸)₂;
jedes R⁴ ist unabhängig Wasserstoff, Alkyl, Halogen oder Halogenalkyl; und
jedes R⁵ und R⁶ ist unabhängig Wasserstoff, Oxo, Alkyl, Alkenyl, Halogen, Halogenalkyl oder Aryl;
oder ein R⁵ und ein R⁶ können zusammen eine geradkettige oder verzweigte Alkylenbrücke bilden;
R⁷ ist eine direkte Bindung; und
jedes R⁸ ist unabhängig Wasserstoff oder Alkyl.

7. Verwendung der Verbindung nach Anspruch 6, wobei die Verbindung der Formel (1) eine Verbindung ist, worin:
m ist 1;
n ist 1;
p ist 2 oder 3;
V ist -C(O)- oder -S(O)₂-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Heterocyclyl, gegebenenfalls substituiertem Heterocyclylalkyl, gegebenenfalls substituiertem Heteroaryl und
gegebenenfalls substituiertem Heteroarylalkyl, wobei die gegebenenfalls substituierten Gruppen wie in Anspruch 1 definiert sind;
R³ ist Alkyl oder -R⁷-N(R⁸)₂;
R⁴ ist Wasserstoff, Alkyl, Halogen oder Halogenalkyl; und
jedes R⁵ und R⁶ ist unabhängig Wasserstoff, Oxo, Alkyl, Halogen oder Halogenalkyl;
oder ein R⁵ und ein R⁶ können zusammen eine Methylenbrücke bilden;
R⁷ ist eine direkte Bindung; und
jedes R⁸ ist unabhängig Wasserstoff oder Alkyl.

8. Verwendung der Verbindung nach Anspruch 7, wobei die Verbindung der Formel (I) ausgewählt ist aus der Gruppe bestehend aus den folgenden:
6-[4-(2-Ethylbutyryl)-piperazin-1-yl]-*N*-(3-phenyl-propyl)-nicotinamid;
6-[4-(4-Methyl-hexanoyl)-piperazin-1-yl]-*N*-(3-phenyl-propyl)-nicotinamid;
*N*-[2-(3-Chlorphenyl)-1-methylethyl]-6-[4-(2-ethylbutyryl)-piperazin-1-yl]-nicotinamid;
*N*-[2-(3-Chlorphenyl)-ethyl]-6-[4-(3-methyl-pentanoyl)-piperazin-1-yl]-nicotinamid;
*N*-[2-(3-Chlorphenyl)-ethyl]-6-[4-(2-ethylbutyryl)-piperazin-1-yl]-nicotinamid;
6-(4-Pentanoyl-piperazin-1-yl)-*N*-(4-phenyl-butyl)-nicotinamid;
*N*-[2-(3-Chlorphenyl)-ethyl]-6-(4-pentanoyl-piperazin-1-yl)-nicotinamid;
6-[4-(2-Ethylbutyryl)-piperazin-1-yl]-*N*-phenethyl-nicotinamid;
6-[4-(3-Methyl-pentanoyl)-piperazin-1-yl]-*N*-phenethyl-nicotinamid;
6-(4-Pentanoyl-piperazin-1-yl)-*N*-(3-phenyl-propyl)nicotinamid;
*N*-[1-Methyl-3-phenyl-propyl)-6-[4-pentanoyl-piperazin-1-yl)-nicotinamid;
6-[4-(3-Methyl-pentanoyl)-piperazin-1-yl]-*N*-(tetrahydro-furan-2-ylmethyl)-nicotinamid;
6-[4-(2-Ethylbutyryl)-piperazin-1-yl]-*N*-(tetrahydro-furan-2-ylmethyl)-nicotinamid;
6-(4-Pentanoyl-piperazin-1-yl)-*N*-(tetrahydro-furan-2-ylmethyl)-nicotinamid;
6-[4-(2-Ethylbutyryl)-piperazin-1-yl]-*N*-(4-phenyl-butyl)-nicotinamid;
*N*-(3-Imidazol-1-yl-propyl)-6-(4-pentanoyl-piperazin-1-yl)-nicotinamid;
6-[4-(2-Ethylbutyryl)-piperazin-1-yl]-*N*-(3-imidazol-1-yl-propyl)-nicotinamid;
*N*-[2-(3H-Imidazol-4-yl)-ethyl)-6-(4-pentanoyl-piperazin-1-yl)-nicotinamid;
*N*-[2-(3H-Imidazol-4-yl)-ethyl]-6-[4-(3-methyl-pentanoyl)-piperazin-1-yl]-nicotinamid;
6-(4-Pentanoyl-piperazin-1-yl)-*N*-phenethyl-nicotinamid;
6-[4-(2-Ethylbutyryl)-piperazin-1-yl]-*N*-[2-(3H-imidazol-4-yl)-ethyl]-nicotinamid;
*N*-(3-Imidazol-1-yl-propyl)-6-[4-(3-methyl-pentanoyl)-piperazin-1-yl]-nicotinamid;
6-[4-(2-Ethylbutyryl)-[1,4]diazepan-1-yl]-*N*-(3-phenyl-propyl)-nicotinamid;
6-[4-(2-Ethylbutyryl)-3-methyl-piperazin-1-yl]-*N*-(3-phenyl-propyl)-nicotinamid;
6-[5-(2-Ethylbutyryl)-2,5-diaza-bicyclo[2.2.1]hept-2-yl]-*N*-(3-phenyl-propyl)-nicotinamid;
6-[4-(Butan-1-sulfonyl)-piperazin-1-yl]-*N*-(3-phenyl-propyl)-nicotinamid;
4-[5-(3-Phenyl-propylcarbamoyl)-pyridin-2-yl]-piperazin-1-carbonsäure-butylamid.

9. Verwendung der Verbindung nach Anspruch 1, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl, wobei die gegebenenfalls substituierten Gruppen wie in Anspruch 1 definiert sind;
R³ ist gegebenenfalls substituiertes Cycloalkyl, gegebenenfalls substituiertes Cycloalkylalkyl oder Cycloalkylalkenyl, wobei die gegebenenfalls substituierten Gruppen wie in Anspruch 1 definiert sind;
jedes R⁴ ist unabhängig Wasserstoff, Alkyl, Alkenyl, Halogen, Halogenalkyl, Aryl oder -R⁹-OR⁸;
jedes R⁵ und R⁶ ist unabhängig Wasserstoff, Oxo, Alkyl, Alkenyl, Halogen, Halogenalkyl oder Aryl;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
R⁹ ist eine direkte Bindung oder eine gerade oder verzweigte Alkylenkette; und
R¹⁰ ist Alkyl, Aryl oder Aralkyl.

10. Verwendung der Verbindung nach Anspruch 9, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Cycloalkyl, gegebenenfalls substituiertem Cycloalkylalkyl oder Cycloalkylalkenyl, wobei die gegebenenfalls substituierten Gruppen wie in Anspruch 1 definiert sind;
R³ ist gegebenenfalls substituiertes Cycloalkyl oder gegebenenfalls substituiertes Cycloalkylalkyl, wobei die gegebenenfalls substituierten Gruppen wie in Anspruch 1 definiert sind;
jedes R⁴ ist unabhängig Wasserstoff, Alkyl, Halogen oder Halogenalkyl;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl
und Aralkyl; und
R¹⁰ ist Alkyl, Aryl oder Aralkyl.

11. Verwendung der Verbindung nach Anspruch 10, wobei die Verbindung der Formel (I) eine Verbindung ist, worin:
m ist 1;
n ist 1;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0 bis 2 ist) oder Alkyl;
R³ ist gegebenenfalls substituiertes Cycloalkyl oder gegebenenfalls substituiertes Cycloalkylalkyl;
R⁴ ist Wasserstoff;
R⁵ ist Wasserstoff;
jedes R⁶ ist Wasserstoff;
R⁷ ist eine gerade oder verzweigte Alkylenkette;
R⁸ ist Wasserstoff oder Alkyl; und
R¹⁰ ist Alkyl, Aryl oder Aralkyl.

12. Verwendung der Verbindung nach Anspruch 11, wobei die Verbindung der Formel (I) ausgewählt ist aus der Gruppe bestehend aus den folgenden:
6-[4-(2-Cyclohexyl-acetyl)-piperazin-1-yl]-*N*-(3-ethoxy-propyl)-nicotinamid;
*N*-Butyl-6-[4-(2-cyclohexyl-acetyl)-piperazin-1-yl]-nicotinamid;
6-[4-(3-Cyclohexyl-propionyl)-piperazin-1-yl]-*N*-(3-methylbutyl)-nicotinamid;
6-[4-(3-Cyclohexyl-propionyl)-piperazin-1-yl]-*N*-(3-methoxy-propyl)-nicotinamid;
*N*-Pentyl-6-[4-(3-trifluormethyl-benzoyl)-piperazin-1-yl]-nicotinamid;
6-[4-(2-Cyclohexyl-acetyl)-piperazin-1-yl]-*N*-(2-methylbutyl)-nicotinamid;
6-[4-(3-Cyclohexyl-propionyl)-piperazin-1-yl]-*N*-(3-isopropoxy-propyl)-nicotinamid;
6-[4-(2-Cyclohexyl-acetyl)-piperazin-1-yl]-*N*-(1,3-dimethylbutyl)-nicotinamid;
6-[4-(2-Cyclohexyl-acetyl)-piperazin-1-yl]-*N*-hexyl-nicotinamid;
6-[4-(2-Cyclohexyl-acetyl)-piperazin-1-yl]-*N*-(1-methylbutyl)-nicotinamid;
6-[4-(2-Cyclohexyl-acetyl)-piperazin-1-yl]-*N*-(3-methylbutyl)-nicotinamid;
6-[4-(2-Cyclohexyl-acetyl)-piperazin-1-yl]-*N*-(2-ethylsulfanyl-ethyl)-nicotinamid;
*N*-(3-Butoxy-propyl)-6-[4-(2-cyclohexyl-acetyl)-piperazin-1-yl]-nicotinamid;
*N*-Butyl-6-[4-(3-Cyclohexyl-propionyl)-piperazin-1-yl]-nicotinamid;
6-[4-(2-Cyclohexyl-acetyl)-piperazin-1-yl]-*N*-pentyl-nicotinamid;
6-[4-(3-Cyclohexyl-propionyl)-piperazin-1-yl]-*N*-(2-ethylsulfanyl-ethyl)-nicotinamid;
6-[4-(2-Cyclohexyl-acetyl)-piperazin-1-yl]-*N*-(3-methoxy-propyl)-nicotinamid;
6-[4-(3-Cyclohexyl-propionyl)-piperazin-1-yl]-*N*-(-methylbutyl)-nicotinamid;
6-[4-(2-Cyclohexyl-acetyl)-piperazin-1-yl]-*N*-(3-isopropoxy-propyl)-nicotinamid;
6-[4-(3-Cyclohexyl-propionyl)-piperazin-1-yl]-*N*-(3-ethoxy-propyl)-nicotinamid;
6-[4-(3-Cyclohexyl-propionyl)-piperazin-1-yl]-*N*-(1,3-dimethylbutyl)-nicotinamid;
6-[4-(3-Cyclohexyl-propionyl)-piperazin-1-yl]-*N*-(2-methylbutyl)-nicotinamid;
6-[4-(3-Cyclohexyl-propionyl)-piperazin-1-yl]-*N*-hexyl-nicotinamid;
6-[4-(3-Cyclohexyl-propionyl)-piperazin-1-yl]-*N*-pentyl-nicotinamid;
6-[4-(3-Cyclohexyl-propionyl)-piperazin-1-yl]-*N*-(3-dimethylamino-propyl)-nicotinamid;
*N*-(3-Ethoxy-propyl)-6-[4-(2-phenyl-cyclopropancarbonyl)-piperazin-1-yl]-nicotinamid;
*N*-(2-Ethylsulfanyl-ethyl)-6-[4-(2-phenyl-cyclopropancarbonyl)-piperazin-1-yl]-nicotinamid;
*N*-(1,3-Dimethylbutyl)-6-[4-(2-phenyl-cyclopropancarbonyl)-piperazin-1-yl]-nicotinamid;
*N*-(3-Methyl-butyl)-6-[4-(2-phenyl-cyclopropancarbonyl)-piperazin-1-yl]-nicotinamid;
*N*-(3-Methoxy-propyl)-6-[4-(2-phenyl-cyclopropancarbonyl)-piperazin-1-yl]-nicotinamid;
*N*-(3-Butoxy-propyl)-6-[4-(2-phenyl-cyclopropancarbonyl)-piperazin-1-yl]-nicotinamid;
*N*-Pentyl-6-[4-(2-phenyl-cyclopropancarbonyl)-piperazin-1-yl]-nicotinamid;
*N*-(3-Dimethylamino-propyl)-6-[4-(2-phenyl-cyclopropancarbonyl)-piperazin-1-yl]-nicotinamid;
*N*-(3-Isopropoxy-propyl)-6-[4-(2-phenyl-cyclopropancarbonyl)-piperazin-1-yl]-nicotinamid;
*N*-(1-Methyl-butyl)-6-[4-(2-phenyl-cyclopropancarbonyl)-piperazin-1-yl]-nicotinamid;
*N*-Butyl-6-[4-(2-phenyl-cyclopropancarbonyl)-piperazin-1-yl]-nicotinamid;
*N*-Hexyl-6-[4-(2-phenyl-cyclopropancarbonyl)-piperazin-1-yl]-nicotinamid;
*N*-(2-Methyl-butyl)-6-[4-(2-phenyl-cyclopropancarbonyl)-piperazin-1-yl]-nicotinamid; und
6-[4-(2-Cyclohexyl-acetyl)-piperazin-1-yl]-*N*-(3-dimethylamino-propyl)-nicotinamid.

13. Verwendung der Verbindung nach Anspruch 9, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl, wobei die gegebenenfalls substituierten Gruppen wie in Anspruch 1 definiert sind;
R³ ist gegebenenfalls substituiertes Cycloalkyl oder gegebenenfalls substituiertes Cycloalkylalkyl, wobei die gegebenenfalls substituierten Gruppen wie in Anspruch 1 definiert sind;
jedes R⁴ ist unabhängig Wasserstoff, Alkyl, Halogen oder Halogenalkyl; und
jedes R⁵ und R⁶ ist unabhängig Wasserstoff, Oxo, Alkyl, Halogen oder Halogenalkyl.

14. Verwendung der Verbindung nach Anspruch 13, wobei die Verbindung der Formel (I) eine Verbindung ist, worin:
m ist 1;
n ist 1;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Heterocyclyl, gegebenenfalls substituiertem Heterocyclylalkyl, gegebenenfalls substituiertem Heteroaryl und gegebenenfalls substituiertem Heteroarylalkyl, wobei die gegebenenfalls substituierten Gruppen wie in Anspruch 1 definiert sind;
R³ ist gegebenenfalls substituiertes Cycloalkyl oder gegebenenfalls substituiertes Cycloalkylalkyl, wobei die gegebenenfalls substituierten Gruppen wie in Anspruch 1 definiert sind;
R⁴ ist Wasserstoff, Alkyl, Halogen oder Halogenalkyl;
R⁵ ist unabhängig Wasserstoff, Oxo, Alkyl, Halogen oder Halogenalkyl; und
jedes R⁶ ist unabhängig Wasserstoff, Oxo, Alkyl, Halogen oder Halogenalkyl.

15. Verwendung der Verbindung nach Anspruch 14, wobei die Verbindung der Formel (I) ausgewählt ist aus der Gruppe bestehend aus den folgenden:
6-[4-(2-Phenyl-cyclopropancarbonyl)-piperazin-1-yl-*N*-(3-phenyl-propyl)-nicotinamid;
*N*-(4-Phenyl-butyl)-6-[4-(2-phenyl-cyclopropancarbonyl)-piperazin-1-yl]-nicotinamid;
6-[4-(2-Cyclohexyl-acetyl)-piperazin-1-yl]-*N*-phenethyl-nicotinamid;
*N*-[2-(3-Chlorphenyl)-ethyl]-6-[4-(2-phenyl-cyclopropancarbonyl)-piperazin-1-yl]-nicotinamid;
*N*-(1-Methyl-3-phenyl-propyl)-6-[4-(2-phenyl-cyclopropancarbonyl)-piperazin-1-yl]-nicotinamid;
*N*-Phenethyl-6-[4-(2-phenyl-cyclopropancarbonyl)-piperazin-1-yl]-nicotinamid;
6-[4-(2-Phenyl-cyclopropancarbonyl)-piperazin-1-yl]-*N*-(tetrahydro-furan-2-ylmethyl)-nicotinamid;
*N*-[2-(3H-Imidazol-4-yl)-ethyl]-6-[4-(2-phenyl-cyclopropancarbonyl)-piperazin-1-yl]-nicotinamid;
*N*-(3-Imidazol-1-yl-propyl)-6-[4-(2-phenyl-cyclopropancarbonyl)-piperazin-1-yl]-nicotinamid;
*N*-[2-(3-Chlorphenyl)-ethyl]-6-[4-(3-cyclohexyl-propionyl)-piperazin-1-yl]-nicotinamid;
6-[4-(2-Cyclohexyl-acetyl)-piperazin-1-yl]-*N*-[2-(3H-imidazol-4-yl)-ethyl]-nicotinamid;
6-[4-(2-Cyclohexyl-acetyl)-piperazin-1-yl]-*N*-(3-phenyl-propyl)-nicotinamid;
*N*-[2-(3-Chlorphenyl)-ethyl]-6-[4-(2-cyclohexyl-acetyl)-piperazin-1-yl]-nicotinamid;
6-[4-(3-Cyclohexyl-propionyl)-piperazin-1-yl]-*N*-phenethyl-nicotinamid;
6-[4-(3-Cyclohexyl-propionyl)-piperazin-1-yl]-*N*-(3-phenyl-propyl)-nicotinamid;
6-[4-(3-Methyl-pentanoyl)-piperazin-1-yl]-*N*-(4-phenyl-butyl)-nicotinamid;
6-[4-(2-Cyclohexyl-acetyl)-piperazin-1-yl]-*N*-(4-phenyl-butyl)-nicotinamid;
6-[4-(2-Cyclohexyl-acetyl)-piperazin-1-yl]-*N*-(1-methyl-3-phenyl-propyl)-nicotinamid;
6-[4-(3-Cyclohexyl-propionyl)-piperazin-1-yl]-*N*-[2-(3H-imidazol-4-yl)-ethyl]-nicotinamid;
6-[4-(3-Cyclohexyl-propionyl)-piperazin-1-yl]-*N*-(tetrahydro-furan-2-ylmethyl)-nicotinamid;
6-[4-(2-Cyclohexyl-acetyl)-piperazin-1-yl]-*N*-(3-imidazol-1-yl-propyl)-nicotinamid;
6-[4-(3-Cyclohexyl-propionyl)-piperazin-1-yl]-*N*-(3-imidazol-1-yl-propyl)-nicotinamid; und
6-[4-(2-Cyclohexyl-acetyl)-piperazin-1-yl]-*N*-(tetrahydro-furan-2-ylmethyl)-nicotinamid.

16. Verwendung der Verbindung nach Anspruch 1, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl, wobei die gegebenenfalls substituierten Gruppen wie in Anspruch 1 definiert sind;
R³ ist gegebenenfalls substituiertes Aryl, wobei die gegebenenfalls substituierte Gruppe wie in Anspruch 1 definiert ist;
jedes R⁴ ist unabhängig Wasserstoff, Alkyl, Alkenyl, Halogen, Halogenalkyl, Aryl oder -R⁹-OR⁸;
jedes R⁵ und R⁶ ist unabhängig Wasserstoff, Oxo, Alkyl, Alkenyl, Halogen, Halogenalkyl oder Aryl;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
R⁹ ist eine direkte Bindung oder eine gerade oder verzweigte Alkylenkette; und
R¹⁰ ist Alkyl, Aryl oder Aralkyl.

17. Verwendung der Verbindung nach Anspruch 16, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0 bis 2 ist), Alkyl, Alkenyl, gegebenenfalls substituiertem Cycloalkyl, gegebenenfalls substituiertem Cycloalkylalkyl oder Cycloalkylalkenyl, wobei die gegebenenfalls substituierten Gruppen wie in Anspruch 1 definiert sind;
R³ ist gegebenenfalls substituiertes Aryl, wobei die gegebenenfalls substituierte Gruppe wie in Anspruch 1 definiert ist;
jedes R⁴ ist unabhängig Wasserstoff, Alkyl, Halogen, Halogenalkyl oder -R⁹-OR⁸;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl und Aralkyl;
R⁹ ist eine direkte Bindung oder eine gerade oder verzweigte Alkylenkette; und
R¹⁰ ist Alkyl, Aryl oder Aralkyl.

18. Verwendung der Verbindung nach Anspruch 17, wobei die Verbindung der Formel (I) eine Verbindung ist, worin:
m ist 1 oder 2;
n ist 1;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, gegebenenfalls substituiertem Cycloalkyl oder gegebenenfalls substituiertem Cycloalkylalkyl, wobei die gegebenenfalls substituierten Gruppen wie in Anspruch 1 definiert sind;
R³ ist gegebenenfalls substituiertes Aryl, wobei die gegebenenfalls substituierte Gruppe wie in Anspruch 1 definiert ist;
jedes R⁴ ist unabhängig Wasserstoff, Halogen, Halogenalkyl oder -R⁹-OR⁸;
R⁵ ist Wasserstoff;
jedes R⁶ ist Wasserstoff;
R⁷ ist eine gerade oder verzweigte Alkylenkette;
R⁸ ist Wasserstoff oder Alkyl;
R⁹ ist eine direkte Bindung oder eine gerade oder verzweigte Alkylenkette; und
R¹⁰ ist Alkyl, Aryl oder Aralkyl.

19. Verwendung der Verbindung nach Anspruch 18, wobei die Verbindung der Formel (I) ausgewählt ist aus der Gruppe bestehend aus den folgenden:
*N*-Butyl-6-[4-(2-mercapto-benzoyl)-piperazin-1-yl]-nicotinamid;
*N*-(3-Methyl-butyl)-6-[4-(2-o-tolyl-acetyl)-piperazin-1-yl]-nicotinamid;
6-{4-[2-(2,4-Dimethyl-phenyl)-acetyl]-piperazin-1-yl}-*N*-(3-methylbutyl)-nicotinamid;
6-[4-(2-Brom-benzoyl)-piperazin-1-yl]-*N*-(3-ethoxypropyl)nicotinamid;
6-{4-[2-(2-Chlor-6-fluor-phenyl)-acetyl]-piperazin-1-yl}-*N*-(3-methylbutyl)-nicotinamid;
6-[4-(3,5-Dichlor-benzoyl)-piperazin-1-yl]-*N*-(3-methoxy-propyl)-nicotinamid;
6-[4-(2-Brom-5-methoxy-benzoyl)-piperazin-1-yl]-*N*-(3-dimethylamino-propyl)-nicotinamid;
6-[4-(2,5-Dichlor-benzoyl)-piperazin-1-yl]-*N*-(3-methylbutyl)-nicotinamid;
6-[4-(2, 5-Dichlor-benzoyl)-piperazin-1-yl]-*N*-(3-ethoxy-propyl)-nicotinamid;
*N*-Butyl-6-[4-(3,5-dichlor-benzoyl)-piperazin-1-yl]-nicotinamid;
6-[4-(2,4-Dichlor-benzoyl)-piperazin-1-yl]-*N*-(3-ethoxy-propyl)-nicotinamid;
6-[4-(2,4-Dichlor-benzoyl)-piperazin-1-yl]-*N*-(2-ethylsulfanyl-ethyl)-nicotinamid;
*N*-(3-Ethoxy-propyl)-6-[4-(4-trifluormethyl-benzoyl)-piperazin-1-yl]-nicotinamid;
*N*-(3-Dimethylamino-propyl)-6-[4-(2,3,4,5-tetrafluor-benzoyl)-piperazin-1-yl]-nicotinamid;
6-[4-(2-Brom-5-methoxy-benzoyl)-piperazin-1-yl]-*N*-(2-ethylsulfanyl-ethyl)-nicotinamid;
*N*-(2-Ethylsulfanyl-ethyl)-6-[4-(3-trifluormethyl-benzoyl)-piperazin-1-yl]-nicotinamid;
6-[4-(2-Brom-benzoyl)-piperazin-1-yl]-*N*-(3-methylbutyl)-nicotinamid;
*N*-Butyl-6-[4-(2,4-dimethyl-benzoyl)-piperazin-1-yl]-nicotinamid;
6-[4-(3,5-Dichlor-benzoyl)-piperazin-1-yl]-*N*-(3-isopropoxy-propyl)-nicotinamid;
6-[4-(2-Brom-5-methoxy-benzoyl)-piperazin-1-yl]-*N*-(3-methylbutyl)-nicotinamid;
*N*-(3-Butoxy-propyl)-6-[4-(2,5-dichlor-benzoyl)-piperazin-1 -yl]-nicotinamid;
*N*-(3-Methyl-butyl)-6-[4-(3-trifluormethyl-benzoyl)-piperazin-1-yl]-nicotinamid;
*N*-(3-Methyl-butyl)-6-[4-(2-trifluormethyl-benzoyl)-piperazin-1-yl]-nicotinamid;
6-[4-(2-Brom-benzoyl)-piperazin-1-yl]-*N*-(3-butoxy-propyl)-nicotinamid;
6-[4-(2,5-Dichlor-benzoyl)-piperazin-1-yl]-*N*-pentyl-nicotinamid;
*N*-(3-Butoxy-propyl)-6-[4-(2,4-dimethyl-benzoyl)-piperazin-1-yl]-nicotinamid;
6-[4-(2-Brom-5-methoxy-benzoyl)-piperazin-1-yl]-*N*-(3-ethoxy-propyl)-nicotinamid;
6-[4-(2,4-Dichlor-benzoyl)-piperazin-1-yl]-*N*-(3-isopropoxy-propyl)-nicotinamid;
*N*-(3-Ethoxy-propyl)-6-[4-(2-trifluormethyl-benzoyl)-piperazin-1-yl]-nicotinamid;
*N*-Pentyl-6-[4-(3-trifluormethyl-benzoyl)-piperazin-1-yl]-nicotinamid;
*N*-Butyl-6-[4-(2-trifluormethyl-benzoyl)-piperazin-1-yl]-nicotinamid;
*N*-Butyl-6-[4-(2,5-dichlor-benzoyl)-piperazin-1-yl]-nicotinamid;
6-[4-(2-Brom-benzoyl)-piperazin-1-yl]-*N*-(3-isopropoxy-propyl)-nicotinamid;
6-[4-(2,5-Dichlor-benzoyl)-piperazin-1-yl]-*N*-(3-isopropoxy-propyl)-nicotinamid;
*N*-(3-Butoxy-propyl)-6-[4-(4-trifluormethyl-benzoyl)-piperazin-1-yl]-nicotinamid;
6-[4-(2,5-Dichlor-benzoyl)-piperazin-1-yl]-*N*-(2-ethylsulfanyl-ethyl)-nicotinamid;
*N*-(3-Butoxy-propyl)-6-[4-(2-trifluormethyl-benzoyl)-piperazin-1-yl]-nicotinamid;
6-[4-(2,5-Dichlor-benzoyl)-piperazin-1-yl]-*N*-(3-methoxy-propyl)-nicotinamid;
6-[4-(2,4-Dimethyl-benzoyl)-piperazin-1-yl]-*N*-(1,3-dimethylbutyl)-nicotinamid;
6-[4-(2,4-Dichlor-benzoyl)-piperazin-1-yl]-*N*-(2-methylbutyl)-nicotinamid;
6-[4-(3,5-Dichlor-benzoyl)-piperazin-1-yl]-*N*-(2-methylbutyl)-nicotinamid;
6-[4-(2,4-Dimethyl-benzoyl)-piperazin-1-yl]-*N*-(2-ethylsulfanyl-ethyl)-nicotinamid;
*N*-(1,3-Dimethylbutyl)-6-[4-(3-trifluormethyl-benzoyl)-piperazin-1-yl]-nicotinamid;
6-[4-(2-Brom-5-methoxy-benzoyl)-piperazin-1-yl]-*N*-(2-methylbutyl)-nicotinamid;
*N*-(3-Dimethylamino-propyl)-6-[4-(2-trifluormethyl-benzoyl)-piperazin-1-yl]-nicotinamid;
*N*-Hexyl-6-[4-(3-trifluormethyl-benzoyl)-piperazin-1-yl]-nicotinamid;
6-[4-(2,4-Dichlor-benzoyl)-piperazin-1-yl]-*N*-(1,3-dimethylbutyl)-nicotinamid;
*N*-(3-Isopropoxy-propyl)-6-[4-(3-trifluormethyl-benzoyl)-piperazin-1-yl]-nicotinamid;
6-[4-(2-Brom-5-methoxy-benzoyl)-piperazin-1-yl]-*N*-hexyl-nicotinamid;
6-[4-(2-Brom-benzoyl)-piperazin-1-yl]-*N*-hexyl-nicotinamid;
*N*-(3-Isopropoxy-propyl)-6-[4-(2-trifluormethyl-benzoyl)-piperazin-1-yl]-nicotinamid;
6-[4-(2-Brom-5-methoxy-benzoyl)-piperazin-1-yl]-*N*-(3-methoxy-propyl)-nicotinamid;
6-[4-(2-Brom-benzoyl)-piperazin-1-yl]-*N*-(1-methylbutyl)-nicotinamid;
6-[4-(3,5-Dichlor-benzoyl)-piperazin-1-yl]-*N*-hexyl-nicotinamid;
*N*-(3-Methoxy-propyl)-6-[4-(2-trifluormethyl-benzoyl)-piperazin-1-yl]-nicotinamid;
6-[4-(2,4-Dichlor-benzoyl)-piperazin-1-yl]-*N*-hexyl-nicotinamid;
*N*-(2-Ethylsulfanyl-ethyl)-6-[4-(2-trifluormethyl-benzoyl)-piperazin-1-yl]-nicotinamid;
*N*-Pentyl-6-[4-(2-trifluormethyl-benzoyl)-piperazin-1-yl]-nicotinamid;
*N*-(2-Methyl-butyl)-6-[4-(3-trifluormethyl-benzoyl)-piperazin-1-yl]-nicotinamid;
*N*-(2-Ethylsulfanyl-ethyl)-6-[4-(4-trifluormethyl-benzoyl)-piperazin-1-yl]-nicotinamid;
6-[4-(2,4-Dichlor-benzoyl)-piperazin-1-yl]-*N*-pentyl-nicotinamid;
6-[4-(2-Brom-benzoyl)-piperazin-1-yl]-*N*-(2-methylbutyl)-nicotinamid;
6-[4-(2-Brom-5-methoxy-benzoyl)-piperazin-1-yl]-*N*-(3-butoxy-propyl)-nicotinamid;
*N*-(1,3-Dimethylbutyl)-6-[4-(4-trifluormethyl-benzoyl)-piperazin-1-yl]-nicotinamid;
6-[4-(3,5-Dichlor-benzoyl)-piperazin-1-yl]-*N*-pentyl-nicotinamid;
*N*-(3-Butoxy-propyl)-6-[4-(3,5-dichlor-benzoyl)-piperazin-1-yl]-nicotinamid;
6-[4-(2-Brom-5-methoxy-benzoyl)-piperazin-1-yl]-*N*-butyl-nicotinamid;
6-[4-(2-Brom-benzoyl)-piperazin-1-yl]-*N*-pentyl-nicotinamid;
*N*-(1,3-Dimethylbutyl)-6-[4-(2-trifluormethyl-benzoyl)-piperazin-1-yl]-nicotinamid;
*N*-(3-Methoxy-propyl)-6-[4-(4-trifluormethyl-benzoyl)-piperazin-1-yl]-nicotinamid;
6-[4-(2,4-Dimethyl-benzoyl)-piperazin-1-yl]-*N*-(1-methylbutyl)-nicotinamid;
6-[4-(2,4-Dichlor-benzoyl)-piperazin-1-yl]-*N*-(3-methoxy-propyl)-nicotinamid;
*N*-(1-Methyl-butyl)-6-[4-(2-trifluormethyl-benzoyl)-piperazin-1-yl]-nicotinamid;
*N*-(3-Dimethylamino-propyl)-6-[4-(3-trifluormethyl-benzoyl)-piperazin-1-yl]-nicotinamid;
6-[4-(2,4-Dichlor-benzoyl)-piperazin-1-yl]-*N*-(1-methylbutyl)-nicotinamid;
6-[4-(2,5-Dichlor-benzoyl)-piperazin-1-yl]-*N*-(1-methylbutyl)-nicotinamid;
*N*-(1-Methyl-butyl)-6-[4-(3-trifluormethyl-benzoyl)-piperazin-1-yl]-nicotinamid;
6-[4-(2,5-Dichlor-benzoyl)-piperazin-1-yl]-*N*-hexyl-nicotinamid;
6-[4-(2,4-Dimethyl-benzoyl)-piperazin-1-yl]-*N*-(3-methoxy-propyl)-nicotinamid;
6-[4-(3,5-Dichlor-benzoyl)-piperazin-1-yl]-*N*-(1-methylbutyl)-nicotinamid;
6-[4-(2,4-Dichlor-benzoyl)-piperazin-1-yl]-*N*-(3-methylbutyl)-nicotinamid;
*N*-(3-Butoxy-propyl)-6-[4-(3-trifluormethyl-benzoyl)-piperazin-1-yl]-nicotinamid;
6-[4-(2,4-Dimethyl-benzoyl)-piperazin-1-yl]-*N*-(3-isopropoxy-propyl)-nicotinamid;
*N-*(1-Methyl-butyl)-6-[4-(4-trifluormethyl-benzoyl)-piperazin-1-yl]-nicotinamid;
*N*-Butyl-6-[4-(2,4-dichlor-benzoyl)-piperazin-1-yl]-nicotinamid;
6-[4-(3,5-Dichlor-benzoyl)-piperazin-1-yl]-*N*-(1,3-dimethylbutyl)-nicotinamid;
*N*-Butyl-6-[4-(3-trifluormethyl-benzoyl)-piperazin-1-yl]-nicotinamid;
6-[4-(2-Brom-5-methoxy-benzoyl)-piperazin-1-yl]-*N*-(1,3-dimethylbutyl)-nicotinamid;
6-[4-(2,4-Dimethyl-benzoyl)-piperazin-1-yl]-*N*-hexyl-nicotinamid;
6-[4-(2,5-Dichlor-benzoyl)-piperazin-1-yl]-*N*-(1,3-dimethylbutyl)-nicotinamid;
*N*-(3-Butoxy-propyl)-6-[4-(2,4-dichlor-benzoyl)-piperazin-1-yl]-nicotinamid;
*N*-(3-Methoxy-propyl)-6-[4-(3-trifluormethyl-benzoyl)-piperazin-1-yl]-nicotinamid;
6-[4-(2,4-Dimethyl-benzoyl)-piperazin-1-yl]-*N*-(3-ethoxy-propyl)-nicotinamid;
6-[4-(2,5-Dichlor-benzoyl)-piperazin-1-yl]-*N*-(2-methylbutyl)-nicotinamid;
*N*-Hexyl-6-[4-(4-trifluormethyl-benzoyl)-piperazin-1-yl]-nicotinamid;
6-[4-(2,4-Dimethyl-benzoyl)-piperazin-1-yl]-*N*-pentyl-nicotinamid;
6-[4-(2-Brom-benzoyl)-piperazin-1-yl]-*N*-(1,3-dimethylbutyl)-nicotinamid;
*N*-Hexyl-6-[4-(2-trifluormethyl-benzoyl)-piperazin-1-yl]-nicotinamid;
6-[4-(2-Brom-benzoyl)-piperazin-1-yl]-*N*-(3-methoxy-propyl)-nicotinamid;
*N*-(2-Methyl-butyl)-6-[4-(4-trifluormethyl-benzoyl)-piperazin-1-yl]-nicotinamid;
6-[4-(2-Brom-5-methoxy-benzoyl)-piperazin-1-yl]-*N*-(1-methylbutyl)-nicotinamid;
6-[4-(2,4-Dimethyl-benzoyl)-piperazin-1-yl]-*N*-(2-methylbutyl)-nicotinamid;
6-[4-(2-Brom-5-methoxy-benzoyl)-piperazin-1-yl]-*N*-pentyl-nicotinamid;
*N*-(2-Methyl-butyl)-6-[4-(2-trifluormethyl-benzoyl)-piperazin-1-yl]-nicotinamid;
6-[4-(3,5-Dichlor-benzoyl)-piperazin-1-yl]-*N*-(3-methylbutyl)-nicotinamid;
6-[4-(2-Brom-benzoyl)-piperazin-1-yl]-*N*-(2-ethylsulfanyl-ethyl)-nicotinamid;
6-[4-(3,5-Dichlor-benzoyl)-piperazin-1-yl]-*N*-(3-methoxy-propyl)-nicotinamid;
6-[4-(2-Brom-5-methoxy-benzoyl)-piperazin-1-yl]-*N*-(3-isopropoxy-propyl)-nicotinamid;
*N*-(3-Butoxy-propyl)-6-[4-(2,3,4,5-tetrafluor-benzoyl)-piperazin-1-yl]-nicotinamid;
*N*-(1, 3-Dimethylbutyl)-6-[4-(2, 3,4, 5-tetrafluor-benzoyl)-piperazin-1-yl]-nicotinamid;
*N*-Butyl-6-[4-(2,3,4,5-tetrafluor-benzoyl)-piperazin-1-yl]-nicotinamid;
*N*-(1-Methyl-butyl)-6-[4-(2,3,4,5-tetrafluor-benzoyl)-piperazin-1-yl]-nicotinamid;
*N*-(3-Methoxy-propyl)-6-[4-(2,3,4,5-tetrafluor-benzoyl)-piperazin-1-yl]-nicotinamid;
*N*-(3-Isopropoxy-propyl)-6-[4-(2,3,4,5-tetrafluor-benzoyl)-piperazin-1-yl]-nicotinamid;
*N*-Pentyl-6-[4-(2,3,4,5-tetrafluor-benzoyl)-piperazin-1-yl]-nicotinamid;
*N*-(2-Methyl-butyl)-6-[4-(2, 3,4, 5-tetrafluor-benzoyl)-piperazin-1-yl]-nicotinamid;
*N*-Hexyl-6-[4-(2,3,4,5-tetrafluor-benzoyl)-piperazin-1-yl]-nicotinamid;
*N*-(3-Methyl-butyl)-6-[4-(2,3,4,5-tetrafluor-benzoyl)-piperazin-1-yl]-nicotinamid;
*N*-(2-Ethylsulfanyl-ethyl)-6-[4-(2,3,4,5-tetrafluor-benzoyl)-piperazin-1-yl]-nicotinamid;
*N*-(3-Ethoxy-propyl)-6-[4-(2,3,4,5-tetrafluor-benzoyl)-piperazin-1-yl]-nicotinamid;
*N*-(3-Dimethylamino-propyl)-6-[4-(4-trifluormethyl-benzoyl)-piperazin-1-yl]-nicotinamid;
*N*-(3-Ethoxy-propyl)-6-[4-(3-trifluormethyl-benzoyl)-piperazin-1-yl]-nicotinamid;
6-[4-(3,5-Dichlor-benzoyl)-piperazin-1-yl]-*N*-(2-ethylsulfanyl-ethyl)-nicotinamid;
*N*-(3-Methyl-butyl)-6-[4-(4-trifluormethyl-benzoyl)-piperazin-1-yl]-nicotinamid;
*N*-Butyl-6-[4-(4-trifluormethyl-benzoyl)-piperazin-1-yl]-nicotinamid;
*N*-(3-Isopropoxy-propyl)-6-[4-(4-trifluormethyl-benzoyl)-piperazin-1-yl]-nicotinamid;
*N*-Pentyl-6-[4-(4-trifluormethyl-benzoyl)-piperazin-1-yl]-nicotinamid;
6-[4-(3,5-Dichlor-benzoyl)-piperazin-1-yl]-*N*-(3-dimethylamino-propyl)-nicotinamid;
6-[4-(2-Brom-benzoyl)-piperazin-1-yl]-*N*-(3-dimethylamino-propyl)-nicotinamid;
6-[4-(2,4-Dichlor-benzoyl)-piperazin-1-yl]-*N*-(3-dimethylamino-propyl)-nicotinamid;
6-[4-(2,5-Dichlor-benzoyl)-piperazin-1-yl]-*N*-(3-dimethylamino-propyl)-nicotinamid;
*N*-(3-Dimethylamino-propyl)-6-[4-(2,4-dimethyl-benzoyl)-piperazin-1-yl]₋nicotinamid;
*N*-(3-Methyl-butyl)-6-[4-(2-trifluormethyl-benzoyl)-piperazin-1-yl]-nicotinamid;
*N*-(3-Methyl-butyl)-4-trifluormethyl-6-[4-(2-trifluormethyl-benzoyl)-piperazin-1-yl]-nicotinamid;
2-Chlor-5-fluor-*N*-(3-methylbutyl)-6-[4-(2-trifluormethyl-benzoyl)-piperazin-1-yl]-nicotinamid;
*N*-(3-Ethoxy-propyl)-6-[4-(2-naphthalin-2-yl-acetyl)-piperazin-1-yl]-nicotinamid;
6-[4-(2-Naphthalin-2-yl-acetyl)-piperazin-1-yl]-*N*-pentyl-nicotinamid;
*N*-Butyl-6-[4-(naphthalin-1-carbonyl)-piperazin-1-yl]-nicotinamid;
*N*-(3-Butoxy-propyl)-6-[4-(2-naphthalin-2-yl-acetyl)-piperazin-1-yl]-nicotinamid;
*N*-Butyl-6-[4-(2-naphthalin-2-yl-acetyl)-piperazin-1-yl]-nicotinamid;
*N*-(3-Butoxy-propyl)-6-[4-(naphthalin-1-carbonyl)-piperazin-1-yl]-nicotinamid;
*N*-(3-Methoxy-propyl)-6-[4-(naphthalin-1-carbonyl)-piperazin-1-yl]-nicotinamid;
*N*-(1-Methyl-butyl)-6-[4-(naphthalin-1-carbonyl)-piperazin-1-yl]-nicotinamid;
6-[4-(Naphthalin-1-carbonyl)-piperazin-1-yl]-*N*-pentyl-nicotinamid;
*N*-(3-Isopropoxy-propyl)-6-[4-(2-naphthalin-2-yl-acetyl)-piperazin-1-yl]-nicotinamid;
*N*-(1-Methyl-butyl)-6-[4-(2-naphthalin-2-yl-acetyl)-piperazin-1-yl]-nicotinamid;
*N*-(3-Methyl-butyl)-6-[4-(2-naphthalin-2-yl-acetyl)-piperazin-1-yl]-nicotinamid;
*N*-(3-Dimethylamino-propyl)-6-[4-(2-naphthalin-2-yl-acetyl)-piperazin-1-yl]-nicotinamid;
*N*-(3-Ethoxy-propyl)-6-[4-(naphthalin-1-carbonyl)-piperazin-1-yl]-nicotinamid;
*N*-Hexyl-6-[4-(naphthalin-1-carbonyl)-piperazin-1-yl]-nicotinamid;
*N*-(2-Ethylsulfanyl-ethyl)-6-[4-(2-naphthalin-2-yl-acetyl)-piperazin-1-yl]-nicotinamid;
*N*-Hexyl-6-[4-(2-naphthalin-2-yl-acetyl)-piperazin-1-yl]-nicotinamid;
*N*-(1,3-Dimethylbutyl)-6-[4-(naphthalin-1-carbonyl)-piperazin-1-yl]-nicotinamid;
*N*-(3-Isopropoxy-propyl)-6-[4-(naphthalin-1-carbonyl)-piperazin-1-yl]-nicotinamid;
*N*-(1,3-Dimethylbutyl)-6-[4-(naphthalin-2-carbonyl)-piperazin-1-yl]-nicotinamid;
*N*-(1,3-Dimethylbutyl)-6-[4-(2-naphthalin-2-yl-acetyl)-piperazin-1-yl]-nicotinamid;
*N*-(2-Methyl-butyl)-6-[4-(naphthalin-1-carbonyl)-piperazin-1-yl]-nicotinamid;
*N*-(2-Ethylsulfanyl-ethyl)-6-[4-(naphthalin-1-carbonyl)-piperazin-1-yl]-nicotinamid;
*N*-(2-Methyl-butyl)-6-[4-(2-naphthalin-2-yl-acetyl)-piperazin-1-yl]-nicotinamid;
*N*-(3-Methyl-butyl)-6-[4-(naphthalin-1-carbonyl)-piperazin-1-yl]-nicotinamid;
*N*-(3-Ethoxy-propyl)-6-[4-(naphthalin-2-carbonyl)-piperazin-1-yl]-nicotinamid;
*N*-(2-Methyl-butyl)-6-[4-(naphthalin-2-carbonyl)-piperazin-1-yl]-nicotinamid;
*N*-(3-Methyl-butyl)-6-[4-(naphthalin-2-carbonyl)-piperazin-1-yl]-nicotinamid;
*N*-(3-Methoxy-propyl)-6-[4-(naphthalin-2-carbonyl)-piperazin-1-yl]-nicotinamid;
*N*-Butyl-6-[4-(naphthalin-2-carbonyl)-piperazin-1-yl]-nicotinamid;
*N*-(3-Butoxy-propyl)-6-[4-(naphthalin-2-carbonyl)-piperazin-1-yl]-nicotinamid;
*N*-(1-Methyl-butyl)-6-[4-(naphthalin-2-carbonyl)-piperazin-1-yl]-nicotinamid;
*N*-(3-Isopropoxy-propyl)-6-[4-(naphthalin-2-carbonyl)-piperazin-1-yl]-nicotinamid;
*N*-Hexyl-6-[4-(naphthalin-2-carbonyl)-piperazin-1-yl]-nicotinamid;
*N*-(3-Dimethylamino-propyl)-6-[4-(naphthalin-1-carbonyl)-piperazin-1-yl]-nicotinamid;
*N*-(2-Ethylsulfanyl-ethyl)-6-[4-(naphthalin-2-carbonyl)-piperazin-1-yl]-nicotinamid;
*N*-(3-Dimethylamino-propyl)-6-[4-(naphthalin-2-carbonyl)-piperazin-1-yl]-nicotinamid;
*N*-(3-Methyl-butyl)-6-[4-(naphthalin-1-carbonyl)-piperazin-1-yl]-nicotinamid;
*N*-(3-Methoxy-propyl)-6-[4-(2-naphthalin-2-yl-acetyl)-piperazin-1-yl]-nicotinamid;
2-Chlor-*N*-(3-methylbutyl)-6-[4-(2-trifluormethyl-benzoyl)-piperazin-1-yl]-nicotinamid;
*N*-(2-Cyclopropyl-ethyl)-6-[4-(3-trifluormethyl-benzoyl)-piperazin-1-yl]-nicotinamid;
*N*-(2-Cyclopropyl-ethyl)-2-methoxy-6-[4-(2-trifluormethyl-benzoyl)-piperazin-1-yl]-nicotinamid;
*N*-(2-Cyclopropyl-ethyl)-6-[4-(5-fluor-2-trifluormethyl-benzoyl)-piperazin-1-yl]-nicotinamid;
2-Oxo-6-[4-(2-trifluormethyl-benzoyl)-piperazin-1-yl]-1,2-dihydro-pyridin-3-carbonsäure-(2-cyclopropylethyl)amid;
*N*-(2-Cyclopropyl-ethyl)-2-hydroxy-6-[4-(2-trifluormethyl-benzoyl)-piperazin-1-yl]-nicotinamid;
*N*-(2-Cyclobutyl-ethyl)-6-[4-(5-fluor-2-trifluormethyl-benzoyl)-piperazin-1-yl]-nicotinamid;
*N*-(3-Cyclopropyl-propyl)-6-[4-(5-fluor-2-trifluormethyt-benzoyl)-piperazin-1-yl]-nicotinamid;
6-[4-(5-Fluor-2-trifluormethyl-benzoyl)-piperäzin-1-yl]-*N*-(4-methyl-pentyl)-nicotinamid; und
*N*-(3,3-Dimethylbutyl)-6-[4-(5-fluor-2-trifluormethyl-benzoyl)-piperazin-1-yl]-nicotinamid.

20. Verwendung der Verbindung nach Anspruch 16, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl, wobei die gegebenenfalls substituierten Gruppen wie in Anspruch 1 definiert sind;
R³ ist gegebenenfalls substituiertes Aryl, wobei die gegebenenfalls substituierte Gruppe wie in Anspruch 1 definiert ist;
jedes R⁴ ist unabhängig Wasserstoff, Alkyl, Halogen oder Halogenalkyl; und
jedes R⁵ und R⁶ ist unabhängig Wasserstoff, Oxo, Alkyl, Halogen oder Halogenalkyl.

21. Verwendung der Verbindung nach Anspruch 20, wobei die Verbindung der Formel (I) eine Verbindung ist, worin:
m ist 1 oder 2;
n ist 1;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Heterocyclyl, gegebenenfalls substituiertem Heterocyclylalkyl, gegebenenfalls substituiertem Heteroaryl und
gegebenenfalls substituiertem Heteroarylalkyl, wobei die gegebenenfalls substituierten Gruppen wie in Anspruch 1 definiert sind;
R³ ist gegebenenfalls substituiertes Aryl, wobei die gegebenenfalls substituierte Gruppe wie in Anspruch 1 definiert ist;
jedes R⁴ ist unabhängig Wasserstoff, Alkyl, Halogen oder Halogenalkyl;
R⁵ ist Wasserstoff, Oxo, Alkyl, Halogen oder Halogenalkyl; und
jedes R⁶ ist unabhängig Wasserstoff, Alkyl, Halogen oder Halogenalkyl.

22. Verwendung der Verbindung nach Anspruch 21, wobei die Verbindung der Formel (I) ausgewählt ist aus der Gruppe bestehend aus den folgenden:
6-[4-(2,5-Dichlor-benzoyl)-piperazin-1-yl]-N-(3-imidazol-1-yl-propyl)-nicotinamid;
6-[4-(2,4-Dichlor-benzoyl)-piperazin-1-yl]-*N*-(3-imidazol-1-yl-propyl)-nicotinamid;
6-[4-(2-Brom-5-methoxy-benzoyl)-piperazin-1-yl]-*N*-(3-phenyl-propyl)-nicotinamid;
6-[4-(2-Brom-benzoyl)-piperazin-1-yl]-*N*-[2-(3H-imidazol-4-yl)-ethyl]-nicotinamid;
*N*-(1-Methyl-2-phenyl-ethyl)-6-[4-(2-trifluormethyl-benzoyl)-piperazin-1-yl]-nicotinamid;
*N*-(1-Methyl-2-phenyl-ethyl)-6-[4-(4-trifluormethyl-benzoyl)-piperazin-1-yl]-nicotinamid;
6-[4-(Naphthalin-1-carbonyl)-piperazin-1-yl]-*N*-(4-phenyl-butyl)-nicotinamid;
*N*-[2-(3-Chlorphenyl)-ethyl]-6-[4-(2-naphthalin-2-yl-acetyl)-piperazin-1-yl]-nicotinamid;
6-[4-(Naphthalin-2-carbonyl)-piperazin-1-yl]-*N*-(3-phenyl-propyl)-nicotinamid;
*N*-(3-Imidazol-1-yl-propyl)-6-[4-(2-naphthalin-2-yl-acetyl)-piperazin-1-yl]-nicotinamid;
6-[4-(Naphthalin-1-carbonyl)-piperazin-1-yl]-*N*-(3-phenyl-propyl)-nicotinamid;
*N*-(1-Methyl-3-phenyl-propyl)-6-[4-(2-naphthalin-2-yl-acetyl)-piperazin-1-yl]-nicotinamid;
6-[4-(2-Naphthalin-2-yl-acetyl)-piperazin-1-yl]-*N*-phenethyl-nicotinamid;
*N*-[2-(3-Chlorphenyl)-ethyl]-6-[4-(naphthalin-1-carbonyl)-piperazin-1-yl]-nicotinamid;
*N*-(1-Methyl-3-phenyl-propyl)-6-[4-(naphthalin-1-carbonyl)-piperazin-1-yl]-nicotinamid;
6-[4-(Naphthalin-1-carbonyl)-piperazin-1-yl]-*N*-phenethyl-nicotinamid;
*N*-[2-(3H-Imidazol-4-yl)-ethyl]-6-[4-(2-naphthalin-2-yl-acetyl)-piperazin-1-yl]-nicotinamid;
6-[4-(2-Naphthalin-2-yl-acetyl)-piperazin-1-yl]-*N*-(4-phenyl-butyl)-nicotinamid;
6-[4-(Naphthalin-2-carbonyl)-piperazin-1-yl]-*N*-(3-phenyl-propyl)-nicotinamid;
*N*-(1-Methyl-3-phenyl-propyl)-6-[4-(naphthalin-2-carbonyl)-piperazin-1-yl]-nicotinamid;
*N*-[2-(3H-Imidazol-4-yl)-ethyl]-6-[4-(naphthalin-1-carbonyl)-piperazin-1-yl]-nicotinamid;
6-[4-(Naphthalin-2-carbonyl)-piperazin-1-yl]-*N*-(tetrahydro-furan-2-ylmethyl)-nicotinamid;
6-[4-(Naphthalin-1-carbonyl)-piperazin-1-yl]-*N*-(tetrahydro-furan-2-ylmethyl)-nicotinamid;
*N*-(3-Imidazol-1-yl-propyl)-6-[4-(naphthalin-2-carbonyl)-piperazin-1-yl]-nicotinamid;
*N*-[2-(3H-Imidazol-4-yl)-ethyl]-6-[4-(naphthalin-2-carbonyl)-piperazin-1-yl]-nicotinamid;
6-[4-(Naphthalin-2-carbonyl)-piperazin-1-yl]-*N*-phenethyl-nicotinamid;
6-[4-(Naphthalin-2-carbonyl)-piperazin-1-yl]-*N*-(4-phenyl-butyl)-nicotinamid;
*N*-[2-(3-Chlorphenyl)-ethyl]-6-[4-(naphthalin-2-carbonyl)-piperazin-1-yl]-nicotinamid;
*N*-(3-Imidazol-1-yl-propyl)-6-[4-(naphthalin-1-carbonyl)-piperazin-1-yl]-nicotinamid;
6-[4-(2-Naphthalin-2-yl-acetyl)-piperazin-1-yl]-*N*-(tetrahydro-furan-2-ylmethyl)-nicotinamid;
6-[4-(2-Brom-benzoyl)-piperazin-1-yl]-*N*-(3-phenyl-propyl)-nicotinamid;
*N*-[2-(3-Chlorphenyl)-ethyl]-6-[4-(2,4-dichlor-benzoyl)-piperazin-1-yl]-nicotinamid;
6-[4-(2,4-Dimethyl-benzoyl)-piperazin-1-yl]-*N*-(4-phenyl-butyl)-nicotinamid;
*N*-Phenethyl-6-[4-(2-p-tolyl-acetyl)-piperazin-1-yl]-nicotinamid;
6-{4-[2-(2,5-Dichlor-phenyl)-acetyl]-piperazin-1-yl}-*N*-(2-methyl-3-phenyl-propyl)-nicotinamid;
6-[4-(2,4-Dimethyl-benzoyl)-piperazin-1-yl]-*N*-(3-phenyl-propyl)-nicotinamid;
*N*-[2-(3-Chlorphenyl)-ethyl]-6-[4-(2-fluor-4-methyl-benzoyl)-piperazin-1-yl]-nicotinamid;
*N*-[2-(3-Chlorphenyl)-ethyl]-6-[4-(2,5-dichlor-benzoyl)-piperazin-1-yl]-nicotinamid;
*N*-(3-Phenyl-propyl)-6-[4-(4-trifluormethyl-benzoyl)-piperazin-1-yl]-nicotinamid;
6-[4-(2-Brom-benzoyl)-piperazin-1-yl]-*N*-(3-imidazol-1-yl-propyl)-nicotinamid;
*N*-(4-Phenyl-butyl)-6-[4-(2-trifluormethyl-benzoyl)-piperazin-1-yl]-nicotinamid;
*N*-(3-Phenyl-propyl)-6-[4-(3-trifluormethyl-benzoyl)-piperazin-1-yl]-nicotinamid;
6-[4-(2-Brom-benzoyl)-piperazin-1-yl]-*N*-[2-(3-chlor-phenyl)-ethyl]-nicotinamid;
6-[4-(2-Brom-benzoyl)-piperazin-1-yl]-*N*-(2-methyl-3-phenyl-propyl)-nicotinamid;
6-[4-(2-Brom-5-methoxy-benzoyl)-piperazin-1-yl]-*N*-(5-phenyl-pentyl)-nicotinamid;
6-[4-(2-Brom-5-methoxy-benzoyl)-piperazin-1-yl]-*N*-[2-(3-chlor-phenyl)-ethyl]-nicotinamid;
*N*-[2-(3-Chlorphenyl)-ethyl]-6-[4-(2-trifluormethyl-benzoyl)-piperazin-1-yl]-nicotinamid;
*N*-(3-Phenyl-propyl)-6-[4-(2-trifluormethyl-benzoyl)-piperazin-1-yl]-nicotinamid;
*N*-(4-Phenyl-butyl)-6-[4-(3-trifluormethyl-benzoyl)-piperazin-1-yl]-nicotinamid;
6-[4-(2,4-Dichlor-benzoyl)-piperazin-1-yl]-*N*-phenethyl-nicotinamid;
6-[4-(2-Brom-benzoyl)-piperazin-1-yl]-*N*-(4-phenyl-butyl)-nicotinamid;
6-[4-(2-Brom-benzoyl)-piperazin-1-yl]-*N*-phenethyl-nicotinamid;
6-[4-(2-Brom-5-methoxy-benzoyl)-piperazin-1-yl]-*N*-(1-methyl-3-phenyl-propyl)-nicotinamid;
6-[4-(3,5-Dichlor-benzoyl)-piperazin-1-yl]-*N*-(3-phenyl-propyl)-nicotinamid;
6-[4-(3,5-Dichlor-benzoyl)-piperazin-1-yl]-*N*-(1-methyl-3-phenyl-propyl)-nicotinamid;
*N*-Phenethyl-6-[4-(4-trifluormethyl-benzoyl)-piperazin-1-yl]-nicotinamid;
6-[4-(2,4-Dimethyl-benzoyl)-piperazin-1-yl]-*N*-phenethyl-nicotinamid;
*N*-Phenethyl-6-[4-(2-trifluormethyl-benzoyl)-piperazin-1-yl]-nicotinamid;
6-[4-(2,4-Dichlor-benzoyl)-piperazin-1-yl]-*N*-(1-methyl-3-phenyl-propyl)-nicotinamid;
6-[4-(2,5-Dichlor-benzoyl)-piperazin-1-yl]-*N*-(3-phenyl-propyl)-nicotinamid;
6-[4-(2-Brom-5-methoxy-benzoyl)-piperazin-1-yl]-*N*-phenethyl-nicotinamid;
6-[4-(2-Brom-5-methoxy-benzoyl)-piperazin-1-yl]-*N*-(3-imidazol-1-yl-propyl)-nicotinamid;
6-[4-(3,5-Dichlor-benzoyl)-piperazin-1-yl]-*N*-(3-imidazol-1-yl-propyl)-nicotinamid;
*N*-[2-(3H-Imidazol-4-yl)-ethyl]-6-[4-(2,3,4,5-tetrafluor-benzoyl)-piperazin-1-yl]-nicotinamid;
*N*-Phenethyl-6-[4-(3-trifluormethyl-benzoyl)-piperazin-1-yl]-nicotinamid;
6-[4-(2,5-Dichlor-benzoyl)-piperazin-1-yl]-*N*-phenethyl-nicotinamid;
6-[4-(3-Methyl-pentanoyl)-piperazin-1-yl]-*N*-(1-methyl-3-phenyl-propyl)-nicotinamid;
6-[4-(2,3,4,5-Tetrafluor-benzoyl)-piperazin-1-yl]-*N*-(tetrahydro-furan-2-ylmethyl)-nicotinamid;
6-[4-(2-Brom-5-methoxy-benzoyl)-piperazin-1-yl]-*N*-(tetrahydro-furan-2-ylmethyl)-nicotinamid;
*N*-(Tetrahydro-furan-2-ylmethyl)-6-[4-(4-trifluormethyl-benzoyl)-piperazin-1-yl]-nicotinamid;
6-[4-(3,5-Dichlor-benzoyl)-piperazin-1-yl]-*N*-(4-phenyl-butyl)-nicotinamid;
6-[4-(2,4-Dimethyl-benzoyl)-piperazin-1-yl]-*N*-(1-methyl-3-phenyl-propyl)-nicotinamid;
6-[4-(2,4-Dimethyl-benzoyl)-piperazin-1-yl]-*N*-(tetrahydro-furan-2-ylmethyl)-nicotinamid;
6-[4-(2-Brom-benzoyl)-piperazin-1-yl]-*N*-(tetrahydro-furan-2-ylmethyl)-nicotinamid;
*N*-(1-Methyl-3-phenyl-propyl)-6-[4-(3-trifluormethyl-benzoyl)-piperazin-1-yl]-nicotinamid;
*N*-Phenethyl-6-[4-(2,3,4,5-tetrafluor-benzoyl)-piperazin-1-yl]-nicotinamid;
*N*-(4-Phenyl-butyl)-6-[4-(2,3,4,5-tetrafluor-benzoyl)-piperazin-1-yl]-nicotinamid;
*N*-(1-Methyl-3-phenyl-propyl)-6-[4-(2,3,4,5-tetrafluor-benzoyl)-piperazin-1-yl]-nicotinamid;
*N*-[2-(3-Chlorphenyl)-ethyl]-6-[4-(2,3,4,5-tetrafluor-benzoyl)-piperazin-1-yl]-nicotinamid;
*N*-(3-Phenyl-propyl)-6-[4-(2,3,4,5-tetrafluor-benzoyl)-piperazin-1-yl]-nicotinamid;
6-[4-(2,5-Dichlor-benzoyl)-piperazin-1-yl]-*N*-(tetrahydro-furan-2-ylmethyl)-nicotinamid;
6-[4-(2,4-Dimethyl-benzoyl)-piperazin-1-yl]-*N*-[2-(3H-imidazol-4-yl)-ethyl]-nicotinamid;
*N*-[2-(3H-Imidazol-4-yl)-ethyl]-6-[4-(3-trifluormethyl-benzoyl)-piperazin-1-yl]-nicotinamid;
*N*-(3-Imidazol-1-yl-propyl)-6-[4-(3-trifluormethyl-benzoyl)-piperazin-1-yl]-nicotinamid;
6-[4-(2,4-Dimethyl-benzoyl)-piperazin-1-yl]-*N*-(3-imidazol-1-yl-propyl)-nicotinamid;
6-[4-(2,5-Dichlor-benzoyl)-piperazin-1-yl]-*N*-[2-(3H-imidazol-4-yl)-ethyl]-nicotinamid;
*N*-[2-(3H-Imidazol-4-yl)-ethyl]-6-[4-(4-trifluormethyl-benzoyl)-piperazin-1-yl]-nicotinamid;
6-[4-(2,4-Dichlor-benzoyl)-piperazin-1-yl]-*N*-[2-(3H-imidazol-4-yl)-ethyl]-nicotinamid;
6-[4-(3,5-Dichlor-benzoyl)-piperazin-1-yl]-*N*-(tetrahydro-furan-2-ylmethyl)-nicotinamid;
*N*-(3-Imidazol-1-yl-propyl)-6-[4-(2-trifluormethyl-benzoyl)-piperazin-1-yl]-nicotinamid;
*N*-(Tetrahydro-furan-2-ylmethyl)-6-[4-(3-trifluormethyl-benzoyl)-piperazin-1-yl]-nicotinamid;
*N*-(Tetrahydro-furan-2-ylmethyl)-6-[4-(2-trifluormethyl-benzoyl)-piperazin-1-yl]-nicotinamid;
6-[4-(2,4-Dichlor-benzoyl)-piperazin-1-yl]-*N*-(tetrahydro-furan-2-ylmethyl)-nicotinamid;
*N*-[2-(3H-Imidazol-4-yl)-ethyl]-6-[4-(2-trifluormethyl-benzoyl)-piperazin-1-yl]-nicotinamid;
6-[4-(2-Brom-5-methoxy-benzoyl)-piperazin-1-yl]-*N*-[2-(3H-imidazol-4-yl)-ethyl]-nicotinamid;
*N*-[2-(3-Chlorphenyl)-ethyl]-6-[4-(3-trifluormethyl-benzoyl)-piperazin-1-yl]-nicotinamid;
*N*-[2-(3-Chlorphenyl)-ethyl]-6-[4-(4-trifluormethyl-benzoyl)-piperazin-1-yl]-nicotinamid;
*N*-[2-(3-Chlorphenyl)-ethyl]-6-[4-(3,5-dichlor-benzoyl)-piperazin-1-yl]-nicotinamid;
*N*-(4-Phenyl-butyl)-6-[4-(4-trifluormethyl-benzoyl)-piperazin-1-yl]-nicotinamid;
6-[4-(2,4-Dichlor-benzoyl)-piperazin-1-yl]-*N*-(4-phenyl-butyl)-nicotinamid;
6-[4-(2,4-Dichlor-benzoyl)-piperazin-1-yl]-*N*-(3-phenyl-propyl)-nicotinamid;
6-[4-(2,5-Dichlor-benzoyl)-piperazin-1-yl]-*N*-(4-phenyl-butyl)-nicotinamid;
6-[4-(3,5-Dichlor-benzoyl)-piperazin-1-yl]-*N*-phenethyl-nicotinamid;
*N*-(3-Imidazol-1-yl-propyl)-6-[4-(4-trifluormethyl-benzoyl)-piperazin-1-yl]-nicotinamid;
6-[4-(3,5-Dichlor-benzoyl)-piperazin-1-yl]-*N*-[2-(3H-imidazol-4-yl)-ethyl]-nicotinamid;
*N*-(3-Imidazol-1-yl-propyl)-6-[4-(2,3,4,5-tetrafluor-benzoyl)-piperazin-1-yl]-nicotinamid;
6-[2-Oxo-4-(2-trifluormethyl-benzoyl)-piperazin-1-yl]-*N*-(3-phenyl-propyl)-nicotinamid;
*N*-(3-Methyl-butyl)-6-[2-oxo-4-(2-trifluormethyl-benzoyl)-piperazin-1-yl]-nicotinamid.

23. Verwendung der Verbindung nach Anspruch 1, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Heteroaryl, gegebenenfalls substituiertes Heteroarylalkyl oder gegebenenfalls substituiertes Heteroarylalkenyl;
jedes R° ist unabhängig Wasserstoff, Alkyl, Alkenyl, Halogen, Halogenalkyl oder Aryl;
jedes R⁵ und R⁶ ist unabhängig Wasserstoff, Oxo, Alkyl, Alkenyl, Halogen, Halogenalkyl oder Aryl;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl; und
R¹⁰ ist Alkyl, Aryl oder Aralkyl.

24. Verwendung der Verbindung nach Anspruch 23, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Cycloalkyl, gegebenenfalls substituiertem Cycloalkylalkyl oder Cycloalkylalkenyl;
R³ ist gegebenenfalls substituiertes Heteroaryl, gegebenenfalls substituiertes Heteroarylalkyl oder gegebenenfalls substituiertes Heteroarylalkenyl;
jedes R⁴ ist unabhängig Wasserstoff, Alkyl, Halogen oder Halogenalkyl;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl
und Aralkyl; und
R¹⁰ ist Alkyl, Aryl oder Aralkyl.

25. Verwendung der Verbindung nach Anspruch 24, wobei die Verbindung der Formel (I) eine Verbindung ist, worin:
m ist 1 oder 2;
n ist 1;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist) oder Alkyl;
R³ ist gegebenenfalls substituiertes Heteroaryl, gegebenenfalls substituiertes Heteroarylalkyl oder gegebenenfalls substituiertes Heteroarylalkenyl;
jedes R⁴ ist unabhängig Wasserstoff, Halogen oder Halogenalkyl;
R⁵ ist Wasserstoff;
jedes R⁶ ist Wasserstoff;
R⁷ ist eine gerade oder verzweigte Alkylenkette;
R⁸ ist Wasserstoff oder Alkyl; und
R¹⁰ ist Alkyl, Aryl oder Aralkyl.

26. Verwendung der Verbindung nach Anspruch 25, wobei die Verbindung der Formel (I) ausgewählt ist aus der Gruppe bestehend aus den folgenden:
6-[4-(3-Methyl-3H-114-thiophen-2-carbonyl)-piperazin-1-yl]-N-pentyl-nicotinamid;
N-(3-Dimethylamino-propyl)-6-[4-(3-methyl-thiophen-2-carbonyl)-piperazin-1-yl]-nicotinamid;
*N*-(3-Dimethylamino-propyl)-6-[4-(thiophen-2-carbonyl)-piperazin-1-yl]-nicotinamid;
6-[4-(2-Chlor-pyridin-3-carbonyl)-piperazin-1-yl]-*N*-(3-ethoxy-propyl)-nicotinamid;
6-[4-(2-Chlor-pyridin-3-carbonyl)-piperazin-1-yl]-*N*-(2-ethylsulfanyl-ethyl)-nicotinamid;
6-[4-(2-Chlor-pyridin-3-carbonyl)-piperazin-1-yl]-*N*-(3-methylbutyl)-nicotinamid;
6-[4-(2-Chlor-pyridin-3-carbonyl)-piperazin-1-yl]-*N*-(3-isopropoxy-propyl)-nicotinamid;
*N*-(2-Ethylsulfanyl-ethyl)-6-[4-(thiophen-2-carbonyl)-piperazin-1-yl]-nicotinamid;
*N*-(3-Ethoxy-propyl)-6-[4-(thiophen-2-carbonyl)-piperazin-1-yl]-nicotinamid;
*N*-(3-Methyl-butyl)-6-[4-(thiophen-2-carbonyl)-piperazin-1-yl]-nicotinamid;
6-[4-(2-Chlor-pyridin-3-carbonyl)-piperazin-1-yl]-*N*-(3-methoxy-propyl)-nicotinamid;
*N*-Pentyl-6-[4-(thiophen-2-carbonyl)-piperazin-1-yl]-nicotinamid;
6-[4-(2-Chlor-pyridin-3-carbonyl)-piperazin-1-yl]-*N*-hexyl-nicotinamid;
*N*-(3-Isopropoxy-propyl)-6-[4-(thiophen-2-carbonyl)-piperazin-1-yl]-nicotinamid;
*N*-(1-Methyl-butyl)-6-[4-(thiophen-2-carbonyl)-piperazin-1-yl]-nicotinamid;
*N*-Hexyl-6-[4-(thiophen-2-carbonyl)-piperazin-1-yl]-nicotinamid;
*N*-(1,3-Dimethylbutyl)-6-[4-(thiophen-2-carbonyl)-piperazin-1-yl]-nicotinamid;
*N*-Butyl-6-(4-(thiophen-2-carbonyl)-piperazin-1-yl]-nicotinamid;
*N*-(3-Methoxy-propyl)-6-[4-(thiophen-2-carbonyl)-piperazin-1-yl]-nicotinamid;
6-[4-(2-Chlor-pyridin-3-carbonyl)-piperazin-1-yl]-*N*-(1,3-dimethylbutyl)-nicotinamid;
*N*-Butyl-6-[4-(2-chlor-pyridin-3-carbonyl)-piperazin-1-yl]-nicotinamid;
6-[4-(2-Chlor-pyridin-3-carbonyl)-piperazin-1-yl]-*N*-(2-methylbutyl)-nicotinamid;
*N*-(2-Methyl-butyl)-6-[4-(thiophen-2-carbonyl)-piperazin-1-yl]-nicotinamid;
6-[4-(2-Chlor-pyridin-3-carbonyl)-piperazin-1-yl]-*N*-(1-methylbutyl)-nicotinamid;
*N*-(1,3-Dimethylbutyl)-6-[4-(3-methyl-thiophen-2-carbonyl)-piperazin-1-yl]-nicotinamid;
*N*-Butyl-6-[4-(3-methyl-thiophen-2-carbonyl)-piperazin-1-yl]-nicotinamid;
*N*-(3-Butoxy-propyl)-6-[4-(3-methyl-thiophen-2-carbonyl)-piperazin-1-yl]-nicotinamid;
*N*-(3-Isopropoxy-propyl)-6-[4-(3-methyl-thiophen-2-carbonyl)-piperazin-1-yl]-nicotinamid;
*N*-(3-Ethoxy-propyl)-6-[4-(3-methyl-thiophen-2-carbonyl)-piperazin-1-yl]-nicotinamid;
*N*-(2-Methyl-butyl)-6-[4-(3-methyl-thiophen-2-carbonyl)-piperazin-1-yl]-nicotinamid;
*N*-(2-Ethylsulfanyl-ethyl)-6-[4-(3-methyl-thiophen-2-carbonyl)-piperazin-1-yl]-nicotinamid;
*N*-(3-Methoxy-propyl)-6-[4-(3-methyl-thiophen-2-carbonyl)-piperazin-1-yl]-nicotinamid;
*N*-(1-Methyl-butyl)-6-[4-(3-methyl-thiophen-2-carbonyl)-piperazin-1-yl]-nicotinamid;
*N*-(3-Methyl-butyl)-6-[4-(3-methyl-thiophen-2-carbonyl)-piperazin-1-yl]-nicotinamid;
*N*-(3-Butoxy-propyl)-6-[4-(2-chlor-pyridin-3-carbonyl)-piperazin-1-yl]-nicotinamid;
*N*-(3-Butoxy-propyl)-6-[4-(thiophen-2-carbonyl)-piperazin-1-yl]-nicotinamid;
6-[4-(2-Chlor-pyridin-3-carbonyl)-piperazin-1-yl]-*N*-pentyl-nicotinamid;
*N*-Hexyl-6-[4-(3-methyl-thiophen-2-carbonyl)-piperazin-1-yl]-nicotinamid; und
6-[4-(2-Chlor-pyridin-3-carbonyl)-piperazin-1-yl]-*N*-(3-dimethylamino-propyl)-nicotinamid.

27. Verwendung der Verbindung nach Anspruch 23, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Heteroaryl, gegebenenfalls substituiertes Heteroarylalkyl oder gegebenenfalls substituiertes Heteroarylalkenyl;
jedes R⁴ ist unabhängig Wasserstoff, Alkyl, Halogen oder Halogenalkyl; und
jedes R⁵ und R⁶ ist unabhängig Wasserstoff, Oxo, Alkyl, Halogen oder Halogenalkyl.

28. Verwendung der Verbindung nach Anspruch 27, wobei die Verbindung der Formel (I) eine Verbindung ist, worin:
m ist 1 oder 2;
nist1;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Heterocyclyl, gegebenenfalls substituiertem Heterocyclylalkyl, gegebenenfalls substituiertem Heteroaryl und
gegebenenfalls substituiertem Heteroarylalkyl;
R³ ist gegebenenfalls substituiertes Heteroaryl, gegebenenfalls substituiertes Heteroarylalkyl oder gegebenenfalls substituiertes Heteroarylalkenyl;
jedes R⁴ ist unabhängig Wasserstoff, Alkyl, Halogen oder Halogenalkyl;
R⁵ ist Wasserstoff, Oxo, Alkyl, Halogen oder Halogenalkyl; und
jedes R⁶ ist unabhängig Wasserstoff, Oxo, Alkyl, Halogen oder Halogenalkyl.

29. Verwendung der Verbindung nach Anspruch 21, wobei die Verbindung der Formel (I) ausgewählt ist aus der Gruppe bestehend aus den folgenden:
*N*-[2-(3-Chlorphenyl)-ethyl]-6-[4-(3-methyl-thiophen-2-carbonyl)-piperazin-1-yl]-nicotinamid;
*N*-[2-(3-Chlorphenyl)-ethyl]-6-{4-[2-(2-chlor-pyridin-3-yl)-acetyl]-piperazin-1-yl}-nicotinamid;
6-[4-(2-Chlor-pyridin-3-carbonyl)-piperazin-1-yl]-*N*-(4-phenyl-butyl)-nicotinamid;
*N*-[2-(3-Chlorphenyl)-ethyl]-6-[4-(thiophen-2-carbonyl)-piperazin-1-yl]-nicotinamid;
6-[4-(2-Chlor-pyridin-3-carbonyl)-piperazin-1-yl]-*N*-(3-phenyl-propyl)-nicotinamid;
*N*-(3-Phenyl-propyl)-6-[4-(thiophen-2-carbonyl)-piperazin-1-yl]-nicotinamid;
*N*-Phenethyl-6-[4-(thiophen-2-carbonyl)-piperazin-1-yl]-nicotinamid;
*N*-(1-Methyl-3-phenyl-propyl)-6-[4-(thiophen-2-carbonyl)-piperazin-1-yl]-nicotinamid;
*N*-(4-Phenyl-butyl)-6-[4-(thiophen-2-carbonyl)-piperazin-1-yl]-nicotinamid;
6-[4-(2-Chlor-pyridin-3-carbonyl)-piperazin-1-yl]-*N*-(1-methyl-3-phenyl-propyl)-nicotinamid;
6-[4-(2-Chlor-pyridin-3-carbonyl)-piperazin-1-yl]-*N*-phenethyl-nicotinamid;
6-(4-(2-Chlor-pyridin-3-carbonyl)-piperazin-1-yl]-*N*-(tetrahydro-furan-2-ylmethyl)-nicotinamid;
6-[4-(3-Methyl-thiophen-2-carbonyl)-piperazin-1-yl]-*N*-(3-phenyl-propyl)-nicotinamid;
*N*-(1-Methyl-3-phenyl-propyl)-6-[4-(3-methyl-thiophen-2-carbonyl)-piperazin-1-yl]-nicotinamid;
6-[4-(3-Methyl-thiophen-2-carbonyl)-piperazin-1-yl]-*N*-(4-phenyl-butyl)-nicotinamid;
6-[4-(3-Methyl-thiophen-2-carbonyl)-piperazin-1-yl]-*N*-phenethyl-nicotinamid;
6-[4-(3-Methyl-thiophen-2-carbonyl)-piperazin-1-yl]-*N*-(tetrahydro-furan-2-ylmethyl)-nicotinamid;
*N*-(3-Imidazol-1-yl-propyl)-6-[4-(thiophen-2-carbonyl)-piperazin-1-yl]-nicotinamid;
*N*-(Tetrahydro-furan-2-ylmethyl)-6-[4-(thiophen-2-carbonyl)-piperazin-1-yl]-nicotinamid;
*N*-[2-(3H-Imidazol-4-yl)-ethyl]-6-[4-(thiophen-2-carbonyl)-piperazin-1-yl]-nicotinamid;
*N*-(3-Imidazol-1-yl-propyl)-6-[4-(3-methyl-thiophen-2-carbonyl)-piperazin-1-yl]-nicotinamid;
*N*-[2-(3H-Imidazol-4-yl)-ethyl]-6-[4-(3-methyl-thiophen-2-carbonyl)-piperazin-1-yl]-nicotinamid;
6-[4-(2-Chlor-pyridin-3-carbonyl)-piperazin-1-yl]-*N*-(3-imidazol-1-yl-propyl)-nicotinamid; und
6-[4-(2-Chlor-pyridin-3-carbonyl)-piperazin-1-yl]-*N*-[2-(3H-imidazol-4-yl)-ethyl]-nicotinamid.

30. Verwendung der Verbindung nach Anspruch 1, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Aralkyl oder gegebenenfalls substituiertes Aralkenyl;
jedes R⁴ ist unabhängig Wasserstoff, Alkyl, Alkenyl, Halogen, Halogenalkyl, Aryl oder -R⁹-OR⁸;
jedes R⁵ und R⁶ ist unabhängig Wasserstoff, Oxo, Alkyl, Alkenyl, Halogen, Halogenalkyl oder Aryl;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
R⁹ ist eine direkte Bindung oder eine gerade oder verzweigte Alkylenkette; und
R¹⁰ ist Alkyl, Aryl oder Aralkyl.

31. Verwendung der Verbindung nach Anspruch 30, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Cycloalkyl, gegebenenfalls substituiertem Cycloalkylalkyl oder Cycloalkylalkenyl;
R³ ist gegebenenfalls substituiertes Aralkyl oder gegebenenfalls substituiertes Aralkenyl;
jedes R⁴ ist unabhängig Wasserstoff, Alkyl, Halogen oder Halogenalkyl;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl
und Aralkyl; und
R¹⁰ ist Alkyl, Aryl oder Aralkyl.

32. Verwendung der Verbindung nach Anspruch 31, wobei die Verbindung der Formel (I) eine Verbindung ist, worin:
m ist 1 oder 2;
n ist 1;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂,- -R⁷-S(O)ₜR¹⁰ (wobei t 0, 1 oder 2 ist) oder Alkyl;
R³ ist gegebenenfalls substituiertes Aralkyl oder gegebenenfalls substituiertes Aralkenyl;
jedes R⁴ ist unabhängig Wasserstoff, Halogen oder Halogenalkyl;
R⁵ ist Wasserstoff;
jedes R⁶ ist Wasserstoff;
R⁷ ist eine gerade oder verzweigte Alkylenkette;
R⁸ ist Wasserstoff oder Alkyl; und
R¹⁰ ist Alkyl, Aryl oder Aralkyl.

33. Verwendung der Verbindung nach Anspruch 32, wobei die Verbindung der Formel (I) ausgewählt ist aus der Gruppe bestehend aus den folgenden:
6-{4-[2-(4-Chlorphenyl)-propionyl]-piperazin-1-yl}-*N*-(3-methylbutyl)-nicotinamid;
6-{4-[2-(2-Chlor-6-fluor-phenyl)-acetyl]-piperazin-1-yl}-*N*-(3-ethoxy-propyl)-nicotinamid;
6-{4-[3-(3,4-Difluor-phenyl)-propionyl]-piperazin-1-yl}-*N*-(3-dimethylamino-propyl)-nicotinamid;
6-{4-[3-(3,4-Difluor-phenyl)-propionyl]-piperazin-1-yl}-*N*-(3-ethoxy-propyl)-nicotinamid;
6-{4-[2-(4-Chlorphenyl)-propionyl]-piperazin-1-yl}-*N*-(3-ethoxy-propyl)-nicotinamid;
*N*-(3-Ethoxy-propyl)-6-[4-(2-phenyl-butyryl)-piperazin-1-yl]-nicotinamid;
*N*-(2-Ethylsulfanyl-ethyl)-6-[4-(2-o-tolyl-acetyl)-piperazin-1-yl]-nicotinamid;
6-[4-(3-Methyl-pentanoyl)-piperazin-1-yl]-*N*-(4-phenyl-butyl)-nicotinamid;
*N*-Butyl-6-{4-[3-(3,4-difluor-phenyl)-propionyl]-piperazin-1-yl}-nicotinamid;
6-{4-[2-(2-Chlor-6-fluor-phenyl)-acetyl]-piperazin-1-yl}-*N*-(3-dimethylamino-propyl)-nicotinamid;
6-{4-[3-(3,4-Difluor-phenyl)-propionyl]-piperazin-1-yl}-*N*-(2-ethylsulfanyl-ethyl)-nicotinamid;
*N*-(3-Methoxy-propyl)-6-[4-(2-phenyl-butyryl)-piperazin-1-yl]-nicotinamid;
*N*-Butyl-6-[4-(2-phenyl-butyryl)-piperazin-1-yl]-nicotinamid;
*N*-(3-Butoxy-propyl)-6-{4-[2-(4-chlor-phenyl)-propionyl]-piperazin-1-yl}-nicotinamid;
*N*-(3-Methoxy-propyl)-6-[4-(2-o-tolyl-acetyl)-piperazin-1-yl]-nicotinamid;
*N*-(3-Methyl-butyl)-6-[4-(2-phenyl-butyryl)-piperazin-1-yl]-nicotinamid;
*N*-(1-Methyl-butyl)-6-[4-(2-p-tolyl-acetyl)-piperazin-1-yl]-nicotinamid;
*N*-Butyl-6-{4-[2-(2-chlor-6-fluor-phenyl)-acetyl]-piperazin-1-yl}-nicotinamid;
*N*-(2-Methyl-butyl)-6-[4-(2-o-tolyl-acetyl)-piperazin-1-yl]-nicotinamid;
6-{4-[3-(3,4-Difluor-phenyl)-propionyl]-piperazin-1-yl}-N-(2-methylbutyl)-nicotinamid;
N-(3-Isopropoxy-propyl)-6-[4-(2-o-tolyl-acetyl)-piperazin-1-yl]-nicotinamid;
6-{4-[3-(3,4-Difluor-phenyl)-propionyl]-piperazin-1-yl}-*N*-(3-methylbutyl)-nicotinamid;
6-{4-[3-(3,4-Difluor-phenyl)-propionyl]-piperazin-1-yl}-*N*-(3-methoxy-propyl)-nicotinamid;
*N*-(1-Methyl-butyl)-6-[4-(2-o-tolyl-acetyl)-piperazin-1-yl]-nicotinamid;
6-{4-[2-(2-Chlor-6-fluor-phenyl)-acetyl]-piperazin-1-yl}-*N*-(2-methylbutyl)-nicotinamid;
6-{4-[2-(2-Chlor-6-fluor-phenyl)-acetyl]-piperazin-1-yl}-*N*-(1-methylbutyl)-nicotinamid;
*N*-(2-Methyl-butyl)-6-[4-(2-phenyl-butyryl)-piperazin-1-yl]-nicotinamid;
*N*-(1,3-Dimethylbutyl)-6-[4-(2-phenyl-butyryl)-piperazin-1-yl]-nicotinamid;
6-{4-[2-(4-Chlorphenyl)-propionyl]-piperazin-1-yl}-*N*-(2-methylbutyl)-nicotinamid;
6-{4-(2-(2-Chlor-6-fluor-phenyl)-acetyl]-piperazin-1-yl}-*N*-(3-isopropoxy-propyl)-nicotinamid;
*N*-(1,3-Dimethylbutyl)-6-[4-(2-p-tolyl-acetyl)-piperazin-1-yl]-nicotinamid;
6-{4-[3-(3,4-Difluor-phenyl)-propionyl]-piperazin-1-yl}-*N*-hexyl-nicotinamid;
*N*-(1-Methyl-butyl)-6-[4-(2-phenyl-butyryl)-piperazin-1-yl]-nicotinamid;
6-{4-[2-(4-Chlorphenyl)-propionyl]-piperazin-1-yl)-*N*-(1-methylbutyl)-nicotinamid;
6-{4-[2-(4-Chlorphenyl)-propionyl]-piperazin-1-yl)-*N*-(1,3-dimethylbutyl)-nicotinamid;
*N*-Butyl-6-[4-(2-p-tolyl-acetyl)-piperazin-1-yl]-nicotinamid;
*N*-(1,3-Dimethylbutyl)-6-[4-(2-o-tolyl-acetyl)-piperazin-1-yl]-nicotinamid;
6-{4-[2-(2-Chlor-6-fluor-phenyl)-acetyl]-piperazin-1-yl}-*N*-(1,3-dimethylbutyl)-nicotinamid;
*N*-(3-Methyl-butyl)-6-[4-(2-p-tolyl-acetyl)-piperazin-1-yl]-nicotinamid;
*N*-Butyl-6-[4-(2-o-tolyl-acetyl)-piperazin-1-yl]-nicotinamid;
*N*-(3-Butoxy-propyl)-6-[4-(2-p-tolyl-acetyl)-piperazin-1-yl]-nicotinamid;
*N*-Pentyl-6-[4-(2-p-tolyl-acetyl)-piperazin-1-yl]-nicotinamid;
*N*-(3-Butoxy-propyl)-6-[4-(2-phenyl-butyryl)-piperazin-1-yl]-nicotinamid;
*N*-Pentyl-6-[4-(2-phenyl-butyryl)-piperazin-1-yl]-nicotinamid;
*N*-(2-Ethylsulfanyl-ethyl)-6-[4-(2-p-tolyl-acetyl)-piperazin-1-yl]-nicotinamid;
6-{4-[2-(4-Chlorphenyl)-propionyl]-piperazin-1-yl}-*N*-(2-ethylsulfanyl-ethyl)-nicotinamid;
*N*-(3-Ethoxy-propyl)-6-[4-(2-o-tolyl-acetyl)-piperazin-1-yl]-nicotinamid;
6-{4-[2-(2-Chlor-6-fluor-phenyl)-acetyl]-piperazin-1-yl}-*N*-(3-methoxy-propyl)-nicotinamid;
6-{4-[3-(3,4-Difluor-phenyl)-propionyl]-piperazin-1-yl}-*N*-(3-isopropoxy-propyl)-nicotinamid;
6-{4-[2-(4-Chlorphenyl)-propionyl]-piperazin-1-yl}-*N*-pentyl-nicotinamid;
*N*-(3-Butoxy-propyl)-6-{4-[2-(2-chlor-6-fluor-phenyl)-acetyl]-piperazin-1-yl}-nicotinamid;
6-{4-[2-(2-Chlor-6-fluor-phenyl)-acetyl]-piperazin-1-yl}-*N*-hexyl-nicotinamid;
6-(4-[2-(4-Chlorphenyl)-propionyl]-piperazin-1-yl}-*N*-(3-methoxy-propyl)-nicotinamid;
*N*-Hexyl-6-[4-(2-o-tolyl-acetyl)-piperazin-1-yl]-nicotinamid;
6-{4-[3-(3,4-Difluor-phenyl)-propionyl]-piperazin-1-yl}-N-(4-phenyl-butyl)-nicotinamid;
*N*-(3-Isopropoxy-propyl)-6-[4-(2-p-tolyl-acetyl)-piperazin-1-yl]-nicotinamid;
6-{4-[3-(3,4-Difluor-phenyl)-propionyl]-piperazin-1-yl}-*N*-(1-methylbutyl)-nicotinamid;
6-{4-[3-(3,4-Difluor-phenyl)-propionyl]-piperazin-1-yl}-*N*-pentyl-nicotinamid;
6-{4-[2-(4-Chlorphenyl)-propionylj-piperazin-1-yl}-*N*-hexyl-nicotinamid;
*N*-Butyl-6-{4-[2-(4-chlor-phenyl)-propionyl]-piperazin-1-yl}-nicotinamid;
6-{4-[2-(2-Chlor-6-fluor-phenyl)-acetyl]-piperazin-1-yl}-*N*-(2-ethylsulfanyl-ethyl)-nicotinamid;
6-{4-[2-(2-Chlor-6-fluor-phenyl)-acetyl]-piperazin-1-yl}-*N*-pentyl-nicotinamid;
*N*-Hexyl-6-[4-(2-phenyl-butyryl)-piperazin-1-yl]-nicotinamid;
*N*-(3-Isopropoxy-propyl)-6-[4-(2-phenyl-butyryl)-piperazin-1-yl]-nicotinamid;
*N*-Pentyl-6-[4-(2-o-tolyl-acetyl)-piperazin-1-yl]-nicotinamid;
*N*-Hexyl-6-[4-(2-p-tolyl-acetyl)-piperazin-1-yl]-nicotinamid;
6-{4-[3-(3,4-Difluor-phenyl)-propionyl]-piperazin-1-yl}-*N*-(1,3-dimethylbutyl)-nicotinamid;
6-[4-(Naphthalin-2-carbonyl)-piperazin-1-yl]-*N*-pentyl-nicotinamid;
6-{4-[2-(4-Chlorphenyl)-propionyl]-piperazin-1-yl}-*N*-(3-dimethylamino-propyl)-nicotinamid;
*N*-(3-Dimethylamino-propyl)-6-[4-(2-phenyl-butyryl)-piperazin-1-yl]-nicotinamid;
*N*-(3-Dimethylamino-propyl)-6-[4-(2-p-tolyl-acetyl)-piperazin-1-yl]-nicotinamid;
*N*-(3-Dimethylamino-propyl)-6-[4-(2-o-tolyl-acetyl)-piperazin-1-yl]-nicotinamid;
*N*-(3-Butoxy-propyl)-6-{4-[3-(3,4-difluor-phenyl)-propionyl]-piperazin-1-yl}-nicotinamid;
*N*-(3-Methoxy-propyl)-6-[4-(2-p-tolyl-acetyl)-piperazin-1-yl]-nicotinamid;
*N*-(2-Methyl-butyl)-6-[4-(2-p-tolyl-acetyl)-piperazin-1-yl]-nicotinamid;
*N*-(3-Butoxy-propyl)-6-[4-(2-o-tolyl-acetyl)-piperazin-1-yl]-nicotinamid; und
6-{4-[2-(4-Chlorphenyl)-propionyl]-piperazin-1-yl}-*N*-(3-isopropoxy-propyl)-nicotinamid.

34. Verwendung der Verbindung nach Anspruch 30, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Aralkyl oder gegebenenfalls substituiertes Aralkenyl;
jedes R⁴ ist unabhängig Wasserstoff, Alkyl, Halogen oder Halogenalkyl; und
jedes R⁵ und R⁶ ist unabhängig Wasserstoff, Oxo, Alkyl, Halogen oder Halogenalkyl.

35. Verwendung der Verbindung nach Anspruch 34, wobei die Verbindung der Formel (I) eine Verbindung ist, worin:
m ist 1 oder 2;
n ist 1;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Heterocyclyl, gegebenenfalls substituiertem Heterocyclylalkyl, gegebenenfalls substituiertem Heteroaryl und
gegebenenfalls substituiertem Heteroarylalkyl;
R³ ist gegebenenfalls substituiertes Aralkyl oder gegebenenfalls substituiertes Aralkenyl;
jedes R⁴ ist unabhängig Wasserstoff, Alkyl, Halogen oder Halogenalkyl; und
R⁵ ist Wasserstoff, Oxo, Alkyl, Halogen oder Halogenalkyl; und
jedes R⁶ ist unabhängig Wasserstoff, Oxo, Alkyl, Halogen oder Halogenalkyl.

36. Verwendung der Verbindung nach Anspruch 35, wobei die Verbindung der Formel (I) ausgewählt ist aus der Gruppe bestehend aus den folgenden:
6-[4-(2-Phenyl-butyryl)-piperazin-1-yl]-*N*-(3-phenyl-propyl)-nicotinamid;
6-{4-[3-(3,4-Difluor-phenyl)-propionyl]-piperazin-1-yl}-*N*-phenethyl-nicotinamid;
*N*-[2-(3-Chlorphenyl)-ethyl]-6-[4-(2-o-tolyl-acetyl)-piperazin-1-yl]-nicotinamid;
6-{4-[2-(2-Chlor-6-fluor-phenyl)-acetyl]-piperazin-1-yl}-*N*-(3-phenyl-propyl)-nicotinamid;
*N*-(1-Methyl-3-phenyl-propyl)-6-[4-(2-p-tolyl-acetyl)-piperazin-1-yl]-nicotinamid;
*N*-[2-(3-Chlorphenyl)-ethyl]-6-[4-(2-phenyl-butyryl)-piperazin-1-yl]-nicotinamid;
6-{4-[2-(2-Chlor-6-fluor-phenyl)-acetyl]-piperazin-1-yl}-*N*-phenethyl-nicotinamid;
*N*-(3-Phenyl-propyl)-6-[4-(2-o-tolyl-acetyl)-piperazin-1-yl]-nicotinamid;
6-{4-[2-(2-Chlor-6-fluor-phenyl)-acetyl]-piperazin-1-yl}-*N*-[2-(3-chlor-phenyl)-ethyl]-nicotinamid;
6-{4-[2-(4-Chlorphenyl)-propionyl]-piperazin-1-yl}-*N*-(3-phenyl-propyl)-nicotinamid;
6-{4-[2-(4-Chlorphenyl)-propionyl]-piperazin-1-yl}-*N*-(1-methyl-3-phenyl-propyl)-nicotinamid;
*N*-[2-(3-Chlorphenyl)-ethyl]-6-{4-[2-(4-chlor-phenyl)-propionyl]-piperazin-1-yl}-nicotinamid;
*N*-[2-(3-Chlorphenyl)-ethyl]-6-[4-(2-p-tolyl-acetyl)-piperazin-1-yl]-nicotinamid;
*N*-Phenethyl-6-[4-(2-p-tolyl-acetyl)-piperazin-1-yl]-nicotinamid;
*N*-(3-Phenyl-propyl)-6-[4-(2-p-tolyl-acetyl)-piperazin-1-yl]-nicotinamid;
*N*-(1-Methyl-3-phenyl-propyl)-6-[4-(2-phenyl-butyryl)-piperazin-1-yl]-nicotinamid;
6-{4-[2-(4-Chlorphenyl)-propionyl]-piperazin-1-yl}-*N*-(4-phenyl-butyl)-nicotinamid;
*N*-(4-Phenyl-butyl)-6-[4-(2-phenyl-butyryl)-piperazin-1-yl]-nicotinamid;
6-{4-[2-(2-Chlor-6-fluor-phenyl)-acetyl]-piperazin-1-yl}-*N*-(1-methyl-3-phenyl-propyl)-nicotinamid;
6-{4-[3-(3,4-Difluor-phenyl)-propionyl]-piperazin-1-yl}-*N*-(3-phenyl-propyl)-nicotinamid;
*N*-Phenethyl-6-[4-(2-o-tolyl-acetyl)-piperazin-1-yl]-nicotinamid;
6-{4-[3-(3,4-Difluor-phenyl)-propionyl]-piperazin-1-yl}-*N*-(1-methyl-3-phenyl-propyl)-nicotinamid;
*N*-(4-Phenyl-butyl)-6-[4-(2-o-tolyl-acetyl)-piperazin-1-yl]-nicotinamid;
*N*-(3-Imidazol-1-yl-propyl)-6-[4-(2-phenyl-butyryl)-piperazin-1-yl]-nicotinamid;
6-{4-[2-(2-Chlor-6-fluor-phenyl)-acetyl]-piperazin-1-yl}-*N*-(4-phenyl-butyl)-nicotinamid;
6-{4-[2-(4-Chlorphenyl)-propionyl]-piperazin-1-yl}-*N*-[2-(3H-imidazol-4-yl)-ethyl]-nicotinamid;
6-{4-[2-(2-Chlor-6-fluor-phenyl)-acetyl]-piperazin-1-yl}-*N*-(3-imidazol-1-yl-propyl)-nicotinamid;
*N*-[2-(3H-lmidazol-4-yl)-ethyl]-6-[4-(2-p-tolyl-acetyl)-piperazin-1-yl]-nicotinamid;
6-{4-[2-(4-Chlorphenyl)-propionyl]-piperazin-1-yl}-*N*-(3-imidazol-1-yl-propyl)-nicotinamid;
*N*-(2-Ethylsulfanyl-ethyl)-6-[4-(2-phenyl-butyryl)-piperazin-1-yl]-nicotinamid;
*N*-(1-Methyl-3-phenyl-propyl)-6-[4-(2-o-tolyl-acetyl)-piperazin-1-yl]-nicotinamid;
6-{4-[3-(3,4-Difluor-phenyl)-propionyl]-piperazin-1-yl}-*N*-phenethyl-nicotinamid;
*N*-Phenethyl-6-[4-(2-phenyl-butyryl)-piperazin-1-yl]-nicotinamid;
*N*-(Tetrahydro-furan-2-ylmethyl)-6-[4-(2-p-tolyl-acetyl)-piperazin-1-yl]-nicotinamid;
6-{4-[2-(4-Chlorphenyl)-propionyl]-piperazin-1-yl}-*N*-(tetrahydro-furan-2-ylmethyl)-nicotinamid;
*N*-(4-Phenyl-butyl)-6-[4-(2-p-tolyl-acetyl)-piperazin-1-yl]-nicotinamid;
6-{4-[3-(3,4-Difluor-phenyl)-propionyl]-piperazin-1-yl}-*N*-(tetrahydro-furan-2-ylmethyl)-nicotinamid;
*N*-(Tetrahydro-furan-2-ylmethyl)-6-[4-(2-o-tolyl-acetyl)-piperazin-1-yl]-nicotinamid;
6-[4-(2-Phenyl-butyryl)-piperazin-1-yl]-*N*-(tetrahydro-furan-2-ylmethyl)-nicotinamid;
6-{4-[3-(3,4-Difluor-phenyl)-propionyl]-piperazin-1-yl}-*N*-[2-(3H-imidazol-4-yl)-ethyl]-nicotinamid;
*N*-[2-(3H-lmidazol-4-yl)-ethyl]-6-[4-(2-o-tolyl-acetyl)-piperazin-1-yl]-nicotinamid;
*N*-(3-Imidazol-1-yl-propyl)-6-[4-(2-o-tolyl-acetyl)-piperazin-1-yl]-nicotinamid;
6-{4-[3-(3,4-Difluor-phenyl)-propionyl]-piperazin-1-yl}-N-(3-imidazol-1-yl-propyl)-nicotinamid;
*N*-[2-(3H-Imidazol-4-yl)-ethyl]-6-[4-(2-phenyl-butyryl)-piperazin-1-yl]-nicotinamid;
*N*-(3-Imidazol-1-yl-propyl)-6-[4-(2-p-tolyl-acetyl)-piperazin-1-yl]-nicotinamid;
6-{4-[2-(2-Chlor-6-fluor-phenyl)-acetyl]-piperazin-1-yl}-*N*-(tetrahydro-furan-2-ylmethyl)-nicotinamid; und
6-{4-[2-(2-Chlor-6-fluor-phenyl)-acetyl]-piperazin-1-yl}-*N*-[2-(3H-imidazol-4-yl)-ethyl]-nicotinamid.

37. Verwendung der Verbindung nach Anspruch 1, wobei der Säuger ein Mensch ist.

38. Verwendung der Verbindung nach Anspruch 37, wobei die Krankheit oder der Zustand eine Krankheit oder ein Zustand ist, die bzw. der mit den Serumspiegeln von Triglycerid, VLDL, HDL, LDL, Gesamtcholesterin oder dem Prozess des reversen Cholesterintransports zusammenhängt.

39. Verwendung der Verbindung nach Anspruch 37, wobei die Krankheit oder der Zustand eine Krankheit oder ein Zustand ist, die bzw. der mit Serumtriglyceridspiegeln zusammenhängt.

40. Verwendung der Verbindung nach Anspruch 37, wobei die Krankheit oder der Zustand eine Krankheit oder ein Zustand ist, die bzw. der mit Serumcholesterinspiegeln zusammenhängt.

41. Verwendung der Verbindung nach Anspruch 37, wobei die Krankheit oder der Zustand Typ 11-Diabetes ist.

42. Verwendung der Verbindung nach Anspruch 37, wobei die Krankheit oder der Zustand Fettleibigkeit ist.

43. Verwendung der Verbindung nach Anspruch 37, wobei die Krankheit oder der Zustand Dyslipidämie ist.

44. Verwendung der Verbindung nach Anspruch 37, wobei die Krankheit oder der Zustand das metabolische Syndrom ist.

45. Verbindung der Formel (I): 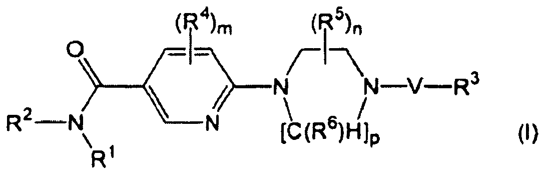 worin:
m ist 1, 2 oder 3;
n ist 1, 2, 3 oder 4;
p ist 2, 3 oder 4;
V ist -C(O)-, -S(O)- oder -S(O)₂-;
R¹ ist Wasserstoff, Alkyl, Alkenyl, Aryl, Aralkyl, Aralkenyl oder Cycloalkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, Aralkyl, wobei der Arylrest gegebenenfalls substituiert sein kann, Aralkenyl, wobei der Arylrest gegebenenfalls substituiert sein kann, gegebenenfalls substituiertem Cycloalkyl, gegebenenfalls substituiertem Cycloalkylalkyl, Cycloalkylalkenyl, gegebenenfalls substituiertem Heterocyclyl, Heterocyclylalkyl, wobei der Heterocyclylrest gegebenenfalls substituiert sein kann, Heterocyclylalkenyl, gegebenenfalls substituiertem Heteroaryl, Heteroarylalkyl, wobei der Heteroarylrest gegebenenfalls substituiert sein kann, und Heteroarylalkenyl, wobei der Heteroarylrest gegebenenfalls substituiert sein kann;
R³ ist ausgewählt aus der Gruppe bestehend aus Wasserstoff, -R⁹-OR⁸, -R⁹-N(R⁸)₂, Alkyl, Alkenyl, gegebenenfalls substituiertem Aryl, Aralkyl, wobei der Arylrest gegebenenfalls substituiert sein kann, Aralkenyl, wobei der Arylrest gegebenenfalls substituiert sein kann, gegebenenfalls substituiertem Cycloalkyl, gegebenenfalls substituiertem Cycloalkylalkyl, Cycloalkylalkenyl, gegebenenfalls substituiertem Heterocyclyl, Heterocyclylalkyl, wobei der Heterocyclylrest gegebenenfalls substituiert sein kann, Heterocyclylalkenyl, gegebenenfalls substituiertem Heteroaryl, Heteroarylalkyl, wobei der Heteroarylrest gegebenenfalls substituiert sein kann, und Heteroarylafkenyl, wobei der Heteroarylrest gegebenenfalls substituiert sein kann;
jedes R⁴ ist unabhängig Wasserstoff, Alkyl, Alkenyl, Halogen, Halogenalkyl, Aryl, Cyano, Nitro, -R⁹-OR⁸, -R⁹-N(R⁸)₂ oder -S(O)ₜR¹⁰ (wobei t 0, 1 oder 2 ist);
jedes R⁵ und R⁶ ist unabhängig Wasserstoff, Oxo, Alkyl, Alkenyl, Halogen, Halogenalkyl oder Aryl;
oder ein R⁵ und ein R⁶ können zusammen eine geradkettige oder verzweigte Alkylenbrücke bilden;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
jedes R⁹ ist unabhängig eine direkte Bindung oder eine gerade oder verzweigte Alkylen- oder Alkenylenkette; und
R¹⁰ ist Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
als ein einzelnes Stereoisomer, eine Mischung von Stereoisomeren, eine racemische Mischung davon von Stereoisomeren, oder als ein Tautomer;
oder als ein pharmazeutisch verträgliches Salz, Solvat, Polymorph oder Prodrug, ausgewählt aus Ester- und Amidderivaten von funktionellen Hydroxyl-, Carboxy-, Mercapto- oder Amino-Gruppen in den Verbindungen der Formel (I), davon zur Verwendung bei der Behandlung einer bzw. eines durch Stearyl-CoA-Desaturase (SCD) vermittelten Krankheit oder Zustands in einem Säuger, wobei die Krankheit oder der Zustand ausgewählt ist aus der Gruppe bestehend aus Typ II-Diabetes, gestörter Glukosetoleranz, Insulinresistenz, Hypertension, Fettleibigkeit, Hypertriglyceridämie, niedrigem HDL, Lipidämie, Dyslipidämie, Mikroalbuminämie, Hyperurikämie, Hyperleptinämie, dem metabolischen Syndrom und einer beliebigen Kombination von diesen; und
wobei es sich bei dem gegebenenfalls vorhandenen Substituenten um einen oder mehrere Substituenten handelt, die unabhängig ausgewählt sind aus der Gruppe bestehend aus Alkyl, Alkenyl, Halogen, Halogenalkyl, Halogenalkenyl, Cyano, Nitro, Aryl, Aralkyl, Cycloalkyl, Cycloalkylalkyl, Heterocyclyl, Heterocyclylalkyl, Heteroaryl, Heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (wobei t 1 oder 2 ist), -R⁹-S(O)ₜOR¹⁰ (wobei t 1 oder 2 ist), -R⁹-S(O)ₜR¹⁰ (wobei t 0, 1 oder 2 ist), und -R⁹-S(O)ₜN(R⁸)₂ (wobei t 1 oder 2 ist).

46. Verbindung zur Verwendung wie in Anspruch 45, wobei die Verbindung der Formel (I) eine Verbindung ist, worin
m ist 1 oder 2;
n ist 1 oder 2;
p ist 2 oder 3;
V ist -C(O)- oder -S(O)₂-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist Alkyl, Alkenyl oder -R⁹-N(R⁸)₂;
jedes R⁴ ist unabhängig Wasserstoff, Alkyl, Alkenyl, Halogen, Halogenalkyl, Aryl oder -R⁹-OR⁸;
jedes R⁵ und R⁶ ist unabhängig Wasserstoff, Oxo, Alkyl, Alkenyl, Halogen, Halogenalkyl oder Aryl;
oder ein R⁵ und ein R⁶ können zusammen eine geradkettige oder verzweigte Alkylenbrücke bilden;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
jedes R⁹ ist unabhängig eine direkte Bindung oder eine gerade oder verzweigte Alkylenkette; und
R¹⁰ ist Alkyl, Aryl oder Aralkyl.

47. Verbindung zur Verwendung wie in Anspruch 46, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Cycloalkyl, gegebenenfalls substituiertem Cycloalkylalkyl oder Cycloalkylalkenyl;
R³ ist Alkyl;
jedes R⁴ ist unabhängig Wasserstoff, Alkyl, Halogen oder Halogenalkyl;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl
und Aralkyl; und
R¹⁰ ist Alkyl, Aryl oder Aralkyl.

48. Verbindung zur Verwendung wie in Anspruch 47, wobei die Verbindung der Formel (I) eine Verbindung ist, worin:
m ist 1;
n ist 1;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist) oder Alkyl;
R³ ist Alkyl;
R⁴ ist Wasserstoff;
R⁵ ist Wasserstoff;
jedes R⁶ ist Wasserstoff;
R⁷ ist eine gerade oder verzweigte Alkylenkette;
R⁸ ist Wasserstoff oder Alkyl; und
R¹⁰ ist Alkyl, Aryl oder Aralkyl.

49. Verbindung zur Verwendung wie in Anspruch 48, wobei die Verbindung der Formel (I) ausgewählt ist aus der Gruppe bestehend aus den folgenden:
*N*-(3-Ethoxy-propyl)-6-[4-(2-ethylbutyryl)-piperazin-1-yl]-nicotinamid;
*N*-(3-Ethoxy-propyl)-6-(4-pentanoyl-piperazin-1-yl)-nicotinamid;
*N*-(3-Ethoxy-propyl)-6-[4-(3-methyl-pentanoyl)-piperazin-1-yl]-nicotinamid;
*N*-(3-Butoxy-propyl)-6-(4-pentanoyl-piperazin-1-yl)-nicotinamid;
*N*-(3-Methyl-butyl)-6-[4-(3-methyl-pentanoyl)-piperazin-1-yl]-nicotinamid;
6-[4-(3-Methyl-pentanoyl)-piperazin-1-yl]-*N*-pentyl-nicotinamid;
*N*-Butyl-6-[4-(3-methyl-pentanoyl)-piperazin-1-yl]-nicotinamid;
*N*-Butyl-6-[4-(2-ethylbutyryl)-piperazin-1-yl]-nicotinamid;
*N*-(3-Methyl-butyl)-6-(4-pentanoyl-piperazin-1-yl)-nicotinamid;
*N*-(2-Ethylsulfanyl-ethyl)-6-(4-pentanoyl-piperazin-1-yl)-nicotinamid;
*N*-(3-Methoxy-propyl)-6-[4-(3-methyl-pentanoyl)-piperazin-1-yl]-nicotinamid;
6-(4-Pentanoyl-piperazin-1-yl)-*N*-pentyl-nicotinamid;
*N*-Hexyl-6-[4-(3-methyl-pentanoyl)-piperazin-1-yl]-nicotinamid;
*N*-(1,3-Dimethylbutyl)-6-[4-(3-methyl-pentanoyl)-piperazin-1-yl]-nicotinamid;
*N*-(1-Methyl-butyl)-6-(4-pentanoyl-piperazin-1-yl)-nicotinamid;
*N*-(3-Dimethylamino-propyl)-6-[4-(2-ethylbutyryl)-piperazin-1-yl]-nicotinamid;
*N*-(3-Methoxy-propyl)-6-(4-pentanoyl-piperazin-1-yl)-nicotinamid;
*N*-(1,3-Dimethylbutyl)-6-(4-pentanoyl-piperazin-1-yl)-nicotinamid;
*N*-(2-Methyl-butyl)-6-(4-pentanoyl-piperazin-1-yl)-nicotinamid;
*N*-(3-Butoxy-propyl)-6-[4-(2-ethylbutyryl)-piperazin-1-yl]-nicotinamid;
6-[4-(2-Ethylbutyryl)-piperazin-1-yl]-*N*-(3-methylbutyl)-nicotinamid;
*N*-Butyl-6-(4-pentanoyl-piperazin-1-yl)-nicotinamid;
6-[4-(2-Ethylbutyryl)-piperazin-1-yl]-*N*-(2-methylbutyl)-nicotinamid;
*N*-(3-Isopropoxy-propyl)-6-(4-pentanoyl-piperazin-1-yl)-nicotinamid;
*N*-(2-Ethylsulfanyl-ethyl)-6-[4-(3-methyl-pentanoyl)-piperazin-1-yl]-nicotinamid;
6-[4-(2-Ethylbutyryl)-piperazin-1-yl]-*N*-(2-ethylsulfanyl-ethyl)-nicotinamid;
6-[4-(2-Ethylbutyryl)-piperazin-1-yl]-*N*-hexyl-nicotinamid;
6-[4-(2-Ethylbutyryl)-piperazin-1-yl]-*N*-(1-methylbutyl)-nicotinamid;
*N*-(2-Methyl-butyl)-6-[4-(3-methyl-pentanoyl)-piperazin-1-yl]-nicotinamid;
*N*-(1,3-Dimethylbutyl)-6-[4-(2-ethylbutyryl)-piperazin-1-yl]-nicotinamid;
6-[4-(2-Ethylbutyryl)-piperazin-1-yl]-*N*-pentyl-nicotinamid;
*N*-(3-Isopropoxy-propyl)-6-[4-(3-methyl-pentanoyl)-piperazin-1-yl]-nicotinamid;
*N*-(3-Dimethylamino-propyl)-6-[4-(3-methyl-pentanoyl)-piperazin-1-yl]-nicotinamid;
6-[4-(2-Ethylbutyryl)-piperazin-1-yl]-*N*-(3-methoxy-propyl)-nicotinamid;
6-[4-(2-Ethylbutyryl)-piperazin-1-yl]-*N*-(3-isopropoxy-propyl)-nicotinamid;
*N*-(3-Butoxy-propyl)-6-[4-(3-methyl-pentanoyl)-piperazin-1-yl]-nicotinamid;
*N*-(1-Methyl-butyl)-6-[4-(3-methyl-pentanoyl)-piperazin-1-yl]-nicotinamid;
*N*-Hexyl-6-(4-pentanoyl-piperazin-1-yl)-nicotinamid; und
*N*-(3-Dimethylamino-propyl)-6-(4-pentanoyl-piperazin-1-yl)-nicotinamid.

50. Verbindung zur Verwendung wie in Anspruch 46, wobei die Verbindung der Formel (I) eine Verbindung ist, worin
m ist 1 oder 2;
n ist 1 oder 2;
p ist 2 oder 3;
V ist -C(O)- oder -S(O)₂-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist Alkyl oder -R⁷-N(R⁸)₂;
jedes R⁴ ist unabhängig Wasserstoff, Alkyl, Halogen oder Halogenalkyl; und
jedes R⁵ und R⁶ ist unabhängig Wasserstoff, Oxo, Alkyl, Alkenyl, Halogen, Halogenalkyl oder Aryl;
oder ein R⁵ und ein R⁶ können zusammen eine geradkettige oder verzweigte Alkylenbrücke bilden;
R⁷ ist eine direkte Bindung; und
jedes R⁸ ist unabhängig Wasserstoff oder Alkyl.

51. Verbindung zur Verwendung wie in Anspruch 50, wobei die Verbindung der Formel (I) eine Verbindung ist, worin:
m ist 1;
nist1;
p ist 2 oder 3;
V ist -C(O)- oder -S(O)₂-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Heterocyclyl, gegebenenfalls substituiertem Heterocyclylalkyl, gegebenenfalls substituiertem Heteroaryl und
gegebenenfalls substituiertem Heteroarylalkyl;
R³ ist Alkyl oder -R⁷-N(R⁸)₂;
R⁴ ist Wasserstoff, Alkyl, Halogen oder Halogenalkyl; und
jedes R⁵ und R⁶ ist unabhängig Wasserstoff, Oxo, Alkyl, Halogen oder Halogenalkyl;
oder ein R⁵ und ein R⁶ können zusammen eine Methylenbrücke bilden;
R⁷ ist eine direkte Bindung; und
jedes R⁸ ist unabhängig Wasserstoff oder Alkyl.

52. Verbindung zur Verwendung wie in Anspruch 51, wobei die Verbindung der Formel (I) ausgewählt ist aus der Gruppe bestehend aus den folgenden:
6-[4-(2-Ethylbutyryl)-piperazin-1-yl]-*N*-(3-phenyl-propyl)-nicotinamid;
6-[4-(4-Methyl-hexanoyl)-piperazin-1-yl]-*N*-(3-phenyl-propyl)-nicotinamid;
*N*-[2-(3-Chlorphenyl)-1-methylethyl]-6-[4-(2-ethylbutyryl)-piperazin-1-yl)-nicotinamid;
*N*-[2-(3-Chlorphenyl)-ethyl]-6-[4-(3-methyl-pentanoyl)-piperazin-1-yl]-nicotinamid;
*N*-[2-(3-Chlorphenyl)-ethyl]-6-[4-(2-ethylbutyryl)-piperazin-1-yl]-nicotinamid;
6-(4-Pentanoyl-piperazin-1-yl)-*N*-(4-phenyl-butyl)-nicotinamid;
*N*-[2-(3-Chlorphenyl)-ethyl]-6-(4-pentanoyl-piperazin-1-yl)-nicotinamid;
6-[4-(2-Ethylbutyryl)-piperazin-1-yl]-*N*-phenethyl-nicotinamid;
6-[4-(3-Methyl-pentanoyl)-piperazin-1-yl]-*N*-phenethyl-nicotinamid;
6-(4-Pentanoyl-piperazin-1-yl)-*N*-(3-phenyl-propyl)nicotinamid;
*N*-(1-Methyl-3-phenyl-propyl)-6-(4-pentanoyl-piperazin-1-yl)-nicotinamid;
6-[4-(3-Methyl-pentanoyl)-piperazin-1-yl]-*N*-(tetrahydro-furan-2-ylmethyl)-nicotinamid;
6-[4-(2-Ethylbutyryl)-piperazin-1-yl]-*N*-(tetrahydro-furan-2-ylmethyl)-nicotinamid;
6-(4-Pentanoyl-piperazin-1-yl)-*N*-(tetrahydro-furan-2-ylmethyl)-nicotinamid;
6-[4-(2-Ethylbutyryl)-piperazin-1-yl]-*N*-(4-phenyl-butyl)-nicotinamid;
*N*-(3-Imidazol-1-yl-propyl)-6-(4-pentanoyl-piperazin-1-yl)-nicotinamid;
6-[4-(2-Ethylbutyryl)-piperazin-1-yl]-*N*-(3-imidazol-1-yl-propyl)-nicotinamid;
*N*-[2-(3H-Imidazol-4-yl)-ethyl]-6-(4-pentanoyl-piperazin-1-yl)-nicotinamid;
*N*-[2-(3H-Imidazol-4-yl)-ethyl]-6-[4-(3-methyl-pentanoyl)-piperazin-1-yl]-nicotinamid;
6-(4-Pentanoyl-piperazin-1-yl)-*N*-phenethyl-nicotinamid;
6-[4-(2-Ethylbutyryl)-piperazin-1-yl]-*N*-[2-(3H-imidazol-4-yl)-ethyl]-nicotinamid;
*N*-(3-Imidazol-1-yl-propyl)-6-[4-(3-methyl-pentanoyl)-piperazin-1-yl]-nicotinamid;
6-[4-(2-Ethylbutyryl)-[1,4]diazepan-1-yl]-*N*-(3-phenyl-propyl)-nicotinamid;
6-[4-(2-Ethylbutyryl)-3-methyl-piperazin-1-yl]-*N*-(3-phenyl-propyl)-nicotinamid;
6-[5-(2-Ethylbutyryl)-2,5-diaza-bicyclo[2.2.1]hept-2-yl]-*N*-(3-phenyl-propyl)-nicotinamid;
6-[4-(Butan-1-sulfonyl)-piperazin-1-yl]-*N*-(3-phenyl-propyl)-nicotinamid;
4-[5-(3-Phenyl-propylcarbamoyl)-pyridin-2-yl]-piperazin-1-carbonsäure-butylamid.

53. Verbindung zur Verwendung wie in Anspruch 45, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Cycloalkyl, gegebenenfalls substituiertes Cycloalkylalkyl oder Cycloalkylalkenyl;
jedes R⁴ ist unabhängig Wasserstoff, Alkyl, Alkenyl, Halogen, Halogenalkyl, Aryl oder -R⁹ -OR⁸;
jedes R⁵ und R⁶ ist unabhängig Wasserstoff, Oxo, Alkyl, Alkenyl, Halogen, Halogenalkyl oder Aryl;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
R⁹ ist eine direkte Bindung oder eine gerade oder verzweigte Alkylenkette; und
R¹⁰ ist Alkyl, Aryl oder Aralkyl.

54. Verbindung zur Verwendung wie in Anspruch 53, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Cycloalkyl, gegebenenfalls substituiertem Cycloalkylalkyl oder Cycloalkylalkenyl;
R³ ist gegebenenfalls substituiertes Cycloalkyl oder gegebenenfalls substituiertes Cycloalkylalkyl;
jedes R⁴ ist unabhängig Wasserstoff, Alkyl, Halogen, oder Halogenalkyl;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl
und Aralkyl; und
R¹⁰ ist Alkyl, Aryl oder Aralkyl.

55. Verbindung zur Verwendung wie in Anspruch 54, wobei die Verbindung der Formel (I) eine Verbindung ist, worin:
m ist 1;
n ist 1;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0 bis 2 ist) oder Alkyl;
R³ ist gegebenenfalls substituiertes Cycloalkyl oder gegebenenfalls substituiertes Cycloalkylalkyl;
R⁴ ist Wasserstoff;
R⁵ ist Wasserstoff;
jedes R⁶ ist Wasserstoff;
R⁷ ist eine gerade oder verzweigte Alkylenkette;
R⁸ ist Wasserstoff oder Alkyl; und
R¹⁰ ist Alkyl, Aryl oder Aralkyl.

56. Verbindung zur Verwendung wie in Anspruch 55, wobei die Verbindung der Formel (I) ausgewählt ist aus der Gruppe bestehend aus den folgenden:
6-[4-(2-Cyclohexyl-acetyl)-piperazin-1-yl)-*N*-(3-ethoxy-propyl)-nicotinamid;
*N*-Butyl-6-[4-(2-cyclohexyl-acetyl)-piperazin-1-yl]-nicotinamid;
6-[4-(3-Cyclohexyl-propionyl)-piperazin-1-yl]-*N*-(3-methylbutyl)-nicotinamid;
6-[4-(3-Cyclohexyl-propionyl)-piperazin-1-yl]-*N*-(3-methoxy-propyl)-nicotinamid;
*N*-Pentyl-6-[4-(3-trifluormethyl-benzoyl)-piperazin-1-yl)-nicotinamid;
6-[4-(2-Cyclohexyl-acetyl)-piperazin-1-yl]-*N*-(2-methylbutyl)-nicotinamid;
6-[4-(3-Cyclohexyl-propionyl)-piperazin-1-yl]-*N*-(3-isopropoxy-propyl)-nicotinamid;
6-[4-(2-Cyclohexyl-acetyl)-piperazin-1-yl]-*N*-(1,3-dimethylbutyl)-nicotinamid;
6-[4-(2-Cyclohexyl-acetyl)-piperazin-1-yl]-*N*-hexyl-nicotinamid;
6-[4-(2-Cyclohexyl-acetyl)-piperazin-1-yl)-*N*-(1-methylbutyl)-nicotinamid;
6-[4-(2-Cyclohexyl-acetyl)-piperazin-1-yl]-*N*-(3-methylbutyl)-nicotinamid;
6-[4-(2-Cyclohexyl-acetyl)-piperazin-1-yl]-*N*-(2-ethylsulfanyl-ethyl)-nicotinamid;
*N*-(3-Butoxy-propyl)-6-[4-(2-cyclohexyl-acetyl)-piperazin-1-yl]-nicotinamid;
*N*-Butyl-6-[4-(3-Cyclohexyl-propionyl)-piperazin-1-yl]-nicotinamid;
6-[4-(2-Cyclohexyl-acetyl)-piperazin-1-yl]-*N*-pentyl-nicotinamid;
6-[4-(3-Cyclohexyl-propionyl)-piperazin-1-yl]-*N*-(2-ethylsulfanyl-ethyl)-nicotinamid;
6-[4-(2-Cyclohexyl-acetyl)-piperazin-1-yl]-*N*-(3-methoxy-propyl)-nicotinamid;
6-[4-(3-Cyclohexyl-propionyl)-piperazin-1-yl]-*N*-(1-methylbutyl)-nicotinamid;
6-[4-(2-Cyclohexyl-acetyl)-piperazin-1-yl]-*N*-(3-isopropoxy-propyl)-nicotinamid;
6-[4-(3-Cyclohexyl-propionyl)-piperazin-1-yl]-*N*-(3-ethoxy-propyl)-nicotinamid;
6-[4-(3-Cyclohexyl-propionyl)-piperazin-1-yl]-*N*-(1,3-dimethylbutyl)-nicotinamid;
6-[4-(3-Cyclohexyl-propionyl)-piperazin-1-yl]-*N*-(2-methylbutyl)-nicotinamid;
6-[4-(3-Cyclohexyl-propionyl)-piperazin-1-yl]-*N*-hexyl-nicotinamid;
6-[4-(3-Cyclohexyl-propionyl)-piperazin-1-yl]-*N*-pentyl-nicotinamid;
6-[4-(3-Cyclohexyl-propionyl)-piperazin-1-yl]-*N*-(3-dimethylamino-propyl)-nicotinamid;
*N*-(3-Ethoxy-propyl)-6-[4-(2-phenyl-cyclopropancarbonyl)-piperazin-1-yl]-nicotinamid;
*N*-(2-Ethylsulfanyl-ethyl)-6-[4-(2-phenyl-cyclopropancarbonyl)-piperazin-1-yl]-nicotinamid;
*N*-(1,3-Dimethylbutyl)-6-[4-(2-phenyl-cyclopropancarbonyl)-piperazin-1-yl]-nicotinamid;
*N*-(3-Methyl-butyl)-6-[4-(2-phenyl-cyclopropancarbonyl)-piperazin-1-yl]-nicotinamid;
*N*-(3-Methoxy-propyl)-6-[4-(2-phenyl-cyclopropancarbonyl)-piperazin-1-yl]-nicotinamid;
*N*-(3-Butoxy-propyl)-6-[4-(2-phenyl-cyclopropancarbonyl)-piperazin-1-yl]-nicotinamid;
*N*-Pentyl-6-[4-(2-phenyl-cyclopropancarbonyl)-piperazin-1-yl]-nicotinamid;
*N*-(3-Dimethylamino-propyl)-6-[4-(2-phenyl-cyclopropancarbonyl)-piperazin-1-yl]-nicotinamid;
*N*-(3-Isopropoxy-propyl)-6-[4-(2-phenyl-cyclopropancarbonyl)-piperazin-1-yl]-nicotinamid;
*N*-(1-Methyl-butyl)-6-[4-(2-phenyl-cyclopropancarbonyl)-piperazin-1-yl]-nicotinamid;
*N*-Butyl-6-[4-(2-phenyl-cyclopropancarbonyl)-piperazin-1-yl]-nicotinamid;
*N*-Hexyl-6-[4-(2-phenyl-cyclopropancarbonyl)-piperazin-1-yl]-nicotinamid;
N-(2-Methyl-butyl)-6-[4-(2-phenyl-cyclopropancarbonyl)-piperazin-1-yl]-nicotinamid; und
6-[4-(2-Cyclohexyl-acetyl)-piperazin-1-yl]-N-(3-dimethylamino-propyl)-nicotinamid.

57. Verbindung zur Verwendung wie in Anspruch 53, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Cycloalkyl oder gegebenenfalls substituiertes Cycloalkylalkyl;
jedes R⁴ ist unabhängig Wasserstoff, Alkyl, Halogen oder Halogenalkyl; und
jedes R⁵ und R⁶ ist unabhängig Wasserstoff, Oxo, Alkyl, Halogen oder Halogenalkyl.

58. Verbindung zur Verwendung wie in Anspruch 57, wobei die Verbindung der Formel (I) eine Verbindung ist, worin:
m ist 1:
n ist 1;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Heterocyclyl, gegebenenfalls substituiertem Heterocyclylalkyl, gegebenenfalls substituiertem Heteroaryl und gegebenenfalls substituiertem Heteroarylalkyl;
R³ ist gegebenenfalls substituiertes Cycloalkyl oder gegebenenfalls substituiertes Cycloalkylalkyl;
R⁴ ist Wasserstoff, Alkyl, Halogen oder Halogenalkyl;
R⁵ ist unabhängig Wasserstoff, Oxo, Alkyl, Halogen oder Halogenalkyl; und
jedes R⁶ ist unabhängig Wasserstoff, Oxo, Alkyl, Halogen oder Halogenalkyl.

59. Verbindung zur Verwendung wie in Anspruch 58, wobei die Verbindung der Formel (I) ausgewählt ist aus der Gruppe bestehend aus den folgenden:
6-[4-(2-Phenyl-cyclopropancarbonyl)-piperazin-1-yl]-*N*-(3-phenyl-propyl)-nicotinamid;
*N*-(4-Phenyl-butyl)-6-(4-(2-phenyl-cyclopropancarbonyl)-piperazin-1-yl]-nicotinamid;
6-[4-(2-Cyclohexyl-acetyl)-piperazin-1-yl]-*N*-phenethyl-nicotinamid;
*N*-[2-(3-Chlorphenyl)-ethyl]-6-[4-(2-phenyl-cyclopropancarbonyl)-piperazin-1-yl]-nicotinamid;
*N*-(1-Methyl-3-phenyl-propyl)-6-[4-(2-phenyl-cyclopropancarbonyl)-piperazin-1-yl]-nicotinamid;
*N*-Phenethyl-6-[4-(2-phenyl-cyclopropancarbonyl)-piperazin-1-yl]-nicotinamid;
6-[4-(2-Phenyl-cyclopropancarbonyl)-piperazin-1-yl]-*N*-(tetrahydro-furan-2-ylmethyl)-nicotinamid;
*N*-[2-(3H-Imidazol-4-yl)-ethyl]-6-[4-(2-phenyl-cyclopropancarbonyl)-piperazin-1-yl]-nicotinamid;
*N*-(3-Imidazol-1-yl-propyl)-6-[4-(2-phenyl-cyclopropancarbonyl)-piperazin-1-yl]-nicotinamid;
*N*-[2-(3-Chlorphenyl)-ethyl]-6-[4-(3-cyclohexyl-propionyl)-piperazin-1-yl]-nicotinamid;
6-[4-(2-Cyclohexyl-acetyl)-piperazin-1-yl]-*N*-[2-(3H-imidazol-4-yl)-ethyl]-nicotinamid;
6-[4-(2-Cyclohexyl-acetyl)-piperazin-1-yl]-*N*-(3-phenyl-propyl)-nicotinamid;
*N*-[2-(3-Chlorphenyl)-ethyl]-6-[4-(2-cyclohexyl-acetyl)-piperazin-1-yl]-nicotinamid;
6-[4-(3-Cyclohexyl-propionyl)-piperazin-1-yl]-*N*-phenethyl-nicotinamid;
6-[4-(3-Cyclohexyl-propionyl)-piperazin-1-yl]-*N*-(3-phenyl-propyl)-nicotinamid;
6-[4-(3-Methyl-pentanoyl)-piperazin-1-yl]-*N*-(4-phenyl-butyl)-nicotinamid;
6-[4-(2-Cyclohexyl-acetyl)-piperazin-1-yl]-*N*-(4-phenyl-butyl)-nicotinamid;
6-[4-(2-Cyclohexyl-acetyl)-piperazin-1-yl]-*N*-(1-methyl-3-phenyl-propyl)-nicotinamid;
6-[4-(3-Cyclohexyl-propionyl)-piperazin-1-yl]-*N*-[2-(3H-imidazol-4-yl)-ethyl]-nicotinamid;
6-[4-(3-Cyclohexyl-propionyl)-piperazin-1-yl]-*N*-(tetrahydro-furan-2-ylmethyl)-nicotinamid;
6-[4-(2-Cyclohexyl-acetyl)-piperazin-1-yl]-*N*-(3-imidazol-1-yl-propyl)-nicotinamid;
6-[4-(3-Cyclohexyl-propionyl)-piperazin-1-yl]-*N*-(3-imidazol-1-yl-propyl)-nicotinamid; und
6-[4-(2-Cyclohexyl-acetyl)-piperazin-1-yl]-*N*-(tetrahydro-furan-2-ylmethyl)-nicotinamid.

60. Verbindung zur Verwendung wie in Anspruch 45, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Aryl;
jedes R⁴ ist unabhängig Wasserstoff, Alkyl, Alkenyl, Halogen, Halogenalkyl, Aryl oder -R⁹-OR⁸;
jedes R⁵ und R⁶ ist unabhängig Wasserstoff, Oxo, Alkyl, Alkenyl, Halogen, Halogenalkyl oder Aryl;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
R⁹ ist eine direkte Bindung oder eine gerade oder verzweigte Alkylenkette; und
R¹⁰ ist Alkyl, Aryl oder Aralkyl.

61. Verbindung zur Verwendung wie in Anspruch 60, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0 bis 2 ist), Alkyl, Alkenyl, gegebenenfalls substituiertem Cycloalkyl, gegebenenfalls substituiertem Cycloalkylalkyl oder Cycloalkylalkenyl;
R³ ist gegebenenfalls substituiertes Aryl;
jedes R⁴ ist unabhängig Wasserstoff, Alkyl, Halogen, Halogenalkyl oder -R⁹-OR⁸;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl und Aralkyl;
R⁹ ist eine direkte Bindung oder eine gerade oder verzweigte Alkylenkette; und
R¹⁰ ist Alkyl, Aryl oder Aralkyl.

62. Verbindung zur Verwendung wie in Anspruch 61, wobei die Verbindung der Formel (I) eine Verbindung ist, worin:
m ist 1 oder 2;
n ist 1;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, gegebenenfalls substituiertem Cycloalkyl oder gegebenenfalls substituiertem Cycloalkylalkyl;
R³ ist gegebenenfalls substituiertes Aryl;
jedes R⁴ ist unabhängig Wasserstoff, Halogen, Halogenalkyl oder -R⁹-OR⁸;
R⁵ ist Wasserstoff;
jedes R⁶ ist Wasserstoff;
R⁷ ist eine gerade oder verzweigte Alkylenkette;
R⁸ ist Wasserstoff oder Alkyl;
R⁹ ist eine direkte Bindung oder eine gerade oder verzweigte Alkylenkette; und
R¹⁰ ist Alkyl, Aryl oder Aralkyl.

63. Verbindung zur Verwendung wie in Anspruch 62, wobei die Verbindung der Formel (I) ausgewählt ist aus der Gruppe bestehend aus den folgenden:
*N*-Butyl-6-[4-(2-mercapto-benzoyl)-piperazin-1-yl]-nicotinamid;
*N*-(3-Methyl-butyl)-6-[4-(2-o-tolyl-acetyl)-piperazin-1-yl]-nicotinamid;
6-{4-[2-(2,4-Dimethyl-phenyl)-acetyl]-piperazin-1-yl}-*N*-(3-methylbutyl)-nicotinamid;
6-[4-(2-Brom-benzoyl)-piperazin-1-yl]-N-(3-ethoxypropyl)nicotinamid;
6-{4-[2-(2-Chlor-6-fluor-phenyl)-acetyl]-piperazin-1-yl}-*N*-(3-methylbutyl)-nicotinamid;
6-[4-(3,5-Dichlor-benzoyl)-piperazin-1-yl]-N-(3-methoxy-propyl)-nicotinamid;
6-[4-(2-Brom-5-methoxy-benzoyl)-piperazin-1-yl]-*N*-(3-dimethylamino-propyl)-nicotinamid;
6-[4-(2,5-Dichlor-benzoyl)-piperazin-1-yl]-*N*-(3-methylbutyl)-nicotinamid;
6-[4-(2,5-Dichlor-benzoyl)-piperazin-1-yl]-*N*-(3-ethoxy-propyl)-nicotinamid;
*N*-Butyl-6-[4-(3,5-dichlor-benzoyl)-piperazin-1-yl]-nicotinamid;
6-[4-(2,4-Dichlor-benzoyl)-piperazin-1-yl]-*N*-(3-ethoxy-propyl)-nicotinamid;
6-[4-(2,4-Dichlor-benzoyl)-piperazin-1-yl]-*N*-(2-ethylsulfanyl-ethyl)-nicotinamid;
*N*-(3-Ethoxy-propyl)-6-[4-(4-trifluormethyl-benzoyl)-piperazin-1-yl]-nicotinamid;
*N*-(3-Dimethylamino-propyl)-6-[4-(2,3,4,5-tetrafluor-benzoyl)-piperazin-1-yl]-nicotinamid;
6-[4-(2-Brom-5-methoxy-benzoyl)-piperazin-1-yl]-*N*-(2-ethylsulfanyl-ethyl)-nicotinamid;
*N*-(2-Ethylsulfanyl-ethyl)-6-[4-(3-trifluormethyl-benzoyl)-piperazin-1-yl]-nicotinamid;
6-[4-(2-Brom-benzoyl)-piperazin-1-yl]-*N*-(3-methylbutyl)-nicotinamid;
*N*-Butyl-6-[4-(2,4-dimethyl-benzoyl)-piperazin-1-yl]-nicotinamid;
6-[4-(3,5-Dichlor-benzoyl)-piperazin-1-yl]-*N*-(3-isopropoxy-propyl)-nicotinamid;
6-[4-(2-Brom-5-methoxy-benzoyl)-piperazin-1-yl]-*N*-(3-methylbutyl)-nicotinamid;
*N*-(3-Butoxy-propyl)-6-[4-(2,5-dichlor-benzoyl)-piperazin-1-yl]-nicotinamid;
*N*-(3-Methyl-butyt)-6-[4-(3-trifluormethyl-benzoyl)-piperazin-1-yl]-nicotinamid;
*N*-(3-Methyl-butyl)-6-[4-(2-trifluormethyl-benzoyl)-piperazin-1-yl]-nicotinamid;
6-[4-(2-Brom-benzoyl)-piperazin-1-yl]-*N*-(3-butoxy-propyl)-nicotinamid;
6-[4-(2,5-Dichlor-benzoyl)-piperazin-1-yl]-*N*-pentyl-nicotinamid;
*N*-(3-Butoxy-propyl)-6-[4-(2,4-dimethyl-benzoyl)-piperazin-1-yl]-nicotinamid;
6-[4-(2-Brom-5-methoxy-benzoyl)-piperazin-1-yl]-*N*-(3-ethoxy-propyl)-nicotinamid;
6-[4-(2,4-Dichlor-benzoyl)-piperazin-1-yl]-*N*-(3-isopropoxy-propyl)-nicotinamid;
*N*-(3-Ethoxy-propyl)-6-[4-(2-trifluormethyl-benzoyl)-piperazin-1-yl]-nicotinamid;
*N*-Pentyl-6-[4-(3-trifluormethyl-benzoyl)-piperazin-1-yl]-nicotinamid;
*N*-Butyl-6-[4-(2-trifluormethyl-benzoyl)-piperazin-1-yl]-nicotinamid;
*N*-Butyl-6-[4-(2,5-dichlor-benzoyl)-piperazin-1-yl]-nicotinamid;
6-[4-(2-Brom-benzoyl)-piperazin-1-yl]-*N*-(3-isopropoxy-propyl)-nicotinamid;
6-[4-(2,5-Dichlor-benzoyl)-piperazin-1-yl]-*N*-(3-isopropoxy-propyl)-nicotinamid;
*N-(*3-Butoxy-propyl)-6-[4-(4-trifluormethyl-benzoyl)-piperazin-1-yl]-nicotinamid;
6-[4-(2,5-Dichlor-benzoyl)-piperazin-1-yl]-*N*-(2-ethylsulfanyl-ethyl)-nicotinamid;
*N*-(3-Butoxy-propyl)-6-[4-(2-trifluormethyl-benzoyl)-piperazin-1-yl]-nicotinamid;
6-[4-(2,5-Dichlor-benzoyl)-piperazin-1-yl]-*N*-(3-methoxy-propyl)-nicotinamid;
6-[4-(2,4-Dimethyl-benzoyl)-piperazin-1-yl]-*N*-(1,3-dimethylbutyl)-nicotinamid;
6-[4-(2,4-Dichlor-benzoyl)-piperazin-1-yl]-*N*-(2-methylbutyl)-nicotinamid;
6-[4-(3,5-Dichlor-benzoyl)-piperazin-1-yl]-*N*-(2-methylbutyl)-nicotinamid;
6-[4-(2,4-Dimethyl-benzoyl)-piperazin-1-yl]-*N*-(2-ethylsulfanyl-ethyl)-nicotinamid;
*N*-(1,3-Dimethylbutyl)-6-[4-(3-trifluormethyl-benzoyl)-piperazin-1-yl]-nicotinamid;
6-[4-(2-Brom-5-methoxy-benzoyl)-piperazin-1-yl]-*N*-(2-methylbutyl)-nicotinamid;
*N*-(3-Dimethylamino-propyl)-6-[4-(2-trifluormethyl-benzoyl)-piperazin-1-yl]-nicotinamid;
*N*-Hexyl-6-[4-(3-trifluormethyl-benzoyl)-piperazin-1-yl]-nicotinamid;
6-[4-(2,4-Dichlor-benzoyl)-piperazin-1-yl]-*N*-(1,3-dimethylbutyl)-nicotinamid;
*N*-(3-Isopropoxy-propyl)-6-[4-(3-trifluormethyl-benzoyl)-piperazin-1-yl]-nicotinamid;
6-[4-(2-Brom-5-methoxy-benzoyl)-piperazin-1-yl]-*N*-hexyl-nicotinamid;
6-[4-(2-Brom-benzoyl)-piperazin-1-yl]-*N*-hexyl-nicotinamid;
*N*-(3-Isopropoxy-propyl)-6-[4-(2-trifluormethyl-benzoyl)-piperazin-1-yl]-nicotinamid;
6-[4-(2-Brom-5-methoxy-benzoyl)-piperazin-1-yl]-*N*-(3-methoxy-propyl)-nicotinamid;
6-[4-(2-Brom-benzoyl)-piperazin-1-yl]-*N*-(1-methylbutyl)-nicotinamid;
6-[4-(3,5-Dichlor-benzoyl)-piperazin-1-yl]-*N*-hexyl-nicotinamid;
*N*-(3-Methoxy-propyl)-6-[4-(2-trifluormethyl-benzoyl)-piperazin-1-yl]-nicotinamid;
6-[4-(2,4-Dichlor-benzoyl)-piperazin-1-yl]-*N*-hexyl-nicotinamid;
*N*-(2-Ethylsulfanyl-ethyl)-6-[4-(2-trifluormethyl-benzoyl)-piperazin-1-yl]-nicotinamid;
*N*-Pentyl-6-[4-(2-trifluormethyl-benzoyl)-piperazin-1-yl]-nicotinamid;
*N*-(2-Methyl-butyl)-6-[4-(3-trifluormethyl-benzoyl)-piperazin-1-yl]-nicotinamid;
*N*-(2-Ethylsulfanyl-ethyl)-6-[4-(4-trifluormethyl-benzoyl)-piperazin-1-yl]-nicotinamid;
6-[4-(2,4-Dichlor-benzoyl)-piperazin-1-yl]-*N*-pentyl-nicotinamid;
6-[4-(2-Brom-benzoyl)-piperazin-1-yl]-*N*-(2-methylbutyl)-nicotinamid;
6-[4-(2-Brom-5-methoxy-benzoyl)-piperazin-1-yl]-*N*-(3-butoxy-propyl)-nicotinamid;
*N*-(1,3-Dimethylbutyl)-6-[4-(4-trifluormethyl-benzoyl)-piperazin-1-yl]-nicotinamid;
6-[4-(3,5-Dichlor-benzoyl)-piperazin-1-yl]-*N*-pentyl-nicotinamid;
*N*-(3-Butoxy-propyl)-6-[4-(3,5-dichlor-benzoyl)-piperazin-1-yl]-nicotinamid;
6-[4-(2-Brom-5-methoxy-benzoyl)-piperazin-1-yl]-*N*-butyl-nicotinamid;
6-[4-(2-Brom-benzoyl)-piperazin-1-yl]-*N*-pentyl-nicotinamid;
*N*-(1,3-Dimethylbutyl)-6-[4-(2-trifluormethyl-benzoyl)-piperazin-1-yl]-nicotinamid;
*N*-(3-Methoxy-propyl)-6-[4-(4-trifluormethyl-benzoyl)-piperazin-1-yl]-nicotinamid;
6-[4-(2,4-Dimethyl-benzoyl)-piperazin-1-yl]-*N*-(1-methylbutyl)-nicotinamid;
6-[4-(2,4-Dichlor-benzoyl)-piperazin-1-yl]-*N*-(3-methoxy-propyl)-nicotinamid;
*N*-(1-Methyl-butyl)-6-[4-(2-trifluormethyl-benzoyl)-piperazin-1-yl]-nicotinamid;
*N*-(3-Dimethylamino-propyl)-6-[4-(3-trifluormethyl-benzoyl)-piperazin-1-yl]-nicotinamid;
6-[4-(2,4-Dichlor-benzoyl)-piperazin-1-yl]-*N*-(1-methylbutyl)-nicotinamid;
6-[4-(2,5-Dichlor-benzoyl)-piperazin-1-yl]-*N*-(1-methylbutyl)-nicotinamid;
*N*-(1-Methyl-butyl)-6-[4-(3-trifluormethyl-benzoyl)-piperazin-1-yl]-nicotinamid;
6-[4-(2,5-Dichlor-benzoyl)-piperazin-1-yl]-*N*-hexyl-nicotinamid;
6-[4-(2,4-Dimethyl-benzoyl)-piperazin-1-yl]-*N*-(3-methoxy-propyl)-nicotinamid;
6-[4-(3,5-Dichlor-benzoyl)-piperazin-1-yl]-*N*-(1-methylbutyl)-nicotinamid;
6-[4-(2,4-Dichlor-benzoyl)-piperazin-1-yl]-*N*-(3-methylbutyl)-nicotinamid;
*N*-(3-Butoxy-propyl)-6-[4-(3-trifluormethyl-benzoyl)-piperazin-1-yl]-nicotinamid;
6-[4-(2,4-Dimethyl-benzoyl)-piperazin-1-yl]-*N*-(3-isopropoxy-propyl)-nicotinamid;
*N*-(1-Methyl-butyl)-6-[4-(4-trifluormethyl-benzoyl)-piperazin-1-yl]-nicotinamid;
*N*-Butyl-6-[4-(2,4-dichlor-benzoyl)-piperazin-1-yl]-nicotinamid;
6-[4-(3,5-Dichlor-benzoyl)-piperazin-1-yl]-*N*-(1,3-dimethylbutyl)-nicotinamid;
*N*-Butyl-6-[4-(3-trifluormethyl-benzoyl)-piperazin-1-yl]-nicotinamid;
6-[4-(2-Brom-5-methoxy-benzoyl)-piperazin-1-yl]-*N*-(1,3-dimethylbutyl)-nicotinamid;
6-[4-(2,4-Dimethyl-benzoyl)-piperazin-1-yl]-*N*-hexyl-nicotinamid;
6-[4-(2,5-Dichlor-benzoyl)-piperazin-1-yl]-*N*-(1,3-dimethylbutyl)-nicotinamid;
*N*-(3-Butoxy-propyl)-6-[4-(2,4-dichlor-benzoyl)-piperazin-1-yl]-nicotinamid;
*N*-(3-Methoxy-propyl)-6-[4-(3-trifluormethyl-benzoyl)-piperazin-1-yl]-nicotinamid;
6-[4-(2,4-Dimethyl-benzoyl)-piperazin-1-yl]-*N*-(3-ethoxy-propyl)-nicotinamid;
6-[4-(2,5-Dichlor-benzoyl)-piperazin-1-yl]-*N*-(2-methylbutyl)-nicotinamid;
*N*-Hexyl-6-[4-(4-trifluormethyl-benzoyl)-piperazin-1-yl]-nicotinamid;
6-[4-(2,4-Dimethyl-benzoyl)-piperazin-1-yl]-*N*-pentyl-nicotinamid;
6-[4-(2-Brom-benzoyl)-piperazin-1-yl)-*N*-(1,3-dimethylbutyl)-nicotinamid;
*N*-Hexyl-6-[4-(2-trifluormethyl-benzoyl)-piperazin-1-yl]-nicotinamid;
6-[4-(2-Brom-benzoyl)-piperazin-1-yl]-*N*-(3-methoxy-propyl)-nicotinamid;
*N*-(2-Methyl-butyl)-6-[4-(4-trifluormethyl-benzoyl)-piperazin-1-yl]-nicotinamid;
6-[4-(2-Brom-5-methoxy-benzoyl)-piperazin-1-yl]-*N*-(1-methylbutyl)-nicotinamid;
6-[4-(2,4-Dimethyl-benzoyl)-piperazin-1-yl]-*N*-(2-methylbutyl)-nicotinamid;
6-[4-(2-Brom-5-methoxy-benzoyl)-piperazin-1-yl]-*N*-pentyl-nicotinamid;
*N*-(2-Methyl-butyl)-6-[4-(2-trifluormethyl-benzoyl)-piperazin-1-yl]-nicotinamid;
6-[4-(3,5-Dichlor-benzoyl)-piperazin-1-yl]-*N*-(3-methylbutyl)-nicotinamid;
6-[4-(2-Brom-benzoyl)-piperazin-1-yl]-*N*-(2-ethylsulfanyl-ethyl)-nicotinamid;
6-[4-(3,5-Dichlor-benzoyl)-piperazin-1-yl]-*N*-(3-methoxy-propyl)-nicotinamid;
6-[4-(2-Brom-5-methoxy-benzoyl)-piperazin-1-yl]-*N*-(3-isopropoxy-propyl)-nicotinamid;
*N*-(3-Butoxy-propyl)-6-[4-(2,3,4,5-tetrafluor-benzoyl)-piperazin-1-yl]-nicotinamid;
*N*-(1,3-Dimethylbutyl)-6-[4-(2,3,4,5-tetrafluor-benzoyl)-piperazin-1-yl]-nicotinamid;
*N*-Butyl-6-[4-(2,3,4,5-tetrafluor-benzoyl)-piperazin-1-yl]-nicotinamid;
*N*-(1-Methyl-butyl)-6-[4-(2,3,4,5-tetrafluor-benzoyl)-piperazin-1-yl]-nicotinamid;
*N*-(3-Methoxy-propyl)-6-[4-(2,3,4,5-tetrafluor-benzoyl)-piperazin-1-yl]-nicotinamid;
*N*-(3-Isopropoxy-propyl)-6-[4-(2,3,4,5-tetrafluor-benzoyl)-piperazin-1-yl]-nicotinamid;
*N*-Pentyl-6-[4-(2,3,4,5-tetrafluor-benzoyl)-piperazin-1-yl]-nicotinamid;
*N*-(2-Methyl-butyl)-6-[4-(2,3,4,5-tetrafluor-benzoyl)-piperazin-1-yl]-nicotinamid;
*N*-Hexyl-6-[4-(2,3,4,5-tetrafluor-benzoyl)-piperazin-1-yl]-nicotinamid;
*N*-(3-Methyl-butyl)-6-[4-(2,3,4,5-tetrafluor-benzoyl)-piperazin-1-yl]-nicotinamid;
*N*-(2-Ethylsulfanyl-ethyl)-6-[4-(2,3,4,5-tetrafluor-benzoyl)-piperazin-1-yl]-nicotinamid;
*N*-(3-Ethoxy-propyl)-6-[4-(2,3,4,5-tetrafluor-benzoyl)-piperazin-1-yl]-nicotinamid;
*N*-(3-Dimethylamino-propyl)-6-[4-(4-trifluormethyl-benzoyl)-piperazin-1-yl]-nicotinamid;
*N*-(3-Ethoxy-propyl)-6-[4-(3-trifluormethyl-benzoyl)-piperazin-1-yl]-nicotinamid;
6-[4-(3,5-Dichlor-benzoyl)-piperazin-1-yl]-*N*-(2-ethylsulfanyl-ethyl)-nicotinamid;
*N*-(3-Methyl-butyl)-6-[4-(4-trifluormethyl-benzoyl)-piperazin-1-yl]-nicotinamid;
*N*-Butyl-6-[4-(4-trifluormethyl-benzoyl)-piperazin-1-yl]-nicotinamid;
*N*-(3-Isopropoxy-propyl)-6-[4-(4-trifluormethyl-benzoyl)-piperazin-1-yl]-nicotinamid;
*N*-Pentyl-6-[4-(4-trifluormethyl-benzoyl)-piperazin-1-yl]-nicotinamid;
6-[4-(3, 5-Dichlor-benzoyl)-piperazin-1-yl]-*N*-(3-dimethylamino-propyl)-nicotinamid;
6-[4-(2-Brom-benzoyl)-piperazin-1-yl]-*N*-(3-dimethylamino-propyl)-nicotinamid;
6-[4-(2,4-Dichlor-benzoyl)-piperazin-1-yl]-*N*-(3-dimethylamino-propyl)-nicotinamid;
6-[4-(2,5-Dichlor-benzoyl)-piperazin-1-yl]-*N*-(3-dimethylamino-propyl)-nicotinamid;
*N*-(3-Dimethylamino-propyl)-6-[4-(2,4-dimethyl-benzoyl)-piperazin-1-yl]-nicotinamid;
*N*-(3-Methyl-butyl)-6-[4-(2-trifluormethyl-benzoyl)-piperazin-1-yl]-nicotinamid;
*N*-(3-Methyl-butyl)-4-trifluormethyl-6-[4-(2-trifluormethyl-benzoyl)-piperazin-1-yl]-nicotinamid;
2-Chlor-5-fluor-*N*-(3-methylbutyl)-6-[4-(2-trifluormethyl-benzoyl)-piperazin-1-yl]-nicotinamid;
*N*-(3-Ethoxy-propyl)-6-[4-(2-naphthalin-2-yl-acetyl)-piperazin-1-yl]-nicotinamid;
6-[4-(2-Naphthalin-2-yl-acetyl)-piperazin-1-yl]-*N*-pentyl-nicotinamid;
*N*-Butyl-6-[4-(naphthalin-1-carbonyl)-piperazin-1-yl]-nicotinamid;
*N*-(3-Butoxy-propyl)-6-[4-(2-naphthalin-2-yl-acetyl)-piperazin-1-yl]-nicotinamid;
*N*-Butyl-6-[4-(2-naphthalin-2-yl-acetyl)-piperazin-1-yl]-nicotinamid;
*N*-(3-Butoxy-propyl)-6-[4-(naphthalin-1-carbonyl)-piperazin-1-yl]-nicotinamid;
*N*-(3-Methoxy-propyl)-6-[4-(naphthalin-1-carbonyl)-piperazin-1-yl]-nicotinamid;
*N*-(1-Methyl-butyl)-6-[4-(naphthalin-1-carbonyl)-piperazin-1-yl]-nicotinamid;
6-[4-(Naphthalin-1-carbonyl)-piperazin-1-yl]-*N*-pentyl-nicotinamid;
*N*-(3-Isopropoxy-propyl)-6-[4-(2-naphthalin-2-yl-acetyl)-piperazin-1-yl]-nicotinamid;
*N*-(1-Methyl-butyl)-6-[4-(2-naphthalin-2-yl-acetyl)-piperazin-1-yl]-nicotinamid;
*N*-(3-Methyl-butyl)-6-[4-(2-naphthalin-2-yl-acetyl)-piperazin-1-yl]-nicotinamid;
*N*-(3-Dimethylamino-propyl)-6-[4-(2-naphthalin-2-yl-acetyl)-piperazin-1-yl]-nicotinamid;
*N*-(3-Ethoxy-propyl)-6-[4-(naphthalin-1-carbonyl)-piperazin-1-yl]-nicotinamid;
*N*-Hexyl-6-[4-(naphthalin-1-carbonyl)-piperazin-1-yl]-nicotinamid;
*N*-(2-Ethylsulfanyl-ethyl)-6-[4-(2-naphthalin-2-yl-acetyl)-piperazin-1-yl]-nicotinamid;
*N*-Hexyl-6-[4-(2-naphthalin-2-yl-acetyl)-piperazin-1-yl]-nicotinamid;
*N*-(1,3-Dimethylbutyl)-6-[4-(naphthalin-1-carbonyl)-piperazin-1-yl]-nicotinamid;
*N*-(3-Isopropoxy-propyl)-6-[4-(naphthalin-1-carbonyl)-piperazin-1-yl]-nicotinamid;
*N*-(1,3-Dimethylbutyl)-6-[4-(naphthalin-2-carbonyl)-piperazin-1-yl]-nicotinamid;
*N*-(1,3-Dimethylbutyl)-6-[4-(2-naphthalin-2-yl-acetyl)-piperazin-1-yl]-nicotinamid;
*N*-(2-Methyl-butyl)-6-[4-(naphthalin-1-carbonyl)-piperazin-1-yl]-nicotinamid;
*N*-(2-Ethylsulfanyl-ethyl)-6-[4-(naphthalin-1-carbonyl)-piperazin-1-yl]-nicotinamid;
*N*-(2-Methyl-butyl)-6-[4-(2-naphthalin-2-yl-acetyl)-piperazin-1-yl]-nicotinamid;
*N*-(3-Methyl-butyl)-6-[4-(naphthalin-1-carbonyl)-piperazin-1-yl]-nicotinamid;
*N*-(3-Ethoxy-propyl)-6-[4-(naphthalin-2-carbonyl)-piperazin-1-yl]-nicotinamid;
*N*-(2-Methyl-butyl)-6-[4-(naphthalin-2-carbonyl)-piperazin-1-yl]-nicotinamid;
*N*-(3-Methyl-butyl)-6-[4-(naphthalin-2-carbonyl)-piperazin-1-yl]-nicotinamid;
*N*-(3-Methoxy-propyl)-6-[4-(naphthalin-2-carbonyl)-piperazin-1-yl]-nicotinamid;
*N*-Butyl-6-[4-(naphthalin-2-carbonyl)-piperazin-1-yl]-nicotinamid;
*N*-(3-Butoxy-propyl)-6-[4-(naphthalin-2-carbonyl)-piperazin-1-yl]-nicotinamid;
*N*-(1-Methyl-butyl)-6-[4-(naphthalin-2-carbonyl)-piperazin-1-yl]-nicotinamid;
*N*-(3-Isopropoxy-propyl)-6-[4-(naphthalin-2-carbonyl)-piperazin-1-yl]-nicotinamid;
*N*-Hexyl-6-[4-(naphthalin-2-carbonyl)-piperazin-1-yl]-nicotinamid;
*N*-(3-Dimethylamino-propyl)-6-[4-(naphthalin-1-carbonyl)-piperazin-1-yl]-nicotinamid;
*N*-(2-Ethylsulfanyl-ethyl)-6-[4-(naphthalin-2-carbonyl)-piperazin-1-yl]-nicotinamid;
*N*-(3-Dimethylamino-propyl)-6-[4-(naphthalin-2-carbonyl)-piperazin-1-yl]-nicotinamid;
*N*-(3-Methyl-butyl)-6-[4-(naphthalin-1-carbonyl)-piperazin-1-yl]-nicotinamid;
*N*-(3-Methoxy-propyl)-6-[4-(2-naphthalin-2-yl-acetyl)-piperazin-1-yl]-nicotinamid;
2-Chlor-*N*-(3-methylbutyl)-6-[4-(2-trifluormethyl-benzoyl)-piperazin-1-yl]-nicotinamid;
*N*-(2-Cyclopropyl-ethyl)-6-[4-(3-trifluormethyl-benzoyl)-piperazin-1-yl]-nicotinamid;
*N*-(2-Cyclopropyl-ethyl)-2-methoxy-6-[4-(2-trifluormethyl-benzoyl)-piperazin-1-yl]-nicotinamid;
*N*-(2-Cyclopropyl-ethyl)-6-[4-(5-fluor-2-trifluormethyl-benzoyl)-piperazin-1-yl]-nicotinamid;
2-Oxo-6-[4-(2-trifluormethyl-benzoyl)-piperazin-1-yl]-1,2-dihydro-pyridin-3-carbonsäure-(2-cyclopropylethyl)amid;
*N*-(2-Cyclopropyl-ethyl)-2-hydroxy-6-[4-(2-trifluormethyl-benzoyl)-piperazin-1-yl]-nicotinamid;
*N*-(2-Cyclobutyl-ethyl)-6-[4-(5-fluor-2-trifluormethyl-benzoyl)-piperazin-1-yl]-nicotinamid;
*N*-(3-Cyclopropyl-propyl)-6-[4-(5-fluor-2-trifluormethyl-benzoyl)-piperazin-1-yl]-nicotinamid;
6-[4-(5-Fluor-2-trifluormethyl-benzoyl)-piperazin-1-yl]-*N*-(4-methyl-pentyl)-nicotinamid; und
*N*-(3,3-Dimethylbutyl)-6-[4-(5-fluor-2-trifluormethyl-benzoyl)-piperazin-1-yl]-nicotinamid.

64. Verbindung zur Verwendung wie in Anspruch 60, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Aryl;
jedes R⁴ ist unabhängig Wasserstoff, Alkyl, Halogen oder Halogenalkyl; und
jedes R⁵ und R⁶ ist unabhängig Wasserstoff, Oxo, Alkyl, Halogen oder Halogenalkyl.

65. Verbindung zur Verwendung wie in Anspruch 64, wobei die Verbindung der Formel (I) eine Verbindung ist, worin:
m ist 1 oder 2;
n ist 1;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Heterocyclyl, gegebenenfalls substituiertem Heterocyclylalkyl, gegebenenfalls substituiertem Heteroaryl und
gegebenenfalls substituiertem Heteroarylalkyl;
R³ ist gegebenenfalls substituiertes Aryl;
jedes R⁴ ist unabhängig Wasserstoff, Alkyl, Halogen oder Halogenalkyl;
R⁵ ist Wasserstoff, Oxo, Alkyl, Halogen oder Halogenalkyl; und
jedes R⁶ ist unabhängig Wasserstoff, Alkyl, Halogen oder Halogenalkyl.

66. Verbindung zur Verwendung wie in Anspruch 65, wobei die Verbindung der Formel (I) ausgewählt ist aus der Gruppe bestehend aus den folgenden:
6-[4-(2,5-Dichlor-benzoyl)-piperazin-1-yl]-*N*-(3-imidazol-1-yl-propyl)-nicotinamid;
6-[4-(2,4-Dichlor-benzoyl)-piperazin-1-yl]-*N*-(3-imidazol-1-yl-propyl)-nicotinamid;
6-[4-(2-Brom-5-methoxy-benzoyl)-piperazin-1-yl]-*N*-(3-phenyl-propyl)-nicotinamid;
6-[4-(2-Brom-benzoyl)-piperazin-1-yl]-*N*-[2-(3H-imidazol-4-yl)-ethyl]-nicotinamid;
*N*-(1-Methyl-2-phenyl-ethyl)-6-[4-(2-trifluormethyl-benzoyl)-piperazin-1-yl]-nicotinamid;
*N*-(1-Methyl-2-phenyl-ethyl)-6-[4-(4-trifluormethyl-benzoyl)-piperazin-1-yl]-nicotinamid;
6-[4-(Naphthalin-1-carbonyl)-piperazin-1-yl]-*N*-(4-phenyl-butyl)-nicotinamid;
*N*-[2-(3-Chlorphenyl)-ethyl]-6-[4-(2-naphthalin-2-yl-acetyl)-piperazin-1-yl]-nicotinamid;
6-[4-(Naphthalin-2-carbonyl)-piperazin-1-yl]-*N*-(3-phenyl-propyl)-nicotinamid;
*N*-(3-Imidazol-1-yl-propyl)-6-[4-(2-naphthalin-2-yl-acetyl)-piperazin-1-yl]-nicotinamid;
6-[4-(Naphthalin-1-carbonyl)-piperazin-1-yl]-*N*-(3-phenyl-propyl)-nicotinamid;
*N*-(1-Methyl-3-phenyl-propyl)-6-[4-(2-naphthalin-2-yl-acetyl)-piperazin-1-yl]-nicotinamid;
6-[4-(2-Naphthalin-2-yl-acetyl)-piperazin-1-yl]-*N*-phenethyl-nicotinamid;
*N*-[2-(3-Chlorphenyl)-ethyl]-6-[4-(naphthalin-1-carbonyl)-piperazin-1-yl]-nicotinamid;
*N*-(1-Methyl-3-phenyl-propyl)-6-[4-(naphthalin-1-carbonyl)-piperazin-1-yl]-nicotinamid;
6-[4-(Naphthalin-1-carbonyl)-piperazin-1-yl]-*N*-phenethyl-nicotinamid;
*N*-[2-(3H-Imidazol-4-yl)-ethyl]-6-[4-(2-naphthalin-2-yl-acetyl)-piperazin-1-yl]-nicotinamid;
6-[4-(2-Naphthalin-2-yl-acetyl)-piperazin-1-yl]-*N*-(4-phenyl-butyl)-nicotinamid;
6-[4-(Naphthalin-2-carbonyl)-piperazin-1-yl]-*N*-(3-phenyl-propyl)-nicotinamid;
*N*-(1-Methyl-3-phenyl-propyl)-6-[4-(naphthalin-2-carbonyl)-piperazin-1-yl]-nicotinamid;
*N*-[2-(3H-Imidazol-4-yl)-ethyl]-6-[4-(naphthalin-1-carbonyl)-piperazin-1-yl]-nicotinamid;
6-[4-(Naphthalin-2-carbonyl)-piperazin-1-yl]-*N-*(tetrahydro-furan-2-ylmethyl)-nicotinamid;
6-[4-(Naphthalin-1-carbonyl)-piperazin-1-yl]-*N*-(tetrahydro-furan-2-ylmethyl)-nicotinamid;
*N*-(3-Imidazol-1-yl-propyl)-6-[4-(naphthalin-2-carbonyl)-piperazin-1-yl]-nicotinamid;
*N*-[2-(3H-Imidazol-4-yl)-ethyl]-6-[4-(naphthalin-2-carbonyl)-piperazin-1-yl]-nicotinamid;
6-[4-(Naphthalin-2-carbonyl)-piperazin-1-yl]-*N*-phenethyl-nicotinamid;
6-[4-(Naphthalin-2-carbonyl)-piperazin-1-yl]-*N*-(4-phenyl-butyl)-nicotinamid;
*N*-[2-(3-Chlorphenyl)-ethyl]-6-[4-(naphthalin-2-carbonyl)-piperazin-1 -yl]-nicotinamid;
*N*-(3-lmidazol-1-yl-propyl)-6-[4-(naphthalin-1-carbonyl)-piperazin-1-yl]-nicotinamid;
6-[4-(2-Naphthalin-2-yl-acetyl)-piperazin-1-yl]-*N*-(tetrahydro-furan-2-ylmethyl)-nicotinamid;
6-[4-(2-Brom-benzoyl)-piperazin-1-yl]-*N*-(3-phenyl-propyl)-nicotinamid;
*N*-[2-(3-Chlorphenyl)-ethyl]-6-[4-(2,4-dichlor-benzoyl)-piperazin-1-yl]-nicotinamid;
6-[4-(2,4-Dimethyl-benzoyl)-piperazin-1-yl]-*N*-(4-phenyl-butyl)-nicotinamid;
*N*-Phenethyl-6-[4-(2-p-tolyl-acetyl)-piperazin-1-yl]-nicotinamid;
6-{4-[2-(2,5-Dichlor-phenyl)-acetyl]-piperazin-1-yl}-*N*-(2-methyl-3-phenyl-propyl)-nicotinamid;
6-[4-(2,4-Dimethyl-benzoyl)-piperazin-1-yl]-*N*-(3-phenyl-propyl)-nicotinamid;
*N*-[2-(3-Chlorphenyl)-ethyl]-6-[4-(2-fluor-4-methyl-benzoyl)-piperazin-1-yl]-nicotinamid;
*N*-[2-(3-Chlorphenyl)-ethyl]-6-[4-(2,5-dichlor-benzoyl)-piperazin-1-yl]-nicotinamid;
*N*-(3-Phenyl-propyl)-6-[4-(4-trifluormethyl-benzoyl)-piperazin-1-yl]-nicotinamid;
6-[4-(2-Brom-benzoyl)-piperazin-1-yl]-*N*-(3-imidazol-1-yl-propyl)-nicotinamid;
*N*-(4-Phenyl-butyl)-6-[4-(2-trifluormethyl-benzoyl)-piperazin-1-yl]-nicotinamid;
*N*-(3-Phenyl-propyl)-6-[4-(3-trifluormethyl-benzoyl)-piperazin-1-yl]-nicotinamid;
6-[4-(2-Brom-benzoyl)-piperazin-1-yl]-*N*-[2-(3-chlor-phenyl)-ethyl]-nicotinamid;
6-[4-(2-Brom-benzoyl)-piperazin-1-yl]-*N*-(2-methyl-3-phenyl-propyl)-nicotinamid;
6-[4-(2-Brom-5-methoxy-benzoyl)-piperazin-1-yl]-*N*-(5-phenyl-pentyl)-nicotinamid;
6-[4-(2-Brom-5-methoxy-benzoyl)-piperazin-1-yl]-*N*-[2-(3-chlor-phenyl)-ethyl]-nicotinamid;
*N*-[2-(3-Chlorphenyl)-ethyl]-6-[4-(2-trifluormethyl-benzoyl)-piperazin-1-yl]-nicotinamid;
*N*-(3-Phenyl-propyl)-6-[4-(2-trifluormethyl-benzoyl)-piperazin-1-yl]-nicotinamid;
*N*-(4-Phenyl-butyl)-6-[4-(3-trifluormethyl-benzoyl)-piperazin-1-yl]-nicotinamid;
6-[4-(2,4-Dichlor-benzoyl)-piperazin-1-yl]-*N*-phenethyl-nicotinamid;
6-[4-(2-Brom-benzoyl)-piperazin-1-yl]-*N*-(4-phenyl-butyl)-nicotinamid;
6-[4-(2-Brom-benzoyl)-piperazin-1-yl]-*N*-phenethyl-nicotinamid;
6-[4-(2-Brom-5-methoxy-benzoyl)-piperazin-1-yl]-*N*-(1-methyl-3-phenyl-propyl)-nicotinamid;
6-[4-(3,5-Dichlor-benzoyl)-piperazin-1-yl]-*N*-(3-phenyl-propyl)-nicotinamid;
6-[4-(3,5-Dichlor-benzoyl)-piperazin-1-yl]-*N*-(1-methyl-3-phenyl-propyl)-nicotinamid;
*N*-Phenethyl-6-[4-(4-trifluormethyl-benzoyl)-piperazin-1-yl]-nicotinamid;
6-[4-(2,4-Dimethyl-benzoyl)-piperazin-1-yl]-*N*-phenethyl-nicotinamid;
*N*-Phenethyl-6-[4-(2-trifluormethyl-benzoyl)-piperazin-1-yl]-nicotinamid;
6-[4-(2,4-Dichlor-benzoyl)-piperazin-1-yl]-*N*-(1-methyl-3-phenyl-propyl)-nicotinamid;
6-[4-(2,5-Dichlor-benzoyl)-piperazin-1-yl]-*N*-(3-phenyl-propyl)-nicotinamid;
6-[4-(2-Brom-5-methoxy-benzoyl)-piperazin-1-yl]-*N*-phenethyl-nicotinamid;
6-[4-(2-Brom-5-methoxy-benzoyl)-piperazin-1-yl]-*N*-(3-imidazol-1-yl-propyl)-nicotinamid;
6-[4-(3, 5-Dichlor-benzoyl)-piperazin-1-yl]-*N*-(3-imidazol-1-yl-propyl)-nicotinamid;
*N*-[2-(3H-Imidazol-4-yl)-ethyl]-6-[4-(2,3,4,5-tetrafluor-benzoyl)-piperazin-1-yl]-nicotinamid;
*N*-Phenethyl-6-[4-(3-trifluormethyl-benzoyl)-piperazin-1-yl]-nicotinamid;
6-[4-(2,5-Dichlor-benzoyl)-piperazin-1-yl]-*N*-phenethyl-nicotinamid;
6-[4-(3-Methyl-pentanoyl)-piperazin-1-yl]-*N*-(1-methyl-3-phenyl-propyl)-nicotinamid;
6-[4-(2,3,4,5-Tetrafluor-benzoyl)-piperazin-1-yl]-*N*-(tetrahydro-furan-2-ylmethyl)-nicotinamid;
6-[4-(2-Brom-5-methoxy-benzoyl)-piperazin-1-yl]-*N*-(tetrahydro-furan-2-ylmethyl)-nicotinamid;
*N*-(Tetrahydro-furan-2-ylmethyl)-6-[4-(4-trifluormethyl-benzoyl)-piperazin-1-yl]-nicotinamid;
6-[4-(3,5-Dichlor-benzoyl)-piperazin-1-yl]-*N*-(4-phenyl-butyl)-nicotinamid;
6-[4-(2,4-Dimethyl-benzoyl)-piperazin-1-yl]-*N*-(1-methyl-3-phenyl-propyl)-nicotinamid;
6-[4-(2,4-Dimethyl-benzoyl)-piperazin-1-yl]-*N*-(tetrahydro-furan-2-ylmethyl)-nicotinamid;
6-[4-(2-Brom-benzoyl)-piperazin-1-yl]-*N*-(tetrahydro-furan-2-ylmethyl)-nicotinamid;
*N*-(1-Methyl-3-phenyl-propyl)-6-[4-(3-trifluormethyl-benzoyl)-piperazin-1-yl]-nicotinamid;
*N*-Phenethyl-6-[4-(2,3,4,5-tetrafluor-benzoyl)-piperazin-1-yl]-nicotinamid;
*N*-(4-Phenyl-butyl)-6-[4-(2,3,4,5-tetrafluor-benzoyl)-piperazin-1-yl]-nicotinamid;
*N*-(1-Methyl-3-phenyl-propyl)-6-[4-(2,3,4,5-tetrafluor-benzoyl)-piperazin-1-yl]-nicotinamid;
*N*-[2-(3-Chlorphenyl)-ethyl]-6-[4-(2,3,4,5-tetrafluor-benzoyl)-piperazin-1-yl]-nicotinamid;
*N*-(3-Phenyl-propyl)-6-[4-(2,3,4,5-tetrafluor-benzoyl)-piperazin-1-yl]-nicotinamid;
6-[4-(2,5-Dichlor-benzoyl)-piperazin-1-yl]-*N*-(tetrahydro-furan-2-ylmethyl)-nicotinamid;
6-[4-(2,4-Dimethyl-benzoyl)-piperazin-1-yl]-*N*-[2-(3H-imidazol-4-yl)-ethyl]-nicotinamid;
*N*-[2-(3H-lmidazol-4-yl)-ethyl]-6-[4-(3-trifluormethyl-benzoyl)-piperazin-1-yl]-nicotinamid;
*N*-(3-Imidazol-1-yl-propyl)-6-[4-(3-trifluormethyl-benzoyl)-piperazin-1-yl]-nicotinamid;
6-[4-(2,4-Dimethyl-benzoyl)-piperazin-1-yl]-*N*-(3-imidazol-1-yl-propyl)-nicotinamid;
6-[4-(2,5-Dichlor-benzoyl)-piperazin-1-yl]-*N*-[2-(3H-imidazol-4-yl)-ethyl]-nicotinamid;
N-[2-(3H-Imidazol-4-yl)-ethyl]-6-[4-(4-trifluormethyl-benzoyl)-piperazin-1-yl]-nicotinamid;
6-[4-(2,4-Dichlor-benzoyl)-piperazin-1-yl]-*N*-[2-(3H-imidazol-4-yl)-ethyl]-nicotinamid;
6-[4-(3,5-Dichlor-benzoyl)-piperazin-1-yl]-*N*-(tetrahydro-furan-2-ylmethyl)-nicotinamid;
*N*-(3-Imidazol-1-yl-propyl)-6-[4-(2-trifluormethyl-benzoyl)-piperazin-1-yl]-nicotinamid;
*N*-(Tetrahydro-furan-2-ylmethyl)-6-[4-(3-trifluormethyl-benzoyl)-piperazin-1-yl]-nicotinamid;
*N*-(Tetrahydro-furan-2-ylmethyl)-6-[4-(2-trifluormethyl-benzoyl)-piperazin-1-yl]-nicotinamid;
6-[4-(2,4-Dichlor-benzoyl)-piperazin-1-yl]-*N*-(tetrahydro-furan-2-ylmethyl)-nicotinamid;
*N*-[2-(3H-Imidazol-4-yl)-ethyl]-6-[4-(2-trifluormethyl-benzoyl)-piperazin-1-yl]-nicotinamid;
6-[4-(2-Brom-5-methoxy-benzoyl)-piperazin-1-yl]-*N*-[2-(3H-imidazol-4-yl)-ethyl]-nicotinamid;
*N*-[2-(3-Chlorphenyl)-ethyl]-6-[4-(3-trifluormethyl-benzoyl)-piperazin-1-yl]-nicotinamid;
*N*-[2-(3-Chlorphenyl)-ethyl]-6-[4-(4-trifluormethyl-benzoyl)-piperazin-1-yl]-nicotinamid;
*N*-[2-(3-Chlorphenyl)-ethyl]-6-[4-(3,5-dichlor-benzoyl)-piperazin-1-yl]-nicotinamid;
*N*-(4-Phenyl-butyl)-6-[4-(4-trifluormethyl-benzoyl)-piperazin-1-yl]-nicotinamid;
6-[4-(2,4-Dichlor-benzoyl)-piperazin-1-yl]-*N*-(4-phenyl-butyl)-nicotinamid;
6-[4-(2,4-Dichlor-benzoyl)-piperazin-1-yl]-*N*-(3-phenyl-propyl)-nicotinamid;
6-[4-(2,5-Dichlor-benzoyl)-piperazin-1-yl]-*N*-(4-phenyl-butyl)-nicotinamid;
6-[4-(3,5-Dichlor-benzoyl)-piperazin-1-yl]-*N*-phenethyl-nicotinamid;
*N*-(3-Imidazol-1-yl-propyl)-6-[4-(4-trifluormethyl-benzoyl)-piperazin-1-yl]-nicotinamid;
6-[4-(3,5-Dichlor-benzoyl)-piperazin-1-yl]-*N*-[2-(3H-imidazol-4-yl)-ethyl]-nicotinamid;
*N*-(3-Imidazol-1-yl-propyl)-6-[4-(2,3,4,5-tetrafluor-benzoyl)-piperazin-1-yl]-nicotinamid;
6-[2-Oxo-4-(2-trifluormethyl-benzoyl)-piperazin-1-yl]-*N*-(3-phenyl-propyl)-nicotinamid;
*N*-(3-Methyl-butyl)-6-[2-oxo-4-(2-trifluormethyl-benzoyl)-piperazin-1-yl]-nicotinamid.

67. Verbindung zur Verwendung wie in Anspruch 45, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Heteroaryl, gegebenenfalls substituiertes Heteroarylalkyl oder gegebenenfalls substituiertes Heteroarylalkenyl;
jedes R⁴ ist unabhängig Wasserstoff, Alkyl, Alkenyl, Halogen, Halogenalkyl oder Aryl;
jedes R⁵ und R⁶ ist unabhängig Wasserstoff, Oxo, Alkyl, Alkenyl, Halogen, Halogenalkyl oder Aryl;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl; und
R¹⁰ ist Alkyl, Aryl oder Aralkyl.

68. Verbindung zur Verwendung wie in Anspruch 67, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Cycloalkyl, gegebenenfalls substituiertem Cycloalkylalkyl oder Cycloalkylalkenyl;
R³ ist gegebenenfalls substituiertes Heteroaryl, gegebenenfalls substituiertes Heteroarylalkyl oder gegebenenfalls substituiertes Heteroarylalkenyl;
jedes R⁴ ist unabhängig Wasserstoff, Alkyl, Halogen oder Halogenalkyl;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl
und Aralkyl; und
R¹⁰ ist Alkyl, Aryl oder Aralkyl.

69. Verbindung zur Verwendung wie in Anspruch 68, wobei die Verbindung der Formel (I) eine Verbindung ist, worin:
m ist 1 oder 2;
nist1;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist) oder Alkyl;
R³ ist gegebenenfalls substituiertes Heteroaryl, gegebenenfalls substituiertes Heteroarylalkyl oder gegebenenfalls substituiertes Heteroarylalkenyl;
jedes R⁴ ist unabhängig Wasserstoff, Halogen oder Halogenalkyl;
R⁵ ist Wasserstoff;
jedes R⁶ ist Wasserstoff;
R⁷ ist eine gerade oder verzweigte Alkylenkette;
R⁸ ist Wasserstoff oder Alkyl; und
R¹⁰ ist Alkyl, Aryl oder Aralkyl.

70. Verbindung zur Verwendung wie in Anspruch 69, wobei die Verbindung der Formel (I) ausgewählt ist aus der Gruppe bestehend aus den folgenden:
6-[4-(3-Methyl-3H-1[4-thiophen-2-carbonyl)-piperazin-1-yl]-*N*-pentyl-nicotinamid;
*N*-(3-Dimethylamino-propyl)-6-[4-(3-methyl-thiophen-2-carbonyl)-piperazin-1-yl]-nicotinamid;
*N*-(3-Dimethylamino-propyl)-6-[4-(thiophen-2-carbonyl)-piperazin-1-yl]-nicotinamid;
6-[4-(2-Chlor-pyridin-3-carbonyl)-piperazin-1-yl]-*N*-(3-ethoxy-propyl)-nicotinamid;
6-[4-(2-Chlor-pyridin-3-carbonyl)-piperazin-1-yl]-*N*-(2-ethylsulfanyl-ethyl)-nicotinamid;
6-[4-(2-Chlor-pyridin-3-carbonyl)-piperazin-1-yl]-*N*-(3-methylbutyl)-nicotinamid;
6-[4-(2-Chlor-pyridin-3-carbonyl)-piperazin-1-yl]-*N*-(3-isopropoxy-propyl)-nicotinamid;
*N*-(2-Ethylsulfanyl-ethyl)-6-[4-(thiophen-2-carbonyl)-piperazin-1-yl]-nicotinamid;
*N*-(3-Ethoxy-propyl)-6-[4-(thiophen-2-carbonyl)-piperazin-1-yl]-nicotinamid;
*N*-(3-Methyl-butyl)-6-[4-(thiophen-2-carbonyl)-piperazin-1-yl]-nicotinamid;
6-[4-(2-Chlor-pyridin-3-carbonyl)-piperazin-1-yl]-*N*-(3-methoxy-propyl)-nicotinamid;
*N*-Pentyl-6-[4-(thiophen-2-carbonyl)-piperazin-1-yl]-nicotinamid;
6-[4-(2-Chlor-pyridin-3-carbonyl)-piperazin-1-yl]-*N*-hexyl-nicotinamid;
*N*-(3-Isopropoxy-propyl)-6-[4-(thiophen-2-carbonyl)-piperazin-1-yl]-nicotinamid;
*N*-(1-Methyl-butyl)-6-[4-(thiophen-2-carbonyl)-piperazin-1-yl]-nicotinamid;
*N*-Hexyl-6-[4-(thiophen-2-carbonyl)-piperazin-1-yl]-nicotinamid;
*N*-(1,3-Dimethylbutyl)-6-[4-(thiophen-2-carbonyl)-piperazin-1-yl]-nicotinamid;
*N*-Butyl-6-[4-(thiophen-2-carbonyl)-piperazin-1-yl]-nicotinamid;
*N*-(3-Methoxy-propyl)-6-[4-(thiophen-2-carbonyl)-piperazin-1-yl]-nicotinamid;
6-[4-(2-Chlor-pyridin-3-carbonyl)-piperazin-1-yl]-*N*-(1,3-dimethylbutyl)-nicotinamid;
*N*-Butyl-6-[4-(2-chlor-pyridin-3-carbonyl)-piperazin-1-yl]-nicotinamid;
6-[4-(2-Chlor-pyridin-3-carbonyl)-piperazin-1-yl]-*N*-(2-methylbutyl)-nicotinamid;
*N*-(2-Methyl-butyl)-6-[4-(thiophen-2-carbonyl)-piperazin-1-yl]-nicotinamid;
6-[4-(2-Chlor-pyridin-3-carbonyl)-piperazin-1-yl]-*N*-(1-methylbutyl)-nicotinamid;
*N*-(1,3-Dimethylbutyl)-6-[4-(3-methyl-thiophen-2-carbonyl)-piperazin-1-yl]-nicotinamid;
*N*-Butyl-6-[4-(3-methyl-thiophen-2-carbonyl)-piperazin-1-yl]-nicotinamid;
*N*-(3-Butoxy-propyl)-6-[4-(3-methyl-thiophen-2-carbonyl)-piperazin-1-yl]-nicotinamid;
*N*-(3-Isopropoxy-propyl)-6-[4-(3-methyl-thiophen-2-carbonyl)-piperazin-1-yl]-nicotinamid;
*N*-(3-Ethoxy-propyl)-6-[4-(3-methyl-thiophen-2-carbonyl)-piperazin-1-yl]-nicotinamid;
*N*-(2-Methyl-butyl)-6-[4-(3-methyl-thiophen-2-carbonyl)-piperazin-1-yl]-nicotinamid;
*N*-(2-Ethylsulfanyl-ethyl)-6-[4-(3-methyl-thiophen-2-carbonyl)-piperazin-1-yl]-nicotinamid;
*N*-(3-Methoxy-propyl)-6-[4-(3-methyl-thiophen-2-carbonyl)-piperazin-1-yl]-nicotinamid;
*N*-(1-Methyl-butyl)-6-[4-(3-methyl-thiophen-2-carbonyl)-piperazin-1-yl]-nicotinamid;
*N*-(3-Methyl-butyl)-6-[4-(3-methyl-thiophen-2-carbonyl)-piperazin-1-yl]-nicotinamid;
*N*-(3-Butoxy-propyl)-6-[4-(2-chlor-pyridin-3-carbonyl)-piperazin-1-yl]-nicotinamid;
*N*-(3-Butoxy-propyl)-6-[4-(thiophen-2-carbonyl)-piperazin-1-yl]-nicotinamid;
6-[4-(2-Chlor-pyridin-3-carbonyl)-piperazin-1-yl]-*N*-pentyl-nicotinamid;
*N*-Hexyl-6-[4-(3-methyl-thiophen-2-carbonyl)-piperazin-1-yl]-nicotinamid; und
6-[4-(2-Chlor-pyridin-3-carbonyl)-piperazin-1-yl]-*N*-(3-dimethylamino-propyl)-nicotinamid.

71. Verbindung zur Verwendung wie in Anspruch 67, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Heteroaryl, gegebenenfalls substituiertes Heteroarylalkyl oder gegebenenfalls substituiertes Heteroarylalkenyl;
jedes R⁴ ist unabhängig Wasserstoff, Alkyl, Halogen oder Halogenalkyl; und
jedes R⁵ und R⁶ ist unabhängig Wasserstoff, Oxo, Alkyl, Halogen oder Halogenalkyl.

72. Verbindung zur Verwendung wie in Anspruch 71, wobei die Verbindung der Formel (I) eine Verbindung ist, worin:
m ist 1 oder 2;
n ist 1;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Heterocyclyl, gegebenenfalls substituiertem Heterocyclylalkyl, gegebenenfalls substituiertem Heteroaryl und
gegebenenfalls substituiertem Heteroarylalkyl;
R³ ist gegebenenfalls substituiertes Heteroaryl, gegebenenfalls substituiertes Heteroarylalkyl oder gegebenenfalls substituiertes Heteroarylalkenyl;
jedes R⁴ ist unabhängig Wasserstoff, Alkyl, Halogen oder Halogenalkyl;
R⁵ ist Wasserstoff, Oxo, Alkyl, Halogen oder Halogenalkyl; und
jedes R⁶ ist unabhängig Wasserstoff, Oxo, Alkyl, Halogen oder Halogenalkyl.

73. Verbindung zur Verwendung wie in Anspruch 65, wobei die Verbindung der Formel (I) ausgewählt ist aus der Gruppe bestehend aus den folgenden:
*N*-[2-(3-Chlorphenyl)-ethyl]-6-[4-(3-methyl-thiophen-2-carbonyl)-piperazin-1-yl]-nicotinamid;
*N*-[2-(3-Chlorphenyl)-ethyl]-6-{4-[2-(2-chlor-pyridin-3-yl)-acetyl]-piperazin-1-yl}-nicotinamid;
6-[4-(2-Chlor-pyridin-3-carbonyl)-piperazin-1-yl]-*N*-(4-phenyl-butyl)-nicotinamid;
*N*-[2-(3-Chlorphenyl)-ethyl]-6-[4-(thiophen-2-carbonyl)-piperazin-1-yl]-nicotinamid;
6-[4-(2-Chlor-pyridin-3-carbonyl)-piperazin-1-yl]-*N*-(3-phenyl-propyl)-nicotinamid;
*N*-(3-Phenyl-propyl)-6-[4-(thiophen-2-carbonyl)-piperazin-1-yl]-nicotinamid;
*N*-Phenethyl-6-[4-(thiophen-2-carbonyl)-piperazin-1-yl]-nicotinamid;
*N*-(1-Methyl-3-phenyl-propyl)-6-[4-(thiophen-2-carbonyl)-piperazin-1-yl]-nicotinamid;
*N*-(4-Phenyl-butyl)-6-[4-(thiophen-2-carbonyl)-piperazin-1-yl]-nicotinamid;
6-[4-(2-Chlor-pyridin-3-carbonyl)-piperazin-1-yl]-*N*-(1-methyl-3-phenyl-propyl)-nicotinamid;
6-[4-(2-Chlor-pyridin-3-carbonyl)-piperazin-1-yl]-*N*-phenethyl-nicotinamid;
6-[4-(2-Chlor-pyridin-3-carbonyl)-piperazin-1-yl]-*N*-(tetrahydro-furan-2-ylmethyl)-nicotinamid;
6-[4-(3-Methyl-thiophen-2-carbonyl)-piperazin-1-yl]-*N*-(3-phenyl-propyl)-nicotinamid;
*N*-(1-Methyl-3-phenyl-propyl)-6-[4-(3-methyl-thiophen-2-carbonyl)-piperazin-1-yl]-nicotinamid;
6-[4-(3-Methyl-thiophen-2-carbonyl)-piperazin-1-yl]-*N*-(4-phenyl-butyl)-nicotinamid;
6-[4-(3-Methyl-thiophen-2-carbonyl)-piperazin-1-yl]-*N*-phenethyl-nicotinamid;
6-[4-(3-Methyl-thiophen-2-carbonyl)-piperazin-1-yl]-*N*-(tetrahydro-furan-2-ylmethyl)-nicotinamid;
*N*-(3-Imidazol-1-yl-propyl)-6-[4-(thiophen-2-carbonyl)-piperazin-1-yl]-nicotinamid;
*N*-(Tetrahydro-furan-2-ylmethyl)-6-[4-(thiophen-2-carbonyl)-piperazin-1-yl]-nicotinamid;
*N*-[2-(3H-Imidazol-4-yl)-ethyl]-6-[4-(thiophen-2-carbonyl)-piperazin-1-yl]-nicotinamid;
*N*-(3-Imidazol-1-yl-propyl)-6-[4-(3-methyl-thiophen-2-carbonyl)-piperazin-1-yl]-nicotinamid;
*N*-[2-(3H-Imidazol-4-yl)-ethyl]-6-[4-(3-methyl-thiophen-2-carbonyl)-piperazin-1-yl]-nicotinamid;
6-[4-(2-Chlor-pyridin-3-carbonyl)-piperazin-1-yl]-*N*-(3-imidazol-1-yl-propyl)-nicotinamid; und
6-[4-(2-Chlor-pyridin-3-cärbonyl)-piperazin-1-yl]-*N*-[2-(3H-imidazol-4-yl)-ethyl]-nicotinamid.

74. Verbindung zur Verwendung wie in Anspruch 45, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Aralkyl oder gegebenenfalls substituiertes Aralkenyl;
jedes R⁴ ist unabhängig Wasserstoff, Alkyl, Alkenyl, Halogen, Halogenalkyl, Aryl oder
- R⁹-OR⁸;
jedes R⁵ und R⁶ ist unabhängig Wasserstoff, Oxo, Alkyl, Alkenyl, Halogen, Halogenalkyl oder Aryl;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
R⁹ ist eine direkte Bindung oder eine gerade oder verzweigte Alkylenkette; und
R¹⁰ ist Alkyl, Aryl oder Aralkyl.

75. Verbindung zur Verwendung wie in Anspruch 74, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Cycloalkyl, gegebenenfalls substituiertem Cycloalkylalkyl oder Cycloalkylalkenyl;
R³ ist gegebenenfalls substituiertes Aralkyl oder gegebenenfalls substituiertes Aralkenyl;
jedes R⁴ ist unabhängig Wasserstoff, Alkyl, Halogen oder Halogenalkyl;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl und Aralkyl; und
R¹⁰ ist Alkyl, Aryl oder Aralkyl.

76. Verbindung zur Verwendung wie in Anspruch 75, wobei die Verbindung der Formel (I) eine Verbindung ist, worin:
m ist 1 oder 2;
nist1;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist) oder Alkyl;
R³ ist gegebenenfalls substituiertes Aralkyl oder gegebenenfalls substituiertes Aralkenyl;
jedes R⁴ ist unabhängig Wasserstoff, Halogen oder Halogenalkyl;
R⁵ ist Wasserstoff;
jedes R⁶ ist Wasserstoff;
R⁷ ist eine gerade oder verzweigte Alkylenkette;
R⁸ ist Wasserstoff oder Alkyl; und
R¹⁰ ist Alkyl, Aryl oder Aralkyl.

77. Verbindung zur Verwendung wie in Anspruch 76, wobei die Verbindung der Formel (I) ausgewählt ist aus der Gruppe bestehend aus den folgenden:
6-{4-[2-(4-Chlorphenyl)-propionyl]-piperazin-1-yl}-*N*-(3-methylbutyl)-nicotinamid;
6-{4-[2-(2-Chlor-6-fluor-phenyl)-acetyl]-piperazin-1-yl}-*N*-(3-ethoxy-propyl)-nicotinamid;
6-{4-[3-(3,4-Difluor-phenyl)-propionyl]-piperazin-1-yl}-*N*-(3-dimethylamino-propyl)-nicotinamid;
6-{4-[3-(3,4-Difluor-phenyl)-propionyl]-piperazin-1-yl}-*N*-(3-ethoxy-propyl)-nicotinamid;
6-{4-[2-(4-Chlorphenyl)-propionyl]-piperazin-1-yl}-*N*-(3-ethoxy-propyl)-nicotinamid;
*N*-(3-Ethoxy-propyl)-6-[4-(2-phenyl-butyryl)-piperazin-1-yl]-nicotinamid;
*N*-(2-Ethylsulfanyl-ethyl)-6-[4-(2-o-tolyl-acetyl)-piperazin-1-yl]-nicotinamid;
6-[4-(3-Methyl-pentanoyl)-piperazin-1-yl]-*N*-(4-phenyl-butyl)-nicotinamid;
*N*-Butyl-6-{4-[3-(3,4-difluor-phenyl)-propionyl]-piperazin-1-yl}-nicotinamid;
6-{4-[2-(2-Chlor-6-fluor-phenyl)-acetyl]-piperazin-1-yl}-*N*-(3-dimethylamino-propyl)-nicotinamid;
6-{4-[3-(3,4-Difluor-phenyl)-propionyl]-piperazin-1-yl}-*N*-(2-ethylsulfanyl-ethyl)-nicotinamid;
*N*-(3-Methoxy-propyl)-6-[4-(2-phenyl-butyryl)-piperazin-1-yl]-nicotinamid;
*N*-Butyl-6-[4-(2-phenyl-butyryl)-piperazin-1-yl]-nicotinamid;
*N*-(3-Butoxy-propyl)-6-{4-[2-(4-chlor-phenyl)-propionyl]-piperazin-1-yl}-nicotinamid;
*N*-(3-Methoxy-propyl)-6-[4-(2-o-tolyl-acetyl)-piperazin-1-yl]-nicotinamid;
*N*-(3-Methyl-butyl)-6-[4-(2-phenyl-butyryl)-piperazin-1-yl]-nicotinamid;
*N*-(1-Methyl-butyl)-6-[4-(2-p-tolyl-acetyl)-piperazin-1-yl]-n icotinamid;
*N*-Butyl-6-{4-[2-(2-chlor-6-fluor-phenyl)-acetyl]-piperazin-1-yl}-nicotinamid;
*N*-(2-Methyl-butyl)-6-[4-(2-o-tolyl-acetyl)-piperazin-1-yl]-nicotinamid;
6-{4-[3-(3,4-Difluor-phenyl)-propionyl]-piperazin-1-yl}-*N*-(2-methylbutyl)-nicotinamid;
*N*-(3-Isopropoxy-propyl)-6-[4-(2-o-tolyl-acetyl)-piperazin-1-yl]-nicotinamid;
6-{4-[3-(3,4-Difluor-phenyl)-propionyl]-piperazin-1-yl}-*N*-(3-methylbutyl)-nicotinamid;
6-{4-[3-(3,4-Difluor-phenyl)-propionyl]-piperazin-1-yl}-*N*-(3-methoxy-propyl)-nicotinamid;
*N*-(1-Methyl-butyl)-6-[4-(2-o-tolyl-acetyl)-piperazin-1-yl]-nicotinamid;
6-{4-[2-(2-Chlor-6-fluor-phenyl)-acetyl]-piperazin-1-yl}-*N*-(2-methylbutyl)-nicotinamid;
6-{4-[2-(2-Chlor-6-fluor-phenyl)-acetyl]-piperazin-1-yl}-*N*-(1-methylbutyl)-nicotinamid;
*N*-(2-Methyl-butyl)-6-[4-(2-phenyl-butyryl)-piperazin-1-yl]-nicotinamid;
*N*-(1,3-Dimethylbutyl)-6-[4-(2-phenyl-butyryl)-piperazin-1-yl]-nicotinamid;
6-{4-[2-(4-Chlorphenyl)-propionyl]-piperazin-1-yl}-*N*-(2-methylbutyl)-nicotinamid;
6-{4-[2-(2-Chlor-6-fluor-phenyl)-acetyl]-piperazin-1-yl}-*N*-(3-isopropoxy-propyl)-nicotinamid;
*N*-(1,3-Dimethylbutyl)-6-[4-(2-p-tolyl-acetyl)-piperazin-1-yl]-nicotinamid;
6-{4-[3-(3,4-Difluor-phenyl)-propionyl]-piperazin-1-yl}-*N*-hexyl-nicotinamid;
*N*-(1-Methyl-butyl)-6-[4-(2-phenyl-butyryl)-piperazin-1-yl]-nicotinamid;
6-{4-[2-(4-Chlorphenyl)-propionyl]-piperazin-1-yl}-*N*-(1-methylbutyl)-nicotinamid;
6-{4-[2-(4-Chlorphenyl)-propionyl]-piperazin-1-yl}-*N*-(1,3-dimethylbutyl)-nicotinamid;
*N*-Butyl-6-[4-(2-p-tolyl-acetyl)-piperazin-1-yl]-nicotinamid;
*N*-(1,3-Dimethylbutyl)-6-[4-(2-o-tolyl-acetyl)-piperazin-1-yl]-nicotinamid;
6-{4-[2-(2-Chlor-6-fluor-phenyl)-acetyl]-piperazin-1-yl}-*N*-(1,3-dimethylbutyl)-nicotinamid;
*N*-(3-Methyl-butyl)-6-[4-(2-p-tolyl-acetyl)-piperazin-1-yl]-nicotinamid;
*N*-Butyl-6-[4-(2-o-tolyl-acetyl)-piperazin-1-yl]-nicotinamid;
*N*-(3-Butoxy-propyl)-6-[4-(2-p-tolyl-acetyl)-piperazin-1-yl]-nicotinamid;
*N*-Pentyl-6-[4-(2-p-tolyl-acetyl)-piperazin-1-yl]-nicotinamid;
*N*-(3-Butoxy-propyl)-6-[4-(2-phenyl-butyryl)-piperazin-1-yl]-nicotinamid;
*N*-Pentyl-6-[4-(2-phenyl-butyryl)-piperazin-1-yl]-nicotinamid;
*N*-(2-Ethylsulfanyl-ethyl)-6-[4-(2-p-tolyl-acetyl)-piperazin-1-yl]-nicotinamid;
6-{4-[2-(4-Chlorphenyl)-propionyl]-piperazin-1-yl}-*N*-(2-ethylsulfanyl-ethyl)-nicotinamid;
*N*-(3-Ethoxy-propyl)-6-[4-(2-o-tolyl-acetyl)-piperazin-1-yl]-nicotinamid;
6-(4-[2-(2-Chlor-6-fluor-phenyl)-acetyl]-piperazin-1-yl}-*N*-(3-methoxy-propyl)-nicotinamid;
6-{4-[3-(3,4-Difluor-phenyl)-propionyl]-piperazin-1-yl}-*N*-(3-isopropoxy-propyl)-nicotinamid;
6-{4-[2-(4-Chlorphenyl)-propionyl]-piperazin-1-yl}-*N*-pentyl-nicotinamid;
*N*-(3-Butoxy-propyl)-6-{4-[2-(2-chlor-6-fluor-phenyl)-acetyl]-piperazin-1-yl}-nicotinamid;
6-{4-[2-(2-Chlor-6-fluor-phenyl)-acetyl]-piperazin-1-yl}-*N*-hexyl-nicotinamid;
6-{4-[2-(4-Chlorphenyl)-propionyl]-piperazin-1-yl}-*N*-(3-methoxy-propyl)-nicotinamid;
*N*-Hexyl-6-[4-(2-o-tolyl-acetyl)-piperazin-1-yl]-nicotinamid;
6-{4-[3-(3,4-Difluor-phenyl)-propionyl]-piperazin-1-yl}-*N*-(4-phenyl-butyl)-nicotinamid;
*N*-(3-Isopropoxy-propyl)-6-[4-(2-p-tolyl-acetyl)-piperazin-1-yl]-nicotinamid;
6-{4-[3-(3,4-Difluor-phenyl)-propionyl]-piperazin-1-yl}-*N*-(1-methylbutyl)-nicotinamid;
6-{4-[3-(3,4-Difluor-phenyl)-propionyl]-piperazin-1-yl}-*N*-pentyl-nicotinamid;
6-{4-[2-(4-Chlorphenyl)-propionyl]-piperazin-1-yl}-*N*-hexyl-nicotinamid;
*N*-Butyl-6-{4-[2-(4-chlor-phenyl)-propionyl]-piperazin-1-yl}-nicotinamid_{;}
6-{4-[2-(2-Chlor-6-fluor-phenyl)-acetyl]-piperazin-1-yl}-*N*-(2-ethylsulfanyl-ethyl)-nicotinamid;
6-{4-[2-(2-Chlor-6-fluor-phenyl)-acetyl]-piperazin-1-yl}-*N*-pentyl-nicotinamid;
*N*-Hexyl-6-[4-(2-phenyl-butyryl)-piperazin-1-yl]-nicotinamid;
*N*-(3-Isopropoxy-propyl)-6-[4-(2-phenyl-butyryl)-piperazin-1-yl]-nicotinamid;
*N*-Pentyl-6-[4-(2-o-tolyl-acetyl)-piperazin-1-yl]-nicotinamid;
*N*-Hexyl-6-[4-(2-p-tolyl-acetyl)-piperazin-1-yl]-nicotinamid;
6-{4-[3-(3,4-Difluor-phenyl)-propionyl]-piperazin-1-yl}-*N*-(1,3-dimethylbutyl)-nicotinamid;
6-[4-(Naphthalin-2-carbonyl)-piperazin-1-yl]-*N*-pentyl-nicotinamid;
6-{4-[2-(4-Chlorphenyl)-propionyl]-piperazin-1-yl}-*N*-(3-dimethylamino-propyl)-nicotinamid;
*N*-(3-Dimethylamino-propyl)-6-[4-(2-phenyl-butyryl)-piperazin-1-yl]-nicotinamid;
*N*-(3-Dimethylamino-propyl)-6-[4-(2-p-tolyl-acetyl)-piperazin-1-yl]-nicotinamid;
*N*-(3-Dimethylamino-propyl)-6-[4-(2-o-tolyl-acetyl)-piperazin-1-yl]-nicotinamid;
*N*-(3-Butoxy-propyl)-6-{4-[3-(3,4-difluor-phenyl)-propionyl]-piperazin-1-yl}-nicotinarnid;
*N*-(3-Methoxy-propyl)-6-[4-(2-p-tolyl-acetyl)-piperazin-1-yl]-nicotinamid;
*N*-(2-Methyl-butyl)-6-[4-(2-p-tolyl-acetyl)-piperazin-1-yl]-nicotinamid;
*N*-(3-Butoxy-propyl)-6-[4-(2-o-tolyl-acetyt)-piperazin-1-yl]-nicotinamid; und
6-{4-[2-(4-Chlorphenyl)-propionyl]-piperazin-1-yl}-*N*-(3-isopropoxy-propyl)-nicotinamid.

78. Verbindung zur Verwendung wie in Anspruch 74, wobei die Verbindung der Formel (I) eine Verbindung ist, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
R³ ist gegebenenfalls substituiertes Aralkyl oder gegebenenfalls substituiertes Aralkenyl;
jedes R⁴ ist unabhängig Wasserstoff, Alkyl, Halogen oder Halogenalkyl; und
jedes R⁵ und R⁶ ist unabhängig Wasserstoff, Oxo, Alkyl, Halogen oder Halogenalkyl.

79. Verbindung zur Verwendung wie in Anspruch 78, wobei die Verbindung der Formel (I) eine Verbindung ist, worin:
m ist 1 oder 2;
n ist 1;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Heterocyclyl, gegebenenfalls substituiertem Heterocyclylalkyl, gegebenenfalls substituiertem Heteroaryl und
gegebenenfalls substituiertem Heteroarylalkyl;
R³ ist gegebenenfalls substituiertes Aralkyl oder gegebenenfalls substituiertes Aralkenyl;
jedes R⁴ ist unabhängig Wasserstoff, Alkyl, Halogen oder Halogenalkyl; und
R⁵ ist Wasserstoff, Oxo, Alkyl, Halogen oder Halogenalkyl; und
jedes R⁶ ist unabhängig Wasserstoff, Oxo, Alkyl, Halogen oder Halogenalkyl.

80. Verbindung zur Verwendung wie in Anspruch 79, wobei die Verbindung der Formel (I) ausgewählt ist aus der Gruppe bestehend aus den folgenden:
6-[4-(2-Phenyl-butyryl)-piperazin-1-yl]-*N*-(3-phenyl-propyl)-nicotinamid;
6-{4-[3-(3,4-Difluor-phenyl)-propionyl]-piperazin-1-yl}-*N*-phenethyl-nicotinamid;
*N*-[2-(3-Chlorphenyl)-ethyl]-6-[4-(2-o-tolyl-acetyl)-piperazin-1-yl]-nicotinamid;
6-{4-[2-(2-Chlor-6-fluor-phenyl)-acetyl]-piperazin-1-yl}-*N*-(3-phenyl-propyl)-nicotinamid;
*N*-(1-Methyl-3-phenyl-propyl)-6-[4-(2-p-tolyl-acetyl)-piperazin-1-yl]-nicotinamid;
*N*-[2-(3-Chlorphenyl)-ethyl]-6-[4-(2-phenyl-butyryl)-piperazin-1-yl]-nicotinamid;
6-{4-[2-(2-Chlor-6-fluor-phenyl)-acetyl]-piperazin-1-yl}-*N*-phenethyl-nicotinamid;
*N*-(3-Phenyl-propyl)-6-[4-(2-o-tolyl-acetyl)-piperazin-1-yl]-nicotinamid;
6-(4-[2-(2-Chlor-6-fluor-phenyl)-acetyl]-piperazin-1-yl)-*N*-[2-(3-chlor-phenyl)-ethyl]-nicotinamid;
6-{4-[2-(4-Chlorphenyl)-propionyl]-piperazin-1-yl}-*N*-(3-phenyl-propyl)-nicotinamid;
6-{4-[2-(4-Chlorphenyl)-propionyl]-piperazin-1-yl}-*N*-(1-methyl-3-phenyl-propyl)-nicotinamid;
*N*-[2-(3-Chlorphenyl)-ethyl]-6-{4-[2-(4-chlor-phenyl)-propionyl]-piperazin-1-yl}-nicotinamid;
*N*-[2-(3-Chlorphenyl)-ethyl]-6-[4-(2-p-tolyl-acetyl)-piperazin-1-yl]-nicotinamid;
*N*-Phenethyl-6-[4-(2-p-tolyl-acetyl)-piperazin-1-yl]-nicotinamid;
*N*-(3-Phenyl-propyl)-6-[4-(2-p-tolyl-acetyl)-piperazin-1-yl]-nicotinamid;
*N*-(1-Methyl-3-phenyl-propyl)-6-[4-(2-phenyl-butyryl)-piperazin-1-yl]-nicotinamid;
6-{4-[2-(4-Chlorphenyl)-propionyl]-piperazin-1-yl}-*N*-(4-phenyl-butyl)-nicotinamid;
*N*-(4-Phenyl-butyl)-6-[4-(2-phenyl-butyryl)-piperazin-1-yl]-nicotinamid;
6-{4-[2-(2-Chlor-6-fluor-phenyl)-acetyl]-piperazin-1-yl}-*N*-(1-methyl-3-phenyl-propyl)-nicotinamid;
6-{4-[3-(3,4-Difluor-phenyl)-propionyl]-piperazin-1-yl}-*N*-(3-phenyl-propyl)-nicotinamid;
*N*-Phenethyl-6-[4-(2-o-tolyl-acetyl)-piperazin-1-yl]-nicotinamid;
6-{4-[3-(3,4-Difluor-phenyl)-propionyl]-piperazin-1-yl}-*N*-(1-methyl-3-phenyl-propyl)-nicotinamid;
*N*-(4-Phenyl-butyl)-6-[4-(2-o-tolyl-acetyl)-piperazin-1-yl]-nicotinamid;
*N*-(3-Imidazol-1-yl-propyl)-6-[4-(2-phenyl-butyryl)-piperazin-1-yl]-nicotinamid;
6-{4-[2-(2-Chlor-6-fluor-phenyl)-acetyl]-piperazin-1-yl}-*N*-(4-phenyl-butyl)-nicotinamid;
6-{4-[2-(4-Chlorphenyl)-propionyl]-piperazin-1-yl}-*N*-[2-(3H-imidazol-4-yl)-ethyl]-nicotinamid;
6-{4-[2-(2-Chlor-6-fluor-phenyl)-acetyl]-piperazin-1-yl}-*N*-(3-imidazol-1-yl-propyl)-nicotinamid;
*N*-[2-(3H-Imidazol-4-yl)-ethyl]-6-[4-(2-p-tolyl-acetyl)-piperazin-1-yl]-nicotinamid;
6-{4-[2-(4-Chlorphenyl)-propionyl]-piperazin-1-yl}-*N*-(3-imidazol-1-yl-propyl)-nicotinamid;
*N*-(2-Ethylsulfanyl-ethyl)-6-[4-(2-phenyl-butyryl)-piperazin-1-yl]-nicotinamid;
*N*-(1-Methyl-3-phenyl-propyl)-6-[4-(2-o-tolyl-acetyl)-piperazin-1-yl]-nicotinamid;
6-{4-[3-(3,4-Difluor-phenyl)-propionyl]-piperazin-1-yl}-*N*-phenethyl-nicotinamid;
*N*-Phenethyl-6-[4-(2-phenyl-butyryl)-piperazin-1-yl]-nicotinamid;
*N*-(Tetrahydro-furan-2-ylmethyl)-6-[4-(2-p-tolyl-acetyl)-piperazin-1-yl]-nicotinamid;
6-{4-[2-(4-Chlorphenyl)-propionyl]-piperazin-1-yl}-*N*-(tetrahydro-furan-2-ylmethyl)-nicotinamid;
*N*-(4-Phenyl-butyl)-6-[4-(2-p-tolyl-acetyl)-piperazin-1-yl]-nicotinamid;
6-{4-[3-(3,4-Difluor-phenyl)-propionyl]-piperazin-1-yl}-*N*-(tetrahydro-furan-2-ylmethyl)-nicotinamid;
*N*-(Tetrahydro-furan-2-ylmethyl)-6-[4-(2-o-tolyl-acetyl)-piperazin-1-yl]-nicotinamid;
6-[4-(2-Phenyl-butyryl)-piperazin-1-yl]-*N*-(tetrahydro-furan-2-ylmethyl)-nicotinamid;
6-{4-[3-(3,4-Difluor-phenyl)-propionyl]-piperazin-1-yl}-*N*-[2-(3H-imidazol-4-yl)-ethyl]-nicotinamid;
*N*-[2-(3H-Imidazol-4-yl)-ethyl]-6-[4-(2-o-tolyl-acetyl)-piperazin-1-yl]-nicotinamid;
*N*-(3-Imidazol-1-yl-propyl)-6-[4-(2-o-tolyl-acetyl)-piperazin-1-yl]-nicotinamid;
6-(4-[3-(3,4-Difluor-phenyl)-propionyl]-piperazin-1-yl)-*N*-(3-imidazol-1-yl-propyl)-nicotinamid;
*N*-[2-(3H-Imidazol-4-yl)-ethyl]-6-[4-(2-phenyl-butyryl)-piperazin-1-yl]-nicotinamid;
*N*-(3-Imidazol-1-yl-propyl)-6-[4-(2-p-tolyl-acetyl)-piperazin-1-yl]-nicotinamid;
6-{4-[2-(2-Chlor-6-fluor-phenyl)-acetyl]-piperazin-1-yl}-*N*-(tetrahydro-furan-2-ylmethyl)-nicotinamid; und
6-{4-[2-(2-Chlor-6-fluor-phenyl)-acetyl]-piperazin-1-yl}-*N*-[2-(3H-imidazol-4-yl)-ethyl]-nicotinamid.

81. Verbindung zur Verwendung wie in Anspruch 45, wobei der Säuger ein Mensch ist.

82. Verbindung zur Verwendung wie in Anspruch 81, wobei die Krankheit oder der Zustand eine Krankheit oder ein Zustand ist, die bzw. der mit den Serumspiegeln von Triglycerid, VLDL, HDL, LDL, Gesamtcholesterin oder dem Prozess des reversen Cholesterintransports zusammenhängt.

83. Verbindung zur Verwendung wie in Anspruch 81, wobei die Krankheit oder der Zustand eine Krankheit oder ein Zustand ist, die bzw. der mit Serumtriglyceridspiegeln zusammenhängt.

84. Verbindung zur Verwendung wie in Anspruch 81, wobei die Krankheit oder der Zustand eine Krankheit oder ein Zustand ist, die bzw. der mit Serumcholesterinspiegeln zusammenhängt.

85. Verbindung zur Verwendung wie in Anspruch 81, wobei die Krankheit oder der Zustand Typ II-Diabetes ist.

86. Verbindung zur Verwendung wie in Anspruch 81, wobei die Krankheit oder der Zustand Fettleibigkeit ist.

87. Verbindung zur Verwendung wie in Anspruch 81, wobei die Krankheit oder der Zustand Dyslipidämie ist.

88. Verbindung zur Verwendung wie in Anspruch 81, wobei die Krankheit oder der Zustand das metabolische Syndrom ist.

89. Pharmazeutische Zusammensetzung umfassend einen pharmazeutisch verträglichen Exzipienten oder Träger und eine therapeutisch wirksame Menge einer Verbindung der Formel (I): 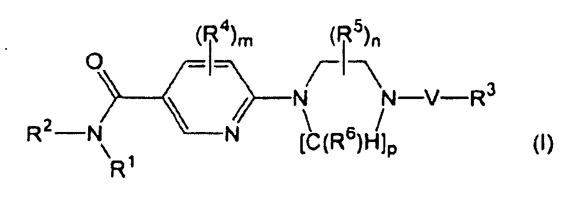 worin:
m ist 1 bis 3;
n ist 1, 2, 3 oder 4;
p ist 2 oder 3;
V ist -C(O)- oder -S(O)₂-;
R¹ ist Wasserstoff, Alkyl, Alkenyl, Aryl, Aralkyl oder Aralkenyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, Aralkyl, wobei der Arylrest gegebenenfalls substituiert sein kann, Aralkenyl, wobei der Arylrest gegebenenfalls substituiert sein kann, gegebenenfalls substituiertem Cycloalkylalkyl, Cycloalkylalkenyl, gegebenenfalls substituiertem Heterocyclyl, Heterocyclylalkyl, wobei der Heterocyclylrest gegebenenfalls substituiert sein kann, Heterocyclylalkenyl, gegebenenfalls substituiertem Heteroaryl, Heteroarylalkyl, wobei der Heteroarylrest gegebenenfalls substituiert sein kann, und Heteroarylalkenyl, wobei der Heteroarylrest gegebenenfalls substituiert sein kann;
R³ ist ausgewählt aus der Gruppe bestehend aus -R⁹-OR⁸, -R⁹-N(R⁸)₂, Alkenyl, gegebenenfalls substituiertem Aralkyl, gegebenenfalls substituiertem Aralkenyl, gegebenenfalls substituiertem Cycloalkyl, Cycloalkylalkyl, wobei der Cycloalkylrest gegebenenfalls substituiert sein kann, Cycloalkylalkenyl, gegebenenfalls substituiertem Heterocyclyl, Heterocyclylalkyl, wobei der Heterocyclylrest gegebenenfalls substituiert sein kann, Heterocyclylalkenyl, Heteroarylalkyl, wobei der Heteroarylrest gegebenenfalls substituiert sein kann, und Heteroarylalkenyl, wobei der Heteroarylrest gegebenenfalls substituiert sein kann;
jedes R⁴ ist unabhängig Wasserstoff, Alkyl, Alkenyl, Halogen, Halogenalkyl, Aryl oder -R⁹-OR⁸;
jedes R⁵ und R⁶ ist unabhängig Wasserstoff, Oxo, Alkyl, Alkenyl, Halogen, Halogenalkyl oder Aryl;
oder ein R⁵ und ein R⁶ können zusammen eine geradkettige oder verzweigte Alkylenbrücke bilden;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
jedes R⁹ ist unabhängig eine direkte Bindung oder eine gerade oder verzweigte Alkylen- oder Alkenylenkette; und
R¹⁰ ist Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
als ein einzelnes Stereoisomer, eine Mischung von Stereoisomeren, oder eine racemische Mischung davon von Stereoisomeren;
oder als ein pharmazeutisch verträgliches Salz, Solvat, Polymorph oder Prodrug, ausgewählt aus Ester- und Amidderivaten von funktionellen Hydroxyl-, Carboxy-, Mercapto- oder
Amino-Gruppen in den Verbindungen der Formel (I), davon; und
wobei es sich bei dem gegebenenfalls vorhandenen Substituenten um einen oder mehrere Substituenten handelt, die unabhängig ausgewählt sind aus der Gruppe bestehend aus Alkyl, Alkenyl, Halogen, Halogenalkyl, Halogenalkenyl, Cyano, Nitro, Aryl, Aralkyl, Cycloalkyl, Cycloalkylalkyl, Heterocyclyl, Heterocyclylalkyl, Heteroaryl, Heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (wobei t 1 oder 2 ist), -R⁹-S(O)ₜOR¹⁰ (wobei t 1 oder 2 ist), -R⁹-S(O)ₜR¹⁰ (wobei t 0, 1 oder 2 ist), und -R⁹-S(O)ₜN(R⁸)₂ (wobei t 1 oder 2 ist).

90. Pharmazeutische Zusammensetzung nach Anspruch 89, wobei die therapeutisch wirksame Menge der Verbindung der Formel (I) eine Menge ist, die zum Modulieren eines Lipidspiegels in einem Säuger wirksam ist, wenn sie dem Säuger verabreicht wird.

91. Pharmazeutische Zusammensetzung nach Anspruch 90, wobei das Lipid Triglycerid ist.

92. Pharmazeutische Zusammensetzung nach Anspruch 90, wobei das Lipid Cholesterin ist.

93. Pharmazeutische Zusammensetzung nach Anspruch 89, wobei die therapeutisch wirksame Menge der Verbindung der Formel (I) eine Menge ist, die zum Modulieren von HDL-Cholesterinspiegeln wirksam ist, wenn sie einem Säuger verabreicht wird.

94. Verbindung der Formel (I): 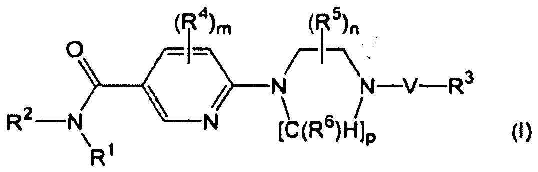 worin:
m ist 1, 2 oder 3;
n ist 1, 2, 3 oder 4;
p ist 2, 3 oder 4;
V ist -C(O)-, -S(O)- oder -S(O)₂-;
R¹ ist Wasserstoff, Alkyl, Alkenyl, Aryl, Aralkyl oder Aralkenyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, Aralkyl, wobei der Arylrest gegebenenfalls substituiert sein kann, Aralkenyl, wobei der Arylrest gegebenenfalls substituiert sein kann, gegebenenfalls substituiertem Cycloalkylalkyl, Cycloalkylalkenyl, gegebenenfalls substituiertem Heterocyclyl, Heterocyclylalkyl, wobei der Heterocyclylrest gegebenenfalls substituiert sein kann, Heterocyclylalkenyl, gegebenenfalls substituiertem Heteroaryl, Heteroarylalkyl, wobei der Heteroarylrest gegebenenfalls substituiert sein kann, und Heteroarylalkenyl, wobei der Heteroarylrest gegebenenfalls substituiert sein kann;
R³ ist Cyclopropyl, substituiert durch gegebenenfalls substituiertes Aryl oder gegebenenfalls substituiertes Heteroaryl;
jedes R⁴ ist unabhängig Wasserstoff, Alkyl, Alkenyl, Halogen, Halogenalkyl, Aryl, Cyano, Nitro, -R⁹-OR⁸, -R⁹-N(R⁸)₂ oder -S(O)ₜR¹⁰ (wobei t 0, 1 oder 2 ist);
jedes R⁵ und R⁶ ist unabhängig Wasserstoff, Oxo, Alkyl, Alkenyl, Halogen, Halogenalkyl oder Aryl;
oder ein R⁵ und ein R⁶ können zusammen eine geradkettige oder verzweigte Alkylenbrücke bilden;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
jedes R⁹ ist unabhängig eine direkte Bindung oder eine gerade oder verzweigte Alkylen- oder Alkenylenkette; und
R¹⁰ ist Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
als ein einzelnes Stereoisomer, eine Mischung von Stereoisomeren, eine racemische Mischung davon von Stereoisomeren, oder als ein Tautomer;
oder als ein pharmazeutisch verträgliches Salz, Solvat, Polymorph oder Prodrug, ausgewählt aus Ester und Amidderivaten von funktionellen Hydroxyl-, Carboxy-, Mercapto- oder
Amino-Gruppen in den Verbindungen der Formel (I), davon; und
wobei es sich bei dem gegebenenfalls vorhandenen Substituenten um einen oder mehrere Substituenten handelt, die unabhängig ausgewählt sind aus der Gruppe bestehend aus Alkyl, Alkenyl, Halogen, Halogenalkyl, Halogenalkenyl, Cyano, Nitro, Aryl, Aralkyl, Cycloalkyl, Cycloalkylalkyl, Heterocyclyl, Heterocyclylalkyl, Heteroaryl, Heteroarylalkyl, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (wobei t 1 oder 2 ist), -R⁹-S(O)ₜOR¹⁰ (wobei t 1 oder 2 ist), -R⁹-S(O)ₜR¹⁰ (wobei t 0, 1 oder 2 ist), und -R⁹-S(O)ₜN(R⁸)₂ (wobei t 1 oder 2 ist).

95. Verbindung nach Anspruch 94, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus Wasserstoff, -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Aryl, gegebenenfalls substituiertem Aralkyl, gegebenenfalls substituiertem Aralkenyl, gegebenenfalls substituiertem Cycloalkylalkyl, Cycloalkylalkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
jedes R⁴ ist unabhängig Wasserstoff, Alkyl, Alkenyl, Halogen, Halogenalkyl, Aryl oder -R⁹-OR⁸;
jedes R⁵ und R⁶ ist unabhängig Wasserstoff, Oxo, Alkyl, Alkenyl, Halogen, Halogenalkyl oder Aryl;
jedes R⁷ ist unabhängig eine gerade oder verzweigte Alkylen- oder Alkenylenkette;
jedes R⁸ ist unabhängig Wasserstoff, Alkyl, Alkenyl, Halogenalkyl, Cycloalkyl, Cycloalkylalkyl, Aryl, Aralkyl, Heterocyclyl, Heterocyclylalkyl, Heteroaryl oder Heteroarylalkyl;
R⁹ ist eine direkte Bindung oder eine gerade oder verzweigte Alkylenkette; und
R¹⁰ ist Alkyl, Aryl oder Aralkyl.

96. Verbindung nach Anspruch 95, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus gegebenenfalls substituiertem Aryl, gegebenenfalls substituiertem Aralkyl, gegebenenfalls substituiertem Aralkenyl, gegebenenfalls substituiertem Heterocyclyl, gegebenenfalls substituiertem Heterocyclylalkyl, Heterocyclylalkenyl, gegebenenfalls substituiertem Heteroaryl, gegebenenfalls substituiertem Heteroarylalkyl und gegebenenfalls substituiertem Heteroarylalkenyl;
jedes R⁴ ist unabhängig Wasserstoff, Alkyl, Halogen oder Halogenalkyl; und
jedes R⁵ und R⁶ ist unabhängig Wasserstoff, Oxo, Alkyl, Halogen oder Halogenalkyl.

97. Verbindung nach Anspruch 96, worin:
m ist 1:
n ist 1;
p ist 2;
V ist -C(O)-;
R¹ ist Wasserstoff oder Alkyl;
R² ist gegebenenfalls substituiertes Aralkyl, gegebenenfalls substituiertes Heteroarylalkyl oder gegebenenfalls substituiertes Heterocyclylalkyl;
R³ ist Cyclopropyl, substituiert durch Phenyl;
jedes R⁴ ist Wasserstoff, Alkyl, Halogen oder Halogenalkyl;
jedes R⁵ ist Wasserstoff, Oxo, Alkyl, Halogen oder Halogenalkyl; und
jedes R⁶ ist Wasserstoff.

98. Verbindung nach Anspruch 97, ausgewählt aus der Gruppe bestehend aus den folgenden:
6-[4-(2-Phenyl-cyclopropancarbonyl)-piperazin-1-yl]-*N*-(3-phenyl-propyl)-nicotinamid;
*N*-(4-Phenyl-butyl)-6-[4-(2-phenyl-cyclopropancarbonyl)-piperazin-1-yl]-nicotinamid;
*N*-(1-Methyl-3-phenyl-propyl)-6-[4-(2-phenyl-cyclopropancarbonyl)-piperazin-1-yl]-nicotinamid;
*N*-Phenethyl-6-[4-(2-phenyl-cyclopropancarbonyl)-piperazin-1-yl]-nicotinamid;
6-[4-(2-Phenyl-cyclopropancarbonyl)-piperazin-1-yl]-*N*-(tetrahydro-furan-2-ylmethyl)-nicotinamid;
*N*-[2-(3H-Imidazol-4-yl)-ethyl]-6-[4-(2-phenyl-cyclopropancarbonyl)-piperazin-1-yl]-nicotinamid; und
*N*-(3-Imidazol-1-yl-propyl)-6-[4-(2-phenyl-cyclopropancarbonyl)-piperazin-1-yl]-nicotinamid.

99. Verbindung nach Anspruch 95, worin:
m ist 1 oder 2;
n ist 1 oder 2;
p ist 2;
V ist -C(O)-;
R¹ ist Wasserstoff oder Alkyl;
R² ist ausgewählt aus der Gruppe bestehend aus -R⁷-OR⁸, -R⁷-N(R⁸)₂, -R⁷-S(O)ₜR¹⁰ (wobei t 0, 1 oder 2 ist), Alkyl, Alkenyl, gegebenenfalls substituiertem Cycloalkylalkyl oder Cycloalkylalkenyl;
jedes R⁴ ist unabhängig Wasserstoff, Alkyl, Halogen oder Halogenalkyl;
jedes R⁵ und R⁶ ist unabhängig Wasserstoff, Oxo, Alkyl, Halogen oder Halogenalkyl;
jedes R⁷ ist eine gerade oder verzweigte Alkylenkette;
jedes R⁸ ist unabhängig Wasserstoff, Alkyl, Halogenalkyl, Cycloalkyl, Cycloalkylalkyl, Aryl
und Aralkyl; und
R¹⁰ ist Alkyl, Aryl oder Aralkyl.

100. Verbindung nach Anspruch 99, worin:
m ist 1;
n ist 1;
p ist 2;
V ist -C(O)-;
R¹ ist Wasserstoff oder Alkyl;
R² ist ausgewählt aus der Gruppe bestehend aus Alkyl, -R⁷-OR⁸, -R⁷-N(R⁸)₂ oder -R⁷-S(O)ₜR¹⁰ (wobei t 0 ist);
R³ ist Cyclopropyl, substituiert durch Phenyl;
R⁴ ist Wasserstoff;
R⁵ ist Wasserstoff;
jedes R⁶ ist Wasserstoff;
R⁷ ist eine gerade oder verzweigte Alkylenkette;
R⁸ ist Wasserstoff oder Alkyl; und
R¹⁰ ist Alkyl, Aryl oder Aralkyl.

101. Verbindung nach Anspruch 100, ausgewählt aus der Gruppe bestehend aus den folgenden:
*N-*(3-Ethoxy-propyl)-6-[4-(2-phenyl-cyclopropancarbonyl)-piperazin-1-yl]-nicotinamid;
*N*-(2-Ethylsulfanyl-ethyl)-6-[4-(2-phenyl-cyclopropancarbonyl)-piperazin-1-yl]-nicotinamid;
*N*-(1,3-Dimethylbutyl)-6-[4-(2-phenyl-cyclopropancarbonyl)-piperazin-1-yl]-nicotinamid;
*N*-(3-Methyl-butyl)-6-[4-(2-phenyl-cyclopropancarbonyl)-piperazin-1-yl]-nicotinamid;
*N*-(3-Methoxy-propyl)-6-[4-(2-phenyl-cyclopropancarbonyl)-piperazin-1-yl]-nicotinamid;
*N*-(3-Butoxy-propyl)-6-[4-(2-phenyl-cyclopropancarbonyl)-piperazin-1-yl]-nicotinamid;
*N*-Pentyl-6-[4-(2-phenyl-cyclopropancarbonyl)-piperazin-1-yl]-nicotinamid;
*N*-(3-Dimethylamino-propyl)-6-[4-(2-phenyl-cyclopropancarbonyl)-piperazin-1-yl)-nicotinamid;
*N*-(3-Isopropoxy-propyl)-6-[4-(2-phenyl-cyclopropancarbonyl)-piperazin-1-yl]-nicotinamid;
*N*-(1-Methyl-butyl)-6-[4-(2-phenyl-cyclopropancarbonyl)-piperazin-1-yl]-nicotinamid;
*N*-Butyl-6-[4-(2-phenyl-cyclopropancarbonyl)-piperazin-1-yl]-nicotinamid;
*N*-Hexyl-6-[4-(2-phenyl-cyclopropancarbonyl)-piperazin-1-yl]-nicotinamid; und
*N*-(2-Methyl-butyl)-6-[4-(2-phenyl-cyclopropancarbonyl)-piperazin-1-yl]-nicotinamid.

## Revendications

1. Utilisation d'un composé de formule (I): 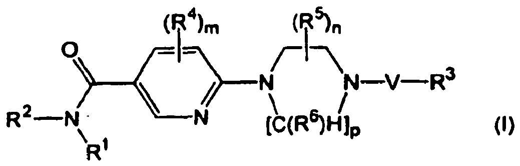 où:
m est 1, 2 ou 3;
n est 1, 2, 3 ou 4;
p est 2, 3 ou 4;
V est -C(O)-, -S(O)- ou -S(O)₂-;
R¹ est l'hydrogène, alkyle, alcényle, aryle, aralkyle, aralcényle ou cycloalkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle, où le radical aryle peut être éventuellement substitué, aralcényle, où le radical aryle peut être éventuellement substitué, cycloalkyle éventuellement substitué, cycloalkylalkyle éventuellement substitué, cycloalkylalcényle, hétérocyclyle éventuellement substitué, hétérocyclylalkyle, où le radical hétérocyclyle peut être éventuellement substitué, hétérocyclylalcényle, hétéroaryle éventuellement substitué, hétéroarylalkyle, où le radical hétéroaryle peut être éventuellement substitué et hétéroarylalcényle, où le radical hétéroaryle peut être éventuellement substitué;
R³ est choisi dans le groupe consistant en l'hydrogène, -R⁹-OR⁸, -R⁹-N(R⁸)₂, alkyle, alcényle, aryle éventuellement substitué, aralkyle, où le radical aryle peut être éventuellement substitué, aralcényle, où le radical aryle peut être éventuellement substitué, cycloalkyle éventuellement substitué, cycloalkylalkyle éventuellement substitué, cycloalkylalcényle, hétérocyclyle éventuellement substitué, hétérocyclylalkyle, où le radical hétérocyclyle peut être éventuellement substitué, hétérocyclylalcényle, hétéroaryle éventuellement substitué, hétéroarylalkyle, où le radical hétéroaryle peut être éventuellement substitué et hétéroarylalcényle, où le radical hétéroaryle peut être éventuellement substitué;
chaque R⁴ est indépendamment l'hydrogène, alkyle, alcényle, halo, haloalkyle, aryle, cyano, nitro, -R⁹-OR⁸, -R⁹-N(R⁸)₂ ou -S(O)ₜR¹⁰ (où t est 0, 1 ou 2);
chaque R⁵ et R⁶ est indépendamment l'hydrogène, oxo, alkyle, alcényle, halo, haloalkyle ou aryle ;
ou un R⁵ et un R⁶ peuvent former ensemble un pont alkylène linéaire ou ramifié;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
chaque R⁹ est indépendamment une liaison directe ou une chaîne alkylène ou alcénylène linéaire ou ramifiée; et
R¹⁰ est alkyle, alcényle, haloalkyle, cycloalkyle, cycloalkylalkyle, aryle, aralkyle, hétérocyclyle, hétérocyclylalkyle, hétéroaryle ou hétéroarylalkyle;
sous forme d'un seul stéréoisomère, d'un mélange de stéréoisomères, d'un mélange racémique de stéréoisomères, ou sous forme de tautomère;
ou sous forme d'un sel pharmaceutiquement acceptable, solvate, polymorphe ou promédicament, choisi parmi les dérivés esters et amides de groupes fonctionnels hydroxyle, carboxy, mercapto ou amino dans les composés de formule (I), de celui-ci pour la fabrication d'un médicament pour traiter une maladie ou affection à médiation par la stéaroyl-CoA désaturase (SCD) chez un mammifère, où la maladie ou affection est choisie dans le groupe consistant en le diabète de type II, la tolérance au glucose dégradée, la résistance à l'insuline, l'hypertension, l'obésité, l'hypertriglycéridémie, les HDL basses, la lipidémie, la dyslipidémie, la micro-albuminémie, l'hyperuricémie, l'hyperleptinémie, le syndrome métabolique et toute combinaison de celles-ci ; et
où le substituant facultatif est un ou plusieurs substituants choisis indépendamment dans le groupe consistant en alkyle, alcényle, halo, haloalkyle, haloalcényle, cyano, nitro, aryle, aralkyle, cycloalkyle, cycloalkylalkyle, hétérocylyle, hétérocyclylalkyle, hétéroaryle, hétéroarylalkyle, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (où t est 1 ou 2), -R⁹-S(O)ₜOR¹⁰ (où t est 1 ou 2), -R⁹-S(O)ₜR¹⁰ (où t est 0, 1 ou 2), et -R⁹-S(O)ₜN(R⁸)₂ (où t est 1 ou 2).

2. Utilisation du composé selon la revendication 1 où le composé de formule (I) est un composé où:
m est 1 ou 2;
n est 1 ou 2;
p est 2 ou 3;
V est -C(O)- ou -S(O)₂-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle éventuellement substitué, aralcényle éventuellement substitué, cycloalkyle éventuellement substitué, cycloalkylalkyle éventuellement substitué, cycloalkylalcényle hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué, où les groupes éventuellement substitués sont définis comme dans la revendication 1;
R³ est alkyle, alcényle ou -R⁹-N(R⁸)₂;
chaque R⁴ est indépendamment l'hydrogène, alkyle, alcényle, halo, haloalkyle, aryle ou -R⁹-OR⁸;
chaque R⁵ et R⁶ est indépendamment l'hydrogène, oxo, alkyle, alcényle, halo, haloalkyle ou aryle;
ou un R⁵ et un R⁶ peuvent former ensemble un pont alkylène linéaire ou ramifié;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
chaque R⁹ est indépendamment une liaison directe ou une chaîne alkylène linéaire ou ramifiée;
et
R¹⁰ est alkyle, aryle ou aralkyle.

3. Utilisation du composé selon la revendication 2 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, alcényle, cycloalkyle éventuellement substitué, cycloalkylalkyle ou cycloalkylalcényle éventuellement substitué, où les groupes éventuellement substitués sont définis comme dans la revendication 1;
R³ est alkyle;
chaque R⁴ est indépendamment l'hydrogène, alkyle, halo, ou haloalkyle;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle; et
R¹⁰ est alkyle, aryle ou aralkyle.

4. Utilisation du composé selon la revendication 3 où le composé de formule (I) est un composé où:
m est 1;
n est 1;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2) ou alkyle;
R³ est alkyle;
R⁴ est l'hydrogène;
R⁵ est l'hydrogène;
chaque R⁶ est l'hydrogène;
R⁷ est une chaîne alkylène linéaire ou ramifiée;
R⁸ est l'hydrogène ou alkyle; et
R¹⁰ est alkyle, aryle ou aralkyle.

5. Utilisation du composé selon la revendication 4 où le composé de formule (I) est choisi dans le groupe consistant en les suivants :
*N*-(3-éthoxy-propyl)-6-[4-(2-éthylbutyryl)-pipérazin-1-yl]-nicotinamide;
*N*-(3-éthoxy-propyl)-6-(4-pentanoyl-pipérazin-1-yl)-nicotinamide;
*N*-(3-éthoxy-propyl)-6-[4-(3-méthyl-pentanoyl)-pipérazin-1-yl]-nicotinamide;
*N*-(3-butoxy-propyl)-6-(4-pentanoyl-pipérazin-1-yl)-nicotinamide;
*N*-(3-méthyl-butyl)-6-[4-(3-méthyl-pentanoyl)-pipérazin-1-yl]-nicotinamide;
6-(4-(3-méthyl-pentanoyl)-pipérazin-1-yl]-*N*-pentyl-nicotinamide;
*N*-butyl-6-[4-(3-méthyl-pentanoyl)-pipérazin-1-yl]-nicotinamide;
*N*-butyl-6-[4-(2-éthylbutyryl)-pipérazin-1-yl]-nicotinamide;
*N*-(3-méthyl-butyl)-6-(4-pentanoyl-pipérazin-1-yl)-nicotinamide;
*N*-(2-éthylsulfanyl-éthyl)-6-(4-pentanoyl-pipérazin-1-yl)-nicotinamide;
*N*-(3-méthoxy-propyl)-6-[4-(3-méthyl-pentanoyl)-pipérazin-1-yl]-nicotinamide;
6-(4-pentanoyl-pipérazin-1-yl)-*N*-pentyl-nicotinamide;
*N*-hexyl-6-[4-(3-méthyl-pentanoyl)-pipérazin-1-yl]-nicotinamide;
*N*-(1,3-diméthylbutyl)-6-[4-(3-méthyl-pentanoyl)-pipérazin-1-yl]-nicotinamide;
*N*-(1-méthyl-butyl)-6-(4-pentanoyl-pipérazin-1-yl)-nicotinamide;
*N*-(3-diméthylamino-propyl)-6-[4-(2-éthylbutyryl)-pipérazin-1-yl]-nicotinamide;
*N*-(3-méthoxy-propyl)-6-(4-pentanoyl-pipérazin-1-yl)-nicotinamide;
*N*-(1,3-diméthylbutyl)-6-(4-pentanoyl-pipérazin-1-yl)-nicotinamide;
*N*-(2-méthyl-butyl)-6-(4-pentanoyl-pipérazin-1-yl)-nicotinamide;
*N*-(3-butoxy-propyl)-6-[4-(2-éthylbutyryl)-pipérazin-1-yl]-nicotinamide;
6-[4-(2-éthylbutyryl)-pipérazin-1-yl]-*N*-(3-méthylbutyl)-nicotinamide;
*N*-butyl-6-(4-pentanoyl-pipérazin-1-yl)-nicotinamide;
6-[4-(2-éthylbutyryl)-pipérazin-1-yl]-*N*-(2-méthylbutyl)-nicotinamide;
*N*-(3-isopropoxy-propyl)-6-(4-pentanoyl-pipérazin-1-yl)-nicotinamide;
*N*-(2-éthylsulfanyl-éthyl)-6-[4-(3-méthyl-pentanoyl)-pipérazin-1-yl]-nicotinamide ;
6-[4-(2-éthylbutyryl)-pipérazin-1-yl]-*N*-(2-éthylsulfanyl-éthyl)-nicotinamide ;
6-[4-(2-éthylbutyryl)-pipérazin-1-yl]-*N*-hexyl-nicotinamide;
6-[4-(2-éthylbutyryl)-pipérazin-1-yl]-*N*-(1-méthylbutyl)-nicotinamide;
*N*-(2-méthyl-butyl)-6-[4-(3-méthyl-pentanoyl)-pipérazin-1-yl]-nicotinamide *N*-(1,3-diméthylbutyl)-6-[4-(2-éthylbutyryl)-pipérazin-1-yl]-nicotinamide;
6-[4-(2-éthylbutyryl)-piperazin-1-yl]-*N*-pentyl-nicotinamide;
*N*-(3-isopropoxy-propyl)-6-[4-(3-méthyl-pentanoyl)-pipérazin-1-yl]-nicotinamide;
*N*-(3-diméthylamino-propyl)-6-[4-(3-méthyl-pentanoyl)-pipérazin-1-yl]-nicotinamide;
6-[4-(2-éthylbutyryl)-pipérazin-1-yl]-*N*-(3-méthoxy-propyl)-nicotinamide;
6-[4-(2-éthylbutyryl)-pipérazin-1-yl]-*N*-(3-isopropoxy-propyl)-nicotinamide;
*N*-(3-butoxy-propyl)-6-[4-(3-méthyl-pentanoyl)-pipérazin-1-yl]-nicotinamide;
*N*-(1-méthyl-butyl)-6-[4-(3-méthyl-pentanoyl)-pipérazin-1-yl]-nicotinamide;
*N*-hexyl-6-(4-pentanoyl-pipérazin-1-yl)-nicotinamide; et
*N*-(3-diméthylamino-propyl)-6-(4-pentanoyl-pipérazin-1-yl)-nicotinamide.

6. Utilisation du composé selon la revendication 2 où le composé de formule (I) est un composé où:
m est 1 ou 2;
n est 1 ou 2;
p est 2 ou 3;
V est -C(O)- ou -S(O)₂- ;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué, où les groupes éventuellement substitués sont définis comme dans la revendication 1;
R³ est alkyle ou -R⁷-N(R⁸)₂;
chaque R⁴ est indépendamment l'hydrogène, alkyle, halo, ou haloalkyle; et
chaque R⁵ et R⁶ est indépendamment l'hydrogène, oxo, alkyle, alcényle, halo, haloalkyle ou aryle;
ou un R⁵ et un R⁶ peuvent former ensemble un pont alkylène linéaire ou ramifié;
R⁷ est une liaison directe; et
chaque R⁸ est indépendamment l'hydrogène ou alkyle.

7. Utilisation du composé selon la revendication 6 où le composé de formule (I) est un composé où:
m est 1;
n est 1 ;
p est 2 ou 3;
V est -C(O)- ou -S(O)₂-;
R¹ est l'hydrogène ou alkyle;
R² est choisi dans le groupe consistant en aryle éventuellement substitué, aralkyle éventuellement substitué, hétérocyclyle éventuellement substitué, hétérocyclylalkyle éventuellement substitué, hétéroaryle éventuellement substitué et hétéroarylalkyle éventuellement substitué, où les groupes éventuellement substitués sont définis comme dans la revendication 1;
R³ est alkyle ou -R⁷-N(R⁸)₂;
R⁴ est l'hydrogène, alkyle, halo ou haloalkyle; et
chaque R⁵ et R⁶ est indépendamment l'hydrogène, oxo, alkyle, halo ou haloalkyle;
ou un R⁵ et un R⁶ peuvent former ensemble un pont méthylène;
R⁷ est une liaison directe; et
chaque R⁸ est indépendamment l'hydrogène ou alkyle.

8. Utilisation du composé selon la revendication 7 où le composé de formule (I) est choisi dans le groupe consistant en les suivants:
6-[4-(2-éthylbutyryl)-pipérazin-1-yl]-*N*-(3-phényl-propyl)-nicoiinamide;
6-[4-(4-méthyl-hexanoyl)-pipérazin-1-yl]-*N*-(3-phényl-propyl)-nicotinamide;
*N*-(2-(3-chlorophényl)-1-méthyléthyl]-6-[4-(2-éthylbutyryl)-pipérazin-1-yl]-nicotinamide ;
*N*-[2-(3-chlorophényl)-éthyl]-6-[4-(3-méthyl-pentanoyl)-pipérazin-1-yl]-nicotinamide;
*N*-[2-(3-chlorophényl)-éthyl]-6-[4-(2-éthylbutyryl)-pipérazin-1-yl]-nicotinamide;
6-(4-pentanoyl-pipérazin-1-yl)-*N*-(4-phényl-butyl)-nicotinamide;
*N*-[2-(3-chlorophényl)-éthyl]-6-(4-pentanoyl-pipérazin-1-yl)-nicotinamide;
6-[4-(2-éthylbutyryl)-pipérazin-1-yl]-*N*-phénéthyl-nicotinamide;
6-[4-(3-méthyl-pentanoyl)-pipérazin-1-yl]-*N*-phénéthyl-nicotinamide;
6-(4-pentanoyl-pipérazin-1-yl)-*N*-(3-phényl-propyl)-nicotinamide;
*N*-(1-méthyl-3-phényl-propyl)-6-(4-pentanoyl-pipérazin-1-yl)-nicotinamide;
6-[4-(3-méthyl-pentanoyl)-pipérazin-1-yl]-*N*-(tétrahydro-furan-2-ylméthyl)-nicotinamide;
6-[4-(2-éthylbutyryl)-pipérazin-1-yl]-*N*-(tétrahydrofuran-2-ylméthyl)-nicotinamide;
6-(4-pentanoyl-pipérazin-1-yl)-*N*-(tétrahydro-furan-2-ylméthyl)-nicotinamide;
6-[4-(2-éthylbutyryl)-pipérazin-1-yl]-*N*-(4-phényl-butyl)-nicotinamide;
*N*-(3-imidazol-1-yl-propyl)-6-(4-pentanoyl-pipérazin-1-yl)-nicotinamide;
6-[4-(2-éthylbutyryl)-pipérazin-1-yl]-*N*-(3-imidazol-1-yl-propyl)-nicotinamide;
*N*-[2-(3H-imidazol-4-yl)-éthyl]-6-(4-pentanoyl-pipérazin-1-yl)-nicotinamide;
*N*-[2-(3H-imidazol-4-yl)-éthyl]-6-[4-(3-méthyl-pentanoyl)-pipérazin-1-yl]-nicotinamide;
6-(4-pentanoyl-pipérazin-1-yl)-*N*-phénéthyl-nicotinamide;
6-[4-(2-éthylbutyryl)-pipérazin-1-yl]-*N*-[2-(3H-imidazol-4-yl)-éthyl]-nicotinamide;
*N*-(3-imidazol-1-yl-propyl)-6-[4-(3-méthyl-pentanoyl)-pipérazin-1-yl]-nicotinamide;
6-[4-(2-éthylbutyryl)-(1,4]diazépan-1-yl]-*N*-(3-phényl-propyl)-nicotinamide;
6-[4-(2-éthylbutyryl)-3-méthyl-pipérazin-1-yl]-*N*-(3-phényl-propyl)-nicotinamide;
6-[5-(2-éthylbutyryl)-2,5-diaza-bicyclo[2.2.1]hept-2-yl]-*N*-(3-phényl-propyl)-nicotinamide;
6-[4-(butane-1-sulfonyl)-pipérazine-1-yl]-*N*-(3-phényl-propyl)-nicotinamide;
butylamide d'acide 4-[5-(3-phényl-propylcarbamoyl)-pyridin-2-yl]-pipérazine-1-carboxylique.

9. Utilisation du composé selon la revendication 1 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle éventuellement substitué, aralcényle éventuellement substitué, cycloalkyle éventuellement substitué, cycloalkylalkyle éventuellement substitué, cycloalkylalcényle, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué, où les groupes éventuellement substitués sont définis comme dans la revendication 1;
R³ est cycloalkyle éventuellement substitué, cycloalkylalkyle éventuellement substitué, ou cycloalkylalcényle, où les groupes éventuellement substitués sont définis comme dans la revendication 1;
chaque R⁴ est indépendamment l'hydrogène, alkyle, alcényle, halo, haloalkyle, aryle ou -R⁹-OR⁸;
chaque R⁵ et R⁶ est indépendamment l'hydrogène, oxo, alkyle, alcényle, halo, haloalkyle ou aryle;
chaque R⁷ est indépendamment une chaîne alkylène ou alcénylène linéaire ou ramifiée; chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle; R⁹ est une liaison directe ou une chaîne alkylène linéaire ou ramifiée; et R¹⁰ est alkyle, aryle ou aralkyle.

10. Utilisation du composé selon la revendication 9 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, alcényle, cycloalkyle éventuellement substitué, cycloalkylalkyle éventuellement substitué ou cycloalkylalcényle, où les groupes éventuellement substitués sont définis comme dans la revendication 1;
R³ est cycloalkyle éventuellement substitué ou cycloalkylalkyle éventuellement substitué, où les groupes éventuellement substitués sont définis comme dans la revendication 1;
chaque R⁴ est indépendamment l'hydrogène, alkyle, halo, ou haloalkyle;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle; et
R¹⁰ est alkyle, aryle ou aralkyle.

11. Utilisation du composé selon la revendication 10 où le composé de formule (I) est un composé où:
m est 1;
n est 1 ;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0 à 2) ou alkyle;
R³ est cycloalkyle éventuellement substitué ou cycloalkylalkyle éventuellement substitué;
R⁴ est l'hydrogène;
R⁵ est l'hydrogène;
chaque R⁶ est l'hydrogène;
R⁷ est une chaîne alkylène linéaire ou ramifiée;
R⁸ est l'hydrogène ou alkyle; et
R¹⁰ est alkyle, aryle ou aralkyle.

12. Utilisation du composé selon la revendication 11 où le composé de formule (I) est choisi dans le groupe consistant en les suivants:
6-[4-(2-cyclohexyl-acétyl)-pipérazin-1-yl)-*N*-(3-éthoxy-propyl)-nicotinamide;
*N*-butyl-6-[4-(2-cyclohexyl-acétyl)-pipérazin-1-yl]-nicotinamide;
6-[4-(3-cyclohexyl-propionyl)-pipérazin-1-yl]-*N*-(3-méthylbutyl)-nicotinamide;
6-[4-(3-cyclohexyl-propionyl)-pipérazin-1-yl]-*N*-(3-méthoxy-propyl)-nicotinamide;
*N*-pentyl-6-[4-(3-trifluorométhyl-benzoyl)-pipérazin-1-yl]-nicotinamide;
6-[4-(2-cyclohexyl-acétyl)-pipérazin-1-yl]-*N*-(2-méthylbutyl)-nicotinamide;
6-[4-(3-cyclohexyl-propionyl)-pipérazin-1-yl]-*N*-(3-isopropoxy-propyl)-nicotinamide ;
6-[4-(2-cyclohexyl-acétyl)-pipérazin-1-yl]-*N*-(1,3-diméthylbutyl)-nicotinamide;
6-[4-(2-cyclohexyl-acétyl)-pipérazin-1-yl]-*N*-hexyl-nicotinamide;
6-[4-(2-cyclohexyl-acétyl)-pipérazin-1-yl]-*N*-(1-méthylbutyl)-nicotinamide;
6-[4-(2-cyclohexyl-acétyl)-pipérazin-1-yl]-*N*-(3-méthylbutyl)-nicotinamide;
6-[4-(2-cyclohexyl-acétyl)-pipérazin-1-yl]-*N*-(2-éthylsulfanyl-éthyl)-nicotinamide;
N-(3-butoxy-propyl)-6-[4-(2-cyclohexyl-acétyl)-pipérazin-1-yl]-nicotinamide;
N-butyl-6-[4-(3-cyclohexyl-propionyl)-pipérazin-1-yl]-nicotinamide;
6-[4-(2-cyclohexyl-acétyl)-pipérazin-1-yl]-*N*-pentyl-nicotionamide;
6-[4-(3-cyclohexyl-propionyl)-pipérazin-1-yl]-*N*-(2-éthylsulfanyl-éthyl)-nicotinamide;
6-[4-(2-cyclohexyl-acétyl)-pipérazin-1-yl]-*N*-(3-méthoxy-propyl)-nicotinamide;
6-[4-(3-cyclohexyl-propionyl)-pipérazin-1-yl]-*N*-(1-méthylbutyl)-nicotinamide;
6-[4-(2-cyclohexyl-acétyl)-pipérazin-1-yl]-*N*-(3-isopropoxy-propyl)-nicotinamide;
6-[4-(3-cyclohexyl-propionyl)-pipérazin-1-yl]-*N*-(3-éthoxy-propyl)-nicotinamide;
6-[4-(3-cyclohexyl-propionyl)-pipérazin-1-yl]-*N*-(1,3-diméthylbutyl)-nicotinamide;
6-[4-(3-cyclohexyl-propionyl)-pipérazin-1-yl]-*N*-(2-méthylbutyl)-nicotinamide;
6-[4-(3-cyclohexyl-propionyl)-pipérazin-1-yl]-*N*-hexyl-nicotinamide;
6-[4-(3-cyclohexyl-propionyl)-pipérazin-1-yl]-*N*-pentyl-nicotinamide;
6-[4-(3-cyclohexyl-propionyl)-pipérazin-1-yl]-*N*-(3-diméthylamino-propyl)-nicotinamide;
*N*-(3-éthoxy-propyl)-6-[4-(2-phényl-cyclopropanecarbonyl)-pipérazin-1-yl]-nicotinamide;
*N*-(2-éthylsulfanyl-éthyl)-6-[4-(2-phényl-cyclopropanecarbonyl)-pipérazin-1-yl]-nicotinamide;
*N*-(1,3-diméthylbuty1)-6-[4-(2-phényl-cyclopropanecarbonyl)-pipérazin-1-yl]-nicotinamide;
*N*-(3-méthyl-butyl)-6-[4-(2-phényl-cyclopropanecarbonyl)-pipérazin-1-yl]-nicotinamide;
*N*-(3-méthoxy-propyl)-6-[4-(2-phényl-cyclopropanecarbonyl)-pipérazin-1-yl]-nicotinamide;
*N*-(3-butoxy-propyl)-6-[4-(2-phényl-cyclopropanecarbonyl)-pipérazin-1-yl)-nicotinamide;
*N*-pentyl-6-[4-(2-phényl-cyclopropanecarbonyl)-pipérazin-1-yl)-nicotinamide;
*N*-(3-diméthylamino-propyl)-6-[4-(2-phényl-cyclopropanecarbonyl)-pipérazin-1-yl]-nicotinamide;
*N*-(3-isopropoxy-propyl)-6-[4-(2-phényl-cyclopropanecarbonyl)-pipérazin-1-yl]-nicotinamide ;
*N*-(1-méthyl-butyl)-6-[4-(2-phényl-cyclopropanecarbonyl)-pipérazin-1-yl]-nicotinamide ;
*N*-butyl-6-[4-(2-phényl-cyclopropanecarbonyl)-pipérazin-1-yl]-nicotinamide;
*N*-hexyl-6-[4-(2-phényl-cyclopropanecarbonyl)-pipérazin-1-yl]-nicotinamide;
*N*-(2-méthyl-butyl)-6-[4-(2-phényl-cyclopropanecarbonyl)-pipérazin-1-yl]-nicotinamide; et
6-[4-(2-cyclohexyl-acétyl)-pipérazin-1-yl]-*N*-(3-diméthylamino-propyl)-nicotinamide.

13. Utilisation du composé selon la revendication 9 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué, où les groupes éventuellement substitués sont comme défini dans la revendication 1;
R³ est cycloalkyle éventuellement substitué ou cycloalkylalkyle éventuellement substitué, où les groupes éventuellement substitués sont comme défini dans la revendication 1;
chaque R⁴ est indépendamment l'hydrogène, alkyle, halo, ou haloalkyle; et
chaque R⁵ et R⁶ est indépendamment l'hydrogène, oxo, alkyle, halo ou haloalkyle.

14. Utilisation du composé selon la revendication 13 où le composé de formule (I) est un composé où:
m est 1;
n est 1;
p est 2;
V est -C(O)-;
R¹ est l'hydrogène ou alkyle;
R² est choisi dans le groupe consistant en aryle éventuellement substitué, aralkyle éventuellement substitué, hétérocyclyle éventuellement substitué, hétérocyclylalkyle éventuellement substitué, hétéroaryle éventuellement substitué et hétéroarylalkyle éventuellement substitué, où les groupes éventuellement substitués sont comme défini dans la revendication 1;
R³ est cycloalkyle éventuellement substitué ou cycloalkylalkyle éventuellement substitué, où les groupes éventuellement substitués sont comme défini dans la revendication 1;
R⁴ est l'hydrogène, alkyle, halo ou haloalkyle;
R⁵ est indépendamment l'hydrogène, oxo, alkyle, halo ou haloalkyle; et
chaque R⁶ est indépendamment l'hydrogène, oxo, alkyle, halo ou haloalkyle.

15. Utilisation du composé selon la revendication 14 où le composé de formule (I) est choisi dans le groupe consistant en les suivants:
6-[4-(2-phényl-cyclopropanecarbonyl)-pipérazin-1-yl]-*N*-(3-phényl-propyl)-nicotinamide;
*N*-(4-phényl-butyl)-6-[4-(2-phényl-cyclopropanecarbonyl)-pipérazin-1-yl]-nicotinamide;
6-[4-(2-cyclohexyl-acétyl)-pipérazin-1-yl]-N-phénéthyl-nicotinamide;
*N*-[2-(3-chlorophényl)-éthyl]-6-[4-(2-phényl-cyclopropanecarbonyl)-pipérazin-1-yl]-nicotinamide;
*N*-(1-méthyl-3-phényl-propyl)-6-[4-(2-phényl-cyclopropanecarbonyl)-pipérazin-1-yl]- nicotinamide;
N-phénéthyl-6-[4-(2-phényl-cyclopropanecarbonyl)-piperazin-1-yl]-nicotinamide;
6-[4-(2-phényl-cyclopropanecarbonyl)-pipérazin-1-yl]-N-(tétrahydro-furan-2-ylméthyl)-nicotinamide;
*N*-[2-(3H-imidazol-4-yl)-éthyl]-6-[4-(2-phényl-cyclopropanecarbonyl)-pipérazin-1-yl]-nicotinamide;
*N*-(3-imidazol-1-yl-propyl)-6-[4-(2-phényl-cyclopropanecarbonyl)-pipérazin-1-yl]-nicotinamide;
*N*-[2-(3-chlorophényl)-éthyl]-6-[4-(3-cyclohexyl-propionyl)-pipérazin-1-yl]-nicotinamide;
6-[4-(2-cyclohexyl-acétyl)-pipérazin-1-yl]-*N*-(2-(3 H-imidazol-4-yl)-éthyl]-nicotinamide;
6-[4-(2-cyclohexyl-acétyl)-pipérazin-1-yl]-*N*-(3-phényl-propyl)-nicotinamide;
*N*-[2-(3-chlorophényl)-éthyl]-6-[4-(2-cyclohexyl-acétyl)-pipérazin-1-yl]-nicotinamide;
6-[4-(3-cyclohexyl-propionyl)-pipérazin-1-yl]-*N*-phénéthyl-nicotinamide;
6-[4-(3-cyclohexyl-propionyl)-pipérazin-1-yl]-*N*-(3-phényl-propyl)-nicotinamide;
6-[4-(3-méthyl-pentanoyl)-pipérazin-1-yl]-*N*-(4-phényl-butyl)-nicotinamide;
6-[4-(2-cyclohexyl-acétyl)-pipérazin-1-yl]-*N*-(4-phényl-butyl)-nicotinamide;
6-[4-(2-cyclohexyl-acétyl)-pipérazin-1-yl]-*N*-(1-méthyl-3-phényl-propyl)-nicotinamide;
6-[4-(3-cyclohexyl-propionyl)-pipérazin-1-yl]-*N*-[2-(3H-imidazol-4-yl)-éthyl]-nicotinamide;
6-[4-(3-cyclohexyl-propionyl)-pipérazin-1-yl]-*N*-(tétrahydro-furan-2-ylméthyl)-nicotinamide;
6-[4-(2-cyclohexyl-acétyl)-pipérazin-1-yl]-*N*-(3-imidazol-1-yl-propyl)-nicotinamide;
6-[4-(3-cyclohexyl-propionyl)-pipérazin-1-yl]-*N*-(3-imidazol-1-yl-propyl)-nicotinamide; et
6-[4-(2-cyclohexyl-acétyl)-pipérazin-1-yl]-*N*-(tétrahydro-furan-2-ylméthyl)-nicotinamide.

16. Utilisation du composé selon la revendication 1 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle éventuellement substitué, aralcényle éventuellement substitué, cycloalkyle éventuellement substitué, cycloalkylalkyle éventuellement substitué, cycloalkylalcényle, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué, où les groupes éventuellement substitués sont comme défini dans la revendication 1;
R³ est aryle éventuellement substitué, où le groupe éventuellement substitué est comme défini dans la revendication 1;
chaque R⁴ est indépendamment l'hydrogène, alkyle, alcényle, halo, haloalkyle, aryle ou -R⁹-OR⁸;
chaque R⁵ et R⁶ est indépendamment l'hydrogène, oxo, alkyle, alcényle, halo, haloalkyle ou aryle ;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
R⁹ est une liaison directe ou une chaîne alkylène linéaire ou ramifiée;
et
R¹⁰ est alkyle, aryle ou aralkyle.

17. Utilisation du composé selon la revendication 16 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0 à 2), alkyle, alcényle, cycloalkyle éventuellement substitué, cycloalkylalkyle éventuellement substitué ou cycloalkylalcényle, où les groupes éventuellement substitués sont comme défini dans la revendication 1;
R³ est aryle éventuellement substitué, où le groupe éventuellement substitué est comme défini dans la revendication 1;
chaque R⁴ est indépendamment l'hydrogène, alkyle, halo, haloalkyle ou -R⁹-OR⁸;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle;
R⁹ est une liaison directe ou une chaîne alkylène linéaire ou ramifiée;
et
R¹⁰ est alkyle, aryle ou aralkyle.

18. Utilisation du composé selon la revendication 17 où le composé de formule (I) est un composé où:
m est 1 ou 2;
n est 1;
p est 2;
V est -C(O)- ;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, cycloalkyle éventuellement substitué ou cycloalkylalkyle éventuellement substitué, où les groupes éventuellement substitués sont comme défini dans la revendication 1;
R³ est aryle éventuellement substitué, où le groupe éventuellement substitué est comme défini dans la revendication 1;
chaque R⁴ est indépendamment l'hydrogène, halo, haloalkyle ou -R⁹-OR⁸;
R⁵ est l'hydrogène;
chaque R⁶ est l'hydrogène;
R⁷ est une chaîne alkylène linéaire ou ramifiée;
R⁸ est l'hydrogène ou alkyle;
R⁹ est une liaison directe ou une chaîne alkylène linéaire ou ramifiée;
et
R¹⁰ est alkyle, aryle ou aralkyle.

19. Utilisation du composé selon la revendication 18 où le composé de formule (I) est choisi dans le groupe consistant en les suivants:
*N*-butyl-6-[4-(2-mercapto-benzoyl)-pipérazin-1-yl]-nicotinamide;
*N*-(3-méthyl-butyl)-6-[4-(2-o-tolyl-acétyl)-pipérazin-1-yl]-nicotinamide;
6-{4-[2-(2,4-diméthyl-phényl)-acétyl]-pipérazin-1-yl}-*N*-(3-méthylbutyl)-nicotinamide;
6-[4-(2-bromo-benzoyl)-pipérazin-1-yl]-*N*-(3-éthoxypropyl)-nicotinamide;
6-{4-[2-(2-chloro-6-fluoro-phényl)-acétyl]-pipérazin-1-yl}-*N*-(3-méthylbutyl)-nicotinamide;
6-[4-(3,5-dichloro-benzoyl)-pipérazin-1-yl]-*N*-(3-méthoxy-propyl)-nicotinamide;
6-[4-(2-bromo-5-méthoxy-benzoyl)-pipérazin-1-yl]-*N*-(3-diméthylamino-propyl)-nicotinamide;
6-[4-(2,5-dichloro-benzoyl)-pipérazin-1-yl]-*N*-(3-méthylbutyl)-nicotinamide;
6-[4-(2,5-dichloro-benzoyl)-pipérazin-1-yl]-*N*-(3-éthoxy-propyl)-nicotinamide;
*N*-butyl-6-[4-(3,5-dichloro-benzoyl)-pipérazin-1-yl]-nicotinamide;
6-[4-(2,4-dichloro-benzoyl)-pipérazin-1-yl]-*N*-(3-éthoxy-propyl)-nicotinamide;
6-[4-(2,4-dichloro-benzoyl)-pipérazin-1-yl]-*N*-(2-éthylsulfanyl-éthyl)-nicotinamide;
*N*-(3-éthoxy-propyl)-6-[4-(4-trifluorométhyl-benzoyl)-pipérazin-1-yl]-nicotinamide;
*N*-(3-diméthylamino-propyl)-6-[4-(2,3,4,5-tétrafluoro-benzoyl)-pipérazin-1-yl]-nicotinamide;
6-[4-(2-bromo-5-méthoxy-benzoyl)-pipérazin-1-yl]-*N*-(2-éthylsulfanyl-éthyl)-nicotinamide;
*N*-(2-éthylsulfanyl-éthyl)-6-[4-(3-trifluorométhyl-benzoyl)-pipérazin-1-yl]-nicotinamide;
6-[4-(2-bromo-benzoyl)-pipérazin-1-yl]-*N*-(3-méthylbutyl)-nicotinamide;
*N*-butyl-6-[4-(2,4-diméthyl-benzoyl)-pipérazin-1-yl]-nicotinamide;
6-[4-(3,5-dichloro-benzoyl)-pipérazin-1-yl]-*N*-(3-isopropoxy-propyl)-nicotinamide ;
6-[4-(2-bromo-5-méthoxy-benzoyl)-pipérazin-1-yl]-*N*-(3-méthylbutyl)-nicotinamide ;
*N*-(3-butoxy-propyl)-6-[4-(2,5-dichloro-benzoyl)-pipérazin-1-yl]-nicotinamide;
*N*-(3-méthyl-butyl)-6-[4-(3-trifluorométhyl-benzoyl)-pipérazin-1-yl]-nicotinamide;
N-(3-méthyl-butyl)-6-[4-(2-trifluorométhyl-benzoyl)-pipérazin-1-yl]-nicotinamide;
6-[4-(2-bromo-benzoyl)-pipérazin-1-yl]-*N*-(3-butoxy-propyl)-nicotinamide;
6-[4-(2,5-dichloro-benzoyl)-pipérazin-1-yl]-*N*-pentyl-nicotinamide;
*N*-(3-butoxy-propyl)-6-[4-(2,4-diméthyl-benzoyl)-pipérazin-1-yl]-nicotinamide;
6-[4-(2-bromo-5-métoxy-benzoyl)-pipérazin-1-yl]-*N*-(3-éthoxy-propyl)-nicotinamide;
6-[4-(2,4-dichloro-benzoyl)-pipérazin-1-yl]-*N*-(3-isopropoxy-propyl)-nicotinamide;
*N*-(3-éthoxy-propyl)-6-[4-(2-trifluorométhyl-benzoyl)-pipérazin-1-yl]-nicotinamide;
*N*-pentyl-6-[4-(3-trifluorométhyl-benzoyl)-pipérazin-1-yl]-nicotinamide;
*N*-butyl-6-[4-(2-trifluorométhyl-benzoyl)-pipérazin-1-yl]-nicotinamide;
*N*-butyl-6-[4-(2,5-dichloro-benzoyl)-pipérazin-1-yl]-nicotinamide;
6-[4-(2-bromo-benzoyl)-pipérazin-1-yl]-*N*-(3-isopropoxy-propyl)-nicotinamide;
6-[4-(2,5-dichloro-benzoyl)-pipérazin-1-yl]-*N*-(3-isopropoxy-propyl)-nicotinamide;
N-(3-butoxy-propyl)-6-[4-(4-trifluorométhyl-benzoyl)-pipérazin-1-yl]-nicotinamide;
6-[4-(2,5-dichloro-benzoyl)-pipérazin-1-yl]-*N*-(2-éthylsulfanyl-éthyl)-nicotinamide ;
N-(3-butoxy-propyl)-6-[4-(2-trifluorométhyl-benzoyl)-pipérazin-1-yl]-nicotinamide;
6-[4-(2,5-Dichloro-benzoyl)-pipérazin-1-yl]-*N*-(3-méthoxy-propyl)-nicotinamide;
6-[4-(2,4-diméthyl-benzoyl)-pipérazin-1-yl]-*N*-(1,3-diméthylbutyl)-nicotinamide;
6-[4-(2,4-dichloro-benzoyl)-pipérazin-1-yl)-*N*-(2-méthylbutyl)-nicotinamide;
6-[4-(3,5-dichloro-benzoyl)-pipérazin-1-yl]-*N*-(2-méthylbutyl)-nicotinamide;
6-[4-(2,4-diméthyl-benzoyl)-pipérazin-1-yl]-*N*-(2-éthylsulfanyl-éthyl)-nicotinamide ;
*N*-(1,3-diméthylbutyl)-6-[4-(3-trifluorométhyl-benzoyl)-pipérazin-1-yl]-nicotinamide;
6-[4-(2-bromo-5-méthoxy-benzoyl)-pipérazin-1-yl]-*N*-(2-méthylbutyl)-nicotinamide ;
*N*-(3-diméthylamino-propyl)-6-[4-(2-trifluorométhyl-benzoyl)-pipérazin-1-yl]-nicotinamide;
*N*-hexyl-6-[4-(3-trifluorométhyl-benzoyl)-pipérazin-1-yl]-nicotinamide;
6-[4-(2,4-dichloro-benzoyl)-pipérazin-1-yl]-*N*-(1,3-diméthylbutyl)-nicotinamide ;
*N*-(3-isopropoxy-propyl)-6-[4-(3-trifluorométhyl-benzoyl)-pipérazin-1-yl]-nicotinamide ;
6-[4-(2-bromo-5-méthoxy-benzoyl)-pipérazin-1-yl]-*N*-hexyl-nicotinamide ;
6-[4-(2-bromo-benzoyl)-pipérazin-1-yl]-*N*-hexyl-nicotinamide;
*N*-(3-isopropoxy-propyl)-6-[4-(2-trifluorométhyl-benzoyl)-pipérazin-1-yl]-nicotinamide;
6-[4-(2-bromo-5-méthoxy-benzoyl)-pipérazin-1-yl]-*N*-(3-méthoxy-propyl)-nicotinamide;
6-[4-(2-bromo-benzoyl)-pipérazin-1-yl]-*N*-(1-méthylbutyl)-nicotinamide;
6-(4-(3,5-dichloro-benzoyl)-pipérazin-1-yl]-*N*-hexyl-nicotinamide;
*N*-(3-méthoxy-propyl)-6-[4-(2-trifluorométhyl-benzoyl)-pipérazin-1-yl]-nicotinamide;
6-[4-(2,4-dichloro-benzoyl)-pipérazin-1-yl]-*N*-hexyl-nicotinamide;
*N*-(2-éthylsulfanyl-éthyl)-6-[4-(2-trifluorométhyl-benzoyl)-pipérazin-1-yl]-nicotinamide;
*N*-pentyl-6-[4-(2-trifluorométhyl-benzoyl)-pipérazin-1-yl]-nicotinamide;
*N*-(2-méthyl-butyl)-6-[4-(3-trifluorométhyl-benzoyl)-pipérazin-1-yl]-nicotinamide;
*N*-(2-éthylsulfanyl-éthyl)-6-[4-(4-trifluorométhyl-benzoyl)-pipérazin-1-yl]-nicotinamide;
6-[4-(2,4-dichloro-benzoyl)-pipérazin-1-yl]-*N*-pentyl-nicotinamide;
6-[4-(2-bromo-benzoyl)-pipérazin-1-yl]-*N*-(2-méthylbutyl)-nicotinamide;
6-[4-(2-bromo-5-méthoxy-benzoyl)-pipérazin-1-yl]-*N*-(3-butoxy-propyl)-nicotinamide;
*N*-(1,3-diméthylbutyl)-6-[4-(4-trifluorométhyl-benzoyl)-pipérazin-1-yl]-nicotinamide;
6-[4-(3,5-dichloro-benzoyl)-pipérazin-1-yl]-*N*-pentyl-nicotinamide;
*N*-(3-butoxy-propyl)-6-[4-(3,5-dichloro-benzoyl)-pipérazin-1-yl]-nicotinamide;
6-[4-(2-bromo-5-méthoxy-benzoyl)-pipérazin-1-yl]-*N*-butyl-nicotinamide;
6-[4-(2-bromo-benzoyl)-pipérazin-1-yl]-*N*-pentyl-nicotinamide;
*N*-(1,3-diméthylbutyl)-6-[4-(2-tritluorométhyl-benzoyl)-pipérazin-1-yl]-nicotinamide;
*N*-(3-méthoxy-propyl)-6-[4-(4-trifluorométhyl-benzoyl)-pipérazin-1-yl]-nicotinamide;
6-[4-(2,4-diméthyl-benzoyl)-pipérazin-1-yl]-*N*-(1-méthylbutyl)-nicotinamide;
6-[4-(2,4-dichloro-benzoyl)-pipérazin-1-yl]-*N*-(3-méthoxy-propyl)-nicotinamide;
*N*-(1-méthyl-butyl)-6-[4-(2-trifluorométhyl-benzoyl)-pipérazin-1-yl]-nicotinamide;
*N*-(3-diméthylamino-propyl)-6-[4-(3-trifluorométhyl-benzoyl)-pipérazin-1-yl]-nicotinamide ;
6-[4-(2,4-dichloro-benzoyl)-pipérazin-1-yl]-*N*-(1-méthylbutyl)-nicotinamide;
6-[4-(2,5-dichloro-benzoyl)-pipérazin-1-yl]-*N*-(1-méthylbutyl)-nicotinamide;
*N*-(1-méthyl-butyl)-6-[4-(3-trifluorométhyl-benzoyl)-pipérazin-1-yl]-nicotinamide;
6-[4-(2,5-dichloro-benzoyl)-pipérazin-1-yl]-*N*-hexyl-nicotinamide;
6-[4-(2,4-diméthyl-benzoyl)-pipérazin-1-yl)-*N*-(3-méthoxy-propyl)-nicotinamide;
6-[4-(3,5-dichloro-benzoyl)-pipérazin-1-yl]-*N*-(1-méthylbutyl)-nicotinamide;
6-[4-(2,4-dichloro-benzoyl)-pipérazin-1-yl]-*N*-(3-méthylbutyl)-nicotinamide;
*N*-(3-butoxy-propyl)-6-(4-(3-trifluorométhyl-benzoyl)-pipérazin-1-yl]-nicotinamide;
6-[4-(2,4-diméthyl-benzoyl)-pipérazin-1-yl]-*N*-(3-isopropoxy-propyl)-nicotinamide;
*N*-(1-méthyl-butyl)-6-[4-(4-trifluorométhyl-benzoyl)-pipérazin-1-yl]-nicotinamide;
*N*-butyl-6-[4-(2,4-dichloro-benzoyl)-pipérazin-1-yl]-nicotinamide;
6-[4-(3,5-dichloro-benzoyl)-pipérazin-1-yl]-*N*-(1,3-diméthylbutyl)-nicotinamide;
*N*-butyl-6-[4-(3-trifluorométhyl-benzoyl)-pipérazin-1-yl]-nicotinamide;
6-[4-(2-bromo-5-méthoxy-benzoyl)-pipérazin-1-yl]-*N*-(1,3-diméthylbutyl)-nicotinamide;
6-[4-(2,4-diméthyl-benzoyl)-pipérazin-1-yl]-*N*-hexyl-nicotinamide;
6-[4-(2,5-dichloro-benzoyl)-pipérazin-1-yl]-*N*-(1,3-diméthylbutyl)-nicotinamide;
*N*-(3-butoxy-propyl)-6-[4-(2,4-dichloro-benzoyl)-pipérazin-1-yl]-nicotinamide;
*N*-(3-méthoxy-propyl)-6-[4-(3-trifluorométhyl-benzoyl)-pipérazin-1-yl]-nicotinamide;
6-[4-(2,4-diméthyl-benzoyl)-pipérazin-1-yl]-*N*-(3-éthoxy-propyl)-nicotinamide;
6-[4-(2,5-dichloro-benzoyl)-pipérazin-1-yl]-*N*-(2-méthylbutyl)-nicotinamide;
*N*-hexyl-6-[4-(4-trifluorométhyl-benzoyl)-pipérazin-1-yl]-nicotinamide;
6-[4-(2,4-diméthyl-benzoyl)-pipérazin-1-yl]-N-pentyl-nicotinamide;
6-[4-(2-bromo-benzoyl)-pipérazin-1-yl]-*N*-(1,3-diméthylbutyl)-nicotinamide;
*N*-hexyl-6-[4-(2-trifluorométhyl-benzoyl)-pipérazin-1-yl]-nicotinamide;
6-[4-(2-bromo-benzoyl)-pipérazin-1-yl]-*N*-(3-méthoxy-propyl)-nicotinamide;
*N*-(2-méthyl-butyl)-6-[4-(4-trifluorométhyl-benzoyl)-pipérazin-1-yl]-nicotinamide;
6-[4-(2-bromo-5-méthoxy-benzoyl)-pipérazin-1-yl]-*N*-(1-méthylbutyl)-nicotinamide;
6-[4-(2,4-diméthyl-benzoyl)-pipérazin-1-yl]-*N*-(2-méthylbutyl)-nicotinamide;
6-[4-(2-bromo-5-méthoxy-benzoyl)-pipérazin-1-yl]-*N*-pentyl-nicotinamide;
*N*-(2-méthyl-butyl)-6-[4-(2-trifluorométhyl-benzoyl)-pipérazin-1-yl]-nicotinamide;
6-(4-(3,5-dichloro-benzoyl)-pipérazin-1-yl]-*N*-(3-méthylbutyl)-nicotinamide;
6-[4-(2-bromo-benzoyl)-pipérazin-1-yl]-*N*-(2-éthylsulfanyl-éthyl)-nicotinamide;
6-[4-(3,5-dichloro-benzoyl)-pipérazin-1-yl]-*N*-(3-méthoxy-propyl)-nicotinamide;
6-[4-(2-bromo-5-méthoxy-benzoyl)-pipérazin-1-yl]-*N*-(3-isopropoxy-propyl)-nicotinamide;
*N*-(3-butoxy-propyl)-6-[4-(2,3,4,5-tétrafluoro-benzoyl)-pipérazin-1-yl]-nicotinamide;
*N*-(1,3-diméthylbutyl)-6-[4-(2,3,4,5-tétrafluoro-benzoyl)-pipérazin-1-yl]-nicotinamide;
*N*-butyl-6-[4-(2,3,4,5-tétrafluoro-benzoyl)-pipérazin-1-yl]-nicotinamide;
*N*-(1-méthyl-butyl)-6-[4-(2,3,4,5-tétrafluoro-benzoyl)-pipérazin-1-yl]-nicotinamide;
*N*-(3-méthoxy-propyl)-6-[4-(2,3,4,5-tétrafluoro-benzoyl)-pipérazin-1-yl]-nicotinamide;
*N*-(3-isopropoxy-propyl)-6-[4-(2,3,4,5-tétrafluoro-benzoyl)-pipérazin-1-yl]-nicotinamide;
*N*-pentyl-6-[4-(2,3,4,5-tétrafluoro-benzoyl)-pipérazin-1-yl]-nicotinamide;
*N*-(2-méthyl-butyl)-6-[4-(2,3,4,5-tétrafluoro-benzoyl)-pipérazin-1-yl]-nicotinamide;
*N*-hexyl-6-[4-(2,3,4,5-tétrafluoro-benzoyl)-pipérazin-1-yl]-nicotinamide;
*N*-(3-méthyl-butyl)-6-[4-(2,3,4,5-tétrafluoro-benzoyl)-pipérazin-1-yl]-nicotinamide;
*N*-(2-éthylsulfanyl-éthyl)-6-[4-(2,3,4,5-tétrafluoro-benzoyl)-pipérazin-1-yl]-nicotinamide;
*N*-(3-éthoxy-propyl)-6-[4-(2,3,4,5-tétrafluoro-benzoyl)-pipérazin-1-yl]-nicotinamide;
*N*-(3-diméthylamino-propyl)-6-[4-(4-trifluorométhyl-benzoyl)-pipérazin-1-yl]-nicotinamide;
*N*-(3-éthoxy-propyl)-6-[4-(3-trifluorométhyl-benzoyl)-pipérazin-1-yl]-nicotinamide;
6-[4-(3,5-dichloro-benzoyl)-pipérazin-1-yl]-*N*-(2-éthylsulfanyl-éthyl)-nicotinamide;
*N*-(3-méthyl-butyl)-6-[4-(4-trifluorométhyl-benzoyl)-pipérazin-1-yl]-nicotinamide;
*N*-butyl-6-[4-(4-trifluorométhyl-benzoyl)-pipérazin-1-yl]-nicotinamide;
*N*-(3-isopropoxy-propyl)-6-[4-(4-trifluorométhyl-benzoyl)-pipérazin-1-yl]-nicotinamide;
*N*-pentyl-6-[4-(4-trifluorométhyl-benzoyl)-pipérazin-1-yl]-nicotinamide;
6-[4-(3,5-dichloro-benzoyl)-pipérazin-1-yl]-*N*-(3-diméthylamino-propyl)-nicotinamide;
6-[4-(2-bromo-benzoyl)-pipérazin-1-yl]-*N*-(3-diméthylamino-propyl)-nicotinamide;
6-[4-(2,4-dichloro-benzoyl)-pipérazin-1-yl]-*N*-(3-diméthylamino-propyl)-nicotinamide ;
6-[4-(2,5-dichloro-benzoyl)-pipérazin-1-yl]-*N*-(3-diméthylamino-propyl)-nicotinamide ;
*N*-(3-diméthylaminopropyl)-6-[4-(2,4-diméthyl-benzoyl)-pipérazin-1-yl]-nicotinamide;
*N*-(3-méthyl-butyl)-6-[4-(2-trifluorométhyl-benzoyl)-pipérazin-1-yl]-nicotinamide ;
*N*-(3-méthyl-butyl)-4-trifluorométhyl-6-[4-(2-trifluorométhyl-benzoyl)-pipérazin-1-yl]-nicotinamide;
2-chloro-5-fluoro-*N*-(3-méthylbutyl)-6-[4-(2-trifluorométhyl-benzoyl)-pipérazin-1-yl]-nicotinamide;
*N*-(3-éthoxy-propyl)-6-[4-(2-naphtalén-2-yl-acétyl)-pipérazin-1-yl]-nicotinamide;
6-[4-(2-naphtalén-2-yl-acétyl)-pipérazin-1-yl]-*N*-pentyl-nicotinamide;
*N*-butyl-6-[4-(naphtalène-1-carbonyl)-pipérazin-1-yl]-nicotinamide;
*N*-(3-butoxy-propyl)-6-[4-(2-naphtalén-2-yl-acétyl)-pipérazin-1-yl]-nicotinamide;
*N*-butyl-6-[4-(2-naphtalén-2-yl-acétyl)-pipérazin-1-yl]-nicotinamide;
*N*-(3-butoxy-propyl)-6-[4-(naphtalène-1-carbonyl)-pipérazin-1-yl]-nicotinamtde;
*N*-(3-méthoxy-propyl)-6-[4-(naphtalène-1-carbonyl)-pipérazin-1-yl]-nicotinamide;
*N*-(1-méthyl-butyl)-6-[4-(naphtalène-1-carbonyl)-pipérazin-1-yl]-nicotinamide;
6-[4-(naphtalène-1-carbonyl)-pipérazin-1-yl]-*N*-pentyl-nicotinamide;
*N*-(3-isopropoxy-propyl)-6-[4-(2-naphtalén-2-yl-acétyl)-pipérazin-1-yl]-nicotinamide;
*N*-(1-méthyl-butyl)-6-[4-(2-naphtalén-2-yl-acétyl)-pipérazin-1-yl]-nicotinamide;
*N*-(3-méthyl-butyl)-6-[4-(2-naphtalén-2-yl-acétyl)-pipérazin-1-yl]-nicotinamide;
*N*-(3-diméthylamino-propyl)-6-[4-(2-naphtalén-2-yl-acétyl)-pipérazin-1-yl]-nicotinamide;
*N*-(3-éthoxy-propyl)-6-[4-(naphtalène-1-carbonyl)-pipérazin-1-yl]-nicotinamide;
*N*-hexyl-6-[4-(naphtalène-1-carbonyl)-pipérazin-1-yl]-nicotinamide;
*N*-(2-éthylsulfanyl-éthyl)-6-[4-(2-naphtalén-2-yl-acétyl)-pipérazin-1-yl]-nicotinamide;
*N*-hexyl-6-(4-(2-naphtalén-2-yl-acétyl)-pipérazin-1-yl]-nicotinamide;
*N*-(1,3-diméthylbutyl)-6-[4-(naphtalène-1-carbonyl)-pipérazin-1-yl]-nicotinamide;
*N*-(3-isopropoxy-propyl)-6-[4-(naphtalène-1-carbonyl)-pipérazin-1-yl]-nicotinamide;
*N*-(1,3-diméthylbutyl)-6-[4-(naphtalène-2-carbonyl)-pipérazin-1-yl]-nicotinamide;
*N*-(1,3-diméthylbutyl)-6-[4-(2-naphtalén-2-yl-acétyl)-pipérazin-1-yl]-nicotinamide;
*N*-(2-méthyl-butyl)-6-[4-(naphtalène-1-carbonyl)-pipérazin-1-yl]-nicotinamide ;
*N*-(2-éthylsulfanyl-éthyl)-6-[4-(naphtalène-1-carbonyl)-pipérazin-1-yl]-nicotinamide ;
*N*-(2-méthyl-butyl)-6-[4-(2-naphtalén-2-yl-acétyl)-pipérazin-1-yl]-nicotinamide;
*N*-(3-méthyl-butyl)-6-[4-(naphtalène-1-carbonyl)-pipérazin-1-yl]-nicotinamide ;
*N*-(3-éthoxy-propyl)-6-[4-(naphtalène-2-carbonyl)-pipérazin-1-yl]-nicotinamide;
*N*-(2-méthyl-butyl)-6-[4-(naphtalène-2-carbonyl)-pipérazin-1-yl]-nicotinamide;
*N*-(3-méthyl-butyl)-6-[4-(naphtalène-2-carbonyl)-pipérazin-1-yl]-nicotinamide;
*N*-(3-méthoxy-propyl)-6-[4-(naphtalène-2-carbonyl)-pipérazin-1-yl]-nicotinamide;
*N*-butyl-6-[4-(naphtalène-2-carbonyl)-pipérazin-1-yl]-nicotinamide;
*N*-(3-butoxy-propyl)-6-[4-(naphtalène-2-carbonyl)-pipérazin-1-yl]-nicotinamide;
*N*-(1-méthyl-butyl)-6-[4-(naphtalène-2-carbonyl)-pipérazin-1-yl]-nicotinamide;
*N*-(3-isopropoxy-propyl)-6-[4-(naphtalène-2-carbonyl)-pipérazin-1-yl]-nicotinamide;
*N*-hexyl-6-[4-(naphtalène-2-carbonyl)-pipérazin-1-yl]-nicotinamide;
*N*-(3-diméthylamino-propyl)-6-[4-(naphtalène-1-carbonyl)-pipérazin-1-yl]-nicotinamide;
*N*-(2-éthylsulfanyl-éthyl)-6-[4-(naphtalène-2-carbonyl)-pipérazin-1-yl]-nicotinamide;
*N*-(3-diméthylamino-propyl)-6-[4-(naphtalène-2-carbonyl)-pipérazin-1-yl]-nicotinamide;
*N*-(3-méthyl-butyl)-6-[4-(naphtalène-1-carbonyl)-pipérazin-1-yl]-nicotinamide;
*N*-(3-méthoxy-propyl)-6-[4-(2-naphtalén-2-yl-acétyl)-pipérazin-1-yl]-nicotinamide;
2-chloro-*N*-(3-méthylbutyl)-6-[4-(2-trifluorométhyl-benzoyl)-pipérazin-1-yl]-nicotinamide;
*N*-(2-cyclopropyl-éthyl)-6-[4-(3-trifluorométhyl-benzoyl)-pipérazin-1-yl]-nicotinamide;
*N*-(2-cyclopropyl-éthyl)-2-méthoxy-6-[4-(2-trifluorométhyl-benzoyl)-pipérazin-1-yl]-nicotinamide;
*N*-(2-cyclopropyl-éthyl)-6-[4-(5-fluoro-2-trifluorométhyl-benzoyl)-pipérazin-1-yl]-nicotinamide;
(2-cyclopropyléthyl)amide d'acide 2-oxo-6-[4-(2-trifluorométhyl-benzoyl)-pipérazin-1-yl]-1,2-dihydro-pyridine-3-carboxylique ;
*N*-(2-cyclopropyl-éthyl)-2-hydroxy-6-[4-(2-trifluorométhyl-benzoyl)-pipérazin-1-yl]-nicotinamide ;
*N*-(2-cyclobutyl-éthyl)-6-[4-(5-fluoro-2-trifluorométhyl-benzoyl)-pipérazin-1-yl]-nicotinamide ;
*N*-(3-cyclopropyl-propyl)-6-[4-(5-fluoro-2-trifluorométhyl-benzoyl)-pipérazin-1-yl]-nicotinamide;
6-[4-(5-fluoro-2-trifluorométhyl-benzoyl)-piperazin-1-yl]-*N*-(4-méthyl-pentyl)-nicotinamide; et
*N*-(3,3-diméthylbutyl)-6-[4-(5-fluoro-2-trifluorométhyl-benzoyl)-pipérazin-1-yl]-nicotinamide.

20. Utilisation du composé selon la revendication 16 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué, où les groupes éventuellement substitués sont comme défini dans la revendication 1;
R³ est aryle éventuellement substitué, où le groupe éventuellement substitué est comme défini dans la revendication 1;
chaque R⁴ est indépendamment l'hydrogène, alkyle, halo, ou haloalkyle; et
chaque R⁵ et R⁶ est indépendamment l'hydrogène, oxo, alkyle, halo ou haloalkyle.

21. Utilisation du composé selon la revendication 20 où le composé de formule (I) est un composé où:
m est 1 ou 2;
n est 1;
p est 2;
V est -C(O)-;
R¹ est l'hydrogène ou alkyle;
R² est choisi dans le groupe consistant en aryle éventuellement substitué, aralkyle éventuellement substitué, hétérocyclyle éventuellement substitué, hétérocyclylalkyle éventuellement substitué, hétéroaryle éventuellement substitué et hétéroarylalkyle éventuellement substitué, où les groupes éventuellement substitués sont comme défini dans la revendication 1;
R³ est aryle éventuellement substitué, où le groupe éventuellement substitué est comme défini dans la revendication 1;
chaque R⁴ est indépendamment l'hydrogène, alkyle, halo ou haloalkyle;
R⁵ est l'hydrogène, oxo, alkyle, halo ou haloalkyle; et
chaque R⁶ est indépendamment l'hydrogène, alkyle, halo ou haloalkyle.

22. Utilisation du composé selon la revendication 21 où le composé de formule (I) est choisi dans le groupe consistant en les suivants:
6-[4-(2,5-dichloro-benzoyl)-pipérazin-1-yl]-*N*-(3-imidazol-1-yl-propyl)-nicotinamide;
6-[4-(2,4-dichloro-benzoyl)-pipérazin-1-yl]-*N*-(3-imidazol-1-yl-propyl)-nicotinamide;
6-[4-(2-bromo-5-méthoxy-benzoyl)-pipérazin-1-yl]-*N*-(3-phenyl-propyl)-nicotinamide;
6-[4-(2-bromo-benzoyl)-pipérazin-1-yl]-*N*-[2-(3H-imidazol-4-yl)-éthyl]-nicotinamide;
*N*-(1-méthyl-2-phényl-éthyl)-6-[4-(2-trifluorométhyl-benzoyl)-pipérazin-1-yl]-nicotinamide;
*N*-(1-méthyl-2-phényl-éthyl)-6-[4-(4-trifluorométhyl-benzoyl)-pipérazin-1-yl]-nicotinamide;
6-[4-(naphtalène-1-carbonyl)-pipérazin-1-yl]-*N*-(4-phényl-butyl)-nicotinamide;
*N*-[2-(3-chlorophényl)-éthyl]-6-[4-(2-naphtalén-2-yl-acétyl)-pipérazin-1-yl]-nicotinamide;
6-[4-(naphtalène-2-carbonyl)-pipérazin-1-yl]-*N*-(3-phényl-propyl)-nicotinamide;
*N*-(3-imidazol-1-yl-propyl)-6-[4-(2-naphtalén-2-yl-acétyl)-pipérazin-1-yl]-nicotinamide;
6-[4-(naphtalène-1-carbonyl)-pipérazin-1-yl]-*N*-(3-phényl-propyl)-nicotinamide;
*N*-(1-méthyl-3-phényl-propyl)-6-[4-(2-naphtalén-2-yl-acétyl)-pipérazin-1-yl]-nicotinamide;
6-[4-(2-naphtalén-2-yl-acétyl)-pipérazin-1-yl]-*N*-phénéthyl-nicotinamide;
*N*-[2-(3-chlorophényl)-éthyl]-6-[4-(naphtalène-1-carbonyl)-pipérazin-1-yl]-nicotinamide;
*N*-(1-méthyl-3-phényl-propyl)-6-[4-(naphtalène-1-carbonyl)-pipérazin-1-yl]-nicotinamide ;
6-[4-(naphtalène-1-carbonyl)-pipérazin-1-yl]-*N*-phénéthyl-nicotinamide;
*N*-[2-(3H-imidazol-4-yl)-éthyl]-6-[4-(2-naphtalén-2-yl-acétyl)-pipérazin-1-yl]-nicotinamide ;
6-[4-(2-naphtalén-2-yl-acétyl)-pipérazin-1-yl]-*N*-(4-phényl-butyl)-nicotinamide;
6-[4-(naphtalène-2-carbonyl)-pipérazin-1-yl]-*N*-(3-phényl-propyl)-nicotinamide;
*N*-(1-méthyl-3-phényl-propyl)-6-[4-(naphtalène-2-carbonyl)-pipérazin-1-yl)-nicotinamide ;
*N*-[2-(3H-imidazol-4-yl)-éthyl]-6-[4-(naphtalène-1-carbonyl)-pipérazin-1-yl]-nicotinamide;
6-[4-(naphtalène-2-carbonyl)-pipérazin-1-yl]-*N*-(tétrahydro-furan-2-ylméthyl)-nicotinamide;
6-[4-(naphtalène-1-carbonyl)-pipérazin-1-yl]-*N*-(tétrahydro-furan-2-ylméthyl)-nicotinamide;
*N*-(3-imidazol-1-yl-propyl)-6-[4-(naphtalène-2-carbonyl)-pipérazin-1-yl]-nicotinamide;
*N*-[2-(3H-imidazol-4-yl)-éthyl]-6-[4-(naphtalène-2-carbonyl)-pipérazin-1-yl]-nicotinamide;
6-[4-(naphtalène-2-carbonyl)-pipérazin-1-yl]-*N*-phénéthyl-nicotinamide;
6-[4-(naphtalène-2-carbonyl)-pipérazin-1-yl]-*N*-(4-phényl-butyl)-nicotinamide;
*N*-[2-(3-chlorophényl)-éthyl]-6-[4-(naphtalène-2-carbonyl)-pipérazin-1-yl]-nicotinamide;
*N*-(3-imidazol-1-yl-propyl)-6-[4-(naphtalène-1-carbonyl)-pipérazin-1-yl]-nicotinamide;
6-[4-(2-naphtalén-2-yl-acétyl)-pipérazin-1-yl]-*N*-(tétrahydro-furan-2-ylméthyl)-nicotinamide;
6-(4-(2-bromo-benzoyl)-pipérazin-1-yl]-*N*-(3-phényl-propyl)-nicotinamide;
*N*-[2-(3-chlorophényl)-éthyl]-6-[4-(2,4-dichloro-benzoyl)-pipérazin-1-yl]-nicotinamide;
6-[4-(2,4-diméthyl-benzoyl)-pipérazin-1-yl]-*N*-(4-phényl-butyl)-nicotinamide;
*N*-phénéthyl-6-[4-(2-p-tolyl-acétyl)-pipérazin-1-yl]-nicotinamide;
6-{4-[2-(2,5-dichloro-phényl)-acétyl]-pipérazin-1-yl}-*N*-(2-méthyl-3-phényl-propyl)-nicotinamide;
6-[4-(2,4-diméthyl-benzoyl)-pipérazin-1-yl]-*N*-(3-phényl-propyl)-nicotinamide;
*N*-[2-(3-chlorophényl)-éthyl]-6-[4-(2-fluoro-4-méthyl-benzoyl)-pipérazin-1-yl]-nicotinamide ;
*N*-[2-(3-chlorophényl)-éthyl]-6-[4-(2,5-dichloro-benzoyl)-pipérazin-1-yl]-nicotinamide ;
*N*-(3-phényl-propyl)-6-[4-(4-trifluorométhyl-benzoyl)-pipérazin-1-yl]-nicotinamide;
6-[4-(2-bromo-benzoyl)-pipérazin-1-yl]-*N*-(3-imidazol-1-yl-propyl)-nicotinamide;
*N*-(4-phényl-butyl)-6-[4-(2-trifluorométhyl-benzoyl)-pipérazin-1-yl]-nicotinamide;
*N*-(3-phényl-propyl)-6-[4-(3-trifluorométhyl-benzoyl)-pipérazin-1-yl]-nicotinamide;
6-[4-(2-bromo-benzoyl)-pipérazin-1-yl]-*N*-[2-(3-chloro-phényl)-éthyl]-nicotinamide;
6-[4-(2-bromo-benzoyl)-pipérazin-1-yl]-*N*-(2-méthyl-3-phényl-propyl)-nicotinamide;
6-[4-(2-bromo-5-méthoxy-benzoyl)-pipérazin-1-yl]-*N*-(5-phényl-pentyl)-nicotinamide;
6-[4-(2-bromo-5-méthoxy-benzoyl)-pipérazin-1-yl]-*N*-[2-(3-chloro-phényl)-éthyl]-nicotinamide;
*N*-[2-(3-chlorophényl)-éthyl]-6-[4-(2-trifluorométhyl-benzoyl)-pipérazin-1-yl]-nicotinamide;
*N*-(3-phényl-propyl)-6-(4-(2-trifluorométhyl-benzoyl)-pipérazin-1-yl]-nicotinamide;
*N*-(4-phényl-butyl)-6-[4-(3-trifluorométhyl-benzoyl)-pipérazin-1-yl]-nicotinamide;
6-[4-(2,4-dichloro-benzoyl)-pipérazin-1-yl]-*N*-phénéthyl-nicotinamide;
6-[4-(2-bromo-benzoyl)-pipérazin-1-yl]-*N*-(4-phényl-butyl)-nicotinamide;
6-[4-(2-bromo-benzoyl)-pipérazin-1-yl]-*N*-phénéthyl-nicotinamide;
6-[4-(2-bromo-5-méthoxy-benzoyl)-pipérazin-1-yl]-*N*-(1-méthyl-3-phényl-propyl)-nicotinamide;
6-[4-(3,5-dichloro-benzoyl)-pipérazin-1-yl]-*N*-(3-phényl-propyl)-nicotinamide;
6-[4-(3,5-dichloro-benzoyl)-pipérazin-1-yl]-*N*-(1-méthyl-3-phényl-propyl)-nicotinamide;
*N*-phénéthyl-6-[4-(4-trifluorométhyl-benzoyl)-pipérazin-1-yl]-nicotinamide;
6-[4-(2,4-diméthyl-benzoyl)-pipérazin-1-yl]-*N*-phénéthyl-nicotinamide;
*N*-phénéthyl-6-[4-(2-trifluorométhyl-benzoyl)-pipérazin-1-yl]-nicotinamide;
6-[4-(2,4-dichloro-benzoyl)-pipérazin-1-yl]-*N*-(1-méthyl-3-phényl-propyl)-nicotinamide;
6-[4-(2,5-dichloro-benzoyl)-pipérazin-1-yl]-*N*-(3-phényl-propyl)-nicotinamide;
6-[4-(2-bromo-5-méthoxy-benzoyl)-pipérazin-1-yl]-*N*-phénéthyl-nicotinamide;
6-[4-(2-bromo-5-méthoxy-benzoyl)-pipérazin-1-yl]-*N*-(3-imidazol-1-yl-propyl)-nicotinamide ;
6-[4-(3,5-dichloro-benzoyl)-pipérazin-1-yl]-*N*-(3-imidazol-1-yl-propyl)-nicotinamide ;
*N*-[2-(3H-imidazol-4-yl)-éthyl]-6-[4-(2,3,4,5-tétrafluoro-benzoyl)-pipérazin-1-yl]-nicotinamide ;
*N*-phénéthyl-6-[4-(3-trifluorométhyl-benzoyl)-pipérazin-1-yl]-nicotinamide ;
6-[4-(2,5-dichloro-benzoyl)-pipérazin-1-yl]-*N*-phénéthyl-nicotinamide;
6-[4-(3-méthyl-pentanoyl)-pipérazin-1-yl]-*N*-(1-méthyl-3-phényl-propyl]-nicotinamide ;
6-[4-(2,3,4,5-tétrafluoro-benzoyl)-pipérazin-1-yl]-*N*-(tétrahydro-furan-2-yl-méthyl)-nicotinamide ;
6-[4-(2-bromo-5-méthoxy-benzoyl)-pipérazin-1-yl]-*N*-(tétrahydro-furan-2-yl-méthyl)-nicotinamide;
*N*-(tétrahydro-furan-2-ylméthyl)-6-[4-(4-trifluorométhyl-benzoyl)-pipérazin-1-yl]-nicotinamide;
6-[4-(3,5-dichloro-benzoyl)-pipérazin-1-yl]-*N*-(4-phényl-butyl)-nicotinamide;
6-[4-(2,4-diméthyl-benzoyl)-pipérazin-1-yl]-*N*-(1-méthyl-3-phényl-propyl)-nicotinamide;
6-[4-(2,4-diméthyl-benzoyl)-pipérazin-1-yl]-*N*-(tétrahydro-furan-2-ylméthyl)-nicotinamide;
6-[4-(2-bromo-benzoyl)-pipérazin-1-yl]-*N*-(tétrahydro-furan-2-ylméthyl)-nicotinamide;
*N*-(1-méthyl-3-phényl-propyl)-6-[4-(3-tritluorométhyl-benzoyl)-pipérazin-1-yl]-nicotinamide;
*N*-phénéthyl-6-[4-(2,3,4,5-tétrafluoro-benzoyl)-pipérazin-1-yl]-nicotinamide;
*N*-(4-phényl-butyl)-6-[4-(2,3,4,5-tétrafluoro-benzoyl)-pipérazin-1-yl]-nicotinamide;
*N*-(1-méthyl-3-phényl-propyl)-6-[4-(2,3,4,5-tétrafluoro-benzoyl)-pipérazin-1-yl]-nicotinamide;
*N*-[2-(3-chlorophényl)-éthyl]-6-[4-(2,3,4,5-tétratluoro-benzoyl)-pipérazin-1-yl]-nicotinamide;
*N*-(3-phényl-propyl)-6-[4-(2,3,4,5-tétrafluoro-benzoyl)-pipérazin-1-yl]-nicotinamide;
6-[4-(2,5-dichloro-benzoyl)-pipérazin-1-yl]-*N*-(tétrahydro-furan-2-ylméthyl)-nicotinamide;
6-[4-(2,4-diméthyl-benzoyl)-pipérazin-1-yl]-*N*-[2-(3H-imidazol-4-yl)-éthyl]-nicotinamide ;
*N*-(2-(3H-imidazol-4-yl)-éthyl]-6-[4-(3-trifluorométhyl-benzoyl)-pipérazin-1-yl]-nicotinamide ;
*N*-(3-imidazol-1-yl-propyl)-6-[4-(3-trifluorométhyl-benzoyl)-pipérazin-1-yl]-nicotinamide;
6-(4-(2,4-diméthyl-benzoyl)-pipérazin-1-yl]-*N*-(3-imidazol-1-yl-propyl)-nicotinamide;
6-[4-(2,5-dichloro-benzoyl)-pipérazin-1-yl]-*N*-[2-(3H-imidazol-4-yl)-éthyl]-nicotinamide;
*N*-[2-(3H-imidazol-4-yl)-éthyl]-6-[4-(4-trifluorométhyl-benzoyl)-pipérazin-1-yl]-nicotinamide ;
6-[4-(2,4-dichloro-benzoyl)-pipérazin-1-yl]-*N*-[2-(3H-imidazol-4-yl)-éthyl]-nicotinamide;
6-[4-(3,5-dichloro-benzoyl)-pipérazin-1-yl]-*N*-(tétrahydro-furan-2-ylméthyl)-nicotinamide;
*N*-(3-imidazol-1-yl-propyl)-6-[4-(2-trifluorométhyl-benzoyl)-pipérazin-1-yl]-nicotinamide;
*N*-(tétrahydro-furan-2-ylméthyl)-6-[4-(3-trifluorométhyl-benzoyl)-pipérazin-1-yl]-nicotinamide;
*N*-(tétrahydro-furan-2-ylméthyl)-6-[4-(2-trifluorométhyl-benzoyl)-pipérazin-1-yl]-nicotinamide;
6-[4-(2,4-dichloro-benzoyl)-pipérazin-1-yl]-*N*-(tétrahydro-furan-2-ylméthyl)-nicotinamide;
*N*-[2-(3H-imidazol-4-yl)-éthyl]-6-[4-(2-trifluorométhyl-benzoyl)-pipérazin-1-yl]-nicotinamide;
6-[4-(2-bromo-5-méthoxy-benzoyl)-pipérazin-1-yl]-*N*-[2-(3H-imidazol-4-yl)-éthyl]-nicotinamide;
*N*-[2-(3-chlorophényl)-éthyl]-6-[4-(3-trifluorométhyl-benzoyl)-pipérazin-1-yl]-nicotinamide;
*N*-[2-(3-chlorophényl)-éthyl]-6-[4-(4-trifluorométhyl-benzoyl)-pipérazin-1-yl]-nicotinamide;
*N*-[2-(3-chlorophényl)-éthyl]-6-[4-(3,5-dichloro-benzoyl)-pipérazin-1-yl]-nicotinamide;
*N*-(4-phényl-butyl)-6-[4-(4-trifluorométhyl-benzoyl)-pipérazin-1-yl]-nicotinamide ;
6-[4-(2,4-dichloro-benzoyl)-pipérazin-1-yl]-*N*-(4-phényl-butyl)-nicotinamide;
6-[4-(2,4-dichloro-benzoyl)-pipérazin-1-yl]-*N*-(3-phényl-propyl)-nicotinamide;
6-[4-(2,5-dichloro-benzoyl)-pipérazin-1-yl]-*N*-(4-phényl-butyl)-nicotinamide;
6-[4-(3,5-dichloro-benzoyl)-pipérazin-1-yl]-*N*-phénéthyl-nicotinamide;
*N*-(3-imidazol-1-yl-propyl)-6-[4-(4-trifluorométhyl-benzoyl)-pipérazin-1-yl]-nicotinamide ;
6-[4-(3,5-dichloro-benzoyl)-pipérazin-1-yl]-*N*-[2-(3H-imidazol-4-yl)-éthyl]-nicotinamide;
*N*-(3-imidazol-1-yl-propyl)-6-[4-(2,3,4,5-tétrafluoro-benzoyl)-pipérazin-1-yl]-nicotinamide;
6-[2-oxo-4-(2-trifluorométhyl-benzoyl)-pipérazin-1-yl]-*N*-(3-phényl-propyl)-nicotinamide;
*N*-(3-méthyl-butyl)-6-[2-oxo-4-(2-trifluorométhyl-benzoyl)-pipérazin-1-yl]-nicotinamide.

23. Utilisation du composé selon la revendication 1 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle éventuellement substitué, aralcényle éventuellement substitué, cycloalkyle éventuellement substitué, cycloalkylalkyle éventuellement substitué, cycloalkylalcényle, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est hétéroaryle éventuellement substitué, hétéroarylalkyle éventuellement substitué ou hétéroarylalcényle éventuellement substitué;
chaque R⁴ est indépendamment l'hydrogène, alkyle, alcényle, halo, haloalkyle ou aryle;
chaque R⁵ et R⁶ est indépendamment l'hydrogène, oxo, alkyle, alcényle, halo, haloalkyle ou aryle;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle; et
R¹⁰ est alkyle, aryle ou aralkyle.

24. Utilisation du composé selon la revendication 23 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, alcényle, cycloalkyle éventuellement substitué, cycloalkylalkyle éventuellement substitué ou cycloalkylalcényle;
R³ est hétéroaryle éventuellement substitué, hétéroarylalkyle éventuellement substitué ou hétéroarylalcényle éventuellement substitué;
chaque R⁴ est indépendamment l'hydrogène, alkyle, halo, ou haloalkyle;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle; et
R¹⁰ est alkyle, aryle ou aralkyle.

25. Utilisation du composé selon la revendication 24 où le composé de formule (I) est un composé où:
m est 1 ou 2;
n est 1;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2) ou alkyle;
R³ est hétéroaryle éventuellement substitué, hétéroarylalkyle éventuellement substitué ou hétéroarylalcényle éventuellement substitué;
chaque R⁴ est indépendamment l'hydrogène, halo ou haloalkyle;
R⁵ est l'hydrogène;
chaque R⁶ est l'hydrogène;
R⁷ est une chaîne alkylène linéaire ou ramifiée;
R⁸ est l'hydrogène ou alkyle; et
R¹⁰ est alkyle, aryle ou aralkyle.

26. Utilisation du composé selon la revendication 25 où le composé de formule (I) est choisi dans le groupe consistant en les suivants:
6-[4-(3-méthyl-3H-1|4-thiophène-2-carbonyl)-pipérazin-1-yl]-*N*-pentyl-nicotinamide;
*N*-(3-diméthylamino-propyl)-6-[4-(3-méthyl-thiophène-2-carbonyl)-pipérazin-1-yl]-nicotinamide;
*N*-(3-diméthylamino-propyl)-6-[4-(thiophène-2-carbonyl)-pipérazin-1-yl]-nicotinamide;
6-[4-(2-chloro-pyridine-3-carbonyl)-pipérazin-1-yl]-*N*-(3-éthoxy-propyl)-nicotinamide;
6-[4-(2-chloro-pyridine-3-carbonyl)-pipérazin-1-yl]-*N*-(2-éthylsulfanyl-éthyl)-nicotinamide;
6-[4-(2-chloro-pyridine-3-carbonyl)-pipérazin-1-yl]-*N*-(3-méthylbutyl)-nicotinamide;
6-[4-(2-chloro-pyridine-3-carbonyl)-pipérazin-1-yl)-*N*-(3-isopropoxy-propyl)-nicotinamide;
*N*-(2-éthylsulfanyl-éthyl)-6-[4-(thiophène-2-carbonyl)-pipérazin-1-yl]-nicotinamide;
*N*-(3-éthoxy-propyl)-6-[4-(thiophène-2-carbonyl)-pipérazin-1-yl]-nicotinamide;
*N*-(3-méthyl-butyl)-6-[4-(thiophène-2-carbonyl)-pipérazin-1-yl]-nicotinamide;
6-[4-(2-chloro-pyridine-3-carbonyl)-pipérazin-1-yl]-*N*-(3-méthoxy-propyl)-nicotinamide;
*N*-pentyl-6-[4-(thiophène-2-carbonyl)-pipérazin-1-yl]-nicotinamide:
6-[4-(2-chloro-pyridine-3-carbonyl)-pipérazin-1-yl]-*N*-hexyl-nicotinamide;
*N*-(3-isopropoxy-propyl)-6-[4-(thiophène-2-carbonyl)-pipérazin-1-yl]-nicotinamide;
*N*-(1-méthyl-butyl)-6-(4-(thiophène-2-carbonyl)-pipérazin-1-yl]-nicotinamide;
*N*-hexyl-6-[4-(thiophène-2-carbonyl)-pipérazin-1-yl]-nicotinamide;
*N*-(1,3-diméthylbutyl)-6-[4-(thiophène-2-carbonyl)-pipérazin-1-yl]-nicotinamide;
*N*-butyl-6-[4-(thiophène-2-carbonyl)-pipérazin-1-yl]-nicotinamide;
*N*-(3-méthoxy-propyl)-6-[4-(thiophène-2-carbonyl)-pipérazin-1-yl]-nicotinamide;
6-[4-(2-chloro-pyridine-3-carbonyl)-pipérazin-1-yl]-*N*-(1,3-diméthylbutyl)-nicotinamide ;
*N*-butyl-6-[4-(2-chloro-pyridine-3-carbonyl)-pipérazin-1-yl]-nicotinamide;
6-[4-(2-chloro-pyridine-3-carbonyl)-pipérazin-1-yl]-*N*-(2-méthylbutyl)-nicotinamide ;
*N*-(2-méthyl-butyl)-6-[4-(thiophène-2-carbonyl)-pipérazin-1-yl]-nicotinamide;
6-[4-(2-chloro-pyridine-3-carbonyl)-pipérazin-1-yl]-*N*-(1-méthylbutyl)-nicotinamide;
*N*-(1,3-diméthylbutyl)-6-[4-(3-méthyl-thiophène-2-carbonyl)-pipérazin-1-yl]-nicotinamide;
*N*-butyl-6-[4-(3-méthyl-thiophène-2-carbonyl)-pipérazin-1-yl]-nicotinamide;
*N*-(3-butoxy-propyl)-6-[4-(3-méthyl-thiophène-2-carbonyl)-pipérazin-1-yl]-nicotinamide;
*N*-(3-isopropoxy-propyl)-6-[4-(3-méthyl-thiophène-2-carbonyl)-pipérazin-1-yl]-nicotinamide;
*N*-(3-éthoxy-propyl)-6-[4-(3-méthyl-thiophène-2-carbonyl)-pipérazin-1-yl]-nicotinamide;
*N*-(2-méthyl-butyl)-6-[4-(3-méthyl-thiophène-2-carbonyl)-pipérazin-1-yl]-nicotinamide;
*N*-(2-éthylsulfanyl-éthyl)-6-(4-(3-méthyl-thiophène-2-carbonyl)-pipérazin-1-yl]-nicotinamide;
*N*-(3-méthoxy-propyl)-6-[4-(3-méthyl-thiophène-2-carbonyl)-pipérazin-1-yl]-nicotinamide;
*N*-(1-méthyl-butyl)-6-[4-(3-méthyl-thiophène-2-carbonyl)-pipérazin-1-yl]-nicotinamide;
*N*-(3-méthyl-butyl)-6-[4-(3-méthyl-thiophène-2-carbonyl)-pipérazin-1-yl]-nicotinamide;
*N*-(3-butoxy-propyl)-6-[4-(2-chloro-pyridine-3-carbonyl)-pipérazin-1-yl]-nicotinamide;
*N*-(3-butoxy-propyl)-6-[4-(thiophène-2-carbonyl)-pipérazin-1-yl]-nicotinamide;
6-[4-(2-chloro-pyridine-3-carbonyl)-pipérazin-1-yl]-*N*-pentyl-nicotinamide;
*N*-hexyl-6-[4-(3-méthyl-thiophène-2-carbonyl)-pipérazin-1-yl]-nicotinamide; et
6-[4-(2-chloro-pyridine-3-carbonyl)-pipérazin-1-yl]-*N*-(3-diméthylamino-propyl)-nicotinamide.

27. Utilisation du composé selon la revendication 23 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est hétéroaryle éventuellement substitué, hétéroarylalkyle éventuellement substitué ou hétéroarylalcényle éventuellement substitué;
chaque R⁴ est indépendamment l'hydrogène, alkyle, halo, ou haloalkyle; et
chaque R⁵ et R⁶ est indépendamment l'hydrogène, oxo, alkyle, halo ou haloalkyle.

28. Utilisation du composé selon la revendication 27 où le composé de formule (I) est un composé où:
m est 1 ou 2;
n est 1;
p est 2;
V est -C(O)-;
R¹ est l'hydrogène ou alkyle;
R² est choisi dans le groupe consistant en aryle éventuellement substitué, aralkyle éventuellement substitué, hétérocyclyle éventuellement substitué, hétérocyclylalkyle éventuellement substitué, hétéroaryle éventuellement substitué et hétéroarylalkyle éventuellement substitué;
R³ est hétéroaryle éventuellement substitué, hétéroarylalkyle éventuellement substitué ou hétéroarylalcényle éventuellement substitué;
chaque R⁴ est indépendamment l'hydrogène, alkyle, halo, ou haloalkyle;
R⁵ est l'hydrogène, oxo, alkyle, halo ou haloalkyle; et
chaque R⁶ est indépendamment l'hydrogène, oxo, alkyle, halo ou haloalkyle.

29. Utilisation du composé selon la revendication 21 où le composé de formule (I) est choisi dans le groupe consistant en les suivants:
*N*-[2-(3-chlorophényl)-éthyl]-6-[4-(3-méthyl-thiophène-2-carbonyl)-pipérazin-1-yl]-nicotinamide;
*N*-[2-(3-chlorophényl)-éthyl]-6-{4-[2-(2-chloro-pyridin-3-yl)-acétyl]-pipérazin-1-yl}-nicotinamide ;
6-[4-(2-chloro-pyridine-3-carbonyl)-pipérazin-1-yl]-*N*-(4-phényl-butyl)-nicotinamide ;
*N*-[2-(3-chlorophényl)-éthyl]-6-[4-(thiophène-2-carbonyl)-pipérazin-1-yl]-nicotinamide ;
6-[4-(2-chloro-pyridine-3-carbonyl)-pipérazin-1-yl]-*N*-(3-phényl-propyl)-nicotinamide;
*N*-(3-phényl-propyl)-6-[4-(thiophène-2-carbonyl)-pipérazin-1-yl]-nicotinamide;
*N*-phénéthyl-6-[4-(thiophène-2-carbonyl)-pipérazin-1-yl]-nicotinamide;
*N*-(1-méthyl-3-phényl-propyl)-6-[4-(thiophène-2-carbonyl)-pipérazin-1-yl]-nicotinamide;
*N*-(4-phényl-butyl)-6-[4-(thiophène-2-carbonyl)-pipérazin-1-yl]-nicotinamide;
6-[4-(2-chloro-pyridine-3-carbonyl)-pipérazin-1-yl]-*N*-(1-méthyl-3-phényl-propyl)-nicotinamide;
6-[4-(2-chloro-pyridine-3-carbonyl)-pipérazin-1-yl]-*N*-phénéthyl-nicotinamide;
6-[4-(2-chloro-pyridine-3-carbonyl)-pipérazin-1-yl]-*N*-(tétrahydro-furan-2-yl-méthyl)-nicotinamide;
6-[4-(3-méthyl-thiophène-2-carbonyl)-pipérazin-1-yl]-*N*-(3-phényl-propyl)-nicotinamide;
*N*-(1-méthyl-3-phényl-propyl)-6-[4-(3-méthyl-thiophène-2-carbonyl)-pipérazin-1-yl]-nicotinamide;
6-(4-(3-méthyl-thiophène-2-carbonyl)-pipérazin-1-yl]-*N*-(4-phényl-butyl)-nicotinamide;
6-[4-(3-méthyl-thiophène-2-carbonyl)-pipérazin-1-yl]-*N*-phénéthyl-nicotinamide;
6-[4-(3-méthyl-thiophène-2-carbonyl)-pipérazin-1-yl]-*N*-(tétrahydro-furan-2-ylméthyl]-nicotinamide;
*N*-(3-imidazol-1-yl-propyl)-6-[4-(thiophène-2-carbonyl)-pipérazin-1-yl]-nicotinamide;
*N*-(tétrahydro-furan-2-ylméthyl)-6-[4-(thiophène-2-carbonyl)-pipérazin-1-yl]-nicotinamide;
*N*-[2-(3H-imidazol-4-yl)-éthyl]-6-[4-(thiophène-2-carbonyl)-pipérazin-1-yl]-nicotinamide;
*N*-(3-imidazol-1-yl-propyl)-6-[4-(3-méthyl-thiophène-2-cartbonyl)-pipérazin-1-yl]-nicotinamide;
*N*-[2-(3H-imidazol-4-yl)-éthyl]-6-[4-(3-méthyl-thiophène-2-carbonyl)-pipérazin-1-yl]-nicotinamide;
6-[4-(2-chloro-pyridine-3-carbonyl)-pipérazin-1-yl]-*N*-(3-imidazol-1-yl-propyl)-nicotinamide; et
6-[4-(2-chloro-pyridine-3-carbonyl)-pipérazin-1-yl]-*N*-[2-(3H-imidazol-4-yl)-éthyl]-nicotinamide.

30. Utilisation du composé selon la revendication 1 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle éventuellement substitué, aralcényle éventuellement substitué, cycloalkyle éventuellement substitué, cycloalkylalkyle éventuellement substitué, cycloalkylalcényle, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est aralkyle éventuellement substitué ou aralcényle éventuellement substitué;
chaque R⁴ est indépendamment l'hydrogène, alkyle, alcényle, halo, haloalkyle, aryle ou -R⁹-OR⁸;
chaque R⁵ et R⁶ est indépendamment l'hydrogène, oxo, alkyle, alcényle, halo, haloalkyle ou aryle;
chaque R⁷ est indépendamment une chaîne alkylène ou alcénylène linéaire ou ramifiée; chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
R⁹ est une liaison directe ou une chaîne alkylène linéaire ou ramifiée; et
R¹⁰ est alkyle, aryle ou aralkyle.

31. Utilisation du composé selon la revendication 30 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, alcényle, cycloalkyle éventuellement substitué, cycloalkylalkyle éventuellement substitué ou cycloalkylalcényle;
R³ est aralkyle éventuellement substitué ou aralcényle éventuellement substitué;
chaque R⁴ est indépendamment l'hydrogène, alkyle, halo, ou haloalkyle;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle; et
R¹⁰ est alkyle, aryle ou aralkyle.

32. Utilisation du composé selon la revendication 31 où le composé de formule (I) est un composé où:
m est 1 ou 2;
n est 1;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2) ou alkyle;
R³ est aralkyle éventuellement substitué ou aralcényle éventuellement substitué;
chaque R⁴ est indépendamment l'hydrogène, halo ou haloalkyle;
R⁵ est l'hydrogène;
chaque R⁶ est l'hydrogène;
R⁷ est une chaîne alkylène linéaire ou ramifiée;
R⁸ est l'hydrogène ou alkyle; et
R¹⁰ est alkyle, aryle ou aralkyle.

33. Utilisation du composé selon la revendication 32 où le composé de formule (I) est choisi dans le groupe consistant en les suivants:
6-{4-[2-(4-chlorophényl)-propionyl]-pipérazin-1-yl}-*N*-(3-méthylbutyl)-nicotinamide;
6-{4-[2-(2-chloro-6-fluoro-phényl)-acétyl]-pipérazin-1-yl}-*N*-(3-éthoxy-propyl)-nicotinamide;
6-{4-[3-(3,4-difluoro-phényl)-propionyl]-pipérazin-1-yl)-*N*-(3-diméthylaminopropyl)-nicotinamide;
6-{4-[3-(3,4-difluoro-phényl)-propionyl]-pipérazin-1-yl}-*N*-(3-éthoxy-propyl)-nicotinamide;
6-{4-[2-(4-chlorophényl)-propionyl]-pipérazin-1-yl}-*N*-(3-éthoxy-propyl)-nicotinamide;
*N*-(3-éthoxy-propyl)-6-[4-(2-phényl-butyryl)-pipérazin-1-yl]-nicotinamide;
*N*-(2-éthylsulfanyl-éthyl)-6-[4-(2-o-tolyl-acétyl)-pipérazin-1-yl]-nicotinamide;
6-[4-(3-méthyl-pentanoyl)-pipérazin-1-yl]-*N*-(4-phényl-butyl)-nicotinamide;
*N*-butyl-6-{4-[3-(3,4-difluoro-phényl)-propionyl]-pipérazin-1-yl}-nicotinamide;
6-{4-[2-(2-chloro-6-fluoro-phényl)-acétyl]-pipérazin-1-yl)-*N*-(3-diméthylaminopropyl)-nicotinamide;
6-{4-[3-(3,4-difluoro-phényl)-propionyl]-pipérazin-1-yl}-*N*-(2-éthylsulfanyléthyl)-nicotinamide;
*N*-(3-méthoxy-propyl)-6-[4-(2-phényl-butyryl)-pipérazin-1-yl]-nicotinamide;
*N*-butyl-6-[4-(2-phényl-butyryl)-pipérazin-1-yl]-nicotinamide;
*N*-(3-butoxy-propyl)-6-{4-[2-(4-chloro-phényl)-propionyl]-pipérazin-1-yl}-nicotinamide;
*N*-(3-méthoxy-propyl)-6-[4-(2-o-tolyl-acétyl)-pipérazin-1-yl]-nicotinamide;
*N*-(3-méthyl-butyl)-6-[4-(2-phényl-butyryl)-pipérazin-1-yl]-nicotinamide;
*N*-(1-méthyl-butyl)-6-[4-(2-p-tolyl-acétyl)-pipérazin-1-yl]-nicotinamide;
*N*-butyl-6-{4-[2-(2-chloro-6-fluoro-phényl)-acétyl]-pipérazin-1-yl}-nicotinamide;
*N*-(2-méthyl-butyl)-6-[4-(2-o-tolyl-acétyl)-pipérazin-1-yl]-nicotinamide;
6-{4-[3-(3,4-difluoro-phényl)-propionyl]-pipérazin-1-yl}-*N*-(2-méthylbutyl)-nicotinamide;
*N*-(3-isopropoxy-propyl)-6-[4-(2-o-tolyl-acétyl)-pipérazin-1-yl]-nicotinamide;
6-{4-[3-(3,4-difluoro-phényl)-propionyl]-pipérazin-1-yl}-*N*-(3-méthylbutyl)-nicotinamide;
6-{4-[3-(3,4-difluoro-phényl)-propionyl]-pipérazin-1-yl}-*N*-(3-méthoxy-propyl)-nicotinamide;
*N*-(1-méthyl-butyl)-6-[4-(2-o-tolyl-acétyl)-pipérazin-1-yl]-nicotinamide;
6-{4-[2-(2-chloro-6-fluoro-phényl)-acétyl]-pipérazin-1-yl}-*N*-(2-méthylbutyl)-nicotinamide;
6-{4-[2-(2-chloro-6-fluoro-phényl)-acétyl]-pipérazin-1-yl}-*N*-(1-méthylbutyl)-nicotinamide ;
*N*-(2-méthyl-butyl)-6-[4-(2-phényl-butyryl)-pipérazin-1-yl]-nicotinamide;
*N*-(1,3-diméthylbutyl)-6-[4-(2-phényl-butyryl)-pipérazin-1-yl]-nicotinamide;
6-{4-[2-(4-chlorophényl)-propionyl]-pipérazin-1-yl}-*N*-(2-méthylbutyl)-nicotinamide ;
6-{4-[2-(2-chloro-6-fluoro-phényl)-acétyl]-pipérazin-1-yl}-*N*-(3-isopropoxypropyl)-nicotinamide ;
*N*-(1,3-diméthylbutyl)-6-[4-(2-p-tolyl-acétyl)-pipérazin-1-yl]-nicotinamide;
6-{4-[3-(3,4-difluoro-phényl)-propionyl]-pipérazin-1-yl}-*N*-hexyl-nicotinamide;
*N*-(1-méthyl-butyl)-6-[4-(2-phényl-butyryl)-pipérazin-1-yl]-nicotinamide;
6-{4-[2-(4-chlorophényl)-propionyl]-pipérazin-1-yl}-*N*-(1-méthylbutyl)-nicotinamide;
6-{4-[2-(4-chlorophényl)-propionyl]-pipérazin-1-yl}-*N*-(1,3-diméthylbutyl)-nicotinamide;
*N*-butyl-6-[4-(2-p-tolyl-acétyl)-pipérazin-1-yl]-nicotinamide;
*N*-(1,3-diméthylbutyl)-6-[4-(2-o-tolyl-acétyl)-pipérazin-1-yl]-nicotinamide;
6-{4-{2-(2-chloro-6-fluoro-phényl)-acétyl]-pipérazin-1-yl}-*N*-(1,3-diméthylbutyl)-nicotinamide;
*N*-(3-méthyl-butyl)-6-[4-(2-p-tolyl-acétyl)-pipérazin-1-yl]-nicotinamide;
*N*-butyl-6-[4-(2-o-tolyl-acétyl)-pipérazin-1-yl]-nicotinamide;
*N*-(3-butoxy-propyl)-6-[4-(2-p-tolyl-acétyl)-pipérazin-1-yl]-nicotinamide;
*N*-pentyl-6-[4-(2-p-tolyl-acétyl)-pipérazin-1-yl]-nicotinamide;
*N*-(3-butoxy-propyl)-6-[4-(2-phényl-butyryl)-pipérazin-1-yl]-nicotinamide;
*N*-pentyl-6-[4-(2-phényl-butyryl)-pipérazin-1-yl]-nicotinamide;
*N*-(2-éthylsulfanyl-éthyl)-6-[4-(2-p-tolyl-acétyl)-pipérazin-1-yl]-nicotinamide;
6-{4-[2-(4-chlorophényl)-propionyl]-pipérazin-1-yl}-*N*-(2-éthylsulfanyl-éthyl)-nicotinamide;
*N*-(3-éthoxy-propyl)-6-[4-(2-o-tolyl-acétyl)-pipérazin-1-yl]-nicotinamide;
6-{4-[2-(2-chloro-6-fluoro-phényl)-acétyl]-pipérazin-1-yl}-*N*-(3-méthoxypropyl)-nicotinamide;
6-{4-[3-(3,4-difluoro-phényl)-propionyl]-pipérazin-1-yl}-*N*-(3-isopropoxypropyl)-nicotinamide;
6-{4-[2-(4-chlorophényl)-propionyl]-pipérazin-1-yl}-*N*-pentyl-nicotinamide;
*N*-(3-butoxy-propyl)-6-{4-[2-(2-chloro-6-fluoro-phényl)-acétyl]-pipérazin-1-yl}-nicotinamide;
6-{4-[2-(2-chloro-6-fluoro-phényl)-acétyl]-pipérazin-1-yl}-*N*-hexyl-nicotinamide;
6-{4-[2-(4-chlorophényl)-propionyl]-pipérazin-1-yl}-*N*-(3-méthoxy-propyl)-nicotinamide ;
*N*-hexyl-6-[4-(2-o-tolyl-acétyl)-pipérazin-1-yl]-nicotinamide;
6-{4-[3-(3,4-difluoro-phényl)-propionyl]-pipérazin-1-yl}-*N*-(4-phényl-butyl)-nicotinamide ;
*N*-(3-isopropoxy-propyl)-6-[4-(2-p-tolyl-acétyl)-pipérazin-1-yl]-nicotinamide;
6-{4-[3-(3,4-difluoro-phényl)-propionyl]-pipérazin-1-yl}-*N*-(1-méthylbutyl)-nicotinamide;
6-{4-[3-(3,4-difluoro-phényl)-propionyl]-pipérazin-1-yl}-*N*-pentyl-nicotinamide;
6-{4-[2-(4-chlorophényl)-propionyl]-pipérazin-1-yl}-*N*-hexyl-nicotinamide;
*N*-butyl-6-{4-[2-(4-chloro-phényl)-propionyl]-pipérazin-1-yl}-nicotinamide;
6-{4-[2-(2-chloro-6-fluoro-phényl)-acétyl]-pipérazin-1-yl}-*N*-(2-éthylsulfanyléthyl)-nicotinamide ;
6-{4-[2-(2-chloro-6-fluoro-phényl)-acétyl]-pipérazin-1-yl}-*N*-pentylnicotinamide;
*N*-hexyl-6-[4-(2-phényl-butyryl)-pipérazin-1-yl]-nicotinamide;
*N*-(3-isopropoxy-propyl)-6-[4-(2-phényl-butyryl)-pipérazin-1-yl]-nicotinamide;
*N*-pentyl-6-[4-(2-o-tolyl-acétyl)-pipérazin-1-yl]-nicotinamide;
*N*-hexyl-6-[4-(2-p-tolyl-acétyl)-pipérazin-1-yl]-nicotinamide;
6-{4-[3-(3,4-difluoro-phényl)-propionyl]-pipérazin-1-yl}-*N-*(1,3-diméthylbutyl)-nicotinamide;
6-[4-(naphtalène-2-carbonyl)-pipérazin-1-yl]-*N*-pentyl-nicotinamide;
6-{4-[2-(4-chlorophényl)-propionyl]-pipérazin-1-yl}-*N*-(3-diméthylaminopropyl)-nicotinamide;
*N*-(3-diméthylamino-propyl)-6-[4-(2-phényl-butyryl)-pipérazin-1-yl]-nicotinamide;
*N*-(3-diméthylamino-propyl)-6-[4-(2-p-tolyl-acétyl)-pipérazin-1-yl]-nicotinamide;
*N*-(3-diméthylamino-propyl)-6-[4-(2-o-tolyl-acétyl)-pipérazin-1-yl]-nicotinamide;
*N*-(3-butoxy-propyl)-6-{4-[3-(3,4-difluoro-phényl)-propionyl]-pipérazin-1-yl}-nicotinamide;
*N*-(3-méthoxy-propyl)-6-[4-(2-p-tolyl-acétyl)-pipérazin-1-yl]-nicotinamide;
*N*-(2-méthyl-butyl)-6-[4-(2-p-tolyl-acétyl)-pipérazin-1-yl]-nicotinamide;
*N*-(3-butoxy-propyl)-6-[4-(2-o-tolyl-acétyl)-pipérazin-1-yl]-nicotinamide; et
6-{4-[2-(4-chlorophényl)-propionyl]-pipérazin-1-yl}-*N*-(3-isopropoxy-propyl)-nicotinamide.

34. Utilisation du composé selon la revendication 30 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est aralkyle éventuellement substitué ou aralcényle éventuellement substitué;
chaque R⁴ est indépendamment l'hydrogène, alkyle, halo, ou haloalkyle; et
chaque R⁵ et R⁶ est indépendamment l'hydrogène, oxo, alkyle, halo ou haloalkyle.

35. Utilisation du composé selon la revendication 34 où le composé de formule (I) est un composé où:
m est 1 ou 2;
n est 1;
p est 2;
V est -C(O)-;
R¹ est l'hydrogène ou alkyle;
R² est choisi dans le groupe consistant en aryle éventuellement substitué, aralkyle éventuellement substitué, hétérocyclyle éventuellement substitué, hétérocyclylalkyle éventuellement substitué, hétéroaryle éventuellement substitué et hétéroarylalkyle éventuellement substitué;
R³ est aralkyle éventuellement substitué ou aralcényle éventuellement substitué;
chaque R⁴ est indépendamment l'hydrogène, alkyle, halo ou haloalkyle; et
R⁵ est l'hydrogène, oxo, alkyle, halo ou haloalkyle; et
chaque R⁶ est indépendamment l'hydrogène, oxo, alkyle, halo ou haloalkyle.

36. Utilisation du composé selon la revendication 35 où le composé de formule (I) est choisi dans le groupe consistant en les suivants:
6-[4-(2-phényl-butyryl)-pipérazin-1-yl]-*N*-(3-phényl-propyl)-nicotinamide;
6-{4-[3-(3,4-difluoro-phényl)-propionyl]-pipérazin-1-yl}-*N*-phénéthylnicotinamide;
*N*-(2-(3-chlorophényl)-éthyl]-6-[4-(2-o-tolyl-acétyl)-pipérazin-1-yl]-nicotinamide;
6-{4-[2-(2-chloro-6-fluoro-phényl)-acétyl]-pipérazin-1-yl}-*N*-(3-phényl-propyl)-nicotinamide ;
*N*-(1-méthyl-3-phényl-propyl)-6-[4-(2-p-tolyl-acétyl)-pipérazin-1-yl]-nicotinamide;
*N*-[2-(3-chlorophényl)-éthyl]-6-[4-(2-phényl-butyryl)-pipérazin-1-yl]-nicotinamide;
6-{4-[2-(2-chloro-6-fluoro-phényl)-acétyl]-pipérazin-1-yl}-*N*-phénéthyl-nicotinamide ;
*N*-(3-phényl-propyl)-6-[4-(2-o-tolyl-acétyl)-pipérazin-1-yl]-nicotinamide;
6-{4-[2-(2-chloro-6-fluoro-phényl)-acétyl]-pipérazin-1-yl}-*N*-[2-(3-chloro-phényl)-éthyl]-nicotinamide;
6-{4-[2-(4-chlorophényl)-propionyl]-pipérazin-1-yl)-*N*-(3-phényl-propyl)-nicotinamide;
6-{4-[2-(4-chlorophényl)-propionyl]-pipérazin-1-yl}-*N*-(1-méthyl-3-phényl-propyl)-nicotinamide;
*N*-[2-(3-chlorophényl)-éthyl]-6-{4-[2-(4-chloro-phényl)-propionyl]-pipérazin-1-yl}-nicotinamide;
*N*-[2-(3-chlorophényl)-éthyl]-6-[4-(2-p-tolyl-acétyl)-pipérazin-1-yl]-nicotinamide;
*N*-phénéthyl-6-[4-(2-p-tolyl-acétyl)-pipérazin-1-yl]-nicotinamide;
*N*-(3-phényl-propyl)-6-[4-(2-p-tolyl-acétyl)-pipérazin-1-yl]-nicotinamide;
*N*-(1-méthyl-3-phényl-propyl)-6-[4-(2-phényl-butyryl)-pipérazin-1-yl]-nicotinamide;
6-{4-[2-(4-chlorophényl)-propionyl]-pipérazin-1-yl}-*N*-(4-phényl-butyl)-nicotinamide;
*N*-(4-phényl-butyl)-6-[4-(2-phényl-butyryl)-pipérazin-1-yl]-nicotinamide;
6-{4-[2-(2-chloro-6-fluoro-phényl)-acétyl]-pipérazin-1-yl}-*N*-(1-méthyl-3-phényl-propyl)-nicotinamide;
6-{4-[3-(3,4-difluoro-phényl)-propionyl]-pipérazin-1-yl}-*N*-(3-phényl-propyl)-nicotinamide;
*N*-phénéthyl-6-[4-(2-o-tolyl-acétyl)-pipérazin-1-yl]-nicotinamide;
6-{4-[3-(3,4-difluoro-phényl)-propionyl)-pipérazin-1-yl}-*N*-(1-méthyl-3-phényl-propyl)-nicotinamide;
*N*-(4-phényl-butyl)-6-[4-(2-o-tolyl-acétyl)-pipérazin-1-yl]-nicotinamide;
*N*-(3-imidazol-1-yl-propyl)-6-[4-(2-phényl-butyryl)-pipérazin-1-yl]-nicotinamide;
6-{4-[2-(2-chloro-6-fluoro-phényl)-acétyl]-pipérazin-1-yl}-*N*-(4-phényl-butyl)-nicotinamide;
6-{4-[2-(4-chlorophényl)-propionyl]-pipérazin-1-yl}-*N*-[2-(3H-imidazol-4-yl)-éthyl]-nicotinamide;
6-{4-[2-(2-chloro-6-fluoro-phényl)-acétyl]-pipérazin-1-yl}-*N*-(3-imidazol-1-yl-propyl)-nicotinamide;
*N*-[2-(3H-imidazol-4-yl)-éthyl]-6-[4-(2-p-tolyl-acétyl)-pipérazin-1-yl]-nicotinamide;
6-{4-[2-(4-chlorophényl)-propionyl]-pipérazin-1-yl}-*N*-(3-imidazol-1-yl-propyl)-nicotinamide;
*N*-(2-éthylsulfanyl-éthyl)-6-[4-(2-phényl-butyryl)-pipérazin-1-yl]-nicotinamide;
*N*-(1-méthyl-3-phényl-propyl)-6-[4-(2-o-totyl-acétyl)-pipérazin-1-yl]-nicotinamide;
6-{4-[3-(3,4-difluoro-phényl)-propionyl]-pipérazin-1-yl}-*N*-phénéthyl-nicotinamide;
*N*-phénéthyl-6-[4-(2-phényl-butyryl)-pipérazin-1-yl]-nicotinamide;
*N*-(tétrahydro-furan-2-ylméthyl)-6-[4-(2-p-tolyl-acétyl)-pipérazin-1-yl]-nicotinamide;
6-{4-[2-(4-chlorophényl)-propionyl]-pipérazin-1-yl}-*N*-(tétrahydro-furan-2-ylméthyl)-nicotinamide;
*N*-(4-phényl-butyl)-6-[4-(2-p-tolyl-acétyl)-pipérazin-1-yl]-nicotinamide;
6-{4-[3-(3,4-difluoro-phényl)-propionyl]-pipérazin-1-yl}-*N*-(tétrahydro-furan-2-ylméthyl)-nicotinamide;
*N*-(tétrahydro-furan-2-ylméthyl)-6-[4-(2-o-tolyl-acétyl)-pipérazin-1-yl]-nicotinamide;
6-[4-(2-phényl-butyryl)-pipérazin-1-yl]-*N*-(tétrahydro-furan-2-ylméthyl)-nicotinamide;
6-{4-[3-(3,4-difluoro-phényl)-propionyl]-pipérazin-1-yl}-*N*-[2-(3H-imidazol-4-yl)-éthyl]-nicotinamide;
*N*-(2-(3H-imidazol-4-yl)-éthyl]-6-[4-(2-o-tolyl-acétyl)-pipérazin-1-yl]-nicotinamide;
*N*-(3-imidazol-1-yl-propyl)-6-[4-(2-o-tolyl-acétyl)-pipérazin-1-yl]-nicotinamide;
6-{4-[3-(3,4-difluoro-phényl)-propionyl]-pipérazin-1-yl}-*N*-(3-imidazol-1-yl-propyl)-nicotinamide;
*N*-[2-(3H-imidazol-4-yl)-éthyl]-6-[4-(2-phényl-butyryl)-pipérazin-1-yl]-nicotinamide;
*N*-(3-imidazol-1-yl-propyl)-6-[4-(2-p-tolyl-acétyl)-pipérazin-1-yl]-nicotinamide;
6-{4-[2-(2-chloro-6-tluoro-phényl)-acétyl]-pipérazin-1-yl }-*N*-(tétrahydro-furan-2-ylméthyl)-nicotinamide; et
6-{4-[2-(2-chloro-6-fluoro-phényl)-acétyl]-pipérazin-1-yl}-*N*-[2-(3H-imidazol-4-yl)-éthyl]-nicotinamide.

37. Utilisation du composé selon la revendication 1 où le mammifère est un humain.

38. Utilisation du composé selon la revendication 37 où la maladie ou affection est une maladie ou affection liée aux niveaux sériques de triglycéride, VLDL, HDL, LDL, cholestérol total ou au processus de transport inverse du cholestérol.

39. Utilisation du composé selon la revendication 37 où la maladie ou affection est une maladie ou affection liée aux niveaux sériques de triglycéride.

40. Utilisation du composé selon la revendication 37 où la maladie ou affection est une maladie ou affection liée aux niveaux sériques de cholestérol.

41. Utilisation du composé selon la revendication 37, où la maladie ou affection est le diabète de type II.

42. Utilisation du composé selon la revendication 37, où la maladie ou affection est l'obésité.

43. Utilisation du composé selon la revendication 37, où la maladie ou affection est la dyslipidémie.

44. Utilisation du composé selon la revendication 37, où la maladie ou affection est le syndrome métabolique.

45. Composé de formule (1): 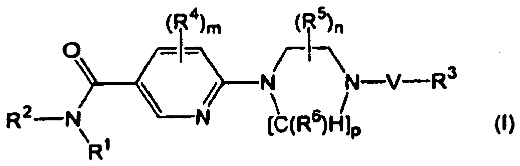 où:
m est 1, 2 ou 3;
n est 1, 2, 3 ou 4;
p est 2, 3 ou 4;
V est -C(O)-, -S(O)- ou -S(O)₂-;
R¹ est l'hydrogène, alkyle, alcényle, aryle, aralkyle, aralcényle ou cycloalkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle, où le radical aryle peut être éventuellement substitué, aralcényle, où le radical aryle peut être éventuellement substitué, cycloalkyle éventuellement substitué, cycloalkylalkyle éventuellement substitué, cycloalkylalcényle, hétérocyclyle éventuellement substitué, hétérocyclylalkyle, où le radical hétérocyclyle peut être éventuellement substitué, hétérocyclylalcényle, hétéroaryle éventuellement substitué, hétéroarylalkyle, où le radical hétéroaryle peut être éventuellement substitué et hétéroarylalcényle, où le radical hétéroaryle peut être éventuellement substitué;
R³ est choisi dans le groupe consistant en l'hydrogène, -R⁹-OR⁸, -R⁹-N(R⁸)₂, alkyle, alcényle, aryle éventuellement substitué, aralkyle, où le radical aryle peut être éventuellement substitué, aralcényle, où le radical aryle peut être éventuellement substitué, cycloalkyle éventuellement substitué, cycloalkylalkyle éventuellement substitué, cycloalkylalcényle, hétérocyclyle éventuellement substitué, hétérocyclylalkyle, où le radical hétérocyclyle peut être éventuellement substitué, hétérocyclylalcényle, hétéroaryle éventuellement substitué, hétéroarylalkyle, où le radical hétéroaryle peut être éventuellement substitué et hétéroarylalcényle, où le radical hétéroaryle peut être éventuellement substitué;
chaque R⁴ est indépendamment l'hydrogène, alkyle, alcényle, halo, haloalkyle, aryle, cyano, nitro, -R⁹-OR⁸, -R⁹-N(R⁸)₂ ou -S(O)ₜR¹⁰ (où t est 0, 1 ou 2);
chaque R⁵ et R⁶ est indépendamment l'hydrogène, oxo, alkyle, alcényle, halo, haloalkyle ou aryle;
ou un R⁵ et un R⁶ peuvent former ensemble un pont alkylène linéaire ou ramifié;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
chaque R⁹ est indépendamment une liaison directe ou une chaîne alkylène ou alcénylène linéaire ou ramifiée; et
R¹⁰ est alkyle, alcényle, haloalkyle, cycloalkyle, cycloalkylalkyle, aryle, aralkyle, hétérocyclyle, hétérocyclylalkyle, hétéroaryle ou hétéroarylalkyle;
sous forme d'un seul stéréoisomère, d'un mélange de stéréoisomères, d'un mélange racémique de stéréoisomères, ou sous forme de tautomère;
ou sous forme de sel pharmaceutiquement acceptable, solvate, polymorphe ou promédicament, choisi parmi les dérivés esters et amides de groupes fonctionnels hydroxyle, carboxy, mercapto ou amino dans les composés de formule (I), destiné à être utilisé dans le traitement d'une maladie ou affection à médiation par SCD chez un mammifère, où la maladie ou affection est choisie dans le groupe consistant en le diabète de type II, la tolérance au glucose dégradée, la résistance à l'insuline, l'hypertension, l'obésité, l'hypertriglycéridémie, les HDL basses, la lipidémie, la dyslipidémie, la microalbuminémie, l'hyperuricémie, l'hyperleptinémie, le syndrome métabolique, et toute combinaison de celles-ci ; et
où le substituant éventuel est un ou plusieurs substituants choisis indépendamment dans le groupe consistant en alkyle, alcényle, halo, haloalkyle, haloalcényle, cyano, nitro, aryle, aralkyle, cycloalkyle, cycloalkylalkyle, hétérocyclyle, hétérocyclylalkyle, hétéroaryle, hétéroarylalkyle, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (où t est 1 ou 2), -R⁹-S(O)ₜOR¹⁰ (où t est 1 ou 2), -R⁹-S(O)ₜR¹⁰ (où t est 0, 1 ou 2), et -R⁹-S(O)ₜN(R⁸)₂ (où t est 1 ou 2).

46. Composé destiné à être utilisé selon la revendication 45 où le composé de formule (I) est un composé où:
m est 1 ou 2;
n est 1 ou 2;
p est 2 ou 3;
V est -C(O)- ou-S(O)₂-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle éventuellement substitué, aralcényle éventuellement substitué, cycloalkyle éventuellement substitué, cycloalkylalkyle éventuellement substitué, cycloalkylalcényle, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est alkyle, alcényle ou -R⁹-N(R⁸)₂;
chaque R⁴ est indépendamment l'hydrogène, alkyle, alcényle, halo, haloalkyle, aryle ou -R⁹-OR⁸;
chaque R⁵ et R⁶ est indépendamment l'hydrogène, oxo, alkyle, alcényle, halo, haloalkyle ou aryle;
ou un R⁵ et un R⁶ peuvent former ensemble un pont alkylène linéaire ou ramifié;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
chaque R⁹ est indépendamment une liaison directe ou une chaîne alkylène linéaire ou ramifiée; et
R¹⁰ est alkyle, aryle ou aralkyle.

47. Composé destiné à être utilisé selon la revendication 46 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, alcényle, cycloalkyle éventuellement substitué, cycloalkylalkyle éventuellement substitué ou cycloalkylalcényle;
R³ est alkyle;
chaque R⁴ est indépendamment l'hydrogène, alkyle, halo, ou haloalkyle;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle; et
R¹⁰ est alkyle, aryle ou aralkyle.

48. Composé destiné à être utilisé selon la revendication 47 où le composé de formule (I) est un composé où:
m est 1;
n est 1;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0,1 ou 2) ou alkyle;
R³ est alkyle;
R⁴ est l'hydrogène;
R⁵ est l'hydrogène;
chaque R⁶ est l'hydrogène;
R⁷ est une chaîne alkylène linéaire ou ramifiée;
R⁸ est l'hydrogène ou alkyle; et
R¹⁰ est alkyle, aryle ou aralkyle.

49. Composé destiné à être utilisé selon la revendication 48 où le composé de formule (I) est choisi dans le groupe consistant en les suivants:
*N*-(3-éthoxy-propyl)-6-[4-(2-éthylbutyryl)-pipérazin-1-yl]-nicotinamide;
*N*-(3-éthoxy-propyl)-6-(4-pentanoyl-pipérazin-1-yl)-nicotinamide;
*N*-(3-éthoxy-propyl)-6-[4-(3-méthyl-pentanoyl)-pipérazin-1-yl]-nicotinamide;
*N*-(3-butoxy-propyl)-6-(4-pentanoyl-pipérazin-1-yl)-nicotinamide;
*N*-(3-méthyl-butyl)-6-[4-(3-méthyl-pentanoyl)-pipérazin-1-yl]-nicotinamide;
6-[4-(3-méthyl-pentanoyl)-pipérazin-1-yl]-*N*-pentyl-nicotinamide;
*N*-butyl-6-[4-(3-méthyl-pentanoyl)-pipérazin-1-yl]-nicotinamide;
*N*-butyl-6-[4-(2-éthylbutyryl)-pipérazin-1-yl]-nicotinamide;
*N*-(3-méthyl-butyl)-6-(4-pentanoyl-pipérazin-1-yl)-nicotinamide;
*N*-(2-éthylsulfanyl-éthyl)-6-(4-pentanoyl-pipérazin-1-yl)-nicotinamide;
*N*-(3-méthoxy-propyl)-6-[4-(3-méthyl-pentanoyl)-pipérazin-1-yl]-nicotinamide;
6-(4-pentanoyl-pipérazin-1-yl)-*N*-pentyl-nicotinamide;
*N*-hexyl-6-[4-(3-méthyl-pentanoyl)-pipérazin-1-yl]-nicotinamide;
*N*-(1,3-diméthylbutyl)-6-[4-(3-méthyl-pentanoyl)-pipérazin-1-yl]-nicotinamide ;
*N*-(1-méthyl-butyl)-6-(4-pentanoyl-pipérazin-1-yl)-nicotinamide;
*N*-(3-diméthylamino-propyl)-6-[4-(2-éthylbutyryl)-pipérazin-1-yl]-ricotinamide;
*N*-(3-méthoxy-propyl)-6-(4-pentanoyl-pipérazin-1-yl)-nicotinamide;
*N*-(1,3-diméthylbutyl)-6-(4-pentanoyl-pipérazin-1-yl)-nicotinamide;
*N*-(2-méthyl-butyl)-6-(4-pentanoyl-pipérazin-1-yl)-nicotinamide;
*N*-(3-butoxy-propyl)-6-[4-(2-éthylbutyryl)-pipérazin-1-yl]-nicotinamide;
6-[4-(2-éthylbutyryl)-pipérazin-1-yl]-*N*-(3-méthylbutyl)-nicotinamide;
*N*-butyl-6-(4-pentanoyl-pipérazin-1-yl)-nicotinamide;
6-[4-(2-éthylbutyryl)-pipérazin-1-yl]-*N*-(2-méthylbutyl)-nicotinamide;
*N*-(3-isopropoxy-propyl)-6-(4-pentanoyl-pipérazin-1-yl)-nicotinamide;
*N*-(2-éthylsulfanyl-éthyl)-6-[4-(3-méthyl-pentanoyl)-pipérazin-1-yl]-nicotinamide;
6-[4-(2-éthylbutyryl)-pipérazin-1-yl]-*N*-(2-éthylsulfanyl-éthyl)-nicotinamide ;
6-[4-(2-éthylbutyryl)-pipérazin-1-yl]-*N*-hexyl-nicotinamide;
6-[4-(2-éthylbutyryl)-pipérazin-1-yl]-*N*-(1-méthylbutyl)-nicotinamide;
*N*-(2-méthyl-butyl)-6-[4-(3-méthyl-pentanoyl)-pipérazin-1-yl]-nicotinamide;
*N*-(1,3-diméthylbutyl)-6-[4-(2-éthylbutyryl)-pipérazin-1-yl]-nicotinamide;
6-[4-(2-éthylbutyryl)-pipérazin-1-yl]-*N*-pentyl-nicotinamide;
*N*-(3-isopropoxy-propyl)-6-[4-(3-méthyl-pentanoyl)-pipérazin-1-yl]-nicotinamide;
*N*-(3-diméthylamino-propyl)-6-[4-(3-méthyl-pentanoyl)-pipérazin-1-yl]-nicotinamide;
6-[4-(2-éthylbutyryl)-pipérazin-1-yl]-*N*-(3-méthoxy-propyl)-nicotinamide;
6-[4-(2-éthylbutyryl)-pipérazin-1-yl]-*N*-(3-isopropoxy-propyl)-nicotinamide;
*N*-(3-butoxy-propyl)-6-[4-(3-méthyl-pentanoyl)-pipérazin-1-yl]-nicotinamide;
*N*-(1-méthyl-butyl)-6-[4-(3-méthyl-pentanoyl)-pipérazin-1-yl]-nicotinamide;
*N*-hexyl-6-(4-pentanoyl-pipérazin-1-yl)-nicotinamide; et
*N*-(3-diméthylamino-propyl)-6-(4-pentanoyl-pipérazin-1-yl)-nicotinamide.

50. Composé destiné à être utilisé selon la revendication 46 où le composé de formule (I) est un composé où:
m est 1 ou 2;
n est 1 ou 2;
p est 2 ou 3;
V est -C(O)- ou -S(O)₂-;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est alkyle ou -R⁷-N(R⁸)₂;
chaque R⁴ est indépendamment l'hydrogène, alkyle, halo, ou haloalkyle; et
chaque R⁵ et R⁶ est indépendamment l'hydrogène, oxo, alkyle, alcényle, halo, haloalkyle ou aryle;
ou un R⁵ et un R⁶ peuvent former ensemble un pont alkylène linéaire ou ramifié;
R⁷ est une liaison directe; et
chaque R⁸ est indépendamment l'hydrogène ou alkyle.

51. Composé destiné à être utilisé selon la revendication 50 où le composé de formule (I) est un composé où:
m est 1;
n est 1;
p est 2 ou 3;
V est -C(O)- ou -S(O)₂-;
R¹ est l'hydrogène ou alkyle;
R² est choisi dans le groupe consistant en aryle éventuellement substitué, aralkyle éventuellement substitué, hétérocyclyle éventuellement substitué, hétérocyclylalkyle éventuellement substitué, hétéroaryle éventuellement substitué et hétéroarylalkyle éventuellement substitué;
R³ est alkyle ou -R⁷-N(R⁸)₂;
R⁴ est l'hydrogène, alkyle, halo ou haloalkyle; et
chaque R⁵ et R⁶ est indépendamment l'hydrogène, oxo, alkyle, halo ou
haloalkyle; ou un R⁵ et un R⁶ peuvent former ensemble un pont méthylène;
R⁷ est une liaison directe; et
chaque R⁸ est indépendamment l'hydrogène ou alkyle.

52. Composé destiné à être utilisé selon la revendication 51 où le composé de formule (I) est choisi dans le groupe consistant en les suivants:
6-[4-(2-éthylbutyryl)-pipérazin-1-yl]-*N*-(3-phényl-propyl)-nicotinamide;
6-[4-(4-méthyl-hexanoyl)-pipérazin-1-yl]-*N*-(3-phényl-propyl)-nicotinamide;
*N*-[2-(3-chlorophényl)-1-méthyléthyl]-6-[4-(2-éthylbutyryl)-pipérazin-1-yl]-nicotinamide ;
*N*-[2-(3-chlorophényl)-éthyl]-6-[4-(3-méthyl-pentanoyl)-pipérazin-1-yl]-nicotinamide;
*N*-[2-(3-chlorophényl)-éthyl]-6-[4-(2-éthylbutyryl)-pipérazin-1-yl]-nicotinamide;
6-(4-pentanoyl-pipérazin-1-yl)-*N*-(4-phényl-butyl)-nicotinamide;
*N*-[2-(3-chlorophényl)-éthyl]-6-(4-pentanoyl-pipérazin-1-yl)-nicotinamide;
6-[4-(2-éthylbutyryl)-pipérazin-1-yl]-*N*-phénéthyl-nicotinamide;
6-[4-(3-méthyl-pentanoyl)-pipérazin-1-yl]-*N*-phénéthyl-nicotinamide;
6-(4-pentanoyl-pipérazin-1-yl)-*N*-(3-phényl-propyl)-nicotinamide;
*N*-(1-méthyl-3-phényl-propyl)-6-(4-pentanoyl-pipérazin-1-yl)-nicotinamide;
6-[4-(3-méthyl-pentanoyl)-pipérazin-1-yl]-*N*-(tétrahydro-furan-2-ylméthyl)-nicotinamide;
6-[4-(2-éthylbutyryl)-pipérazin-1-yl]-*N*-(tétrahydro-furan-2-ylméthyl)-nicotinamide;
6-(4-pentanoyl-pipérazin-1-yl)-*N*-(tétrahydro-furan-2-ylméthyl)-nicotinamide;
6-[4-(2-éthylbutyryl)-pipérazin-1-yl]-*N*-(4-phényl-butyl)-nicotinamide;
*N*-(3-imidazol-1-yl-propyl)-6-(4-pentanoyl-pipérazin-1-yl)-nicotinamide;
6-[4-(2-éthylbutyryl)-pipérazin-1-yl]-*N*-(3-imidazol-1-yl-propyl)-nicotinamide;
*N*-[2-(3H-imidazol-4-yl)-éthyl]-6-(4-pentanoyl-pipérazin-1-yl)-nicotinamide;
*N*-[2-(3H-imidazol-4-yl)-éthyl]-6-[4-(3-méthyl-pentanoyl)-pipérazin-1-yl]-nicotinamide;
6-(4-pentanoyl-pipérazin-1-yl)-*N*-phénéthyl-nicotinamide;
6-[4-(2-éthylbutyryl)-pipérazin-1-yl]-*N*-[2-(3H-imidazol-4-yl)-éthyl]-nicotinamide;
*N*-(3-imidazol-1-yl-propyl)-6-[4-(3-méthyl-pentanoyl)-pipérazin-1-yl]-nicotinamide;
6-[4-(2-éthylbutyryl)-[1,4]diazépan-1-yl]-*N*-(3-phényl-propyl)-nicotinamide;
6-[4-(2-éthylbutyryl)-3-méthyl-pipérazin-1-yl]-*N*-(3-phényl-propyl)-nicotinamide;
6-[5-(2-éthylbutyryl)-2,5-diaza-bicyclo[2.2.1]hept-2-yl]-*N*-(3-phényl-propyl)-nicotinamide ;
6-[4-(butane-1-sulfonyl)-pipérazine-1-yl]-*N*-(3-phényl-propyl)-nicotinamide;
butylamide d'acide 4-[5-(3-phényl-propylcarbamoyl)-pyridin-2-yl]-pipérazine-1-carboxylique.

53. Composé destiné à être utilisé selon la revendication 45 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle éventuellement substitué, aralcényle éventuellement substitué, cycloalkyle éventuellement substitué, cycloalkylalkyle éventuellement substitué, cycloalkylalcényle, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est cycloalkyle éventuellement substitué, cycloalkylalkyle éventuellement substitué, ou cycloalkylalcényle;
chaque R⁴ est indépendamment l'hydrogène, alkyle, alcényle, halo, haloalkyle, aryle ou -R⁹-OR⁸;
chaque R⁵ et R⁶ est indépendamment l'hydrogène, oxo, alkyle, alcényle, halo, haloalkyle ou aryle;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
R⁹ est une liaison directe ou une chaîne alkylène linéaire ou ramifiée;
et
R¹⁰ est alkyle, aryle ou aralkyle.

54. Composé destiné à être utilisé selon la revendication 53 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, alcényle, cycloalkyle éventuellement substitué, cycloalkylalkyle éventuellement substitué ou cycloalkylalcényle;
R³ est cycloalkyle éventuellement substitué ou cycloalkylalkyle éventuellement substitué;
chaque R⁴ est indépendamment l'hydrogène, alkyle, halo, ou haloalkyle;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle; et
R¹⁰ est alkyle, aryle ou aralkyle.

55. Composé destiné à être utilisé selon la revendication 54 où le composé de formule (I) est un composé où:
m est 1;
n est 1;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0 à 2) ou alkyle;
R³ est cycloalkyle éventuellement substitué ou cycloalkylalkyle éventuellement substitué;
R⁴ est l'hydrogène;
R⁵ est l'hydrogène;
chaque R⁶ est l'hydrogène;
R⁷ est une chaîne alkylène linéaire ou ramifiée;
R⁸ est l'hydrogène ou alkyle; et
R¹⁰ est alkyle, aryle ou aralkyle.

56. Composé destiné à être utilisé selon la revendication 55 où le composé de formule (I) est choisi dans le groupe consistant en les suivants:
6-[4-(2-cyclohexyl-acétyl)-pipérazin-1-yl]-*N*-(3-éthoxy-propyl)-nicotinamide;
*N*-butyl-6-[4-(2-cyclohexyl-acétyl)-pipérazin-1-yl]-nicotinamide;
6-[4-(3-cyclohexyl-propionyl)-pipérazin-1-yl]-*N*-(3-méthylbutyl)-nicotinamide;
6-[4-(3-cyclohexyl-propionyl)-pipérazin-1-yl]-*N*-(3-méthoxy-propyl)-nicotinamide;
*N*-pentyl-6-[4-(3-trifluorométhyl-benzoyl)-pipérazin-1-yl]-nicotinamide;
6-[4-(2-cyclohexyl-acétyl)-pipérazin-1-yl]-*N*-(2-méthylbutyl)-nicotinamide;
6-[4-(3-cyclohexyl-propionyl)-pipérazin-1-yl]-*N*-(3-isopropoxy-propyl)-nicotinamide ;
6-[4-(2-cyclohexyl-acétyl)-pipérazin-1-yl]-*N*-(1,3-diméthylbutyl)-nicotinamide;
6-[4-(2-cyclohexyl-acétyl)-pipérazin-1-yl]-*N*-hexyl-nicotinamide;
6-[4-(2-cyclohexyl-acétyl)-pipérazin-1-yl]-*N*-(1-méthylbutyl)-nicotinamide ;
6-[4-(2-cyclohexyl-acétyl)-pipérazin-1-yl]-*N*-(3-méthylbutyl)-nicotinamide;
6-[4-(2-cyclohexyl-acétyl)-pipérazin-1-yl]-*N*-(2-éthylsulfanyl-éthyl)-nicotinamide ;
*N*-(3-butoxy-propyl)-6-[4-(2-cyclohexyl-acétyl)-pipérazin-1-yl]-nicotinamide ;
*N*-butyl-6-[4-(3-cyclohexyl-propionyl)-pipérazin-1-yl]-nicotinamide;
6-[4-(2-cyclohexyl-acétyl)-pipérazin-1-yl]-*N*-pentyl-nicotinamide;
6-[4-(3-cyclohexyl-propionyl)-pipérazin-1-yl]-*N*-(2-éthylsulfanyl-éthyl)-nicotinamide;
6-[4-(2-cyclohexyl-acétyl)-pipérazin-1-yl]-*N*-(3-méthoxy-propyl)-nicotinamide;
6-[4-(3-cyclohexyl-propionyl)-pipérazin-1-yl]-*N-*(1-méthylbutyl)-nicotinamide ;
6-[4-(2-cyclohexyl-acétyl)-pipérazin-1-yl]-*N*-(3-isopropoxy-propyl)-nicotinamide ;
6-[4-(3-cyclohexyl-propionyl)-pipérazin-1-yl]-*N*-(3-éthoxy-propyl)-nicotinamide;
6-[4-(3-cyclohexyl-propionyl)-pipérazin-1-yl]-*N*-(1,3-diméthylbutyl)-nicotinamide;
6-[4-(3-cyclohexyl-propionyl)-pipérazin-1-yl]-*N*-(2-méthylbutyl)-nicotinamide;
6-[4-(3-cyclohexyl-propionyl)-pipérazin-1-yl]-*N*-hexyl-nicotinamide;
6-[4-(3-cyclohexyl-propionyl)-pipérazin-1-yl]-*N*-pentyl-nicotinamide;
6-[4-(3-cyclohexyl-propionyl)-pipérazin-1-yl]-N-(3-diméthylamino-propyl)-nicotinamide;
N-(3-éthoxy-propyl)-6-[4-(2-phényl-cyclopropanecarbonyl)-pipérazin-1-yl]-nicotinamide;
*N*-(2-éthylsulfanyl-éthyl)-6-[4-(2-phényl-cyclopropanecarbonyl)-pipérazin-1-yl]-nicotinamide;
*N*-(1,3-diméthylbutyl)-6-[4-(2-phényl-cyclopropanecarbonyl)-pipérazin-1-yl]-nicotinamide;
*N*-(3-méthyl-butyl)-6-[4-(2-phényl-cyclopropanecarbonyl)-pipérazin-1-yl]-nicotinamide;
*N*-(3-méthoxy-propyl)-6-(4-(2-phényl-cyclopropanecarbonyl)-pipérazin-1-yl]-nicotinamide;
*N*-(3-butoxy-propyl)-6-[4-(2-phényl-cyclopropanecarbonyl)-pipérazin-1-yl]-nicotinamide ;
*N*-pentyl-6-[4-(2-phényl-cyclopropanecarbonyl)-pipérazin-1-yl]-nicotinamide ;
*N*-(3-diméthylamino-propyl)-6-[4-(2-phényl-cyclopropanecarbonyl)-pipérazin-1-yl]-nicotinamide ;
*N*-(3-isopropoxy-propyl)-6-[4-(2-phényl-cyclopropanecarbonyl)-pipérazin-1-yl]-nicotinamide ;
*N*-(1-méthyl-butyl)-6-(4-(2-phényl-cyclopropanecarbonyl)-pipérazin-1-yl]-nicotinamide;
*N*-butyl-6-[4-(2-phényl-cyclopropanecarbonyl)-pipérazin-1-yl]-nicotinamide;
*N*-hexyl-6-[4-(2-phényl-cyclopropanecarbonyl)-pipérazin-1-yl]-nicotinamide;
*N*-(2-méthyl-butyl)-6-[4-(2-phényl-cyclopropanecarbonyl)-pipérazin-1-yl]-nicotinamide; et
6-[4-(2-cyclohexyl-acétyl)-pipérazin-1-yl]-*N*-(3-diméthylamino-propyl)-nicotinamide.

57. Composé destiné à être utilisé selon la revendication 53 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est cycloalkyle éventuellement substitué ou cycloalkylalkyle éventuellement substitué;
chaque R⁴ est indépendamment l'hydrogène, alkyle, halo, ou haloalkyle; et
chaque R⁵ et R⁶ est indépendamment l'hydrogène, oxo, alkyle, halo ou haloalkyle.

58. Composé destiné à être utilisé selon la revendication 57 où le composé de formule (I) est un composé où:
m est 1;
n est 1;
p est 2;
V est -C(O)-;
R¹ est l'hydrogène ou alkyle;
R² est choisi dans le groupe consistant en aryle éventuellement substitué, aralkyle éventuellement substitué, hétérocyclyle éventuellement substitué, hétérocyclylalkyle éventuellement substitué, hétéroaryle éventuellement substitué et hétéroarylalkyle éventuellement substitué;
R³ est cycloalkyle éventuellement substitué ou cycloalkylalkyle éventuellement substitué;
R⁴ est l'hydrogène, alkyle, halo ou haloalkyle;
R⁵ est indépendamment l'hydrogène, oxo, alkyle, halo ou haloalkyle;
et
chaque R⁶ est indépendamment l'hydrogène, oxo, alkyle, halo ou haloalkyle.

59. Composé destiné à être utilisé selon la revendication 58 où le composé de formule (I) est choisi dans le groupe consistant en les suivants:
6-[4-(2-phényl-cyclopropanecarbonyl)-pipérazin-1-yl]-*N*-(3-phényl-propyl)-nicotinamide;
*N*-(4-phényl-butyl)-6-[4-(2-phényl-cyclopropanecarbonyl)-pipérazin-1-yl]-nicotinamide;
6-[4-(2-cyclohexyl-acétyl)-pipérazin-1-yl]-*N*-phénéthyl-nicotinamide;
*N*-[2-(3-chlorophényl)-éthyl]-6-[4-(2-phényl-cyclopropanecarbonyl)-pipérazin-1-yl]-nicotinamide;
*N*-(1-méthyl-3-phényl-propyl)-6-[4-(2-phényl-cyclopropanecarbonyl)-pipérazin-1-yl]-nicotinamide;
*N*-phénéthyl-6-[4-(2-phényl-cyclopropanecarbonyl)-pipérazin-1-yl]-nicotinamide;
6-[4-(2-phényl-cyclopropanecarbonyl)-pipérazin-1-yl]-*N*-(tétrahydro-furan-2-ylméthyl)-nicotinamide;
*N*-[2-(3H-imidazol-4-yl)-éthyl]-6-[4-(2-phényl-cyclopropanecarbonyl)-pipérazin-1-yl]-nicotinamide;
*N*-(3-imidazol-1-yl-propyl)-6-[4-(2-phényl-cyclopropanecarbonyl)-pipérazin-1-yl]-nicotinamide;
*N*-[2-(3-chlorophényl)-éthyl]-6-[4-(3-cyclohexyl-propionyl)-pipérazin-1-yl]-nicotinamide;
6-[4-(2-cyclohexyl-acétyl)-pipérazin-1-yl]-*N*-[2-(3H-imidazol-4-yl)-éthyl]-nicotinamide;
6-[4-(2-cyclohexyl-acétyl)-pipérazin-1-yl]-*N*-(3-phényl-propyl)-nicotinamide;
*N*-[2-(3-chlorophényl)-éthyl]-6-[4-(2-cyclohexyl-acétyl)-pipérazin-1-yl]-nicotinamide ;
6-[4-(3-cyclohexyl-propionyl)-pipérazin-1-yl]-*N*-phénéthyl-nicotinamide;
6-[4-(3-cyclohexyl-propionyl)-pipérazin-1-yl]-*N*-(3-phényl-propyl)-nicotinamide;
6-[4-(3-méthyl-pentanoyl)-pipérazin-1-yl]-*N*-(4-phényl-butyl)-nicotinamide;
6-[4-(2-cyclohexyl-acétyl)-pipérazin-1-yl]-*N*-(4-phényl-butyl)-nicotinamide;
6-[4-(2-cyclohexyl-acétyl)-pipérazin-1-yl]-*N*-(1-méthyl-3-phényl-propyl)-nicotinamide;
6-[4-(3-cyclohexyl-propionyl)-pipérazin-1-yl]-*N*-[2-(3H-imidazol-4-yl)-éthyl]-nicotinamide;
6-[4-(3-cyclohexyl-propionyl)-pipérazin-1-yl]-*N*-(tétrahydro-furan-2-ylméthyl)-nicotinamide;
6-[4-(2-cyclohexyl-acétyl)-pipérazin-1-yl]-*N*-(3-imidazol-1-yl-propyl)-nicotinamide;
6-[4-(3-cyclohexyl-propionyl)-pipérazin-1-yl]-*N*-(3-imidazol-1-yl-propyl)-nicotinamide; et
6-[4-(2-cyclohexyl-acétyl)-pipérazin-1-yl]-*N*-(tétrahydro-furan-2-ylméthyl)-nicotinamide.

60. Composé destiné à être utilisé selon la revendication 45 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle éventuellement substitué, aralcényle éventuellement substitué, cycloalkyle éventuellement substitué, cycloalkylalkyle éventuellement substitué, cycloalkylalcényle, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est aryle éventuellement substitué;
chaque R⁴ est indépendamment l'hydrogène, alkyle, alcényle, halo, haloalkyle, aryle ou -R⁹-OR⁸;
chaque R⁵ et R⁶ est indépendamment l'hydrogène, oxo, alkyle, alcényle, halo, haloalkyle ou aryle;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
R⁹ est une liaison directe ou une chaîne alkylène linéaire ou ramifiée;
et
Ruz est alkyle, aryle ou aralkyle.

61. Composé destiné à être utilisé selon la revendication 60, où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0 à 2), alkyle, alcényle, cycloalkyle éventuellement substitué, cycloalkylalkyle éventuellement substitué ou cycloalkylalcényle;.
R³ est aryle éventuellement substitué;
chaque R⁴ est indépendamment l'hydrogène, alkyle, halo, haloalkyle ou -R⁹-OR⁸;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle;
R⁹ est une liaison directe ou une chaîne alkylène linéaire ou ramifiée;
et
R¹⁰ est alkyle, aryle ou aralkyle.

62. Composé destiné à être utilisé selon la revendication 61 où le composé de formule (I) est un composé où:
m est 1 ou 2;
n est 1;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, cycloalkyle éventuellement substitué ou cycloalkylalkyle éventuellement substitué;
R³ est aryle éventuellement substitué;
chaque R⁴ est indépendamment l'hydrogène, halo, haloalkyle ou -R⁹-R⁸;
R⁵ est l'hydrogène;
chaque R⁶ est l'hydrogène;
R⁷ est une chaîne alkylène linéaire ou ramifiée;
R⁸ est l'hydrogène ou alkyle;
R⁹ est une liaison directe ou une chaîne alkylène linéaire ou ramifiée;
et
R¹⁰ est alkyle, aryle ou aralkyle.

63. Composé destiné à être utilisé selon la revendication 62, où le composé de formule (I) est choisi dans le groupe consistant en les suivants:
*N*-butyl-6-[4-(2-mercapto-benzoyl)-pipérazin-1-yl]-nicotinamide;
*N*-(3-méthyl-butyl)-6-[4-(2-o-tolyl-acétyl)-pipérazin-1-yl]-nicotinamide;
6-{4-[2-(2,4-diméthyl-phényl)-acétyl]-pipérazin-1-yl}-*N*-(3-méthylbutyl)-nicotinamide;
6-[4-(2-bromo-benzoyl)-pipérazin-1-yl]-*N*-(3-éthoxypropyl)-nicotinamide;
6-{4-[2-(2-chloro-6-fluoro-phényl)-acétyl]-pipérazin-1-yl}-*N*-(3-méthylbutyl)-nicotinamide;
6-[4-(3,5-dichloro-benzoyl)-pipérazin-1-yl]-*N*-(3-méthoxy-propyl)-nicotinamide;
6-[4-(2-bromo-5-méthoxy-benzoyl)-pipérazin-1-yl]-*N*-(3-diméthylamino-propyl)-nicotinamide;
6-[4-(2,5-dichloro-benzoyl)-pipérazin-1-yl]-*N*-(3-méthylbutyl)-nicotinamide;
6-[4-(2,5-dichloro-benzoyl)-pipérazin-1-yl]-*N*-(3-éthoxy-propyl)-nicotinamide;
*N*-butyl-6-[4-(3,5-dichloro-benzoyl)-pipérazin-1-yl]-nicotinamide;
6-[4-(2,4-dichloro-benzoyl)-pipérazin-1-yl]-*N*-(3-éthoxy-propyl)-nicotinamide;
6-[4-(2,4-dichloro-benzoyl)-pipérazin-1-yl]-*N*-(2-éthylsulfanyl-éthyl)-nicotinamide;
*N*-(3-éthoxy-propyl)-6-[4-(4-trifluorométhyl-benzoyl)-pipérazin-1-yl]-nicotinamide;
*N*-(3-diméthylamino-propyl)-6-[4-(2,3,4,5-tétrafluoro-benzoyl)-pipérazin-1-yl]-nicotinamide;
6-[4-(2-bromo-5-méthoxy-benzoyl)-pipérazin-1-yl]-*N*-(2-éthylsulfanyl-éthyl)-nicotinamide;
*N*-(2-éthylsulfanyl-éthyl)-6-[4-(3-trifluorométhyl-benzoyl)-pipérazin-1-yl]-nicotinamide;
6-[4-(2-bromo-benzoyl)-pipérazin-1-yl]-*N*-(3-méthylbutyl)-nicotinamide;
*N*-butyl-6-[4-(2,4-diméthyl-benzoyl)-pipérazin-1-yl]-nicotinamide;
6-[4-(3,5-dichloro-benzoyl)-pipérazin-1-yl]-*N*-(3-isopropoxy-propyl)-nicotinamide;
6-[4-(2-bromo-5-méthoxy-benzoyl)-pipérazin-1-yl]-*N*-(3-méthylbutyl)-nicotinamide;
*N*-(3-butoxy-propyl)-6-[4-(2,5-dichloro-benzoyl)-pipérazin-1-yl]-nicotinamide;
*N*-(3-méthyl-butyl)-6-[4-(3-trifluorométhyl-benzoyl)-pipérazin-1-yl]-nicotinamide;
*N*-(3-méthyl-butyl)-6-[4-(2-trifluorométhyl-benzoyl)-pipérazin-1-yl]-nicotinamide;
6-[4-(2-bromo-benzoyl)-pipérazin-1-yl]-*N*-(3-butoxy-propyl)-nicotinamide ;
6-[4-(2,5-dichloro-benzoyl)-pipérazin-1-yl]-*N*-pentyl-nicotinamide;
*N*-(3-butoxy-propyl)-6-[4-(2,4-diméthyl-benzoyl)-pipérazin-1-yl]-nicotinamide ;
6-[4-(2-bromo-5-méthoxy-benzoyl)-pipérazin-1-yl]-*N*-(3-éthoxy-propyl)-nicotinamide;
6-[4-(2,4-dichloro-benzoyl)-pipérazin-1-yl]-*N*-(3-isopropoxy-propyl)-nicotinamide;
*N*-(3-éthoxy-propyl)-6-(4-(2-trifluorométhyl-benzoyl)-pipérazin-1-yl]-nicotinamide ;
*N*-pentyl-6-[4-(3-trifluorométhyl-benzoyl)pipérazin-1-yl]-nicotinamide ;
*N*-butyl-6-[4-(2-trifluorométhyl-benzoyl)-pipérazin-1-yl]-nicotinamide;
*N*-butyl-6-(4-(2,5-dichloro-benzoyl)-pipérazin-1-yl]-nicotinamide;
6-[4-(2-bromo-benzoyl)-pipérazin-1-yl]-*N*-(3-isopropoxy-propyl)-nicotinamide;
6-(4-(2,5-dichloro-benzoyl)-pipérazin-1-yl]-*N*-(3-isopropoxy-propyl)-nicotinamide;
*N*-(3-butoxy-propyl)-6-[4-(4-trifluorométhyl-benzoyl)-piperazin-1-yl]-nicotinamide ;
6-[4-(2,5-dichloro-benzoyl)-pipérazin-1-yl]-*N*-(2-éthylsulfanyl-éthyl)-nicotinamide;
*N*-(3-butoxy-propyl)-6-[4-(2-trifluorométhyl-benzoyl)-pipérazin-1-yl]-nicotinamide;
6-[4-(2,5-dichloro-benzoyl)-pipérazin-1-yl]-*N*-(3-méthoxy-propyl)-nicotinamide;
6-[4-(2,4-diméthyl-benzoyl)-pipérazin-1-yl]-*N*-(1,3-diméthylbutyl)-nicotinamide ;
6-[4-(2,4-dichloro-benzoyl)-pipérazin-1-yl]-*N*-(2-méthylbutyl)-nicotinamide;
6-[4-(3,5-dichloro-benzoyl)-pipérazin-1-yl]-*N*-(2-méthylbutyl)-nicotinamide;
6-[4-(2,4-diméthyl-benzoyl)-pipérazin-1-yl]-*N*-(2-éthylsulfanyl-éthyl)-nicotinamide;
*N*-(1,3-diméthylbutyl)-6-[4-(3-trifluorométhyl-benzoyl)-pipérazin-1-yl]-nicotinamide;
6-[4-(2-bromo-5-méthoxy-benzoyl)-pipérazin-1-yl]-*N*-(2-méthylbutyl)-nicotinamide ;
*N*-(3-diméthylamino-propyl)-6-[4-(2-trifluorométhyl-benzoyl)-pipérazin-1-yl]-nicotinamide;
*N*-hexyl-6-[4-(3-trifluorométhyl-benzoyl)-pipérazin-1-yl]-nicotinamide;
6-[4-(2,4-dichloro-benzoyl)-pipérazin-1-yl]-*N*-(1,3-diméthylbutyl)-nicotinamide ;
*N*-(3-isopropoxy-propyl)-6-[4-(3-trifluorométhyl-benzoyl)-pipérazin-1-yl]-nicotinamide;
6-[4-(2-bromo-5-méthoxy-benzoyl)-pipérazin-1-yl]-*N*-hexyl-nicotinamide;
6-[4-(2-bromo-benzoyl)-pipérazin-1-yl]-*N*-hexyl-nicotinamide;
*N*-(3-isopropoxy-propyl)-6-[4-(2-trifluorométhyl-benzoyl)-pipérazin-1-yl]-nicotinamide ;
6-[4-(2-bromo-5-méthoxy-benzoyl)-pipérazin-1-yl]-*N*-(3-méthoxy-propyl)-nicotinamide ;
6-[4-(2-bromo-benzoyl)-pipérazin-1-yl]-*N*-(1-méthyl-butyl)-nicotinamide ;
6-[4-(3,5-dichloro-benzoyl)-pipérazin-1-yl]-*N*-hexyl-nicotinamide;
*N*-(3-méthoxy-propyl)-6-[4-(2-trifluorométhyl-benzoyl)-pipérazin-1-yl]-nicotinamide;
6-[4-(2,4-dichloro-benzoyl)-pipérazin-1-yl]-*N*-hexyl-nicotinamide;
*N*-(2-éthylsulfanyl-éthyl)-6-[4-(2-trifluorométhyl-benzoyl)-pipérazin-1-yl]-nicotinamide;
*N*-pentyl-6-[4-(2-trifluoro-méthyl-benzoyl)-pipérazin-1-yl]-nicotinamide;
*N*-(2-méthyl-butyl)-6-[4-(3-trifluorométhyl-benzoyl)-pipérazin-1-yl]-nicotinamide;
*N*-(2-éthylsulfanyl-éthyl)-6-[4-(4-trifluorométhyl-benzoyl)-pipérazin-1-yl]-nicotinamide;
6-[4-(2,4-dichloro-benzoyl)-pipérazin-1-yl]-*N*-pentyl-nicotinamide;
6-[4-(2-bromo-benzoyl)-pipérazin-1-yl]-*N*-(2-méthylbutyl)-nicotinamide;
6-[4-(2-bromo-5-méthoxy-benzoyl)-pipérazin-1-yl]-*N*-(3-butoxy-propyl)-nicotinamide;
*N*-(1,3-diméthylbutyl)-6-[4-(4-trifluorométhyl-benzoyl)-pipérazin-1-yl]-nicotinamide;
6-[4-(3,5-dichloro-benzoyl)-pipérazin-1-yl]-*N*-pentyl-nicotinamide;
*N*-(3-butoxy-propyl)-6-[4-(3,5-dichloro-benzoyl)-pipérazin-1-yl]-nicotinamide;
6-[4-(2-bromo-5-méthoxy-benzoyl)-pipérazin-1-yl]-*N*-butyl-nicotinamide;
6-[4-(2-bromo-benzoyl)-pipérazin-1-yl]-*N*-pentyl-nicotinamide;
*N*-(1,3-diméthylbutyl)-6-[4-(2-trifluorométhyl-benzoyl)-pipérazin-1-yl]-nicotinamide;
*N*-(3-méthoxy-propyl)-6-[4-(4-trifluorométhyl-benzoyl)-pipérazin-1-yl]-nicotinamide;
6-[4-(2,4-diméthyl-benzoyl)-pipérazin-1-yl]-*N*-(1-méthylbutyl)-nicotinamide;
6-[4-(2,4-dichloro-benzoyl)-pipérazin-1-yl]-*N*-(3-méthoxy-propyl)-nicotinamide;
*N*-(1-méthyl-butyl)-6-[4-(2-trifluorométhyl-benzoyl)-pipérazin-1-yl]-nicotinamide;
*N*-(3-diméthylamino-propyl)-6-[4-(3-trifluorométhyl-benzoyl)-pipérazin-1-yl]-nicotinamide;
6-[4-(2,4-dichloro-benzoyl)-pipérazin-1-yl]-*N*-(1-méthylbutyl)-nicotinamide;
6-[4-(2,5-dichloro-benzoyl)-pipérazin-1-yl]-*N*-(1-méthylbutyl)-nicotinamide;
*N*-(1-méthyl-butyl)-6-[4-(3-trifluorométhyl-benzoyl)-pipérazin-1-yl]-nicotinamide;
6-[4-(2,5-dichloro-benzoyl)-pipérazin-1-yl]-*N*-hexyl-nicotinamide;
6-[4-(2,4-diméthyl-benzoyl)-pipérazin-1 -yl]-*N*-(3-méthoxy-propyl)-nicotinamide;
6-[4-(3,5-dichloro-benzoyl)-pipérazin-1-yl]-*N*-(1-méthylbutyl)-nicotinamide;
6-[4-(2,4-dichloro-benzoyl)-pipérazin-1-yl]-*N*-(3-méthylbutyl)-nicotinamide;
*N*-(3-butoxy-propyl)-6-[4-(3-trifluorométhyl-benzoyl)-pipérazin-1-yl]-nicotinamide ;
6-[4-(2,4-diméthyl-benzoyl)-pipérazin-1-yl]-*N*-(3-isopropoxy-propyl)-nicotinamide ;
*N*-(1-méthyl-butyl)-6-[4-(4-trifluorométhyl-benzoyl)-pipérazin-1-yl]-nicotinamide;
*N*-butyl-6-(4-(2,4-dichloro-benzoyl)-pipérazin-1-yl]-nicotinamide;
6-[4-(3,5-dichloro-benzoyl)-pipérazin-1-yl]-*N*-(1,3-diméthylbutyl)-nicotinamide;
*N*-butyl-6-[4-(3-trifluorométhyl-benzoyl)-pipérazin-1-yl]-nicotinamide ;
6-[4-(2-bromo-5-méthoxy-benzoyl)-pipérazin-1-yl]-*N*-(1,3-diméthylbutyl)-nicotinamide;
6-[4-(2,4-diméthyl-benzoyl)-pipérazin-1-yl]-*N*-hexyl-nicotinamide;
6-[4-(2,5-dichlorobenzoyl)-pipérazin-1-yl]-*N*-(1,3-diméthylbutyl)-nicotinamide;
*N*-(3-butoxy-propyl)-6-[4-(2,4-dichlorobenzoyl)-pipérazin-1-yl]-nicotinamide;
*N*-(3-méthoxy-propyl)-6-[4-(3-trifluorométhyl-benzoyl)-pipérazin-1-yl]-nicotinamide;
6-[4-(2,4-diméthyl-benzoyl)-pipérazin-1-yl]-*N*-(3-éthoxy-propyl)-nicotinamide;
6-[4-(2,5-dichloro-benzoyl)-pipérazin-1-yl]-*N*-(2-méthylbutyl)-nicotinamide;
*N*-hexyl-6-[4-(4-trifluorométhyl-benzoyl)-pipérazin-1-yl]-nicotinamide ;
6-[4-(2,4-diméthyl-benzoyl)-pipérazin-1-yl]-*N*-pentyl-nicotinamide;
6-[4-(2-bromo-benzoyl-pipérazin-1-yl]-*N*-(1,3-diméthylbutyl)-nicotinamide;
*N*-hexyl-6-[4-(2-trifluorométhyl-benzoyl)-pipérazin-1-yl]-nicotinamide;
6-[4-(2-bromo-benzoyl)-pipérazin-1-yl]-*N*-(3-méthoxy-propyl)-nicotinamide ;
*N*-(2-méthyl-butyl)-6-[4-(4-trifluorométhyl-benzoyl)-pipérazin-1-yl]-nicotinamide ;
6-[4-(2-bromo-5-méthoxy-benzoyl)-pipérazin-1-yl]-*N*-(1-méthylbutyl)-nicotinamide;
6-[4-(2,4-diméthyl-benzoyl)-pipérazin-1-yl]-*N*-(2-méthylbutyl)-nicotinamide;
6-(4-(2-bromo-5-méthoxy-benzoyl)-pipérazin-1-yl]-*N*-pentyl-nicotinamide;
*N*-(2-méthyl-butyl)-6-[4-(2-trifluorométhyl-benzoyl)-pipérazin-1-yl]-nicotinamide ;
6-[4-(3,5-dichloro-benzoyl)-pipérazin-1-yl]-*N*-(3-méthylbutyl)-nicotinamide;
6-[4-(2-bromo-benzoyl)-pipérazin-1-yl]-*N*-(2-éthylsulfanyl-éthyl)-nicotinamide;
6-[4-(3,5-dichloro-benzoyl)-pipérazin-1-yl]-*N*-(3-méthoxy-propyl)-nicotinamide ;
6-[4-(2-bromo-5-méthoxy-benzoyl)-pipérazin-1-yl]-*N*-(3-isopropoxy-propyl)-nicotinamide ;
*N*-(3-butoxy-propyl)-6-[4-(2,3,4,5-tétrafluoro-benzoyl)-pipérazin-1-yl]-nicotinamide ;
*N*-(1,3-diméthylbutyl)-6-[4-(2,3,4,5-tétrafluoro-benzoyl)-pipérazin-1-yl]-nicotinamide;
*N*-butyl-6-[4-(2,3,4,5-tétrafluoro-benzoyl)-pipérazin-1-yl]-nicotinamide;
*N*-(1-méthyl-butyl)-6-[4-(2,3,4,5-tétrafluoro-benzoyl)-pipérazin-1-yl]-nicotinamide;
*N*-(3-méthoxy-propyl)-6-[4-(2,3,4,5-tétrafluoro-benzoyl)-pipérazin-1-yl]-nicotinamide;
*N*-(3-isopropoxy-propyl)-6-[4-(2,3,4,5-tétrafluoro-benzoyl)-pipérazin-1-yl]-nicotinamide;
*N*-pentyl-6-[4-(2,3,4,5-tétrafluoro-benzoyl)-pipérazin-1-yl]-nicotinamide;
*N*-(2-méthyl-butyl)-6-[4-(2,3,4,5-tétrafluoro-benzoyl)-pipérazin-1-yl]-nicotinamide;
*N*-hexyl-6-[4-(2,3,4,5-tétrafluoro-benzoyl)-pipérazin-1-yl]-nicotinamide;
*N*-(3-méthyl-butyl)-6-[4-(2,3,4,5-tétrafluoro-benzoyl)-pipérazin-1-yl]-nicotinamide;
*N*-(2-éthylsulfanyl-éthyl)-6-[4-(2,3,4,5-tétrafluoro-benzoyl)-pipérazin-1-yl]-nicotinamide;
*N*-(3-éthoxy-propyl)-6-[4-(2,3,4,5-tétrafluoro-benzoyl)-pipérazin-1-yl]-nicotinamide;
*N*-(3-diméthylamino-propyl)-6-[4-(4-trifluorométhyl-benzoyl)-pipérazin-1-yl]-nicotinamide;
*N*-(3-éthoxy-propyl)-6-[4-(3-trifluorométhyl-benzoyl)-pipérazin-1-yl]-nicotinamide;
6-[4-(3,5-dichloro-benzoyl)-pipérazin-1-yl]-*N*-(2-éthylsulfanyl-éthyl)-nicotinamide;
*N*-(3-méthyl-butyl)-6-[4-(4-trifluorométhyl-benzoyl)-pipérazin-1-yl]-nicotinamide;
*N*-butyl-6-[4-(4-trifluorométhyl-benzoyl)-pipérazin-1-yl]-nicotinamide;
*N*-(3-isopropoxy-propyl)-6-[4-(4-trifluorométhyl-benzoyl)-pipérazin-1-yl]-nicotinamide;
*N*-pentyl-6-[4-(4-trifluorométhyl-benzoyl)-pipérazin-1-yl]-nicotinamide;
6-[4-(3,5-dichloro-benzoyl)-pipérazin-1-yl]-*N*-(3-diméthylamino-propyl)-nicotinamide;
6-[4-(2-bromo-benzoyl)-pipérazin-1-yl]-*N*-(3-diméthylamino-propyl)-nicotinamide;
6-[4-(2,4-dichloro-benzoyl)-pipérazin-1-yl]-*N*-(3-diméthylamino-propyl)-nicotinamide;
6-[4-(2,5-dichloro-benzoyl)-pipérazin-1-yl]-*N*-(3-diméthylamino-propyl)-nicotinamide;
*N*-(3-diméthylamino-propyl)-6-[4-(2,4-diméthyl-benzoyl)-pipérazin-1-yl]-nicotinamide ;
*N*-(3-méthyl-butyl)-6-[4-(2-trifluorométhyl-benzoyl)-pipérazin-1-yl]-nicotinamide ;
*N*-(3-méthyl-butyl)-4-trifluorométhyl-6-[4-(2-trifluorométhyl-benzoyl)-pipérazin-1-yl]-nicotinamide ;
2-chloro-5-fluoro-*N*-(3-méthylbutyl)-6-[4-(2-trifluorométhyl-benzoyl)-pipérazin-1-yl]-nicotinamide ;
*N*-(3-éthoxy-propyl)-6-[4-(2-naphtalén-2-yl-acétyl)-pipérazin-1-yl]-nicotinamide ;
6-[4-(2-naphtalén-2-yl-acétyl)-pipérazin-1-yl]-*N*-pentyl-nicotinamide;
*N*-butyl-6-[4-(naphtalène-1-carbonyl)-pipérazin-1-yl]-nicotinamide ;
*N*-(3-butoxy-propyl)-6-[4-(2-naphtalén-2-yl-acétyl)-pipérazin-1-yl]-nicotinamide ;
*N*-butyl-6-[4-(2-naphtalén-2-ylacétyl)-pipérazin-1-yl]-nicotinamide;
*N*-(3-butoxy-propyl)-6-[4-(naphtalène-1-carbonyl)-pipérazin-1-yl]-nicotinamide;
*N*-(3-méthoxy-propyl)-6-[4-(naphtalène-1-carbonyl)-pipérazin-1-yl]-nicotinamide;
*N*-(1-méthyl-butyl)-6-[4-(naphtalène-1-carbonyl)-pipérazin-1-yl]-nicotinamide;
6-[4-(naphtalène-1-carbonyl)-pipérazin-1-yl]-*N*-pentyl-nicotinamide;
*N*-(3-isopropoxy-propyl)-6-[4-(2-naphtalén-2-yl-acétyl)-pipérazin-1-yl]-nicotinamide;
*N*-(1-méthyl-butyl)-6-[4-(2-naphtalén-2-yl-acétyl)-pipérazin-1-yl]-nicotinamide;
*N*-(3-méthyl-butyl)-6-[4-(2-naphtalén-2-yl-acétyl)-pipérazin-1-yl]-nicotinamide ;
*N*-(3-diméthylamino-propyl)-6-[4-(2-naphtalén-2-yl-acétyl)-pipérazin-1-yl]-nicotinamide ;
*N*-(3-éthoxy-propyl)-6-[4-(naphtalène-1-carbonyl)-pipérazin-1-yl]-nicotinamide;
*N*-hexyl-6-[4-(naphtalène-1-carbonyl)-pipérazin-1-yl]-nicotinamide;
*N*-(2-éthylsulfanyl-éthyl)-6-[4-(2-naphtalén-2-yl-acétyl)-pipérazin-1-yl]-nicotinamide;
*N*-hexyl-6-[4-(2-naphtalén-2-yl-acétyl)-pipérazin-1-yl)-nicotinamide;
*N*-(1,3-diméthylbutyl)-6-[4-(naphtalène-1-carbonyl)-pipérazin-1-yl]-nicotinamide;
*N*-(3-isopropoxy-propyl)-6-(4-(naphtalène-1-carbonyl)-pipérazin-1-yl]-nicotinamide;
*N*-(1,3-diméthylbutyl)-6-[4-(naphtalène-2-carbonyl)-pipérazin-1-yl]-nicotinamide;
*N*-(1,3-diméthylbutyl)-6-[4-(2-naphtalén-2-yl-acétyl)-pipérazin-1-yl]-nicotinamide;
*N*-(2-méthyl-butyl)-6-(4-(naphtalène-1-carbonyl)-pipérazin-1-yl]-nicotinamide;
*N*-(2-éthylsulfanyl-éthyl)-6-[4-(naphtalène-1-carbonyl)-pipérazin-l-yl]-nicotinamide ;
*N*-(2-méthyl-butyl)-6-[4-(2-naphtalén-2-yl-acétyl)-pipérazin-1-yl]-nicotinamide ;
*N*-(3-méthyl-butyl)-6-[4-(naphtalène-1-carbonyl)-pipérazin-1-yl]-nicotinamide;
*N*-(3-éthoxy-propyl)-6-[4-(naphtalène-2-carbonyl)-pipérazin-1-yl]-nicotinamide;
*N*-(2-méthyl-butyl)-6-[4-(naphtalène-2-carbonyl)-pipérazin-1-yl]-nicotinamide;
*N*-(3-méthyl-butyl)-6-[4-(naphtalène-2-carbonyl)-pipérazin-1-yl]-nicotinamide ;
*N*-(3-méthoxy-propyl)-6-[4-(naphtalène-2-carbonyl)-pipérazin-1-yl]-nicotinamide ;
*N*-butyl-6-[4-(naphtalène-2-carbonyl)-pipérazin-1-yl]-nicotinamide;
*N*-(3-butoxy-propyl)-6-[4-(naphtalène-2-carbonyl)-pipérazin-1-yl]-nicotinamide ;
*N*-(1-méthyl-butyl)-6-[4-(naphtalène-2-carbonyl)-pipérazin-1-yl]-nicotinamide ;
*N*-(3-isopropoxy-propyl)-6-[4-(naphtalène-2-carbonyl)-pipérazin-1-yl]-nicotinamide;
*N*-hexyl-6-[4-(naphtalène-2-carbonyl)-pipérazin-1-yl]-nicotinamide ;
*N*-(3-diméthylamino-propyl)-6-[4-(naphtalène-1-carbonyl)-pipérazin-1-yl]-nicotinamide;
*N*-(2-éthylsulfanyl-éthyl)-6-[4-(naphtalène-2-carbonyl)-pipérazin-1-yl]-nicotinamide;
*N*-(3-diméthylamino-propyl)-6-[4-(naphtalène-2-carbonyl)-pipérazin-1-yl]-nicotinamide;
*N*-(3-méthyl-butyl)-6-[4-(naphtalène-1-carbonyl)-pipérazin-1-yl]-nicotinamide;
*N*-(3-méthoxy-propyl)-6-[4-(2-naphtalén-2-yl-acétyl)-pipérazin-1-yl]-nicotinamide;
2-chloro-*N*-(3-méthylbutyl)-6-[4-(2-trifluorométhyl-benzoyl)-pipérazin-1-yl]-nicotinamide;
*N*-(2-cyclopropyl-éthyl)-6-[4-(3-trifluorométhyl-benzoyl)-pipérazin-1-yl]-nicotinamide;
*N*-(2-cyclopropyl-éthyl)-2-méthoxy-6-[4-(2-trifluorométhyl-benzoyl)-pipérazin-1-yl]-nicotinamide;
*N*-(2-cyclopropyl-éthyl)-6-[4-(5-fluoro-2-trifluorométhyl-benzoyl)-pipérazin-1-yl]-nicotinamide;
(2-cyclopropyléthyl)amide d'acide 2-oxo-6-[4-(2-tritluorométhyl-benzoyl)-pipérazin-1-yl]-1,2-dihydro-pyridine-3-carboxylique;
*N*-(2-cyclopropyl-éthyl)-2-hydroxy-6-[4-(2-trifluorométhyl-benzoyl)-pipérazin-1-yl]-nicotinamide;
*N*-(2-cyclobutyl-éthyl)-6-[4-(5-fluoro-2-trifluorométhyl-benzoyl)-pipérazin-1-yl]-nicotinamide;
*N*-(3-cyclopropyl-propyl)-6-[4-(5-fluoro-2-trifluorométhyl-benzoyl)-pipérazin-1-yl]-nicotinamide ;
6-[4-(5-fluoro-2-trifluorométhyl-benzoyl)-pipérazin-1-yl]-*N*-(4-méthyl-pentyl)-nicotinamide ; et
*N*-(3,3-diméthylbutyl)-6-[4-(5-fluoro-2-trifluorométhyl-benzoyl)-pipérazin-1-yl]-nicotinamide.

64. Composé destiné à être utilisé selon la revendication 60, où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est aryle éventuellement substitué;
chaque R⁴ est indépendamment l'hydrogène, alkyle, halo, ou haloalkyle; et
chaque R⁵ et R⁶ est indépendamment l'hydrogène, oxo, alkyle, halo ou haloalkyle.

65. Composé destiné à être utilisé selon la revendication 64 où le composé de formule (I) est un composé où:
m est 1 ou 2;
n est 1;
p est 2;
V est -C(O)-;
R¹ est l'hydrogène ou alkyle;
R² est choisi dans le groupe consistant en aryle éventuellement substitué, aralkyle éventuellement substitué, hétérocyclyle éventuellement substitué, hétérocyclylalkyle éventuellement substitué, hétéroaryle éventuellement substitué et hétéroarylalkyle éventuellement substitué;
R³ est aryle éventuellement substitué;
chaque R⁴ est indépendamment l'hydrogène, alkyle, halo ou haloalkyle;
R⁵ est l'hydrogène, oxo, alkyle, halo ou haloalkyle; et
chaque R⁶ est indépendamment l'hydrogène, alkyle, halo ou haloalkyle.

66. Composé destiné à être utilisé selon la revendication 65 où le composé de formule (I) est choisi dans le groupe consistant en les suivants :
6-[4-(2,5-dichloro-benzoyl)-pipérazin-1-yl]-*N*-(3-imidazol-1-yl-propyl)-nicotinamide;
6-[4-(2,4-dichloro-benzoyl)-pipérazin-1-yl]-*N*-(3-imidazol-1-yl-propyl)-nicotinamide;
6-[4-(2-bromo-5-méthoxy-benzoyl)-pipérazin-1-yl]-*N*-(3-phényl-propyl)-nicotinamide;
6-[4-(2-bromo-benzoyl)-pipérazin-1-yl]-*N*-[2-(3H-imidazol-4-yl)-éthyl]-nicotinamide;
*N*-(1-méthyl-2-phényl-éthyl)-6-[4-(2-trifluorométhyl-benzoyl)-pipérazin-1-yl]-nicotinamide;
*N*-(1-méthyl-2-phényl-éthyl)-6-[4-(4-trifluorométhyl-benzoyl)-pipérazin-1-yl]-nicotinamide;
6-[4-(naphtalène-1-carbonyl)-pipérazin-1-yl]-*N*-(4-phényl-butyl)-nicotinamide;
*N*-[2-(3-chlorophényl)-éthyl]-6-[4-(2-naphtalén-2-yl-acétyl)-pipérazin-1-yl]-nicotinamide;
6-[4-(naphtalène-2-carbonyl)-pipérazin-1-yl]-*N*-(3-phényl-propyl)-nicotinamide;
*N*-(3-imidazol-1-yl-propyl)-6-[4-(2-naphtalén-2-yl-acétyl)-pipérazin-1-yl]-nicotinamide;
6-[4-(naphtalène-1-carbonyl)-pipérazin-1-yl]-*N*-(3-phényl-propyl)-nicotinamide;
*N*-(1-méthyl-3-phényl-propyl)-6-[4-(2-naphtalén-2-yl-acétyl)-pipérazin-1-yl]-nicotinamide;
6-[4-(2-naphtalén-2-yl-acétyl)-pipérazin-1-yl]-*N*-phénéthyl-nicotinamide;
*N*-[2-(3-chlorophényl)-éthyl]-6-[4-(naphtalène-1-carbonyl)-pipérazin-1-yl]-nicotinamide;
*N*-(1-méthyl-3-phényl-propyl)-6-[4-(naphtalène-1-carbonyl)-pipérazin-1-yl]-nicotinamide;
6-[4-(naphtalène-1-carbonyl)-pipérazin-1-yl]-*N*-phénéthyl-nicotinamide;
*N*-[2-(3H-imidazol-4-yl)-éthyl]-6-[4-(2-naphtalén-2-yl-acétyl)-pipérazin-1-yl]-nicotinamide;
6-[4-(2-naphtalén-2-yl-acétyl)-pipérazin-1-yl]-*N*-(4-phényl-butyl)-nicotinamide;
6-[4-(naphtalène-2-carbonyl)-pipérazin-1-yl]-*N*-(3-phényl-propyl)-nicotinamide;
*N*-(1-méthyl-3-phényl-propyl)-6-[4-(naphtalène-2-carbonyl)-pipérazin-1-yl]-nicotinamide ;
*N*-[2-(3H-imidazol-4-yl)-éthyl]-6-[4-(naphtalène-1-carbonyl)-pipérazin-1-yl]-nicotinamide ;
6-[4-(naphtalène-2-carbonyl)-pipérazin-1-yl]-*N*-(tétrahydro-furan-2-ylméthyl)-nicotinamide ;
6-[4-(naphtalène-1-carbonyl)-pipérazin-1-yl]-*N*-(tétrahydro-furan-2-ylméthyl)-nicotinamide ;
*N*-(3-imidazol-1-yl-propyl)-6-[4-(naphtalène-2-carbonyl)-pipérazin-1-yl]-nicotinamide ;
*N*-[2-(3H-imidazol-4-yl)-éthyl]-6-[4-(naphtalène-2-carbonyl)-pipérazin-1-yl]-nicotinamide;
6-[4-(naphtalène-2-carbonyl)-pipérazin-1-yl]-*N*-phénéthyl-nicotinamide ;
6-[4-(naphtalène-2-carbonyl)-pipérazin-1-yl]-*N*-(4-phényl-butyl)-nicotinamide;
*N*-(2-(3-chlorophényl)-éthyl]-6-[4-(naphtalène-2-carbonyl)-pipérazin-1-yl]-nicotinamide;
*N*-(3-imidazol-1-yl-propyl)-6-[4-(naphtalène-1-carbonyl)-pipérazin-1-yl]-nicotinamide;
6-[4-(2-naphtalén-2-yl-acétyl)-pipérazin-1-yl]-*N*-(tétrahydro-furan-2-ylméthyl)-nicotinamide;
6-[4-(2-bromo-benzoyl)-pipérazin-1-yl]-*N*-(3-phényl-propyl)-nicotinamide;
*N*-[2-(3-chlorophényl)-éthyl]-6-[4-(2,4-dichloro-benzoyl)-pipérazin-1-yl]-nicotinamide;
6-[4-(2,4-diméthyl-benzoyl)-pipérazin-1-yl]-*N*-(4-phényl-butyl)-nicotinamide;
*N*-phénéthyl-6-[4-(2-p-tolyl-acétyl)-pipérazin-1-yl]-nicotinamide;
6-(4-[2-(2,5-dichloro-phényl)-acétyl]-pipérazin-1-yl)-*N*-(2-méthyl-3-phényl-propyl)-nicotinamide;
6-[4-(2,4-diméthyl-benzoyl)-pipérazin-1-yl]-*N*-(3-phényl-propyl)-nicotinamide;
*N*-(2-(3-chlorophényl)-éthyl]-6-(4-(2-fluoro-4-méthyl-benzoyl)-pipérazin-1-yl]-nicotinamide;
*N*-[2-(3-chlorophényl)-éthyl]-6-[4-(2,5-dichloro-benzoyl)-pipérazin-1-yl]-nicotinamide ;
*N*-(3-phényl-propyl)-6-[4-(4-trifluorométhyl-benzoyl)-pipérazin-1-yl]-nicotinamide;
6-[4-(2-bromo-benzoyl)-pipérazin-1-yl]-*N*-(3-imidazol-1-yl-propyl)-nicotinamide;
*N*-(4-phényl-butyl)-6-[4-(2-trifluorométhyl-benzoyl)-pipérazin-1-yl]-nicotinamide;
*N*-(3-phényl-propyl)-6-[4-(3-trifluorométhyl-benzoyl)-pipérazin-1-yl]-nicotinamide;
6-[4-(2-bromo-benzoyl)-pipérazin-1-yl]-*N*-[2-(3-chloro-phényl)-éthyl]-nicotinamide;
6-[4-(2-bromo-benzoyl)-pipérazin-1-yl]-*N*-(2-méthyl-3-phényl-propyl)-nicotinamide ;
6-[4-(2-bromo-5-méthoxy-benzoyl)-pipérazin-1-yl]-*N*-(5-phényl-pentyl)-nicotinamide;
6-[4-(2-bromo-5-méthoxy-benzoyl)-pipérazin-1-yl]-*N*-[2-(3-chloro-phényl)-éthyl]-nicotinamide;
*N*-[2-(3-chlorophényl)-éthyl]-6-[4-(2-trifluorométhyl-benzoyl)-pipérazin-1-yl]-nicotinamide;
*N*-(3-phényl-propyl)-6-[4-(2-trifluorométhyl-benzoyl)-pipérazin-1-yl]-nicotinamide;
*N*-(4-phényl-butyl)-6-[4-(3-trifluorométhyl-benzoyl)-pipérazin-1-yl]-nicotinamide;
6-[4-(2,4-dichloro-benzoyl)-pipérazin-1-yl]-*N*-phénéthyl-nicotinamide;
6-[4-(2-bromo-benzoyl)-pipérazin-1-yl]-*N*-(4-phényl-butyl)-nicotinamide;
6-[4-(2-bromo-benzoyl)-pipérazin-1-yl]-*N*-phénéthyl-nicotinamide;
6-[4-(2-bromo-5-méthoxy-benzoyl)-pipérazin-1-yl]-*N*-(1-méthyl-3-phényl-propyl)-nicotinamide;
6-[4-(3,5-dichloro-benzoyl)-pipérazin-1-yl]-*N*-(3-phényl-propyl)-nicotinamide;
6-[4-(3,5-dichloro-benzoyl)-pipérazin-1-yl]-*N*-(1-méthyl-3-phényl-propyl)-nicotinamide;
*N*-phénéthyl-6-[4-(4-trifluorométhyl-benzoyl)-pipérazin-1-yl]-nicotinamide;
6-[4-(2,4-diméthyl-benzoyl)-pipérazin-1-yl]-*N*-phénéthyl-nicotinamide;
*N*-phénéthyl-6-[4-(2-trifluorométhyl-benzoyl)-pipérazin-1-yl]-nicotinamide;
6-[4-(2,4-dichloro-benzoyl)-pipérazin-1-yl]-*N*-(1-méthyl-3-phényl-propyl)-nicotinamide;
6-[4-(2,5-dichloro-benzoyl)-pipérazin-1-yl]-*N*-(3-phényl-propyl)-nicotinamide;
6-[4-(2-bromo-5-méthoxy-benzoyl)-pipérazin-1-yl]-*N*-phénéthyl-nicotinamide;
6-[4-(2-bromo-5-méthoxy-benzoyl)-pipérazin-1-yl]-*N*-(3-imidazol-1-yl-propyl)-nicotinamide;
6-[4-(3,5-dichloro-benzoyl)-pipérazin-1-yl]-*N*-(3-imidazol-1-yl-propyl)-nicotinamide;
*N*-[2-(3H-imidazol-4-yl)-éthyl]-6-[4-(2,3,4,5-tétrafluoro-benzoyl)-pipérazin-1-yl]-nicotinamide;
*N*-phénéthyl-6-[4-(3-trifluorométhyl-benzoyl)-pipérazin-1-yl]-nicotinamide;
6-[4-(2,5-dichloro-benzoyl)-pipérazin-1-yl]-*N*-phénéthyl-nicotinamide;
6-[4-(3-méthyl-pentanoyl)-pipérazin-1-yl]-*N*-(1-méthyl-3-phényl-propyl)-nicotinamide ;
6-[4-(2,3,4,5-tétrafluoro-benzoyl)-pipérazin-1-yl]-*N*-(tétrahydro-furan-2-yl-méthyl)-nicotinamide ;
6-[4-(2-bromo-5-méthoxy-benzoyl)-pipérazin-1-yl]-*N*-(tétrahydro-furan-2-yl-méthyl)-nicotinamide;
*N*-(tétrahydro-furan-2-ylméthyl)-6-[4-(4-trifluorométhyl-benzoyl)-pipérazin-1-yl]-nicotinamide;
6-[4-(3,5-dichloro-benzoyl)-pipérazin-1-yl]-*N*-(4-phényl-butyl)-nicotinamide;
6-[4-(2,4-diméthyl-benzoyl)-pipérazin-1-yl]-*N*-(1-méthyl-3-phényl-propyl)-nicotinamide;
6-[4-(2,4-diméthyl-benzoyl)-pipérazin-1-yl]-*N*-(tétrahydro-furan-2-ylméthyl)-nicotinamide;
6-[4-(2-bromo-benzoyl)-pipérazin-1-yl]-*N*-(tétrahydro-furan-2-ylméthyl)-nicotinamide;
*N*-(1-méthyl-3-phényl-propyl)-6-[4-(3-trifluorométhyl-benzoyl)-pipérazin-1-yl]-nicotinamide;
*N*-phénéthyl-6-[4-(2,3,4,5-tétrafluoro-benzoyl)-pipérazin-1-yl]-nicotinamide;
*N*-(4-phényl-butyl)-6-[4-(2,3,4,5-tétrafluoro-benzoyl)-pipérazin-1-yl]-nicotinamide ;
*N*-(1-méthyl-3-phényl-propyl)-6-[4-(2,3,4,5-tétrafluoro-benzoyl)-pipérazin-1-yl]-nicotinamide;
*N*-[2-(3-chlorophényl)-éthyl]-6-[4-(2,3,4,5-tétrafluoro-benzoyl)-pipérazin-1-yl]-nicotinamide;
*N*-(3-phényl-propyl)-6-[4-(2,3,4,5-tétrafluoro-benzoyl)-pipérazin-1-yl]-nicotinamide;
6-[4-(2,5-dichloro-benzoyl)-pipérazin-1-yl]-*N*-(tétrahydro-furan-2-ylméthyl)-nicotinamide;
6-[4-(2,4-diméthyl-benzoyl)-pipérazin-1-yl]-*N*-[2-(3H-imidazol-4-yl)-éthyl]-nicotinamide;
*N*-[2-(3H-imidazol-4-yl)-éthyl]-6-[4-(3-trifluorométhyl-benzoyl)-pipérazin-1-yl]-nicotinamide;
*N*-(3-imidazol-1-yl-propyl)-6-[4-(3-trifluorométhyl-benzoyl)pipérazin-1-yl]-nicotinamide;
6-[4-(2,4-diméthyl-benzoyl)-pipérazin-1-yl]-*N*-(3-imidazol-1-yl-propyl)-nicotinamide;
6-[4-(2,5-dichloro-benzoyl)-pipérazin-1-yl]-*N*-[2-(3H-imidazol-4-yl)-éthyl]-nicotinamide ;
*N*-[2-(3H-imidazol-4-yl)-éthyl]-6-[4-(4-tri fluorométhyl-benzoyl)-pipérazin-1-yl]-nicotinamide ;
6-[4-(2,4-dichloro-benzoyl)-pipérazin-1-yl]-*N*-[2-(3H-imidazol-4-yl)-éthyl]-nicotinamide;
6-[4-(3,5-dichloro-benzoyl)-pipérazin-1-yl]-*N*-(tétrahydro-furan-2-ylméthyl)-nicotinamide;
*N*-(3-imidazol-1-yl-propyl)-6-[4-(2-tritluorométhyl-benzoyl)-pipérazin-1-yl]-nicotinamide;
*N*-(tétrahydro-furan-2-ylméthyl)-6-[4-(3-trifluorométhyl-benzoyl)-pipérazin-1-yl]-nicotinamide;
*N*-(tétrahydro-furan-2-ylméthyl)-6-[4-(2-trifluorométhyl-benzoyl)-pipérazin-1-yl]-nicotinamide;
6-[4-(2,4-dichloro-benzoyl)-pipérazin-1-yl]-*N*-(tétrahydro-furan-2-ylméthyl)-nicotinamide;
*N*-[2-(3H-imidazol-4-yl)-éthyl]-6-(4-(2-trifluorométhyl-benzoyl)-pipérazin-1-yl]-nicotinamide;
6-[4-(2-bromo-5-méthoxy-benzoyl)-pipérazin-1-yl]-*N*-[2-(3H-imidazol-4-yl)-éthyl]-nicotinamide;
*N*-[2-(3-chlorophényl)-éthyl]-6-[4-(3-trifluorométhyl-benzoyl)-pipérazin-1-yl]-nicotinamide;
*N*-(2-(3-chlorophényl)-éthyl]-6-[4-(4-trifluorométhyl-benzoyl)-pipérazin-1-yl]-nicotinamide;
*N*-[2-(3-chlorophényl)-éthyl]-6-[4-(3,5-dichloro-benzoyl)-pipérazin-1-yl]-nicotinamide;
*N*-(4-phényl-butyl)-6-[4-(4-trifluorométhyl-benzoyl)-pipérazin-1-yl]-nicotinamide;
6-[4-(2,4-dichloro-benzoyl)-pipérazin-1-yl]-*N*-(4-phényl-butyl)-nicotinamide;
6-(4-(2,4-dichloro-benzoyl)-pipérazin-1-yl]-*N*-(3-phényl-propyl)-nicotinamide;
6-[4-(2,5-dichloro-benzoyl)-pipérazin-1-yl]-*N*-(4-phényl-butyl)-nicotinamide;
6-[4-(3,5-dichloro-benzoyl)-pipérazin-1-yl]-*N*-phénéthyl-nicotinamide;
*N*-(3-imidazol-1-yl-propyl)-6-[4-(4-trifluorométhyl-benzoyl)-pipérazin-1-yl]-nicotinamide;
6-[4-(3,5-dichloro-benzoyl)-pipérazin-1-yl]-*N*-[2-(3H-imidazol-4-yl)-éthyl)-nicotinamide;
*N*-(3-imidazol-1-yl-propyl)-6-[4-(2,3,4,5-tétrafluoro-benzoyl)-pipérazin-1-yl]-nicotinamide ;
6-[2-oxo-4-(2-trifluorométhyl-benzoyl)-pipérazin-1-yl]-*N*-(3-phényl-propyl)-nicotinamide ;
*N*-(3-méthyl-butyl)-6-[2-oxo-4-(2-trifluorométhyl-benzoyl)-pipérazin-1-yl]-nicotinamide.

67. Composé destiné à être utilisé selon la revendication 45 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle éventuellement substitué, aralcényle éventuellement substitué, cycloalkyle éventuellement substitué, cycloalkylalkyle éventuellement substitué, cycloalkylalcényle, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est hétéroaryle éventuellement substitué, hétéroarylalkyle éventuellement substitué ou hétéroarylalcényle éventuellement substitué;
chaque R⁴ est indépendamment l'hydrogène, alkyle, alcényle, halo, haloalkyle ou aryle;
chaque R⁵ et R⁶ est indépendamment l'hydrogène, oxo, alkyle, alcényle, halo, haloalkyle ou aryle;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle; et
R¹⁰ est alkyle, aryle ou aralkyle.

68. Composé destiné à être utilisé selon la revendication 67 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, alcényle, cycloalkyle éventuellement substitué, cycloalkylalkyle éventuellement substitué ou cycloalkylalcényle;
R³ est hétéroaryle éventuellement substitué, hétéroarylalkyle éventuellement substitué ou hétéroarylalcényle éventuellement substitué;
chaque R⁴ est indépendamment l'hydrogène, alkyle, halo, ou haloalkyle;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle; et
R¹⁰ est alkyle, aryle ou aralkyle.

69. Composé destiné à être utilisé selon la revendication 68 où le composé de formule (I) est un composé où:
m est 1 ou 2;
n est 1;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2) ou alkyle;
R³ est hétéroaryle éventuellement substitué, hétéroarylalkyle éventuellement substitué ou hétéroarylalcényle éventuellement substitué;
chaque R⁴ est indépendamment l'hydrogène, halo ou haloalkyle;
R⁵ est l'hydrogène;
chaque R⁶ est l'hydrogène;
R⁷ est une chaîne alkylène linéaire ou ramifiée;
R⁸ est l'hydrogène ou alkyle; et
R¹⁰ est alkyle, aryle ou aralkyle.

70. Composé destiné à être utilisé selon la revendication 69 où le composé de formule (I) est choisi dans le groupe consistant en les suivants:
6-[4-(3-méthyl-3H-114-thiophène-2-carbonyl)-pipérazin-1-yl]-*N*-pentyl-nicotinamide;
*N*-(3-diméthylamino-propyl)-6-[4-(3-méthyl-thiophène-2-carbonyl)-pipérazin-1-yl]-nicotinamide ;
*N*-(3-diméthylamino-propyl)-6-[4-(thiophène-2-carbonyl)-pipérazin-1-yl]-nicotinamide ;
6-[4-(2-chloro-pyridine-3-carbonyl)-pipérazin-1-yl]-*N*-(3-éthoxy-propyl)-nicotinamide;
6-[4-(2-chloro-pyridine-3-carbonyl)-pipérazin-1-yl)-*N*-(2-éthylsulfanyl-éthyl)-nicotinamide;
6-[4-(2-chloro-pyridine-3-carbonyl)-pipérazin-1-yl]-*N*-(3-méthylbutyl)-nicotinamide;
6-[4-(2-chloro-pyridine-3-carbonyl)-pipérazin-1-yl]-*N*-(3-isopropoxy-propyl)-nicotinamide;
*N*-(2-éthylsulfanyl-éthyl)-6-[4-(thiophène-2-carbonyl)-pipérazin-1-yl]-nicotinamide ;
*N*-(3-éthoxy-propyl)-6-[4-(thiophène-2-carbonyl)-pipérazin-1-yl]-nicotinamide;
*N*-(3-méthyl-butyl)-6-[4-(thiophène-2-carbonyl)-pipérazin-1-yl]-nicotinamide;
6-[4-(2-chloro-pyridine-3-carbonyl)-pipérazin-1-yl]-*N*-(3-méthoxy-propyl)-nicotinamide;
*N*-pentyl-6-[4-(thiophène-2-carbonyl)-pipérazin-1-yl]-nicotinamide;
6-[4-(2-chloro-pyridine-3-carbonyl)-pipérazin-1-yl]-*N*-hexyl-nicotinamide;
*N*-(3-isopropoxy-propyl)-6-[4-(thiophène-2-carbonyl)-pipérazin-1-yl]-nicotinamide;
*N*-(1-méthyl-butyl)-6-(4-(thiophène-2-carbonyl)-pipérazin-1-yl]-nicotinamide;
*N*-hexyl-6-[4-(thiophène-2-carbonyl)-pipérazin-1-yl]-nicotinamide;
*N*-(1,3-diméthylbutyl)-6-[4-(thiophène-2-carbonyl)-pipérazin-1-yl]-nicotinamide;
*N*-butyl-6-[4-(thiophène-2-carbonyl)-pipérazin-1-yl]-nicotinamide;
*N*-(3-méthoxy-propyl)-6-(4-(thiophène-2-carbonyl)-pipérazin-1-yl]-nicotinamide;
6-[4-(2-chloro-pyridine-3-carbonyl)-pipérazin-1-yl]-*N*-(1,3-diméthylbutyl)-nicotinamide;
*N*-butyl-6-[4-(2-chloro-pyridine-3-carbonyl)-pipérazin-1-yl]-nicotinamide;
6-[4-(2-chloro-pyridine-3-carbonyl)-pipérazin-1-yl]-*N*-(2-méthylbutyl)-nicotinamide;
*N*-(2-méthyl-butyl)-6-[4-(thiophène-2-carbonyl)-pipérazin-1-yl]-nicotinamide;
6-[4-(2-chloro-pyridine-3-carbonyl)-pipérazin-1-yl]-*N*-(1-méthylbutyl)-nicotinamide;
*N*-(1,3-diméthylbutyl)-6-[4-(3-méthyl-thiophène-2-carbonyl)-pipérazin-1-yl]-nicotinamide ;
*N*-butyl-6-[4-(3-méthyl-thiophène-2-carbonyl)-pipérazin-1-yl]-nicotinamide;
*N*-(3-butoxy-propyl)-6-[4-(3-méthyl-thiophène-2-carbonyl)-pipérazin-1-yl]-nicotinamide ;
*N*-(3-isopropoxy-propyl)-6-[4-(3-méthyl-thiophène-2-carbonyl)-pipérazin-1-yl]-nicotinamide;
*N*-(3-éthoxy-propyl)-6-[4-(3-méthyl-thiophène-2-carbonyl)-pipérazin-1-yl]-nicotinamide;
*N*-(2-méthyl-butyl)-6-[4-(3-méthyl-thiophène-2-carbonyl)-pipérazin-1-yl]-nicotinamide;
*N*-(2-éthylsulfanyl-éthyl)-6-[4-(3-méthyl-thiophène-2-carbonyl)-pipérazin-1-yl]-nicotinamide;
*N*-(3-méthoxy-propyl)-6-[4-(3-méthyl-thiophène-2-carbonyl)-pipérazin-1-yl]-nicotinamide;
*N*-(1-méthyl-butyl)-6-[4-(3-méthyl-thiophène-2-carbonyl)-pipérazin-1-yl]-nicotinamide;
*N*-(3-méthyl-butyl)-6-[4-(3-méthyl-thiophène-2-carbonyl)-pipérazin-1-yl]-nicotinamide;
*N*-(3-butoxy-propyl)-6-[4-(2-chloro-pyridine-3-carbonyl)-pipérazin-1-yl]-nicotinamide;
*N*-(3-butoxy-propyl)-6-[4-(thiophène-2-carbonyl)-pipérazin-1-yl]-nicotinamide;
6-[4-(2-chloro-pyridine-3-carbonyl)-pipérazin-1-yl]-*N*-pentyl-nicotinamide;
*N*-hexyl-6-[4-(3-méthyl-thiophène-2-carbonyl)-pipérazin-1-yl]-nicotinamide; et
6-[4-(2-chloro-pyridine-3-carbonyl)-pipérazin-1-yl]-*N*-(3-diméthylamino-propyl)-nicotinamide.

71. Composé destiné à être utilisé selon la revendication 67 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est hétéroaryle éventuellement substitué, hétéroarylalkyle éventuellement substitué ou hétéroarylalcényle éventuellement substitué;
chaque R⁴ est indépendamment l'hydrogène, alkyle, halo, ou haloalkyle; et
chaque R⁵ et R⁶ est indépendamment l'hydrogène, oxo, alkyle, halo ou haloalkyle.

72. Composé destiné à être utilisé selon la revendication 71 où le composé de formule (I) est un composé où:
m est 1 ou 2;
n est 1;
p est 2;
V est -C(O)-;
R¹ est l'hydrogène ou alkyle;
R² est choisi dans le groupe consistant en aryle éventuellement substitué, aralkyle éventuellement substitué, hétérocyclyle éventuellement substitué, hétérocyclylalkyle éventuellement substitué, hétéroaryle éventuellement substitué et hétéroarylalkyle éventuellement substitué;
R³ est hétéroaryle éventuellement substitué, hétéroarylalkyle éventuellement substitué ou hétéroarylalcényle éventuellement substitué;
chaque R⁴ est indépendamment l'hydrogène, alkyle, halo, ou haloalkyle;
R⁵ est l'hydrogène, oxo, alkyle, halo ou haloalkyle; et
chaque R⁶ est indépendamment l'hydrogène, oxo, alkyle, halo ou haloalkyle.

73. Composé destiné à être utilisé selon la revendication 65 où le composé de formule (I) est choisi dans le groupe consistant en les suivants:
*N*-[2-(3-chlorophényl)-éthyl]-6-[4-(3-méthyl-thiophène-2-carbonyl)-pipérazin-1-yl]-nicotinamide;
*N*-[2-(3-chlorophényl)-éthyl]-6-{4-[2-(2-chloro-pyridin-3-yl)-acétyl]-pipérazin-1-yl}-nicotinamide;
6-[4-(2-chloro-pyridine-3-carbonyl)-pipérazin-1-yl]-*N*-(4-phényl-butyl)-nicotinamide;
*N*-[2-(3-chlorophényl)-éthyl]-6-[4-(thiophène-2-carbonyl)-pipérazin-1-yl]-nicotinamide;
6-[4-(2-chloro-pyridine-3-carbonyl)-pipérazin-1-yl]-*N*-(3-phényl-propyl)-nicotinamide;
*N*-(3-phényl-propyl)-6-[4-(thiophène-2-carbonyl)-pipérazin-1-yl]-nicotinamide;
*N*-phénéthyl-6-[4-(thiophène-2-carbonyl)-pipérazin-1-yl]-nicotinamide;
*N*-(1-méthyl-3-phényl-propyl)-6-[4-(thiophène-2-carbonyl)-pipérazin-1-yl]-nicotinamide ;
*N*-(4-phényl-butyl)-6-[4-(thiophène-2-carbonyl)-pipérazin-1-yl]-nicotinamide;
6-[4-(2-chloro-pyridine-3-carbonyl)-pipérazin-1-yl]-*N*-(1-méthyl-3-phényl-propyl)-nicotinamide;
6-[4-(2-chloro-pyridine-3-carbonyl)-pipérazin-1-yl]-*N*-phénéthyl-nicotinamide;
6-[4-(2-chloro-pyridine-3-carbonyl)-pipérazin-1-yl]-*N*-(tétrahydro-furan-2-yl-méthyl)-nicotinamide;
6-[4-(3-méthyl-thiophène-2-carbonyl)-pipérazin-1-yl]-*N*-(3-phényl-propyl)-nicotinamide;
*N*-(1-méthyl-3-phényl-propyl)-6-[4-(3-méthyl-thiophène-2-carbonyl)-pipérazin-1-yl]-nicotinamide;
6-[4-(3-méthyl-thiophène-2-carbonyl)-pipérazin-1-yl]-*N*-(4-phényl-butyl)-nicotinamide;
6-[4-(3-méthyl-thiophène-2-carbonyl)-pipérazin-1-yl]-*N*-phénéthyl-nicotinamide;
6-[4-(3-méthyl-thiophène-2-carbonyl)-pipérazin-1-yl]-*N*-(tétrahydrofuran-2-yl-méthyl)-nicotinamide;
*N*-(3-imidazol-1-yl-propyl)-6-[4-(thiophène-2-carbonyl)-pipérazin-1-yl]-nicotinamide;
*N*-(tétrahydro-furan-2-ylméthyl)-6-[4-(thiophène-2-carbonyl)-pipérazin-1-yl]-nicotinamide;
*N*-[2-(3H-imidazol-4-yl)-éthyl]-6-[4-(thiophène-2-carbonyl)-pipérazin-1-yl]-nicotinamide;
*N*-(3-imidazol-1-yl-propyl)-6-[4-(3-méthyl-thiophène-2-carbonyl)-pipérazin-1-yl]-nicotinamide;
*N*-[2-(3H-imidazol-4-yl)-éthyl]-6-[4-(3-méthyl-thiophène-2-carbonyl)-pipérazin-yl]-nicotinamide;
6-[4-(2-chloro-pyridine-3-carbonyl)-pipérazin-1-yl]-*N*-(3-imidazol-1-yl-propyl)-nicotinamide; et
6-[4-(2-chloro-pyridine-3-carbonyl)-pipérazin-1-yl]-*N*-[2-(3H-imidazol-4-yl)-éthyl]-nicotinamide.

74. Composé destiné à être utilisé selon la revendication 45 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle éventuellement substitué, aralcényle éventuellement substitué, cycloalkyle éventuellement substitué, cycloalkylalkyle éventuellement substitué, cycloalkylalcényle, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est aralkyle éventuellement substitué ou aralcényle éventuellement substitué;
chaque R⁴ est indépendamment l'hydrogène, alkyle, alcényle, halo, haloalkyle, aryle ou -R⁹-OR⁸;
chaque R⁵ et R⁶ est indépendamment l'hydrogène, oxo, alkyle, alcényle, halo, haloalkyle ou aryle;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
R⁹ est une liaison directe ou une chaîne alkylène linéaire ou ramifiée;
et
R¹⁰ est alkyle, aryle ou aralkyle.

75. Composé destiné à être utilisé selon la revendication 74 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, alcényle, cycloalkyle éventuellement substitué, cycloalkylalkyle éventuellement substitué ou cycloalkylalcényle;
R³ est aralkyle éventuellement substitué ou aralcényle éventuellement substitué;
chaque R⁴ est indépendamment l'hydrogène, alkyle, halo, ou haloalkyle;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle; et
R¹⁰ est alkyle, aryle ou aralkyle.

76. Composé destiné à être utilisé selon la revendication 75 où le composé de formule (I) est un composé où:
m est 1 ou 2;
n est 1;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰(où t est 0, 1 ou 2) ou alkyle;
R³ est aralkyle éventuellement substitué ou aralcényle éventuellement substitué;
chaque R⁴ est indépendamment l'hydrogène, halo ou haloalkyle;
R⁵ est l'hydrogène;
chaque R⁶ est l'hydrogène;
R⁷ est une chaîne alkylène linéaire ou ramifiée;
R⁸ est l'hydrogène ou alkyle; et
R¹⁰ est alkyle, aryle ou aralkyle.

77. Composé destiné à être utilisé selon la revendication 76 où le composé de formule (I) est choisi dans le groupe consistant en les suivants:
6-{4-[2-(4-chlorophényl)-propionyl]-pipérazin-1-yl}-*N*-(3-méthylbutyl)-nicotinamide;
6-{4-[2-(2-chloro-6-fluoro-phényl)-acétyl]-pipérazin-1-yl}-*N*-(3-éthoxy-propyl)-nicotinamide;
6-{4-[3-(3,4-difluoro-phényl)-propionyl]-pipérazin-1-yl}-*N*-(3-diméthylamino-propyl)-nicotinamide;
6-{4-[3-(3,4-difluoro-phényl)-propionyl]-pipérazin-1-yl}-*N*-(3-éthoxy-propyl)-nicotinamide;
6-{4-[2-(4-chlorophényl)-propionyl]-pipérazin-1-yl}-*N*-(3-éthoxy-propyl)-nicotinamide;
*N*-(3-éthoxy-propyl)-6-[4-(2-phényl-butyryl)-pipérazin-1-yl]-nicotinamide;
*N*-(2-éthylsulfanyl-éthyl)-6-[4-(2-o-tolyl-acétyl)-pipérazin-1-yl]-nicotinamide;
6-[4-(3-méthyl-pentanoyl)-pipérazin-1-yl]-*N*-(4-phényl-butyl)-nicotinamide;
*N*-butyl-6-{4-[3-(3,4-difluoro-phényl)-propionyl]-pipérazin-1-yl}-nicotinamide;
6-{4-[2-(2-chloro-6-fluoro-phényl)-acétyl]-pipérazin-1-yl}-*N*-(3-diméthylamino-propyl)-nicotinamide;
6-{4-[3-(3,4-difluoro-phényl)-propionyl]-pipérazin-1-yl}-*N*-(2-éthylsulfanyl-éthyl)-nicotinamide;
*N*-(3-méthoxy-propyl)-6-[4-(2-phényl-butyryl)-pipérazin-1-yl]-nicotinamide;
*N*-butyl-6-[4-(2-phényl-butyryl)-pipérazin-1-yl]-nicotinamide;
*N*-(3-butoxy-propyl)-6-{4-[2-(4-chloro-phényl)-propionyl]-pipérazin-1-yl}-nicotinamide;
*N*-(3-méthoxy-propyl)-6-[4-(2-o-tolyl-acétyl)-pipérazin-1-yl]-nicotinamide;
*N*-(3-méthyl-butyl)-6-[4-(2-phényl-butyryl)-pipérazin-1-yl]-nicotinamide;
*N*-(1-méthyl-butyl)-6-[4-(2-p-tolyl-acétyl)-pipérazin-1-yl]-nicotinamide;
*N*-butyl-6-{4-[2-(2-chloro-6-fluoro-phényl)-acétyl]-pipérazin-1-yl}-nicotinamide;
*N*-(2-méthyl-butyl)-6-(4-(2-o-tolyl-acétyl)-pipérazin-1-yl]-nicotinamide;
6-{4-[3-(3,4-difluoro-phényl)-propionyl]-pipérazin-1-yl}-*N*-(2-méthylbutyl)-nicotinamide;
*N*-(3-isopropoxy-propyl)-6-[4-(2-o-tolyl-acétyl)-pipérazin-1-yl]-nicotinamide;
6-{4-[3-(3,4-difluoro-phényl)-propionyl]-pipérazin-1-yl}-*N*-(3-méthylbutyl)-nicotinamide;
6-{4-[3-(3,4-difluoro-phényl)-propionyl]-pipérazin-1-yl}-*N*-(3-méthoxy-propyl)-nicotinamide;
*N*-(1-méthyl-butyl)-6-(4-(2-o-totyl-acétyl)-pipérazin-1-yl]-nicotinamide;
6-{4-[2-(2-chloro-6-fluoro-phényl)-acétyl]-pipérazin-1-yl}-*N*-(2-méthylbutyl)-nicotinamide;
6-{4-[2-(2-chloro-6-fluoro-phényl)-acétyl]-pipérazin-1-yl}-*N*-(1-méthylbutyl)-nicotinamide;
*N*-(2-méthyl-butyl)-6-[4-(2-phényl-butyryl)-pipérazin-1-yl]-nicotinamide;
*N*-(1,3-diméthylbutyl)-6-[4-(2-phényl-butyryl)-pipérazin-1-yl]-nicotinamide;
6-{4-[2-(4-chlorophényl)-propionyl]-pipérazin-1-yl}-*N*-(2-méthylbutyl)-nicotinamide;
6-{4-[2-(2-chloro-6-fluoro-phényl)-acétyl]-pipérazin-1-yl]-*N*-(3-isopropoxy-propyl)-nicotinamide;
*N*-(1,3-diméthylbutyl)-6-[4-(2-p-tolyl-acétyl)-pipérazin-1-yl]-nicotinamide;
6-{4-[3-(3,4-difluoro-phényl)-propionyl]-pipérazin-1-yl}-*N*-hexyl-nicotinamide;
*N*-(1-méthyl-butyl)-6-[4-(2-phényl-butyryl)-pipérazin-1-yl]-nicotinamide;
6-{4-[2-(4-chlorophényl)-propionyl]-pipérazin-1-yl}-*N*-(1-méthylbutyl)-nicotinamide ;
6-{4-[2-(4-chlorophényl)-propionyl]-pipérazin-1-yl}-*N*-(1,3-diméthylbutyl)-nicotinamide ;
*N*-butyl-6-[4-(2-p-tolyl-acétyl)-pipérazin-1-yl]-nicotinamide;
*N*-(1,3-diméthylbutyl)-6-[4-(2-o-tolyl-acétyl)-pipérazin-1-yl]-nicotinamide;
6-{4-[2-(2-chloro-6-fluoro-phényl)-acétyl]-pipérazin-1-yl}-*N*-(1,3-diméthyl-butyl)-nicotinamide;
*N*-(3-méthyl-butyl)-6-[4-(2-p-tolyl-acétyl)-pipérazin-1-yl]-nicotinamide;
*N*-butyl-6-[4-(2-o-tolyl-acétyl)-pipérazin-1-yl]-nicotinamide;
*N*-(3-butoxy-propyl)-6-[4-(2-p-tolyl-acétyl)-pipérazin-1-yl]-nicotinamide;
*N*-pentyl-6-[4-(2-p-tolyl-acétyl)-pipérazin-1-yl]-nicotinamide;
*N*-(3-butoxy-propyl)-6-[4-(2-phényl-butyryl)-pipérazin-1-yl]-nicotinamide;
*N*-pentyl-6-[4-(2-phényl-butyryl)-pipérazin-1-yl]-nicotinamide;
*N*-(2-éthylsulfanyl-éthyl)-6-[4-(2-p-tolyl-acétyl)-pipérazin-1-yl]-nicotinamide;
6-{4-[2-(4-chlorophényl)-propionyl]-pipérazin-1-yl}-*N*-(2-éthylsulfanyl-éthyl)-nicotinamide;
*N*-(3-éthoxy-propyl)-6-[4-(2-o-tolyl-acétyl)-pipérazin-1-yl]-nicotinamide;
6-{4-[2-(2-chloro-6-fluoro-phényl)-acétyl]-pipérazin-1-yl}-*N*-(3-méthoxy-propyl)-nicotinamide;
6-{4-[3-(3,4-difluoro-phényl)-propionyl]-pipérazin-1-yl}-*N*-(3-isopropoxy-propyl)-nicotinamide;
6-{4-[2-(4-chlorophényl)-propionyl]-pipérazin-1-yl}-*N*-pentyl-nicotinamide;
*N*-(3-butoxy-propyl)-6-{4-[2-(2-chloro-6-fluoro-phényl)-acétyl]-pipérazin-1-yl}-nicotinamide;
6-{4-[2-(2-chloro-6-fluoro-phényl)-acétyl]-pipérazin-1-yl}-*N*-hexyl-nicotinamide;
6-{4-[2-(4-chlorophényl)-propionyl]-pipérazin-1-yl}-*N*-(3-méthoxy-propyl)-nicotinamide;
*N*-hexyl-6-[4-(2-o-tolyl-acétyl)-pipérazin-1-yl]-nicotinamide;
6-{4-[3-(3,4-difluoro-phényl)-propionyl]-pipérazin-1-yl}-*N*-(4-phényl-butyl)-nicotinamide;
*N*-(3-isopropoxy-propyl)-6-[4-(2-p-tolyl-acétyl)-pipérazin-1-yl]-nicotinamide;
6-{4-[3-(3,4-difluoro-phényl)-propionyl]-pipérazin-1-yl}-*N*-(1-méthylbutyl)-nicotinamide ;
6-{4-[3-(3,4-difluoro-phényl)-propionyl]-pipérazin-1-yl}-*N*-pentyl-nicotinamide;
6-{4-[2-(4-chlorophényl)-propionyl]-pipérazin-1-yl}-*N*-hexyl-nicotinamide;
*N*-butyl-6-{4-[2-(4-chlorophényl)-propionyl]-pipérazin-1-yl}-nicotinamide;
6-{4-[2-(2-chloro-6-fluoro-phényl)-acétyl]-pipérazin-1-yl}-*N*-(2-éthylsulfanyl-éthyl)-nicotinamide ;
6-{4-[2-(2-chloro-6-fluoro-phényl)-acétyl]-pipérazin-1-yl}-*N*-pentyl-nicotinamide;
*N*-hexyl-6-[4-(2-phényl-butyryl)-pipérazin-1-yl]-nicotinamide;
*N*-(3-isopropoxy-propyl)-6-[4-(2-phényl-butyryl)-pipérazin-1-yl)-nicotinamide;
*N*-pentyl-6-[4-(2-o-tolyl-acétyl)-pipérazin-1-yl]-nicotinamide;
*N*-hexyl-6-[4-(2-p-tolyl-acétyl)-pipérazin-1-yl]-nicotinamide;
6-{4-[3-(3,4-difluoro-phényl)-propionyl]-pipérazin-1-yl}-*N*-(1,3-diméthylbutyl)-nicotinamide;
6-[4-(naphtalène-2-carbonyl)-pipérazin-1-yl]-*N*-pentyl-nicotinamide;
6-{4-[2-(4-chlorophényl)-propionyl]-pipérazin-1-yl}-N-(3-diméthylamino-propyl)-nicotinamide;
*N*-(3-diméthylamino)-propyl)-6-[4-(2-phényl-butyryl)-pipérazin-1-yl]-nicotinamide;
*N*-(3-diméthylamino-propyl)-6-[4-(2-p-tolyl-acétyl)-pipérazin-1-yl]-nicotinamide;
*N*-(3-diméthylamino-propyl)-6-[4-(2-o-tolyl-acétyl)-pipérazin-1-yl]-nicotinamide;
*N*-(3-butoxy-propyl)-6-{4-[3-(3,4-difluoro-phényl)-propionyl]-pipérazin-1-yl}-nicotinamide;
*N*-(3-méthoxy-propyl)-6-[4-(2-p-tolyl-acétyl)-pipérazin-1-yl]-nicotinamide;
*N*-(2-méthyl-butyl)-6-[4-(2-p-tolyl-acétyl)-pipérazin-1-yl]-nicotinamide;
*N*-(3-butoxy-propyl)-6-[4-(2-o-tolyl-acétyl)-pipérazin-1-yl]-nicotinamide; et
6-{4-[2-(4-chlorophényl)-propionyl]-pipérazin-1-yl}-*N*-(3-isopropoxy-propyl)-nicotinamide.

78. Composé destiné à être utilisé selon la revendication 74 où le composé de formule (I) est un composé où:
m est 1 ou 2;
n est 1 ou 2;
p est 2;
V est -C(O)-;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
R³ est aralkyle éventuellement substitué ou aralcényle éventuellement substitué;
chaque R⁴ est indépendamment l'hydrogène, alkyle, halo, ou haloalkyle; et
chaque R⁵ et R⁶ est indépendamment l'hydrogène, oxo, alkyle, halo ou haloalkyle.

79. Composé destiné à être utilisé selon la revendication 78 où le composé de formule (I) est un composé où:
m est 1 ou 2;
n est 1;
p est 2;
V est -C(O)-;
R¹ est l'hydrogène ou alkyle;
R² est choisi dans le groupe consistant en aryle éventuellement substitué, aralkyle éventuellement substitué, hétérocyclyle éventuellement substitué, hétérocyclylalkyle éventuellement substitué, hétéroaryle éventuellement substitué et hétéroarylalkyle éventuellement substitué;
R³ est aralkyle éventuellement substitué ou aralcényle éventuellement substitué;
chaque R⁴ est indépendamment l'hydrogène, alkyle, halo ou haloalkyle; et
R⁵ est l'hydrogène, oxo, alkyle, halo ou haloalkyle; et
chaque R⁶ est indépendamment l'hydrogène, oxo, alkyle, halo ou haloalkyle.

80. Composé destiné à être utilisé selon la revendication 79 où le composé de formule (I) est choisi dans le groupe consistant en les suivants:
6-[4-(2-phényl-butyryl)-pipérazin-1-yl]-*N*-(3-phényl-propyl)-nicotinamide;
6-(4-[3-(3,4-difluoro-phényl)-propionyl]-pipérazin-1-yl)-*N*-phénéthyl-nicotinamide;
*N*-[2-(3-chlorophényl)-éthyl]-6-[4-(2-o-tolyl-acétyl)-pipérazin-1-yl]-nicotinamide;
6-{4-[2-(2-chloro-6-fluoro-phényl)-acétyl]-pipérazin-1-yl}-*N*-(3-phényl-propyl)-nicotinamide;
*N*-(1-méthyl-3-phényl-propyl)-6-[4-(2-p-tolyl-acétyl)-pipérazin-1-yl]-nicotinamide;
*N*-[2-(3-chlorophényl)-éthyl]-6-(4-(2-phényl-butyryl)-pipérazin-1-yl]-nicotinamide;
6-{4-[2-(2-chloro-6-fluoro-phényl)-acétyl]-pipérazin-1-yl}-*N*-phénéthyl-nicotinamide;
*N*-(3-phényl-propyl)-6-[4-(2-o-tolyl-acétyl)-pipérazin-1-yl]-nicotinamide;
6-{4-[2-(2-chloro-6-fluoro-phényl)-acétyl]-pipérazin-1-yl}-*N*-[2-(3-chloro-phényl)-éthyl]-nicotinamide;
6-{4-[2-(4-chlorophényl)-propionyl]-pipérazin-l-yl}-*N*-(3-phényl-propyl)-nicotinamide ;
6-{4-[2-(4-chlorophényl)-propionyl]-pipérazin-1-yl}-*N*-(1-méthyl-3-phényl-propyl)-nicotinamide ;
*N*-[2-(3-chlorophényl)-éthyl]-6-{4-[2-(4-chloro-phényl)-propionyl]-pipérazin-1-yl}-nicotinamide;
*N-*[2-(3-chlorophényl)-éthyl]-6-[4-(2-p-tolyl-acétyl)-pipérazin-1-yl]-nicotinamide ;
*N*-phénéthyl-6-[4-(2-p-tolyl-acétyl)-pipérazin-1-yl]-nicotinamide;
*N*-(3-phényl-propyl)-6-[4-(2-p-tolyl-acétyl)-pipérazin-1-yl]-nicotinamide;
*N*-(1-méthyl-3-phényl-propyl)-6-[4-(2-phényl-butyryl)-pipérazin-1-yl]-nicotinamide;
6-{4-[2-(4-chlorophényl)-propionyl]-pipérazin-1-yl}-*N*-(4-phényl-butyl)-nicotinamide;
*N*-(4-phényl-butyl)-6-[4-(2-phényl-butyryl)-pipérazin-1-yl]-nicotinamide;
6-{4-[2-(2-chloro-6-fluoro-phényl)-acétyl]-pipérazin-1-yl}-*N*-(1-méthyl-3-phényl-propyl)-nicotinamide;
6-{4-[3-(3,4-difluoro-phényl)-propionyl]-pipérazin-1-yl}-*N*-(3-phényl-propyl)-nicotinamide;
*N*-phénéthyl-6-[4-(2-o-tolyl-acétyl)-pipérazin-1-yl]-nicotinamide;
6-{4-[3-(3,4-difluoro-phényl)-propionyl]-pipérazin-1-yl}-*N*-(1-méthyl-3-phényl-propyl)-nicotinamide;
*N*-(4-phényl-butyl)-6-[4-(2-o-tolyl-acétyl)-pipérazin-1-yl]-nicotinainide;
*N*-(3-imidazol-1-yl-propyl)-6-[4-(2-phényl-butyryl)-pipérazin-1-yl]-nicotinamide;
6-{4-[2-(2-chloro-6-fluoro-phényl)-acétyl]-pipérazin-l-yl}-*N*-(4-phényl-butyl)-nicotinamide;
6-{4-[2-(4-chlorophényl)-propionyl]-pipérazin-1-yl}-*N*-[2-(3H-imidazol-4-yl)-éthyl]-nicotinamide;
6-{4-[2-(2-chloro-6-fluoro-phényl)-acétyl]-pipérazin-l-yl}-*N*-(3-imidazol-1-yl-propyl)-nicotinamide;
*N*-[2-(3H-imidazol-4-yl)-éthyl]-6-[4-(2-p-tolyl-acétyl)-pipérazin-1-yl]-nicotinamide;
6-{4-[2-(4-chlorophényl)-propionyl]-pipérazin-1-yl}-*N*-(3-imidazol-1-yl-propyl)-nicotinamide;
*N*-(2-éthylsulfanyl-éthyl)-6-[4-(2-phényl-butyryl)-pipérazin-1-yl]-nicotinamide;
*N*-(1-méthyl-3-phényl-propyl)-6-[4-(2-o-tolyl-acétyl)-pipérazin-1-yl]-nicotinamide;
6-{4-[3-(3,4-difluoro-phényl)-propionyl]-pipérazin-1-yl}-*N*-phénéthyl-nicotinamide;
*N*-phénéthyl-6-[4-(2-phényl-butyryl)-pipérazin-1-yl]-nicotinamide;
*N*-(tétrahydro-furan-2-ylméthyl)-6-[4-(2-p-tolyl-acétyl)-pipérazin-1-yl]-nicotinamide ;
6-{4-[2-(4-chlorophényl)-propionyl]-pipérazin-1-yl}-*N*-(tétrahydrofuran-2-ylméthyl)-nicotinamide;
*N*-(4-phényl-butyl)-6-[4-(2-p-tolyl-acétyl)-pipérazin-1-yl]-nicoiinamide ;
6-{4-[3-(3,4-difluoro-phényl)-propionyl]-pipérazin-1-yl}-*N*-(tétrahydrofuran-2-ylméthyl)-nicotinamide;
*N*-(tétrahydro-furan-2-ylméthyl)-6-[4-(2-o-tolyl-acétyl)-pipérazin-1-yl]-nicotinamide ;
6-[4-(2-phényl-butyryl)-pipérazin-1-yl]-*N*-(tétrahydro-furan-2-ylméthyl)-nicotinamide;
6-{4-[3-(3,4-difluoro-phényl)-propionyl]-pipérazin-1-yl}-*N*-[2-(3H-imidazol-4-yl)-éthyl]-nicotinamide;
*N*-[2-(3H-imidazol-4-yl)-éthyl]-6-[4-(2-o-tolyl-acétyl)-pipérazin-1-yl]-nicotinamide;
*N*-(3-imidazol-1-yl-propyl)-6-[4-(2-o-tolyl-acétyl)-pipérazin-1-yl]-nicotinamide;
6-{4-[3-(3,4-difluoro-phényl)-propionyl]-pipérazin-1-yl}-*N*-(3-imidazol-1-yl-propyl)-nicotinamide;
*N*-[2-(3H-imidazol-4-yl)-éthyl]-6-[4-(2-phényl-butyryl)-pipérazin-1-yl]-nicotinamide;
*N*-(3-imidazol-1-yl-propyl)-6-[4-(2-p-tolyl-acétyl)-pipérazin-1-yl]-nicotinamide;
6-(4-[2-(2-chloro-6-fluoro-phényl)-acétyl]-pipérazin-1-yl)-*N*-(tétrahydrofuran-2-ylméthyl)-nicotinamide; et
6-{4-[2-(2-chloro-6-fluoro-phényl)-acétyl]-pipérazin-1-yl}-*N*-[2-(3H-imidazol-4-yl)-éthyl]-nicotinamide.

81. Composé destiné à être utilisé selon la revendication 45 où le mammifère est un humain.

82. Composé destiné à être utilisé selon la revendication 81 où la maladie ou affection est une maladie ou affection liée aux niveaux sériques de triglycéride, VLDL, HDL, LDL, cholestérol total ou au processus de transport inverse du cholestérol.

83. Composé destiné à être utilisé selon la revendication 81 où la maladie ou affection est une maladie ou affection liée aux niveaux sériques de triglycérides.

84. Composé destiné à être utilisé selon la revendication 81 où la maladie ou affection est une maladie ou affection liée aux niveaux sériques de cholestérol.

85. Composé destiné à être utilisé selon la revendication 81 où la maladie ou affection est le diabète de type II.

86. Composé destiné à être utilisé selon la revendication 81 où la maladie ou affection est l'obésité.

87. Composé destiné à être utilisé selon la revendication 81 où la maladie ou affection est la dyslipidémie.

88. Composé destiné à être utilisé selon la revendication 81 où la maladie ou affection est le syndrome métabolique.

89. Composition pharmaceutique comprenant un excipient ou vecteur pharmaceutiquement acceptable et une quantité thérapeutiquement efficace d'un composé de formule (I): 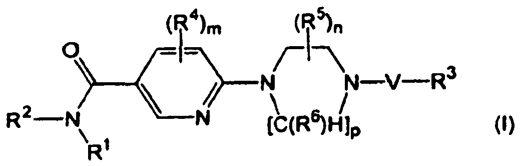 où:
m est 1 à 3;
n est 1, 2, 3 ou 4;
p est 2 ou 3;
V est -C(O)- ou -S(O)₂-;
R¹ est l'hydrogène, alkyle, alcényle, aryle, aralkyle, ou aralcényle;
R² est choisi dans le groupe consistant en l'hydrogène, -R⁷-OR⁸, -R⁷-N(R⁸)₂, -R⁷-S(O)ₜR¹⁰(où t est 0, 1 ou 2), alkyle, alcényle, aryle éventuellement substitué, aralkyle, où le radical aryle peut être éventuellement substitué, aralcényle, où le radical aryle peut être éventuellement substitué, cycloalkylalkyle éventuellement substitué, cycloalkylalcényle, hétérocyclyle éventuellement substitué, hétérocyclylalkyle, où le radical hétérocyclyle peut être éventuellement substitué, hétérocyclylalcényle, hétéroaryle éventuellement substitué, hétéroarylalkyle, où le radical hétéroaryle peut être éventuellement substitué et hétéroarylalcényle, où le radical hétéroaryle peut être éventuellement substitué;
R³ est choisi dans le groupe consistant en -R⁹-OR⁸, -R⁹-N(R⁸)₂, alcényle, aralkyle éventuellement substitué, aralcényle éventuellement substitué, cycloalkyle éventuellement substitué, cycloalkylalkyle, où le radical cycloalkyle peut être éventuellement substitué, cycloalkylalcényle, hétérocyclyle éventuellement substitué, hétérocyclylalkyle, où le radical hétérocyclyle peut être éventuellement substitué, hétérocyclylalcényle, hétéroarylalkyle, où le radical hétéroaryle peut être éventuellement substitué et hétéroarylalcényle, où le radical hétéroaryle peut être éventuellement substitué;
chaque R⁴ est indépendamment l'hydrogène, alkyle, alcényle, halo, haloalkyle, aryle ou -R⁹-OR⁸;
chaque R⁵ et R⁶ est indépendamment l'hydrogène, oxo, alkyle, alcényle, halo, haloalkyle ou aryle;
ou un R⁵ et un R⁶ peuvent former ensemble un pont alkylène linéaire ou ramifié;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
chaque R⁹ est indépendamment une chaîne alkylène ou alcénylène linéaire ou ramifiée; et
R¹⁰ est alkyle, alcényle, haloalkyle, cycloalkyle, cycloalkylalkyle, aryle, aralkyle, hétérocyclyle, hétérocyclylalkyle, hétéroaryle ou hétéroarylalkyle;
sous forme d'un seul stéréoisomère, d'un mélange de stéréoisomères, ou d'un mélange racémique de stéréoisomères;
ou sous forme d'un sel pharmaceutiquement acceptable, solvate, polymorphe ou promédicament, choisi parmi les dérivés esters et amides de groupes fonctionnels hydroxyle, carboxy, mercapto ou amino dans les composés de formule (I) ; et
où le substituant éventuel est un ou plusieurs substituants choisis indépendamment dans le groupe consistant en alkyle, alcényle, halo, haloalkyle, haloalcényle, cyano, nitro, aryle, aralkyle, cycloalkyle, cycloalkylalkyle, hétérocyclyle, hétérocyclylalkyle, hétéroaryle, hétéroarylalkyle, -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (où t est 1 ou 2), -R⁹-S(O)ₜOR¹⁰ (où t est 1 ou 2), -R⁹-S(O)ₜR¹⁰ (où t est 0, 1 ou 2), et -R⁹-S(O)ₜN(R⁸)₂ (où t est 1 ou 2).

90. Composition pharmaceutique selon la revendication 89 où la quantité thérapeutiquement efficace du composé de formule (I) est une quantité efficace pour moduler un niveau de lipide chez un mammifère quand elle est administrée au mammifère.

91. Composition pharmaceutique selon la revendication 90 où le lipide est un triglycéride.

92. Composition pharmaceutique selon la revendication 90 où le lipide est le cholestérol.

93. Composition pharmaceutique selon la revendication 89 où la quantité thérapeutiquement efficace du composé de formule (I) est une quantité efficace pour moduler les niveaux de HDL-cholestérol quand elle est administrée à un mammifère.

94. Composé de formule (I): 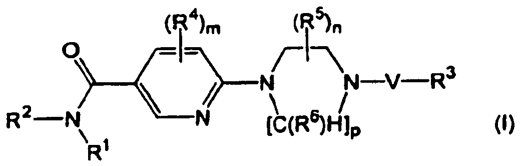 où:
m est 1, 2 ou 3;
n est 1, 2, 3 ou 4;
p est 2, 3 ou 4;
V est -C(O)-, -S(O)- ou -S(O)₂-;
R¹ est l'hydrogène, alkyle, alcényle, aryle, aralkyle ou aralcényle;
R² est choisi dans le groupe consistant en l'hydrogène, -R⁷-OR⁸, -R⁷-N(R⁸)₂, -R⁷-S(O)ₜR¹⁰ (où t est 0, 1 ou 2), alkyle, alcényle, aryle éventuellement substitué, aralkyle, où le radical aryle peut être éventuellement substitué, aralcényle, où le radical aryle peut être éventuellement substitué, cycloalkylalkyle éventuellement substitué, cycloalkylalcényle, hétérocyclyle éventuellement substitué, hétérocyclylalkyle, où le radical hétérocyclyle peut être éventuellement substitué, hétérocyclylalcényle, hétéroaryle éventuellement substitué, hétéroarylalkyle, où le radical hétéroaryle peut être éventuellement substitué et hétéroarylalcényle, où le radical hétéroaryle peut être éventuellement substitué;
R³ est cyclopropyle substitué par aryle éventuellement substitué ou hétéroaryle éventuellement substitué ;
chaque R⁴ est indépendamment l'hydrogène, alkyle, alcényle, halo, haloalkyle, aryle, cyano, nitro, -R⁹-OR⁸, -R⁹-N(R⁸)₂ ou -S(O)ₜR¹⁰ (où t est 0, 1 ou 2);
chaque R⁵ et R⁶ est indépendamment l'hydrogène, oxo, alkyle, alcényle, halo, haloalkyle ou aryle;
ou un R⁵ et un R⁶ peuvent former ensemble un pont alkylène linéaire ou ramifié;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
chaque R⁹ est indépendamment une liaison directe ou une chaîne alkylène ou alcénylène linéaire ou ramifiée ; et
R¹⁰ est alkyle, alcényle, haloalkyle, cycloalkyle, cycloalkylalkyle, aryle, aralkyle, hétérocyclyle, hétérocyclylalkyle, hétéroaryle ou hétéroarylalkyle;
sous forme d'un seul stéréoisomère, d'un mélange de stéréoisomères, d'un mélange racémique de stéréoisomères, ou sous forme d'un tautomère ;
ou sous forme d'un sel pharmaceutiquement acceptable, solvate, polymorphe ou promédicament, choisi parmi les dérivés esters et amides de groupes fonctionnels hydroxyle, carboxy, mercapto ou amino dans les composés de formule (I) ; et
où le substituent éventuel est un ou plusieurs substituants choisis indépendamment dans le groupe consistant en alkyle, alcényle, halo, haloalkyle, haloalcényle, cyano, nitro, aryle, aralkyle, cycloalkyle, cycloalkylalkyle, hétérocyclyle, hétérocyclylalkyle, hétéroaryle, hétéroarylalkyle -R⁹-OR⁸, -R⁹-N(R⁸)₂, -R⁹-C(O)R⁸, -R⁹-C(O)OR⁸, -R⁹-C(O)N(R⁸)₂, -R⁹-N(R⁸)C(O)OR¹⁰, -R⁹-N(R⁸)C(O)R¹⁰, -R⁹-N(R⁸)(S(O)ₜR¹⁰) (où t est 1 ou 2), -R⁹-S(O)ₜOR¹⁰ (où t est 1 ou 2), -R⁹-S(O)ₜR¹⁰ (où t est 0, 1 ou 2) et -R⁹-S(O)ₜN(R⁸)₂ (où test 1 ou 2).

95. Composé selon la revendication 94 où:
m est 1 ou 2;
n est 1 ou 2;
p est 2;
V est -C(O)-;
R¹ est l'hydrogène ou alkyle;
R² est choisi dans le groupe consistant en l'hydrogène, -R⁷-OR⁸, -R⁷-N(R⁸)₂, -R⁷-S(O)ₜR¹⁰(où t est 0, 1 ou 2), alkyle, alcényle, aryle éventuellement substitué, aralkyle éventuellement substitué, aralcényle éventuellement substitué, cycloalkylalkyle éventuellement substitué, cycloalkylalcényle, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
chaque R⁴ est indépendamment l'hydrogène, alkyle, alcényle, halo, haloalkyle, aryle ou -R⁹-OR⁸;
chaque R⁵ et R^{b} est indépendamment l'hydrogène, oxo, alkyle, alcényle, halo, haloalkyle ou aryle;
chaque R⁷ est indépendamment une chaîne alkylène ou alcénylène linéaire ou ramifiée;
chaque R⁸ est indépendamment l'hydrogène, alkyle, alcényle, haloalkyle, cycloalkyle, cycloalkylalkyle, aryle, aralkyle, hétérocyclyle, hétérocyclylalkyle, hétéroaryle ou hétéroarylalkyle;
R⁹ est une liaison directe ou une chaîne alkylène linéaire ou ramifiée;
et
R¹⁰ est alkyle, aryle ou aralkyle.

96. Composé selon la revendication 95 où:
m est 1 ou 2;
n est 1 ou 2;
p est 2;
V est -C(O)-;
R¹ est l'hydrogène ou alkyle;
R² est choisi dans le groupe consistant en aryle éventuellement substitué, aralkyle éventuellement substitué, aralcényle éventuellement substitué, hétérocyclyle éventuellement substitué, hétérocyclylalkyle éventuellement substitué, hétérocyclylalcényle, hétéroaryle éventuellement substitué, hétéroarylalkyle éventuellement substitué et hétéroarylalcényle éventuellement substitué;
chaque R⁴ est indépendamment l'hydrogène, alkyle, halo, ou haloalkyle; et
chaque R⁵ et R⁶ est indépendamment l'hydrogène, oxo, alkyle, halo ou haloalkyle.

97. Composé selon la revendication 96 où:
m est 1;
n est 1;
p est 2;
V est -C(O)-;
R¹ est l'hydrogène ou alkyle;
R² est aralkyle éventuellement substitué, hétéroarylalkyle éventuellement substitué, ou hétérocyclylalkyle éventuellement substitué;
R³ est cyclopropyle substitué par phényle ;
R⁴ est l'hydrogène, alkyle, halo ou haloalkyle;
R⁵ est l'hydrogène, oxo, alkyle, halo ou haloalkyle; et
chaque R⁶ est l'hydrogène.

98. Composé selon la revendication 97 choisi dans le groupe consistant en les suivants :
6-[4-(2-phényl-cyclopropanecarbonyl)-pipérazin-1-yl]-*N*-(3-phényl-propyl)-nicotinamide;
*N*-(4-phényl-butyl)-6-[4-(2-phényl-cyclopropanecarbonyl)-pipérazin-1-yl]-nicotinamide ;
*N*-(1-méthyl-3-phényl-propyl)-6-[4-(2-phényl-cyclopropanecarbonyl)-pipérazin-1-yl]-nicotinamide;
*N*-phénéthyl-6-[4-(2-phényl-cyclopropanecarbonyl)-pipérazin-1-yl]-nicotinamide;
6-[4-(2-phényl-cyclopropanecarbonyl)-pipérazin-1-yl]-*N*-(tétrahydro-furan-2-yl-méthyl)-nicotinamide;
*N*-[2-(3H-imidazol-4-yl)-éthyl]-6-[4-(2-phényl-cyclopropanecarbonyl)-pipérazin-1-yl]-nicotinamide; et
*N*-(3-imidazol-1-yl-propyl)-6-[4-(2-phényl-cyclopropanecarbonyl)-pipérazin- 1 - yl]-nicotinamide.

99. Composé selon la revendication 95 où:
m est 1 ou 2;
n est 1 ou 2;
p est 2;
V est -C(O)-;
R¹ est l'hydrogène ou alkyle;
R² est choisi dans le groupe consistant en -R⁷-OR⁸, -R⁷-N(R⁸)₂, -R⁷-S(O)ₜR¹⁰ (où t est 0, 1 ou 2), alkyle, alcényle, cycloalkylalkyle éventuellement substitué ou cycloalkylalcényle;
chaque R⁴ est indépendamment l'hydrogène, alkyle, halo, ou haloalkyle;
chaque R⁵ et R⁶ est indépendamment l'hydrogène, oxo, alkyle, halo ou haloalkyle;
chaque R⁷ est une chaîne alkylène linéaire ou ramifiée;
chaque R⁸ est indépendamment l'hydrogène, alkyle, haloalkyle, cycloalkyle, cycloalkylalkyle, aryle et aralkyle; et
R¹⁰ est alkyle, aryle ou aralkyle.

100. Composé selon la revendication 99 où:
m est 1;
n est 1;
p est 2;
V est -C(O)-;
R¹ est l'hydrogène ou alkyle;
R² est choisi dans le groupe consistant en alkyle, -R⁷-OR⁸, -R⁷-N(R⁸)₂, ou -R⁷-S(O)ₜR¹⁰ (où t est 0);
R³ est cyclopropyle substitué par phényle;
R⁴ est l'hydrogène;
R⁵ est l'hydrogène;
chaque R⁶ est l'hydrogène;
R⁷ est une chaîne alkylène linéaire ou ramifiée;
R⁸ est l'hydrogène ou alkyle;
et R¹⁰ est alkyle, aryle ou aralkyle.

101. Composé selon la revendication 100 choisi dans le groupe consistant en les suivants:
*N*-(3-éthoxy-propyl)-6-[4-(2-phényl-cyclopropanecarbonyl)-pipérazin-1-yl]-nicotinamide;
*N*-(2-éthylsulfanyl-éthyl)-6-[4-(2-phényl-cyclopropanecarbonyl)-pipérazin- 1 -yl]-nicotinamide;
*N*-(1,3-diméthylbutyl)-6-[4-(2-phényl-cyclopropanecarbonyl)-pipérazin-1-yl]-nicotinamide;
*N*-(3-méthyl-butyl)-6-[4-(2-phényl-cyclopropanecarbonyl)-pipérazin-1-yl]-nicotinamide;
*N*-(3-méthoxy-propyl)-6-[4-(2-phényl-cyclopropanecarbonyl)-pipérazin-1-yl]-nicotinamide;
*N*-(3-butoxy-propyl)-6-[4-(2-phényl-cyclopropanecarbonyl)-pipérazin-1-yl]-nicotinamide;
*N*-pentyl-6-[4-(2-phényl-cyclopropanecarbonyl)-pipérazin-1-yl]-nicotinamide;
*N*-(3-diméthylamino-propyl)-6-[4-(2-phényl-cyclopropanecarbonyl)-pipérazin-1-yl]-nicotinamide;
*N*-(3-isopropoxy-propyl)-6-[4-(2-phényl-cyclopropanecarbonyl)-pipérazin-1-yl]-nicotinamide;
*N*-(1-méthyl-butyl)-6-[4-(2-phényl-cyclopropanecarbonyl)-pipérazin-l-yl]-nicotinamide;
*N*-butyl-6-[4-(2-phényl-cyclopropanecarbonyl)-pipérazin-1-yl]-nicotinamide;
*N*-hexyl-6-[4-(2-phényl-cyclopropanecarbonyl)-pipérazin-1-yl]-nicotinamide; et
*N*-(2-méthyl-butyl)-6-[4-(2-phényl-cyclopropanecarbonyl)-pipérazin-1-yl]-nicotinamide.
